(19) Europäisches Patentamt / European Patent Office / Office européen des brevets

(11) **EP 1 831 399 B1**

(12) **EUROPEAN PATENT SPECIFICATION**

(45) Date of publication and mention of the grant of the patent: **05.08.2009 Bulletin 2009/32**

(21) Application number: **05853011.4**

(22) Date of filing: **02.12.2005**

(51) Int Cl.: ***C12Q 1/68*** *(2006.01)*

(86) International application number: **PCT/US2005/043974**

(87) International publication number: **WO 2006/071466 (06.07.2006 Gazette 2006/27)**

(54) **METHODS AND NUCLEIC ACIDS FOR THE ANALYSIS OF GENE EXPRESSION ASSOCIATED WITH THE PROGNOSIS OF PROSTATE CELL PROLIFERATIVE DISORDERS**

VERFAHREN UND NUKLEINSÄUREN ZUR ANALYSE VON MIT DER PROGNOSE VON STÖRUNGEN DER PROLIFERATION VON PROSTATAZELLEN ASSOZIIERTER GENEXPRESSION

PROCEDES ET ACIDES NUCLEIQUES POUR L'ANALYSE DE L'EXPRESSION GENETIQUE ASSOCIEE AU PRONOSTIC DE TROUBLES PROLIFERATIFS DE CELLULES PROSTATIQUES

(84) Designated Contracting States: **AT BE BG CH CY CZ DE DK EE ES FI FR GB GR HU IE IS IT LI LT LU LV MC NL PL PT RO SE SI SK TR**

(30) Priority: **02.12.2004 US 632426 P**
**02.12.2004 US 633250 P**
**14.03.2005 US 662220 P**
**03.10.2005 US 723125 P**
**03.10.2005 US 723054 P**
**30.11.2005 US 740736 P**

(43) Date of publication of application: **12.09.2007 Bulletin 2007/37**

(73) Proprietor: **Epigenomics AG**
**10178 Berlin (DE)**

(72) Inventors:
- **COTTRELL, Susan**
  **Seattle, WA 98103 (US)**
- **MODEL, Fabian**
  **12683 Berlin (DE)**
- **HAEFLIGER, Carolina**
  **4052 Basel (CH)**
- **WEISS, Gunter**
  **10437 Berlin (DE)**
- **DISTLER, Jürgen**
  **12163 Berlin (DE)**
- **SLEDZIEWSKI, Andrew, Z.**
  **Shoreline, WA 98177 (US)**
- **SONG, Xiaoling**
  **Woodinville, WA 98072 (US)**
- **SKILLMAN, Thomas, L.**
  **Kennydale, WA 98056 (US)**
- **THOMAS, Jeffrey G.**
  **Oakland, CA 94618 (US)**

(74) Representative: **Krauss, Jan**
**Forrester & Boehmert**
**Pettenkoferstrasse 20-22**
**80336 München (DE)**

(56) References cited:
**EP-A- 1 379 694**
**WO-A-01/19845**
**WO-A-02/070742**
**WO-A-20/04035803**
**WO-A-20/05121360**

- **PAKNESHAN POUYA ET AL: "Methylation status of uPA promoter as a molecular mechanism regulating prostate cancer invasion and growth in vitro and in vivo." FASEB JOURNAL, vol. 17, no. 9, June 2003 (2003-06), pages 1081-1088 URL, XP002385912 ISSN: 0892-6638 cited in the application**
- **CHUN ET AL: "Prostate cancer nomograms: An update" EUROPEAN UROLOGY, vol. 50, no. 5, 11 August 2006 (2006-08-11), pages 914-926, ISSN: 0302-2838**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **COOPERBERG ET AL: "The contemporary management of prostate cancer in the United States: Lessons from the Cancer of the Prostate Strategic Urologic Research Endeavor (CaPSURE), a national disease registry." JOURNAL OF UROLOGY, vol. 171, no. 4, April 2004 (2004-04), pages 1393-1401, ISSN: 0022-5347**
- **D'AMICO ET AL: "Identifying men diagnosed with clinically localized prostate cancer who are at high risk for death from prostate cancer" JOURNAL OF UROLOGY, vol. 176, no. 6,part2, December 2006 (2006-12), pages S11-S15, ISSN: 0022-5347**
- **HUMPHREY PA: "Gleason grading and prognostic factors in carcinoma of the prostate." MODERN PATHOLOGY, vol. 17, no. 3, 13 February 2004 (2004-02-13), pages 292-306, ISSN: 0893-3952**
- **KATTAN ET AL: "Pretreatment nomogram that predicts 5-year probability of metastasis following three-dimensional conformal radiation therapy for localized prostate cancer." JOURNAL OF CLINICAL ONCOLOGY, vol. 21, no. 24, 15 December 2003 (2003-12-15), pages 4568-4571, ISSN: 0732-183X**
- **WALSH ET AL: "Clinical practice. Localized prostate cancer." THE NEW ENGLAND JOURNAL OF MEDICINE, vol. 357, no. 26, 27 December 2007 (2007-12-27), pages 2696-2705, United States ISSN: 1533-4406**
- **DATABASE NCCN [Online] National Comprehensive Cancer Network; "NCCN Clinical Practice Guidelines in Oncology: Prostate Cancer" retrieved from HTTP: //WWW.NCCN.ORG/PROFESSIONALS/ PHYSICIA N_GLS/PDF/PROSTATE.PDF**
- **HEIDENREICH ET AL: "Guidelines on prostate cancer" [Online] StartDateMarker 2007, EndDateMarker ISBN: 978-90-70244-59-0 Retrieved from the Internet: URL:HTTP: //WWW.UROWEB.ORG/FILEADMIN/TX_EAU GUIDELINES/PROSTATE%20CANCER.PDF>**


Remarks:

The file contains technical information submitted after the application was filed and not included in this specification

**Description**

FIELD OF THE INVENTION

Aspects of the present invention relate to human DNA sequences that exhibit heterogenous expression patterns in prostate cancer patients, and more particularly to novel compositions and methods for providing a prognosis of said patients.

SEQUENCE LISTING

A Sequence Lasting, pursuant to PCT Administrative Instructions Part 8, Section 801(a), has been provided on compact disc (1 of 1) as a 3.25 MB text file (476_0001.txt).

BACKGROUND

*Prostate cancer.* Prostate cancer is the most common malignancy among men in the United States (-200,000 new cases per year), and the sixth leading cause of male cancer-related deaths worldwide (-204,000 per year). Prostate cancer is primarily a disease of the elderly, with approximately 16% of men between the ages of 60 and 79 having the disease. According to some estimates at autopsy, 80% of all men over 80 years of age have some form of prostate disease (*e.g.*, cancer, BPH. prostatitis, etc). Benign prostate hypertrophy is present in about 50% of men aged 50 or above, and in 95% of men aged 75 or above. Prostate cancer, based on these reports, is often not a disease that men die from, but more typically-with. Recent evidence suggests that the incidence of prostate cancer may in fact be declining, likely as result of better treatment, better surgery, and earlier detection.

*Diagnosis of prostate cancer*, *molecular approaches.* Current guidelines for prostate cancer screening have been suggested by the American Cancer Society and are as follows: At 50 years of age, health care professionals should offer a blood test for prostate specific antigen (PSA) and perform a digital rectal exam (DRE). It is recommended that high risk populations, such as African Americans and those with a family history of prostate disease, should begin screening at 45 years of age. Men without abnormal prostate pathology generally have a PSA level in blood below 4ng/ml.PSA levels between 4ng/ml and 10ng/ml (called the 'Grey Zone') have a 25% chance of having prostate cancer. The result is that 75% of the time, men with an abnormal DRE and a PSA in this *grey zone* have a negative, or a seemingly unnecessary biopsy. Above the grey zone, the likelihood of having prostate cancer is significant (> 67%) and increases even further as PSA levels go up. Numerous methods exist for measuring PSA (percent-free PSA, PSA velocity, PSA density, etc.), and each has an associated accuracy for detecting the presence of cancer. Yet, even with the minor improvements in detection, and the reported drops in mortality associated with screening, the frequency of false positives remains high. Reduced specificity results in part from increased blood PSA associated with BPH, and prostatis. It has also been estimated that up to 45% of prostate biopsies under current guidelines are falsely negative, resulting in decreased sensitivity even with biopsy.

TRUS guided biopsy is considered the 'gold standard' for diagnosing prostate cancer. Recommendations for biopsy are based upon abnormal PSA levels and or an abnormal DREs. For PSA there is a grey zone where a high percentage of biopsies are perhaps not necessary. Yet the ability to detect cancer in this grey zone (PSA levels of 4.0 to 10 ng/ml) is difficult without biopsy. Due to this lack of specificity, 75% of men undergoing a biopsy do not have cancer. Yet without biopsy, those with cancer would be missed, resulting in increased morbidity and mortality. Unfortunately, the risks associated with an unnecessary biopsy are also high.

Molecular markers would offer the advantage that they can be used to efficiently analyze even very small tissue samples, and samples whose tissue architecture has not been maintained. Within the last decade, numerous genes have been studied with respect to differential expression among benign hyperplasia of the prostate and different grades of prostate cancer. However, no single marker has as yet been shown to be sufficient for the prognostic classification of prostate tumors in a clinical setting.

Alternatively, high-dimensional mRNA-based approaches may, in particular instances, provide a means to distinguish between different tumor types and benign and malignant lesions. However, application of such approaches as a routine diagnostic tool in a clinical environment is impeded and substantially limited by the extreme instability of mRNA, the rapidly occurring expression changes following certain triggers (*e.g.*, sample collection), and, most importantly, by the large amount of mRNA needed for analysis which often cannot be obtained from a routine biopsy (see, *e.g.*, Lipshutz, R. J. et al., Nature Genetics 21:20-24, 1999; Bowtell, D. D. L. Nature Genetics Suppl. 21:25-32, 1999).

Aberrant genetic methylation in prostate cancer has been observed in several genes including GSTPi, AR, p16 (CDKN2a/INK4a), CD44, CDH1. Genome-wide hypomethylation for example of the LINE-1 repetitive element has also been associated with tumor progression (Santourlidis, S., et al., Prostate 39:166-74, 1999).

*Prostate Cancer Treatment Options.* There are many treatment strategies available to patients diagnosed with prostate cancer, and the decision for the patients and physicians is often unclear. Because prostate cancer can be a slowly developing disease, many men choose a treatment approach called watchful waiting, or conservative management. As the names imply, this approach does not include any radical therapy intended to cure the patient. Instead, the disease is carefully monitored using PSA tests and DREs. The ideal patient for this approach is one whose tumor is slow growing and non-invasive, and who is therefore likely to die of other causes before the prostate cancer becomes problematic.

For younger patients with localized disease, curative treatment is more appropriate. Radical prostatectomy is used to remove the prostate and hopefully all traces of the tumor. The surgical margins, seminal vesicles, and sometimes lymph nodes are tested for the presence of cancer, and in each case the presence of cancer correlates with reduced disease free survival Overall, about 70% of men remain free of disease ten years after surgery (Roehl, et al., 2004). Radical prostatectomy is a significant surgery, with side effects including blood loss, incontinence, and impotence. The rate of intraoperative and postoperative complications is estimated to be less than 2% (Lepor, et al., 2001).

Radiation therapy is also used to attempt to cure prostate cancer patients. Patients can choose either external beam radiation or brachytherapy (radioactive seed implants). The rates of survival and the side effects are similar to radical prostatectomy (D'amico, et al., 1998). For both radical prostatectomy and radiation therapy, the probability of survival is highly dependent on the stage and differentiation of the tumor. Localized indolent tumors are more likely to be cured.

Hormonal therapy is often used for patients whose cancer has spread beyond the prostate or for patients whose cancer has recurred after prostatectomy or radiation therapy. In other words, hormonal therapy is used to control cancer but not to cure it. Hormonal therapy is sometimes used in conjunction with other therapies such as radiation or as a neo-adjuvant therapy prior to surgery. The goal of hormonal therapy is to reduce the stimulatory effect of androgens on the prostate tumor. The reduction in hormones is achieved through orchiectomy, lutenizing hormone-releasing hormone (LHRH) analogs, and antiandrogens. Side effects of hormonal therapy can include impotence, hot flashes, fatigue, and reduced libido. Eventually, prostate tumors become insensitive to androgens and hormonal therapy is no longer effective.

After the tumor has spread outside the capsule and hormonal therapy has failed, chemotherapy can be used to relieve pain or delay the progression of the disease. The response to chemotherapy is variable, and lives are extended for only a minority of patients. Bisphosphonates are used to reduce the osteolytic activity of tumors metastasised to the bones.

*Prostate Cancer Prognosis Estimation.* DRE, TRUS, biopsy, and PSA provide initial staging information on the tumor, but MRI, CT scans, ProstaScint scans and bone scans are used to determine the spread of the cancer beyond the prostatic capsule. These tests are not used on every prostate cancer patient, but only those with some likelihood of metastases. If metastases can be confirmed, the patient will receive treatment designed to slow the progression of the disease. If no metastases are detected, a patient is a candidate for potentially curative treatments such as prostatectomy and radiation therapy. Prior to the removal of the prostate, lymph nodes are sometimes dissected as a final test for metastases. If metastases are present in the dissected nodes, the surgery may be aborted. Analysis of the tissue surgically removed during prostatectomy is the final and gold standard staging technique for those patients who choose to undergo surgery. Frequently, analysis of the surgical specimens shows that the patient was originally *understaged* by the diagnostic tests (Bostwick, 1997).

An accurate estimation of prognosis is crucial for selection of the most appropriate treatment for each patient. Since organ confined prostate cancer cannot lead to death, estimation of prognosis is also an estimation of the presence or likelihood of development of metastases. A patient who is likely to develop cancer outside of the prostatic capsule will receive more extensive diagnostic work-up, including MRI and CT scans, and possibly more radical treatments, including surgery and radiation.

An initial prognostic assessment is made from the results of a PSA test, DRE, and biopsy analysis. The size, location, and method of detection of the tumor are combined to give a staging score on the TNM scale. Patients with higher stage tumors and high PSA values are more likely to have cancer that has spread or will spread outside of the prostate. A histological analysis of the biopsy allows a pathologist to determine the Gleason score. The Gleason score is a composite of the two most prevalent grades in the tissue sample, and the grades can range between one and five. A higher grade indicates more extreme dedifferentiation, and higher composite scores correlate with higher probability for metastasis and reduced disease free survival.

Prostate cancer nomograms have been developed and modified to predict the risk of cancer recurrence after primary therapy based on PSA levels, Gleason grading, and pre-operative staging information (Kattan et al 2003; Kattan et al 1998; Potter et al 2001). The data is derived from actual patient survival rates in cohorts of thousands of patients at multiple institutions. As with all prognostic measurements in prostate cancer, the estimate of recurrence risk by the nomogram is also an estimate of the likelihood of presence of cancer outside the prostatic capsule. Because the clinical characteristics of the cancers that patients are presenting with have changed with the widespread use of PSA, the nomograms are out of date and are not widely used. However, the general process of integrating Gleason, stage, and PSA information is still used.

Patients with cancer that has spread to lymph nodes or other metastatic sites are treated with systemic therapies such as hormonal therapies. Patients with localized disease (T1-T3) are candidates for definitive, curative treatments such as surgery or radiation. Those patients with localized disease who are thought to be low-risk are ideal candidates for watchful waiting. Those with intermediate risk are ideal for monotherapy such as surgery or radiation. Those with high risk localized disease should be considered for multimodal therapies or clinical trials.

After surgery, more prognostic information is available because the tumor spread can be directly analyzed. During some prostatectomies, the lymph nodes are directly dissected and the nodal status is confirmed. In all surgeries, the tumor spread to the seminal vesicles and the margin status are checked. Positive nodes, seminal vesicles, and margins all indicate an inferior prognosis and may suggest that the patient should receive adjuvant treatment.

*Molecular prostate cancer prognostic markers; deficiencies of prior art approaches.* As an alternative to current approaches to the prognostic classification of prostate carcinoma patients a variety of molecular approaches are currently being explored. It is anticipated that the development of suitable molecular markers will have significant advantages over current approaches in terms of accuracy, cost-effectiveness and/or patient invasiveness. A variety of molecular markers have been discovered including monoclonal antibodies. In a study by Xu et al (ICDB/95613763) 114 cases of prostate cancer showed that 57% of the bone marrow specimens had elevated OVX1 levels (greater than 7.2 U/ml). In other experiments, OVX1 levels were about 2-fold higher in serum samples from androgen-independent than from androgen-dependent prostate cancer patients (p less than 0.001), suggesting that serum OVX1 levels may be able to predict the progression of prostate cancer, since this disease when it progresses typically becomes androgen-independent. Expression of the PSCA protein and mRNA has been positively correlated with adverse tumor characteristics, such as increasing pathological grade (poor cell differentiation), worsening clinical stage and androgen-independence and speculatively with prostate carcinogenesis (Jpn J Clin Oncol. 4:414-9, 2004). Other prospective mRNA analysis markers include Hepsin. Expression of Hepsin showed significant difference between patients at lower risk (pT2, G2 and Gleason score less than 7) and higher risk (pT3/4, G3 and Gleason score 7 or greater) for relapse (J Urol, 171:187-91, 2004).

The GSTPi gene is the most well characterized prostate carcinoma diagnostic marker. Zhou et al. (J Urol, 171: 2195-8.2004) recently correlated expression of the GSTPi gene with Gleason grade and cancer volume. Furthermore, use of the gene GSTPi as a marker for the detection of prostate carcinomas located in the peripheral zone (i.e., with a high likelihood of metastasis) has also been described in US 2004146868.

Another methylation marker which may be suitable for the prognostic classification of prostate carcinomas is uPA. Rabbani et al. (The FASEB Journal 17:1081-1088, 2003) have shown that the uPA promoter is hypermethylated in hormone-responsive PrEC and LNCaP cells and hypomethylated in hormone-insensitive PC-3 cells. De-methylation of the promoter in the LNCaP cell lines resulted in increase of mRNA analysis and resulted in an increase in the invasive capacity. Singal et al., analysed methylation of a gene panel consisting of glutathione s-transferase Pi1 (GSTP1), retinoic acid receptor beta (RARB), CD44, E-cadherin (ECAD) and RAS association domain family protein 1A (RASSF1A) in prostate cancer. A methylation index (MI) was calculated as the total number of genes methylated, higher MI was noted in stage III as compared to stage II disease, and in Gleason score 7 as compared to Gleason score 6 samples. Singal et al. thus concluded that the results suggest that the methylation of the gene panel in correlated with clinicopathological features of poor prognosis.

*Pronounced need in the art.* Significantly, however, none of the heretofore mentioned markers are sufficiently developed to provide a marker for the prognosis of prostate cell proliferative disorders that is sufficiently robust and/or accurate for effective use in a clinical setting.

While accurate diagnosis of prostate carcinoma is important, the most pressing need in prostate cancer treatment is for information to guide the treatment planning decision. Leaders in the field agree that many patients with clinically insignificant disease receive unnecessary radical treatments such as prostatectomy or radiation therapy. However, twenty percent of patients who do receive these curative treatments experience disease recurrence. A molecular test could help select patients for the optimal treatment choice and thereby reduce over and under treatment

Currently the therapy choice is made based on the likelihood of spread of the disease. Low-risk patients are candidates for watchful waiting. Medium and high risk patients should receive surgery or radiation, and the high risk patients are candidates for additional adjuvant treatments. Staging, Gleason, and PSA are currently used to estimate this risk, but the combined information from these tests is insufficient. Very few patients are recommended for watchful waiting because clinicians cannot be sure which cancers are indolent Furthermore, many patients who receive monotherapy experience a recurrence.

Gleason grading currently plays a primary role in prognostic assessment. Patients with localized disease and high Gleason scores (8-10) always undergo radical treatments. Patients with low Gleason scores (2-5) have the option of deferring curative treatment and opting for watchful waiting, however many chose to undergo curative therapy soon after diagnosis. For patients with mid-range Gleason scores, which is the majority of patients diagnosed today, clinicians must use other less-reliable prognostic indicators for further information.

Accordingly there is a pronounced need in the art for a novel, effective prognostic test, and in particular one that would predict the probability that a cancer has or is likely to spread outside of the prostate based on the methylation patterns of biopsy samples. This type of information is highly valuable in the diagnostic and treatment planning processes. This information would initially be used in reaching the decision about whether imaging tests are necessary to check for metastasis for a complete diagnostic work-up. Surgery is unnecessary for any patient whose cancer has already spread, but if a patient is not selected for imaging the metastases will not be detected until surgery or later.

Furthermore there is a pronounced need in the art for a novel and effective prostate cell proliferative disorder molecular classification test, and in particular one that would be suitable for the analysis of biopsy samples to improve the stratification of patients into low, intermediate, and high risk categories so that optimal treatment plans can be selected for each patient. With accurate stratification, patients and doctors can choose watchful waiting with confidence that there is little risk for early recurrence. This test would therefore reduce the number of unnecessary surgeries and radiation treatments.

Additionally, with improved estimations of which patients are likely to recur with monotherapy, physicians can make better use of available adjuvant treatments. If a patient chooses to undergo surgery, the test can be repeated on prostatectomy samples to verify the assessment of his need for adjuvant therapy. The benefits of different adjuvant therapy approaches are still being worked out in clinical trials, and a molecular test could provide valuable information to stratify patients for this additional treatment or for clinical trials. PITX2 (Paired-like homeodomain transcription factor 2), also known as PTX2, RIEG1, or ARP1, encodes a member of the RIEG/PITX homeobox family, which is in the bicoid class of homeodomain proteins. PITX2 encodes several alternative transcripts, and mutations in the gene lead to the autosomal-dominant disorder Rieger's syndrome, a developmental disorder predominantly affecting the eye (Semina *et al.,* 1996). The protein acts as a transcription factor and is involved in the development of several major organs. It is induced by the WNT pathway, and mediates cell-type specific proliferation by inducing growth-regulating genes (Kioussi *et al.* 2002).

Toyota *et al.* (2001) found hypermethylation of the gene in a large proportion of acute myeloid leukemias. Several studies by the applicant (see WO 2005/059172) have demonstrated that hypermethylation of PITX2 is associated with poor prognosis for breast cancer patients.

The GPR7 marker is located in a CpG island in the promoter region of an intronless gene on chromosome 10. GPR7, or G-protein receptor 7, is a receptor for neuropeptide W and neuropeptide B (Shimomura *et al.* 2002; Tanaka *et al.* 2003). The expression of GPR7 has been studied in the brain, and is expressed mainly in the cerebellum and frontal cortex (O'Dowd *et al*. 1995). Ishii et al. (2003) studied the phenotype of mice lacking a functional copy of GPR7. The mice developed adult-onset obesity and metabolic defects such as decreased energy expenditure and increased blood levels of glucose and insulin. Interestingly, these phenotypes were only detected in male mice. The GPR7 ligands, neuropeptides W and B, have also been implicated in metabolism and obesity in separate studies (Samson *et al.* 2004; Levine *et al.* 2005). GPR7, which is similar in sequence to opioid preceptors, may also have a role in pain signaling (Zaratin *et al.* 2005).

SEQ ID NO: 63 is located within the regulatory region of HIST2H2BF on chromosome 1 in a region with several histone genes. The histone content and status of chromatin can influence the expression of the encoded gene. Methylation and altered expression of a histone gene in prostate cancer could cause chromatin changes throughout the genome that alter gene expression in ways that result in more aggressive tumor properties. There are no published articles on the function of this Particular histone.

The marker referred to as SEQ ID NO: 35 is located on chromosome 3 downstream of the FOXL2 (Forkhead transcription factor) gene and within or near predicted genes or ESTs. Although it is downstream, it is anticipated that methylation of this marker effects the expression of FOXL2, which is mutated in the blepharophimosis-ptosis epicanthus inversus syndrome (BPES). This syndrome is characterized by eye, craniofacial, and ovarian abnormalities. Methylation of the marker may also affect the expression of the EST, or the EST may be shown to be an alternative exon for the FOXL2 gene.

WO 01/19845 discloses a method for detecting a cellular proliferative disorder (e.g. prostate cancer) in a subject comprising: a) contacting a nucleic acid-containing specimen from the subject with an agent that provides a determination of the methylation state of at least one gene or associated regulatory region of the gene (e.g. PITX2); and b) identifying aberrant methylation of regions of the gene or regulatory region, wherein aberrant methylation is identified as being different when compared to the same regions of the gene or associated regulatory region in a subject not having said cellular proliferative, thereby detecting a cellular proliferative disorder in the subject. WO 01/19845 does not disclose a method for providing a prognosis of a subject with a prostate cell proliferative disorder.

EP 1 379 694 discloses methods, systems and computer program products for determining the biological effect and/or activity of drugs, chemical substances and/or pharmaceutical compositions using their effect on DNA-methylation as a marker for their biological effect(s). PITX2 is mentioned. WO 02/070742 discloses a method for the development of gene panels for diagnostic and therapeutic purposes based on the expression and methylation status of specific genes. PITX2 is mentioned. Finally, WO 2004/035803 also discloses the bisulphite treated PITX2 gene.

None of the above references discloses the association between the methylation pattern of PITX2 and the prognosis of a post-surgical prostate cell proliferative disorder, and that methylation of the PITX2 gene can be used for providing said prognosis of a prostate cell proliferative disorder.

SUMMARY OF THE INVENTION

The present invention provides novel and efficacious methods and nucleic acids for providing a prognosis of prostate cell proliferative disorders.

The invention solves this longstanding need in the art by providing a method for providing a prognosis of a subject with a prostate cell proliferative disorder comprising the following steps of: a) determining the methylation status of the gene PITX2 and/or regulatory regions thereof in a biological sample obtained from said subject; and b) determining therefrom the prognosis of said subject whereby hypermethylation is indicative of poor prognosis.

The present invention is generally limited to the methylation analysis of the gene PITX2 and/or regulatory regions thereof. Other preferred aspects of the present invention are claimed in claims 2 to 7.

The present invention provides a method for ascertaining genetic and/or epigenetic parameters of genomic DNA. The method has utility for the improved prognostic classification of prostate cell proliferative disorders, more specifically by enabling the improved identification of and differentiation between aggressive and non-aggressive forms of said disorder. The invention presents several substantial improvements over the state of the art. Although some methylation assays for the detection of cancer are known, there is currently no molecular classification system for the *prognostic* classification of tumours.

The DNA source may be any suitable source. Preferably, the source of the DNA sample is selected from the group consisting of cells or cell lines, histological slides, biopsies, paraffin-embedded tissue, bodily fluids, ejaculate, urine, blood, and combinations thereof. Preferably, the source is biopsies, bodily fluids, ejaculate, urine, or blood.

Specifically, the present invention provides a method for providing a prognosis of prostate cell proliferative disorders, comprising: obtaining a biological sample comprising genomic nucleic acid(s); contacting the nucleic acid(s), or a fragment thereof, with one reagent or a plurality of reagents sufficient ‚iT" for distinguishing between methylated and non methylated CpG dinucleotide sequences within the gene PITX.

Preferably, said sequence is selected from the goup consisting of SEQ ID Nos: 962 -965

BRIEF DESCRIPTION OF THE DRAWINGS

Figure 1 shows a ROC plot for a SEQ ID NO:19 MSP assay run on 26 frozen radical prostatectomy samples from patients with early PSA recurrence, and on 30 samples from patients with no PSA recurrence after at least 48 months.

Figure 2 shows a ROC plot for a SEQ ID NO:35 MSP assay run on 26 frozen radical prostatectomy samples from patients with early PSA recurrence and 30 samples from patients with no PSA recurrence after at least 48 months.

Figure 3 shows a ROC plot for a SEQ ID NO: 37 MSP assay run on 26 frozen radical prostatectomy samples from patients with early PSA recurrence and 30 samples from patients with no PSA recurrence after at least 48 months.

Figure 4 shows a ROC plot for a SEQ ID NO: 7 MSP assay run on 26 frozen radical prostatectomy samples from patients with early PSA recurrence and 30 samples from patients with no PSA recurrence after at least 48 months.

Figure 5 shows a ROC plot for a SEQ ID NO: 63 MSP assay run on 26 frozen radical prostatectomy samples from patients with early PSA recurrence and 30 samples from patients with no PSA recurrence after at least 48 months.

Figure 6 shows a ROC plot for a SEQ ID NO: 8 MSP assay run on 26 frozen radical prostatectomy samples from patients with early PSA recurrence and 30 samples from patients with no PSA recurrence after at least 48 months.

Figure 7 shows a ROC plot for a SEQ ID NO: 64 MSP assay run on 26 frozen radical prostatectomy samples from patients with early PSA recurrence and 30 samples from patients with no PSA recurrence after at least 48 months.

Figure 8 shows a gel electrophoresis analysis on 12 DNA samples. 200 ng per DNA was applied to a 0.8 % agarose gel. The gel was run for 4 hours at 80 Volt. The size marker (Invitrogen, No.: 10496-016) contains the following fragments: 10.000 bp, 6.000 bp.

Figure 9 shows ALF Express analyses of multiplex PCR products (8plex, mPCR SetD2) compared to single PCR products (sPCR Set D2). The size standard (lanes 1,4) contained fragments of the following lengths: 50, 100, 150, 200, 250, 300, 350, 400, 450, 500 bp. All fragments could be amplified. An undesired side product (220 bp) was observed.

Figure 10 shows performance of multiplex PCR. Lane 1: 100bp marker. Lanes 2-11: multiplex PCR performance of 10 test samples, lane 12: positive control, lane 13: H2O control.

Figure 11 shows Tumor vs. Lymphocyte samples, ranked by Wilcoxon statistics. Bonferroni corrected p-values (upper) and AUCs (lower) are shown to the right of the data matrix. Each column represents one sample; each row one oligonucleotide. Oligonucleotides are grouped per marker candidate. The indicated markers are ordered from top to bottom with increasing AUC. On the right side of each marker Bonferroni corrected Wilcoxon p-value and AUC are given. Below the AUC sensitivity at a specificity of - 0.75 are given enclosed in brackets. Methylation data are centered and normalized to one standard deviation for individual oligonucleotides. The color represents the relative distance of the oligonucleotide methylation status from the mean value. Light grey represents hypomethylated CpGs within an oligonucleotide while dark grey indicates hypermethylated CpGs within an oligonucleotide.

Figure 12 shows high Gleason vs. Low Gleason marker rankings. The plot displays uncorrected p-values from the genewise Wilcoxon rank statistics analysis. Lower and upper dotted lines show 5% Bonferroni and FDR limits, respectively.

Figure 13 shows high Gleason vs. Low Gleason methylation matrix of the 10 markers with best AUC. Gleason scores are shown above each group of samples. Each column represents one sample; each row one oligonucleotide (1, 2, or 3 CpG sites each). Oligonucleotides are grouped per marker candidate. The indicated markers are ordered from top to bottom with increasing AUC. On the right side of each marker Bonferroni corrected Wilcoxon p-value and AUC are given. Below the AUC sensitivity at a specificity of - 0.75 are given enclosed in brackets. Methylation data are centered and normalized to one standard deviation for individual oligonucleotides. The color represents the relative distance of the oligonucleotide methylation status from the mean value. Light grey represents hypomethylated CpGs within an oligonucleotide while dark grey indicates hypermethylated CpGs within an oligonucleotide.

Figure 14 shows Early Recurrence vs. No recurrence marker rankings. The plot gives uncorrected p-values from the genewise Wilcoxon rank test analysis. Lower and upper dotted lines show 5% Bonferroni and FDR limits, respectively.

Figure 15 shows Early Recurrence vs. No recurrence methylation matrix of the 10 markers with best AUC. Each column represents one sample; each row one oligonucleotide (1, 2, or 3 CpG sites each). Oligonucleotides are grouped per marker candidate. The indicated markers are ordered from top to bottom with increasing AUC. On the right side of each marker Bonferroni corrected Wilcoxon p-value and AUC are given. Below the AUC sensitivity at a specificity of ~ 0.75 are given enclosed in brackets. Methylation data are centered and normalized to one standard deviation for individual oligonucleotides. The color represents the relative distance of the oligonucleotide methylation status from the mean value. Light grey represents hypomethylated CpGs within an oligonucleotide while dark grey indicates hypermethylated CpGs within an oligonucleotide.

For Figures 16-88, each figure shows the sequence of the analysed amplificate of each respective SEQ ID NO. In each figure, an analyzed amplificate is displayed in a 'wrapped' series of panels, where the first row (top row) in each panel shows the *genomic* sequence being amplified (the genomic sequence row), and where forward and reverse amplification primers (defining an 'amplicon') are shown in the panel row (the primer display row) immediately below the first row. The row below the primer display row (or, in panels not displaying a primer, the row below the genomic display row) is the bisulfite converted sequence of the amplificate (the bisulfite converted sequence row; wherein CpG positions are marked red). The remaining rows displayed in some panels, show the sequences of detection oligonucleotides (CG and TG oligos) used to analyze the amplificate.

Figure 16 shows an Amplificate of SEQ ID NO:14.
Figure 17 shows an Amplificate of SEQ ID NO:15.
Figure 18 shows an Amplificate of SEQ ID NO:16.
Figure 19 shows an Amplificate of SEQ ID NO:17.
Figure 20 shows an Amplificate of SEQ ID NO:18
Figure 21 shows an Amplificate of SEQ ID NO:19
Figure 22 shows an Amplificate of SEQ ID NO:20
Figure 23 shows an Amplificate of SEQ ID NO:21
Figure 24 shows an Amplificate of SEQ ID NO:22
Figure 25 shows an Amplificate of SEQ ID NO:23
Figure 26 shows an Amplificate of SEQ ID NO:24
Figure 27 shows an Amplificate of SEQ ID NO:25
Figure 28 shows an Amplificate of SEQ ID NO:26
Figure 29 shows an Amplificate of SEQ ID NO:27
Figure 30 shows an Amplificate of SEQ ID NO:28
Figure 31 shows an Amplificate of SEQ ID NO:29
Figure 32 shows an Amplificate of SEQ ID NO:30
Figure 33 shows an Amplificate of SEQ ID NO:31
Figure 34 shows an Amplificate of SEQ ID NO:32 (amplificate A)
Figure 35 shows an Amplificate of SEQ ID NO:32 (amplificate B)
Figure 36 shows an Amplificate of SEQ ID NO:33
Figure 37 shows an Amplificate of SEQ ID NO:34
Figure 38 shows an Amplificate of SEQ ID NO:35
Figure 39 shows an Amplificate of SEQ ID NO:13

Figure 40 shows an Amplificate of SEQ ID NO:36
Figure 41 shows an Amplificate of SEQ ID NO:37
Figure 42 shows an Amplificate of SEQ ID NO:1
Figure 43 shows an Amplificate of SEQ ID NO:2
Figure 44 shows an Amplificate of SEQ ID NO:3
Figure 45 shows an Amplificate of SEQ ID NO:4
Figure 46 shows an Amplificate of SEQ ID NO:5
Figure 47 shows an Amplificate of SEQ ID NO:6
Figure 48 shows an Amplificate of SEQ ID NO:7
Figure 49 shows an Amplificate of SEQ ID NO:38
Figure 50 shows an Amplificate of SEQ ID NO:39
Figure 60 shows an Amplificate of SEQ ID NO:40
Figure 61 shows an Amplificate of SEQ ID NO:41
Figure 62 shows an Amplificate of SEQ ID NO:42
Figure 63 shows an Amplificate of SEQ ID NO:43
Figure 64 shows an Amplificate of SEQ ID NO:44
Figure 65 shows an Amplificate of SEQ ID NO:45
Figure 66 shows an Amplificate of SEQ ID NO:46
Figure 67 shows an Amplificate of SEQ ID NO:47
Figure 68 shows an Amplificate of SEQ ID NO:48
Figure 69 shows an Amplificate of SEQ ID NO:49
Figure 70 shows an Amplificate of SEQ ID NO:50
Figure 71 shows an Amplificate of SEQ ID NO:51
Figure 72 shows an Amplificate of SEQ ID NO:52
Figure 73 shows an Amplificate of SEQ ID NO:53
Figure 74 shows an Amplificate of SEQ ID NO:54
Figure 75 shows an Amplificate of SEQ ID NO:55
Figure 76 shows an Amplificate of SEQ ID NO:56
Figure 77 shows an Amplificate of SEQ ID NO:57
Figure 78 shows an Amplificate of SEQ ID NO:58
Figure 79 shows an Amplificate of SEQ ID NO:59
Figure 80 shows an Amplificate of SEQ ID NO:60
Figure 81 shows an Amplificate of SEQ ID NO:61
Figure 82 shows an Amplificate of SEQ ID NO:62
Figure 83 shows an Amplificate of SEQ ID NO:8
Figure 84 shows an Amplificate of SEQ ID NO:9
Figure 85 shows an Amplificate of SEQ ID NO:10
Figure 86 shows an Amplificate of SEQ ID NO:11
Figure 87 shows an Amplificate of SEQ ID NO:12 (amplificate A)
Figure 88 shows an Amplificate of SEQ ID NO:12 (amplificate B)

Figure 89 shows the distribution of follow up times of patients as analysed in Example 5. The white bars represent the distribution of all censored (no PSA relapse) patients. The grey bars show the distribution of the PSA-free survival time for all of the relapse patients. Frequency is shown on the Y-axis and time (months) is shown on the X-axis.

Figure 90 shows Kaplan-Meier survival analysis of the PITX2 marker (A & B) and ROC curve analysis (C) of the marker PITX2 in differentiating between prostate cancer patients according to Example 5. Proportion of recurrence-free patients is shown on the Y-axis, time in years is shown on the x-axis.

Figure 91 shows Kaplan-Meier survival analysis of the GPR7 marker (A & B) and ROC curve analysis (C) of the marker PITX2 in differentiating between prostate cancer patients according to Example 5. Proportion of recurrence-free patients is shown on the Y-axis, time in years is shown on the x-axis.

Figure 92 shows Kaplan-Meier survival analysis of the SEQ ID NO: 63 marker (A & B) and ROC curve analysis (C) of the marker PITX2 in differentiating between prostate cancer patients according to Example 5. Proportion of recurrence-free patients is shown on the Y-axis, time in years is shown on the x-axis.

Figure 93 shows Kaplan-Meier survival analysis of the SEQ ID NO: 35 marker (A & B) and ROC curve analysis (C) of the marker PITX2 in differentiating between prostate cancer patients according to Example 5. Proportion of recurrence-free patients is shown on the Y-axis, time in years is shown on the x-axis.

Figure 94 shows Kaplan-Meier survival analysis of the ABHD9 marker (A & B) and ROC curve analysis (C) of the marker PITX2 in differentiating between prostate cancer patients according to Example 5. Proportion of recurrence-free patients is shown on the Y-axis, time in years is shown on the x-axis.

Figure 95 shows Kaplan-Meier survival analysis of the CCND2 marker (A & B) and ROC curve analysis (C) of the marker PITX2 in differentiating between prostate cancer patients according to Example 5. Proportion of recurrence-free patients is shown on the Y-axis, time in years is shown on the x-axis.

Figure 96 shows Kaplan-Meier survival analysis of PITX2 performance on sub-populations based on stage according to Example 5.

Figure 97 shows Kaplan-Meier survival analysis of PITX2 performance on sub-populations based on Gleason score according to Example 5.

Figure 98 shows Kaplan-Meier survival analysis of PITX2 performance on sub-populations based on nomogram score according to Example 5.

Figure 99 shows Kaplan-Meier survival analysis of SEQ ID NO: 63 performance on sub-populations based on High Gleason score according to Example 5.

Figure 100 shows Kaplan-Meier survival analysis of SEQ ID NO: 63 performance on sub-populations based on poor nomogram score according to Example 5.

Figure 101 shows Kaplan-Meier survival analysis of SEQ ID NO: 35 performance on T2 sub-populations according to Example 5.

Figure 102 shows the detected amplificate in both frozen and PET samples in the early biochemical relapse vs. no biochemical relapse comparisons using the assay of SEQ ID NO: 19 shown in Table 12 as detailed in Example 6.

Figure 103 shows the detected amplificate in both frozen and PET samples in the early biochemical relapse vs. no biochemical relapse comparisons using the assay of SEQ ID NO: 63 shown in Table 12 as detailed in Example 6.

Figure 104 shows the detected amplificate in both frozen and PET samples in the early biochemical relapse vs. no biochemical relapse comparisons using the assay of SEQ ID NO: 35 shown in Table 12 as detailed in Example 6.

Figure 105 shows the detected amplificate in both frozen and PET samples in the early biochemical relapse vs. no biochemical relapse comparisons using the assay of SEQ ID NO: 37 shown in Table 12 as detailed in Example 6.

Figure 106 shows the detected amplificate in PET samples only in the early biochemical relapse vs. no biochemical relapse comparisons using the assay of SEQ ID NO: 19 shown in Table 12 as detailed in Example 6.

Figure 107 shows the detected amplificate in PET samples only in the early biochemical relapse vs. no biochemical relapse comparisons using the assay of SEQ ID NO: 63 shown in Table 12 as detailed in Example 6.

Figure 108 shows the detected amplificate in PET samples only in the early biochemical relapse vs. no biochemical relapse comparisons using the assay of SEQ ID NO: 35 shown in Table 12 as detailed in Example 6.

Figure 109 shows the detected amplificate in PET samples only in the early biochemical relapse vs. no biochemical relapse comparisons using the assay of SEQ ID NO: 37 shown in Table 12 as detailed in Example 6.

Figure 110 shows the detected amplificate in frozen samples only in the early biochemical relapse vs. no biochemical relapse comparisons using the assay of SEQ ID NO: 19 shown in Table 12 as detailed in Example 6.

Figure 111 shows the detected amplificate in frozen samples only in the early biochemical relapse vs. no biochemical relapse comparisons using the assay of SEQ ID NO: 63 shown in Table 12 as detailed in Example 6.

Figure 112 shows the detected amplificate in frozen samples only in the early biochemical relapse vs. no biochemical relapse comparisons using the assay of SEQ ID NO: 35 shown in Table 12 as detailed in Example 6.

Figure 113 shows the detected amplificate in frozen samples only in the early biochemical relapse vs. no biochemical relapse comparisons using the assay of SEQ ID NO: 37 shown in Table 12 as detailed in Example 6.

Figure 114 shows the detected amplificate in both frozen and PET samples in the High Gleason vs. Low Gleason comparisons using the assay of SEQ ID NO: 19 shown in Table 12 as detailed in Example 6.

Figure 115 shows the detected amplificate in both frozen and PET samples in the High Gleason vs. Low Gleason comparisons using the assay of SEQ ID NO: 63 shown in Table 12 as detailed in Example 6.

Figure 116 shows the detected amplificate in both frozen and PET samples in the High Gleason vs. Low Gleason comparisons using the assay of SEQ ID NO: 35 shown in Table 12 as detailed in Example 6.

Figure 117 shows the detected amplificate in both frozen and PET samples in the High Gleason vs. Low Gleason comparisons using the assay of SEQ ID NO: 37 shown in Table 12 as detailed in Example 6.

Figure 118 shows the detected amplificate in PET samples only in the High Gleason vs. Low Gleason comparisons using the assay of SEQ ID NO: 19 shown in Table 12 as detailed in Example 6.

Figure 119 shows the detected amplificate in PET samples only in the High Gleason vs. Low Gleason comparisons using the assay of SEQ ID NO: 63 shown in Table 12 as detailed in Example 6.

Figure 120 shows the detected amplificate in PET samples only in the High Gleason vs. Low Gleason comparisons using the assay of SEQ ID NO: 35 shown in Table 12 as detailed in Example 6.

Figure 121 shows the detected amplificate in PET samples only in the High Gleason vs. Low Gleason comparisons using the assay of SEQ ID NO: 37 shown in Table 12 as detailed in Example 6.

Figure 122 shows the detected amplificate in frozen samples only in the High Gleason vs. Low Gleason comparisons using the assay of SEQ ID NO: 19 shown in Table 12 as detailed in Example 6.

Figure 123 shows the detected amplificate in frozen samples only in the High Gleason vs. Low Gleason comparisons using the assay of SEQ ID NO: 63 shown in Table 12 as detailed in Example 6.

Figure 124 shows the detected amplificate in frozen samples only in the High Gleason vs. Low Gleason comparisons using the assay of SEQ ID NO: 35 shown in Table 12 as detailed in Example 6.

Figure 125 shows the detected amplificate in frozen samples only in the High Gleason vs. Low Gleason comparisons using the assay of SEQ ID NO: 37 shown in Table 12 as detailed in Example 6.

## DETAILED DESCRIPTION OF THE INVENTION

### Definitions:

As used herein the term expression shall be taken to mean the transcription and translation of a gene. The level of expression of a gene may be determined by the analysis of any factors associated with or indicative of the level of transcription and translation of a gene including but not limited to methylation analysis, loss of heterozygosity (hereinafter also referred to as LOH), RNA expression levels and protein expression levels.

Furthermore the activity of the transcribed gene may be affected by genetic variations such as but not limited genetic mutations (including but not limited to SNPs, point mutations, deletions, insertions, repeat length, rearrangements and other polymorphisms).

As used herein the term "prognosis" shall be taken to mean a prediction of the progression of the disease (for example but not limited to regression, stasis and metastasis), in particular aggressiveness and metastatic potential of a prostate tumor.

As used herein the term "prognostic marker" shall be taken to mean an indicator of a prediction of the progression of the disease, in particular aggressiveness and metastatic potential of a prostate tumor.

As used herein the term "prognostic classification" shall be taken to mean the classification of a prostate cell proliferative disorder according to a prediction of the progression of the disease, in particular aggressiveness and metastatic potential of a prostate tumor.

It is preferably used to define patients with high, low and intermediate risks of death or recurrence after treatment that result from the inherent heterogeneity of the disease process. As used herein the term "aggressive" as used with respect to prostate tumor shall be taken to mean a prostate cell proliferative disorder that has the biological capability to rapidly spread outside of the prostate. Indicators of tumor aggressivness standard in the art include but are not limited to tumor stage, tumor grade, Gleason grade, nodal status and survival. As used herein the term "survival" shall not be limited to mean survival until mortality (wherein said mortality may be either irrespective of cause or prostate cell proliferative disorder related) but may be used in combination with other terms to define clinical terms, for example but not limited to "recurrence-free survival" (wherein the term recurrence shall include both localized and distant recurrence); metastasis free survival; disease free survival (wherein the term disease shall include prostate cancer and diseases associated therewith). The length of said survival may be calculated by reference to a defined start point (e.g. time of diagnosis or start of treatment) and a defined end point (e.g. death, recurrence or metastasis).

The term "Observed/Expected Ratio" ("O/E Ratio") refers to the frequency of CpG dinucleotides within a particular DNA sequence, and corresponds to the [number of CpG sites / (number of C bases x number of G bases)] .

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 kb, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG CpG dinucleotide sequences) within a DNA sequence include "unmethylated," "fully-methylated" and "hemi-methylated."

The term "hemi-methylation" or "hemimethylation" refers to the methylation state of a palindromic CpG methylation site, where only a single cytosine in one of the two CpG dinucleotide sequences of the palindromic CpG methylation site is methylated (*e.g.*, 5'-CC$^{M}$GG-3' (top strand): 3'-GGCC-5' (bottom strand)).

The term 'AUC' as used herein is an abbreviation for the area under a curve. In particular it refers to the area under a Receiver Operating Characteristic (ROC) curve. The ROC curve is a plot of the true positive rate against the false positive rate for the different possible cutpoints of a diagnostic test. It shows the tradeoff between sensitivity and specificity depending on the selected cutpoint (any increase in sensitivity will be accompanied by a decrease in specificity).The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "hypermethylation" refers to the average methylation state corresponding to an *increased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "hypomethylation" refers to the average methylation state corresponding to a *decreased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylations. Further epigenetic parameters include, for example, the acetylation of histones which, however, cannot be directly analyzed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific blocking probes (also referred to herein as blockers) covering CpG positions between, or covered by the amplification primers enable methylation-specific selective amplification of a nucleic acid sample.

The term "HeavyMethyl™ MethyLight™ assay, in the embodiment thereof implemented herein, refers to a HeavyMethyl™ MethyLight™ assay, which is a variation of the MethyLight™ assay, wherein the MethyLight™assay is combined with methylation specific blocking probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., Cancer Res. 59:2307-12, 1999, and in WO 00/26401A1.

The term "hybridization" is to be understood as a bond of an oligonucleotide to a complementary sequence along the lines of the Watson-Crick base pairings in the sample DNA, forming a duplex structure.

"Stringent hybridization conditions," as defined herein, involve hybridizing at 68°C in 5x SSC/5x Denhardt's solution/1.0% SDS, and washing in 0.2x SSC/0.1 % SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "array SEQ ID NO," "composite array SEQ ID NO," or "composite array sequence" refer to a sequence, hypothetical or otherwise, consisting of a head-to-tail (5' to 3') linear composite of all individual contiguous sequences of a subject array (e.g., a head-to-tail composite of SEQ ID NO:1-71, in that order).

The terms "array SEQ ID NO node," "composite array SEQ ID NO node," or "composite array sequence node" refer to a *junction* between any two individual contiguous sequences of the "array SEQ ID NO," the "composite array SEQ ID NO," or the "composite array sequence."

In reference to composite array sequences, the phrase "contiguous nucleotides" refers to a contiguous sequence region of any individual contiguous sequence of the composite array, but does not include a region of the composite array sequence that includes a "node," as defined herein above.

Overview:

The present invention provides for molecular genetic markers that have novel utility for providing a prognosis of prostate cell proliferative disorders. In particular embodiments said markers may be used for classifying the tumor according to aggressiveness and/or invasiveness. It is particularly preferred that the method and nucleic acids according to the invention are utilised for the prognosis of prostate cell proliferative disorders.

The term 'prognosis' is taken to mean a prediction of outcome of disease progression (wherein the term progression shall be taken to also include recurrence after treatment). Prognosis may be expressed in terms of overall patient survival, disease- or relapse-free survival, increased tumor-related complications and rate of progression of tumour or metastases, wherein a decrease in any of said factors (with the exception of increased tumor-related complications rate of progression) as relative to a pre-determined level, is a 'negative' outcome and increase thereof is a 'positive' outcome. A decrease in tumor-related complications and/or rate of progression of tumour or metastases as relative to a pre-determined level, is considered a 'positive' outcome and increase thereof is a 'negative' outcome.

Hereinafter prognosis may also be referred to in terms of 'aggressiveness' wherein an aggressive cancer is determined to have a high risk of negative outcome and wherein a non-aggressive cancer has a low risk of negative outcome.

In one aspect the prognostic marker according to the present invention is used to provide an estimate of the risk of negative outcome. Characterisation of a prostate cancer in terms of predicted outcome enables the physician to determine the risk of recurrence and/or death. This aids in treatment selection as the absolute reduction of risk of recurrence and death after treatments such as adjuvant hormonal, chemo-, and radiation therapy can be determined based on the predicted negative outcome. The absolute reduction in risk attributable to treatment may then be compared to the drawbacks of said treatment (e.g. side effects, cost) in order to determine the suitability of said treatment for the patient. Conversely, wherein a cancer is characterised as non-aggressive (i.e. positive outcome with low risk of death and/or recurrence) the patient will derive low absolute benefit from adjuvant or other treatment and may be appropriately treated by watchful waiting. Therein lies a great advantage of the present invention. By providing a means for determining which patients will not significantly benefit from treatment the present invention identifies suitable candidates for watchful waiting and prevents the over-prescription of therapies.

According to the predicted outcome (i.e. prognosis) of the disease an appropriate treatment or treatments may be selected. Wherein a cancer is characterised as aggressive it is particularly preferred that adjuvant treatment such as, but not limited to, hormonal, chemo- or radiation therapy is provided in addition to or instead of further treatments.

The herein described markers have further utility in predicting outcome of a patient after surgical treatment. This will hereinafter also be referred to as a 'predictive' marker. Over expression of the genes according to Table 11 (in particular FOXL2, SEQ ID NO: 35, SEQ ID NO: 63, HIST2H2BF, GPR7 and most preferably PITX2), are associated with negative outcome of prostate cancer patients. Patients with predicted positive outcome (i.e. hypomethylation or over-expression) after said treatment will accordingly have a decreased absolute reduction of risk of recurrence and death after treatment with post surgical adjuvant therapies. Patients with predicted negative outcome (i.e. hypermethylation) after said treatment will accordingly have a relatively larger absolute reduction of risk of recurrence and death after post surgical adjuvant treatment. Accordingly patients with a negative outcome after said treatment will be considered more suitable candidates for adjuvant treatment than patients with a positive outcome. Patients with a positive outcome may accordingly be prevented from over prescription of adjuvant treatment.

*Bisulfite modification of DNA is an art-recognized tool used to assess CpG methylation status.* 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, *e.g.*, PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil, which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analyzed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyze individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (*e.g.*, Zeschnigk M, et al., Eur J Hum Genet. 5:94-98, 1997), is currently only used in research. In all instances, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyze individual cytosine positions, or treated by enzymatic digestion (Xiong & Laird, Nucleic Acids Res., 25:2532-4, 1997). Detection by hybridization has also been described in the art (Olek et al., WO 99/28498). Additionally, use of the bisulfite technique for methylation detection with respect to individual genes has been described (Grigg & Clark, Bioessays, 16:431-6, 1994; Zeschnigk M, et al., Hum Mol Genet., 6:387-95, 1997: Feil R, et al., Nucleic Acids Res., 22:695-, 1994; Martin V, et al., Gene, 157:261-4, 1995; WO 9746705 and WO 9515373).

The present invention provides for the use of the bisulfite technique, in combination with one or more methylation assays, for determination of the methylation status of CpG dinudotide sequences within sequences from SEQ ID NO: 961 According to the present invention, determination of the methylation status of CpG dinudotide sequences within SEQ ID NO: 961has prognostic utility.

*Methylation Assay Procedures.* Various methylation assay procedures are known in the art, and can be used in conjunction with the present invention. These assays allow for determination of the methylation state of one or a plurality of CpG dinucleotides (*e.g.*, CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA is used, *e.g.*, the method described by Sadri & Homsby (Nucl. Acids Res. 24:5058-5059, 1996), or COBRA (Combined Bisulfite Restriction Analysis) (Xiong & Laird, Nucleic Acids Res. 25:2532-2534,1997).

COBRA. COBRA analysis is a quantitative methylation assay useful for determining DNA methylation levels at specific gene loci in small amounts of genomic DNA (Xiong & Laird, Nucleic Acids Res. 25:2532-2534. 1997). Briefly, restriction enzyme digestion is used to reveal methylation-dependent sequence differences in PCR products of sodium bisutfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples. Typical reagents (*e.g.*, as might be found in a typical COBRA-based kit) for COBRA analysis may include, but are not limited to: PCR primers for specific gene (or bisulfite treated DNA sequence or CpG island); restriction enzyme and appropriate buffer; gene-hybridization oligo; control hybridization oligo; kinase labeling kit for oligo probe: and labelled nucleotides. Additionally, bisulfite conversion reagents may include: DNA denaturation buffer, sulfonation buffer; DNA recovery reagents or kits (*e.g.*, precipitation, ultrafiltration, affinity column); desulfonation buffer, and DNA recovery components.

Preferably, assays such as "MethyLight™" (a fluorescence-based real-time PCR technique) (Eads et al., Cancer Res. 59:2302-2306, 1999), Ms-SNuPE (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), and methylated CpG island amplification ("MCA"; Toyota et al., Cancer Res. 59:2307-12, 1999) are used alone or in combination with other of these methods.

*MethyLight™.* The MethyLight™ assay is a high-throughput quantitative methylation assay that utilizes fluorescence-based real-time PCR (TaqMan™) technology that requires no further manipulations after the PCR step (Eads et al., Cancer Res. 59:2302-2306, 1999). Briefly, the MethyLight™ process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed either in an "unbiased" (with primers that do not overlap known CpG methylation sites) PCR reaction, or in a "biased" (with PCR primers that overlap known CpG dinucleotides) reaction. Sequence discrimination can occur either at the level of the amplification process or at the level of the fluorescence detection process, or both.

The MethyLight™ assay may be used as a quantitative test for methylation patterns in the genomic DNA sample, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the "MSP" technique), or with oligonucleotides covering potential methylation sites.

The MethyLight™ process can by used with a "TaqMan®" probe in the amplification process. For example, double-stranded genomic DNA is treated with sodium bisulfite and subjected to one of two sets of PCR reactions using TaqMan® probes; *e.g.*, with either biased primers and TaqMan® probe, or unbiased primers and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (*e.g.*, as might be found in a typical MethyLightTM-based kit) for MethyLightTM analysis may include, but are not limited to: PCR primers for specific gene (or bisulfite treated DNA sequence or CpG island); TaqMan® probes; optimized PCR buffers and deoxynucleotides; and Taq polymerase.

*Ms-SNuPE.* The Ms-SNuPE technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (*e.g.*, microdissected pathology sections), and it avoids utilization of restriction enzymes for determining the methylation status at CpG sites.

Typical reagents (*e.g.*, as might be found in a typical Ms-SNuPE-based kit) for Ms-SNuPE analysis may include, but are not limited to: PCR primers for specific gene (or bisulfite treated DNA sequence or CpG island); optimized PCR buffers and deoxynucleotides; gel extraction kit; positive control primers: Ms-SNuPE primers for specific gene; reaction buffer (for the Ms-SNuPE reaction); and labelled nucleotides. Additionally, bisulfite conversion reagents may include: DNA denaturation buffer; sulfonation buffer; DNA recovery regents or kit (*e.g.*, precipitation, ultrafiltrafion, affinity column); desulfonation buffer; and DNA recovery components.

MSP. MSP (methylation-specific PCR) allows for assessing the methylation status of virtually any group of CpG sites within a CpG island, independent of the use of methytation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. Typical reagents (*e.g.*, as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

*MCA*. The MCA technique is a method that can be used to screen for altered methylation patterns in genomic DNA, and to isolate specific sequences associated with these changes (Toyota et al., Cancer Res. 59:2307-12, 1999). Briefly, restriction enzymes with different sensitivities to cytosine methylation in their recognition sites are used to digest genomic DNAs from primary tumors, cell lines, and normal tissues prior to arbitrarily primed PCR amplification. Fragments that show differential methylation are cloned and sequenced after resolving the PCR products on high-resolution polyacrylamide gels. The cloned fragments are then used as probes for Southern analysis to confirm differential methylation of these regions. Typical reagents (*e.g.*, as might be found in a typical MCA- based kit) for MCA analysis may include, but are not limited to: PCR primers for arbitrary priming Genomic DNA; PCR buffers and nucleotides, restriction enzymes and appropriate buffers; gene-hybridization oligos or probes; control hybridization oligos or probes.

Genomic Sequences According to SEQ ID NO: 961 and Non-naturally Occuring Treated Variants Thereof According to SEQ ID Nos: 962 - 965 were Determined to have Utility for Providing a Prognosis of Prostate Cell Proliferative Disorders.

Particularly preferred is the use of microarrays. The microarray analysis process can be divided into two main parts. First is the immobilization of known gene sequences onto glass slides or other solid support followed by hybridization of the fluorescently labelled cDNA (comprising the sequences to be interrogated) to the known genes immobilized on the glass slide. After hybridization, arrays are scanned using a fluorescent microarray scanner. Analyzing the relative fluorescent intensity of different genes provides a measure of the differences in gene expression. DNA arrays can be generated by immobilizing presynthesized oligonucleotides onto prepared glass slides. In this case, representative gene sequences are manufactured and prepared using standard oligonucleotide synthesis and purification methods. These synthesized gene sequences are complementary to the genes of interest (most preferably PITX2) and tend to be shorter sequences in the range of 25-70 nucleotides. Alternatively, immobilized oligos can be chemically synthesized in-situ on the surface of the slide. In situ oligonucleotide synthesis involves the consecutive addition of the appropriate nucleotides to the spots on the microarray; spots not receiving a nucleotide are protected during each stage of the process using physical or virtual masks.

In expression profiling microarray experiments, the RNA templates used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labeling of the cDNA can be accomplished by either direct labeling or indirect labeling methods. During direct labeling, fluorescently modified nucleotides (e.g., Cy®3- or Cy®5-dCTP) are incorporated directly into the cDNA during the reverse transcription. Alternatively, indirect labeling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labelling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like. To perform differential gene expression analysis, cDNA generated from different RNA samples are labelled with Cy®3. The resulting labelled cDNA is purified to remove unincorporated nucleotides, free dye and residual RNA. Following purification, the labeled cDNA samples are hybridised to the microarray. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2nd ed., 1989). The microarray is scanned post-hybridization using a fluorescent microarray scanner. The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length probe. RNA probes are typically used instead of DNA probes due to the ease of generating single-stranded RNA probes and the reproducibility and reliability of RNA:RNA duplex digestion with RNases (Ausubel *et al*. 2003), particularly preferred are probes with high specific activities.

Particularly preferred is the use of microarrays. The microarray analysis process can be divided into two main parts. First is the immobilization of known gene sequences onto glass slides or other solid support followed by hybridization of the fluorescently labelled cDNA (comprising the sequences to be interrogated) to the known genes immobilized on the glass slide. After hybridization, arrays are scanned using a fluorescent microarray scanner. Analyzing the relative fluorescent intensity of different genes provides a measure of the differences in gene expression. DNA arrays can be generated by immobilizing presynthesized oligonucleotides onto prepared glass slides. In this case, representative gene sequences are manufactured and prepared using standard oligonucleotide synthesis and purification methods. These synthesized gene sequences are complementary to the genes of interest (such as PITX2 or other genes according to Table 11) and tend to be shorter sequences in the range of 25-70 nucleotides. Alternatively, immobilized oligos can be chemically synthesized in situ on the surface of the slide. In situ oligonucleotide synthesis involves the consecutive addition of the appropriate nucleotides to the spots on the microarray; spots not receiving a nucleotide are protected during each stage of the process using physical or virtual masks.

In expression profiling microarray experiments, the RNA templates used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labeling of the cDNA can be accomplished by either direct labeling or indirect labeling methods. During direct labeling, fluorescently modified nucleotides (e.g., Cy®3- or Cy®5-dCTP) are incorporated directly into the cDNA during the reverse transcription. Alternatively, indirect labeling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labelling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like. To perform differential gene expression analysis, cDNA generated from different RNA samples are labelled with Cy®3. The resulting labelled cDNA is purified to remove unincorporated nucleotides, free dye and residual RNA. Following purification, the labeled cDNA samples are hybridised to the microarray. The stringency of hybridisation is determined by a number of factors during hybridisation and during the washing procedure, including temperature, ionic strength, length of time and concentration of formamide.

The expression level of the gene and/or regulatory regions of PITX2 is determined by analysis of the level of methylation of said gene and/or regulatory regions thereof. It is preferred that the level of methylation of said genes, genomic sequences and/or regulatory regions thereof is determined by determing the methylation status or level of at least one CpG dinucleotide thereof. It is further preferred that the level of methylation of said gene and/or regulatory regions thereof is determined by determing the methylaiton status or level of a plurality of CpG dinucleotides thereof. Said gene and/or regulatory regions is selected from PITX2. Said analysis comprises the following steps:

i) contacting genomic DNA obtained from the subject with at least one reagent, or series of reagents that distinguishes between methylated and non-methylated CpG dinucleotides within at least one target region of the genomic DNA, wherein said contiguous nucleotides comprise at least one CpG dinucleotide sequence; and
ii) classifying the prostate cell proliferative disorders according to its prognosis as determined from the methylation status of said target regions analysed in i).

Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. Preferably, the source of the DNA sample is selected from the group consisting of cells or cell lines, histological slides, biopsies, paraffin-embedded tissue, bodily fluids, ejaculate, urine, blood, and combinations thereof. Preferably, the source is biopsies, bodily fluids, ejaculate, urine, or blood. The genomic DNA sample is then treated in such a manner that cytosine bases which are unmethylated at the 5'-position are converted to uracil, thymine, or another base which is dissimilar to cytosine in terms of hybridization behavior. This will be understood as 'treatment' herein.
The above described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.
The treated DNA is then analysed in order to determine the methylation state of one or more target gene sequences (prior to the treatment) associated with the development of prostate carcinoma. It is particularly preferred that the target region comprises, or hybridizes under stringent conditions to at least 16 contiguous nucleotides of gene or genomic sequence selected from the gene PITX2. It is further described that the sequences of said genes as described in the accompanying sequence listing are analysed. The method of analysis may be selected from those known in the art, including those listed herein. Particularly preferred are MethyLight™, MSP™ and the use of blocking oligonucleotides as will be described herein. It is further preferred that any oligonucleotides used in such analysis (including primers, blocking oligonucleotides and detection probes) should be reverse complementary, identical, or hybridize under stringent or highly stringent conditions to an at least 16-base-pair long segment of the base sequences of one or more of SEQ ID NO: 962 to SEQ ID NO: 965 and sequences complementary thereto.It is preferred that any oligonucleotides used in such analysis (including primers, blocking oligonucleotides and detection probes) should be reverse complementary, identical, or hybridize under stringent or highly stringent conditions to an at least 16-base-pair long segment of the base sequences of one or more of SEQ ID Nos: 962 - 965 and sequences complementary thereto
Aberrant methylation, of the gene or genomic sequence according to PITX2, is associated with the prognosis of prostate cell proliferative disorders. Analysis of the sequence enables the prognostic classification of prostate cell proliferative disorders. More preferably hypermethylation of one or more of said gene or genomic sequence is associated with poor prognosis. Hypermethylation is in general associated with under expression of mRNA and accordingly polpeptides.
In one embodiment the method discloses the use of the gene PITX2.
In the most preferred embodiment of the invention, the detection of prostate cell proliferative disorders is enabled by means of analysis of the methylation status of PITX2 and its promoter or regulatory elements. Methods for the methylation analysis of genes are described herein.
In one embodiment the method discloses the use of one or more genes or genomic sequences selected from the group consisting of the gene PITX2 as marker for providing a prognosis of prostate cell proliferative disorders.
In the most preferred embodiment of the invention, the detection of prostate cell proliferative disorders is enabled by means of analysis of the methylation status of said genes or genomic sequences and their promoter or regulatory elements. Methods for the methylation analysis of genes are described herein.
The present invention may also be described in certain embodiments as the use of a *kit* in providing a prognosis of a prostate cell proliferative disorder state through testing of a biological sample.
Particular embodiments of the present invention provide a novel application of the analysis of methylation levels and/or patterns within said sequences that enables a prognostic classification and thereby improved treatment of prostate cell proliferative disorders. Treatment of prostate cell proliferative disorders is directly linked with disease prognosis in particular aggressiveness, and the disclosed method thereby enables the physician and patient to make better and more informed treatment decisions.

FURTHER IMPROVEMENTS

The present invention describes novel uses for genomic sequences selected from the group consisting of SEQ ID NO: 961.

An objective of the invention comprises analysis of the methylation state of one or more CpG dinucleotides within at least one of the genomic sequences selected from the group consisting of SEQ ID SEQ ID NO: 961 and sequences complementary thereto.

The disclosed invention discloses treated nucleic acids, derived from SEQ ID NO: 961 wherein the treatment is suitable to convert at least one unmethylated cytosine base of the genomic DNA sequence to uracil or another base that is detectably dissimilar to cytosine in terms of hybridization. The genomic sequences in question may comprise one, or more, consecutive or random methylated CpG positions. Said treatment preferably comprises use of a reagent selected from the group consisting of bisulfite, hydrogen sulfite, disulfite, and combinations thereof.

The sequences of SEQ ID NO: 962 TO SEQ ID NO: 965 provide non-naturally occurring modified versions of the nucleic acid according to SEQ ID NO: 961, wherein the modification of each genomic sequence results in the synthesis of a nucleic acid having a sequence that is unique and distinct from said genomic sequence as follows. For each sense strand genomic DNA, *e.g.*, SEQ ID NO:1, four converted versions are disclosed. A first version wherein "C" is converted to "T," but "CpG" remains "CpG" (*i.e.*, corresponds to case where, for the genomic sequence, all "C" residues of CpG dinucleotide sequences are methylated and are thus not converted); a second version discloses the complement of the disclosed genomic DNA sequence (*i.e.* antisense strand), wherein "C" is converted to "T," but "CpG" remains "CpG" (*i.e.*, corresponds to case where, for all "C" residues of CpG dinucleotide sequences are methylated and are thus not converted). The 'upmethylated' converted sequences of SEQ ID NO:1 to SEQ ID NO:64 and SEQ ID NO: 961 correspond to SEQ ID NO:65 to SEQ ID NO:728. A third chemically converted version of each genomic sequences is provided, wherein "C" is converted to "T" for all "C" residues, including those of "CpG" dinucleotide sequences (*i.e.*, corresponds to case where, for the genomic sequences, all "C" residues of CpG dinucleotide sequences are unmethylated): a final chemically converted version of each sequence, discloses the complement of the disclosed genomic DNA sequence (*i.e.* antisense strand), wherein "C" is converted to "T" for all "C" residues, including those of "CpG" dinucleotide sequences (*i.e.*, corresponds to case where, for the complement (antisense strand) of each genomic sequence, all "C" residues of CpG dinucleotide sequences are unmethylated). The 'downmethylated' converted sequences of SEQ ID NO:1 to SEQ ID NO:64 and SEQ ID NO: 961 correspond to SEQ ID NO:65 to SEQ ID NO:320 and SEQ ID NO: 962 to SEQ ID NO: 965.

In an alternative preferred embodiment, such analysis comprises the use of an oligonucleotide or oligomer for detecting the cytosine methylation state within genomic or treated (chemically modified) DNA, according to SEQ ID NO: 962 to SEQ ID NO: 965. Said oligonucleotide or oligomer comprising a nucleic acid sequence having a length of at least nine (9) nucleotides which hybridizes, under moderately stringent or stringent conditions (as defined herein above), to a treated nucleic acid sequence according to SEQ ID NO: 962 to SEQ ID NO: 965 and/or sequences complementary thereto,

Thus, the present invention describes nucleic acid molecules (*e.g.*, oligonucleotides and peptide nucleic acid (PNA) molecules (PNA-oligomers)) that hybridize under moderately stringent and/or stringent hybridization conditions to all or a portion of the sequences SEQ ID NO: 1 to SEQ ID NO: 320 and SEQ ID NO: 961 to 965 , or to the complements thereof. Particularly described is a nucleic acid molecule that hybridizes under moderately stringent and/or stringent hybridization conditions to all or a portion of the sequences SEQ ID NO: 65 to SEQ ID NO: 320 and SEQ ID NO: 962 to SEQ ID NO: 965 but is not identical to or complementary to SEQ ID NO: 1 to SEQ ID NO: 64 and SEQ ID NO: 961 or other human genomic DNA. Further described is a nucleic acid molecule that hybridizes under moderately stringent and/or stringent hybridization conditions to all or a portion of the sequences SEQ ID Nos: 133,134,261,262, 189,190,317,318, 101,102,229,230, 962 - 965 but is not identical to or complementary to SEQ ID Nos: 961, 35, 63 and 19 or other human genomic DNA.

The hybridizing portion of the hybridizing nucleic acids is typically at least 9, 15, 20, 25, 30 or 35 nucleotides in length. However, longer molecules have inventive utility, and are thus within the scope of the present invention.

Preferably, the hybridizing portion of the hybridizing nucleic acids is at least 95%, or at least 98%, or 100% identical to the sequence, or to a portion thereof of SEQ ID NO: 962 to 965, or to the complements thereof.

Hybridizing nucleic acids of the type described herein can be used, for example, as a primer (*e.g.*, a PCR primer), or a diagnostic and/or prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence SEQ ID NO: 961 (such as allelic variants and SNPs), rather than identical, it is useful to first establish the lowest temperature at which only homologous hybridization occurs with a particular concentration of salt (*e.g.*, SSC or SSPE). Then, assuming that 1% mismatching results in a 1˚C decrease in the Tm, the temperature of the final wash in the hybridization reaction is reduced accordingly (for example, if sequences having > 95% identity with the probe are sought, the final wash temperature is decreased by 5˚C). In practice, the change in Tm can be between 0.5˚C and 1.5˚C per 1% mismatch.

Examples of oligonucleotides of length X (in nucleotides), as indicated by polynucleotide positions with reference to, *e.g.*, SEQ ID NO:1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:

> n to (n + (x-1)):
> where n=1, 2, 3,...(Y-(X-1));
> where Y equals the length (nucleotides or base pairs) of SEQ ID NO: 1 (7572);
> where X equals the common length (in nucleotides) of each oligonucleotide in the set (*e.g.*, X=20 for a set of consecutively overlapping 20-mers); and
> where the number (Z) of consecutively overlapping oligomers of length X for a given SEQ ID NO of length Y is equal to Y-(X-1). For example Z= 7572-19= 7553 for either sense or antisense sets of SEQ ID NO:1, where X=20.

Preferably, the set is limited to those oligomers that comprise at least one CpG, TpG or CpA dinucleotide.

Examples of 20-mer oligonucleotides include the following set of oligomers (and the antisense set complementary thereto), indicated by polynucleotide positions with reference to SEQ ID NO:1:
1-20, 2-21, 3-22, 4-23, 5-24, ......7553-7572.

Preferably, the set is limited to those oligomers that comprise at least one CpG, TpG or CpA dinucleotide.

Likewise, examples of inventive 25-mer oligonucleotides include the following set of 2,256 oligomers (and the antisense set complementary thereto), indicated by polynucleotide positions with reference to SEQ ID NO: 1:
1-25, 2-26, 3-27, 4-28, 5-29, ...... 7553-7572.

Preferably, the set is limited to those oligomers that comprise at least one CpG, TpG or CpA dinucleotide.

The present invention describes, for each of SEQ ID NO:1 to SEQ ID NO:320 and SEQ ID NO: 961 to SEQ ID NO: 965 (sense and antisense), multiple consecutively overlapping sets of oligonucleotides or modified oligonucleotides of length X, where, *e.g.*, X= 9, 10, 17, 20, 22, 23, 25, 27, 30 or 35 nucleotides.

The oligonucleotides or oligomers constitute effective tools useful to ascertain genetic and epigenetic parameters of the genomic sequence corresponding to SEQ ID NO: 961. Preferred sets of such oligonucleotides or modified oligonucleotides of length X are those consecutively overlapping sets of oligomers corresponding to SEQ ID NO. 1 to SEQ ID NO:320 and SEQ ID NO: 961 to SEQ ID NO: 965 (and to the complements thereof). Preferably, said oligomers comprise at least one CpG, TpG or CpA dinucleotide.

Particularly preferred oligonucleotides or oligomers are those
in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG. TpG or CpA dinucleotide is positioned within the fifth to ninth nucleotide from the 5'-end.

The oligonucleotides can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG). palmityl moieties, and others as disclosed in, for example. United States Patent Numbers 5,514,758. 5,565,552, 5,567,810, 5,574,142, 5.585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, e.g., a chromophore, fluorophor, peptide, hybridization-triggered cross-linking agent, transport agent. hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers for use in the present invention are typically used in 'sets,' which contain at least one oligomer for analysis of at least one of the CpG dinucleotides of genomic sequences SEQ ID NO: 961 and sequences complementary thereto, or to the corresponding CpG, TpG or CpA dinucleotide within a sequence of the treated nucleic acids according to SEQ ID NO: 962 to SEQ ID NO: 965 and sequences complementary thereto. However, it is anticipated that for economic or other factors it may be preferable to analyze a limited selection of the CpG dinucleotides within said sequences, and the content of the set of oligonucleotides is altered accordingly.

Therefore, in particular embodiments, the present invention describes a set of at least two (2) (oligonucleotides and/or PNA-oligomers) useful for detecting the cytosine methylation state in treated genomic (DNA SEQ ID NO: 962 to SEQ ID NO: 965), or in genomic (DNA SEQ ID NO: 961 and sequences complementary thereto). These probes enable diagnosis and/or classification of genetic and epigenetic parameters of prostate cell proliferative disorders.

In preferred embodiments, at least one, and more preferably all members of a set of oligonucleotides is bound to a solid phase.

The present invention describes a set of at least two (2) oligonucleotides that are used as primer oligonucleotides for amplifying DNA sequences of one of SEQ ID NO:1 to SEQ ID NO:320 and SEQ 10 NO: 961 to SEQ ID NO: 965 and sequences complementary thereto, or segments thereof.

It is anticipated that the oligonucleotides may constitute all or part of an "array" or "DNA chip" (i.e.. an arrangement of different oligonucleotides and/or PNA-oligomers bound to a solid phase). Such an array of different oligonucleotide- and/or PNA-oligomer sequences can be characterized, for example. in that it is arranged on the solid phase in the form of a rectangular or hexagonal lattice. The solid-phase surface may be composed of silicon, glass, polystyrene, aluminum, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics *(*Nature Genetics Supplement, Volume 21, January 1999, and from the literature cited therein). Fluorescently labeled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example, via a confocal microscope. Cy3 and Cy5 dyes, besides many others, are commercially available.

It is also anticipated that the oligonucleotides, or particular sequences thereof, may constitute all or part of an 'virtual arrays' wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (i.e., each analyte) can be uniquely bound by a unique label and thus detected (each label is directly counted, resulting in a digital read-out of each molecular species in the mixture).

It is particularly preferred that the oligomers are utilised for the prognosis prostate cell proliferative disorders. This is enabled by use of said sets for providing a prognosis of a biological sample isolated from a patient. Particularly preferred are those sets of oligomers that comprise at least two oligonucleotides selected from one of the following sets of oligonucleotides.

In one embodiment of the method, this is achieved by analysis of the methylation status of at least one target sequence comprising, or hybridizing under stringent conditions to at least 16 contiguous nucleotides of a gene or sequence selected from the group consisting of the gene or genomic sequence of PITX2.

In the first step*,* a sample of the tissue to be analysed is obtained. The source may be any suitable source. Preferably, the source of the DNA sample is selected from the group consisting of cells or cell lines, histological slides, biopsies, paraffin-embedded tissue, body fluids, ejaculate, urine, blood, and combinations thereof. Preferably, the source is biopsies, bodily fluids, ejaculate, urine, or blood.

Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Once the nucleic acids have been extracted, the genomic double stranded DNA is used in the analysis.

The genomic DNA sample is treated in such a manner that cytosine bases which are unmethylated at the 5'-position are converted to uracil, thymine, or another base which is dissimilar to cytosine in terms of hybridization behavior. This will be understood as 'pretreatment' or 'treatment' herein.

The above-described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

Fragments of the treated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). The set of primer oligonucleotides includes at least two oligonucleotides whose sequences are each reverse complementary, identical, or hybridize under stringent or highly stringent conditions to an at least 16-base-pair long segment of the base sequences of one of SEQ ID NO: 962 to SEQ ID NO: 965 and sequences complementary thereto.

In an alternate embodiment of the method, the methylation status of preselected CpG positions within the nucleic acid sequences comprising SEQ ID NO: 961 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridizes to a bisulfite treated CpG dinucleotide. Therefore, the sequence of said primers comprises at least one CpG dinucleotide. MSP primers specific for non-methylated DNA contain a T at the position of the C positions in the CpG. Preferably, therefore, the base sequence of said primers is required to comprise a sequence having a length of at least 9 nucleotides which hybridizes to a treated nucleic acid sequence SEQ ID Nos: 962 - 965 and sequences complementary thereto, wherein the base sequence of said oligomers comprises at least one CpG dinucleotide.

A further preferred embodiment of the method comprises the use of blocker oligonucleotides. The use of such blocker oligonucleotides has been described by Yu et al., BioTechniques 23:714-720. 1997. Blocking probe oligonucleotides are hybridized to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free' hydroxyl group. For example, 3'-O-acetyl oligonucleotides are representative of a preferred class of blocker molecules.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (e.g., with excess blocker), degradation of the Mocker oligonucleotide will be substantially precluded. This is because the polymerase will not extend the primer toward, and through (in the 5'-3' direction) the blocker a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridizes to a treated nucleic acid sequence according to one of SEQ ID NO: 962 to SEQ ID NO: 965 and sequences complementary thereto, wherein the base sequence of said oligonucleotides comprises at least one CpG, TpG or CpA dinucleotide. More preferably the base sequence of said *blocking oligonucleotides* is required to comprise a sequence having a length of at least 9 nucleotides which hybridizes to a treated nucleic acid sequence according to one of SEQ ID Nos: 962 - 965 and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labeled amplficates have a single positive or negative net charge. allowing for better detectability in the mass spectrometer. The detection may be carried out and visualized by means of, e.g., matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapour phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends. 1:147-57, 1995). The sensitivity with respect to nucleic acid analysis is approximately 100-times less than for peptides, and decreases disproportionately with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

The amplificates obtained during the method are analysed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analyzed by means of hybridization-based methods such as, but not limited to, array technology and probe based technologies as well as by means of techniques such as sequencing and template directed extension.

In one embodiment of the method, the amplificates synthesised are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG . TpG or CpA dinucleotide.

The dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within the sequence according to SEQ ID NO: 961, and the equivalent positions within SEQ ID NO: 962 to SEQ ID NO: 965. Said oligonucleotides may also be present in the form of peptide nucleic acids. The non-hybridized amplificates are then removed.The hybridized amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes that are hybridised to the bisulfite treated DNA, concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; *also see* United States Patent No. 6,331,393) employing a dual-labeled fluorescent oligonucleotide probe (TaqMan™ PCR, using an ABI Prism 7700 Sequence Detection System, Perkin Elmer Applied Biosystems, Foster City, California). The Taqman™ PCR reaction employs the use of a nonextendible interrogating oligonucleotide, called a TaqMan™ probe, which, in preferred embodiments, is designed to hybridize to a GpC-rich sequence located between the forward and reverse amplification primers. The TaqMan™ probe further comprises a Fluorescent "reporter moiety" and a quencher moiety" covalently bound to linker moieties (*e.g.*, phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, also known as the MethylLight™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

A further suitable method for the use of probe oligonucleotides for the assessment of methylation by analysis of bisulfite treated nucleic acids In a further preferred embodiment of the method, the method comprises the use of template-directed oligonucleotide extension, such as MS-SNuPE as described by Gonzalgo & Jones, Nucleic Acids Res. 25:2529-2531, 1997.

In yet a further embodiment of the method, the method comprises sequencing and subsequent sequence analysis of the amplificate generated in the *third step* of the method (Sanger F., et al., Proc Natl Acad Sci USA 74: 5463-5467, 1977).

Best mode

In the most preferred embodiment of the method the genomic nucleic acids are isolated and treated according to the first three steps of the method outlined above, namely:

a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties: and wherein
d) amplifying subsequent to treatment in c) is carried out in a methylation specific manner namely by use of methylation specific primers or *blocking oligonucleotides,* and further wherein
e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID NO: 962 to SEQ ID NO: 965 and sequences complementary thereto, wherein the base sequence of said oligomers comprises at least one CpG dinucleotide. More preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID Nos: 962 - 965 and sequences complementary thereto, wherein the base sequence of said oligomers comprises at least one CpG dinucleotide.
In an alternative most preferred embodiment of the method, the subsequent amplification of d) is carried out in the presence of blocking *oligonucleotides,* as described above. Said blocking oligonucleotides comprising a sequence having a Length of at least 9 nucleotides which hybridizes to a treated nucleic acid sequence according to one of SEQ ID NO: 961 to 965 and sequences complementary thereto, wherein the base sequence of said oligomers comprises at least one CpG. TpG or CpA dinucleotide. Preferably said *blocking* oligonucleotides comprising a sequence having a length of at least 9 nucleotides which hybridizes to a treated nucleic acid sequence according to one of SEQ ID Nos: 962 - 965 and sequences complementary thereto, wherein the base sequence of said oligomers comprises at least one CpG, TpG or CpA dinucleotide. Step e) of the method, namely the detection of the specific amplificates indicative of the methylation status of one or more CpG positions according to SEQ ID NO: 961 is carried out by means of real-time detection methods as described above.
Subsequent to the determination of the methylation state of the genomic nucleic acids the prognosis of the prostate cancer is deduced based upon the methylation state of at least one CpG dinucleotide sequence of SEQ ID NO: 961, or an average, or a value rejecting an average methylation state of a plurality of CpG dinucleotide sequences of SEQ ID NO: 961. Preferably said prognosis is based upon the methylation state of at least one CpG dinucleotide sequence of the genes PITX2, or an average, or a value reflecting an average methylation state of a plurality of CpG dinucleotide sequences of SEQ ID NO: 961. Hypomethylation of said CpG positions are associated with good prognosis, and hypermethylation is associated with poor prognosis. Hypermethylation is in general associated with under expression of mRNA and accordingly polpeptides. The cut-off point for determining hypo and hyper methylation is may be the median methylation level for a given population, or is preferably an optimized cut-off level. For the analysis of PITX2 it is preferred that the cut-off is between 20% and 10% methylation, and most preferably 14.27%.
Wherein the methods according to the present invention of expression analysis (most preferably by means of methylation analysis), of the herein described markers (preferably PITX2) are used to determine the prognosis of a prostate cancer said methods are preferably used in combination with other clinical prognostic variables used to determine prognosis most preferably Gleason score but also including nomogram score and PSA level (i.e. that said variables are factored in or taken into account). In a preferred embodiment of the invention prognostic expression analysis of each of the markers PITX2 as herein described is carried out on patients who present with organ confined (T2) prostate cancer. PITX2 has a distinct advantage in determinig the prognosis of T2 prostate cancer, whereas it is current clinical practise that all patients presenting with T2 prostate cancer are deemed to have a good prognosis. According to the present invention said T2 patients with poor prognosis (hypermethylation) would have a prognosis more typical of patients presenting with T3 (non organ-confined) prostate cancer and may be treated appropriately. Furthermore prognostic expression analysis of each of the marker PITX2 has further utility in the analysis of patients presenting high Gleason score (8 or higher). Said patients are currently considered to have a poor prognosis, however by means of the herein described method of PITX2 expression analysis it is for the first time possible to differentiate high Gleason score patients with a poor prognosis (hypermethylation) from those with a good prognosis. Currently all patients with high Gleason score are considered candidates for post-surgical adjuvant treatment, accordingly the method according to the invention enables the prevention of over-treatment of said patients.

Kits

Moreover, an additional aspect of the present invention is the use of a kit comprising, for example: a bisulfite-containing reagent; a set of primer oligonucleotides containing at least two oligonucleotides whose sequences in each case correspond, are complementary, or hybridize under stringent or highly stringent conditions to a 16-base long segment of the sequences SEQ ID NO: 961 to 965; oligonucleotides and/or PNA-oligomers; as well as instructions for carrying out and evaluating the described method. In a further preferred embodiment, said kit may further comprise standard reagents for performing a CpG position-specific methylation analysis, wherein said analysis comprises one or more of the following techniques: MS-SNuPE, MSP, MethyLight™, HeavyMethyl™. COBRA, and nucleic acid sequencing. However, a kit along the lines of the present invention can also contain only part of the aforementioned components. Preferably said kit comprises a bisulfite-containing reagent; a set of primer oligonucleotides containing at least two oligonucleotides whose sequences in each case correspond, are complementary, or hybridize under stringent or highly stringent conditions to a 16-base long segment of the sequences SEQ ID Nos: 962 - 965; oligonucleotides and/or PNA-oligomers: as well as instructions for carrying out and evaluating the described method. In a further preferred embodiment, said kit may further comprise standard reagents for performing a CpG position-specific methylation analysis, wherein said analysis comprises one or more of the following techniques: MS-SNuPE, MSP, MethyLight ™, HeavyMethyl™, COBRA, and nucleic acid sequencing. However, a kit for use along the lines of the present invention can also contain only part of the aforementioned components.

The described invention further provides the use of a composition of matter useful for providing a prognosis of prostate cancer patients. Said composition comprising at least one nucleic acid 18 base pairs in length of a segment of a nucleic acid sequence selected from the group consisting SEQ ID Nos: 962 - 965, and one or more substances taken from the group comprising: magnesium chloride, dNTP, taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID Nos: 962 - 965 and sequences complementary thereto. It is preferred that said composition of matter comprises a buffer solution appropriate for the stabilization of said nucleic acid in an aqueous solution and enabling polymerase based reactions within said solution. Suitable buffers are known in the art and commercially available.

While the present invention has been described with specificity in accordance with certain of its preferred embodiments, the following EXAMPLES and TABLES serve only to illustrate the invention and are not intended to limit the invention within the principles and scope of the broadest interpretations and equivalent configurations thereof.

TABLES 1-12

**TABLE 1: Experimental set-up of MeST screening with number of samples in pools.**

| Experiment | Number of comparisons | Aggressive group | Nonaggressive group |
|---|---|---|---|
| PSA recurrence vs no recurrence | 3 | 5 tumors from patients who had PSA recurrences <2yr after surgery | 5 tumors from patients without PSA recurrence after at least 4 years follow up |
| Late stage vs Early stage | 1 | 5 stage III and IV tumors | 5 stage I and II tumors |
| High Gleason vs. Low Gleason | 1 | 5 grade 4 or 5 tumors | 5 grade 1, 2, or 3 tumors |

| | | | |
|---|---|---|---|
| NAT (PSA recurrence) vs. NAT (no recurrence) | 1 | 4 normal samples from tissue adjacent to tumors from patients who had PSA recurrences <2yr after surgery | 4 normal tissue samples adjacent to tumors from patients without PSA recurrence after at least 4 years follow up |
| Peripheral Zone vs. Transition Zone | 1 | 5 peripheral zone tumors | 5 transition zone tumors |

TABLE 2. Summarized results of MeST Screening experiments

| Experiment | |
|---|---|
| Total number of MeSTs<br>scoring > 0 | 441<br>278 |
| MeSTs from MCA<br>scoring > 0 | 242<br>126 |
| MeSTs from AP-PCR<br>scoring > 0 | 199<br>152 |

TABLE 3: Total number of MeSTs and number of positive MeSTs from each screening comparison.

| Experiment | Number of MeSTs | Number of Positive MeSTs | Percent Positive MeSTs |
|---|---|---|---|
| PSA recurrence vs. no recurrence I | 41 | 28 | 68% |
| PSA recurrence vs. no recurrence II | 113 | 74 | 65% |
| PSA recurrence vs. no recurrence II | 69 | 43 | 62% |
| Late stage vs Early stage | 64 | 39 | 61% |
| High Gleason vs. Low Gleason | 63 | 48 | 76% |
| NAT (PSA recurrence) vs. NAT (no recurrence) | 103 | 66 | 64% |
| Peripheral Zone vs. Transition Zone | 76 | 48 | 63% |

TABLE 4 Summary of Real-time PCR data for seven candidates. P values are from a Wilcoxon test and are uncorrected for multiple comparisons. The sensitivity is calculated when the specificity is set at 0.85 or greater.

| Candidate | Gene Name | AUC | Sensivity | Specificity | P value (Wilcoxon) |
|---|---|---|---|---|---|
| SEQ ID NO : 19 | GPR7 | 0.76 | 0.50 | 0.87 | 0.0008 |
| SEQ ID NO : 35 | Genomic region downstream of FOXL2 | 0.75 | 0.54 | 0.87 | 0.0016 |
| SEQ ID NO : 37 | ABHD9 | 0.7 | 0.38 | 0.86 | 0.0115 |
| SEQ ID NO : 7 | GSTP1 | 0.69 | 0.38 | 0.87 | 0.0176 |
| SEQ ID NO : 63 | HIST2H2BF regulatory region | 0.68 | 0.35 | 0.87 | 0.0241 |
| SEQ ID NO : 8 | RARB | 0.68 | 0.50 | 0.85 | 0.0214 |
| SEQ ID NO : 64 | PMF1 | 0.47 | 0.15 | 0.87 | 0.7138 |

TABLE 5 A summary of the samples used in the chip study. For the outcome categories, "No recurrence" means the patient did not have a relapse and at least 48 months follow up information was available. "Early recurrence" indicates that the patient experienced PSA relapse in less than 2 years after surgery. "Other" includes patients with no follow up information and patients who did not fit the criteria for the recurrence categories.

| Gleason Categories | Sample Number | Outcome categories |
|---|---|---|
| A) Low Gleason ( 1+2, 2+1, 2+2, 2+3, 3+2, and 3+3) | 135 | 1. No recurrence (n=42) |
| | | 2. Early recurrence (n=6) |
| | | 3. Other (n=87) |
| B) Intermediate Gleason (2+4, 4+2, 3+4, 4+3, 2+5, 5+2) | 73 | 1.No recurrence (n=34) |
| | | 2. Early recurrence (n=33) |
| | | 3. Other (n=6) |
| C) High Gleason (3+5, 5+3, 4+5, 5+4, and 5+5) | 99 | 1. No recurrence |
| | | 2. Early recurrence |
| | | 3. Other |
| D) No Gleason information | 4 | 1. No recurrence (n=3) |
| | | 2. Early recurrence (n=0) |
| | | 3. Other (n=1) |

TABLE 6 Corrected Wilcoxon p-value / AUC / Sensitivity / Specificity of the amplificates. Sensitivity is reported at a fixed specificity of -0.75.

| **Marker** | **p-value** | **AUC** | **Sensitivity** | **Specificity** |
|---|---|---|---|---|
| SEQ NO: 19 | 2.05E-06 | 0.72 | 0.58 | 0.75 |
| SEQ ID NO: 41 | 2.17E-06 | 0.71 | 0.51 | 0.75 |
| SEQ ID NO: 37 | 4.15E-06 | 0.71 | 0.55 | 0.75 |
| SEQ ID NO: 51 | 8.68E-06 | 0.71 | 0.51 | 0.75 |
| SEQ ID NO: 35 | 2.60E-05 | 0.70 | 0.48 | 0.75 |
| SEQ ID NO: 4 | 5.52E-05 | 0.69 | 0.51 | 0.75 |
| SEQ ID NO: 17 | 5.89E-05 | 0.69 | 0.51 | 0.75 |
| SEQ ID NO: 16 | 6.82E-05 | 0.69 | 0.47 | 0.75 |
| SEQ ID NO: 9 | 9.30E-05 | 0.69 | 0.57 | 0.75 |
| SEQ ID NO: 47 | 1.80E-04 | 0.68 | 0.46 | 0.75 |
| SEQ ID NO: 49 | 2.05E-04 | 0.68 | 0.49 | 0.75 |
| SEQ ID NO: 42 | 3.84E-04 | 0.68 | 0.45 | 0.75 |
| SEQ ID NO: 57 | 4.34E-04 | 0.68 | 0.49 | 0.75 |
| SEQ ID NO: 1 | 7.44E-04 | 0.67 | 0.51 | 0.75 |
| SEQ ID NO: 7 | 8.06E-04 | 0.67 | 0.43 | 0.75 |
| SEQ ID NO: 62 | 8.06E-04 | 0.67 | 0.49 | 0.75 |
| SEQ ID NO: 8 | 9.30E-04 | 0.67 | 0.47 | 0.75 |
| SEQ ID NO: 58 | 1.24E-03 | 0.67 | 0.46 | 0.75 |
| SEQ ID NO: 3 | 2.79E-03 | 0.66 | 0.48 | 0.75 |

(continued)

| Marker | p-value | AUC | Sensitivity | Specificity |
|---|---|---|---|---|
| SEQ ID NO: 13 | 3.84E-03 | 0.66 | 0.45 | 0.75 |
| SEQ ID NO: 23 | 8.68E-03 | 0.65 | 0.43 | 0.75 |
| SEQ ID NO: 29 | 9.30E-03 | 0.65 | 0.41 | 0.75 |
| SEQ ID NO: 39 | 9.30E-03 | 0.65 | 0.41 | 0.75 |
| SEQ ID NO: 5 | 1.05E-02 | 0.65 | 0.47 | 0.75 |
| SEQ ID NO: 56 | 0.03 | 0.64 | 0.46 | 0.75 |
| SEQ ID NO: 10 | 0.10 | 0.62 | 0.41 | 0.75 |
| SEQ ID NO: 2 | 0.11 | 0.62 | 0.40 | 0.75 |
| SEQ ID NO: 6 | 0.13 | 0.62 | 0.40 | 0.75 |
| SEQ ID NO: 50 | 0.25 | 0.61 | 0.42 | 0.75 |
| SEQ ID NO: 33 | 0.36 | 0.61 | 0.47 | 0.75 |
| SEQ ID NO: 15 | 0.37 | 0.61 | 0.35 | 0.75 |
| SEQ ID NO: 60 | 0.39 | 0.61 | 0.43 | 0.75 |
| SEQ ID NO: 55 | 0.46 | 0.60 | 0.31 | 0.75 |
| SEQ ID NO: 52 | 0.47 | 0.60 | 0.38 | 0.75 |
| SEQ ID NO:20 | 0.87 | 0.60 | 0.33 | 0.75 |
| SEQ ID NO: 21 | 0.87 | 0.60 | 0.42 | 0.75 |
| SEQ ID NO: 24 | 1.00 | 0.59 | 0.35 | 0.75 |
| SEQ ID NO: 54 | 1.00 | 0.59 | 0.36 | 0.75 |
| SEQ ID NO: 30 | 1.00 | 0.59 | 0.36 | 0.75 |
| SEQ ID NO: 43 | 1.00 | 0.42 | 0.22 | 0.75 |
| SEQ ID NO: 48 | 1.00 | 0.58 | 0.35 | 0.75 |
| SEQ ID NO: 45 | 1.00 | 0.58 | 0.32 | 0.75 |
| SEQ ID NO: 26 | 1.00 | 0.58 | 0.38 | 0.75 |
| SEQ ID NO: 31 | 1.00 | 0.57 | 0.42 | 0.75 |
| SEQ ID NO: 38 | 1.00 | 0.56 | 0.34 | 0.75 |
| SEQ ID NO: 28 | 1.00 | 0.56 | 0.36 | 0.75 |
| SEQ ID NO: 32 | 1.00 | 0.56 | 0.33 | 0.75 |
| SEQ ID NO: 40 | 1.00 | 0.56 | 0.30 | 0.75 |
| SEQ ID NO: 27 | 1.00 | 0.55 | 0.34 | 0.75 |
| SEQ ID NO: 61 | 1.00 | 0.45 | 0.21 | 0.75 |
| SEQ ID NO: 11 | 1.00 | 0.54 | 0.34 | 0.75 |
| SEQ ID NO: 44 | 1.00 | 0.54 | 0.28 | 0.75 |
| SEQ ID NO: 53 | 1.00 | 0.54 | 0.21 | 0.75 |
| SEQ ID NO: 22 | 1.00 | 0.54 | 0.28 | 0.75 |
| SEQ ID NO: 18 | 1.00 | 0.47 | 0.20 | 0.75 |
| SEQ ID NO: 59 | 1.00 | 0.47 | 0.25 | 0.75 |
| SEQ ID NO: 36 | 1.00 | 0.47 | 0.22 | 0.75 |

(continued)

| Marker | p-value | AUC | Sensitivity | Specificity |
|---|---|---|---|---|
| SEQ ID NO: 12 | 1.00 | 0.53 | 0.24 | 0.75 |
| SEQ ID NO: 34 | 1.00 | 0.52 | 0.27 | 0.75 |
| SEQ ID NO: 46 | 1.00 | 0.48 | 0.19 | 0.75 |
| SEQ ID NO: 25 | 1.00 | 0.51 | 0.25 | 0.75 |
| SEQ ID NO: 14 | 1.00 | 0.51 | 0.24 | 0.75 |

TABLE 7: Corrected Wilcoxon p-value / AUC / Sensitivity / Specificity of the amplificates. Sensitivity is reported at a fixed specificity of -0.75.

| Marker | p-value | AUC | Sensitivity | Specificity |
|---|---|---|---|---|
| SEQ ID NO: 19 | 2.42E-04 | 0.72 | 0.59 | 0.76 |
| SEQ ID NO: 35 | 2.48E-04 | 0.72 | 0.59 | 0.76 |
| SEQ ID NO: 37 | 0.05 | 0.66 | 0.37 | 0.76 |
| SEQ ID NO: 20 | 0.07 | 0.66 | 0.44 | 0.76 |
| SEQ ID NO: 13 | 0.08 | 0.65 | 0.46 | 0.76 |
| SEQ ID NO: 9 | 0.11 | 0.65 | 0.37 | 0.76 |
| SEQ ID NO: 41 | 0.11 | 0.65 | 0.37 | 0.76 |
| SEQ ID NO: 57 | 0.11 | 0.65 | 0.41 | 0.76 |
| SEQ ID NO: 51 | 0.12 | 0.65 | 0.49 | 0.76 |
| SEQ ID NO: 4 | 0.22 | 0.64 | 0.40 | 0.76 |
| SEQ ID NO: 1 | 0.24 | 0.64 | 0.41 | 0.76 |
| SEQ ID NO: 10 | 0.57 | 0.62 | 0.38 | 0.76 |
| SEQ ID NO: 15 | 0.61 | 0.62 | 0.38 | 0.76 |
| SEQ ID NO: 17 | 0.74 | 0.62 | 0.32 | 0.76 |
| SEQ ID NO: 58 | 0.93 | 0.62 | 0.40 | 0.76 |
| SEQ ID NO: 62 | 1.00 | 0.61 | 0.49 | 0.76 |
| SEQ ID NO: 43 | 1.00 | 0.39 | 0.11 | 0.76 |
| SEQ ID NO: 29 | 1.00 | 0.61 | 0.37 | 0.76 |
| SEQ ID NO: 16 | 1.00 | 0.60 | 0.35 | 0.76 |
| SEQ ID NO: 23 | 1.00 | 0.60 | 0.40 | 0.76 |
| SEQ ID NO: 5 | 1.00 | 0.60 | 0.44 | 0.76 |
| SEQ ID NO: 42 | 1.00 | 0.60 | 0.32 | 0.76 |
| SEQ ID NO: 49 | 1.00 | 0.59 | 0.37 | 0.76 |
| SEQ ID NO: 3 | 1.00 | 0.59 | 0.40 | 0.76 |
| SEQ ID NO: 47 | 1.00 | 0.59 | 0.35 | 0.76 |
| SEQ ID NO: 7 | 1.00 | 0.59 | 0.27 | 0.76 |
| SEQ ID NO: 28 | 1.00 | 0.59 | 0.37 | 0.76 |
| SEQ ID NO: 2 | 1.00 | 0.59 | 0.29 | 0.76 |
| SEQ ID NO: 18 | 1.00 | 0.42 | 0.14 | 0.76 |

(continued)

| Marker | p-value | AUC | Sensitivity | Specificity |
|---|---|---|---|---|
| SEQ ID NO: 45 | 1.00 | 0.58 | 0.38 | 0.76 |
| SEQ ID NO: 8 | 1.00 | 0.58 | 0.25 | 0.76 |
| SEQ ID NO: 46 | 1.00 | 0.42 | 0.13 | 0.76 |
| SEQ ID NO: 30 | 1.00 | 0.58 | 0.32 | 0.76 |
| SEQ ID NO: 39 | 1.00 | 0.58 | 0.35 | 0.76 |
| SEQ ID NO: 54 | 1.00 | 0.57 | 0.32 | 0.76 |
| SEQ ID NO: 25 | 1.00 | 0.43 | 0.17 | 0.76 |
| SEQ ID NO: 6 | 1.00 | 0.57 | 0.30 | 0.76 |
| SEQ ID NO: 11 | 1.00 | 0.57 | 0.41 | 0.76 |
| SEQ ID NO: 36 | 1.00 | 0.57 | 0.33 | 0.76 |
| SEQ ID NO: 60 | 1.00 | 0.57 | 0.32 | 0.76 |
| SEQ ID NO: 61 | 1.00 | 0.43 | 0.13 | 0.76 |
| SEQ ID NO: 55 | 1.00 | 0.56 | 0.27 | 0.76 |
| SEQ ID NO: 33 | 1.00 | 0.56 | 0.27 | 0.76 |
| SEQ ID NO: 21 | 1.00 | 0.56 | 0.21 | 0.76 |
| SEQ ID NO: 56 | 1.00 | 0.56 | 0.32 | 0.76 |
| SEQ ID NO: 24 | 1.00 | 0.56 | 0.27 | 0.76 |
| SEQ ID NO: 38 | 1.00 | 0.55 | 0.37 | 0.76 |
| SEQ ID NO: 48 | 1.00 | 0.55 | 0.33 | 0.76 |
| SEQ ID NO: 26 | 1.00 | 0.45 | 0.22 | 0.76 |
| SEQ ID NO: 59 | 1.00 | 0.54 | 0.38 | 0.76 |
| SEQ ID NO: 34 | 1.00 | 0.47 | 0.22 | 0.76 |
| SEQ ID NO: 52 | 1.00 | 0.53 | 0.22 | 0.76 |
| SEQ ID NO: 50 | 1.00 | 0.53 | 0.27 | 0.76 |
| SEQ ID NO: 14 | 1.00 | 0.47 | 0.21 | 0.76 |
| SEQ ID NO: 22 | 1.00 | 0.47 | 0.27 | 0.76 |
| SEQ ID NO: 32 | 1.00 | 0.52 | 0.29 | 0.76 |
| SEQ ID NO: 53 | 1.00 | 0.52 | 0.25 | 0.76 |
| SEQ ID NO: 44 | 1.00 | 0.48 | 0.27 | 0.76 |
| SEQ ID NO: 40 | 1.00 | 0.49 | 0.13 | 0.76 |
| SEQ ID NO: 27 | 1.00 | 0.50 | 0.33 | 0.76 |
| SEQ ID NO: 12 | 1.00 | 0.50 | 0.21 | 0.76 |
| SEQ ID NO: 31 | 1.00 | 0.50 | 0.16 | 0.76 |

TABLE 8: Corrected Wilcoxon p-value / AUC / Sensitivity / Specificity of the amplificates. Sensitivity is reported at a fixed specificity of -0.75.

| Marker | p-value | AUC | Sensitivity | Specificity |
|---|---|---|---|---|
| SEQ ID NO: 19 | 2.42E-04 | 0.72 | 0.59 | 0.76 |

(continued)

| Marker | p-value | AUC | Sensitivity | Specificity |
|---|---|---|---|---|
| SEQ ID NO: 35 | 2.48E-04 | 0.72 | 0.59 | 0.76 |
| SEQ ID NO: 37 | 0.05 | 0.66 | 0.37 | 0.76 |
| SEQ ID NO: 20 | 0.07 | 0.66 | 0.44 | 0.76 |
| SEQ ID NO: 13 | 0.08 | 0.65 | 0.46 | 0.76 |
| SEQ ID NO:9 | 0.11 | 0.65 | 0.37 | 0.76 |
| SEQ ID NO:41 | 0.11 | 0.65 | 0.37 | 0.76 |
| SEQ ID NO: 57 | 0.11 | 0.65 | 0.41 | 0.76 |
| SEQ ID NO: 51 | 0.12 | 0.65 | 0.49 | 0.76 |
| SEQ ID NO: 4 | 0.22 | 0.64 | 0.40 | 0.76 |
| SEQ ID NO: 1 | 0.24 | 0.64 | 0.41 | 0.76 |
| SEQ ID NO: 10 | 0.57 | 0.62 | 0.38 | 0.76 |
| SEQ ID NO: 15 | 0.61 | 0.62 | 0.38 | 0.76 |
| SEQ ID NO: 17 | 0.74 | 0.62 | 0.32 | 0.76 |
| SEQ ID NO: 58 | 0.93 | 0.62 | 0.40 | 0.76 |
| SEQ ID NO: 62 | 1.00 | 0.61 | 0.49 | 0.76 |
| SEQ ID NO: 43 | 1.00 | 0.39 | 0.11 | 0.76 |
| SEQ ID NO: 29 | 1.00 | 0.61 | 0.37 | 0.76 |
| SEQ ID NO: 16 | 1.00 | 0.60 | 0.35 | 0.76 |
| SEQ ID NO: 23 | 1.00 | 0.60 | 0.40 | 0.76 |
| SEQ ID NO: 5 | 1.00 | 0.60 | 0.44 | 0.76 |
| SEQ ID NO: 42 | 1.00 | 0.60 | 0.32 | 0.76 |
| SEQ ID NO: 49 | 1.00 | 0.59 | 0.37 | 0.76 |
| SEQ ID NO: 3 | 1.00 | 0.59 | 0.40 | 0.76 |
| SEQ ID NO: 47 | 1.00 | 0.59 | 0.35 | 0.76 |
| SEQ ID NO: 7 | 1.00 | 0.59 | 0.27 | 0.76 |
| SEQ ID NO: 28 | 1.00 | 0.59 | 0.37 | 0.76 |
| SEQ ID NO: 2 | 1.00 | 0.59 | 0.29 | 0.76 |
| SEQ ID NO: 18 | 1.00 | 0.42 | 0.14 | 0.76 |
| SEQ ID NO: 45 | 1.00 | 0.58 | 0.38 | 0.76 |
| SEQ ID NO: 8 | 1.00 | 0.58 | 0.25 | 0.76 |
| SEQ ID NO: 46 | 1.00 | 0.42 | 0.13 | 0.76 |
| SEQ ID NO: 30 | 1.00 | 0.58 | 0.32 | 0.76 |
| SEQ ID NO: 39 | 1.00 | 0.58 | 0.35 | 0.76 |
| SEQ ID NO: 54 | 1.00 | 0.57 | 0.32 | 0.76 |
| SEQ ID NO: 25 | 1.00 | 0.43 | 0.17 | 0.76 |
| SEQ ID NO: 6 | 1.00 | 0.57 | 0.30 | 0.76 |
| SEQ ID NO: 11 | 1.00 | 0.57 | 0.41 | 0.76 |
| SEQ ID NO: 36 | 1.00 | 0.57 | 0.33 | 0.76 |

(continued)

| Marker | p-value | AUC | Sensitivity | Specificity |
|---|---|---|---|---|
| SEQ ID NO: 60 | 1.00 | 0.57 | 0.32 | 0.76 |
| SEQ ID NO: 61 | 1.00 | 0.43 | 0.13 | 0.76 |
| SEQ ID NO: 55 | 1.00 | 0.56 | 0.27 | 0.76 |
| SEQ ID NO: 33 | 1.00 | 0.56 | 0.27 | 0.76 |
| SEQ ID NO: 21 | 1.00 | 0.56 | 0.21 | 0.76 |
| SEQ ID NO: 56 | 1.00 | 0.56 | 0.32 | 0.76 |
| SEQ ID NO:24 | 1.00 | 0.56 | 0.27 | 0.76 |
| SEQ ID NO:38 | 1.00 | 0.55 | 0.37 | 0.76 |
| SEQ ID NO:48 | 1.00 | 0.55 | 0.33 | 0.76 |
| SEQ ID NO: 26 | 1.00 | 0.45 | 0.22 | 0.76 |
| SEQ ID NO: 59 | 1.00 | 0.54 | 0.38 | 0.76 |
| SEQ ID NO: 34 | 1.00 | 0.47 | 0.22 | 0.76 |
| SEQ ID NO: 52 | 1.00 | 0.53 | 0.22 | 0.76 |
| SEQ ID NO: 50 | 1.00 | 0.53 | 0.27 | 0.76 |
| SEQ ID NO: 14 | 1.00 | 0.47 | 0.21 | 0.76 |
| SEQ ID NO: 22 | 1.00 | 0.47 | 0.27 | 0.76 |
| SEQ ID NO: 32 | 1.00 | 0.52 | 0.29 | 0.76 |
| SEQ ID NO: 53 | 1.00 | 0.52 | 0.25 | 0.76 |
| SEQ ID NO: 44 | 1.00 | 0.48 | 0.27 | 0.76 |
| SEQ ID NO: 40 | 1.00 | 0.49 | 0.13 | 0.76 |
| SEQ ID NO: 27 | 1.00 | 0.50 | 0.33 | 0.76 |
| SEQ ID NO:12 | 1.00 | 0.50 | 0.21 | 0.76 |
| SEQ ID NO: 31 | 1.00 | 0.50 | 0.16 | 0.76 |

TABLE 9: Primers according to EXAMPLE 3.

| *Gene* | *Primers:* | *Amplificate Length:* |
|---|---|---|
| CCND2<br>(SEQ ID NO: 1) | AAAAACAACCTTAACTC<br>AAACAT<br>(SEQ ID NO: 322)<br>TTTGGAGGGATAGAAT<br>GTGA<br>(SEQ ID NO: 321) | 504 |

(continued)

| *Gene* | *Primers:* | *Amplificate Length:* |
|---|---|---|
| CDKN2A<br>(SEQ ID NO: 2) | AGATTATTAGTTTTTATT<br>TGAGGGATT<br>(SEQ ID NO: 323)<br>CCACCCTAACTCTAACC<br>ATTC<br>(SEQ ID NO: 324) | |
| CD44<br>(SEQ ID NO: 3) | TTTTTGTTTGGGTGTGT<br>TTT<br>(SEQ ID NO: 325)<br>CACTTAACTCCAATCCC<br>CC<br>(SEQ ID NO: 326) | 403 |
| EDNRB1<br>(SEQ ID NO: 4) | CAAAAACTTCTCAAATC<br>AACAA<br>(SEQ ID NO: 328)<br>GAAGGGATGAATGAAT<br>AAAAGT<br>(SEQ ID NO: 327) | 446 |
| ELK1<br>(SEQ ID NO: 5) | TCCAATAAACACAAACC<br>TAAATC<br>(SEQ ID NO: 330)<br>ATATGGGATTGATGGA<br>AGATAG<br>(SEQ ID NO: 329) | |
| FOS<br>(SEQ ID NO: 6) | TTTTTATTTAGGATGAG<br>GGATATT<br>(SEQ ID NO: 331)<br>ACTACTTCCCACCCAAC<br>C<br>(SEQ ID NO: 332) | |

(continued)

| Gene | Primers: | Amplificate Length: |
|---|---|---|
| GSTP1 (SEQ ID NO: 7) | CTACTATCTATTTACTC CCTAAAC (SEQ ID NO: 334) TTGGTTTTATGTTGGGA GTTTTG (SEQ ID NO: 333) | 347 |
| RARB (SEQ ID NO: 8) | GGGAGTTTTTAAGTTTT GTGAG (SEQ ID NO: 335) TTAATCTTTTTCCCAAC CC (SEQ ID NO: 336) | 488 |
| PTGS2 (SEQ ID NO: 9) | AACATATCAACCTTTCT TAACCTT (SEQ ID NO: 338) AGAGGGGGTAGTTTTT ATTTTT (SEQ ID NO: 337) | 344 |
| RASSF1 (SEQ ID NO: 10) | AGTGGGTAGGTTAAGT GTGTTG (SEQ ID NO: 339) CCCCAAAATCCAAACTA AA (SEQ ID NO: 340) | 319 |
| ESR2 (SEQ ID NO: 11) | AGTTGGAGAAATTGAAA AGATTA (SEQ ID NO: 341) TAACAAACCCAAAACCT CTCTA (SEQ ID NO: 342) | 441 |

(continued)

| Gene | Primers: | Amplificate Length: |
|---|---|---|
| DRG1 (SEQ ID NO: 12) | TTTAGTTGTGAAAAAGG GATTT (SEQ ID NO: 343) CCCTATAACCTCCACAC TATCTC (SEQ ID NO: 344) | 436 |
| DRG1 (SEQ ID NO: 12) | CCCATCCCACAATTAAA A (SEQ ID NO: 346) GTTTTGGAGGGAGTAG AGATT (SEQ ID NO: 345) | |
| CMYA3 (SEQ ID NO: 13) | ACTCCCCAAAATCCCA CT (SEQ ID NO: 348) TGTTTTAGGTTTGATGG ATTAGA (SEQ ID NO: 347) | 488 |
| ONECUT2 (SEQ ID NO: 14) | GAAGAGGTGTTGAGAA ATTAAAA (SEQ ID NO: 349) CCCACCCTAACTTACCA TAAA (SEQ ID NO: 350) | 462 |
| MX1 (SEQ ID NO: 15) | TGTAGGAGAGGTTGGG AAG (SEQ ID NO: 351) CCAAACATAACATCCAC TAAAA (SEQ ID NO: 352) | 341 |

(continued)

| *Gene* | *Primers:* | *Amplificate Length:* |
|---|---|---|
| DOCK10 (SEQ ID NO: 16) | TACCTCTTCCCTCTACC AAAC (SEQ ID NO: 354) GTTTTTAAGTGTTGGGT GATTT (SEQ ID NO: 353) | 459 |
| BTG4 (SEQ ID NO: 17) | AAGAGTTTTAGGAAATG TGTTTTT (SEQ ID NO: 355) TTCTACTCACCAAACCC TCTAC (SEQ ID NO: 356) | 199 |
| DMRTC2 (SEQ ID NO: 18) | TAAGGTAAGGGAAGGT TAGAAA (SEQ ID NO: 357) ATTACCACAACCTCCAA TAAAA (SEQ ID NO: 358) | 477 |
| GPR7 (SEQ ID NO: 19) | TTATTATTTTAGATGGA GTGAGGTT (SEQ ID NO: 359) ACCCAAATTACCCCACA A (SEQ ID NO: 360) | 442 |
| FAT (SEQ ID NO: 20) | TACTCACCCCAATCTTC ACTA (SEQ ID NO: 362) AGATGTTTTATATTTGT TTGGGA (SEQ ID NO: 361) | 444 |

(continued)

| *Gene* | *Primers:* | *Amplificate Length:* |
|---|---|---|
| ISL1 (SEQ ID NO: 21) | ATCTCCCAAAAACAATC ACA (SEQ ID NO: 364) TAAAAATGGAAGGGAA GATAGA (SEQ ID NO: 363) | 412 |
| GPRK5 (SEQ ID NO: 22) | TTGTGGTTATTTTGAGA TGGTA (SEQ ID NO: 365) CCCTCCCCCTAACTAAA A (SEQ ID NO: 366) | 416 |
| SLC35F2 (SEQ ID NO: 23) | TTTATTTATTAGGTGAA GAGTTTGTT (SEQ ID NO: 367) TCCTCCTACCACCCTAA AA (SEQ ID NO: 368) | 366 |
| C14orf59 (SEQ ID NO: 24) | AAAACTCCTCCCCTCTA TAAAT (SEQ ID NO: 370) TTGGAGAGATGTGTTG GTTAG (SEQ ID NO: 369) | 492 |
| SNRPN (SEQ ID NO: 25) | CCCCTCTCATTACAACA ATACT (SEQ ID NO: 372) TTTTTAGAATAAAGGAT TTTAGGG (SEQ ID NO: 371) | 407 |

(continued)

| Gene | Primers: | Amplificate Length: |
|---|---|---|
| ARHGEF18 (SEQ ID NO: 26) | TTTTAGGAATGTAGGATATAAGGG (SEQ ID NO: 373) CCCCACATAAAAACCTATCC (SEQ ID NO: 374) | 454 |
| SNX8 (SEQ ID NO: 27) | TCCCTCCTAACTCAACACTAA (SEQ ID NO: 376) TAGGGTTTGATGATGTGATTTT (SEQ ID NO: 375) | 273 |
| FBN2 (SEQ ID NO: 28) | GAGAGGGAGGGTTAAGGTT (SEQ ID NO: 377) ACTACTACCTCCTTTCCCAAAT (SEQ ID NO: 378) | 193 |
| HOXB5 (SEQ ID NO: 29) | CTCCTCAATTCTCACCAAAA (SEQ ID NO: 380) GTGGAAAAAGGAGAGTAAATTG (SEQ ID NO: 379) | 356 |
| LIMK1 (SEQ ID NO: 30) | AAACCCTACTTCCTACAAACAA (SEQ ID NO: 382) AGGGAGGTTTGGTGTATTTT (SEQ ID NO: 381) | |

(continued)

| Gene | Primers: | Amplificate Length: |
|---|---|---|
| PSD/Q9H469 (SEQ ID NO: 31) | AAGGTATTATTTTTGGG GTTTT (SEQ ID NO: 383) AACTATCCAACCTCTTC CACTT (SEQ ID NO: 384) | 333 |
| SLC38A1 (SEQ ID NO: 32) | GGGTGTTGGGGAGTTT TA (SEQ ID NO: 385) CTTACAATAACTCACTA TCCTTTCC (SEQ ID NO: 386) | 334 |
| SLC38A1 (SEQ ID NO: 32) | ACAAACATCCTTTAATA ATTTCTCC (SEQ ID NO: 388) AGAGTGTGGTATTAGAT TTGGTTTT (SEQ ID NO: 387) | 317 |
| HIST1H4J (SEQ ID NO: 33) | TTAGTTGAGAAAGTGG GGGT (SEQ ID NO: 389) CTACCTCAAACCAAAAT CCTC (SEQ ID NO: 390) | 421 |
| Q96S01 (SEQ ID NO: 34) | ACCCCAATCAACTACAT AACTAA (SEQ ID NO: 392) GTGAGAGTGGGTGTTG AAAT (SEQ ID NO: 391) | 498 |

(continued)

| *Gene* | *Primers:* | *Amplificate Length:* |
|---|---|---|
| Genomic region downstream of FOXL2 (SEQ ID NO: 35) | ATTTTAGGTGTAAGTTTAAGGTTGT (SEQ ID NO: 393)<br>ATCTACCTTTCCCCACCC (SEQ ID NO: 394) | 473 |
| ORC4L (SEQ ID NO: 36) | GTTGAGAGGTAAGGTATGAAGG (SEQ ID NO: 395)<br>TTAATTCCCCTCTTTAACCTAATAA (SEQ ID NO: 396) | 477 |
| ABHD9 (SEQ ID NO: 37) | GTGTTAGGGTTTAGGGGTTT (SEQ ID NO: 397)<br>CCTTTCCAACCTCTTCCT (SEQ ID NO: 398) | 209 |
| CD37 (SEQ ID NO: 38) | CCTCATCAACCAACCCTA (SEQ ID NO: 400)<br>TAGATGGGGATAGGAAGTTGT (SEQ ID NO: 399) | 466 |
| GRN (SEQ ID NO: 39) | CATTCCAAACTAACCCCA (SEQ ID NO: 402)<br>TTATTAGGAGAGGGGAAGAAGT (SEQ ID NO: 401) | 466 |

(continued)

| Gene | Primers: | Amplificate Length: |
|---|---|---|
| NOTCH1 (SEQ ID NO: 41) | CCCACCCTAAAAACTCA CTA (SEQ ID NO: 406) GGAGGGGTTTGAGTAA TTG (SEQ ID NO: 405) | 424 |
| MLLT3 (SEQ ID NO: 42) | GATTAGGTTTGGAGTT GTTTTT (SEQ ID NO: 407) AATCACATCCATCTTTC ACTTT (SEQ ID NO: 408) | |
| SOLH (SEQ ID NO: 43) | CCCAACTCCCCAAATAA A (SEQ ID NO: 410) GGGGATAAGTGGTTAA TGAGT (SEQ ID NO: 409) | 389 |
| SEQ ID NO: 44 (SEQ ID NO: 44) | ATAGTGTGGATTTTTAG GGATATT (SEQ ID NO: 411) CAAAACCTATTCCCCTA CCT (SEQ ID NO: 412) | 448 |
| Q8N365 (SEQ ID NO: 45) | GTAGTAAATGGGTTATG GATTTTAG (SEQ ID NO: 413) CCATACCACCCACAAA CA (SEQ ID NO: 414) | 475 |

(continued)

| *Gene* | *Primers:* | *Amplificate Length:* |
|---|---|---|
| Q9NWV0 (SEQ ID NO: 46) | TTTTAGTAGTGGATTTG GTTAGTATT (SEQ ID NO: 415) CATCTCTTAACCCCACT TTCA (SEQ ID NO: 416) | 330 |
| SEQ ID NO: 47 (SEQ ID NO: 47) | TCCCTCTAAAAACCAAA AATC (SEQ ID NO: 418) GAGGAAAGAAGGAGGA TATAGG (SEQ ID NO: 417) | 335 |
| H2AFY2 (SEQ ID NO: 48) | GTTAAAGGGGTATTGG TTTTT (SEQ ID NO: 419) TTTCTTTTTCTCTCACC TATTAAAC (SEQ ID NO: 420) | 490 |
| RHOC (SEQ ID NO: 49) | GTAAGAGGGATAGGGA ATTGG (SEQ ID NO: 421) AACACCCAAACCAAAAT AAAA (SEQ ID NO: 422) | 440 |
| NR2E1 (SEQ ID NO: 50) | GGAGTTTGTGAAAAGT GGG (SEQ ID NO: 423) ACTCAACAAATACAATA ATCTAAACC (SEQ ID NO: 424) | 429 |

(continued)

| Gene | Primers: | Amplificate Length: |
|---|---|---|
| KBTBD6 (SEQ ID NO: 51) | ACTATACCAACAAAACT ACAAAATAAA (SEQ ID NO: 426) GAAGGTTGAGGAGGAG TTAGA (SEQ ID NO: 425) | 228 |
| TRPM4 (SEQ ID NO: 52) | GGTTGGAAAGTGGAGG ATT (SEQ ID NO: 427) CCAACTCTAAAAACAAA AACAA (SEQ ID NO: 428) | 438 |
| TCEB3BP1 (SEQ ID NO: 53) | GAGTTGGTTTTGTTGAG GTGT (SEQ ID NO: 429) AAAATACCTTCCCACTA ACCTT (SEQ ID NO: 430) | 325 |
| Q8NCX8 (SEQ ID NO: 54) | TAGTGGAATTATGAGG GGG (SEQ ID NO: 431) CCAAATACACCTCTACC AAAA (SEQ ID NO: 432) | 300 |
| SEQ ID NO: 55 (SEQ ID NO: 55) | CCTTACCCTCCTCTCCT AAA (SEQ ID NO: 434) TAGGATTTGTGGTTGGT GTT (SEQ ID NO: 433) | 384 |

(continued)

| Gene | Primers: | Amplificate Length: |
|---|---|---|
| SNAPC2 (SEQ ID NO: 56) | GGTTTAGGGTATTTTAA GGGG (SEQ ID NO: 435) AAACTAAATCCAACTCC CAAA (SEQ ID NO: 436) | 362 |
| PTPRN2 (SEQ ID NO: 57) | CCCTCTACTCACTTTAC CAAAA (SEQ ID NO: 438) GGGGAGGTGTTTAGTG GTT (SEQ ID NO: 437) | 378 |
| WDFY3 (SEQ ID NO: 58) | TGTTGGTGGTTATTTTT AATTTTT (SEQ ID NO: 439) ACCCAATTATCCTTTCT CAAC (SEQ ID NO: 440) | 435 |
| ZNF566 (SEQ ID NO: 59) | GAGGATTTGTGTTAAG GTTTTT (SEQ ID NO: 441) CCAACTCCACTATCTAC CACAT (SEQ ID NO: 442) | 457 |
| Q9NP73 (SEQ ID NO: 60) | GGGGTTTTAAGAGTTG GTTTT (SEQ ID NO: 443) CCTCCCTCACTCACTTA CAA (SEQ ID NO: 444) | |

(continued)

| Gene | Primers: | Amplificate Length: |
|---|---|---|
| SEQ ID NO: 61 (SEQ ID NO: 61) | TGGGGATATTGGATGT TTTT (SEQ ID NO: 445) CCTTCCAACCTAACCTC C (SEQ ID NO: 446) | 497 |
| Q86SP6 (SEQ ID NO: 62) | CCCAACACTCATTTACA CTATCT (SEQ ID NO: 448) GGAGTTTTAATTTTTGG GATTT (SEQ ID NO: 447) | 342 |

TABLE 10: Detection oligonucleotides according to Example 3.

| No: | Gene | Oligo: |
|---|---|---|
| 1 | ONECUT2 (SEQ ID NO: 14) | TAGAGGCGCGGGTTAT (SEQ ID NO: 449) |
| 2 | ONECUT2 (SEQ ID NO: 14) | TAGAGGTGTGGGTTAT (SEQ ID NO: 450) |
| 3 | ONECUT2 (SEQ ID NO: 14) | TTGCGATTGGTACGTA (SEQ ID NO: 451) |
| 4 | ONECUT2 (SEQ ID NO: 14) | TGTGATTGGTATGTAGT (SEQ ID NO: 452) |
| 5 | ONECUT2 (SEQ ID NO: 14) | TTTTGTGCGTACGGAT (SEQ ID NO: 453) |
| 6 | ONECUT2 (SEQ ID NO: 14) | TTTTTGTGTGTATGGAT (SEQ ID NO: 454) |
| 7 | ONECUT2 (SEQ ID NO: 14) | TTAAGCGGGCGTTGAT (SEQ ID NO: 455) |
| 8 | ONECUT2 (SEQ ID NO: 14) | TTAAGTGGGTGTTGAT (SEQ ID NO: 456) |
| 9 | MX1 (SEQ ID NO: 15) | GTTACGAGTTTATTCGA (SEQ ID NO: 457) |
| 10 | MX1 (SEQ ID NO: 15) | GGTTATGAGTTTATTTGAA (SEQ ID NO: 458) |
| 11 | MX1 (SEQ ID NO: 15) | AACGCGCGAAAGTAAA (SEQ ID NO: 459) |
| 12 | MX1 (SEQ ID NO: 15) | TTGGGAATGTGTGAAA (SEQ ID NO: 460) |

(continued)

| No: | Gene | Oligo: |
|---|---|---|
| 13 | MX1 (SEQ ID NO: 15) | GAGTTTTCGTCGATTT (SEQ ID NO: 461) |
| 14 | MX1 (SEQ ID NO: 15) | AGGAGTTTTTGTTGATT (SEQ ID NO: 462) |
| 15 | MX1 (SEQ ID NO: 15) | TATGCGCGGGAAGATT (SEQ ID NO: 463) |
| 16 | MX1 (SEQ ID NO: 15) | GTATGTGTGGGAAGAT (SEQ ID NO: 464) |
| 17 | DOCK10 (SEQ ID NO: 16) | GATCGGAATTCGGGTT (SEQ ID NO: 465) |
| 18 | DOCK10 (SEQ ID NO: 16) | ATTGGAATTTGGGTTG (SEQ ID NO: 466) |
| 19 | DOCK10 (SEQ ID NO: 16) | TAGTAGTCGCGTTTTT (SEQ ID NO: 467) |
| 20 | DOCK10 (SEQ ID NO: 16) | AGTAGTTGTGTTTTTGG (SEQ ID NO: 468) |
| 21 | DOCK10 (SEQ ID NO: 16) | ATTTTCGCGGGAAGTT (SEQ ID NO: 469) |
| 22 | DOCK10 (SEQ ID NO: 16) | GTGATTTTTGTGGGAA (SEQ ID NO: 470) |
| 23 | BTG4 (SEQ ID NO: 17) | AGTTAGCGTTTGTCGG (SEQ ID NO: 471) |
| 24 | BTG4 (SEQ ID NO: 17) | TAGTTAGTGTTTGTTGG (SEQ ID NO: 472) |
| 25 | BTG4 (SEQ ID NO: 17) | TTCGGCGTCGATGTAT (SEQ ID NO: 473) |
| 26 | BTG4 (SEQ ID NO: 17) | TTTGGTGTTGATGTATT (SEQ ID NO: 474) |
| 27 | DMRTC2 (SEQ ID NO: 18) | GGGTATTCGACGTTTT (SEQ ID NO: 475) |
| 28 | DMRTC2 (SEQ ID NO: 18) | AGGGGTATTTGATGTTT (SEQ ID NO: 476) |
| 29 | DMRTC2 (SEQ ID NO: 18) | ATTCGAAGCGTTATTG (SEQ ID NO: 477) |
| 30 | DMRTC2 (SEQ ID NO: 18) | TTTGAAGTGTTATTGGT (SEQ ID NO: 478) |
| 31 | DMRTC2 (SEQ ID NO: 18) | AAATCGTATTGGTCGT (SEQ ID NO: 479) |
| 32 | DMRTC2 (SEQ ID NO: 18) | AAATTGTATTGGTTGTTT (SEQ ID NO: 480) |
| 33 | DMRTC2 (SEQ ID NO: 18) | AATTCGTGTATAGATCGG (SEQ ID NO: 481) |
| 34 | DMRTC2 (SEQ ID NO: 18) | ATTTGTGTATAGATTGGG (SEQ ID NO: 482) |

(continued)

| No: | Gene | Oligo: |
|---|---|---|
| 35 | DMRTC2 (SEQ ID NO: 18) | AAAAGTAGCGCGAGTT (SEQ ID NO: 483) |
| 36 | DMRTC2 (SEQ ID NO: 18) | AGTAGTGTGAGTTTGG (SEQ ID NO: 484) |
| 37 | GPR7 (SEQ ID NO: 19) | GAACGTAGTCGCGGTT (SEQ ID NO: 485) |
| 38 | GPR7 (SEQ ID NO: 19) | GGAATGTAGTTGTGGT (SEQ ID NO: 486) |
| 39 | GPR7 (SEQ ID NO: 19) | ATTTTGGCGAATTCGG (SEQ ID NO: 487) |
| 40 | GPR7 (SEQ ID NO: 19) | TGGTGAATTTGGGGGA (SEQ ID NO: 488) |
| 41 | GPR7 (SEQ ID NO: 19) | TTAGTCGGTAGGCGTT (SEQ ID NO: 489) |
| 42 | GPR7 (SEQ ID NO: 19) | ATTTAGTTGGTAGGTGT (SEQ ID NO: 490) |
| 43 | GPR7 (SEQ ID NO: 19) | TTTTCGTAGTCGGCGG (SEQ ID NO: 491) |
| 44 | GPR7 (SEQ ID NO: 19) | TTTTTTGTAGTTGGTGG (SEQ ID NO: 492) |
| 45 | FAT (SEQ ID NO: 20) | TAAGCGTTAATAGAACGA (SEQ ID NO: 493) |
| 46 | FAT (SEQ ID NO: 20) | AGTGTTAATAGAATGAAAT (SEQ ID NO: 494) |
| 47 | FAT (SEQ ID NO: 20) | TTGTATTTCGTCGTTAT (SEQ ID NO: 495) |
| 48 | FAT (SEQ ID NO: 20) | TTTTGTTGTTATAGGAGT (SEQ ID NO: 496) |
| 49 | FAT (SEQ ID NO: 20) | ATAGGAGTCGTCGAGA (SEQ ID NO: 497) |
| 50 | FAT (SEQ ID NO: 20) | ATAGGAGTTGTTGAGAG (SEQ ID NO: 498) |
| 51 | ISL1 (SEQ ID NO: 21) | TTAATGCGGTCGGTTA (SEQ ID NO: 499) |
| 52 | ISL1 (SEQ ID NO: 21) | AGTTAATGTGGTTGGT (SEQ ID NO: 500) |
| 53 | GPRK5 (SEQ ID NO: 22) | TTTGATTCGCGGTCGG (SEQ ID NO: 501) |
| 54 | GPRK5 (SEQ ID NO: 22) | ATTTGATTTGTGGTTGG (SEQ ID NO: 502) |
| 55 | GPRK5 (SEQ ID NO: 22) | TATCGTCGGTCGAGTT (SEQ ID NO: 503) |
| 56 | GPRK5 (SEQ ID NO: 22) | TTTATTGTTGGTTGAGT (SEQ ID NO: 504) |

(continued)

| No: | Gene | Oligo: |
|---|---|---|
| 57 | GPRK5 (SEQ ID NO: 22) | ATGTCGAGAGTTCGTA (SEQ ID NO: 505) |
| 58 | GPRK5 (SEQ ID NO: 22) | GTTGAGAGTTTGTATGT (SEQ ID NO: 506) |
| 59 | SLC35F2 (SEQ ID NO: 23) | TTTCGGCGTTTAAAAT (SEQ ID NO: 507) |
| 60 | SLC35F2 (SEQ ID NO: 23) | TTTTTGGTGTTTAAAATTT (SEQ ID NO: 508) |
| 61 | SLC35F2 (SEQ ID NO: 23) | ATTTTCGAAGTGTCGG (SEQ ID NO: 509) |
| 62 | SLC35F2 (SEQ ID NO: 23) | TTTGAAGTGTTGGGTT (SEQ ID NO: 510) |
| 63 | SLC35F2 (SEQ ID NO: 23) | TTTCGGAAGACGGGAG (SEQ ID NO: 511) |
| 64 | SLC35F2 (SEQ ID NO: 23) | TTTTGGAAGATGGGAG (SEQ ID NO: 512) |
| 65 | SLC35F2 (SEQ ID NO: 23) | AATTCGGTCGTCGTTT (SEQ ID NO: 513) |
| 66 | SLC35F2 (SEQ ID NO: 23) | AGAATTTGGTTGTTGTT (SEQ ID NO: 514) |
| 67 | C14orf59 (SEQ ID NO: 24) | GACGTAGGGACGGAGA (SEQ ID NO: 515) |
| 68 | C14orf59 (SEQ ID NO: 24) | GATGTAGGGATGGAGA (SEQ ID NO: 516) |
| 69 | C14orf59 (SEQ ID NO: 24) | TATCGTGGTTTTTTACGTAT (SEQ ID NO: 517) |
| 70 | C14orf59 (SEQ ID NO: 24) | ATTGTGGTTTTTTATGTATA (SEQ ID NO: 518) |
| 71 | C14orf59 (SEQ ID NO: 24) | GTGTTCGAGAGCGAGT (SEQ ID NO: 519) |
| 72 | C14orf59 (SEQ ID NO: 24) | TGTTTGAGAGTGAGTGT (SEQ ID NO: 520) |
| 73 | C14orf59 (SEQ ID NO: 24) | TTTATTCGGTGTTCGA (SEQ ID NO: 521) |
| 74 | C14orf59 (SEQ ID NO: 24) | TATTTGGTGTTTGAGAG (SEQ ID NO: 522) |
| 75 | SNRPN (SEQ ID NO: 25) | TTTTTGCGGTCGCGTA (SEQ ID NO: 523) |
| 76 | SNRPN (SEQ ID NO: 25) | TTTTGTGGTTGTGTAGG (SEQ ID NO: 524) |
| 77 | SNRPN (SEQ ID NO: 25) | AGTATGCGCGTTAGTT (SEQ ID NO: 525) |
| 78 | SNRPN (SEQ ID NO: 25) | TGAGTATGTGTGTTAGT (SEQ ID NO: 526) |

(continued)

| No: | Gene | Oligo: |
|---|---|---|
| 79 | SNRPN (SEQ ID NO: 25) | TAGCGGTAGGTTTCGTA (SEQ ID NO: 527) |
| 80 | SNRPN (SEQ ID NO: 25) | TAGTGGTAGGTTTTGTA (SEQ ID NO: 528) |
| 81 | SNRPN (SEQ ID NO: 25) | TTTGCGTTAGATTCGT (SEQ ID NO: 529) |
| 82 | SNRPN (SEQ ID NO: 25) | TGTGTTAGATTTGTTGT (SEQ ID NO: 530) |
| 83 | ARHGEF18 (SEQ ID NO: 26) | GTCGATTCGGTTGATT (SEQ ID NO: 531) |
| 84 | ARHGEF18 (SEQ ID NO: 26) | AAGGTTGATTTGGTTG (SEQ ID NO: 532) |
| 85 | ARHGEF18 (SEQ ID NO: 26) | GGCGGTTTCGAAGATT (SEQ ID NO: 533) |
| 86 | ARHGEF18 (SEQ ID NO: 26) | AGATGGGTGGTTTTGA (SEQ ID NO: 534) |
| 87 | ARHGEF18 (SEQ ID NO: 26) | AAGTCGGTTATGAGCGA (SEQ ID NO: 535) |
| 88 | ARHGEF18 (SEQ ID NO: 26) | AAGTTGGTTATGAGTGA (SEQ ID NO: 536) |
| 89 | ARHGEF18 (SEQ ID NO: 26) | TGGTCGATACGGTATT (SEQ ID NO: 537) |
| 90 | ARHGEF18 (SEQ ID NO: 26) | TTGTGGTTGATATGGT (SEQ ID NO: 538) |
| 91 | SNX8 (SEQ ID NO: 27) | AGGACGCGATAGGGAT (SEQ ID NO: 539) |
| 92 | SNX8 (SEQ ID NO: 27) | AGGATGTGATAGGGAT (SEQ ID NO: 540) |
| 93 | SNX8 (SEQ ID NO: 27) | ATTTCGTCGTATGTGA (SEQ ID NO: 541) |
| 94 | SNX8 (SEQ ID NO: 27) | ATTTTGTTGTATGTGAAG (SEQ ID NO: 542) |
| 95 | SNX8 (SEQ ID NO: 27) | GTCGTTTGCGTATTTA (SEQ ID NO: 543) |
| 96 | SNX8 (SEQ ID NO: 27) | GGTTGTTTGTGTATTTAA (SEQ ID NO: 544) |
| 97 | SNX8 (SEQ ID NO: 27) | ATTGTATACGCGCGTT (SEQ ID NO: 545) |
| 98 | SNX8 (SEQ ID NO: 27) | TTGTATATGTGTGTTGG (SEQ ID NO: 546) |
| 99 | FBN2 (SEQ ID NO: 28) | TAAAGCGAGTAGACGG (SEQ ID NO: 547) |
| 100 | FBN2 (SEQ ID NO: 28) | TATAAAGTGAGTAGATGG (SEQ ID NO: 548) |

(continued)

| No: | Gene | Oligo: |
|---|---|---|
| 101 | HOXB5 (SEQ ID NO: 29) | ATAGTTTTCGGCGGGT (SEQ ID NO: 549) |
| 102 | HOXB5 (SEQ ID NO: 29) | TATAGTTTTTGGTGGGT (SEQ ID NO: 550) |
| 103 | HOXB5 (SEQ ID NO: 29) | TTTTTCGGCGTAGATA (SEQ ID NO: 551) |
| 104 | HOXB5 (SEQ ID NO: 29) | TGTTTTTTGGTGTAGAT (SEQ ID NO: 552) |
| 105 | HOXB5 (SEQ ID NO: 29) | AGTCGAGGGCGTTAGA (SEQ ID NO: 553) |
| 106 | HOXB5 (SEQ ID NO: 29) | AGTTGAGGGTGTTAGA (SEQ ID NO: 554) |
| 107 | HOXB5 (SEQ ID NO: 29) | TTTTCGAGGAATTCGT (SEQ ID NO: 555) |
| 108 | HOXB5 (SEQ ID NO: 29) | TTTTTTGAGGAATTTGTT (SEQ ID NO: 556) |
| 109 | LIMK1 (SEQ ID NO: 30) | TATCGGATTATCGCGG (SEQ ID NO: 557) |
| 110 | LIMK1 (SEQ ID NO: 30) | ATTGGATTATTGTGGGG (SEQ ID NO: 558) |
| 111 | LIMK1 (SEQ ID NO: 30) | GTCGGTAGTTTATCGGAT (SEQ ID NO: 559) |
| 112 | LIMK1 (SEQ ID NO: 30) | GTTGGTAGTTTATTGGAT (SEQ ID NO: 560) |
| 113 | LIMK1 (SEQ ID NO: 30) | TAGGAGACGTTACGTT (SEQ ID NO: 561) |
| 114 | LIMK1 (SEQ ID NO: 30) | AGATGTTATGTTAGGGT (SEQ ID NO: 562) |
| 115 | PSD/Q9H469 (SEQ ID NO: 31) | TTTTTCGAAGCGGATT (SEQ ID NO: 563) |
| 116 | PSD/Q9H469 (SEQ ID NO: 31) | TTTGAAGTGGATTTTGG (SEQ ID NO: 564) |
| 117 | PSD/Q9H469 (SEQ ID NO: 31) | GAAACGCGGTTTAAAT (SEQ ID NO: 565) |
| 118 | PSD/Q9H469 (SEQ ID NO: 31) | GGAAATGTGGTTTAAATT (SEQ ID NO: 566) |
| 119 | SLC38A1 (SEQ ID NO: 32) | TTTGCGGTAACGTTTA (SEQ ID NO: 567) |
| 120 | SLC38A1 (SEQ ID NO: 32) | TTGTGGTAATGTTTAGG (SEQ ID NO: 568) |
| 121 | SLC38A1 (SEQ ID NO: 32) | TAGCGGTCGCGGATTA (SEQ ID NO: 569) |
| 122 | SLC38A1 (SEQ ID NO: 32) | GTAGTGGTTGTGGATT (SEQ ID NO: 570) |

(continued)

| No: | Gene | Oligo: |
|---|---|---|
| 123 | SLC38A1 (SEQ ID NO: 32) | TTAGGGACGCGAATTA (SEQ ID NO: 571) |
| 124 | SLC38A1 (SEQ ID NO: 32) | AGGGATGTGAATTAGG (SEQ ID NO: 572) |
| 125 | SLC38A1 (SEQ ID NO: 32) | TGCGTTTAAGATCGCGT (SEQ ID NO: 573) |
| 126 | SLC38A1 (SEQ ID NO: 32) | TGTGTTTAAGATTGTGT (SEQ ID NO: 574) |
| 127 | SLC38A1 (SEQ ID NO: 32) | ATTTCGGTTTTCGAAA (SEQ ID NO: 575) |
| 128 | SLC38A1 (SEQ ID NO: 32) | ATATTTTGGTTTTTGAAAA (SEQ ID NO: 576) |
| 129 | SLC38A1 (SEQ ID NO: 32) | AGAGCGTAGTTGATTCGA (SEQ ID NO: 577) |
| 130 | SLC38A1 (SEQ ID NO: 32) | AGAGTGTAGTTGATTTGA (SEQ ID NO: 578) |
| 131 | SLC38A1 (SEQ ID NO: 32) | AGGAATTACGTACGTT (SEQ ID NO: 579) |
| 132 | SLC38A1 (SEQ ID NO: 32) | TATGTATGTTTGGAGGG (SEQ ID NO: 580) |
| 133 | SLC38A1 (SEQ ID NO: 32) | TTTGTAACGCGGGGAA (SEQ ID NO: 581) |
| 134 | SLC38A1 (SEQ ID NO: 32) | TTTTGTAATGTGGGGA (SEQ ID NO: 582) |
| 135 | HIST1H4J (SEQ ID NO: 33) | TATGGCGGTGATCGTT (SEQ ID NO: 583) |
| 136 | HIST1H4J (SEQ ID NO: 33) | TTTATGGTGGTGATTGT (SEQ ID NO: 584) |
| 137 | HIST1H4J (SEQ ID NO: 33) | TTACGGCGTTTCGGAT (SEQ ID NO: 585) |
| 138 | HIST1H4J (SEQ ID NO: 33) | TTATGGTGTTTTGGATT (SEQ ID NO: 586) |
| 139 | HIST1H4J (SEQ ID NO: 33) | ATGCGTTTTACGTCGT (SEQ ID NO: 587) |
| 140 | HIST1H4J (SEQ ID NO: 33) | AGATGTGTTTTATGTTGT (SEQ ID NO: 588) |
| 141 | HIST1H4J (SEQ ID NO: 33) | TATTGTCGCGTAGTAT (SEQ ID NO: 589) |
| 142 | HIST1H4J (SEQ ID NO: 33) | GGATATTGTTGTGTAGT (SEQ ID NO: 590) |
| 143 | HIST1 H4J (SEQ ID NO: 33) | ATCGAAATCGTAGAGG (SEQ ID NO: 591) |
| 144 | HIST1 H4J (SEQ ID NO: 33) | ATTGAAATTGTAGAGGG (SEQ ID NO: 592) |

(continued)

| No: | Gene | Oligo: |
|---|---|---|
| 145 | Q96S01 (SEQ ID NO: 34) | ATCGGTTTTTCGAGGT (SEQ ID NO: 593) |
| 146 | Q96S01 (SEQ ID NO: 34) | ATTGGTTTTTTGAGGTT (SEQ ID NO: 594) |
| 147 | Q96S01 (SEQ ID NO: 34) | GGTCGATTTTCGCGTA (SEQ ID NO: 595) |
| 148 | Q96S01 (SEQ ID NO: 34) | TGGTTGATTTTTGTGTA (SEQ ID NO: 596) |
| 149 | GENOMIC REGION DOWNSTREAM FROM FOXL2 (SEQ ID NO: 35) | AATCGTGCGGTTGATA (SEQ ID NO: 597) |
| 150 | GENOMIC REGION DOWNSTREAM FROM FOXL2 (SEQ ID NO: 35) | TGTAGAATTGTGTGGT (SEQ ID NO: 598) |
| 151 | GENOMIC REGION DOWNSTREAM FROM FOXL2 (SEQ ID NO: 35) | AAAATTCGAGGTCGGG (SEQ ID NO: 599) |
| 152 | GENOMIC REGION DOWNSTREAM FROM FOXL2 (SEQ ID NO: 35) | AAAATTTGAGGTTGGG (SEQ ID NO: 600) |
| 153 | GENOMIC REGION DOWNSTREAM FROM FOXL2 (SEQ ID NO: 35) | TTTTCGCGGTTCGGAG (SEQ ID NO: 601) |
| 154 | GENOMIC REGION DOWNSTREAM FROM FOXL2 (SEQ ID NO: 35) | TTTGTGGTTTGGAGAA (SEQ ID NO: 602) |
| 155 | GENOMIC REGION DOWNSTREAM FROM FOXL2 (SEQ ID NO: 35) | AATAGGCGATGTACGG (SEQ ID NO: 603) |
| 156 | GENOMIC REGION DOWNSTREAM FROM FOXL2 (SEQ ID NO: 35) | TAGGTGATGTATGGGT (SEQ ID NO: 604) |
| 157 | GENOMIC REGION DOWNSTREAM FROM FOXL2 (SEQ ID NO: 35) | TTGGTCGGTTAATCGA (SEQ ID NO: 605) |
| 158 | GENOMIC REGION DOWNSTREAM FROM FOXL2 (SEQ ID NO: 35) | TTTGGTTGGTTAATTGA (SEQ ID NO: 606) |
| 159 | GENOMIC REGION DOWNSTREAM FROM FOXL2 (SEQ ID NO: 35) | TAGCGGTCGCGAAAAT (SEQ ID NO: 607) |
| 160 | GENOMIC REGION DOWNSTREAM FROM FOXL2 (SEQ ID NO: 35) | AGTTTAGTGGTTGTGA (SEQ ID NO: 608) |
| 161 | CMYA3 (SEQ ID NO: 13) | TTTACGCGGGGTTTTA (SEQ ID NO: 609) |
| 162 | CMYA3 (SEQ ID NO: 13) | TTTATGTGGGGTTTTAG (SEQ ID NO: 610) |
| 163 | CMYA3 (SEQ ID NO: 13) | TTACGTCGTTATTAGGT (SEQ ID NO: 611) |
| 164 | CMYA3 (SEQ ID NO: 13) | TTTTATGTTGTTATTAGGT (SEQ ID NO: 612) |
| 165 | CMYA3 (SEQ ID NO: 13) | TATTTGGACGTCGGGT (SEQ ID NO: 613) |
| 166 | CMYA3 (SEQ ID NO: 13) | TATTTGGATGTTGGGT (SEQ ID NO: 614) |

(continued)

| No: | Gene | Oligo: |
|---|---|---|
| 167 | CMYA3 (SEQ ID NO: 13) | TTTGTCGGAAAGCGGA (SEQ ID NO: 615) |
| 168 | CMYA3 (SEQ ID NO: 13) | TTTGTTGGAAAGTGGA (SEQ ID NO: 616) |
| 169 | ORC4L (SEQ ID NO: 36) | ATTCGGATCGTTACGT (SEQ ID NO: 617) |
| 170 | ORC4L (SEQ ID NO: 36) | ATTTGGATTGTTATGTTT (SEQ ID NO: 618) |
| 171 | ORC4L (SEQ ID NO: 36) | ATAAGACGGAGTTCGT (SEQ ID NO: 619) |
| 172 | ORC4L (SEQ ID NO: 36) | AAGATGGAGTTTGTTTG (SEQ ID NO: 620) |
| 173 | ORC4L (SEQ ID NO: 36) | TTGCGTTATCGACGTT (SEQ ID NO: 621) |
| 174 | ORC4L (SEQ ID NO: 36) | ATTTTGTGTTATTGATGT (SEQ ID NO: 622) |
| 175 | ORC4L (SEQ ID NO: 36) | GAGTAACGCGTGTGAT (SEQ ID NO: 623) |
| 176 | ORC4L (SEQ ID NO: 36) | TATGAGTAATGTGTGTG (SEQ ID NO: 624) |
| 177 | ABHD9 (SEQ ID NO: 37) | TATTTGGGCGCGATAG (SEQ ID NO: 625) |
| 178 | ABHD9 (SEQ ID NO: 37) | ATTTGGGTGTGATAGG (SEQ ID NO: 626) |
| 179 | ABHD9 (SEQ ID NO: 37) | GTGGGACGCGTTGAAG (SEQ ID NO: 627) |
| 180 | ABHD9 (SEQ ID NO: 37) | TGTGGGATGTGTTGAA (SEQ ID NO: 628) |
| 181 | ABHD9 (SEQ ID NO: 37) | GGCGGTTTCGATAGAA (SEQ ID NO: 629) |
| 182 | ABHD9 (SEQ ID NO: 37) | GGGTGGTTTTGATAGA (SEQ ID NO: 630) |
| 183 | CCND2 (SEQ ID NO: 1) | ATAAGTCGTTCGAGGT (SEQ ID NO: 631) |
| 184 | CCND2 (SEQ ID NO: 1) | AAGTTGTTTGAGGTGT (SEQ ID NO: 632) |
| 185 | CCND2 (SEQ ID NO: 1) | TAGCGGTTACGTAGGA (SEQ ID NO: 633) |
| 186 | CCND2 (SEQ ID NO: 1) | AGTGGTTATGTAGGAAA (SEQ ID NO: 634) |
| 187 | CCND2 (SEQ ID NO: 1) | TACGTGTTTTAACGTAT (SEQ ID NO: 635) |
| 188 | CCND2 (SEQ ID NO: 1) | TGATATGTGTTTTAATGTA (SEQ ID NO: 636) |

(continued)

| No: | Gene | Oligo: |
|---|---|---|
| 189 | CCND2 (SEQ ID NO: 1) | TTAGGGTCGTCGTAGGT (SEQ ID NO: 637) |
| 190 | CCND2 (SEQ ID NO: 1) | TTAGGGTTGTTGTAGGT (SEQ ID NO: 638) |
| 191 | CCND2 (SEQ ID NO: 1) | TTAGTACGGTCGGTTT (SEQ ID NO: 639) |
| 192 | CCND2 (SEQ ID NO: 1) | GTTAGTATGGTTGGTTT (SEQ ID NO: 640) |
| 193 | CDKN2A (SEQ ID NO: 2) | TAGGTATCGCGTACGT (SEQ ID NO: 641) |
| 194 | CDKN2A (SEQ ID NO: 2) | TTAGGTATTGTGTATGTT (SEQ ID NO: 642) |
| 195 | CDKN2A (SEQ ID NO: 2) | TTCGCGTCGTGGAGTA (SEQ ID NO: 643) |
| 196 | CDKN2A (SEQ ID NO: 2) | TTTTGTGTTGTGGAGTA (SEQ ID NO: 644) |
| 197 | CDKN2A (SEQ ID NO: 2) | TAGTCGCGCGTAGGTA (SEQ ID NO: 645) |
| 198 | CDKN2A (SEQ ID NO: 2) | TAGTTGTGTGTAGGTAT (SEQ ID NO: 646) |
| 199 | CDKN2A (SEQ ID NO: 2) | TAGGTATCGTGCGATA (SEQ ID NO: 647) |
| 200 | CDKN2A (SEQ ID NO: 2) | GTAGGTATTGTGTGATAT (SEQ ID NO: 648) |
| 201 | CD44 (SEQ ID NO: 3) | TAGGTTCGGTTCGTTAT (SEQ ID NO: 649) |
| 202 | CD44 (SEQ ID NO: 3) | TAGGTTTGGTTTGTTATT (SEQ ID NO: 650) |
| 203 | CD44 (SEQ ID NO: 3) | GTTTCGCGTTTAGGGA (SEQ ID NO: 651) |
| 204 | CD44 (SEQ ID NO: 3) | GTTTTGTGTTTAGGGAT (SEQ ID NO: 652) |
| 205 | CD44 (SEQ ID NO: 3) | GTTCGTTTCGGATATTA (SEQ ID NO: 653) |
| 206 | CD44 (SEQ ID NO: 3) | TTTGTTTTGGATATTATGG (SEQ ID NO: 654) |
| 207 | CD44 (SEQ ID NO: 3) | TTTGGCGTAGATCGGT (SEQ ID NO: 655) |
| 208 | CD44 (SEQ ID NO: 3) | TTTGGTGTAGATTGGT (SEQ ID NO: 656) |
| 209 | EDNRB1 (SEQ ID NO: 4) | TTCGTTTTTCGGGAAG (SEQ ID NO: 657) |
| 210 | EDNRB1 (SEQ ID NO: 4) | TTTGTTTTTTGGGAAGG (SEQ ID NO: 658) |

(continued)

| No: | Gene | Oligo: |
|---|---|---|
| 211 | EDNRB1 (SEQ ID NO: 4) | TAGAGTCGGATTCGTT (SEQ ID NO: 659) |
| 212 | EDNRB1 (SEQ ID NO: 4) | AGTTGGATTTGTTTGTA (SEQ ID NO: 660) |
| 213 | EDNRB1 (SEQ ID NO: 4) | GTATTTTCGTAGCGTT (SEQ ID NO: 661) |
| 214 | EDNRB1 (SEQ ID NO: 4) | TGGTATTTTTGTAGTGTT (SEQ ID NO: 662) |
| 215 | EDNRB1 (SEQ ID NO: 4) | ATTTCGAGTAAACGGT (SEQ ID NO: 663) |
| 216 | EDNRB1 (SEQ ID NO: 4) | TTTGAGTAAATGGTGGA (SEQ ID NO: 664) |
| 217 | ELK1 (SEQ ID NO: 5) | TGTCGTACGTTATGTT (SEQ ID NO: 665) |
| 218 | ELK1 (SEQ ID NO: 5) | GGTGTTGTATGTTATGT (SEQ ID NO: 666) |
| 219 | ELK1 (SEQ ID NO: 5) | TGGGCGTAGTAGTCGG (SEQ ID NO: 667) |
| 220 | ELK1 (SEQ ID NO: 5) | ATGGGTGTAGTAGTTGG (SEQ ID NO: 668) |
| 221 | ELK1 (SEQ ID NO: 5) | ATTGGGTTTCGCGTAGG (SEQ ID NO: 669) |
| 222 | ELK1 (SEQ ID NO: 5) | ATTGGGTTTTGTGTAGG (SEQ ID NO: 670) |
| 223 | ELK1 (SEQ ID NO: 5) | TTGATTGGCGGACGAG (SEQ ID NO: 671) |
| 224 | ELK1 (SEQ ID NO: 5) | TTGATTGGTGGATGAG (SEQ ID NO: 672) |
| 225 | FOS (SEQ ID NO: 6) | TACGGATTTGGTCGTTT (SEQ ID NO: 673) |
| 226 | FOS (SEQ ID NO: 6) | TATGGATTTGGTTGTTT (SEQ ID NO: 674) |
| 227 | FOS (SEQ ID NO: 6) | TTCGATTAGTTCGGAT (SEQ ID NO: 675) |
| 228 | FOS (SEQ ID NO: 6) | TATTTTGATTAGTTTGGAT (SEQ ID NO: 676) |
| 229 | FOS (SEQ ID NO: 6) | TTTCGTGGTTTTATCGTA (SEQ ID NO: 677) |
| 230 | FOS (SEQ ID NO: 6) | TTTTGTGGTTTTATTGTA (SEQ ID NO: 678) |
| 231 | FOS (SEQ ID NO: 6) | GTCGAGCGTAGAGTAT (SEQ ID NO: 679) |
| 232 | FOS (SEQ ID NO: 6) | TAGGAGGTTGAGTGTA (SEQ ID NO: 680) |

(continued)

| No: | Gene | Oligo: |
|---|---|---|
| 233 | GSTP1 (SEQ ID NO: 7) | GTCGGTCGTAGAGGGG (SEQ ID NO: 681) |
| 234 | GSTP1 (SEQ ID NO: 7) | GTTGGTTGTAGAGGGG (SEQ ID NO: 682) |
| 235 | GSTP1 (SEQ ID NO: 7) | TTCGCGGTTTTCGAGT (SEQ ID NO: 683) |
| 236 | GSTP1 (SEQ ID NO: 7) | TTTGTGGTTTTTGAGTT (SEQ ID NO: 684) |
| 237 | GSTP1 (SEQ ID NO: 7) | GTCGCGCGTATTTATT (SEQ ID NO: 685) |
| 238 | GSTP1 (SEQ ID NO: 7) | GGGTTGTGTGTATTTAT (SEQ ID NO: 686) |
| 239 | GSTP1 (SEQ ID NO: 7) | GAGTCGTCGCGTAGTT (SEQ ID NO: 687) |
| 240 | GSTP1 (SEQ ID NO: 7) | GGAGTTGTTGTGTAGTT (SEQ ID NO: 688) |
| 241 | CD37 (SEQ ID NO: 38) | ATCGAGAGCGTTATGA (SEQ ID NO: 689) |
| 242 | CD37 (SEQ ID NO: 38) | AGGAATATTGAGAGTGT (SEQ ID NO: 690) |
| 243 | CD37 (SEQ ID NO: 38) | TATCGAGCGAGTCGGT (SEQ ID NO: 691) |
| 244 | CD37 (SEQ ID NO: 38) | TATTGAGTGAGTTGGTT (SEQ ID NO: 692) |
| 245 | CD37 (SEQ ID NO: 38) | TTAGCGTACGTGACGG (SEQ ID NO: 693) |
| 246 | CD37 (SEQ ID NO: 38) | AGTGTATGTGATGGGG (SEQ ID NO: 694) |
| 247 | CD37 (SEQ ID NO: 38) | GGGTACGTAGTTACGT (SEQ ID NO: 695) |
| 248 | CD37 (SEQ ID NO: 38) | TATGTAGTTATGTGGGT (SEQ ID NO: 696) |
| 249 | GRN (SEQ ID NO: 39) | TAGCGCGATGATTCGT (SEQ ID NO: 697) |
| 250 | GRN ' (SEQ ID NO: 39) | AGTGTGATGATTTGTTT (SEQ ID NO: 698) |
| 251 | GRN (SEQ ID NO: 39) | TAATCGGGTAGCGTTT (SEQ ID NO: 699) |
| 252 | GRN (SEQ ID NO: 39) | TAGTAATTGGGTAGTGT (SEQ ID NO: 700) |
| 253 | GRN (SEQ ID NO: 39) | TTCGATTTCGCGGTTT (SEQ ID NO: 701) |
| 254 | GRN (SEQ ID NO: 39) | GTTTGATTTTGTGGTTT (SEQ ID NO: 702) |

(continued)

| No: | Gene | Oligo: |
|---|---|---|
| 255 | GRN (SEQ ID NO: 39) | TTTAACGGGGCGTTAT (SEQ ID NO: 703) |
| 256 | GRN (SEQ ID NO: 39) | AATGGGGTGTTATTGT (SEQ ID NO: 704) |
| 257 | EPAS1 (SEQ ID NO: 40) | TTCGGCGTTATTCGAG (SEQ ID NO: 705) |
| 258 | EPAS1 (SEQ ID NO: 40) | GGTTTGGTGTTATTTGA (SEQ ID NO: 706) |
| 259 | EPAS1 (SEQ ID NO: 40) | TATTCGTGCGGTTTTA (SEQ ID NO: 707) |
| 260 | EPAS1 (SEQ ID NO: 40) | ATTTGTGTGGTTTTAGT (SEQ ID NO: 708) |
| 261 | EPAS1 (SEQ ID NO: 40) | GAATTTAACGCGCGGT (SEQ ID NO: 709) |
| 262 | EPAS1 (SEQ ID NO: 40) | GGAATTTAATGTGTGGT (SEQ ID NO: 710) |
| 263 | EPAS1 (SEQ ID NO: 40) | TTCGCGAGTTTTTCGG (SEQ ID NO: 711) |
| 264 | EPAS1 (SEQ ID NO: 40) | TTTGTGAGTTTTTTGGTA (SEQ ID NO: 712) |
| 265 | NOTCH1 (SEQ ID NO: 41) | TTTACGGGCGGGAGTT (SEQ ID NO: 713) |
| 266 | NOTCH1 (SEQ ID NO: 41) | TTTTATGGGTGGGAGT (SEQ ID NO: 714) |
| 267 | NOTCH1 (SEQ ID NO: 41) | TAGCGGGCGAGTAGTT (SEQ ID NO: 715) |
| 268 | NOTCH1 (SEQ ID NO: 41) | TAGTGGGTGAGTAGTT (SEQ ID NO: 716) |
| 269 | NOTCH1 (SEQ ID NO: 41) | AGGCGGTTTCGATTTT (SEQ ID NO: 717) |
| 270 | NOTCH1 (SEQ ID NO: 41) | TGGAGGTGGTTTTGAT (SEQ ID NO: 718) |
| 271 | NOTCH1 (SEQ ID NO: 41) | ATTTCGGCGGTTGGAT (SEQ ID NO: 719) |
| 272 | NOTCH1 (SEQ ID NO: 41) | AGGTAATTTTGGTGGTT (SEQ ID NO: 720) |
| 273 | MLLT3 (SEQ ID NO: 42) | TAATTCGGTTAGATTTTCGG (SEQ ID NO: 721) |
| 274 | MLLT3 (SEQ ID NO: 42) | TAATTTGGTTAGATTTTTGG (SEQ ID NO: 722) |
| 275 | MLLT3 (SEQ ID NO: 42) | TTTAGAGTCGCGTTTT (SEQ ID NO: 723) |
| 276 | MLLT3 (SEQ ID NO: 42) | TAGAGTTGTGTTTTTGT (SEQ ID NO: 724) |

(continued)

| No: | Gene | Oligo: |
|---|---|---|
| 277 | MLLT3 (SEQ ID NO: 42) | TAGAATAGCGCGGTTA (SEQ ID NO: 725) |
| 278 | MLLT3 (SEQ ID NO: 42) | GATAGAATAGTGTGGTTA (SEQ ID NO: 726) |
| 279 | SOLH (SEQ ID NO: 43) | TAGGGGACGTGTACGA (SEQ ID NO: 727) |
| 280 | SOLH (SEQ ID NO: 43) | TAGGGGATGTGTATGA (SEQ ID NO: 728) |
| 281 | SOLH (SEQ ID NO: 43) | GACGGAACGTATGTTT (SEQ ID NO: 729) |
| 282 | SOLH (SEQ ID NO: 43) | TGGGGATGGAATGTAT (SEQ ID NO: 730) |
| 283 | SOLH (SEQ ID NO: 43) | ATTCGTGGGGACGGAA (SEQ ID NO: 731) |
| 284 | SOLH (SEQ ID NO: 43) | ATTTGTGGGGATGGAA (SEQ ID NO: 732) |
| 285 | SEQ ID NO: 44 (SEQ ID NO: 44) | TAGGGTTCGTTCGTATT (SEQ ID NO: 733) |
| 286 | SEQ ID NO: 44 (SEQ ID NO: 44) | TTTAGGGTTTGTTTGTAT (SEQ ID NO: 734) |
| 287 | SEQ ID NO: 44 (SEQ ID NO: 44) | TTACGATTTTCGGGGT (SEQ ID NO: 735) |
| 288 | SEQ ID NO: 44 (SEQ ID NO: 44) | TTTATGATTTTTGGGGT (SEQ ID NO: 736) |
| 289 | SEQ ID NO: 44 (SEQ ID NO: 44) | AAGTAGACGTCGGAGA (SEQ ID NO: 737) |
| 290 | SEQ ID NO: 44 (SEQ ID NO: 44) | TAGATGTTGGAGAGGG (SEQ ID NO: 738) |
| 291 | SEQ ID NO: 44 (SEQ ID NO: 44) | TGTCGTTACGTTTAGG (SEQ ID NO: 739) |
| 292 | SEQ ID NO: 44 (SEQ ID NO: 44) | GTTGTTATGTTTAGGGT (SEQ ID NO: 740) |
| 293 | Q8N365 (SEQ ID NO: 45) | AGATTCGGAGAGACGG (SEQ ID NO: 741) |
| 294 | Q8N365 (SEQ ID NO: 45) | TAGATTTGGAGAGATGG (SEQ ID NO: 742) |
| 295 | Q8N365 (SEQ ID NO: 45) | AGATCGGCGTAGGGAT (SEQ ID NO: 743) |
| 296 | Q8N365 (SEQ ID NO: 45) | AGATTGGTGTAGGGAT (SEQ ID NO: 744) |
| 297 | Q8N365 (SEQ ID NO: 45) | TACGTGTTATCGGCGA (SEQ ID NO: 745) |
| 298 | Q8N365 (SEQ ID NO: 45) | TATGTGTTATTGGTGATA (SEQ ID NO: 746) |

(continued)

| No: | Gene | Oligo: |
|---|---|---|
| 299 | Q8N365 (SEQ ID NO: 45) | TACGTGTCGGGTCGTA (SEQ ID NO: 747) |
| 300 | Q8N365 (SEQ ID NO: 45) | GTATGTGTTGGGTTGTA (SEQ ID NO: 748) |
| 301 | Q9NWV0 (SEQ ID NO: 46) | GTCGCGTGGATACGTG (SEQ ID NO: 749) |
| 302 | Q9NWV0 (SEQ ID NO: 46) | GGTTGTGTGGATATGT (SEQ ID NO: 750) |
| 303 | Q9NWV0 (SEQ ID NO: 46) | TTGGCGATTTTTACGA (SEQ ID NO: 751) |
| 304 | Q9NWV0 (SEQ ID NO: 46) | TTGGTGATTTTTATGAGA (SEQ ID NO: 752) |
| 305 | Q9NWV0 (SEQ ID NO: 46) | TGTCGTAGCGTTATGT (SEQ ID NO: 753) |
| 306 | Q9NWV0 (SEQ ID NO: 46) | AGGTGTTGTAGTGTTAT (SEQ ID NO: 754) |
| 307 | SEQ ID NO: 47 (SEQ ID NO: 47) | GTAACGCGTTTGGTTT (SEQ ID NO: 755) |
| 308 | SEQ ID NO: 47 (SEQ ID NO: 47) | TGGTAATGTGTTTGGT (SEQ ID NO: 756) |
| 309 | SEQ ID NO: 47 (SEQ ID NO: 47) | TTCGAGCGTTTTACGT (SEQ ID NO: 757) |
| 310 | SEQ ID NO: 47 (SEQ ID NO: 47) | TTTTGAGTGTTTTATGTT (SEQ ID NO: 758) |
| 311 | SEQ ID NO: 47 (SEQ ID NO: 47) | TTACGTGGGAGCGTTT (SEQ ID NO: 759) |
| 312 | SEQ ID NO: 47 (SEQ ID NO: 47) | TTATGTGGGAGTGTTT (SEQ ID NO: 760) |
| 313 | SEQ ID NO: 47 (SEQ ID NO: 47) | TAGTTTTACGCGGGTA (SEQ ID NO: 761) |
| 314 | SEQ ID NO: 47 (SEQ ID NO: 47) | AGTTTTATGTGGGTATG (SEQ ID NO: 762) |
| 315 | H2AFY2 (SEQ ID NO: 48) | ATGAAAGGCGCGAGAA (SEQ ID NO: 763) |
| 316 | H2AFY2 (SEQ ID NO: 48) | ATGAAAGGTGTGAGAA (SEQ ID NO: 764) |
| 317 | H2AFY2 (SEQ ID NO: 48) | GAATCGTGGTTTCGTT (SEQ ID NO: 765) |
| 318 | H2AFY2 (SEQ ID NO: 48) | GGAATTGTGGTTTTGT (SEQ ID NO: 766) |
| 319 | H2AFY2 (SEQ ID NO: 48) | TAGTTTATCGCGGTAA (SEQ ID NO: 767) |
| 320 | H2AFY2 (SEQ ID NO: 48) | TTTATTGTGGTAAATGGT (SEQ ID NO: 768) |

(continued)

| No: | Gene | Oligo: |
|---|---|---|
| 321 | RHOC (SEQ ID NO: 49) | TGCGGTTCGAAGATTA (SEQ ID NO: 769) |
| 322 | RHOC (SEQ ID NO: 49) | GGTGTGGTTTGAAGAT (SEQ ID NO: 770) |
| 323 | RHOC (SEQ ID NO: 49) | GAACGCGTTTTAGCGT (SEQ ID NO: 771) |
| 324 | RHOC (SEQ ID NO: 49) | GGAATGTGTTTTAGTGT (SEQ ID NO: 772) |
| 325 | RHOC (SEQ ID NO: 49) | TTTTAGCGTCGGGGAT (SEQ ID NO: 773) |
| 326 | RHOC (SEQ ID NO: 49) | TTTTAGTGTTGGGGAT (SEQ ID NO: 774) |
| 327 | NR2E1 (SEQ ID NO: 50) | TAGTCGTATTAGCGGT (SEQ ID NO: 775) |
| 328 | NR2E1 (SEQ ID NO: 50) | TAGTTGTATTAGTGGTTT (SEQ ID NO: 776) |
| 329 | NR2E1 (SEQ ID NO: 50) | TGCGTTTTATTCGCGG (SEQ ID NO: 777) |
| 330 | NR2E1 (SEQ ID NO: 50) | TGTGTTTTATTTGTGGT (SEQ ID NO: 778) |
| 331 | NR2E1 (SEQ ID NO: 50) | TTTCGAAGTTTCGCGG (SEQ ID NO: 779) |
| 332 | NR2E1 (SEQ ID NO: 50) | TTTGAAGTTTTGTGGG (SEQ ID NO: 780) |
| 333 | NR2E1 (SEQ ID NO: 50) | TAGCGCGAATCGTTTA (SEQ ID NO: 781) |
| 334 | NR2E1 (SEQ ID NO: 50) | AGTGTGAATTGTTTAGT (SEQ ID NO: 782) |
| 335 | KBTBD6 (SEQ ID NO: 51) | AGACGTTTCGCGTTTT (SEQ ID NO: 783) |
| 336 | KBTBD6 (SEQ ID NO: 51) | GAAGATGTTTTGTGTTT (SEQ ID NO: 784) |
| 337 | KBTBD6 (SEQ ID NO: 51) | TTTCGGGAAGACGTTT (SEQ ID NO: 785) |
| 338 | KBTBD6 (SEQ ID NO: 51) | AGTTTTGGGAAGATGT (SEQ ID NO: 786) |
| 339 | KBTBD6 (SEQ ID NO: 51) | TATTAGCGTTCGTCGT (SEQ ID NO: 787) |
| 340 | KBTBD6 (SEQ ID NO: 51) | GTTATTAGTGTTTGTTGT (SEQ ID NO: 788) |
| 341 | TRPM4 (SEQ ID NO: 52) | TAGGTGAGCGTCGGAT (SEQ ID NO: 789) |
| 342 | TRPM4 (SEQ ID NO: 52) | TAGGTGAGTGTTGGAT (SEQ ID NO: 790) |

(continued)

| No: | Gene | Oligo: |
|---|---|---|
| 343 | TRPM4<br>(SEQ ID NO: 52) | AGTAGAGTCGGCGGAG<br>(SEQ ID NO: 791) |
| 344 | TRPM4<br>(SEQ ID NO: 52) | AGTAGAGTTGGTGGAG<br>(SEQ ID NO: 792) |
| 345 | TCEB3BP1<br>(SEQ ID NO: 53) | GACGTTAGCGAGTATT<br>(SEQ ID NO: 793) |
| 346 | TCEB3BP1<br>(SEQ ID NO: 53) | AGGGATGTTAGTGAGT<br>(SEQ ID NO: 794) |
| 347 | TCEB3BP1<br>(SEQ ID NO: 53) | TGTCGGGTCGAGTAGT<br>(SEQ ID NO: 795) |
| 348 | TCEB3BP1<br>(SEQ ID NO: 53) | TGTTGGGTTGAGTAGT<br>(SEQ ID NO: 796) |
| 349 | Q8NCX8<br>(SEQ ID NO: 54) | TTACGGCGGGTTTTTA<br>(SEQ ID NO: 797) |
| 350 | Q8NCX8<br>(SEQ ID NO: 54) | TTATGGTGGGTTTTTATT<br>(SEQ ID NO: 798) |
| 351 | Q8NCX8<br>(SEQ ID NO: 54) | TTCGGTTTATACGATTT<br>(SEQ ID NO: 799) |
| 352 | Q8NCX8<br>(SEQ ID NO: 54) | ATTTGGTTTATATGATTTTT<br>(SEQ ID NO: 800) |
| 353 | Q8NCX8<br>(SEQ ID NO: 54) | TTACGTCGATTATCGG<br>(SEQ ID NO: 801) |
| 354 | Q8NCX8<br>(SEQ ID NO: 54) | TATGTTGATTATTGGGGT<br>(SEQ ID NO: 802) |
| 355 | SEQ ID NO: 55<br>(SEQ ID NO: 55) | AATTTACGCGGGTGTT<br>(SEQ ID NO: 803) |
| 356 | SEQ ID NO: 55<br>(SEQ ID NO: 55) | GGAATTTATGTGGGTG<br>(SEQ ID NO: 804) |
| 357 | SEQ ID NO: 55<br>(SEQ ID NO: 55) | TAGTTCGTTTCGGTTA<br>(SEQ ID NO: 805) |
| 358 | SEQ ID NO: 55<br>(SEQ ID NO: 55) | AGTTTGTTTTGGTTAGT<br>(SEQ ID NO: 806) |
| 359 | SEQ ID NO: 55<br>(SEQ ID NO: 55) | GTCGTGTACGAATTCGT<br>(SEQ ID NO: 807) |
| 360 | SEQ ID NO: 55<br>(SEQ ID NO: 55) | GTTGTGTATGAATTTGTT<br>(SEQ ID NO: 808) |
| 361 | SEQ 10 NO: 55<br>(SEQ ID NO: 55) | AGCGTTTAAGTCGCGG<br>(SEQ ID NO: 809) |
| 362 | SEQ ID NO: 55<br>(SEQ ID NO: 55) | TAGTGTTTAAGTTGTGG<br>(SEQ ID NO: 810) |
| 363 | SNAPC2<br>(SEQ ID NO: 56) | TTACGATTTCGGTGTT<br>(SEQ ID NO: 811) |
| 364 | SNAPC2<br>(SEQ ID NO: 56) | TAGAGTTATGATTTTGGT<br>(SEQ ID NO: 812) |

(continued)

| No: | Gene | Oligo: |
|---|---|---|
| 365 | SNAPC2 (SEQ ID NO: 56) | AGGCGTGCGTCGATAT (SEQ ID NO: 813) |
| 366 | SNAPC2 (SEQ ID NO: 56) | AGGTGTGTGTTGATATT (SEQ ID NO: 814) |
| 367 | SNAPC2 (SEQ ID NO: 56) | GTAGCGTCGAGGGTTT (SEQ ID NO: 815) |
| 368 | SNAPC2 (SEQ ID NO: 56) | GTAGTGTTGAGGGTTT (SEQ ID NO: 816) |
| 369 | SNAPC2 (SEQ ID NO: 56) | ATACGTTGACGTAGTT (SEQ ID NO: 817) |
| 370 | SNAPC2 (SEQ ID NO: 56) | TTGTATATGTTGATGTAGT (SEQ ID NO: 818) |
| 371 | PTPRN2 (SEQ ID NO: 57) | GACGTAGTTCGTACGT (SEQ ID NO: 819) |
| 372 | PTPRN2 (SEQ ID NO: 57) | GGATGTAGTTTGTATGT (SEQ ID NO: 820) |
| 373 | PTPRN2 (SEQ ID NO: 57) | TTAGTCGTTTCGAGAT (SEQ ID NO: 821) |
| 374 | PTPRN2 (SEQ ID NO: 57) | GTTTTAGTTGTTTTGAGA (SEQ ID NO: 822) |
| 375 | PTPRN2 (SEQ ID NO: 57) | TTACGCGTATCGGGAT (SEQ ID NO: 823) |
| 376 | PTPRN2 (SEQ ID NO: 57) | TATGTGTATTGGGATTTA (SEQ ID NO: 824) |
| 377 | PTPRN2 (SEQ ID NO: 57) | TTCGCGTTTCGTAAGA (SEQ ID NO: 825) |
| 378 | PTPRN2 (SEQ ID NO: 57) | TTTGTGTTTTGTAAGATAT (SEQ ID NO: 826) |
| 379 | WDFY3 (SEQ ID NO: 58) | TATTGAGTCGGTCGTA (SEQ ID NO: 827) |
| 380 | WDFY3 (SEQ ID NO: 58) | ATTGAGTTGGTTGTAGA (SEQ ID NO: 828) |
| 381 | WDFY3 (SEQ ID NO: 58) | TAGATAGCGTCGTTGG (SEQ ID NO: 829) |
| 382 | WDFY3 (SEQ ID NO: 58) | TAGATAGTGTTGTTGGA (SEQ ID NO: 830) |
| 383 | ZNF566 (SEQ ID NO: 59) | ATAAAATACGACGTTGA (SEQ ID NO: 831) |
| 384 | ZNF566 (SEQ ID NO: 59) | ATAAAATATGATGTTGAATT (SEQ ID NO: 832) |
| 385 | ZNF566 (SEQ ID NO: 59) | AGCGTTTTTTACGAAT (SEQ ID NO: 833) |
| 386 | ZNF566 (SEQ ID NO: 59) | TAGTGTTTTTTATGAATGA (SEQ ID NO: 834) |

(continued)

| No: | Gene | Oligo: |
|---|---|---|
| 387 | Q9NP73 (SEQ ID NO: 60) | TATTACGGATTTTCGTT (SEQ ID NO: 835) |
| 388 | Q9NP73 (SEQ ID NO: 60) | GTTATTATGGATTTTTGTT (SEQ ID NO: 836) |
| 389 | Q9NP73 (SEQ ID. NO: 60) | TGCGTTATATTCGGTT (SEQ ID NO: 837) |
| 390 | Q9NP73 (SEQ ID NO: 60) | AGTTGTGTTATATTTGGT (SEQ ID NO: 838) |
| 391 | Q9NP73 (SEQ ID NO: 60) | AATTCGCGTTATGAAG (SEQ ID NO: 839) |
| 392 | Q9NP73 (SEQ ID NO: 60) | TTGAGGAATTTGTGTTAT (SEQ ID NO: 840) |
| 393 | Q9NP73 (SEQ ID NO: 60) | GTCGGCGTTCGATAGT (SEQ ID NO: 841) |
| 394 | Q9NP73 (SEQ ID NO: 60) | TGTTGGTGTTTGATAGT (SEQ ID NO: 842) |
| 395 | SEQ ID NO: 61 (SEQ ID NO: 61) | AGAATTCGTAAACGGG (SEQ ID NO: 843) |
| 396 | SEQ ID NO: 61 (SEQ ID NO: 61) | ATTTGTAAATGGGGGT (SEQ ID NO: 844) |
| 397 | SEQ ID NO: 61 (SEQ ID NO: 61) | TTCGGTTATCGACGGT (SEQ ID NO: 845) |
| 398 | SEQ ID NO: 61 (SEQ ID NO: 61) | TTTGGTTATTGATGGTT (SEQ ID NO: 846) |
| 399 | Q86SP6 (SEQ ID NO: 62) | ATCGTGAGCGTAGCGT (SEQ ID NO: 847) |
| 400 | Q86SP6 (SEQ ID NO: 62) | ATTGTGAGTGTAGTGTA (SEQ ID NO: 848) |
| 401 | Q86SP6 (SEQ ID NO: 62) | TAGGCGTGAGAATCGG (SEQ ID NO: 849) |
| 402 | Q86SP6 (SEQ ID NO: 62) | TAGGTGTGAGAATTGG (SEQ ID NO: 850) |
| 403 | Q86SP6 (SEQ ID NO: 62) | ATCGTGTTCGGATCGG (SEQ ID NO: 851) |
| 404 | Q86SP6 (SEQ ID NO: 62) | TATTGTGTTTGGATTGG (SEQ ID NO: 852) |
| 405 | Q86SP6 (SEQ ID NO: 62) | AGGTTCGAGTTTGCGG (SEQ ID NO: 853) |
| 406 | Q86SP6 (SEQ ID NO: 62) | TAGGTTTGAGTTTGTGG (SEQ ID NO: 854) |
| 407 | Q86SP6 (SEQ ID NO: 62) | TATAAAGCGCGAGTGT (SEQ ID NO: 855) |
| 408 | Q86SP6 (SEQ ID NO: 62) | TATAAAGTGTGAGTGTG (SEQ ID NO: 856) |

(continued)

| No: | Gene | Oligo: |
|---|---|---|
| 409 | RARB (SEQ ID NO: 8) | ATGTCGAGAACGCGAG (SEQ ID NO: 857) |
| 410 | RARB (SEQ ID NO: 8) | GGATGTTGAGAATGTGA (SEQ ID NO: 858) |
| 411 | RARB (SEQ ID NO: 8) | AGCGATTCGAGTAGGG (SEQ ID NO: 859) |
| 412 | RARB (SEQ ID NO: 8) | AGTGATTTGAGTAGGG (SEQ ID NO: 860) |
| 413 | RARB (SEQ ID NO: 8) | TATCGTCGGGGTAGGA (SEQ ID NO: 861) |
| 414 | RARB (SEQ ID NO: 8) | TATTGTTGGGGTAGGA (SEQ ID NO: 862) |
| 415 | RARB (SEQ ID NO: 8) | AACGTATTCGGAAGGT (SEQ ID NO: 863) |
| 416 | RARB (SEQ ID NO: 8) | GAATGTATTTGGAAGGT (SEQ ID NO: 864) |
| 417 | PTGS2 (SEQ ID NO: 9) | AGCGTTTTCGAGAGTT (SEQ ID NO: 865) |
| 418 | PTGS2 (SEQ ID NO: 9) | GAGTGTTTTTGAGAGTT (SEQ ID NO: 866) |
| 419 | PTGS2 (SEQ ID NO: 9) | TTACGTCGGGATAGAT (SEQ ID NO: 867) |
| 420 | PTGS2 (SEQ ID NO: 9) | GGAAGTTATGTTGGGA (SEQ ID NO: 868) |
| 421 | PTGS2 (SEQ ID NO: 9) | TATCGTTTTAGGCGTA (SEQ ID NO: 869) |
| 422 | PTGS2 (SEQ ID NO: 9) | TTTATTGTTTTAGGTGTAT (SEQ ID NO: 870) |
| 423 | RASSF1 (SEQ ID NO: 10) | TACGGGTATTTTCGCGT (SEQ ID NO: 871) |
| 424 | RASSF1 (SEQ ID NO: 10) | ATATGGGTATTTTTGTGT (SEQ ID NO: 872) |
| 425 | RASSF1 (SEQ ID NO: 10) | AGAGCGCGTTTAGTTT (SEQ ID NO: 873) |
| 426 | RASSF1 (SEQ ID NO: 10) | GAGAGTGTGTTTAGTTT (SEQ ID NO: 874) |
| 427 | ESR2 (SEQ ID NO: 11) | TAGGAACGTTCGACGT (SEQ ID NO: 875) |
| 428 | ESR2 (SEQ ID NO: 11) | TTTAGGAATGTTTGATGT (SEQ 10 NO: 876) |
| 429 | ESR2 (SEQ ID NO: 11) | ATGGCGTTTTTCGTAG (SEQ ID NO: 877) |
| 430 | ESR2 (SEQ ID NO: 11) | TAGATGGTGTTTTTTGT (SEQ ID NO: 878) |

(continued)

| No: | Gene | Oligo: |
|---|---|---|
| 431 | ESR2 (SEQ ID NO: 11) | ATAAGCGATTTAACGAT (SEQ ID NO: 879) |
| 432 | ESR2 (SEQ ID NO: 11) | AGTGATTTAATGATAAGTT (SEQ ID NO: 880) |
| 433 | ESR2 (SEQ ID NO: 11) | ATTTCGAGGATTACGT (SEQ ID NO: 881) |
| 434 | ESR2 (SEQ ID NO: 11) | ATATTTTGAGGATTATGTT (SEQ ID NO: 882) |
| 435 | DRG1 (SEQ ID NO: 12) | TAGTCGTTAAAACGTAG (SEQ ID NO: 883) |
| 436 | DRG1 (SEQ ID NO: 12) | AGTTGTTAAAATGTAGATT (SEQ ID NO: 884) |
| 437 | DRG1 (SEQ ID NO: 12) | ATCGTATTGGTTCGCGG (SEQ ID NO: 885) |
| 438 | DRG1 (SEQ ID NO: 12) | ATTGTATTGGTTTGTGG (SEQ ID NO: 886) |
| 439 | DRG1 (SEQ ID NO: 12) | TTTACGTTTTGCGATT (SEQ ID NO: 887) |
| 440 | DRG1 (SEQ ID NO: 12) | AGTTTTATGTTTTGTGAT (SEQ ID NO: 888) |
| 441 | DRG1 (SEQ ID NO: 12) | ATTTTTACGCGGAATT (SEQ ID NO: 889) |
| 442 | DRG1 (SEQ ID NO: 12) | GTGATTTTTATGTGGAAT (SEQ ID NO: 890) |
| 443 | DRG1 (SEQ ID NO: 12) | ATTCGAAGTATCGCGT (SEQ ID NO: 891) |
| 444 | DRG1 (SEQ ID NO: 12) | ATTTGAAGTATTGTGTTT (SEQ ID NO: 892) |
| 445 | DRG1 (SEQ ID NO: 12) | ATTTCGAATTTCGAGTA (SEQ ID NO: 893) |
| 446 | DRG1 (SEQ ID NO: 12) | TTTGAATTTTGAGTAGAG (SEQ ID NO: 894) |
| 447 | DOCK10 (SEQ ID NO: 16) | TATATTGTCGGGGAGA (SEQ ID NO: 895) |
| 448 | DOCK10 (SEQ ID NO: 16) | ATATTGTTGGGGAGAG (SEQ ID NO: 896) |
| 449 | BTG4 (SEQ ID NO: 17) | GTGTTGCGTAGAGATA (SEQ ID NO: 897) |
| 450 | BTG4 (SEQ ID NO: 17) | GGTGTTGTGTAGAGAT (SEQ ID NO: 898) |
| 451 | BTG4 (SEQ ID NO: 17) | TTGGTTTTTCGGAATAA (SEQ ID NO: 899) |
| 452 | BTG4 (SEQ ID NO: 17) | GGTTTTTTGGAATAAGAT (SEQ ID NO: 900) |

(continued)

| No: | Gene | Oligo: |
|---|---|---|
| 453 | GPR7 (SEQ ID NO: 19) | ATTGAGGGCGTATAGA (SEQ ID NO: 901) |
| 454 | GPR7 (SEQ ID NO: 19) | TGAGGGTGTATAGATTT (SEQ ID NO: 902) |
| 455 | GPR7 (SEQ ID NO: 19) | GGAGATCGAAGTTTGT (SEQ ID NO: 903) |
| 456 | GPR7 (SEQ ID NO: 19) | GGGGAGATTGAAGTTT (SEQ ID NO: 904) |
| 457 | ISL1 (SEQ ID NO: 21) | AGATTTTGCGAAAGATA (SEQ ID NO: 905) |
| 458 | ISL1 1 (SEQ ID NO: 21) | TTAGATTTTGTGAAAGATA (SEQ ID NO: 906) |
| 459 | ISL1 (SEQ ID NO: 21) | AAGATATCGAAATTAAGTT (SEQ ID NO: 907) |
| 460 | ISL1 (SEQ ID NO: 21) | AAGATATTGAAATTAAGTTT (SEQ ID NO: 908) |
| 461 | GPRK5 (SEQ ID NO: 22) | AGATTTTCGAGGGAGA (SEQ ID NO: 909) |
| 462 | GPRK5 (SEQ ID NO: 22) | AGATTTTTGAGGGAGAT (SEQ ID NO: 910) |
| 463 | FBN2 (SEQ ID NO: 28) | AATTGGTCGTTAGTTTT (SEQ ID NO: 911) |
| 464 | FBN2 (SEQ ID NO: 28) | GTTAATTGGTTGTTAGTT (SEQ ID NO: 912) |
| 465 | FBN2 (SEQ ID NO: 28) | TTAGGGATCGGATTTG (SEQ ID NO: 913) |
| 466 | FBN2 (SEQ ID NO:28) | ATTAGGGATTGGATTTG (SEQ ID NO: 914) |
| 467 | LIMK1 (SEQ ID NO: 30) | TTTAATTCGAAAGGGAA (SEQ ID NO: 915) |
| 468 | LIMK1 (SEQ ID NO: 30) | TTAATTTGAAAGGGAAAG (SEQ ID NO: 916) |
| 469 | PSD/Q9H469 (SEQ ID NO: 31) | AGGTTAGTCGAGAAGT (SEQ ID NO: 917) |
| 470 | PSD/Q9H469 (SEQ ID NO: 31) | AGGTTAGTTGAGAAGTA (SEQ ID NO: 918) |
| 471 | PSD/Q9H469 (SEQ ID NO: 31) | ATTGTTACGAAGTGGA (SEQ ID NO: 919) |
| 472 | PSD/Q9H469 (SEQ ID NO: 31) | TTGTTATGAAGTGGAAG (SEQ ID NO: 920) |
| 473 | Q96S01 (SEQ ID NO: 34) | TATTGATCGGTTGGAG (SEQ ID NO: 921) |
| 474 | Q96S01 (SEQ ID NO: 34) | TATTGATTGGTTGGAGG (SEQ ID NO: 922) |

(continued)

| No: | Gene | Oligo: |
|---|---|---|
| 475 | ABHD9 (SEQ ID NO: 37) | AGTTAGAGCGTATTATTT (SEQ ID NO: 923) |
| 476 | ABHD9 (SEQ ID NO: 37) | AATAGTTAGAGTGTATTATT (SEQ ID NO: 924) |
| 477 | MLLT3 (SEQ ID NO: 42) | TTAGTGGTCGGAGATA (SEQ ID NO: 925) |
| 478 | MLLT3 (SEQ ID NO: 42) | AGTGGTTGGAGATAGA (SEQ ID NO: 926) |
| 479 | SOLH (SEQ ID NO: 43) | TATATTTCGTGAGGGTA (SEQ ID NO: 927) |
| 480 | SOLH (SEQ ID NO: 43) | TATATTTTGTGAGGGTAG (SEQ ID NO: 928) |
| 481 | Q9NWV0 (SEQ ID NO: 46) | ATTTTTACGAGAAGGTT (SEQ ID NO: 929) |
| 482 | Q9NWV0 (SEQ ID NO: 46) | GATTTTTATGAGAAGGTT (SEQ ID NO: 930) |
| 483 | H2AFY2 (SEQ ID NO: 48) | ATGGGAATCGTGGTTT (SEQ ID NO: 931) |
| 484 | H2AFY2 (SEQ ID NO: 48) | ATGGGAATTGTGGTTT (SEQ ID NO: 932) |
| 485 | RHOC (SEQ ID NO: 49) | AGGTTTACGGAAAAGG (SEQ ID NO: 933) |
| 486 | RHOC (SEQ ID NO: 49) | AAGGTTTATGGAAAAGG (SEQ ID NO: 934) |
| 487 | KBTBD6 (SEQ ID NO: 51) | TAGAGTCGGGTTTTGTA (SEQ ID NO: 935) |
| 488 | KBTBD6 (SEQ ID NO: 51) | TAGAGTTGGGTTTTGTA (SEQ ID NO: 936) |
| 489 | TRPM4 (SEQ ID NO: 52) | ATGGGGGTCGAGATTT (SEQ ID NO: 937) |
| 490 | TRPM4 (SEQ ID NO: 52) | ATGGGGGTTGAGATTT (SEQ ID NO: 938) |
| 491 | TCEB3BP1 (SEQ ID NO: 53) | GGTAGTCGGTATTAGG (SEQ ID NO: 939) |
| 492 | TCEB3BP1 (SEQ ID NO: 53) | TGGTAGTTGGTATTAGG (SEQ ID NO: 940) |
| 493 | TCEB3BP1 (SEQ ID NO: 53) | TGTAGTCGAAGGTTAG (SEQ ID NO: 941) |
| 494 | TCEB3BP1 (SEQ ID NO: 53) | TGTAGTTGAAGGTTAGT (SEQ ID NO: 942) |
| 495 | Q8NCX8 (SEQ ID NO: 54) | GGAGTTTATTCGGTTTAT (SEQ ID NO: 943) |
| 496 | Q8NCX8 (SEQ ID NO: 54) | GGAGTTTATTTGGTTTATA (SEQ ID NO: 944) |

(continued)

| *No:* | *Gene* | *Oligo:* |
|---|---|---|
| 497 | WDFY3 (SEQ ID NO: 58) | AATTGTAGTCGTTTAGTT (SEQ ID NO: 945) |
| 498 | WDFY3 (SEQ ID NO: 58) | AAATTGTAGTTGTTTAGTT (SEQ ID NO: 946) |
| 499 | ZNF566 (SEQ ID NO: 59) | TATTTTTTCGGTAGAGAT (SEQ ID NO: 947) |
| 500 | ZNF566 (SEQ ID NO: 59) | TTTTTTTGGTAGAGATTG (SEQ ID NO: 948) |
| 501 | ZNF566 (SEQ ID NO: 59) | ATGGGTTGCGTTTATA (SEQ ID NO: 949) |
| 502 | ZNF566 (SEQ ID NO: 59) | TGGGTTGTGTTTATAAG (SEQ ID NO: 950) |
| 503 | SEQ ID NO: 61 (SEQ ID NO: 61) | TTTAGGGCGAGGTTAT (SEQ ID NO: 951) |
| 504 | SEQ ID NO: 61 (SEQ ID NO: 61) | TTTAGGGTGAGGTTATT (SEQ ID NO: 952) |
| 505 | SEQ ID NO: 61 (SEQ ID NO: 61) | TGTATATTTCGTAGGGT (SEQ ID NO: 953) |
| 506 | SEQ ID NO: 61 (SEQ ID NO: 61) | TTGTATATTTTGTAGGGT (SEQ ID NO: 954) |
| 507 | PTGS2 (SEQ ID NO: 9) | ATTTGAGCGGTTTTGA (SEQ ID NO: 955) |
| 508 | PTGS2 (SEQ ID NO: 9) | AATTTGAGTGGTTTTGA (SEQ ID NO: 956) |
| 509 | RASSF1 (SEQ ID NO: 10) | AGTAAATCGGATTAGGA (SEQ ID NO: 957) |
| 510 | RASSF1 (SEQ ID NO: 10) | AGTAAATTGGATTAGGAG (SEQ ID NO: 958) |
| 511 | DRG1 (SEQ ID NO: 12) | TGTATGAACGTGTAGTT (SEQ ID NO: 959) |
| 512 | DRG1 (SEQ ID NO: 12) | GTGTATGAATGTGTAGT (SEQ ID NO: 960) |

TABLE 11: Genes and sequences according to sequence listing.

| Gene (HUGO or SPTREMBL ID or EST GeneID) | Ref.-Seq | Genomic SEQ ID NO: | Sense methylated converted SEQ ID NO: | Antisense methylated converted SEQ ID NO: | Sense unmethylated converted SEQ ID NO: | Antisense unmethylated converted SEQ ID NO: |
|---|---|---|---|---|---|---|
| CCND2 | NM_001759 | 1 | 65 | 66 | 193 | 194 |
| CDRN2A | NM_000077 | 2 | 67 | 68 | 195 | 196 |
| CD44 | NM_000610 | 3 | 69 | 70 | 197 | 198 |
| EDNRB1 | NM_000115 | 4 | 71 | 72 | 199 | 200 |
| ELK1 | NM_005229 | 5 | 73 | 74 | 201 | 202 |
| FOS | MM_005252 | 6 | 75 | 76 | 203 | 204 |
| GSTP1 | NM_000852 | 7 | 77 | 78 | 205 | 206 |
| RARB | NM_000965 | 8 | 79 | 80 | 207 | 208 |
| PTGS2 | NM_000963 | 9 | 81 | 82 | 209 | 210 |
| RASSF1 | NM_170715 | 10 | 83 | 84 | 211 | 212 |
| ESR2 | NM_001437 | 11 | 85 | 86 | 213 | 214 |
| DRG1 | NM_004147 | 12 | 87 | 88 | 215 | 216 |
| CMYA3 | | 13 | 89 | 90 | 217 | 218 |
| ONECUT2 | NM_004852 | 14 | 91 | 92 | 219 | 220 |
| MX1 | NM_002462 | 15 | 93 | 94 | 221 | 222 |
| DOCK10 | | 16 | 95 | 96 | 223 | 224 |
| BTG4 | NM_017589 | 17 | 97 | 98 | 225 | 226 |
| DMRTC2 | NM_033052 | 18 | 99 | 100 | 227 | 228 |
| GPR7 | NM_005285 | 19 | 101 | 102 | 229 | 230 |
| FAT | NM_005245 | 20 | 103 | 104 | 231 | 232 |
| ISL1 | NM_002202 | 21 | 105 | 106 | 233 | 234 |
| GPRK5 | NM_005308 | 22 | 107 | 108 | 235 | 236 |
| SLC35F2 | NM_017515 | 23 | 109 | 110 | 237 | 238 |

(continued)

| Gene (HUGO or SPTREMBL ID or EST GeneID) | Ref.-Seq | Genomic SEQ ID NO: | Sense methylated converted SEQ ID NO: | Antisense methylated converted SEQ ID NO: | Sense unmethylated converted SEQ ID NO: | Antisense unmethylated converted SEQ ID NO: |
|---|---|---|---|---|---|---|
| C14orf59 | NM_174976 | 24 | 111 | 112 | 239 | 240 |
| SNRPN | NM_003097 | 25 | 113 | 114 | 241 | 242 |
| ARHGEF18 | NM_015318 | 26 | 115 | 116 | 243 | 244 |
| SNX8 | NM_013321 | 27 | 117 | 118 | 245 | 246 |
| FBN2 | NM_001999 | 28 | 119 | 120 | 247 | 248 |
| HOXB5 | NM_002147 | 29 | 121 | 122 | 249 | 250 |
| LIMK1 | NM_002314 | 30 | 123 | 124 | 251 | 252 |
| PSD; Q9H469 | NM_002779; NM_ 024326 | 31 | 125 | 126 | 253 | 254 |
| SLC38A1 | NM_030674 | 32 | 127 | 128 | 255 | 256 |
| HIST1H4J | NM_003495 | 33 | 129 | 130 | 257 | 258 |
| Q96S01 | Not applicable | 34 | 131 | 132 | 259 | 260 |
| Genomic region downstream of FOXL2 | NM_023067 | 35 | 133 | 134 | 261 | 262 |
| ORC4L | NM_002552 | 36 | 135 | 136 | 263 | 264 |
| ABHD9 | NM_024794 | 37 | 137 | 138 | 265 | 266 |
| CD37 | NM_001774 | 38 | 139 | 140 | 267 | 268 |
| GRN | NM_002087 | 39 | 141 | 142 | 269 | 270 |
| EPAS1 | NM_001430 | 40 | 143 | 144 | 271 | 272 |
| NOTCH1 | NM_017617 | 41 | 145 | 146 | 273 | 274 |
| MLLT3 | NM_004529 | 42 | 147 | 148 | 275 | 276 |
| SOLH | NM_005632 | 43 | 149 | 150 | 277 | 278 |
| ENSESTG00002636932 | Not applicable | 44 | 151 | 152 | 279 | 280 |
| Q8N365 | NM_144697 | 45 | 153 | 154 | 281 | 282 |

(continued)

| Gene (HUGO or SPTREMBL ID or EST GeneID) | Ref.-Seq | Genomic SEQ ID NO: | Sense methylated converted SEQ ID NO: | Antisense methylated converted SEQ ID NO: | Sense unmethylated converted SEQ ID NO: | Antisense unmethylated converted SEQ ID NO: |
|---|---|---|---|---|---|---|
| Q9NWV0 | NM_017891 | 46 | 155 | 156 | 283 | 284 |
| ENST00000339569 | Not applicable | 47 | 157 | 158 | 285 | 286 |
| H2AFY2 | NM_018649 | 48 | 159 | 160 | 287 | 288 |
| RHOC | NM_005167 | 49 | 161 | 162 | 289 | 290 |
| NR2E1 | NM_003269 | 50 | 163 | 164 | 291 | 292 |
| KBTBD6 | NM_152903 | 51 | 165 | 166 | 293 | 294 |
| TRPM4 | NM_017636 | 52 | 167 | 168 | 295 | 296 |
| TCEB3BP1 | NM_020695 | 53 | 169 | 170 | 297 | 298 |
| Q8NCX8 | NM_178564 | 54 | 171 | 172 | 299 | 300 |
| Genomic sequence | Genomic sequence | 55 | 173 | 174 | 301 | 302 |
| SNAPC2 | NM_003083 | 56 | 175 | 176 | 303 | 304 |
| PTPRN2 | NM_002847 | 57 | 177 | 178 | 305 | 306 |
| WDFY3 | NM_014991 | 58 | 179 | 180 | 307 | 308 |
| ZNF566 | NM_032838 | 59 | 181 | 182 | 309 | 310 |
| Q9NP73 | NM_018466 | 60 | 183 | 184 | 311 | 312 |
|  | NM_173660 | 61 | 185 | 186 | 313 | 314 |
| Q86SP6 | Not applicable | 62 | 187 | 188 | 315 | 316 |
| HIST2H2BF regulatory region | NM_003529 | 63 | 189 | 190 | 317 | 318 |
| PMF1 | NM_000711 | 64 | 191 | 192 | 319 | 320 |
| PITX2 | NM_000325 | 961 | 962 | 963 | 964 | 965 |

TABLE 12: Primers and Probes for MSP-MethyLight™ Assays.

| | Forward | Reverse | Probe |
|---|---|---|---|
| SEQ ID NO: 19 | tgcggattttggcgaattc | **aaaaacccgccgactacgaa** | **aactaaaacgcctaccgactaaatatccgcct** |
| SEQ ID NO: 35 | gagttttcgcggttcgga | cgcgaccgctaaactcg | cgaccaaatccgaacccgtacatcg |
| SEQ ID NO: 37 | cggtatgtcgtcgcgtttc | ctaacaccgcttcgccg | cctaaccgacaacgccgccgtaat |
| SEQ ID NO: 7 | agttgcgcggcgatttc | **gccccaatactaaatcacgacg** | cggtcgacgttcggggtgtagcg |
| SEQ ID NO: 63 | atggcgtataggttcgtgttttc | **cttccaacgactaatacgcgaa** | cgcttccaaaactcgaccgtaataacgc |
| SEQ ID NO: 8 | **atataaactaaaaaacgaaacgataaacga** | ttttacgtttttttatttgcggc | cgaacgaacgcaaacgaaacaccg |
| SEQ ID NO: 64 | ccactaactccgtaccgtacgtat | ggttagcgagtcgatcggtt | acgttctcgtctccgctaaattatccgc |
| SEQ ID NO: 43 | tcgtttttttagtcgtttgggtc | tatcgaaaccccgaaccg | caccgtcgcctcccacgaca |
| SEQ ID NO: 40 | ttttcgtttttttttcggtcgtt | gaccgcgcaaaaaaactcg | cgctcgaataacgccgaacccg |
| SEQ ID NO: 32 | ctcgcacaaaaacgaaaatacg | agttcgcgtttttaaacgttgtc | ccgacaccgacccacgcgt |
| SEQ ID NO: 37 | cggtatgtcgtcgcgtttc | ctaacaccgcttcgccg | cctaaccgacaacgccgccgtaat |
| SEQ ID NO: 9 | aaacgaaaactctacccgaatacg | cgcggttttggcgttt | ccgaaatccccgatacgcgacg |
| SEQ ID NO: 19 | tgcggattttggcgaattc | **aaaaacccgccgactacgaa** | **aactaaaacgcctaccgactaaatatccgcct** |
| SEQ ID NO: 34 | gtatttatttggtaatttcgtattataattcgag | **gactaaaaacgcgaaatccga** | acgatccgatctaaaaaccgactcttcgaa |
| SEQ ID NO: 35 | gagttttcgcggttcgga | cgcgaccgctaaactcg | cgaccaaatccgaacccgtacatcg |

Table 13: Components for all QM assays according to Example 5

| Component | Company | Stock conc |
|---|---|---|
| Reaction buffer ROX | Eurogentec | 10x |
| MgCL2 | Eurogentec | 50mM |
| DTNPs | MBI | 25mM each |
| Forward primer | TIB Molbiol | 6,25μM |
| Reverse primer | TIB Molbiol | 6,25μM |
| cg Probe | Eurogentec | 4μM |
| tg probe | Eurogentec | 4μM |
| HotGoldStar- Taq | Eurogentec | 5U/μl |
| Water | Fluka | |

Table 14: Optimized Reaction conditions for all QM assays according to Example 5

| Gene | dNTPs | Buffer | $MgCL_2$ | Primers | Probes | Taq | Baseline | Threshold | Annealing |
|---|---|---|---|---|---|---|---|---|---|
| PITX2 | 250μM | 1X | 3mM | 625nM | 200nM | 1U | 3 / 23 | 0,05 | 62°C |
| Chr3-EST | 250μM | 1X | 3.5mM | 625nM | 200nM | 1U | 6 / 22 | 0,05 | 60°C |
| ABHD9 | 250μM | 1X | 2.5mM | 625nM | 200nM | 1U | 6 / 25 | 0,08 | 60°C |
| GPR7 | 250μM | 1X | 3mM | 625nM | 200nM | 1U | 6 / 24 | 0,05 | 60°C |
| HIST 2H2BF | 250μM | 1X | 3mM | 625nM | 200nM | 1U | 3 / 22 | 0,05 | 60°C |
| OCND2 | 250μM | 1X | 3mM | 625nM | 200nM | 1U | 3 / 22 | 0,08 | 60°C |

Table 15: Cycle program for QM assays according to Example 5. For annealing temperatures see Table 14.

| | **T[°C]** | **t** | **Cycles** |
|---|---|---|---|
| Initial denat. | 95.0 | 10min | |
| Denaturation | 95.0 | 15sec | 45x (PITX2 50x) |
| Annealing | Variable | 60sec | |

Table 16: Clinical characteristics of the patient population according to Example 5. Age is given as the mean, and all other variables are given as the number of patients. Not all information was available for all patients.

| Clinical Variable | | Baylor | Stanford | VMMC | Total |
|---|---|---|---|---|---|
| Age (mean) | | 61.1 | 61.7 | 61.1 | 61.3 |
| PSA | 0-4 | 25 | 33 | 18 | 76 |
| | 4-10 | 120 | 139 | 99 | 358 |
| | >10 | 60 | 72 | 30 | 162 |
| Gleason Score | 5-6 | 137 | 164 | 118 | 419 |
| | 7 | 37 | 44 | 19 | 100 |
| | 8-10 | 26 | 31 | 25 | 82 |

(continued)

| Clinical Variable | | Baylor | Stanford | VMMC | Total |
|---|---|---|---|---|---|
| Stage | Organ-confined | 110 | 211 | 113 | 434 |
| | Not organ-confined | 94 | 33 | 35 | 162 |
| PSA-based recurrence | | 22 | 10 | 13 | 45 |
| Decision to treat based recurrence | | 3 | 14 | 4 | 21 |
| Total Samples | | 206 | 244 | 162 | 612 |

Table 17: Performance of the six markers according to Example 5 using the median methylation level as a cut-off.

| | P value | Events in hypomethylated group | Events in hypermethylated group | AUC (5 years) |
|---|---|---|---|---|
| PITX2 | 0.000017 | 15 | 49 | 0.64 |
| GPR7 | 0.0016 | 20 | 45 | 0.64 |
| HIST2H2BF regulatory region | 0.018 | 22 | 43 | 0.60 |
| SEQ ID NO: 35 | 0.0059 | 21 | 44 | 0.61 |
| ABHD9 | 0.018 | 22 | 43 | 0.58 |
| CCND2 | 0.22 | 27 | 38 | 0.61 |

Table 18: Results of the Cox regression analysis for PITX2 according to Example 5. Using stepwise regression the marker remains in the model. P-values refer to the null-hypothesis "hazard ratio equals zero".

| Variable | P value | Hazard Ratio | Lower Confidence Interval | Upper Confidence Interval |
|---|---|---|---|---|
| PITX2 | 0.0043 | 2.222 | 1.284 | 3.845 |
| Disease stage | 0.0692 | 1.713 | 0.965 | 3.061 |
| Gleason category | 0.0107 | 1.798 | 1.146 | 2.821 |
| PSA | 0.075 | 1.254 | 0.977 | 1.609 |
| Nomogram category | 0.0866 | 2.187 | 0.894 | 5.353 |

Table 19: Results of the Cox regression analysis for SEQ ID NO: 63 according to Example 5. The marker remains in the model.

| Variable | P value | Hazard Ratio | Lower Confidence Interval | Upper Confidence Interval |
|---|---|---|---|---|
| SEQ ID NO: 63 | 0.0239 | 2.918 | 1.152 | 7.393 |
| Disease stage | 0.0599 | 1.735 | 0.977 | 3.081 |
| Gleason category | 0.0106 | 1.799 | 1.146 | 2.822 |
| PSA | 0.0732 | 1.25 | 0.979 | 1.596 |
| Nomogram category | 0.0384 | 2.526 | 1.051 | 6.071 |

Table 20: Primer and probe sequences of assays according to Example 5.

| CCND2 | | | |
|---|---|---|---|
| Primers + Probes | Sequence | Label 5' | Label 3' |
| Forward primer | Ttttgtaaagatagttttgatttaagtat (SEQ ID NO: 983) | | |
| Reverse primer | caaactttctccctaaaaacc (SEQ ID NO: 984) | | |
| CG-probe | Cgccgccaacacgatcg (SEQ ID NO: 985) | FAM | BHQ1 |
| TG-probe | Caccaccaacacaatcaaccctaacac (SEQ ID NO: 986) | HEX | BHQ1 |
| | | | |
| SEQ ID NO: 63 | | | |
| Primers + Probes | Sequence | Label 5' | Label 3' |
| Forward primer | tgattattatgtttaaggatatttagttg (SEQ ID NO: 987) | | |
| Reverse primer | caataactctaaaaaaaacctttaaatc (SEQ ID NO: 988) | | |
| CG-probe | Cgctccccgcgaatacgacg (SEQ ID NO: 989) | FAM | BHQ1 |
| TG-probe | Taaacccactccccacaaatacaacaaac (SEQ ID NO: 990) | HEX | BHQ1 |
| | | | |
| GRP7 | | | |
| Primers + Probes | Sequence | Label 5' | Label 3' |
| Forward primer | Catccctacacttccaaac (SEQ ID NO: 991) | | |
| Reverse primer | Ggagttgttaggagaaaagtt (SEQ ID NO: 992) | | |
| CG-probe | Cgaacacccaaccgacaaacg (SEQ ID NO: 993) | FAM | BHQ1 |
| TG-probe | Caaacacccaaccaacaaacatctca (SEQ ID NO: 994) | HEX | BHQ1 |
| | | | |
| Chr3_EST | | | |
| Primers + Probes | Sequence | Label 5' | Label 3' |
| Forward primer | ttgtagggtttttttgggtt (SEQ ID NO: 995) | | |
| Reverse primer | Ctcaaaacccttaaaaacataaa (SEQ ID NO:996) | | |
| CG-probe | Ataaccacactacgcgcctcc (SEQ ID NO: 997) | FAM | BHQ1 |
| TG-probe | Ataaccacactacacacctcccaca (SEQ ID NO: 998) | Yakima Yellow | BHQ1 |
| | | | |
| ABHD9 | | | |
| Primers + Probes | Sequence | Label 5' | Label 3' |
| Forward primer | Ggtgttagggtttaggggtt (SEQ ID NO: 999) | | |
| Reverse primer | Ccaaatatttacctaacactcaaata (SEQ ID NO: 1000) | | |
| CG-probe | Aactattttctatcgaaaccgcccg (SEQ ID NO: 1001) | FAM | BHQ1 |
| TG-probe | Aactattttctatcaaaaccacccacctct (SEQ ID NO: 1002) | Yakima Yellow | BHQ1 |
| | | | |
| PITX2 | | | |
| Primers + Probes | Sequence | Label 5' | Label 3' |
| Forward primer | Gtaggggagggaagtagatgtt (SEQ ID NO: 1003) | | |
| Reverse primer | Ttctaatcctcctttccacaataa (SEQ ID NO: 1004) | | |

(continued)

| PITX2 | | | |
|---|---|---|---|
| CG-probe | Agtcggagtcgggagagcga (SEQ ID NO: 1005) | FAM | TAMRA |
| TG-probe | Agttggagttgggagagtgaaaggaga (SEQ ID NO: 1006) | VIC | TAMRA |
| CCND2 | | | |
| Primers + Probes | Sequence | Label 5' | Label 3' |
| Forward primer | tttttgtaaagatagttttgatttaagtat | | |
| Reverse primer | caaactttctccctaaaaacc | | |
| CG-probe | cgccgccaacacgatcg | FAM | BHQ1 |
| TG-probe | caccaccaacacaatcaaccctaacac | HEX | BHQ1 |
| | | | |
| SEQ ID NO: 63 | | | |
| Primers + Probes | Sequence | Label 5' | Label 3' |
| Forward primer | tgattattatgtttaaggatatttagttg | | |
| Reverse primer | caataactctaaaaaaaacctttaaatc | | |
| CG-probe | cgctccccgcgaatacgacg | FAM | BHQ1 |
| TG-probe | taaacccactccccacaaatacaacaaac | HEX | BHQ1 |
| | | | |
| GRP7 | | | |
| Primers + Probes | Sequence | Label 5' | Label 3' |
| Forward primer | catccctacacttccaaac | | |
| Reverse primer | ggagttgttaggagaaaagtt | | |
| CG-probe | cgaacacccaaccgacaaacg | FAM | BHQ1 |
| TG-probe | caaacacccaaccaacaaacatctca | HEX | BHQ1 |
| | | | |
| Chr3_EST | | | |
| Primers + Probes | Sequence | Label 5' | Label 3' |
| Forward primer | ttgtagggttttttgggtt | | |
| Reverse primer | ctcaaaacccttaaaaacataaa | | |
| CG-probe | ataaccacactacgcgcctcc | FAM | BHQ1 |
| TG-probe | ataaccacactacacacctcccaca | Yakima Yellow | BHQ1 |
| | | | |
| ABHD9 | | | |
| Primers + Probes | Sequence | Label 5' | Label 3' |
| Forward primer | ggtgttagggtttaggggtt | | |
| Reverse primer | ccaaatatttacctaacactcaaata | | |
| CG-probe | aactattttctatcgaaaccgcccg | FAM | BHQ1 |
| TG-probe | aactattttctatcaaaaccacccacctct | Yakima Yellow | BHQ1 |
| | | | |

(continued)

| PITX2 | | | |
|---|---|---|---|
| Primers + Probes | Sequence | Label 5' | Label 3' |
| Forward primer | gtaggggagggaagtagatgtt | | |
| Reverse primer | ttctaatcctcctttccacaataa | | |
| CG-probe | agtcggagtcgggagagcga | FAM | TAMRA |
| TG-probe | agttggagttgggagagtgaaaggaga | VIC | TAMRA |

Table 21

| SEQ ID NO | Classification | Tissue Type | AUC | Sensitivity | Specificity |
|---|---|---|---|---|---|
| 19 | Recurrance | Frozen & PET | 0.62 | 0.35 | 0.86 |
| 63 | Recurrance | Frozen & PET | 0.68 | 0.34 | 0.85 |
| 35 | Recurrance | Frozen & PET | 0.6 | 0.27 | 0.85 |
| 37 | Recurrance | Frozen & PET | 0.61 | 0.18 | 0.85 |
| 19 | Recurrance | PET | 0.58 | 0.5 | 0.88 |
| 63 | Recurrance | PET | 0.63 | 0.17 | 0.89 |
| 35 | Recurrance | PET | 0.69 | 0.33 | 0.89 |
| 37 | Recurrance | PET | 0.69 | 0.17 | 0.89 |
| 19 | Recurrance | Frozen | 0.62 | 0.34 | 0.86 |
| 63 | Recurrance | Frozen | 0.68 | 0.31 | 0.86 |
| 35 | Recurrance | Frozen | 0.6 | 0.28 | 0.85 |
| 37 | Recurrance | Frozen | 0.6 | 0.2 | 0.85 |
| 19 | Gleason | Frozen & PET | 0.72 | 0.49 | 0.86 |
| 63 | Gleason | Frozen & PET | 0.73 | 0.39 | 0.86 |
| 35 | Gleason | Frozen & PET | 0.62 | 0.21 | 0.86 |
| 37 | Gleason | Frozen & PET | 0.76 | 0.48 | 0.86 |
| 19 | Gleason | PET | 0.72 | 0.36 | 0.89 |
| 63 | Gleason | PET | 0.7 | 0.38 | 0.86 |
| 35 | Gleason | PET | 0.56 | 0.12 | 0.86 |
| 37 | Gleason | PET | 0.77 | 0.5 | 0.86 |
| 19 | Gleason | Frozen | 0.74 | 0.56 | 0.87 |
| 63 | Gleason | Frozen | 0.76 | 0.58 | 0.86 |
| 35 | Gleason | Frozen | 0.65 | 0.28 | 0.87 |
| 37 | Gleason | Frozen | 0.77 | 0.47 | 0.87 |

**EXAMPLES**

Investigation Overview

The objective of the present investigation is to develop genetic markers that can identify prostate cancer patients that have aggressive tumors with metastatic potential. It was decided to use methylation analysis to identify differentially expressed genomic markers.

The investigation started with a genome-wide screening step to discover novel markers. This approach utilizes molecular biology methods for the determination of differential methylation between predefined groups of patient samples. Differentially methylated sequences identified using said genomic screening methods are herein referred to as Methylation Sequence Tags (also referred to as MeSTs). The genome-wide screening step identifies differentially methylated CpG sites. Additional information concerning the area surrounding the identified CpG site is obtained by BLAST analysis of the sequence found in the screening step (MeST or Methylation Sequence Tag) and mapping to the human genome.
Following identification of candidates by genome-wide screening, MSP MethyLight™ assays were developed for a subset of the promising candidates. These assays were used to analyze methylation in 56 prostatectomy samples with clinical outcome information. MSP MethyLight™ assays are sensitive and quantitative and they rely on CpG co-methylation; therefore, this assay format provides complementary data to DNA array technology.
All of the promising MeST candidates and some additional candidates were then analyzed by methylation oligonucleotide array using the applicant's proprietary chip technology as described in further detail below. This process provides information concerning the preliminary performance of the MeSTs or candidate genes if an appropriate sample set is used. Candidate markers will be selected based on data obtained from the chip data analysis. Selection is mainly based on the AUC of the marker in the discrimination between the desired classes.
To complete the development process, the candidate markers selected for assay development from the chip study were tested in real-time PCR assays for further validation on the target population and on the sample material used for a potential diagnostic or prognostic test (paraffin embedded tissues).

## EXAMPLE 1: MeST Screening

Experimental Design

Pooled genomic DNA from prostate cancer samples was used for genome-wide screening of markers associated with tumor aggressiveness. Two different methods were applied: Methylation Specific-Arbitrarily Primed Polymerase Chain Reaction (MS-APPCR) (Liang *et al.,* 1998) and Methylated CpG Island Amplification (MCA) (Toyota *et al*., 1999). These technologies distinguish between methylated and unmethylated CpG sites through the use of methylation sensitive enzymes n general, genomic DNA is cut with a methylation sensitive restriction enzyme. Methylated fragments are preferentially amplified because cleavage at unmethylated sites prevents amplification of the unmethylated targets. Methylated sequence tag (MeST) fragments obtained using these techniques are sequenced and mapped to the human genome using the BLAST utility in the Ensembl database (www.ensembl.org).
The primary definition of tumor aggressiveness for the screening phase was based on PSA recurrence after radical prostatectomy. An aggressive tumor was defined as one that recurred in less than 24 months. A non-aggressive tumor was defined as one that did not recur after at least 48 months of follow up with regular PSA testing. Five samples in each category were pooled, and there were three pools for each category. The median time to PSA recurrence for the patients with aggressive tumors was 5.1 months. The median follow up time for patients without recurrence was 60.3 months. None of these patients received neo-adjuvant or adjuvant therapies before PSA relapse.
We also included four other comparisons with alternative indicators of aggressiveness. Samples with Gleason grades four and five were compared to samples with Gleason grades one, two, and three. Late stage tumors (III and IV) were compared to early stage tumors (I and II). Peripheral zone tumors were compared to transition zone tumors. Lastly, normal tissues adjacent to tumors in patients with early PSA recurrence (<2 years) were compared to normal tissues adjacent to tumors in patients with no PSA recurrence (>4 years follow up).

Screening

The Mest screening process usually results in a large number of sequences that represent potential markers. Some of them are redundant or cannot be matched to the genome. The remaining sequences are selected using a scoring procedure assessing:

Appearance using multiple methods
Appearance in multiple pool comparisons of the same type
Location in CpG island
Location in promoter region
Location near or within gene sequence
Association of nearby gene with cancer
Class of gene (transcription factor, growth factor, etc.)
Repetitive element (negative score)
In this scoring scheme, a MeST sequence receives one point for each of the positive criteria (first seven criteria), and receives a score of minus 8 for having repetitive sequence content greater than 50 % (negative score). In the latter case the MeST always has an overall negative score. Tables 2 and 3 summarize the results of the MeST screening experiments.

Using the scoring criteria and literature based investigation of potential gene function, 80 genes were selected for chip amplicon design. MeSTs from the comparisons based on PSA recurrence were prioritized, but many of the MeSTs from the other comparisons were included in the amplicon design list.

**EXAMPLE 2: Real-time PCR Study**

Experimental Design

MSP assays were developed on the Taqman™ 7900 for strong candidates from MeST screening in order to obtain early data on the performance of the MeSTs as markers of prostate cancer aggressiveness.

As used herein the term MSP-MethyLight shall be taken to mean an assay comprising the amplification of a bisulfite treated sequence by means of methylation specific primers and the detection of resultant amplificates by means of MethyLight detection oligonucleotides (also referred to as 'probes').

MSP-MethyLight assays were developed for a number of MeSTs (see Table 12). The assays were tested on artificially methylated DNA and dilutions of methylated DNA in unmethylated DNA to ensure assay performance. All assays were able to amplify as little as 100 picograms of methylated DNA in the presence or absence of 20 to 100 nanograms of unmethylated DNA. Most of the assays were quantitative between 0.1 and 100% methylation.

Of the 36 assays, 21 were methylated in a pool of prostate tumor DNAs. These 21 assays were first tested on 46 samples from the screening process. These 46 samples included 14 prostatectomies from patients who recurred in less than 24 months and 18 prostatectomies from patients who did not recur after at least 48 months. In addition, there were fourteen patients without follow up information; nine were high Gleason (Score 8-10) and 5 were low Gleason (Score 2-6). The data from this experiment were used to choose seven assays for an independent sample set.

The second sample set consisted of 26 frozen radical prostatectomy samples from patients with early PSA recurrence, with a median time to PSA recurrence of 6 months, and 30 samples from patients with no PSA recurrence after at least 48 months (median follow up time was 60 months). The Methylight assays were used to measure the amount of DNA methylated at each locus by comparing the threshold cycle (Ct) to a standard curve of methylated DNA. A control assay was used to measure the total amount of DNA. All samples were run in triplicate for all assays. The primer and probe sequences are listed in Table 12. The ratio of the methylated DNA to the total amount of DNA was used to indicate the methylation status of each candidate in each sample.

Results

The methylation values for each assay were used to construct ROC curves (Figures 2 to 8) and calculate sensitivity, specificity, and p values. The data are summarized in Table 4. The AUC values for some of the candidates suggest that the methylation of the marker could have prognostic value. Six candidates had an AUC of 0.68 or greater. The strongest candidates, SEQ ID NO: 19 (GPR7) and SEQ ID NO: 35 (genomic region downstream of FOXL2), were significant by a Wilcoxon test after Bonferroni correction. When the specificity is set to 87% for these two assays, the sensitivity is around 50% for each. (SEQ ID NOs correlated to gene names in table 11.)

**EXAMPLE 3: Chip Study**

In the chip study, a gene panel composed of candidate genes and selected MeSTs were analyzed on 329 samples using the applicant's microarray technology.

Sample Set

The sample set included 329 frozen samples obtained from radical prostatectomies. Only samples with an estimated percent tumor of at least 70% were used, and the median estimated percent tumor (by volume) was 90%. Some sample providers achieved high percent tumor either by coring out a section of the frozen prostate known to contain tumor or by dissecting normal tissue away from the tumor. Patients who received neo-adjuvant therapy were not excluded from the study. Clinical information on disease free survival was not used for any patient receiving adjuvant therapy prior to disease recurrence.

Gleason scores were available for almost all prostatectomies. For some samples, the Gleason score of the portion of the tumor provided to the applicant was also available. The sample set consisted of samples that qualified for one of the two extreme Gleason categories (high or low), samples from patients that qualified for the two relapse categories (early or no relapse), or samples that fell into multiple categories (e.g., high Gleason and early recurrence). Within the sample set, there were 135 samples with low Gleason scores (1 +2, 2+1, 2+2, 2+3, 3+2, and 3+3). There were 99 samples with high Gleason scores (3+5, 5+3, 4+4, 4+5, 5+4, and 5+5). For some of the samples, clinical follow up information was available. Sixty-five patients experienced PSA recurrence in less than two years, and 88 patients did not recur after at least 4 years follow up.

For control purposes additional samples were included. In order to control the quality and the functionality of oligos, unmethylated (phi-29 DNA) and artificially methylated DNAs (Promega) were used. Additionally, 16 DNA samples from lymphocytes were processed in parallel to the test samples.

Array

The array contained oligos representing 62 different candidates. Fifty-one of the candidates were MeSTs. One MeST, SEQ ID NO:32, was represented by two non-overlapping amplicons, both near exon one of the gene. For all of the MeSTs, the amplicon was designed as close to the MeST sequence as possible in a CpG rich region. An amplicon for the X chromosome gene ELK1 was included for analysis of male and female control lymphocyte samples.

Other analysed genes included CCND2 (Cyclin D2 Padar et al 2003), CD44 (Woodson et al 2004), EDNRB1 (endothelin receptor B; Woodson et al 2004; Nelson et al 1997), GSTP1 (glutathione S-transferase pi; Maruyama et al 2002), RARB (retinoic acid receptor, beta; Singal et al 2004), PTGS2 (prostaglandin-endoperoxide synthase 2; Yegnasubramanian et al 2004), RASSF1 (Ras association domain family 1; Liu et al 2002), ESR2 (estrogen receptor 2; Zhu et al 2004), DRG1 (developmentally regulated GTP binding protein 1; Bandyopadhyay et al 2003), and CDKN2A (p16; Halvorsen et al 2000). DRG1 was represented with two amplicons. In all cases, CpG rich areas near the promoter or exon 1 were targeted. For p16, the CpG rich area encompassing exon 2 was used because higher methylation rates have been noted in the literature (Nguyen et al 2000).

A complete overview of all analyzed genes can be found in Table 11.

Statistical Methods; Analysis of Chip Data

From Raw Hybridization Intensities to Methylation Ratios

The log methylation ratio (log(CG/TG)) at each CpG position is determined according to a standardized preprocessing pipeline that includes the following steps:

- For each spot the median background pixel intensity is subtracted from the median foreground pixel intensity. This gives a good estimate of background corrected hybridization intensities;
- For both CG and TG detection oligonucleotides of each CpG position, the background corrected median of the 4 redundant spot intensities is taken;
- For each chip and each CG/TG oligo pair, the log(CG/TG) ratio is calculated; and
- For each sample, the median of log(CG/TG) intensities over the redundant chip repetitions is taken.
  This log ratio has the property that the hybridization noise has approximately constant variance over the full range of possible methylation rates (Huber *et al.,* 2002).

Principle Component Analysis

The principle component analysis (PCA) projects measurement vectors (e.g. chip data, methylation profiles on several CpG sites etc.) onto a new coordinate system. The new coordinate axes are referred to as principal components. The first principal component spans the direction of the largest variance of the data. Subsequent components are ordered by decreasing variance and are orthogonal and uncorrelated to each other. Different CpG positions contribute with different weights to the extension of the data cloud along different components. PCA is an unsupervised technique, i.e. it does not take into account any group or label information of the data points (for further details see e.g. Ripley, 1996).

PCA is typically used to project high dimensional data (in our case methylation-array data) onto lower dimensional subspaces in order to visualize or extract features with high variance from the data. In the present report we used 2 dimensional projections for statistical quality control of the data. We investigated the effect of different process parameters on the chip data and excluded that changing process parameters caused large alterations in the measurement values.

A robust version of PCA was used to detect single outlier chips and exclude them from further analysis (Model *et al.,* 2002).

$T^2$ Control Charts

To control the general stability of the chip production process we use methods from the field of multivariate statistical process control (MVSPC). Our major tool is the $T^2$ control chart, which is used to detect significant deviations of the chip process from normal working conditions (Model *et al.,* 2002).
The $T^2$ chart is constructed as follows:

1. Order the chip data with respect to a process parameter (e.g. hybridization date or spotting robot);
2. Define a historic data set, which describes the chip process under normal working conditions (e.g. the first 75 hybridized chips). In the chart, data from the historical data set are indicated by a special plot symbol; and
3. Compute the distance of every new chip to the historic data set. If the distance of several consecutive chips exceeds a given control limit the process has to be regarded as out of control.

Use of $T^2$ charts to monitor the chip production process allows us to efficiently detect and eliminate most systematic error sources.

Hypothesis testing

Our main task is to identify markers that can make a significant contribution to the class prediction of samples. A significant contribution is detected when the null-hypothesis that a prediction model including the marker does not improve classification performance over a model without the marker can be rejected with $p<0.05$. Because we apply this test to a whole set of potential markers, we have to correct the p-values for multiple testing. We do this by applying the conservative Bonferroni correction, which simply multiplies the single marker p-values with the number of potential markers tested. We also give results with the less conservative False Screening Rate (FDR) method (Dudoit et al 2002).
Throughout this report a marker (sometimes also simply refered to as gene or amplicon) is a genomic region of interest (ROI). It usually consists of several CpG positions in the respective area. For testing the null hypothesis that a marker has no predictive power we use the Wilcoxon rank sum tests to compare groups. A significant test result ($p<0.05$) indicates a shift between the distributions of the respective methylation logratios, i.e. *In(CG/TG).* The mean of all oligos for each mrker was used to combine CpGs before Wilcoxon statistics were generated. This approach has the advantage that it favors markers showing co-methylation.
A significant p-value for a marker means that the methylation of this ROI has some systematic correlation to the question of interest as given by the two classes. In general a significant p-value using the Wilcoxon rank sum test also implies good classification performance.

Class prediction by ROC analysis

Receiver Operation Characteristic (ROC) analysis was used to estimate how well the CpG ensemble of a selected marker can differentiate between different tissue classes. An ROC curve is a plot of true positive rate (sensitivity) versus false positive rate (1 - specificity) for a marker over all possible test thresholds. The Area Under the Curve (AUC) of an ROC curve gives the probability of properly classifying a random sample and can thus be used to evaluate overall marker performance. The AUC is related to the Wilcoxon test statistic and comparative ranking of markers by AUCs or Wilcoxon p-values is equivalent. The mean of all oligos for each marker was used to combine CpGs before ROC analysis. This approach has the advantage that it favors markers showing co-methylation.

Experimental Performance

DNA Extraction

Samples were received from external collaborators either as frozen tissues or extracted genomic DNA. DNA from tissue samples was isolated at Epigenomics Berlin using the Qiagen DNA Mini Kit.
The DNA quality of all delivered and extracted samples was first assessed by photometrical measurements. Extinctions at 260 nm and 280 nm as well as A260/280 ratios were determined and the resulting concentrations were calculated. For most of the DNAs used, A260/280 ratios between 1.6 and 1.9 were determined indicating sufficient purity. For some samples ratios in the range of 1.2-1.5 were calculated. Nevertheless, these DNAs were processed as well.
After photometrical measurements 200 ng of the genomic DNAs were applied to a 0.8% agarose gel and gel electrophoresis was performed. Figure 8 shows a typical gel image. No or only minor signs of degradation were observed, indicating a good overall quality of the DNAs used.
Bisulfite Treatment and Multiplex PCR
Total genomic DNAs from all selected samples as well as control DNAs were bisulfite treated converting unmethylated cytosines to uracil. Methylated cytosines are conserved. Bisulfite treatment was performed using Epigenomics' dioxane bisulfite treatment process. In order to avoid a joint processing of all samples with the same biological background resulting in a potential process-bias in the data later on, the samples were randomly grouped into processing batches. Batches of 50 samples were randomized for the Gleason score and PSA outcome. Two independent bisulfite reactions were performed per DNA sample. After bisulfitation, 11.25 ng of each sample was used in 8 subsequent multiplex PCR (mPCR) reactions containing 8 primer pairs each.
For monitoring the mPCR results, gel electrophoresis was performed for all PCR products. To find the best composition of eight primer pairs in a mPCR-set, ALF analysis was used, comparing a mixture of single PCR products with different variants of mPCR-sets.

ALF Express Analyses:

For evaluation of the amplified fragments, mPCR products of Promega DNA were analyzed using the ALF Express-technology. The results for those mPCRs were compared to the mixture of single PCR products. Figure 9 illustrates the result for an 8-plex PCR. All 64 fragments (eight 8-plex-PCRs) selected for the study could be amplified in the performed mPCR experiments. In some cases undesired side products were obtained.

Agarose Gel Electrophoresis:

As mentioned above two independent bisulfite reactions and PCRs were performed per DNA sample and the PCR products obtained were applied to a 2% agarose gel. In Figure 10 a typical gel image is shown illustrating the mPCR performance for 10 samples. No visible PCR product implies failure of bisulfite treatment or PCR amplification. The PCR was then repeated with twice the amount of DNA (22.5 ng). Bisulfite treated DNAs that failed again were excluded from the study. f we obtained only one hybridization probe (64 pooled PCR products) from a sample, 4 chips were hybridized using this single probe. If the probes from two independent bisulfite treatments of a sample were successfully amplified, both probes were hybridized onto two chips each.
Four (4) out of 331 samples processed (including control samples) could not be amplified, despite several attempts. These samples were not further processed.

Results of Chip Study

Our primary analysis was a comparison of samples with high Gleason scores (8-10) and low Gleason scores (2-6 with no grade 4 or 5 component). These classes are categories A and C in Table 5. For our second comparison, we used a group of samples from patients with early PSA recurrence after surgery (<2 years) and a group with no recurrence (>4 years follow up). These classes are A1, B1, C1, and D1 for the no recurrence group and A2, B2, and C2 for the recurrence group (see Table 5). These two sample sets (Gleason and clinical outcome) overlapped somewhat. Our third comparison analyzed only patients with intermediate Gleason (3+4, 4+3, 2+5, 5+2, 2+4, 4+2), to determine whether methylation of our candidates sequences correlates with early recurrence in these patients. Therefore, only categories B1 and B2 were included.

*Tumor Tissue vs. Lymphocytes*

In order to evaluate the diagnostic value of the chip, sixteen lymphocyte samples were included into the study. Prostate cancer tissues and lymphocytes were compared using Wilcoxon rank sum statistics. A ranked display for the ten best amplificates is given in Figure 12. Whereas the lymphocyte group is somewhat homogeneous, the figure displays larger variability for the prostate cancer samples. Differences between groups are significant at the 0.05 level (after 5% false discovery rate correction) for amplificates of CDRN2A, ELK1, GSTP1, RARB, PTGS2, RASSF1, ESR2, ONECUT2, BTG4, SLC35F2, HOXB5, LIMK1, HISTIH4J, SEQ ID NO: 35, EPAS1, NOTCH1, SEQ ID NO: 55, PTPRN2, Q9NP73, MX1, DOCK10, CCND2, ISL1, SNAPC2, GRN, H2AFY2, WDFY3, FOS, FAT, Q86SP6, SLC38A1, SNRPN, GPRK5, FBN2, ARHGEF18, RHOC, KBTBD6, NR2E1, PSD, DRG1, Q8N365, SEQ ID NO: 44, Q96S01, CD37, CMYA3, SEQ ID NO: 61, Q8NCX8 and ZNF566

Candidate markers for Gleason

High Gleason vs. Low Gleason Comparison
Wilcoxon rank statistics were used to analyze differences in methylation profiles of patients classified as high Gleason (Score 8-10) and low Gleason (Score 2-6, no grade 4 or 5 component). The high Gleason class consists of 98 samples and the low Gleason class consists of 135 samples. Figure 12 shows the results of this analysis. For 25 amplificates, the Bonferroni corrected p-value of the Wilcoxon test is below 0.05. For a discussion of biological relevance see section below. Figure 13 displays the methylation matrix of the 10 best markers.
The AUC/sensitivity/specificity of the candidate marker amplificates are given in Table 6.
Figure 13 shows the High Gleason vs. Low Gleason methylation matrix of the 10 markers with best AUC. Each column represents one sample; each row one oligonucleotide (1, 2, or 3 CpG sites each). Oligonucleotides are grouped per marker candidate. The indicated markers are ordered from top to bottom with increasing AUC. On the right side of each marker Bonferroni corrected Wilcoxon p-value and AUC are given. Below the AUC sensitivity at a specificity of - 0.75 are given enclosed in brackets. Methylation data are centered and normalized to one standard deviation for individual oligonucleotides.
The color represents the relative distance of the oligonucleotide methylation status from the mean value. Light grey represents hypomethylated CpGs within an oligonucleotide while dark grey indicates hypermethylated CpGs within an oligonucleotide.

Candidate markers for PSA recurrence

Early Recurrence vs. No Recurrence Comparison

We next analyzed differences in methylation profiles of patients classified as early recurrence (PSA relapse in less than 24 months) and no recurrence (no PSA relapse after at least 4 years).
For three (3) amplificates the Bonferroni corrected p-value of the likelihood-ratio (LR) test is below 0.05. For a discussion of biological relevance see below.
The AUC/sensitivity/specificity of the top marker amplificates are given in Table 7.
Figure 15 shows Early Recurrence vs. No recurrence methylation matrix of the 10 markers with best AUC. Each column represents one sample; each row one oligonucleotide (1, 2, or 3 CpG sites each). Oligonucleotides are grouped per marker candidate. The indicated markers are ordered from top to bottom with increasing AUC. On the right side of each marker Bonferroni corrected Wilcoxon p-value and AUC are given. Below the AUC sensitivity at a specificity of - 0.75 are given enclosed in brackets. Methylation data are centered and normalized to one standard deviation for individual oligonucleotides. The color represents the relative distance of the oligonucleotide methylation status from the mean value. Light grey represents hypomethylated CpGs within an oligonucleotide while dark grey indicates hypermethylated CpGs within an oligonucleotide.

Candidate markers for PSA recurrence in patients with intermediate Gleason Scores

Early Recurrence vs. No Recurrence Comparison

Finally, we analyzed differences in methylation profiles of intermediate Gleason samples from patients classified as early recurrence (PSA relapse in less than 24 months) and no recurrence (no PSA relapse after at least 4 years). Intermediate Gleason included all patients with scores 2+5, 5+2, 3+4, 4+3, 2+4, 4+2, 1+5, 5+1, and these patients are a subset of the group used in section 6.6.2. The majority were Gleason 3+4 or 4+3. Although no amplificates displayed a Bonferroni corrected p-value below 0.05, several markers showed promising AUCs (see Table 8). It is likely this comparison was underpowered due to the small sample set for this comparison.

For a discussion of biological relevance see below.

Co-methylation revealed by microarray analysis

Due to the design of the current chip study, we were able to determine areas within marker fragments that were co-methylated. In this design, at least two oligo pairs, each containing 1, 2 or 3 CpG sites, were included for each marker fragments analyzed. Details of CpG sites targeted in the array analysis can be found in figures 16 onwards. Evidence of co-methylation is apparent in the ranked matrix figures. In the ranked matrix figures from the microarray analysis each marker fragment is grouped horizontally. Since each marker fragment represents one to three amplicons and a minimum of four and up to thirty individual CpG sites, extensive information concerning the methylation status of the fragment can be determined. Consecutive dark grey boxes within the grouping of a fragment in a vertical direction indicate co-methylation of the oligonucleotides (and CpGs within that oligo). These data will be further analysed for the most discriminatory areas within a fragment and this information will be utilized for real-time PCR assay design.

Results Summary

In the primary analysis, a comparison of high and low Gleason samples, 25 markers met the criteria for statistical significance using very conservative statistical methodology. This comparison relies on Gleason as a surrogate indicator of aggressiveness, but it was used as the primary analysis because Gleason information was available for nearly all tumors. Fewer samples were available for the additional analysis, based on time to PSA relapse, but still two markers reached statistical significance.

Discussion

Biological Aspects

MeST Screening

The MeST Screening process was very successful, yielding over 400 candidates. In the real-time PCR and chip studies, the MeST candidates performed well. In the real-time PCR study, three MeSTs outperformed GSTP1. In the chip study, the top five candidates in the Gleason comparison are all MeSTs. Therefore, the screening process contributed valuable candidate markers for distinguishing aggressive and non-aggressive tumors.

Furthermore, MeSTs from all of the screening comparisons were represented in the list of top scoring candidates. The top candidate marker in the Gleason comparison, GPR7, was discovered in two outcome comparisons, the Gleason comparison, and the comparison based on stage. Another top performer, DOCK10, was discovered in the comparison based on prostatic zone. Of the top markers, only ABHD9 was discovered in the comparison of normal tissue adjacent to tumors from patients in the two outcome categories. We conclude that all of the screening genome-wide screening comparisons yielded important candidate markers.

Candidate evaluation by MethyLight

Many candidate MeSTs were chosen for real-time PCR assay development while samples were being collected for the chip study. The assays were pre-screened on a pooled DNA sample from many prostate tumor samples. This step allowed pre-selecting only those assays that could potentially be informative Over one-third of the assays were ruled out at this step. Next, the remaining assays were tested on the DNA from the screening samples in order to prioritize the assays. Then, on the final set of 56 independent samples, six of the seven prioritized assays performed well.

The success of the real-time PCR experiment suggests that there is significant co-methylation in these markers. Therefore, real-time PCR assays, which all require some degree of co-methylation, will be suitable candidates for a final assay choice. The real-time PCR experiment relied heavily on quantification of methylation differences: For nearly all of the assays, the difference between the early recurrence group and the non-recurrence group was a quantitative methylation difference. The final assay type will need strong quantitative abilities.

Candidate evaluation by Methylation Array

The chip experiment was highly successful, demonstrating marker potential for many candidate sequences. In the comparison of high and low Gleason samples, 25 amplicons were significantly different. The vast majority of these are hypermethylated in high Gleason samples. The three candidates analyzed by real-time PCR were among the top six markers in this Gleason comparison. Therefore there is consistency between the two methods of measuring methylation.

The comparison based on patient PSA relapse characteristics had lower sample numbers. Despite these low numbers, we were still able to prove that at least three (3) of our candidate markers can significantly distinguish patients experiencing early relapse from patients not experiencing relapse. In general, methylation is higher in patients experiencing early recurrence. In this comparison, the top three candidates in the real-time PCR study were the top three most significant markers in the chip analysis. The AUCs for GPR7, SEQ ID NO: 35 (downstream of FOXL2), and ABHD9 were 0.72, 0.72, and 0.66 respectively in the chip clinical outcome data and 0.76, 0.75, and 0.70 respectively in the real-time PCR clinical outcome data.

Treatment for patients with high and low Gleason is often clear. Anyone with high Gleason will be recommended for aggressive treatment, including definitive treatment (surgery or radiation) and possibly adjuvant therapy. Patients with low Gleason have the option of deferring definitive treatment. While there are still some uncertainties for these patients, the best options are even less clear for patients with intermediate Gleason levels. Furthermore, the majority of patients being diagnosed with prostate cancer today have intermediate Gleason scores of 6 or 7. These are the patients that can be helped the most by a molecular classification test. The amplicons with the highest AUCs in the comparison based on clinical outcome were GPR7 (AUC=0.72) and SEQ ID NO: 35 (AUC=0.72). When this comparison was restricted to patients with intermediate Gleason scores (1+5, 5+1, 2+4, 4+2, 3+4, 4+3, 2+5, 5+2), the AUC for both of these markers was still 0.72 or greater. These results suggest that a methylation-based assay will provide information even for patients with middle range Gleason scores.

Biology of Marker Genes

Several interesting markers were identified by the real-time PCR and chip studies. One of the real-time PCR markers is a G protein coupled receptor (GPR7; SEQ ID NO:19), however very little is known about the gene product. A second marker from the real-time study is located in a CpG island in the promoter of the gene Abhydrolase Domain containing 9 (ABHD9; SEQ ID NO:37). The closest gene to SEQ ID NO:35 is FOXL2. The MeST is in a CpG island several kilobases downstream of this gene. SEQ ID NO:63 is in an area with several histone genes.
Additional markers emerged in the chip study. NOTCH1 (SEQ ID NO:41) controls a signalling pathway that regulates interactions between adjacent cells. Many labs have studied the role of this gene in carcinogenesis and metastasis. Little is known about many of the candidates, including DOCK10 (SEQ ID NO:16), SEQ ID NO:51, which is in the promoter of a gene called Kelch repeat and BTB (POZ) domain-containing 6, and SEQ ID NO:17, which is located between an EST and B-cell Translocation Gene 4. BTG4 has been shown to have growth inhibitory properties (Buanne et al 2000).
PTGS2 (SEQ ID NO:9) is the only gene previously shown in the literature to be more methylated in prostate tumors of patients who recurred soon after prostatectomy (Yegnasubramanian et al 2004). PTGS2, also known as cyclo-oxygenase (COX2), is a further promising candidate in both the Gleason and the clinical outcome analyses, with AUCs of 0.69 and 0.65 respectively. GSTP1 is the most highly studied methylation marker in prostate cancer, and while there are no published data directly demonstrating its prognostic value, there is some evidence that its methylation correlates with Gleason grade (Maruyama et al 2002). However, this correlation was not confirmed in another study (Woodson et al 2004). In the instant data, GSTP1 methylation significantly correlates with Gleason grade, but the AUC in the clinical outcome comparison is only 0.58.

Medical Aspects

The methylation candidates that have emerged from our Gleason comparison are informative prognostic markers. We have shown that some of these candidates that correlate with Gleason categories can also predict PSA relapse, even in patients with intermediate Gleason scores. Therefore it is likely that our analysis based on Gleason will provide markers that provide additional information to Gleason.
As individual markers, the chip candidates reach 40-60% sensitivity when the specificity is set at 75%. In the real-time study, the sensitivity of three of the markers was higher, reaching 50-60% at a specificity of 85%. The enhanced performance in the real-time might be due to the quantitative abilities of MSP-MethyLight. Approximately 20% of patients experience relapses within 5-10 years after surgery. If this were set as the prevalence of aggressive tumors in the radical prostatectomy population, then a marker such as ours with 50% sensitivity and 85% specificity would have a negative predictive value of 0.87 and a positive predictive value of 0.45. Therefore, a marker with this performance would define a group of patients with only a 13% chance of recurrence after surgery and a group of patients with a 45% chance of recurrence. The first group could just be monitored for PSA rise, and the second group would be candidates for adjuvant therapies.
While these candidates have been studied in prostatectomy samples, they will also be useful for analysis of biopsies. A marker that predicts outcome after prostatectomy correlates with the aggressiveness and metastatic potential of the tumor, and these properties will also be present in the biopsy. After biopsy and staging tests, patients opt for watchful waiting, definitive curative therapy, or a combination of treatments (such as surgery plus "radiation or androgen ablation). A molecular test with high negative predictive value would allow more patients to choose watchful waiting. A molecular test with sufficiently high positive predictive value would select a subset of patients who should not receive radiation or surgery only. Thus, these candidate methylation markers have the potential to reduce both under and over treatment of prostate cancer.

**EXAMPLE 4: Real time quantitative methylation analysis**

Genomic DNA was analyzed using the Real Time PCR technique after bisulfite conversion.

The QM assay (= Quantitative Methylation Assay) is a Real-time PCR based method for quantitative DNA methylation detection. The assay principle is based on non-methylation specific amplification of the target region and a methylation specific detection by competitive hybridization of two different probes specific for the CG or the TG status, respectively. For the present study, TaqMan probes were used that were labeled with two different fluorescence dyes ("FAM" for CG specific probes, "VIC" for TG specific probes) and were further modified by a quencher molecule ("TAMRA" or "Minor Groove Binder/non-fluorescent quencher").

Evaluation of the QM assay raw data is possible with two different methods:

1. Measuring absolute fluorescence intensities (FI) in the logarithmic phase of amplification Difference in threshold cycles (Ct) of CG and TG specific probe.

In the following series of quantitative methylation assays the amount of sample DNA amplified is quantified by reference to the gene GSTP1 to normalize for input DNA. For standardization, the primers and the probe for analysis of the GSTP1 gene lack CpG dinucleotides so that amplification is possible regardless of methylation levels. As there are no methylation variable positions, only one probe oligonucleotide is required.

The reactions are calibrated by reference to DNA standards of known methylation levels in order to quantify the levels of methylation within the sample. The DNA standards were composed of bisulfite treated phi29 amplified genomic DNA (i.e. unmethlyated), and/or phi29 amplified genomic DNA treated with Sss1 methylase enzyme (thereby methylating each CpG position in the sample), which is then treated with bisulfite solution. Seven different reference standards were used with 0%, (i.e. phi29 amplified genomic DNA only), 5%, 10%, 25%, 50%, 75% and 100% (i.e. phi29 Sss1 treated genomic only).

Bisulfite treatment

Bisulfite treatment was carried out based on the method disclosed by Olek et al. Nucleic Acids Res. 1996 Dec 15;24(24):5064-6, and optimized to the applicant's laboratory workflow.

Quantification Standards

The reactions are calibrated by reference to DNA standards of known methylation levels in order to quantify the levels of methylation within the sample. The DNA standards were composed of bisulfite treated phi29 amplified human genomic DNA (Promega) (i.e. unmethlyated), and/or phi29 amplified genomic DNA treated with Sss1 Methylase enzyme (thereby methylating each CpG position in the sample), which is then treated with bisulfite solution. Seven different reference standards were used with 0%, (i.e. phi29 amplified genomic DNA only), 5%, 10%, 25%, 50%, 75% and 100% (i.e. phi29 Sss1 treated genomic only). 2000 ng batches of human genomic DNA (Promega) were treated with bisulfite. To generate methylated MDA DNA, 13 tubes of 4.5 μg MDA-DNA (700 ng/μl) was treated with Sss1.

Control assay

The GSTP1-C3 assay design makes it suitable for quantitating DNAs from different sources, including fresh/frozen samples, remote samples such as plasma or serum, and DNA obtained from archival specimen such as paraffin embedded material. The following oligonucleotides were used in the reaction to amplify the control amplificate:

| | |
|---|---|
| Control Primer1: | GGAGTGGAGGAAATTGAGAT (SEQ ID NO:966) |
| Control Primer2: | CCACACAACAAATACTCAAAAC (SEQ ID NO:967) |
| Control Probe: | FAM-TGGGTGTTTGTAATTTTTGTTTTGTGTTAGGTT-TAMRA (SEQ ID NO:968) |
| Cycle program (40 cycles): | 95°C, 10 min |
| | 95°C, 15 sec |
| | 58°C, 1 min |

Assay design and reaction conditions

Two assays were developed for the analysis of the gene PITX2(SEQ ID NO:961) Assay 1:

Primers: GTAGGGGAGGGAAGTAGATGTT (SEQ ID NO:969)
TTCTAATCCTCCTTTCCACAATAA (SEQ ID NO:970)

Probes: FAM-AGTCGGAGTCGGGAGAGCGA-TAMRA (SEQ ID NO:971)
VIC-AGTTGGAGTTGGGAGAGTGAAAGGAGA -TAMRA (SEQ ID NO:972)

Amplicon (SEQ ID NO:973):

GtAGGGGAGGGAAGtAGATGttAGCGGGtCGAAGAGTCGGGAGtCGGAGtCGGGAGAGCGAAAGGAG AGGGGAttTGGCGGGGtAtTTAGGAGttAAtCGAGGAGtAGGAGtACGGAtTttAtTGTGGAAAGGAGGAttA GAA

Length of fragment: 143 bp

Positions of primers, probes and CpG dinucleotides ar highlighted.

| | |
|---|---|
| PCR components (supplied by Eurogentec) : | 3 mM MgCl2 buffer, 10x buffer, Hotstart TAQ, 200 μM dNTP, 625 nM each primer, 200 nM each probe |
| Cycle program (45 cycles): | 95°C, 10 min |
| | 95°C, 15 sec |
| | 62°C, 1 min |

Assay 2 :

Primers: AACATCTACTTCCCTCCCCTAC (SEQ ID NO:974)
GTTAGTAGAGATTTTATTAAATTTTATTGTAT (SEQ ID NO:975)

Probes: FAM-TTCGGTTGCGCGGT-MGBNQF (SEQ ID NO:976)
VIC-TTTGGTTGTGTGGTTG- MGBNQF (SEQ ID NO:977)

Amplicon (SEQ ID NO:978):

GTtAGtAGAGATTttAttAAATttAtTGtAtAGTGGCGCGCGGGCGGtCGGtCGAGttCGGttGCGCGGtTGGC GATttAGGAGCGAGtAtAGCGttCGGGCGAGCGtCGGGGGGAGCGAGtAGGGGCGACGAGAAACGAGG tAGGGGAGGGAAGtAGATGtt

Length of fragment: 164 bp
The positions of probes, primers and CpG positions are highlighted.

The probes cover three co-methylated CpG positions.

| | |
|---|---|
| PCR components (supplied by Eurogentec): | 2,5 mM MgCl2 buffer, 10x buffer, Hotstart TAQ, 200 μM dNTP, 625 nM each primer, 200 nM each probe |
| Program (45 cycles): | 95°C. 10 min |
| | 95°C,15 sec |
| | 60°C, 1 min |

The extent of methylation at a specific locus was determined by the following formulas: Using absolute fluorescence intensity: methylation rate = 100 * I (CG) / (I(CG)) + I(TG) (I = Intensity of the fluorescence of CG-probe or TG-probe)
Using treshold cycle Ct : methylation rate = 100 * CG (CG + TH) = 100/ (1 + TG/CG) = 100/ (1+2Λdelta(ct))

(assuming PCR efficiency E=2; delta (Ct)= Ct (methylated) - Ct (unmethylated) )

The main goal of this phase of the investigation was to confirm the significance of previously identified marker candidates and optimize methylation cut-offs. The markers should be suitable to split patients who undergo prostatectomy into two groups: one with a high chance of PSA recurrence and one with a low chance of PSA recurrence. In addition, the markers should provide additional information to Gleason grade analysis. Markers meeting these criteria will have an important clinical role in selection of prostatectomy patients for adjuvant therapy.

The applicant had previously identified several markers with significantly higher methylation levels in patients who experienced PSA recurrence within 24 months of surgery compared to patients who did not experience PSA recurrence (see above Examples 1 to 4). Six of these markers were transferred to a real-time platform (QM Assay). These assays were used to analyze the methylation levels of 612 paraffin embedded prostatectomy samples from a cohort of node-negative patients from three institutions.

The primary aim of the invention was to provide markers that can differentiate between patients with low chance for PSA recurrence after surgery and those with a high chance for PSA recurrence. The performance of these markers as compared to traditional prognostic indicators such as Gleason grading and stage information is also provided. It is a further aim of the present invention to determine where the markers are most informative in relation to current clinical prognostic assessment and accordingly provide particularly preferred use embodiments of the present invention. It is particularly preferred that a molecular test according to the present invention is combined, either formally or informally, with information from other prognostic sources, in particular Gleason grading.

Methods: QM Assay Description

Each QM-assay was developed to enhance performance without drastically altering standard conditions in order to allow future multiplexing. Primer and probe concentrations, $MgCl_2$ concentration and annealing temperature were optimized under fixed buffer and polymerase conditions. The assays were designed and optimized to ensure quantitative methylation analysis of each marker between 10 and 100 percent methylation. The assay products were checked on an agarose gel and no undesired products were detected. The results of the optimization procedure are shown in the following tables.

Sample Set

Paraffin-embedded prostatectomy tissue samples from 605 patients were analyzed. The samples were provided by the Baylor College of Medicine SPORE, Stanford University Department of Urology, and Virginia Mason Hospital in Seattle. The samples from Stanford and Virginia Mason were prepared by first finding the surgical block with the highest percent tumor, then sectioning the block. Three tubes were prepared, each with three 10 micron thick sections. The procedure was slightly different at Baylor. A core of tissue was removed from the tumor within the prostatectomy block, and then this core was cut into 10 micron sections. Ten sections were included into each of three tubes.

An adjacent section was mounted on a slide and H&E stained for histological analysis. A pathologist reviewed these slides for an independent determination of Gleason grading and percent tumor. The Gleason results were used for all analyses in this report. The original provider Gleason values are available, but they were not used for analysis due to known and hypothetical biases among the providers. Stanford, for instance, uses a percentage Gleason 4/5 for reporting grade, while the other two providers use the traditional system. The measured Gleason values provided an independent and uniform measurement.

A few samples were found to have no tumor cells on the H&E slide, and these patients were omitted from the analysis. In addition, we found a few patients that did not have a PSA nadir after surgery. These patients were also excluded from the study. In total, 612 patients were included in the data analysis.

Due to their coring technique, the percent tumor of the samples provided by Baylor were higher than the other providers. All patients, aged 40-80, undergoing surgery at the three institutions during certain years were included in the study, with the exception of patients who received neo-adjuvant or adjuvant therapy (before PSA rise) and patients with positive nodes at the time of surgery. For Baylor, the time period was 1993-1998, for Virginia Mason it was 1996-2000, and for Stanford it was 1996-1999.

The overall cohort is similar to other prostatectomy cohorts described in the literature, such as the cohort collected by William Catalona and described in 2004 (Roehl *et al.*). The patient cohorts from each provider are similar for nearly all clinical parameters. One exception is the type of recurrence. While other institutions typically wait until the patient's PSA rises to 0.2 ng/ml or higher after surgery, the Stanford Department of Urology treats many patients when their PSA rises to 0.05. Therefore, Stanford has a higher rate of recurrence based on the decision to treat criteria and a lower rate of recurrence based on the PSA level (0.2ng/ml) criteria. See section 6.1 for a summary of the event definition criteria. Figure 89 provides a histogram of follow-up times for the patient cohort (all three providers included). The white bars consist of the patients who did not have a recurrence before they were censored, and the shaded bars consist of the patients who experienced recurrence. By selecting patients who received surgery from 1993-2000, we have ensured that the median follow-up time of the cohort (66 months) is long enough to have a significant number of patients who have relapsed.

For deparaffination, the 627 provided PET samples were processed directly in the tube in which they were delivered by the providers. One ml (Virginia Mason and Baylor) or 1.8 ml (Stanford) of limonene was added to each tube and incubated at room temperature for 10 minutes in a thermomixer with occasional vortexing. The samples were centrifuged at 16,000 x g for 5 minutes. The limonene supernatant was removed, and if no pellet was detected, centrifugation was repeated at higher speed and the remaining limonene was removed. For samples from Stanford, the deparaffination process was repeated once with 1.6 ml of limonene to get rid of residual paraffin.

For lysis of the tissue, 190 μl lysis buffer and 20 μl proteinase K was added to each deparaffinated sample. For Stanford samples, 570 μl lysis buffer and 60 μl proteinase K was used. After vortexing, samples were centrifuged briefly and incubated on a thermoshaker at 60˚C for 40 hours. After the incubation, samples were checked to ensure that lysis was complete, and the proteinase was then inactivated at 95˚C for 10 minutes. If the lysed samples were not directly used for DNA extraction, they were stored at -20˚C.

The lysates were randomized based on the sample provider and PSA recurrence. The DNA was isolated using a QIAGEN DNeasy Tissue kit with a few modifications. 400 μl buffer AL/E was distributed to collection tubes and 200 μl of lysate were added. The samples were mixed by shaking for 15 seconds.

The lysate/buffer mixtures were applied to the 96-well DNeasy plate columns. The plate was sealed and centrifuged at 5790xg for 10 minutes. The columns were washed once with 500 μl of AW1 and then 500 μl AW2. The DNA was eluted with 120 μl buffer AE. Therefore, the final volume of extracted DNA was approximately 120μl. The DNA was stored at -20˚C.

<u>Bisulfite Treatment</u>

The CFF real-time PCR assay was used to quantify the DNA concentration of the samples after extraction.

**CFF sequence:**

**TAAGAGTAATAATGGATGGATGATGGATAGATGAATGGATGAAGAAAGAAAGGATGAGTGAGAGAA AGGAAGGGAGATGGGAGG (84bp) (SEQ ID NO: 979)**

| | |
|---|---|
| CFF-Forward primer | TAAGAGTAATAATGGATGGATGATG (SEQ ID NO: 980) |
| CFF-Reverse primer | CCTCCCATCTCCCTTCC (SEQ ID NO: 981) |
| CFF TaqMan probe | ATGGATGAAGAAAGAAAGGATGAGT (SEQ ID NO: 982) |

We adjusted the concentration of each genomic DNA sample so that 1ug of CFF1 measured DNA was present in 44 μl. The bisulfite treatment of genomic DNA derived from paraffin embedded tissue was performed using a 96 well protocol. Forty-four μl genomic DNA (with approximately 1μg of amplifiable DNA), 83 μl 4.9M bisulfite solution (pH 5.45-5.5), and 13 μL DME solution were pipetted into the wells of the plate. The samples were thoroughly mixed then placed in a thermocycler with the following program:

- 5:00 min denaturation of DNA at 99˚C
- 22:00 min incubation at 60˚C
- 3:00 min denaturation of DNA at 99˚C
- 1:27:00 hours incubation at 60˚C
- 3:00 min denaturation of DNA at 99˚C
- 2:57:00 hours incubation at 60˚C
- Cooling at 20˚C

After the incubations, each sample was divided into two 70 μL aliquots. Each aliquot was combined with 280 μL of prepared Buffer AVL/Carrier RNA and 280 μL ethanol. The wells were sealed and the samples were mixed vigorously for 15 seconds. The plate was incubated for 10 minutes at room temperature. The first aliquot was applied to the OIAamp 96 plate and the plate was centrifuged for four minutes at 5790 x g. The process was repeated with the second aliquot so that both aliquots were applied to the same binding column. The columns were washed with 500 μL buffer AW1, then 500 μL 0.2 M NaOH, and then twice with 500 μL buffer AW2. The DNA was eluted with 100 μL elution buffer (Qiagen) pre-heated to 70 deg C. The bisDNAs were stored at -20°C.

The bisulfite treated DNA samples were stored in 8 x 96 well plates (plate 01-08). The samples and controls were combined onto two 384-well PCR reaction plates for each QM assay. Each QM assay plate contained the samples of 4 x 96 well plates (85 wells actually used per plate) and 1 x96 well plate with standard DNA (7 mixtures of the calibration DNA and water for the no template control PCR reaction).

The QM assay plates were run three times.

The 384-well PCR plates were pipetted with the TECAN workstation. The pipetting program transferred first 10 μl of the mastermix and then 10 μl of the respective DNA into the designated well. The master mix was pipetted in a falcon tube and distributed to 8 x 500 μl screw cap vials for automatic pipetting with TECAN workstation.

All QM assays were run on an ABI TAQMAN 7900HT real-time device (SDS 2.2. software) with a reaction volume of 20 μl. PITX2 and CCND2 assays were run with 9600 emulation, and the other assays were not. An automatic sample setup was used to transfer the correct sample names and detector/reporter dyes to the TAQMAN software. The cycling conditions were manually adjusted and ROX was used as passive reference dye. All 384 well PCR plates we analyzed with the SDS2.2 software using the manual analysis settings (baseline setting with start and stop values and manual threshold) to produce results files for each run individually.

Methods: Evaluation of Marker Performance

Definition of Events

After a successful prostatectomy on a patient with non-metastatic disease, there should be no prostate cells left in his body and therefore his PSA levels should drop to zero. A patient's PSA levels are typically measured every 6-12 months after surgery to ensure that the patient remains free of prostate cancer. If PSA becomes detectable and rises to a certain level, the doctor and patient may decide on additional therapy. Therefore, the return and rise of PSA levels are the primary indication of disease recurrence.

A post-surgical PSA relapse is typically indicated by either a gradual or rapid rise in levels over a series of sequential tests. Depending on the clinical characteristics of the patient or the approach of the institution, patients may be treated as soon as PSA is detected, when it reaches a certain threshold, or when clinical symptoms accompany the PSA rise. Most institutions consider a PSA level of 0.2 ng/ml to be significant, and if a patient's PSA reaches this level and is confirmed to be rising in subsequent tests, he will be offered additional therapy. Stanford Department of Urology, one of the sample providers, considers 0.05 ng/ml to be a PSA recurrence, and considers treatment for patients when their PSA reaches this level.

An event in this study includes all PSA-based recurrences. A PSA level of 0.2 ng/ml, confirmed in subsequent tests, has been demonstrated to provide the best sensitivity and specificity for detection of recurrence (Freedland *et al.* 2003). Rise of PSA to this level normally precedes any development of clinical recurrence; therefore, nearly all of the patients in this study are free of clinical recurrence at the time of PSA recurrence. Because Stanford often treats patients with PSA recurrence before they reach this cut-off of 0.2ng/ml, many of their recurrence patients would be censored in the present study if the PSA level of 0.2ng/ml was the only considered event. Therefore, patients from any of the three institutions who receive therapy due to PSA levels are also considered an event in this study. To summarize, an event is defined in the present study as any rise in PSA to 0.2 ng/ml (confirmed in subsequent test) OR a decision to treat the patient based on PSA criteria.

Raw QM Data Processing

All analyses in this report are based on the CT evaluation. Assuming optimal real-time PCR conditions in the exponential amplification phase, the concentration of methylated DNA ($C_{meth}$) can be determined by

$$C_{meth} = \frac{100}{1 + 2^{(CT_{CG} - CT_{TG})}} [\%],$$

where
$CT_{CG}$ denotes the threshold cycle of the CG reporter (FAM channel) and
$CT_{TG}$ denotes the threshold cycle of the TG reporter (VIC channel).

The thresholds for the cycles were determined by visual inspection of the amplification plots (ABI PRISM 7900 HT Sequence Detection System User Guide). The values for the cycles ($CT_{CG}$ and $CT_{TG}$ ) were calculated with these thresholds by the ABI 7900 software. Whenever the amplification curve did not exceed the threshold, the value of the cycle was set to the maximum cycle e.g. 50.

The R software package, version 2.2. (Gentleman and Ihaka 1997), was used for the statistical analysis.

In addition, we used the "survival" package, version 2.11-5 (http://cran.at.r-project.org/src/contrib/Descriptions/survival.html), for survival analysis.

Proprietary code was used for k-fold-cross validation, ROC analysis and plot functions. Each dataset is represented in a proprietary data object, called "Annotated Data Matrix" (ADM). This data object contains the measurements after quality control and averaging, as well as all necessary annotations for the samples and assays.

QM Assay calibration curves

A series of mixtures of methylated MDA-DNA and unmethylated MDA-DNA, ranging from 0 to 100 percent methylated, were included in triplicate on each QM PCR plate. These DNAs were used to ensure uniform QM assay performance on all PCR plates. All assays showed strong quantitative abilities between 10 and 100%, and some assays were able to consistently distinguish 5% methylated DNA from unmethylated DNA.

Statistical Methods

After quality control, each assay was statistically analyzed.

Cox Regression

The relation between recurrence-free survival times (RFS) and covariates were analyzed using Cox

Proportional Hazard models (Cox and Oates 1984; Harrel 2001).

The hazard, i.e. the instantaneous risk of a relapse, is modeled as

$$h(t \mid x) = h_0(t) \cdot exp(\beta x) \qquad (3)$$

and

$$h(t \mid x_1, \ldots, x_k) = h_0(t) \cdot exp(\beta_1 x_1 + \ldots + \beta_k x_k) \qquad (4)$$

for univariate and multiple regression analyses, respectively, where $k$ is 10, $m$ is 100$t$ is the time measured in months after surgery, $h_0(t)$ is the (unspecified) baseline hazard, $x_i$ are the covariates (e.g. measurements of the assays) and $\beta_i$ are the regression coefficients (parameters of the model). $\beta_i$ will be estimated by maximizing the partial likelihood of the Cox Proportional Hazard model

Likelihood ratio tests are performed to test whether methylation is related to the hazard. The difference between 2Log(Likelihood) of the full model and the null-model is approximately $\chi^2$-distributed with $k$ degrees of freedom under the null hypotheses $\beta_1 = ... = \beta_k = 0$.

The assumption of proportional hazards wre evaluated by scaled Schoenfeld residuals (Themau and Grambsch 2000). For the calculation, analysis and diagnostics of the Cox Proportional Hazard Model the R functions "coxph" and "coxph.zph" of the "survival" package are used.

Stepwise Regression Analysis

For multiple Cox regression models a stepwise procedure (Venables and Ripley 1999; Harrel 2001) was used in order to find sub-models including only relevant variables. Two effects are usually achieved by these procedures:

- Variables (methylation rates) that are basically unrelated to the dependent variable (DFS/MFS) are excluded as they do not add relevant information to the model.
- Out of a set of highly correlated variables, only the one with the best relation to the dependent variable is retained. Inclusion of both types of variables can lead to numerical instabilities and a loss of power. Moreover, the predictor's performance can be low due to over-fitting.

The applied algorithm aims at minimizing the Akaike information criterion (AIC). The AIC is related to the performance of a model, smaller values promise better performance. Whereas the inclusion of additional variables always improves the model fit and thus increases the likelihood, the second term penalizes the estimation of additional parameters. The best model will present a compromise model with good fit and usually a small or moderate number of variables. Stepwise regression calculation with AIC are done with the R function "step".

Kaplan-Meier Survival Curves and Log-Rank Tests

Survival curves were estimated from RFS data using Kaplan-Meier estimator for survival (Kaplan and Meier, 1958). Log-rank tests (Cox and Oates 1984) are used to test for differences of two survival curves, e.g. survival in hyper- vs. hypomethylated groups. In addition, a variant of the Log-rank test usually referred to as the Generalized Wilcoxon test was applied (for description see Hosmer and Lemeshow 1999). For the Kaplan-Meier analysis the functions "survfit" and "survdiff" of the "survival" package are used.

Independence of single markers and marker panels from other covariates

To check whether the present markers give additional and independent information, other relevant clinical factors were included in the Cox Proportional Hazard model and the p-values for the weights for every factor were calculated (Wald-Test) (Thernau *et al.* 2000). For the analysis of additional factors in the Cox Proportional Hazard model, the R function "coxph" is used.

Multiple Test Corrections

No correction for multiple testing was done.

Density Estimation

For numerical variables, kernel density estimation was performed with a Gaussian kernel and variable bandwidth. The bandwidth is determined using Silverman's "rule-of-thumb" (Silverman 1986). For the calculation of the densities the R function "density" is used.

Analysis of Sensitivity and Specificity

The method of calculating sensitivity and specificity using the Bayes-formula was based on the Kaplan-Meier estimates (Heagerty *et al.* 2000) for the survival probabilities in the marker positive and marker negative groups for a given time $T_{Threshold}$. The ROCs were calculated for different reference times $T_{Threshold}$ (3 year, 4 years, 5 years, 6 years).

k-fold Crossvalidation

For the analysis of model selection and model robustness k-fold crossvalidation (Hastie *et al*. 2001) was used. The set of observations is randomly split into k chunks. In turn, every chunk was used as a test set, whereas the remaining k-1 chunks constitute the training set. This procedure is repeated m times.

Results

The 605 samples were processed as described above. All samples were analyzed with six marker QM assays with three replicates. The data were filtered for quality control, and analyzed as described in the methods section. The clinical performance of each marker is summarized below and the Kaplan-Meier survival curves and ROC curves according to figures 90 to 95. P-values for comparison of survival curves reported in the graphs are based on the ordinary Log-rank test. The results of using the Generalized Wilcoxon test are essentially the same (data not shown).
The performance of the markers was first examined using the median methylation level as a cut-off. Since this cut-off was fixed before looking at the data, the p values can be used to judge the performance of the markers. Any marker with a significant p value using the median methylation as a cut-off is considered to be validated. The median methylation level might not be the best cut-off for all markers, and for these markers the prognostic separation can be further optimized by choosing the methylation cut-off that results in the lowest p value. Since the cut-off is optimized specifically for p value, the p value no longer can be used to indicate statistical significance.

For judging the significance of the marker performance using the median methylation as a cut-off, we used a p value of 0.005 (assuming correction for 6 comparisons). Based on p-value (less than 0.008) and event separation, PITX2 is the strongest candidate. GPR7along with SEQ ID NO:35. Therefore, these two markers are considered validated markers of post-surgical prostate cancer prognosis., SEQ ID NO: 63 was not significant using the median methylation level as a cut-off (p values 0.018 and 0.0059), but perform well when the methylation cut-off is optimized. See Table 17 for results.

Figure 90 A shows the Kaplan-Meier survival analysis of the PITX2 marker of the 585 patient samples that passed the quality control filter using the optimized methylation cut-off value (13.5%). Figure 90B shows the Kaplan-Meier survival analysis of the PITX2 marker using the predefined median methylation value as a cut-off, the p-value was 0.000017. Figure 90C shows the ROC curve analysis of the PITX2 marker after 5 years of follow-up. The median methylation cut-off is marked as a triangle, and the optimized methylation cut-off is shown as a diamond. The AUC was 0.64. Figure 91 A shows the Kaplan-Meier survival analysis of the GPR7 marker of the 596 patient samples that passed the quality control filter using the optimized methylation cut-off value (18.06%). Figure 91B shows the Kaplan-Meier survival analysis of said marker using the predefined median methylation value as a cut-off, the p-value was 0.0016. Figure 91C shows the ROC curve analysis of said marker after 5 years of follow-up. The median methylation cut-off is marked as a triangle, and the optimized methylation cut-off is shown as a diamond. The AUC was 0.64.
Figure 92 A shows the Kaplan-Meier survival analysis of the SEQ ID NO:63 marker of the 599 patient samples that passed the quality control filter using the optimized methylation cut-off value (5.79%).
Figure 92B shows the Kaplan-Meier survival analysis of said marker using the predefined median methylation value as a cut-off, the p-value was 0.018. Figure 92C shows the ROC curve analysis of said marker after 5 years of follow-up. The median methylation cut-off is marked as a triangle, and the optimized methylation cut-off is shown as a diamond. The AUC was 0.60.
Figure 93 A shows the Kaplan-Meier survival analysis of the SEQ ID NO:35 marker of the 598 patient samples that passed the quality control filter using the optimized methylation cut-off value (36.77%).
Figure 93B shows the Kaplan-Meier survival analysis of said marker using the predefined median methylation value as a cut-off, the p-value was 0.059. Figure 93C shows the ROC curve analysis of said marker after 5 years of follow-up. The median methylation cut-off is marked as a triangle, and the optimized methylation cut-off is shown as a diamond. The AUC was 0.61.
Figure 94 A shows the Kaplan-Meier survival analysis of the ABHD9 marker of the 592 patient samples that passed the quality control filter using the optimized methylation cut-off value (28.41%). Figure 94B shows the Kaplan-Meier survival analysis of said marker using the predefined median methylation value as a cut-off, the p-value was 0.018. Figure 94C shows the ROC curve analysis of said marker after 5 years of follow-up. The median methylation cut-off is marked as a triangle, and the optimized methylation cut-off is shown as a diamond. The AUC was 0.58.
Figure 95 A shows the Kaplan-Meier survival analysis of the CCND2 marker of the 604 patient samples that passed the quality control filter using the optimized methylation cut-off value (2.22%). Figure 95B shows the Kaplan-Meier survival analysis of said marker using the predefined median methylation value as a cut-off, the p-value was 0.22. Figure 95C shows the ROC curve analysis of said marker after 5 years of follow-up. The median methylation cut-off is marked as a triangle, and the optimized methylation cut-off is shown as a diamond. The AUC was 0.61.

Evaluation of Markers on Clinical Subsets of the Patients

Several clinical prognostic factors are commonly used for assessing prostate cancer. Histological analysis of the tumor with quantification of the tumor differentiation state using the Gleason grading system is a particularly important prognostic indicator in current clinical practice. The analysis was continued by determining whether the markers could improve Gleason analysis by subdividing patients within a Gleason category. We also investigated whether the markers could add information to other prognostic indicators, such as nomogram risk estimation (Han *et al*. 2003) and disease stage.
For these analyses, we used Kaplan-Meier analysis to determine whether our markers are still informative on population sub-groups, and Cox regression analysis to determine whether the markers provide information independent of the prognostic clinical variables. Gleason score (using Charite Gleason calls) was divided into three groups (6 or lower, 7, and 8 through 10), stage was divided into two groups (T2/organ-confined and T3/non-organ confined), PSA was divided into four groups (0 to 4 ng/mi, 4 to 10 ng/ml, 10 to 20 ng/ml, and greater than 20 ng/ml), and nomogram estimation of 5 year PSA-free survival was divided into two groups (90 to 100% and 0 to 89%).

PITX2

With Cox regression modeling, PITX2 is a valuable prognostic marker independent of other clinical prognostic information (Table 18). In other words, PITX2 methylation adds more information to Gleason than either PSA or disease stage. The hazard ratio for PITX2 is 2.2. In the survival analysis of sub-groups, PITX2 has the potential to be a significant marker for all prostate cancer patients.

It is particularly interesting to see strong separation within the patient sub-group with organ-confined disease (Figure 96). Patients with organ-confined disease (T2) should be cured by surgery. Those that are not cured by surgery must have had some cells leave the prostate before surgery, and therefore had tumor cells with aggressive characteristics early in the development. PITX2 can separate the T2 group into a hypomethylated group with a very small chance for recurrence (~5%) and a hyper-methylated group with a prognosis more like T3 patients.

Figure 96 shows the survival analysis of PITX2 performance on sub-populations based on stage. The upper left plot shows the performance of disease stage as a prognostic marker. The upper right plot shows the performance of PITX2 on pT2 patients. The lower left plot shows the performance of PITX2 on pT3 patients.

PITX2 is also capable of stratifying patients within Gleason sub-categories. Figure 97 shows that survival analysis on low Gleason patients (Score 5 or 6) and high Gleason patients (Score 8, 9, or 10) results in low p values. Patients with high Gleason scores are currently candidates for clinical trials on post-surgical adjuvant therapies. But the PITX2 values suggest that this is not a uniform group. PITX2 hypomethylated, high Gleason patients have 85% probability of disease free survival at ten years, while hypermethylated high Gleason patients have a very low chance (~35%). These patients with high likelihood for disease recurrence are the patients who should be selected for adjuvant therapy or clinical trials.

Figure 97 shows the survival analysis of PITX2 performance on sub-populations based on Gleason score categories. The upper left plot shows the performance of Gleason score as a prognostic marker. Gleason 5 and 6 patients are marked A, Gleason 7 patients are marked B, and Gleason 8, 9, and 10 patients are marked C. The upper right plot shows the performance of PITX2 on Gleason 5 and 6 patients. The lower left plot shows the performance of PITX2 on Gleason 7 patients. The lower right plot shows the performance of PITX2 on Gleason 8, 9, and 10 patients.

Prostate cancer nomograms are created based on large cohorts of patients. They mathematically combine information from stage, Gleason, and pre-operative PSA levels into one prognostic indicator. As Figure 98 shows, the nomogram by itself is very strong. But PITX2 is capable of further sub-dividing the patients.

Figure 98 shows the survival analysis of PITX2 performance on sub-populations based on nomogram risk estimation. The upper left plot shows the performance of the nomogram as a prognostic marker. The upper right plot shows the performance of PITX2 on patients with a 90% chance of 5-year PSA-free survival according to the nomogram. The lower left plot shows the performance of PITX2 on patients with less than 90% chance of 5-year PSA-free survival according to the nomogram.

SEQ ID NO:63

With Cox regression analysis, SEQ ID NO:63 is a valuable prognostic marker independent of other clinical prognostic information (Table 19). The hazard ratio is 2.9. In the survival analysis of sub-groups, SEQ ID NO:63 seems to have the potential to be a significant marker for some sub-groups, such as high Gleason patients (Figure 99) and patients with poor nomogram-based prognosis (Figure 100).

Figure 99 shows the survival analysis of SEQ ID NO:63 performance on Gleason score 8, 9, and 10 patients.

Figure 100 shows the survival analysis of SEQ ID NO:63 performance on patients with less than 90% chance of 5-year PSA-free survival according to the nomogram.

SEQ ID NO:35 is a marker for some sub-groups, such as pT2 patients (Figure 101).

Discussion

PITX2, SEQ ID NO:35, and GPR7 all show significant prognostic information when the median methylation level is used as a cut-off. Setting the methylation cut-off even higher than the median improves the performance of these three markers. This has the effect of decreasing the marker positive group and increasing the specificity of the test. The median methylation level is not optimal for SEQ ID NO: 63. Instead, a lower cut-off more clearly separates the good and bad prognosis groups for this marker. The optimized methylation cut-off values for these four markers all fall in the range for which their respective assays are technically well suited.

The patients whose samples were analyzed in this study are representative of the population who would be targeted for a prostatectomy test. Therefore, it is possible to speculate on the information these markers could provide for future patients. PITX2, for example, has a sensitivity of around 60% and a specificity of 70%. In the Kaplan-Meier analysis in Figure 90, the marker positive group has approximately three times the risk of recurrence after ten years that the marker negative group has. In Figure 97, Gleason 8-10 patients that are positive for PITX2 have a 65% chance for PSA recurrence in 10 years. In contrast, the Gleason 8-10 patients who were marker negative had only a 15% chance of PSA relapse. The addition of the methylation marker information to the Gleason stratification will allow clinicians to identify a poor prognosis sub-group who can most benefit from adjuvant therapy. If these methylation markers are incorporated into the patient selection procedure for adjuvant therapy clinical trials, clinicians may begin to see a clear benefit to the addition of early adjuvant treatments for poor prognosis patients.
In addition to adding information to Gleason, PITX2 and some of the other markers can also stratify patients with organ-confined disease. Patients with disease that is truly confined to the organ will be cured by complete removal of the organ. Patients with disease that appears to be confined to the organ, but have undetected micrometastases, will not be cured by surgery. These two groups of patients, both with small operable lesions, have tumors with very different capacities for metastases. PITX2 and some of the other markers seem to be detecting these underlying differences in basic tumor aggressiveness.
The ability of these markers to add information to currently used markers is essential. Gleason and staging already provide significant prognostic information, a new test that would not replace but complement these traditional sources of information is both more valuable and more likely to be readily adopted in clinical practice.
In the analysis of the markers on sub-groups of patients, the markers often seemed strongest on patients with poor prognosis based on traditional clinical variables. Gleason 8-10 patients and patients with low nomogram probability for PSA free survival are well stratified by the present markers into good and poor prognosis groups. For a prostatectomy test, these are the ideal patients to target, since the test would be used to select a group of poor prognosis patients who can most benefit from adjuvant therapy. For T3 patients (non-organ-confined disease), the marker SEQ ID NO: 63 is preferred. Overall, this analysis demonstrates that the present markers are especially well suited for identifying poor prognosis patients.

**Example 6: Test of assays on paraffin embedded tissue.**

In the following analysis, methylation within paraffin embedded prostate tissue samples was analysed by means of the assays shown in Table 12 for the analysis of SEQ ID NO: 19, 35, 37 and 63. This was then compared to the same measurement carried out upon the frozen samples described in Example 2.

Samples

The samples were paraffin embedded prostatectomy samples or fresh frozen tissues as described in Example 3. The samples were sectioned, the tissue was lysed, the DNA was then extracted and bisulfite converted.
309 paraffin embedded samples were available, of these all samples with at least 1 ng of DNA per PCR were included in the analysis, with between 1 and 10ng of DNA per PCR being used.

Reagents:

1 x Taqman PCR Buffer A
0.25mM dNTPs
3.5mM $MgCl2$
900nM each primer
300nM probe
1 unit AmpliTaq Gold

Thermal Cycling profile:

Step 1. 95˚C->10min (Taq Activation)
Repititions: 1

Step 2.95˚C->15s (Denaturation)

63,0˚C->1 min (Annealing/Extension)
Repititions:50

The following categories were compared

1. Early biochemical relapse (PSA relapse after prostatectomy in less than 24 months) vs. no biochemical relapse (no significant rise in PSA during at least 4 years of PSA monitoring after surgery)
132 samples were available in the non-relapse group and 59 samples available in the early relapse group.

2. High Gleason (score =8-10) vs Low Gleason (Score =2-6 with no grade 4 or 5 component) 59 samples were available in the high Gleason group and 64 samples available in the low Gleason group.

Results

Results are shown in Figures 102 to 125, and were calculated using the Wilcoxon test as described above.

Figure 102 shows the detected amplificate in both frozen and PET samples in the early biochemical relapse vs. no biochemical relapse comparisons using the assay of SEQ ID NO:19 shown in Table 12.
Figure 103 shows the detected amplificate in both frozen and PET samples in the early biochemical relapse vs. no biochemical relapse comparisons using the assay of SEQ ID NO:63 shown in Table 12.
Figure 104 shows the detected amplificate in both frozen and PET samples in the early biochemical relapse vs. no biochemical relapse comparisons using the assay of SEQ ID NO:35 shown in Table 12.
Figure 105 shows the detected amplificate in both frozen and PET samples in the early biochemical relapse vs. no biochemical relapse comparisons using the assay of SEQ ID NO:37 shown in Table 12.
Figure 106 shows the detected amplificate in PET samples only in the early biochemical relapse vs. no biochemical relapse comparisons using the assay of SEQ ID NO:19 shown in Table 12.
Figure 107 shows the detected amplificate in PET samples only in the early biochemical relapse vs. no biochemical relapse comparisons using the assay of SEQ ID NO:63 shown in Table 12.
Figure 108 shows the detected amplificate in PET samples only in the early biochemical relapse vs. no biochemical relapse comparisons using the assay of SEQ ID NO:35 shown in Table 12.
Figure 109 shows the detected amplificate in PET samples only in the early biochemical relapse vs. no biochemical relapse comparisons using the assay of SEQ ID NO:37 shown in Table 12.
Figure 110 shows the detected amplificate in frozen samples only in the early biochemical relapse vs. no biochemical relapse comparisons using the assay of SEQ ID NO:19 shown in Table 12.
Figure 111 shows the detected amplificate in frozen samples only in the early biochemical relapse vs. no biochemical relapse comparisons using the assay of SEQ ID NO:63 shown in Table 12.
Figure 112 shows the detected amplificate in frozen samples only in the early biochemical relapse vs. no biochemical relapse comparisons using the assay of SEQ ID NO:35 shown in Table 12.
Figure 113 shows the detected amplificate in frozen samples only in the early biochemical relapse vs. no biochemical relapse comparisons using the assay of SEQ ID NO:37 shown in Table 12.
Figure 114 shows the detected amplificate in both frozen and PET samples in the High Gleason vs. Low Gleason comparisons using the assay of SEQ ID NO:19 shown in Table 12.
Figure 115 shows the detected amplificate in both frozen and PET samples in the High Gleason vs. Low Gleason comparisons using the assay of SEQ ID NO:63 shown in Table 12.
Figure 116 shows the detected amplificate in both frozen and PET samples in the High Gleason vs. Low Gleason comparisons using the assay of SEQ ID NO:35 shown in Table 12.
Figure 117 shows the detected amplificate in both frozen and PET samples in the High Gleason vs. Low Gleason comparisons using the assay of SEQ ID NO:37 shown in Table 12.
Figure 118 shows the detected amplificate in PET samples only in the High Gleason vs. Low Gleason comparisons using the assay of SEQ ID NO:19 shown in Table 12.
Figure 119 shows the detected amplificate in PET samples only in the High Gleason vs. Low Gleason comparisons using the assay of SEQ ID NO:63 shown in Table 12.

Figure 120 shows the detected amplificate in PET samples only in the High Gleason vs. Low Gleason comparisons using the assay of SEQ ID NO:35 shown in Table 12.

Figure 121 shows the detected amplificate in PET samples only in the High Gleason vs. Low Gleason comparisons using the assay of SEQ ID NO:37 shown in Table 12.

Figure 122 shows the detected amplificate in frozen samples only in the High Gleason vs. Low Gleason comparisons using the assay of SEQ ID NO:19 shown in Table 12.

Figure 123 shows the detected amplificate in frozen samples only in the High Gleason vs. Low Gleason comparisons using the assay of SEQ ID NO:63 shown in Table 12.

Figure 124 shows the detected amplificate in frozen samples only in the High Gleason vs. Low Gleason comparisons using the assay of SEQ ID NO:35 shown in Table 12.

Figure 125 shows the detected amplificate in frozen samples only in the High Gleason vs. Low Gleason comparisons using the assay of SEQ ID NO:37 shown in Table 12.

```
accccgagtt tctctttaat tggggcactg actcttccca ctttgggatc ccaaggcact   28140
cggtgtgtat gcagattcct cccttgtggt cttcaccatg tggtttcgta gcaggtctct   28200
ggttcaatga tattttatag tcatagccct catattcatt atcatgatct caatgtttag   28260
gcttttagtg tatttatatt aaacctgctt tattagtaag ctggagcaca caggagagat   28320
gggggcaagt aaggactcag cagagctcaa attcagacat gtttaaatgg ctttgactgt   28380
gtaaagtgtg gcaatgcttc ctgctgccct agctttccac tctaagcttc acatgtcctc   28440
tggctaatga agtgtgatat aggccacatg ctaggaataa tagtatttgt tgagaacaaa   28500
gtgaactcag gaaacctggt atacacaaaa tgcatc                             28536
```

<210> 962
<211> 28536
<212> DNA
<213> Artificial Sequence

<220>
<223> chemically treated genomic DNA (Homo sapiens)

<400> 962

```
gttttgggag ttaggttttt ttcgttggat gggaaaagga ggtgcggaaa ggggtaggcg      60
gtttgcgggt tagcggggat gagagttgtg gggagaacgg aggagagagt aaaattattt     120
tttttggaag tttaagggaa gggagttgag gtgagagaaa tggtttttttt taggttttaa    180
gttttcgttt ttagtttgta ttatttaagt cggggaagtg ataatttttt ttaaatttcg     240
atttttattt tttgtttttt gtagattgat tgatgttttc gcgttgtttt agtttagtaa     300
ttaaattagg tcgaggtata tttttttttat ttagagtttt taaattattt attaatttgt    360
aggagattta aatttttcgg gtaggtttcg ttagcgtttt tagtttttat ttttaaattt     420
tttttttttag ttttttttt ttttttcgtt ttttggtttt tttcgttttt tattaggttg     480
agaggcggag aggtcgggat cgttgggttt tgcggtttta gaagttaaag ttcgttcgtg     540
gggatcgttt tttttttttat tgtttttaag tgaataatat ggtgggaggt gattttcgtt    600
taaagggttt ttatttgttt tttattgttg cgtgattttt tcgtaagtcg gaggtagagt     660
tttaaaaaga aagaaagtgt gcgttgagta ttgattttaa atcggggata ttatttgcga     720
tttttttaag tggtagtagt agttttttgt agaataaaga tttagtgttc gagttttaaa     780
agtttttagg taaagtttta tttagatgaa agtaattaag gcgaaaaaat aatgatattg     840
tcggttttag aaaatcggcg gttatttttt gttttaagtt ttttagcgtg gtgtttgttt     900
tcgtttattt tggttgtgcg gggggttgat aagtataata aaagatttta gtatattttt     960
ttttttattt ttataacgat ttttttagcg ttatgaggat gaattttttg ggattaagtg    1020
gtatttgttt ttatttgtta tgaaattaaa ttttatattt ttttaaagtt gaaaatcgcg    1080
gataaagaag gatcgggtgt ttaaagttta tttaaatatt tatatcgaat ttattttttg    1140
tttagatgtt agaggatggt agggagggtt agggttgtaa tttattttga tttgttttat    1200
tgttttgtag tttgtaaatt ataattttgt ataattttag gaataagtag gtttagaatt    1260
aatgggttaa tattatttaa aataaaatat cgggagtatg atttttgttt taattatata    1320
ttgttcgata agatttagga aattttattt taatttttta atttttgagt tttttgagaa    1380
attgaagggt tgtagttatt agaaattatt tttgtttttt ttaatgtttt taaaggattt    1440
ggaaatagta atgtaatata atatgaagga ttttttttatt taagtttgaa gataaacgtg   1500
gaagtttaaa tattttgaat tggagatgtt tttttgtaaa tttattatag atgtattttt    1560
tttgagagaa atgtatataa attatatatg tatatatgta taaatatttt tttagaataa    1620
tttgttagag gtgtaggttt tttcgtaaag gagtaaattt gagagttatt ttaagttgat    1680
ggatttgtta aatttttttt ttttttttt ttttttatat tatttgttag ttataatgga     1740
atttttttagg tttaagttaa agaaaaattg gagagataaa attagatttt gtagtttttt   1800
tttttttcgg gaatgttttt tttttttttt ttagtttttg atgaatggtt attatttatt    1860
tttattaaat ttaaataagg attgttgttt tgtatgttta attaggtagg tagagggaat    1920
tggtttgttt aggaagtagt gattgagatg ttttggttaa gttagtgata gaggagggga    1980
gaaagaattt agattaattt gtatgtagta tattttattt ttatgaaata aaatatattt    2040
gttttatatt tgttgaaaag taaaataata atattgtacg aaatgttata tatagggtag    2100
gttgtatata gtagttttag aaatattatt gtatttatta gagaaattat tttaaaattg    2160
atatttatat atttttttata ataataataa tatgttagaa atatatagtg tggtatttag   2220
tatatatatt tttttgttcg taagcgaaaa attttaatcg tttttgtata atatgtttta    2280
ttttaaagtt taatttttaa aaatattgtt gtgatattat taataattgt ttttataaaa    2340
ttaatttgat atttcgatat atatatattt ttttagttat ttaaatgtta ataatgttaa    2400
atttaaaaaa taataagttt atagtaatgt taaaatgtta tatttagtta aatatttgtt    2460
tgtgtatgtg tttttgtaat tgttagaaat atttgtagtg aaagatgtta gatattgagg    2520
```

```
atattttttt gaaattaaag gagttttttt tttgatttag tggttttttt tttttatat    2580
agtttttttt tttttttttt tttttagtgt ttacgatttt ttagtataat ttttagtttt    2640
ttaagggcgg agttgtttta ttcggtaagg ttttaggatt tcggcgttgt gggtgcggtt    2700
tatacgggtc ggtttattgt atattggtaa gtatttaggt tggaggtcgg gttttgtacg    2760
ttggcgtagt cgaagttgga gtgttgtttt gtttttagtt ttaggttggt taggttcgag    2820
ttatacgtgt ttttataaat atacggagga gtcggcggcg cgtaaggata ggtaggcgtc    2880
ggtatcgcgg aatttagcga cgggttattt aggttgttta agttatttag gttgttgaga    2940
ttggagttcg ggacgtttgt tattgttgag ggtattatgt tggacgatat gtttatggac    3000
gagatagagt tgggtgggga aaatatgttt tgtgatgata gggggttgac gtttatagag    3060
ttgaagaagg ggaagttttt ggtggatagg gaggcggatg taaggttttt ggcggtttag    3120
ttgttgtagg aatagtttgg gtatatgtcg tcgtagggtt gtatgagttt attgaattgc    3180
ggttcgaagt tatttttgta tagttcggtt tgttggttgc gttttttttt tttttatttg    3240
gttcgacgat ttttgaatta aatttggggg cggttggggt aagggagtaa atagatgtta    3300
tagtgtagat tattaaaatt tttatcggag gttaatttttc gttttttttc gatatatacg    3360
ttagcgtatt tatatatttt ggtttcgttt tattgtatcg ttttgtatat taagatatta    3420
gggttagttt ttagttattg gttcgggttt ttattaagcg taggagattt ggtttgtttt    3480
ggtttgcgag ttgggattcg gagttacgtt ataaatttta gtcgaacgta tggagatttg    3540
cggacggttt gattatttag ttaggcgttt ttttaggttt aaaaatattt aatgtaaaat    3600
aaacgcgggg tagtaggttt ttttaatttt tttcggggta ttttgtaaat ttgtttttat    3660
tttaaagtta tagatttacg gatgaggaga aggggttgga agggtattag aggatcgttt    3720
ttttttttac gtaatttttt tttttttttt ttgattttta ttgtcgtttt ttattttttg    3780
gtacgtgttt ttttaatagg gattaggtcg ttaatatttt ttttcgttta gtaaaataat    3840
taaataaaga gtaaaagatt attttttcgt tagttcgtta attttaggag tttggtatat    3900
taaatttcgg gaattcggaa agggtagttt tggagatttt ttttttttttc gttttgtttt    3960
tttttttattt taagtttatt ataggtttgt tcgcgcgtta ggtttagtcg ggtcgtttgg    4020
ttttgtaggc ggttatttag gtcggtcggt ttttattcgt gttcggtggt ttagtcgtaa    4080
tttcgatttt aatttatatc gggttttttt gtcgttttag acggcggttt ttgtgtattg    4140
gagagaggtt tggtttgaga tattcgagtt gatattagtg atgttttata ttatatattt    4200
tcgtcgggtt tagtcgtgta attcgttttt tttttttttt tttttatttt tgattttttt    4260
tttatttttt ttttttttttg tattcgattg ttataaaaag tacgttttat ttttatttgg    4320
ttcgataagt agtcgttttg gaaggagagg tagttgtaag gagagtttag cgtcgcggtt    4380
ataaagtatt agggtggagt tgcggaatag cgggcggggt gggagggcgt tttcgaagga    4440
ttttagaaaa tttatagatt ttgtttttaa ttatttgtta tttttatttt aggttattta    4500
aattttgttt aggcgagaag agtacgtgag aggttcgttt ttttgatgtg taagagagtt    4560
aatgaaagat tgattttgtt taaaattacg tcgtttagga tttagttttg gttttggata    4620
gttaaattaa aattattttt aatttttttt cggtttttta tttattagta tagttttatg    4680
ttttgtataa atgttattta gagagtgttt ttattttttt tgatttggga gagtattttg    4740
gtttttattt tttttatcgt tgtttttttt tttttgtttg ttttgtttta atcgggggtt    4800
ttattttttt tatttagagt atttaatttt ttttttttaa tagtaaagtt tttggatgtc    4860
gtttgatttg tttgattttg ttttttgttt ttagaatttt aataaatttg gaatttttta    4920
tcgattagta taaattagga cgttgttatt gggttattta tttgagttta tttttgttaa    4980
tttataaagt atagatttgt tataaagtta aggtaagttt tttttataaa attatgatta    5040
taatttagaa gagggggtgt gagttttaat ttttagagtt taatttttga gagaagataa    5100
ataaattaag tagaaaagtt tttttttttt tttttttttt tttttaaga ggattagtag    5160
ttgtgtatta aaattttgtt ttcggagatt ataaaattag gaaatagggt gtgtgggaga    5220
gatttgaatg gtcgaaataa tcgtaaagaa ggtgtaagaa gcgcgagttt aggagggaaa    5280
aagttgggtt agggtcggga taaaggtttt ttagggaggg ttaatttttt cgtgtttttg    5340
gcgggttttt tttgttaaag gtttataggt tggagtttgt tcgcggtttt tggtttggta    5400
gggattttat tagttttgtt ttggtaattg taagttagga atataatgtt ttgtgtaggg    5460
gattgtttat gtagtttagt tcgtgagatc gcgggatggc ggggtagtga gtcggtgtcg    5520
ttttgggagt ttgagttagg gcggtagttt tgtcggtttc ggagagggaa ttgtaatttc    5580
gtaattaggt cgtcgcgagg ttttttgttt ttgtaaagtt gcgttttatc ggcgtttttt    5640
taggcggcgt tgttttttat attttttttt ggtttatttg gtttgtattt ttataatatt    5700
tttttttttat tttttttaga tttcgtgttg gtttttattc ggattcgggt tttcgtaagg    5760
ttggtttata tagcgatttt ttcgcgtgtg gatatgttcg ggtagcggtt tttttggaaa    5820
gtggttttta gtttttggag ttgttggttg gtaaagtgag ttcgttgtcg tttttgtcgt    5880
ttttttttag acgggttttc ggcgtttacg tttttatttt ttttttgttg gtttttattt    5940
tttttgaaa acgaaatata tatatttttt cgttagtatg tttatttgta acgcggacgt    6000
taattggatc ggcggtagaa gtcgtggaag agttgggttg tttggcgtcg gaggagggtg    6060
cgcgcggcgg tttcgggtcg cgaggagcgt tgcgtttgtg gggtgtgtag gcgtaagtgt    6120
gggtgttcgc gttttatttt tttttttttt ttagcgtcgt acgttttatt tatatgttta    6180
```

```
ttttattgta gcggtatatt tatttttata gtttgtgttt ttaagtatat ttatatattt    6240
ttgcgtagat atattaaatt ttttgggacg cgtatacgcg cgtggtttat agattttttt    6300
tttttttcgta gaaagtttag atttttatgc ggtttgggaa ggttaggaaa agatgtgggg    6360
attcggttgg gtatcgaagt tcgtcggttt ttttttaaaa aaaaaaaaaa aatgtttttt    6420
cgcgaagggt atttttgagt ggttttaggt aattttttaa cgagtggagt ttttcgggag    6480
ttgaaagtcg agaggaaaat agggatagag gtcggcggtt tttgaaggtt ttcgaattaa    6540
gatgttggga tttttgtgat ttaggaaata gaagggaggt tagggtacga atagagaggg    6600
cggtagaatt gttcgcgttt ttagcgtttt aggagtcggg tcggtcgagg gagaattaaa    6660
gggatgcggg gtagttaaaa tttcggtttt cggaagtttt gcggggagtt aggcgaacga    6720
ttatttttat tacgtttttt ttcggagggg ttgatttttt tggggcgaga gggagcgggt    6780
ggcgtagagt agttgagcgg gaatgtttgt agggcggcgc ggcgttttat ttgcggtttt    6840
cgggttggag gtgtcggaga tggtgtgtat ttttagtttg tgtttggagg agtttagcga    6900
tcggggttga tcgggagtta gaatcgaagt tatggttaac ggttggggat ggtgatagga    6960
agatgaggag acggtcgata gtttggtttt cgttgttcgg tgttttaagt gaagcgggtt    7020
ttttatgtag tttatggacg agggagcgcg acgttttatt agtttttggt tattgtttcg    7080
tcgagttttc gtagtcgtcg ttgttcgttt cgggtcgcgt tttaggcgcg gagtttttc     7140

gttgcgggga gagttagggg acgtaatttt cgtcgagttt ttaagttaag ttgttttcgt    7200
ttttttcgga aggtttaagc gaaaaagttc ggagacggaa agttagcggg taaacgaaga    7260
tatgggatgt gggtagaagg gtattattta gagcgttttt agggagtagg tttttaagtt    7320
ttaaagcgaa ataagagtgg gtaaagattt tttttttttt tttttttttt ttttaagaat    7380
tttttttaata aggaaagtta acgtcgatcg cgttttgttc gttttttttt tacgcggtag    7440
ttttgataga gaagtgttaa gagtgatagg gataggtagg tgatattaga ttttttgcgg    7500
cggtagtagt cgttgtagtt acgacgcggt tttttgagcg tatttttcgt aacgcgtata    7560
cgtatatttt tcgggcggtc gaataggagt cgggttttgt cgtagtttag ttttaggtat    7620
ttaggcgagc gacggattag atttgcggtt tcgcgttttt ttgttggttt aatattttaa    7680
aattagaggc gggtttttttg gtgtcgagac gttatttcgt cgcggttttt tttagttttt    7740
ttcgttttcg ttttttttta gatttttttt cgggtgcgat tgacgtggtt tcgtattaat    7800
taggacgttt cgagtcgcgg tggagggatt gttttgtttg tatttattag tagtgcgggg    7860
tcgggttatt gtttcgtcgt gcgtattggg tttatatagg taagttttcg ggaatttagt    7920
ttttgtttag tttaaggcga ttcggttttt agtacgaatt taaaggtgaa gagatgaggt    7980
taggagtcga aggtttggga gaagagagtg gaatggttaa gaagagaaag gtataaggat    8040
taataagata tttatttttt gtgttttatt atatttattt ttaatttttt attttatata    8100
aaaaggagat acgttattta aaattagaaa atttgaaaaa tagtaataaa ttattttttc    8160
gattttaaat tttttaaata gtttgttaag tgaatgttgc gttaatttga agaagtttta    8220
attgtaaaga agatagagtt ttgaaaaggt aggttaataa attagaaatc gagaagtaaa    8280
tggattcgtt aaaagaaaat tattttgatt ttaaacgaat aattgtttgg tggtttattt    8340
tggatttata taagaataaa aagtcgtttt agattacgtt ttttgtgatg tttattagtt    8400
tttagataga aaatatataa tagaagagaa attttaattt agcgttttta aaatgttgaa    8460
agtttattta ttttatttaa cgttgattaa gatatatatt ttagattttt taaatttttt    8520
gtatattgta ttaagttcgt tttaattcga gagagttacg ttttaaattc gatttttttg    8580
tttattttat tattaattag atttaaattt ataaagtttg tagaattaat aattttgagt    8640
taattatata tgaaatatgt tttaatgaat ttttatataa ttaagaatgt tgttaaataa    8700
ttaattttaa ggataatttt taatagttat tttttttttt tagtgagttt aaggttgttt    8760
tgagttatta aagtttaagt aggtagaagg ggtgtgtgtg agttaagggc gaaaagttta    8820
gaattgcgtt taattagtaa aagtaaaatt ttatttatat aaaataaaaa aaattatttt    8880
tggagatatt aatttttttat agtattgttt ttaagtaaat ttaattttta aagaaattaa    8940
agaaagaaat ttaaatatat ttaaaataat ttttgaaagt tttttttgttt tttagtatag   9000
gttagttgga gaggataaat taattttttt tgggtttttg tatgggcgat tgttttatta    9060
tggagttagt gttattattt ttgaatgtgt atttgtttga tattatagtt aatgatttgt    9120
aatgttagta tgaagtattt ttaaaatatt ttttttttgt ttttgtttat aagattggga    9180
aatttattcg atgtggaata aagtggatga agtagattat aaatatattt gtaatttatg    9240
tgttttttttt ttgttttgat tatttttaaa ttttatttgt aatttttttt tattttaaat    9300
ttgtagttta aagacgtata tgagaattgt tttttagttt tttttttatta gtattatttt  9360
attttaagaa taatttagtt gtaagggagg aatttttttta tagtaagttt taaattagta  9420
tttttgtttt taattttttta ttttatttta ttttatttta tatatataga tatttgttta  9480
gagtaaaata tatttttatg tgataggttt gtattagttg aggtttatat atttagttat    9540
attaggtttt gtaattttat tattaaatta tatatattat attagtagtt tgttggtaaa    9600
gaaggttaaa ttaatttata ttttgtttat tatttggtgt ttaaatgacg tattttattt    9660
cggagatttg gcggagaatt ttttttttag attttatagc gttttattga agataatgtt    9720
tttatatttg tagtggtttt taatttgata agattttaat ttgtttaagt tttttaaata    9780
```

agggttttaa atgtttttag tcgttttttt attgaatttt ttttaatttt tttaagatta 9840
taaagtatat gtgtaaagta aatatttttt tttattgtat tgttagtcga tgatttataa 9900
ttaagttaat aagaatttag tttttttttg ttgaatgtgt ttattaatta tattttagtt 9960
tttttttta aattttagaa tagttgtggt ttttataata ttatgttttt taaagtttta 10020
ttttatgaag ggattttatt atattaaaga atgaaaaaaa tttttattgt agttagtata 10080
tatagttttt tattttttgt tttttaagat ttaaatttta gagttgtaaa tatttttgga 10140
agtttgggtg ttaatgtttt attttagaaa gtcgagaagt tttatagagt tatatagatt 10200
tttaaattta tttttttataa atttatagaa ttttgataaa agtttttggtg gttttatttt 10260
atcgatggaa tttttattac gataaatata tatgtatgaa ggattttaat tagtttttaa 10320
agtggttgaa aaatttaagg gtacgtgatt gtttttttata gtgttaacgt gtgcgagatg 10380
ttggaagtat tggggattag tagtagttta gatgtttaaa aagataaggt gttttaattt 10440
gtgtggattt attgaagtta agtggtgaat aaagataatt atttagataa tttagattaa 10500
agtaaaagta aaattatatt tatttgtata tatatattta tatttatttt atattataga 10560
tatatatacg tatatatata ttggttttgt aaataattga tttaaagtga ggattttttt 10620
tgtatttttt tagtaggagt tttaatattt ttttaatttt ttaattattt tatatattta 10680
tagtagcggc gattgggtga tatttttttt aggtttttttg tgtggtagga tattaatatg 10740
ataagtttgt atggggaaaa ggaggtatgt ggtgggaatt aagaaatatt gtttagtgaa 10800
aattttgtgg tatggtggtg gttgatttttg gagatttaat gtatataaga tttgtgggtg 10860
tataggtata ggtagtatgg atgagaaagg ggttagaaga aaataaattt tatgtatttt 10920
gtgattttag tattattgtg atttttggtt aagttttttt taattggttt tagaaattat 10980
tatgagttta gtttttaata tagaaatttt taatacggag aatattggtg ggattttggt 11040
agggaaatta gaggtgttgt atggtttacg tggggtaaag aaggaaagtt tagtgtcggc 11100
gtgaggtttt gagtttggga gatattaggg gttgtttcga ttggggtttt ttgtttattt 11160
ttttaaagaa agattttaga ggagggaaat gtgtgatatg gggttagtcg tgttttgtgt 11220
tggtatttgt tatcgattat tagttttaaa gttttattta attttatatt ttttagtgtt 11280
agttgtgtaa agtttttttg gttatggtag tgagcggttg ggttgtgtcg ttaaattttt 11340
cgtattaatt tggtttggga tttaattaag tgatttttga tttttggaaa gagtttgttt 11400
ttagagttta tttagaagat ggtttaatta gatatttttt tgagttgtta ggttttagac 11460
gggtgggagt tttgttttgt ttaagttagt ttaaggacga ggttcgtttg gatttagttt 11520
ggagttacgt gatgggcgtg agtgtgtgag tttttggtaa ggcgtagagg ttagatggag 11580
attttgtatt ttgttcgaga agtgttttat ttttttaat atttggtttt tttttgtata 11640
taaattaagt tgaaaatagt ttattattta ttattttta tagttatgga attaaataat 11700
ttagaaatta aaagttttat tgtagttgtt ttttttttta tttttttaaat ggaatttaaa 11760
aagttttggt ttgttaaaag gggaagatta tttttgaat tggaagtttg tagatatatt 11820
gagtaatagt tatttttttt gggtttttgt aaatggtatt tattttttta atttatagtt 11880
ttagttgttt aattatttga gatttggggt aattatttgg gggaatagtg tttagatggt 11940
agtgggagtt attattttat agtggtttgg ggaagagaag agaaagagat tagaggaggg 12000
ggtatttgtt aaaattattt aacgaatatg ttgttaatgt ttttttatat ttgtatgtta 12060
ttgttatagt tttttaggt gttattgagt ttttagaaag taattatttg tcgaattaag 12120
taaaataagg agaatggtat agtatatgtg tttggagaag gggaaggaag ggtggaatat 12180
gaaattgagt atagatattt aggttaggaa agaaggaagt ggtaaggggt taaatgaagt 12240
tttatttttt cgttattttt ttaaataata gttggattaa atatttattt gttttttttt 12300
ttttttttt ttttttttt ttagtttatg tttatttttt tttttattt tttttgtttt 12360
ttttttttt tgtttttttt tttttagata tgttggtagt taatatttag tattagttgt 12420
tatggtgatt ataaattatt taaatttaaa aatatttatt tataatttga gatgaagttt 12480
ttattttttt agcgaaataa tattttaaaa gttgttagtt gataaaaaaa aaggaattta 12540
ttttattgta gtaatttaag taatatatta tttttaaagg tttaaattaa aatgttagtt 12600
tgttaaaaat atgttggtag agttttggat atttttttcg ttagattttt ataaagaagt 12660
tgattttgtt atttttggtc gtttttttaat atatatatat aattttgtat gttttttttt 12720
tttttattaa ttttttttt ttttattaat tattttagtt tttaaagatt ttacgtattg 12780
ggttttaaaa gaaaagaatt ttttttttgga ttagaaaata gttttattgg ttgttgaagt 12840
gaaagatgtg gggtttaggg ggaaaggtta ttaggattat aatggcggtg gtggtaggag 12900
gttattttag aggagttaag aagaaaaaaa aatgtaggga gaaggattgg aggtggaaag 12960
atagagtaat agaaaattga gttgggtggt taggtgttgc ggtgaagttt agtttcgaaa 13020
tgataggtat atattttttt atttttgttt ttttttttt tgagagaaaa tttttttagt 13080
tagagatttt ggggggtagg aggcgggtaa acgtcgttgt agttgggttt tttgtttttt 13140
attttgggtt tgttgttttt tgtttatttt ggattatagg gtattcgttt agatgaagag 13200
ttattaatta tttatttagt taatattagg aagacgataa agtttttttat ataggattaa 13260
gaagatatta gattgtttta ttagtatatt tttgtttacg aataagtttt cgttatatat 13320
ttttttttt ttcgagtcgt tttaataatt gttgtatata ttagtagcgg gcggtgagga 13380
ataatagtcg aattagtttt aagaaatttt gtgtatcgag ttagtagcga ggaacgcgat 13440

ttgtgaagat tatttttgcg ggtagggatt cgtagggatt gattattttc ggataattgg 13500
tataattttt tttgggggtg aaaaattata acgcggcggg gtattttta agtgagttgt 13560
agatttgatt ggcgcggggg gtgggggagg ggaggggaga atgggatggc ggaggtcggg 13620
cggaggaaag aaaatggaaa attttttta tttttatttc gttgtttttt ttttaagttt 13680
tatgtttttt tcggtaagta tttgtttttt tcgcgttagt tatagttaga gtttttttatt 13740
ttttttata tattttttt tttttgataa ggtttaggat tttggttatt attttacgta 13800
ttattatttt gcgttttgtt agagacggtt tgggttgatt tcgttggtgt ttatgttagg 13860
attaaaattt ttttatgacg gcgaggaaaa ttgtattatt tgtttttagg gggtatatta 13920
ggagtttacg tagtatatgt ttcgtaaata ttcgttgatt gaatgagagg cgcgggggcg 13980
gggcggcgga gaggggtttg cggtcgttaa ggtcgttagg gttaatttag gtttttcgaa 14040
gaaggttggg atcgagttgt tgtcgcgtgt gaaggtgtgt gtcgcggttg ggggtgttat 14100
aacgggttat ggagtttatt ttttagaggg aggaagtttg tgtatattag cgatttgggt 14160
cgaatatttt tagtttattt attgggttaa agttatttaa taattaatgc gtttttgggg 14220
gaggtcgggg gaagtattcg tttttgtcg ggacgtaaag ttaggcgtta ggtttaaagg 14280
ggttgtagtg tagttcgatt ttagtacgga atttagagtc gtcgttttga aatttttaa 14340
gttagtgata gaggagggtt agttcgtttt tttttgagg gtgaagatta ttttatgagt 14400
ttttttagg atttttaaag taagaaaagt taaagaaagg tttttttgtt ttagggggtc 14460
gtttttagtt ggtttttata ttattttttg ttagttgcgt ttatttttt ttaaattttt 14520
ggtttttagg ggtttttagt cgttttcgtg ttattttcgt ttcggggttt tatttaggtt 14580
gggtattcgt ttaatggata ttaaggagaa tgggatttat tagggaagga aggtagagtt 14640
tggattgttt agaggtggat tttgtttata tagaacgttt agtttttaac gaggatatgg 14700
tatttttggg cggcgttggg ggtagaggcg gagagggtag cgtaatagat tattacggtt 14760
ttttgaagaa gttatatgtt attgtgaatt tttttttttt ttaaaagtaa agaaaaattt 14820
taaaaaaata taagaaataa atttttttgt tttataagta ggttgtggtt aggattttgg 14880
atattttata agttaattta aaattaggga aggataggtg ttttattttt tagtagtgtt 14940
atagttttgt ttattcgtgt gattttttt tgtcgtttat tagtttttta aaagttaatt 15000
aaattaagat ttttagtatt tttttttatt atatgttttt ttttaattaa tggtattaaa 15060
cgtgtttagg tagtaatttt tttttcggt taaaaagtag aaaaagatat attgagtgta 15120
ggggaagagt tttttacgtg tattaaaata atgcgggttt gaaagtaatg gttaagaaag 15180
taattattta ttattttag tttttatgt gttagttatt aaaagcgaac gattaggggc 15240
gtttgcgcgg tttgtgattg gcgtaagtag aattttttg tttttagtcg tttttgttt 15300
atttacgagg attttatttt atcgtaggtt tttttatttg tttttagaat gtatttttta 15360
tgtttaggaa atttggggta gggattgggg gaaggagata ttttgcgttt ttttggtttt 15420
tagtataata agaaatgtta gtttcggttt ggcgattgcg agttcgtttc gcgcgaagcg 15480
agattgggga gttttttag tttggtcgga gttagggttg agttcgcgta aagtattttt 15540
tttagaagtt atcgttgttt ttgattttta attatatttt aaatatatta cggtttaata 15600
atttattttt attatcgatt attaaataga agaagaattt aatataattt aaatgataga 15660
aattacgcga cgttattttt gtattgtttt tatttaattt tatggggtta atttcggata 15720
agtgagcgtt taattggttt agtagggcga ttggcggtgt agtgtagtgt tcgggcgtga 15780
agtattggat cgttttagac gttttatttt aataaatgat tattttttt tagatttacg 15840
gggaaatttt aatgtaagat ttttgttttt tttttagtaa tacggtttgt tttttgatc 15900
ggggttttaa atcgtttttt tttattttat attatatttg tatttttata ttttaattcg 15960
gaaagagggg gttaggggtc gagggttgtg gggggggggg ggttttattt gtttatatta 16020
ttaaaggtta atagtttttt aaggttaggt atttatttat tacggagtta ggaaaataga 16080
ggaatagtaa atttgagggg ttttttttta tgtatttgaa aagaaaggta ttttttttt 16140
tttatttttt atattttttt tttcgtttta tagaataagt tttaatttag gaaaggtttg 16200
tggcgtaggt tggagatttt ttaatttttt atataagttt gtagattttt tttggtaatg 16260
tttttcgatt tttttagagt gaaattagtt aattaagtaa cgatatcgtt aaaatttaag 16320
gtttggtaat tagtatttta ggtaggttcg cgtcgatagg ggtaaatttt tattttattt 16380
tgggttgtta agtatagtgg ttgtttttta gtttttttagg gatgttgtcg gtttttcgtt 16440
ttttttttaa ttagttaaag taaattttcg ttaatttaag tttttttttgt ttgtttttcg 16500
cgatgaatcg cgtatttata agtttgggtg gggcgtggtt gagagtttga gtgattgagt 16560
gggttttggt ggtgttgcgc gtagcgggat ataacgagcg atagaggtcg ttgttggatt 16620
attttttttac gttagtttag acgtcgaggt ttgttggagc gtgcgtaggg gattagatta 16680
tagggagcga gcgagaggga gagagaggtg ttgggtttta ggagtgtagt ataatttggg 16740
gaaaggaatt aacgtttttg ggacggttgt ttttcgtttt atttagaggc ggagtgttta 16800
agtttaagta gtaggcgcgt taggtttggc ggtttcgttt ttttgcgttt cgttcgaggt 16860
ttagagtttc ggaggcgggt gtttagcgcg cggtttgcgt tttttttttcg gttttattat 16920
tggcgttagg atgttgtcgc gggaagaatt tgttgttggt tgtttttttt cggtttttag 16980
gagagttcgt gaattcgatt tttttgattt cggagttttt ggagaagaga tatttaacgg 17040
tcgtcggttg tacgtttggg ttacgcgcgc gttcgtttta cgtgcggaga gaggcgtttc 17100

```
ggatcgcggt cgaaaggagt cggggacggg aggaggggga ggggcgaggt aggtcggagg   17160
agaaagaggg ataaagagta aagatttagt tagaggaaag agttgacggt attttcgttt   17220
ttcggatttt ttggtaattc ggggtaggat ggcgtatttt ttttgcgttt tttcggttgt   17280
cggcggtttt agtcgggagg agtaggttgg ggggtttcgg tatatagcgc gtcgttgttt   17340
tttagtttat cgtttcgtta tagggagagg ttattggcga tttggttttg atttttttttt   17400
tgtttaggtt ggtttttcgg ggaagcgttt ttcgttgggg tttcgtcgta gggttagtgt   17460
ttttttgtcg tttttacgtg gcgcggtttt tcgttcgatg attcgggtag gagaaggggg   17520
tttttattta attgtatata cgtcgatatt agtttgcggt agttggtttt tatttttcgt   17580
tatttgtaaa atagaagaga aggaaggttg taagaagcgg cggtcgtcga gtgagtaggg   17640
tttagatgag attacgttat attagttgtt aggcgtttat tgtgtgttag gttttaggcg   17700
tgttttttcg atttgatagt ttttggttgt gtagtagtat ttttagttta gtttcgggtt   17760
taggatattt atttattaag aggggatttt tttttagagt tgtcgtaaaa gtgtttagag   17820
gttagaggat tataaagtta tagtgtgttg gggaggttgt ggatttattt ttaagaattt   17880
cggtgtcggg gttaagaatt tatttgaacg taatggtagc gggagtgggt gggtggagag   17940
gatttttttt tttgggaagt tgtatgtaaa gattattttt tagtgtttgt ttattagttg   18000
gagttcggta aatatttgta gaatattagc gttaacgtgt ttttgtttta gatagtagtt   18060
tttttcggtt ttttgtaatt ttgaaacgaa cgggtttttg gtttagggtg ttttaggagc   18120
gagttgagtt cgggtttttt atttattagg agttattttt ttatatttag ttatattttt   18180
ttttagagat attaattcgg ttatttattt atttattata aataattatt ttaaagtatg   18240
atttaagatc gtagaggaga gatattgggt ggattgagcg agattgagga gagtagggta   18300
aacgtttttg gagggtttat tgttcgttaa ggacggagaa atagttttgg tataattgtt   18360
atttagtttt tttttttttt ttttcgggcg agttaaattt tttttacgtt tttaattata   18420
acgtagcgag ttaagtattt aacgcgtttt tttttttttg ttataggtaa gtcgggagag   18480
gtgggtttcg aggggtttta tcgggtgggt agaagagtcg cggttgtttt aaagataaga   18540
aaagaaggtt tagggttttt taggtttttt cgattttagc gtttgttttt ttttacgtta   18600
attagggtac gtcgacgatc ggagggttta tttcgcgcgg gtgcggggat cggggtggga   18660
gtaagcgttg tcgggttggc ggaggtatag aggcggggta gggagttgcg ggtttgtttt   18720
tggtttgagt atcgtttttt tgcgtttcgg tttttttttga agggagttgg gttttgggga   18780
gtttttggtt aaggtcgttg tttataggag gggttgttcg gcgttgtggc gtggggattt   18840
agggtgggga cggttaggcg gttttttttat tcgttagcga gaacgcgggc ggggattttg   18900
tcgattcgat ttttgtgggt tcgtgggttt agaagtagta gtttggcggt tttagattta   18960
gtgattttgt agtaaaatta taggattagt ttttgattga gatgtttgtt cgtgagatat   19020
tataaaattt attattatag ttttttatta attcgatatg aagtaatata gatgggattt   19080
tattagttta gattttaaat gtttatttat gataatttcg gaggaaattt gtatgttatt   19140
attatttcga taattttttt tttttatatg tttgaattgg ttgtattatt agttggtagt   19200
cggagtattg tagatggtaa ttgtaaatag tttttattta tttatttttt ttaaagaatg   19260
aaatatataa aagaaaaaga ttgcgttgtt tggtgtaaag ttagttaatt attatatatt   19320
ttttttttta ttttttcgtg ttttagtgtt gaagattaaa taaagtaata taaaataaat   19380
ttttaagaat ttatagagtt ttattttaag gattgaaaag aaggttaagg cgtgtttttt   19440
agtttatttt tatatgtttt tgtgatttgg agatttattt tgtagttaaa atgagttttg   19500
agatttgtat ttttatgttt tatttaatga ttaggtttat tagaagaatt gagtttaaat   19560
aattggggaa gataattttt taaaaagaga tttttaattt tcgtttgttg atttttaaat   19620
ttgttttatt aagataagtt ttttgtgaga aatttggttg ttagatttcg gaattggttt   19680
taatggttaa ttttataaat tgagatggga gatttttttt gatgggaggt agtttttatt   19740
tttaaagttt atgttttagt tggaatgtat atgttaagga tttttgtttt ggttaatttg   19800
ggttttatat tgtgagtata taaaaagtat tatacggtta acggaggacg aggaattatg   19860
gtaaagtagg taggtaagtt ttaagaaata aaataatttg ttaaaaaata atttttgatg   19920
attatcgtaa gattgaaagt gtaggaaaaa tatagttcga ataattttag attttttttat   19980
attttttttt tttttatata ttttgttatt ttataataaa atttttaatg gaaagtttaa   20040
aaataaatag tataggaata tgtgttttaa atgaattaaa ttgtgaaatt agttagtaaa   20100
ttaatttgta gtaagtaatt atttaaggaa attaaaatat tgtttagttt agttttgtat   20160
tttattatgt gtatgcgttt tttataatta attaatataa gtgttttagg aatatttgaa   20220
gataaatacg tttaatttaa ggaataaagt atttaaataa tttaagtgta attttgttga   20280
gttaaagtaa aatattttat aaatgaagtg gttatttaat tttttaggga aagtttggtt   20340
attgaaatgt tgtatgttta tgttatatta ataaaaattt ttaatttatt ttgtttatgt   20400
gttttgtttt tttgatatta ttggtatttg aattttagat ggatttttgt taaaatgata   20460
ttttgtgtga taaaagtatt tttagttttg attgatagat taaaataaat gtaaggaaat   20520
ttttttaaat tagattaatt ttttataaaa atattttaga atgtatgaat tttgatattt   20580
atatttataa tggtaaaagt ttttttcgtt tagtttagta agataatatt tatataaaag   20640
agtaaaaaaa aattatatta ttttatgata gtttgatttt taaattgttt aagaaagtaa   20700
agtggttaaa ttggaaaaga ggaatatatt tcggaggttt agaatcgaaa atttttttttt   20760
```

taatttttag ttggaaaata attttttgta tttatttaaa gtgtattttt tgaagtgtta 20820
gattggagtt gattggtgat taatttaaag gagttataat ttaaagaaat ggtgagagtt 20880
tggtatttag gtttggtttt taggtaattc gtttgggttt gagaggttat taattgttag 20940
ttaagatgga attttttttt tttttttttt tttttaatgg ataataatgg gaagggggtt 21000
aatttttag tagttgaaat tttgtattta gtttttatt ttgagaatgt taatttttgg 21060
ttcgaggatt tgtttttgta gtgttggtat cgagatttaa gggaagatat ttcgttttaa 21120
atgttagtta cggtttggtt tttttttcga ttttagtatt ttgtagattg ttagtgtttg 21180
tggcgggga cgaaaggaat agggttttgt aaggtttgtt tgtcgattgc gttattttgg 21240
gcgaaattta gttttaaaag ttataaatta tttacggtga agatttttcg aagtggaata 21300
aatttttaga ttcgtattat tttatatttt tgcgggatag atggttttta tttatcggtt 21360
atcgggagag agttgttgtt ttcgcgtttt attgtttttc ggggcgattt ttagcgagtc 21420
gagttttcgg ttgtacggta agcgttcgaa agtcgggttt gagaggattg tagggttttt 21480
gagggtgtta agtttcgaag gagtttacgg gtgtattggg gttttcgaaa tttagtcgtt 21540
attggtagtt tttttttgtt tttttttagt tttttcgttc ggtttcgtat tttttttttt 21600
tttttttttt tttatttttt tttttttttt ttgtttttat ttcgtgtggg gagtgacgtg 21660
acgttagtag agattttatt aaattttatt gtatagtggc gcgcgggcgg tcggtcgagt 21720
tcggttgcgc ggttggcgat ttaggagcga gtatagcgtt cgggcgagcg tcggggggag 21780
cgagtagggg cgacgagaaa cgaggtaggg gagggaagta gatgttagcg ggtcgaagag 21840
tcgggagtcg gagtcgggag agcgaaagga gaggggattt ggcggggtat ttaggagtta 21900
atcgaggagt aggagtacgg atttttattg tggaaaggag gattagaagg gaggatggga 21960
tggaagagaa gaaaaagtaa tttgcgttaa ttcggtagtt ttaataaatt aaagggggag 22020
cgttagggta gcggggagat agaaacgtat ttttggggag taaattagga cgggttggga 22080
ggaagcgata gggaaagtgg tttaagagac ggaataaagg ataatgttta tggggttgtt 22140
tgggacgagg cgtgtggagt gtgggtgtga gcgtgcgtgt gtgatttttt tttaggtttg 22200
tagagttgag gaaagaggtt atagtaaaga gggattgcgg agggaggaaa gtgagagatc 22260
ggtagagggc gggagtggag gtgggcgcgg tggggatggg agaggatgag tgaagagaaa 22320
tttagaagaa tggagtgagt tagtgggaga gggtgggagg gttatagtcg ggagcgaacg 22380
agttaggttt gttagttggg gaaggtcggg acgttgggtt tagtttagtt gggatatcgc 22440
gttcgaggtt aaggcgggtg gattaggtat gttgagagtg tcggcgtata ggtgggtacg 22500

gttacgtatt gatttagtgt ttacgaaggg tttgtattgg ataaggttta gacgtttata 22560
gagtttagaa ttttttttgt tgtatttata tttaataagt ttattttggg ttacggatat 22620
tttattttt aaaatgacga ggttaaggtt tttggcgagg atggtattaa attgtacggg 22680
atagaagtgg gggtggggga gagagttttt tttaagttta tatttgtttt tgtaaagtaa 22740
agagtatgtg aaattatagg gtatattttt attcgaaaag tgtgttttat ttttgaattt 22800
tgattttttg attttttgat ttgagtaaag atgtgtattt tggtagtgag tagaatattt 22860
tggttttgtt ttgtttttga gtggaaggat tataaatata attcgtttgg aggattaggt 22920
gtgaaggttt ttgttaggta tatgggataa tgttttttta attttaaggg tattttgtta 22980
atgtatgttt ttggaaagtg tcggaatata gttattgttt ttggattcgg attttttat 23040
taatattaat ttttgtttga gagtaaaatt taggttcgtt attaaaaaga tatttttttg 23100
gtttttaatt gagaataaag ttttttttaa aagttgtatt gttttttta aattaatata 23160
ttaatattcg taattttaga aatatatagt gattcgggag aatgtgtata aaatagatac 23220
gtttaaaaaa gtttggcgtt taaaattaat tttagttatt atataggtgt tgggtttttt 23280
ttatttttgg gggttgtttg gaatatgtta tgtgtttttt tgaattattt cgtgttttga 23340
atttatttga gttagtagta aaaataggta aataaatttg tttaatttgt tttgagtgtt 23400
aaattttttt attttgaaat agttaatagt cgatagatgg atttatttta tggaaagggt 23460
tagtttttt agttacgaag aaaattgatt agagatttat attttaagtt atttttaatt 23520
tttacgtaat attcgtgaaa atttaaattt tttttttta tttagtggaa atttaaagta 23580
gtgttattta aggggagaga aatgaggggg aaaatgttta cgtgttgttt aattgtattt 23640
ttttttgat tttgagaatt tttattttg gtttttgaaa tttcgtcgag gtaagaaaat 23700
taaatttttt taataagttt tataattgaa ttttagttat aggatatcgg aaagtgtagt 23760
tcgagaaaga tatttttatt tttgtttatc gacgattttt gtagtttttt tattttttg 23820
agtaatgggt taataatttt tttttttttt tttttattt tgtagagatt aagaggcgtt 23880
cgtagtagaa cggttttgtt tttagttggt ggcgaggata ggtaatttta tggaaaagtt 23940
ggaagagaat gagaaaatta aagatagaaa gatttagaga ttcgcggaga gatataggga 24000
gagggaaggg agttgcgttg aaaagacgta aagatacgcg cgtgtaattt tttttttttt 24060
taggttttag aggtttgtaa attagggttg agaggaaggg gttcgggaag tttacgtttt 24120
tttcgttttt tttttgtttg gagtttcgtt cgttagaggt tggttaattt tagtttcggt 24180
cgtcgtagat attgcgttga gtttttgggt tttcgttttg tttagcgtta gtgtagttga 24240
agtgagtagt tggtgggaaa tgtaaatggt ttttggagaa atagaagata tagaatgatt 24300
tttattttt tttcgagtgt gtggaaggag ttggatatac gttttacgtt tttaattttt 24360

```
tttttatatt tttagttata tttttattaa ataattaatt aatgtttaga attattaggg   24420
aatatattag gtatgtaatc gtagaagtag ggtgttgggg ggttataaat tatcgagttg   24480
atttaagacg tggattttag gttttttttt tgttaaagta gtaaaggaag agcgggtttt   24540
ggcgattgta tttagatttc gattatttta aattagaagg gggtggaggg agcgtttaag   24600
taaagtaagt aatttttttgt tttgtagatg taaataagat tgtagtatta aaggtattag   24660
tttttttagg gttagatcgt ttggattggg agtttgggga aggggagata ttaattttac   24720
gtatttgtga attttaagga tgttatattt ttatataaat aattttagtg cggatttttt   24780
ggaatggggg gagtaatatt tttattttag aatattaaaa tatttttttt ttaaagcgta   24840
tatttttttt attttttaa aattttgaat tatgtttaaa gataatagtt ttttagtaaa   24900
ttggagtatt ggattatttt ttttattttt ttttatcgat attttgatga tttgatttta   24960
atgtgtgggg ggtataggga attaaatata gtttataaaa ttaagtttag atgaaatagt   25020
gttggttaag tgggtttaga taatttttaa tgagaatttt aattatattt tttttttaa   25080
tatgttgaga taagtgatag aatcgttaga atggtaatta aattggaaag tttagggaga   25140
ataataattt cgtgattaaa ttggggtaaa atcgtggata aatgtggggt gattttcgtt   25200
aatttttgt tatttaagag ttaggatttg ggaaaggtat agtattattt tagagttcgt   25260
tgtgacgggt tgtgtgttat tatttatttt ttttattttg gattatgatt ttaattttgg   25320
taagtaattt ttttagtttt ttatttgata ataagcgagt atgtaaatat taatggttag   25380
cgatgtttaa ttgttttaaa tattattgat ttgttggttg ttttaaattg ttttttagt   25440
ttaggttttg ttttcgaatt gtttatttta gaggtttgat ttatgttttc gatgttataa   25500
tataataatt gtttttttaa aaaaggtatt taagatgaat taattgattt gtatataaat   25560
taaaattatt atgcgttgtc gatttcggtg ttttataatt atttcgaaat tagtatttaa   25620
ttatttgagt taaaagaata tataaatgtt tgtattgatt tattaatgaa ttatttaatt   25680
aaaacgttcg ggtaatgttg ggcgttggaa agattgttaa attaagatat attataggag   25740
ggatatgaag attagaaagg taatagatta atatttcgta tttaaaacgg agttttcggt   25800
gatttttagt tttaattttg gagtaggggt ttttttttt tgttgttaaa aagattttgt   25860
gtttgtttgt gagtgagtgt atttaagtgg aaggaacgtt tttacggtta cggtggttta   25920
ggttttttgt tcggatcggg attttatagt tttaatttag gagcgttaaa ttttggaaga   25980
tttcgggtta gttttggagg tgcgtggttt cgtaagtcgt taggttaagt ttgttttttt   26040
tgtttgtttt ttcggtaggt tgggcgcgtt atggtagtga gtttttcgcg taaacggaga   26100
gttggaatta aagttgatat ttaatagata tgttaattga gtatttattt tcgttttgag   26160
aataggaata aaaggtagtt ttttttaaga gaggcggtgt aaaggtacgt tataggagtt   26220
tagaaaaggt tggcggcggg aaatttgtag tttgggggtt agttaatatt ttttttttatt   26280
ttaagtattt attgatttgt tgttgttatt tttggcgacg tagaaggata tttgaaagaa   26340
tttttgatgg ggttttgatt tgagaaagga ggtgatttgt ttaggttttt attaaatttt   26400
taattattat attaattgtt tttttttatt ttttattcga ttttttttt tttgtttatt   26460
tttaattttt taattattta gaaatttttt tattttttag tggtttttttt tttgtagtag   26520
ttttttattc gaattttttt ttcgtttttt cgtggtaggg tttgtatatt gattttttttg   26580
atttttggta tatttgggtt ttttgaaatt ttttaatttt tttagatttg aggatggtag   26640
gttttatttt ttttattgtg tgtatatatt tagagatatg aaaatttata tagattgttt   26700
ttaaatttag ggtatttaat agatgttttt tttttagttc gttttttgat ttgaaatgtt   26760
tgtttgattt taatttggat attatttttt tttgtttttt ttttttttaaa gtagtttgga   26820
tatgtgtgta agtgagttta gaatagtttt atttatattt tttattaaat tgtaaataaa   26880
agaagaatta atgaagtaga ttggtatata gattgtatta agagttcgaa tttttagttt   26940
ttggattttt tatttaattt tggttgttat ttatattgat agagttattt taagtagagg   27000
tttagagaaa tttgtattgt gggataatag gtaaagttat agtaaaaagt ggaataattt   27060
taaagttatt ttattagaat gtaaattgta tttttgggtt ttgttcgtaa ttatttagtt   27120
ttaatatata tagagttaga taggaaaaaa taggttaata tagttattgg tattagagaa   27180
gataaatttt atgggttttt tagtgaaaag aagattttta aagtttataa tttttgatta   27240
tttaatttta tttataattg tgggaatgaa taagatatta attgttttat gtattttatt   27300
tatattaatt aatttgtgtt tttattaaaa gtagttatat agaatttttt ttaattttttg   27360
gtagtaagtt tagaaaatga agtttatagt tattttgaat tggatatatt ttttgagttg   27420
attattttttg taagtgtagg aatataatat tgttttttta tggtttttttt gtattttttt   27480
agggtttgta agtttttatt aggtttgata ttattgtttg ggtttatatt tattataagt   27540
aaatttgatt attatgttga ttttaaaata gtttatttgg ttagtataat tttagttttt   27600
aaattataaa aatttttttaa tatacgaagt ttttagtttt tattttttttt agtttttttgt   27660
ttatttaaaa tttttatttt aattggtgta agtaataata atttgtatta ttatttgtat   27720
ttttttttatt tttttggaga ttgggttgga ttttagagag aatattagta ttattattat   27780
tataaataat aaaatttaaa agtaaagttt ttatttgtat gataattggt atttggaatg   27840
tttttgattt atttaatgtt attttataaa ggtattttgt aaatttttttt ggaattttta   27900
gtaagagttt gtagtaattg gaataatttt ttgggaagat attttttttg atgggttttt   27960
agtttttgga ggaatagatt gagagtaatt agggagggag gggatattgg aaattggtag   28020
```

```
ttacgttagt tgaaataagt ttgggtttag taaggtgatt gatgttgtgg ttgatttttt   28080
atttcgagtt ttttttaat tggggtattg atttttttta ttttgggatt ttaaggtatt   28140
cggtgtgtat gtagattttt tttttgtggt ttttattatg tggtttcgta gtaggttttt   28200
ggtttaatga tattttatag ttatagtttt tatatttatt attatgattt taatgtttag   28260
gtttttagtg tatttatatt aaatttgttt tattagtaag ttggagtata taggagagat   28320
gggggtaagt aaggatttag tagagtttaa atttagatat gtttaaatgg ttttgattgt   28380
gtaaagtgtg gtaatgtttt ttgttgtttt agttttttat tttaagtttt atatgttttt   28440
tggttaatga agtgtgatat aggttatatg ttaggaataa tagtatttgt tgagaataaa   28500
gtgaatttag gaaatttggt atatataaaa tgtatt                              28536
```

<210> 963
<211> 28536
<212> DNA
<213> Artificial Sequence

<220>
<223> chemically treated genomic DNA (Homo sapiens)

<400> 963

```
gatgtatttt gtgtatatta ggttttttga gtttattttg tttttaataa atattattat      60
ttttagtatg tggtttatat tatattttat tagttagagg atatgtgaag tttagagtgg     120
aaagttaggg tagtaggaag tattgttata ttttatatag ttaaagttat ttaaatatgt     180
ttgaatttga gttttgttga gtttttattt gtttttattt tttttgtgtg ttttagttta     240
ttaataaagt aggtttaata taaatatatt aaaagtttaa atattgagat tatgataatg     300
aatatgaggg ttatgattat aaaatattat tgaattagag atttgttacg aaattatatg     360
gtgaagatta taagggagga atttgtatat atatcgagtg ttttgggatt ttaaagtggg     420
aagagttagt gttttaatta aagagaaatt cggggtaggg aattaattat aatattagtt     480
attttattga atttaggttt gttttagttg acgtagttgt taatttttaa tgtttttttt     540
ttttttaatt gtttttaatt tattttttta gaaattgaaa gtttattaaa gaggatattt     600
ttttagagag ttgttttagt tattataagt ttttgttaga aattttaaga aagtttataa     660
ggtattttta taaggtggta ttgagtaagt tagaagtatt ttaagtatta attattatgt     720
aaataagggt tttattttta gattttgttg tttgtggtgg tggtgatgtt ggtgtttttt     780
ttggaattta gtttaatttt taaaaaagta aaaggagtat aaatagtaat ataaattatt     840
attatttata ttaattaaaa tagaagtttt gaatgagtaa ggagttagag gaggtaaaaa     900
ttgggaattt cgtatattaa aaggttttta taatttgaaa attaagatta tgttggttaa     960
ataggttgtt ttaaaattag tatagtaatt aaatttgttt gtaatgaatg tagatttaaa    1020
tagtgatgtt agatttgata aggatttgta aattttaaaa gagtgtaaaa agattataga    1080
agaataatat tatatttttg tatttataga aatggttagt ttaagagatg tatttagttt    1140
agggtggttg taagttttat tttttgaatt tgttattaga agttaaaaga aattttgtat    1200
aattgttttt gatggaaata taaattggtt gatgtaaatg aaatatataa agtagttggt    1260
gttttattta tttttataat tataaatgaa attaaatgat taaaaattat agattttggg    1320
gatttttttt ttattgagga gtttatggaa tttgtttttt ttagtattaa taattgtgtt    1380
gatttatttt tttttgttta attttgtata tattaaaatt aggtggttac gaataaaatt    1440
tagaaatata atttatattt taataaaatg attttaaaat tattttattt tttattgtgg    1500
ttttatttgt tgttttataa tgtaggtttt tttgggtttt tgtttagaat gattttgtta    1560
atgtagatga tagttagagt tgaatgggga atttagaaat tggggattcg ggtttttgat    1620
gtaatttata tgttaattta ttttattagt ttttttttta tttatagttt ggtaaagaat    1680
atgggtggag ttgttttggg tttatttgta tatatgttta aattgttttg aaaaaggaag    1740
ggtaagaaag agtggtattt aagttggaat taggtaggta ttttagatta agagacgaat    1800
tggaaaggga atatttgtta gatattttgg gtttgaaggt agtttgtgta agtttttata    1860
tttttgagtg tgtgtatata gtggagaggg tggagtttgt tatttttaaa tttgaaaaga    1920
ttgagagatt ttagagggtt tagatgtgtt aaaggttaga gggattaata tataggtttt    1980
attacggaaa ggcggggaaa aggttcgaat agaaaattgt tgtagaaggg aagttattga    2040
gaggtaaggg agtttttgaa taattaaaaa gttaagaata agtaaaagga aggaggtcgg    2100
gtgggggata aaaaaaagta gttgatgtgg taattaagaa tttggtggga gtttgggtag    2160
gttatttttt tttttagatt agagttttat tagaaatttt tttaagtgtt tttttgcgtc    2220
gttaaagatg ataatagtaa attaataagt gtttgaaatg aaaggggatg ttgattagtt    2280
tttaggttat agatttttcg tcgttagttt tttttgaatt tttatagcgt gtttttgtat    2340
cgtttttttt aagaagagtt atttttttatt tttattttta ggacgaaggt aagtgtttag    2400
ttagtatatt tattaaatgt tagttttggt tttagttttt cgtttgcgcg gaaagtttat    2460
```

```
tgttatagcg cgtttagttt gtcggagggg tagatagaaa aagtaagttt ggtttggcga 2520
tttgcggggt tacgtatttt tagggttggt tcggagtttt ttagagttta acgtttttgg 2580
gttagaattg taaggtttcg gttcgagtaa agggtttgag ttatcgtagt cgtgggagcg 2640
tttttttat ttgaatgtat ttatttataa ataagtataa aattttttta atagtagagg 2700
agaaagattt ttgttttaaa attaaagttg ggaattatcg gaaatttcgt tttgagtgcg 2760
agatattggt ttgttatttt tttaattttt atattttttt tgtaatatgt tttgatttaa 2820
taattttttt agcgtttagt attgttcgga cgttttaatt gggtaattta ttagtgagtt 2880
aatataggta tttatatatt tttttagttt aagtggttaa gtattaattt cgaaatgatt 2940
ataaaatatc ggaatcggta acgtatagta attttaattt atatgtaaat taattggttt 3000
atttaaatg tttttttaa aaaaataatt attgtattgt agtatcggag gtatggatta 3060
aatttttaga atagataatt cggaaataag atttggatta ggaagataat ttagaatagt 3120
taataaatta ataatgtttg aggtagttaa atatcgttag ttattggtat ttatatattc 3180
gtttgttgtt agataaggag ttggggaaat tgtttgttag ggttgagatt ataatttaga 3240
gtgaagaaag taaatggtag tatatagttc gttatagcgg gttttgaaat aatattgtat 3300
tttttttaaa ttttgatttt tgggtgatag ggagttggcg gaggttattt tatatttgtt 3360
tacggttttg ttttaatttg attacgaaat tgttgttttt tttgagtttt ttaatttgat 3420
tattatttta acggttttgt tatttgtttt aatatattgg ggggaggagt gtaattgaga 3480
tttttattaa aaattatttg aatttattta gttagtattg ttttatttaa gtttagtttt 3540
atgggttgta tttaattttt tgtgtttttt atatattaaa attagattat taaaatgtcg 3600
gtaggaaagg gtgaaggaaa tggtttaatg ttttagttta ttggaagatt attattttta 3660
gatatagttt aaaatttga ggaaataaaa aggatatacg ttttgggggg aaaatgtttt 3720
aatattttag aatgggggta ttattttttt tattttagag aattcgtatt ggagttgttt 3780
atgtaaaaat gtaatatttt tgaaatttat agatacgtaa ggttagtgtt ttttttttt 3840
taggttttta gtttaggcga tttagtttta aaggagttag tatttttgat gttataattt 3900
tgtttatatt tgtagggtag agaattgttt gttttgtttg gacgtttttt ttattttttt 3960
ttaatttgaa gtaatcggaa tttaaatata gtcgttaagg ttcgtttttt ttttattgtt 4020
ttgataaggg aaaaatttga aatttacgtt ttaaattagt tcggtggttt gtagtttttt 4080
agtattttgt ttttacgatt gtatgtttaa tgtatttttt ggtgattttg ggtattaatt 4140
agttgtttaa taggagtatg attaaaaatg taaaagaagg attaggagcg tgaaacgtat 4200
gtttagtttt ttttatatat tcgaggaggg aatgagaatt attttgtatt ttttattttt 4260
ttaggagtta tttgtatttt ttattagttg tttattttag ttgtattggc gttgggtaag 4320
gcgaggattt aaaagtttag cgtagtgttt gcggcggtcg ggattggggt taattagttt 4380
ttggcgggcg agatttaga tagaaggggg gcgagaggaa cgtgagtttt tcgagttttt 4440
tttttttagt tttggtttgt aaatttttga aatttgaaag gggagggagt tgtacgcgcg 4500
tatttttgcg tttttttagc gtaatttttt tttttttttt tgtgtttttt cgcggatttt 4560
tgaatttttt tgtttttggt ttttttattt ttttttaatt tttttatgag attgtttatt 4620
ttcgttatta gttgaaggta aggtcgtttt gttacgagcg ttttttaatt tttataaaat 4680
gaaaagaaaa aaagggagga ttattagttt attatttaga ggaatgggga ggttgtaaaa 4740
atcgtcgatg ggtagaggtg aagatgtttt tttcggattg tatttttcgg tgttttgtaa 4800
ttagagttta gttgtgggat ttgttgaaga aatttgattt ttttgtttcg gcgagatttt 4860
aaaaattaga aatagaaatt tttagagtta gagaggaaat ataattaaat agtacgtggg 4920
tatttttttt tttatttttt ttttttaaa taatattgtt ttgagttttt attgggtaaa 4980
gagagaaagt ttgagttttt acggatgtta cgtggaggtt agaaatggtt taaaatgtag 5040
atttttaatt agttttttc gtggttgaag aggttaattt tttttataaa atgagtttat 5100
ttgtcgattg ttagttattt taaagtgaag ggatttagta tttaaaataa attgagtaag 5160
tttgtttgtt tgtttttatt gttaatttaa atgaatttaa aatacggagt aatttaagaa 5220
aatatataat atgttttaga tagtttttaa aagtagggaa agtttagtat ttatatagtg 5280
attagggtta gttttaagcg ttaagttttt ttaaacgtat ttattttatg tatatttttt 5340
cgagttatta tatattttta aaattgcgag tattggtata ttgatttagg aagagtaata 5400
taatttttag agggaatttt atttttaatt agggattaaa gagatgtttt tttaatagcg 5460
ggtttgagtt ttgtttttaa gtaggaatta atattggtgg gaaaattcga atttaggagt 5520
aatggttgtg tttcggtatt ttttaaaaat atatattaat aggatgtttt tgagattgaa 5580
aaaatattgt tttatatgtt tggtagaagt ttttatattt ggttttttag gcgaattata 5640
tttatagttt ttttatttag aggtaggata gagttaaaat attttgttta ttattaaaat 5700
atatattttt gtttaagtta agaaattaga aaattagggt ttagaagtaa ggtatatttt 5760
tcgagtgaga atatgttttg taattttata tattttttgt tttgtaggag taaatgtgga 5820
tttgagggaa attttttttt ttattttttat ttttatttcg tgtaatttaa tattattttc 5880
gttaggaatt ttaatttcgt tattttaaaa aatgagatat tcgtgattta gggtgaattt 5940
gttgaatgta ggtatagtag aggaaatttt agattttatg agcgtttgag ttttgtttag 6000
tgtaaatttt tcgtgaatat tgggttagtg cgtggtcgtg tttatttgtg cgtcgatatt 6060
tttagtatgt ttggtttatt cgttttgatt tcgggcgcgg tgttttagtt aagttgggtt 6120
```

tagcgtttcg gtttttttta gttgataagt ttagttcgtt cgttttcggt tgtggttttt 6180
ttattttttt ttattagttt attttatttt tttagatttt tttttattta ttttttttta 6240
tttttatcgc gtttattttt attttcgttt tttatcggtt ttttattttt tttttttcgt 6300
agtttttttt tgttgtgatt ttttttttta attttgtagg tttgaaagaa ggttatatac 6360
gtacgtttat atttatattt tatacgtttc gttttaaata attttatgaa tattgttttt 6420
tgtttcgttt tttgggttat ttttttttgtc gttttttttt agttcgtttt gatttgtttt 6480
ttaaaagtac gtttttgttt tttcgttgtt ttggcgtttt ttttttgatt tattagggtt 6540
gtcgggttgg cgtagattgt tttttttttt tttttatttt attttttttt ttggtttttt 6600
tttttatagt gggagttcgt gtttttgttt ttcggttggt ttttaagtgt ttcgttaggt 6660
tttttttttt ttcgtttttt cggtttcggt tttcgatttt tcggttcgtt ggtatttgtt 6720
tttttttttt gtttcgtttt tcgtcgtttt tgttcgtttt tttcggcgtt cgttcgggcg 6780
ttgtgttcgt ttttggatcg ttagtcgcgt agtcgggttc ggtcggtcgt tcgcgcgtta 6840
ttgtgtagtg gagtttggtg gaatttttgt tgacgttacg ttattttttta tacggagtag 6900
gagtagaggg aagagagagg gatgagaggg agggagagga gagagagtgc gagatcgagc 6960
gagaaagttg gagaggagta gaaagaaatt gttagtggcg gttagatttc ggaggtttta 7020
gtgtattcgt ggattttttc ggaatttggt atttttagga gttttgtagt tttttttaggt 7080
tcggttttcg ggcgtttgtc gtgtagtcgg aggttcggtt cgttggaaat cgtttcggga 7140
agtagtggga cgcggagata gtagtttttt ttcggtagtc ggtaagtgga ggttatttat 7200
ttcgtaggga tgtgagataa tgcgagtttg gaaatttgtt ttatttcgga gaatttttat 7260
cgtaggtgat ttgtggtttt tggggttaag tttcgtttaa ggtaacgtag tcggtaaata 7320
gattttgtaa agttttgttt ttttcgtttt tcgttataga tattaataat ttatagggtg 7380
ttgaagtcga gagggaagtt agatcgtggt tggtatttaa aacgaggtat tttttttttaa 7440
atttcggtgt taatattgta ggaataaatt ttcgggttaa ggattagtat ttttaagata 7500
aagggttggg tataaagttt tagttattgg aagattagtt tttttttttat tgttatttat 7560
tgggaaaaaa aagaaaagaa aaagatttta ttttaattgg tagttagtga ttttttaggt 7620
ttaagcgaat tatttgggag ttaggtttgg atgttaagtt tttattattt ttttggattg 7680
taattttttt aaattgatta ttagttaatt ttaatttggt attttaggag atatatttta 7740
aatggatgta gagaattatt ttttagttgg agattaagaa aaaaattttc gattttaaat 7800
tttcgaaata tgtttttttt ttttagttta attattttat ttttttaagt aatttagaaa 7860
ttaaattatt ataaggtggt gtgatttttt tttatttttt tgtgtgagta ttgttttatt 7920
aaattaaacg gaaaaaattt ttattattat aaatgtaaat attagaattt atatatttta 7980
aaatattttt atgaaaaatt aatttgattt aaagaaattt ttttgtattt gttttagttt 8040
attaattaaa attaaagatg tttttattat ataaaatatt attttggtag aaatttattt 8100
aaaatttaaa tattaataat attaagaaaa taaagtatat aagtaaaata aattgaagat 8160
ttttgttgat gtaatatgag tatataatat tttaataatt aaattttttt taaaaaatta 8220
aatagttatt ttatttgtgg aatgttttat tttaatttag taaaattata tttaaattat 8280
ttaggtgttt tgtttttttaa gttaagcgtg tttgttttta aatgttttta aagtatttat 8340
attaattggt tgtaaagaac gtatatatat ggtaaaatat agaattgaat tgagtagtat 8400
tttaattttt ttaaataatt atttattata aattaattta ttggttaatt ttataattta 8460
gtttatttaa aatatatgtt tttgtgttgt ttatttttaa atttttttatt aaagattttg 8520
ttatggggta ataaagtgta tgaaaagggg ggaaatgtga aaggatttgg gattattcga 8580
attgtatttt ttttgtattt ttagttttgc ggtagttatt agaaattatt ttttagtaaa 8640
ttgttttatt ttttagggtt tgtttgtttg ttttgttatg gtttttcgtt tttcgttagt 8700
cgtgtagtgt tttttgtgtg tttataatat aaaatttaag ttggttaaaa taagagtttt 8760
tggtatatat attttaatta gaatatgaat tttgggggtg agaattattt tttattagga 8820
aaagtttttt attttaattt gtgagattag ttattgaagt tagtttcgaa gtttggtagt 8880
taaatttttt atagaagatt tgttttgata gggtaagttt aaggattagt aggcgggaat 8940
tggaggtttt tttttaaaaa attatttttt ttagttattt agatttagtt tttttagtag 9000
gtttggttat taaatgaagt ataaaaatgt aagttttaag gtttattttg attgtaaaat 9060
aaatttttaa gttataagga tatgtaggag tgagttaagg aatacgtttt gatttttttt 9120
ttagtttttta gagtggagtt ttatgagttt ttgaagattt gttttgtatt gttttgtttg 9180
gtttttagta ttgaagtacg gggaagtggg gggaagaatg tgtaataatt gattgatttt 9240
atattaagta acgtaatttt ttttttttgt atattttatt ttttaaaaaa aataaataaa 9300
taaaaattat ttgtagttat tatttgtagt gtttcggtta ttagttaata atgtagttag 9360
tttagatata taaaaaaaaa agattatcga aatgatgatg atatgtaaat ttttttcgaa 9420
attattataa gtaaatattt gaagtttgga ttaataaaat tttatttgtg ttattttata 9480
tcgagttagt agaaagttgt gataatgaat tttgtaatat tttacgaata gatattttaa 9540
ttagggatta attttgtgat tttattgtag aattattaaa tttggagtcg ttaaattgtt 9600
attttttgggt ttacgggttt ataaggatcg aatcggtaga gttttcgttc gcgttttcgt 9660
tagcgggtgg gggaatcgtt tggtcgtttt tattttggat ttttacgtta tagcgtcggg 9720
tagttttttt tgtaggtagc gattttggtt agaggttttt tagggtttag ttttttttag 9780

```
gagaggtcga gacgtaggga aacggtattt aggttagagg taggttcgta gtttttgtt     9840
tcgtttttgt gttttcgtta attcgataac gtttgttttt atttcgattt tcgtattcgc    9900
gcgaagtggg tttttcggtc gtcggcgtat tttggttagc gtggagagag gtaggcgttg    9960
agatcgaagg ggtttaggga gttttggatt ttttttttt gtttttaaag taatcgcggt    10020
tttttatttt attcggtgga gtttttcgag atttattttt ttcggtttgt ttgtggtaga   10080
gaagggggag cgcgttaaat gtttggttcg ttgcgttgtg gttgaaaacg tgaaaaagat   10140
ttggttcgtt cgggagagaa agggggagaa ttgggtagta gttatattag agttattttt   10200
tcgtttttgg cgggtagtaa attttttaag aacgtttgtt ttgtttttt tagtttcgtt    10260
tagtttattt agtgtttttt ttttgcgatt ttaaattata ttttagggta attatttgta   10320
gtaagtaaat aaatggtcgg gttagtattt ttaggagaaa gtgtggttaa atatggaaaa   10380
gtggtttttg atggatgaga ggttcgaatt tagttcgttt ttgaaatatt ttaggttaag   10440
agttcgttcg ttttagaatt atagaaaatc gagggaaatt gttgtttagg ataggggtac   10500
gttggcgttg atgttttata aatgtttatc gagttttaat taatggataa gtattgaagg   10560
gtggtttttg tatatagttt tttaaagaga aaagtttttt ttatttattt attttcgttg   10620

ttattgcgtt tagatgagtt tttaatttcg gtatcgagat ttttgaaagt aggtttatag   10680
ttttttagt atattgtggt tttatagttt tttaattttt gggtattttt gcggtaattt    10740
tggagggaga ttttttttg ataaataaat gttttgggtt cgaggttagg ttggagatgt    10800
tgttgtatag ttagaggttg ttaggtcgga aaaatacgtt tgaagtttag tatatagtag   10860
gcgtttaata gttagtgtaa cgtagtttta tttgagtttt gtttattcga cggtcgtcgt   10920
tttttatagt ttttttttt ttttgttttg tagataacgg ggaatggaga ttaattgtcg    10980
taaattggtg tcggcgtgtg tgtaattagg taagaatttt tttttttgt tcgggttatc    11040
ggacgggagg tcgcgttacg tgagggcggt aagagggtat tggttttgcg gcgaggtttt   11100
agcgaggggc gtttttcga ggggttagtt tgggtaggaa ggaaattaga attaaatcgt    11160
tagtggtttt tttttgtggc ggggcggtgg attaggaagt agcggcgcgt tgtgtatcga   11220
agtttttag tttatttttt tcggttggaa tcgtcggtaa tcggggaggc gtagaaagag    11280
tacgttattt tgtttcgggt tgttagaggg ttcgggggac ggggatgtcg ttagtttttt   11340
tttttaattg ggtttttgtt ttttgttttt ttttttttt cggtttgttt cgttttttt     11400
tttttttcg ttttcggttt ttttcggtcg cggttcggga cgttttttt cgtacgtggg     11460
gcgggcgcgc gcgtggttta ggcgtgtagt cggcggtcgt tgaatgtttt tttttaaag    11520
atttcgaaat taaaaaggtc gagtttacgg atttttttga gagtcgaaaa gaggtagtta   11580
gtagtaagtt tttttcgcgg tagtattttg gcgttaatgg taaggtcggg agggaagcgt   11640
aggtcgcgcg ttgggtattc gttttcggga ttttgggttt cgggcgaagc gtaagaaggc   11700
gaggtcgtta gatttgacgc gtttgttgtt tgaatttaga tatttcgttt ttgggtggga   11760
cgggaagtag tcgttttagg gacgttaatt ttttttttta aattatattg tatttttgag   11820
atttaatatt ttttttttt tttcgttcgt tttttgtggt ttgatttttt gcgtacgttt    11880
tagtaaattt cggcgtttag gttggcgtgg aaaagtggtt taatagcgat ttttgtcgtt   11940
cgttatattt cgttgcgcgt agtattatta gggtttattt agttatttag gtttttagtt   12000
acgttttatt tagatttgtg ggtgcgcggt ttatcgcggg aggtaagtaa gggaaatttg   12060
agttggcgaa ggtttgtttt ggttggttgg gggaggggcg gggggtcgat aatatttttg   12120
aagagttgga gggtagttat tgtgtttagt agtttagggt agaatggagg tttgtttttg   12180
tcgacgcgaa tttgtttgaa gtattggttg ttaggttttg ggttttggcg atgtcgttgt   12240
ttgattggtt ggttttattt tggaggaatc gagggatatt gttagaggag gtttataggt   12300
ttatgtaaaa agttaaaaag tttttaattt acgttatagg tttttttga attgaaattt    12360
gttttatggg gcggaggggg gggtgtaagg gatggaggag ggaagatgtt ttttttttta   12420
aatatatgga aaaaaatttt ttaaatttat tgttttttta tttttttggt ttcgtagtaa   12480
ataagtgttt agttttagga ggttattgat ttttgataat gtgagtagat aaagtttttt   12540
ttttttata gttttcggtt tttaattttt ttttttcgga ttaaagtgta agaatataaa    12600
tgtaatatgg gatggagggg ggcgatttgg gatttcggtt aaaaaaataa atcgtattat   12660
taagaagaaa ataaaggttt tgtattggag ttttttcgtg aatttgagag aaaatgatta   12720
tttgttgaaa tgaagcgttt aaagcgattt agtgttttac gttcggatat tgtattatat   12780
cgttagtcgt tttgttgggt tagttaaacg tttatttgtt cgggattaat tttatggggt   12840
taaatggggg taatgtagag ataacgtcgc gtgatttttg ttatttagat tgtgttaaat   12900
tttttttttg tttgataatc ggtagtaaaa ataaattatt agatcgtagt atgtttggga   12960
tatggttaaa aattaagagt agcgatgatt tttggggaga atgttttgcg cgggtttagt   13020
tttggtttcg gttagattag aggagttttt taatttcgtt tcgcgcgggg cgggttcgta   13080
gtcgttaagt cgaggttgat atttttttatt gtgttgggag ttagagagac gtaaaatgtt  13140
tttttttttt agtttttattt ttaggttttt tagatatggg gaatgtattt tgaggatagg  13200
tggagaagtt tacggtagga tggggttttc gtaggtgagt aggaaacggt taagagtaga   13260
ggagttttgt ttgcgttagt tataagtcgc gtaggcgttt ttggtcgttc gtttttgata   13320
attagtatat aaagaattag aaataatgaa tgattgtttt tttaattatt atttttaggt   13380
```

```
tcgtattgtt ttagtgtacg tgaaaggttt ttttttata tttaatatgt tttttttat 13440
tttttgatcg aaaagaaaaa ttgttgttta aatacgttta atgttattaa ttaagaaaag 13500
gtatgtaatg ggaagaaatg ttgaaaattt tgatttaatt ggtttttaag gaattagtag 13560
acgataaaaa aaaattatac gagtgggtaa agttatagta ttgttgaagg atagagtatt 13620
tattttttt tgattttaag ttaatttatg gaatatttaa agtttggtt atagtttgtt 13680
tgtaaaataa aaggatttat tttttgtgtt tttttaaagt tttttttgt ttttaaagag 13740
aaaaaaagtt tataatgata tatgattttt ttaaaaggtc gtgatagttt attacgttat 13800
ttttttcgtt tttgttttta acgtcgttta aaaatattat gttttcgtta aagattaaac 13860
gttttgtata ggtagagttt atttttaagt agtttaggtt ttgtttttt tttttagtga 13920
gttttatttt ttttggtatt tattgggcgg atgtttagtt tggatagaat ttcgaaacgg 13980
gggtagtacg agagcgattg gagattttta aaagttagag gtttgagaga gggtggacgt 14040
agttagtaga agatggtgta gaagttagtt gagaacgatt ttttagagta aagagatttt 14100
tttttggttt tttttgtttt gggggttttg aaaggaattt ataaaatggt ttttattttt 14160
aggaggagga cggattgatt ttttttgtt attggtttaa aaagttttag ggcggcggtt 14220
ttgggtttcg tgttgaaatc ggattgtatt gtagtttttt tggatttgac gtttggtttt 14280
gcgtttcgat aaggggcggg tattttttc ggttttttt aggaacgtat taattgttaa 14340
atagttttgg tttagtggat gggttgaaag tgttcgattt aagtcgttgg tgtgtataga 14400
tttttttttt ttgggaggtg ggttttatgg ttcgttgtgg tatttttagt cgcgatatat 14460
atttttatac gcggtagtag ttcggtttta atttttttcg aaggatttgg gttaattttg 14520
gcggttttgg cggtcgtaga tttttttcg tcgtttcgtt ttcgcgtttt ttatttaatt 14580
agcgaatgtt tgcggagtat atattacgtg gatttttaat gtattttttg aaagtaaata 14640
atatagtttt tttcgtcgtt atgaagggat tttaatttta atatggatat tagcgagatt 14700
agtttagatc gtttttagta aaacgtaaaa tggtggtgcg tggggtggtg attaaggttt 14760
tgagttttgt tagaaagaag gggatgtgta gagaaaggtg gagaatttta gttgtggtta 14820
gcgcggaagg gataggtgtt tgtcgaaggg ggtatgaggt ttgaggaaaa agtaacgaaa 14880
taggggtaag gagagttttt tattttttt ttttcgttcg attttcgtta ttttattttt 14940
tttttttttt ttttattttt cgcgttaatt aaatttgtag tttatttgaa aggtgtttcg 15000
tcgcgttgtg gtttttttatt tttaggggaa attgtattag ttgttcgaaa gtagttagtt 15060
tttgcggatt tttgttcgta aaagtggttt ttataggtcg cgtttttcgt tgttgattcg 15120
gtatataaag ttttttaagg ttggttcggt tgttattttt tatcgttcgt tgttaatata 15180
tgtagtagtt gttagagcgg ttcgggggaa aaggaaatgt ataacgaaag tttattcgtg 15240
agtaggaata tattaatgga ataatttgat gttttttaa ttttatgtaa aaagttttgt 15300
cgttttttta atattgattg aatgggtaat taatggtttt ttatttaggc gaatattttg 15360
taatttaaga taggtaaaag ataataagtt taaggtagaa gataaaaggt ttaattgtag 15420
cggcgtttgt tcgtttttta ttttttaggg tttttgatta ggaaagtttt tttttagagg 15480
agaaaaaggt aggagtggga gaatatatat ttattatttc ggggttagat tttatcgtag 15540
tatttgatta tttagtttag ttttttgtta ttttgttttt ttatttttag tttttttttt 15600
tgtatttttt ttttttttta attttttag gatgattttt tattattatc gttattatgg 15660
ttttaataat tttttttttt aaattttata ttttttattt tagtaattaa tgaggttgtt 15720
ttttgattta ggaggagatt tttttttttt agaatttaat gcgtagagtt tttgagaatt 15780
aaagtagttg gtaggggagg aagaaattaa tagaaaggga gagagtatat agaattgtgt 15840
gtgtatgtta aagagcgatt aggaatgata gagttaattt ttttgtgagg atttgacggg 15900
aagagtgttt aagattttat tagtatgttt ttaataggtt gatattttaa tttaaatttt 15960
tagaagtaat atattatttg ggttattata atgaggtggg tttttttttt tttgttagtt 16020
gatagttttt aaaatattat ttcgttaggg aaataaaagt tttattttag attataggtg 16080
ggtattttg gatttaggtg atttatggtt attatgataa ttaatgttga atgttagtta 16140
ttagtatgtt tgggagagag aaaatagaaa gaagggagag taaaagaaat agaaaaggga 16200
gatggatata agttggagag ggaagaaaag agaaaaagag gaagatagat gagtgtttaa 16260
tttaattgtt gtttaaaaaa gtggcggggg ggtgggattt tatttagttt tttgttattt 16320
tttttttttt tgatttggat atttatgttt aattttatat tttattttt tttttttttt 16380
tttaaatata tgtgttatat tattttttt attttattta gttcggtaag tagttgtttt 16440
ttggagattt agtgatattt aggaaaattg tggtagtaat atgtaaatgt gaggaagtat 16500
taatagtatg ttcgttgagt gattttagta aatgtttttt tttttaattt tttttttttt 16560
tttttttag gttattgtga ggtggtaatt tttattgtta tttgaatatt gtttttttag 16620
gtagttattt taaattttaa atggttgagt agttagagtt gtgggttgga aaaatgggta 16680
ttatttgtag ggatttagag agggtggttg ttgtttaata tatttataga tttttaattt 16740
agaaaataat tttttttttt tgataagtta gagtttttta aattttattt aggaaatggg 16800
gaaaaggata gttatagtga agtttttaat ttttgggtta tttggtttta tagttatgag 16860
gggtggtggg tagtggattg tttttagttt ggtttgtatg tagagaaaag ttagatattg 16920
gagggggtgg ggtattttc gggtaggatg taaggttttt atttgatttt tgcgttttat 16980
taggagttta tatatttacg tttattacgt ggttttaagt tgagtttagg cgggtttcgt 17040
```

```
ttttgagtta gtttgggtag ggtaggattt ttattcgttt aaggtttaat agtttaggga   17100
gatgtttaat taagttattt tttgggtgaa ttttgaagat agattttttt taaaagttag   17160
agattatttg gttgagtttt aggttagatt gatacggaga gtttggcggt atagtttaat   17220
cgtttattgt tatggttaga gggattttgt ataattaata ttgaagagtg tgaaattaaa   17280
taagatttta agattggtaa tcggtggtaa atattagtat aaaatacggt tgattttatg   17340
ttatatattt ttttttttta gggttttttt ttgaaagaat aagtaagaaa ttttaatcga   17400
gataattttt gatgtttttt agatttaaaa ttttacgtcg gtattgggtt tttttttttt   17460
gttttacgtg agttatgtag tatttttagt tttttattta ggattttatt aatgtttttc   17520
gtattggaaa tttttgtgtt agaggttgaa tttatagtaa tttttaaaat taattaagaa   17580
gaatttagtt agaggttata gtaatgttgg aattataaaa tgtataagat ttattttttt   17640
ttggtttttt ttttatttat gttgtttatg tttgtgtatt tataagtttt atgtatatta   17700
aatttttaaa attaattatt attatgttat agagttttta ttggatagtg tttttttagtt   17760
tttattatat attttttttt ttttatgtag atttattatg ttggtgtttt gttatatagg   17820
gggtttgaga agaatgttat ttaatcgtcg ttgttgtgag tgtgtaaagt gattaggaga   17880
ttaggagaat gttgaaattt ttgttggaaa aatgtaaaga aaatttttat tttgagttag   17940
ttgtttatag agttagtgtg tgtgtgcgtg tgtgtgtttg taatataaaa tggatgtgaa   18000
tatatatata taaatagata tggttttgtt tttattttaa tttgaattat ttagataatt   18060
gtttttattt attatttgat tttaatgggt ttatataaat taggatattt tatttttttta   18120
ggtatttagg ttgttgttga tttttagtgt ttttaatatt tcgtatacgt tggtattatg   18180
aggagtagtt acgtgttttt gggtttttta attattttgg aggttgattg aggtttttta   18240
tatatgtata tttgtcgtga tgaaagtttt atcggtagag tggagttatt agagttttta   18300
ttaaaatttt gtgggtttat gagagatggg tttagaaatt tatatggttt tgtggggttt   18360
ttcggttttt taaaataagg tattaatatt taagttttta aaaatatttg tagttttggg   18420
gtttgaattt tgaaaaataa ggagtgaggg gttgtgtata ttaattatag tggagatttt   18480
ttttattttt taatgtgatg gagttttttt atgaaatgaa gttttaaggg gtatggtatt   18540
gtggggatta tagttatttt gaggtttaaa agaagaaatt ggaatatgat tagtaaatat   18600
atttagtaga aaagagttgg atttttattg atttagttat aggttatcgg ttggtagtgt   18660
aatgggagga aatatttatt ttatatatat attttatgat tttgggggaa ttagaggaaa   18720
tttaataaga aaacggttag aaatatttaa aatttttatt taaaagattt aagtaaatta   18780
gagtttttatt agattaaaaa ttattataaa tgtaagagta ttgtttttag tgaaacgttg   18840
tggggtttga gaaggagatt tttcgttaaa ttttcgggat aaaatgcgtt atttaagtat   18900
tagataatga gtagaatgta aattaattta attttttttta ttaataggtt gttagtgtaa   18960
tgtgtataat ttagtgataa gattgtagga tttaatatag ttggatgtat gagttttagt   19020
taatgtagat ttgttatatg aggatgtgtt ttattttgag taggtgtttg tatgtgtgga   19080
atggggtaaa gtggaataaa aggttaaaag tagaaatgtt gatttaaagt ttattatgaa   19140
gaaatttttt ttttgtagtt aaattatttt taaagtggga tgatattggt gaagaaagat   19200
tgaaaaataa tttttatgtg cgtttttgga ttgtaagttt aaaatgggga ggagttgtag   19260
atagggtttg ggggtggtta gggtaaagga gagatatata agttgtaaat atatttgtag   19320
tttgttttat ttattttgtt ttatatcgaa taagtttttt aattttgtga ataaggataa   19380
ggagggagtg ttttaaagat attttatgtt ggtattgtaa attattgatt gtaatgttaa   19440
ataaatatat atttagagat gataatatta attttatagt aaaataatcg tttatgtaga   19500
aatttagagg agattagttt gtttttttta gttgatttat gttgggggat aaaaggattt   19560
ttaaaaatta ttttgaatat gtttggattt ttttttttaa tttttttgga aattaaattt   19620
gtttggaaat agtgttataa agagttgatg tttttaaagg tgattttttt tgttttatat   19680
aaataaggtt ttgtttttgt tagttgagcg tagttttagg tttttcgttt ttagtttata   19740
tatatttttt ttgtttgttt ggattttaat ggtttaagat agttttgagt ttattgggaa   19800
aagaaaatga ttgttaaaaa ttatttttga aattggttat ttggtaatat ttttaattgt   19860
atggaaattt attaaggtat attttatata taattagttt aaggttgttg attttatagg   19920
ttttatggat ttaaatttga ttgataataa agtaaataag agagtcgaat ttaaagcgtg   19980
gtttttttcgg gttaggacga gtttaatata gtgtataagg aatttgaaag atttaggata   20040
tgtgttttaa ttaacgttaa gtagaatgga taagttttta gtattttgaa aacgttgggt   20100
tagggttttt tttttattgt gtgtttttttg tttggggatt aataagtatt atagagaacg   20160
tgatttgagg cgattttttta tttttgtata aatttagagt gaattattaa atagttgttc   20220
gtttaaagtt aaggtaattt ttttttgacg ggtttatttg tttttcgatt tttaatttat   20280
tagtttgttt ttttagggtt ttgttttttt tgtaattaaa gttttttttag attagcgtag   20340
tatttatttg ataggttgtt tggaaaattt aagatcggag aggtgatttg ttgttgtttt   20400
ttaaattttt tagttttaag taacgtgttt ttttttttata tggggtgggg gattggaaat   20460
ggatgtagtg agatataaag agtgggtgtt ttgttgattt ttgtattttt tttttttttga   20520
ttattttatt tttttttttt aagttttcga tttttagttt tattttttta tttttgggtt   20580
cgtattaaaa gtcggatcgt tttgggttgg gtaggagttg aattttcggg agtttgtttg   20640
tgtagattta gtgcgtacgg cgaggtagta gttcggtttc gtattgttga taggtgtagg   20700
```

```
taggatagtt tttttatcgc ggttcggggc gttttgattg gtgcggagtt acgttagtcg   20760
tattcggaga agggtttggg aggaggcgga ggcggagagg gttggggagg gtcgcggcgg   20820
agtgacgttt cggtattagg aagttcgttt ttggttttaa gatgttaggt taatagggaa   20880
gcgcggagtc gtagatttgg ttcgtcgttc gtttgggtgt ttggagttga gttgcggtaa   20940
ggttcggttt ttgttcgatc gttcgagggg tgtgcgtgtg cgcgttgcgg agggtgcgtt   21000
tagagggtcg cgtcgtggtt gtagcggttg ttgtcgtcgt aggggattta atattattta   21060
tttgtttttg ttatttttga tatttttttg ttagggttgt cgcgtggggg gggggcgggt   21120
agagcgcggt cggcgttagt ttttttttatt ggaggggttt ttgggggagg gagggagaga   21180
agaagggggt ttttgtttat ttttgtttcg ttttggagtt tggaagtttg ttttttaaag   21240
acgttttgag tggtgttttt ttgtttatat tttatgtttt cgtttgttcg ttgatttttc   21300
gttttcggat tttttcgttt gagtttttcg gaggagacgg gggtagtttg gtttgagaat   21360
tcggcggggg ttgcgttttt tggttttttt cgtagcgggg aaatttcgcg tttagagcgc   21420
gattcggagc gggtagcggc ggttacgggg gttcggcggg gtagtagtta aggattagta   21480
gagcgtcgcg ttttttcgtt tatgaattgt atgaaaggtt cgttttattt ggagtatcga   21540
gtagcgggga ttaagttgtc ggtcgttttt ttattttttt gttattattt ttagtcgtta   21600
gttatggttt cggttttggt tttcggttag tttcggtcgt tggatttttt taagtatagg   21660
ttggaggtgt atattatttt cgatattttt agttcggagg tcgtaggtaa ggcgtcgcgt   21720
cgttttgtag atattttcgt ttagttgttt tgcgttattc gttttttttc gttttaagga   21780
agttagtttt ttcgggggga ggcgtggtgg gagtggtcgt tcgtttggtt tttcgtagaa   21840
ttttcgggag tcggaatttt gattatttcg tattttttta gttttttttc gatcggttcg   21900
gtttttgggg cgttaagggc gcgagtaatt ttgtcgtttt ttttattcgt attttggttt   21960
tttttttgtt ttttgggtta taaaaatttt agtatttgga ttcgaggatt tttagaggtc   22020
gtcgattttt gtttttgttt ttttttcggt ttttagtttt cgaggagttt tattcgttag   22080
gaaattgttt gaaattattt agaaatgttt ttcgcgaaga ggtatttttt tttttttttt   22140
gggaaagggt cggcgaattt cggtgtttaa tcgaattttt atattttttt ttagtttttt   22200
taaatcgtat ggaaatttga gtttttttgcg aggggggaggg gggtttgtaa atttacgcgcg   22260
tgtgcgcgtt ttaggagatt tggtgtgttt gcgtagaggt gtataaatat atttgaaagt   22320
ataggttata aaagtgaatg tgtcgttgta gtgagataaa tatgtaaata aaacgtgcgg   22380
cgttgggggga ggggaggaaa tggggcgcgg atatttatat ttgcgtttgt atattttata   22440
ggcgtagcgt ttttcgcggt tcggagtcgt cgcgcgtatt ttttttcggc gttaggtagt   22500
ttagtttttt tacggttttt gtcgtcggtt tagttggcgt tcgcgttgta ggtgggtatg   22560
ttgacgggaa agtgtgtgtg tttcgttttt agagaaagat aaaagttagt aggggaagaa   22620
tgaggacgtg ggcgtcgagg attcgtttaa gaagaagcgg taaaggcggt agcggattta   22680
ttttattagt tagtagtttt aggagttgga ggttattttt tagaggaatc gttattcgga   22740
tatgtttata cgcgaagaaa tcgttgtgtg gattaatttt acggaagttc gagttcgggt   22800
aggagttagt acggagtttg ggagggatgg ggggaggatg ttgtggaggt ataggttaag   22860
tagattagga gagaatgtgg aaggtagcgt cgtttgggag ggcgtcggtg gggcgtagtt   22920
ttgtaaaggt agaaggtttc gcggcggttt ggttgcgaga ttatagtttt tttttcgagg   22980
tcgataggat tgtcgttttg gtttaggttt ttagagcggt atcggtttat tgtttcgtta   23040
tttcgcgatt ttacgagttg ggttgtatgg gtaatttttt gtataggata ttgtgttttt   23100
ggtttgtagt tgttagagta gagttaataa aatttttatt aggttaagag tcgcgaatag   23160
gttttaattt gtgagttttt aataaggaaa attcgttaga gatacggaag agttggtttt   23220
ttttgggaaa tttttgtttc ggttttggtt tagttttttt ttttttgggt tcgcgttttt   23280
tatatttttt ttacggttgt ttcggttatt taggtttttt ttatatattt tattttttag   23340
ttttgtgatt ttcgggagta aagttttaat atataattat tagttttttt agaaggagaa   23400
agaaaaaaag aagaaagatt tttttgtttg gtttatttat tttttttttag gagttgaatt   23460
ttggaaattg aaatttatat tttttttttt aaattataat tatagttttg taaaaagggt   23520
ttattttaat tttgtagtaa atttgtattt tatggattgg taaaaatgag tttaaataaa   23580
taatttaata gtaacgtttt ggtttatgtt ggtcggtgga agattttaaa tttgttagga   23640
ttttggaagt agaaaataga attaagtaaa ttaagcggta tttagaggtt ttgttgttaa   23700
aaaaaaaaaa ttaagtgttt tgggtagaaa aaataaagtt ttcggttaga gtagagtaaa   23760
taaaaagaag aaaataacga taaaaagaat aaagattaaa atgttttttt aaattagagg   23820
gaatgaagat attttttggg tggtatttgt gtaaggtatg aggttatgtt ggtggataaa   23880
aggtcgggaa gaagttgaaa atggttttag tttaattgtt tagagttaga gttgggtttt   23940
gggcggcgtg gttttgagta aggttagttt tttattagtt tttttgtata ttaagggaac   24000
gggtttttta cgtatttttt tcgtttgagt aaagtttaga tggtttaggg tagaaatggt   24060
aagtaattaa agatagagtt tatgggtttt ttgggatttt tcgaaaacgt ttttttattt   24120
cgttcgttat ttcgtagttt tattttagtg ttttgtagtc gcggcgttgg gttttttttg   24180
tagttgtttt tttttttagg gcggttgttt gtcgagttaa gtgggagtga ggcgtgtttt   24240
ttatagtagt cgggtgtaaa gaggaagggg gataaaaagg aaattaagaa tgaaaggaaa   24300
aagagaaaaa gcggattata cggttgggtt cggcggagat gtgtaatgtg aaatattatt   24360
```

ggtgttagtt cggatatttt aggttaggtt ttttttaat atataaaagt cgtcgtttgg 24420
ggcgataggg aggttcgatg tggattggga tcggggttgc ggttgggtta tcggatacgg 24480
gtggaagtcg gtcggtttgg gtggtcgttt gtaaagttaa acgattcggt tgggtttggc 24540
gcgcggatag gtttgtggtg ggtttagggt aaagaagagg tagagcgaaa gaagggggaa 24600
tttttaaaat tattttttttc gggtttcgg agtttaatat gttaagtttt tggagttaac 24660
gagttgacga agaggtggtt ttttgttttt tatttggttg ttttgttagg cgagaaagag 24720
tgttggcggt ttagtttttg ttaagggagt acgtattagg gggtggggga cgatagtgga 24780
ggttagggaa ggaagggagg aattgcgtgg gagaaagagc gattttttag tgtttttta 24840
gtttttttt tttattcgtg ggtttgtggt tttggaatgg aagtaagttt gtaaggtgtt 24900
tcgggaaggg ttggaaaagt ttgttgtttc gcgtttgttt tatattaagt gtttttggat 24960
ttggagaaac gtttggttga gtgattaaat cgttcgtagg tttttatgcg ttcggttgag 25020
gtttgtggcg tagtttcgag ttttagttcg taggttagag tagattaggt tttttgcgtt 25080
tggtggagat tcgggttagt aattgaaagt tggttttggt attttggtgt gtagggcggt 25140
gtagtgaagc gaggttaggg tgtgtgagtg cgttagcgtg tgtgtcgggg gaaggcgggg 25200
gttggttttc gatggaagtt ttagtaattt gtattgtggt atttgtttgt tttttgttt 25260
taatcgtttt taggtttggt ttaagaatcg tcgggttaaa tggagaaaga gggagcgtaa 25320
ttagtaggtc gagttatgta agaatggttt cgggtcgtag tttaatgggt ttatgtagtt 25380
ttacgacgat atgtatttag gttattttta taataattgg gtcgttaagg gttttatatt 25440
cgttttttta tttattaaga gtttttttt ttttaatttt atgaacgtta attttttgtt 25500
attatagagt atgttttttt tatttaattt tatttcgttt atgagtatgt cgtttagtat 25560
ggtgttttta gtagtgatag gcgtttcggg ttttagtttt aatagtttga ataatttgaa 25620
taatttgagt agttcgtcgt tgaatttcgc ggtgtcgacg tttgtttgtt tttacgcgtc 25680
gtcgattttt tcgtatgttt atagggatac gtgtaattcg agtttggtta gtttgagatt 25740
gaaagtaaag tagtatttta gtttcggtta cgttagcgtg tagaattcgg tttttaattt 25800
gagtgtttgt tagtatgtag tggatcggtt cgtgtgagtc gtatttatag cgtcgggatt 25860
ttaggatttt gtcggatggg gtaatttcgt ttttgaaaga ttgggaatta tgttagaagg 25920
tcgtgggtat taaagaaagg gagagaaaga gaagttatat agagaaaagg aaattattga 25980

attaaagaga gagtttttttt gattttaaag ggatgttttt agtgtttgat attttttatt 26040
ataagtattt ttaatagttg taaggatata tatataaata aatgtttgat tggatatgat 26100
attttaatat tattataagt ttgttatttt ttaagtttag tattgttaat atttaaatga 26160
ttgaaaggat gtatatatat cgaaatgtta aattaatttt ataaaagtag ttgttagtaa 26220
tattataata gtgtttttaa aggttaggtt ttaaaataaa gtatgttata tagaagcgat 26280
taggattttt cgtttgcgag taagggagtg tatatattaa atgttatatt gtatgttttt 26340
aatatattat tattattata aaaaatgtgt gaatattagt tttagaatag tttttttggt 26400
ggatgtaatg atgtttttga aattgttatg tataatttat tttgtgtata atatttcgta 26460
taatattatt gttttatttt ttagtaaata tgaaataaat gtgttttatt ttatgggagt 26520
aaaatatatt gtatataaat tggtttggat ttttttttt ttttttttgtt attaatttgg 26580
ttaggatatt ttagttattg ttttttaaat aaattagttt tttttgtttg tttagttaaa 26640
tatataaggt agtagttttt atttaaattt ggtagaaata aatgatagtt atttattaga 26700
aattaaaaag aaaaaaaaag gtattttcgg gggggaaaag ggttataaaa tttaattttg 26760
ttttttttaat tttttttttgg tttaaattta gaggatttta ttatggttag taaataatat 26820
gaaaaagaaa aaagaagaaa gaaatttagt aagtttatta gtttaaaatg atttttaagt 26880
ttattttttt acggggaaat ttatattttt agtaaattgt tttggagaaa tatttgtgta 26940
tgtatatatg tatagtttat atgtattttt tttaggagga atatatttat aataaattta 27000
tagggaaata tttttagttt aaaatattta ggtttttacg tttattttta ggtttaagta 27060
gagagatttt ttatgttata ttgtattatt atttttaaat tttttggaga tattaaaaga 27120
aataaagatg atttttaata attatagttt tttagttttt taaagaattt aggggttgag 27180
aggttagagt ggagtttttt gagttttgtc gagtaatatg tagttgaggt aaaggttatg 27240
ttttcggtgt tttgttttaa ataatattga tttattaatt ttaaatttgt ttgtttttga 27300
aattatatag gattatagtt tgtaaattgt aggataatga agtaaattaa gatgaattat 27360
agttttggtt ttttttgtta tttttgata tttaaatagg gaatgagttc ggtgtgagtg 27420
tttaaatgaa ttttaagtat tcgatttttt tttattcgcg attttagtt ttaaaaaaat 27480
gtgaaatttg attttataat aaatagaaat aaatattatt tagttttaga gaatttattt 27540
ttatggcgtt aggagggtcg ttgtggaggt gggggaggg atgtgttgag atttttttgtt 27600
atgtttgtta atttttcgta taattaaagt gggcgagaat aaatattacg ttggggaatt 27660
tagagtaaaa agtaatcgtc gattttttgg agtcgataat attattgttt tttcgtttta 27720
gttgttttta tttgaatgaa attttattta gaagtttttg gagttcgaat attgagtttt 27780
tgttttgtaa gaaattgttg ttgttattta aagagatcgt agataatgtt ttcgatttaa 27840
gattagtgtt taacgtatat tttttttttt tttaaagttt tgttttcgat ttgcggaggg 27900
attacgtagt agtgggggggt agataaaagt tttttggacg agggttattt tttattatgt 27960

```
tgtttatttg agagtagtgg agaggaaaac ggtttttacg ggcggatttt ggtttttgga   28020
gtcgtagggt ttagcggttt cggtttttttc gtttttagt ttggtagggg gcggggaggg   28080
ttaaagggcg gaggggaagg aaggagttag aagaggggat ttggggatgg gggttggaag   28140
cgttaacgag atttgttcgg aggatttagg ttttttgtag gttggtgagt gatttgggag   28200
ttttgggtaa aagaggtgta tttcggttta gtttggttgt tgaattagaa taacgcgagg   28260
atattaatta atttgtagga aataaaaaat ggaagtcgag gtttaggaag agttgttatt   28320
ttttcgattt gagtggtgta ggttgggggc ggagatttgg gatttaagag aggttatttt   28380
ttttatttta gttttttttt tttggatttt taaaaggaat aattttattt tttttttcgt   28440
tttttttata atttttattt tcgttagttc gtaggtcgtt tgtttttttt cgtatttttt   28500
ttttttattt agcgagagaa atttagtttt tagagt                             28536
```

<210> 964
<211> 28536
<212> DNA
<213> Artificial Sequence

<220>
<223> chemically treated genomic DNA (Homo sapiens)

<400> 964

```
gttttgggag ttaggttttt tttgttggat gggaaaagga ggtgtggaaa ggggtaggtg      60
gtttgtgggt tagtggggat gagagttgtg gggagaatgg aggagagagt aaaattattt     120
tttttggaag tttaagggaa gggagttgag gtgagagaaa tggttttttt taggttttaa     180
gtttttgttt ttagtttgta ttatttaagt tggggaagtg ataatttttt ttaaattttg     240
atttttattt tttgtttttt gtagattgat tgatgttttt gtgttgtttt agtttagtaa     300
ttaaattagg ttgaggtata ttttttttat ttagagtttt taaattattt attaatttgt     360
aggagattta aatttttttgg gtaggttttg ttagtgtttt tagtttttat ttttaaattt     420
ttttttttag ttttttttttt ttttttttgtt ttttggtttt ttttgttttt tattaggttg     480
agaggtggag aggttgggat tgttgggttt tgtggtttta gaagttaaag tttgtttgtg     540
gggattgttt ttttttttat tgtttttaag tgaataatat ggtgggaggt gatttttgtt     600
taaagggttt ttatttgttt tttattgttg tgtgattttt ttgtaagttg gaggtagagt     660
tttaaaaaga aagaaagtgt gtgttgagta ttgattttaa attggggata ttatttgtga     720
tttttttaag tggtagtagt agttttttgt agaataaaga tttagtgttt gagttttaaa     780
agtttttagg taaagtttta tttagatgaa agtaattaag gtgaaaaaat aatgatattg     840
ttggttttag aaaattggtg gttatttttt gttttaagtt ttttagtgtg gtgtttgttt     900
ttgtttattt tggttgtgtg gggggttgat aagtataata aaagatttta gtatattttt     960
tttttttattt ttataatgat ttttttagtg ttatgaggat gaattttttg ggattaagtg    1020
gtatttgttt ttatttgtta tgaaattaaa ttttatattt ttttaaagtt gaaaattgtg    1080
gataaagaag gattgggtgt ttaaagttta tttaaatatt tatattgaat ttattttttg    1140
tttagatgtt agaggatggt agggagggtt agggttgtaa tttattttga tttgttttat    1200
tgttttgtag tttgtaaatt ataattttgt ataattttag gaataagtag gtttagaatt    1260
aatgggttaa tattatttaa aataaaatat tgggagtatg atttttgttt taattatata    1320
ttgtttgata agatttagga aattttattt taattttttta atttttgagt tttttgagaa    1380
attgaagggt tgtagttatt agaaattatt tttgtttttt ttaatgtttt taaaggattt    1440
ggaaatagta atgtaatata atatgaagga ttttttttatt taagtttgaa gataaatgtg    1500
gaagtttaaa tattttgaat tggagatgtt tttttgtaaa tttattatag atgtattttt    1560
tttgagagaa atgtatataa attatatatg tatatatgta taaatatttt tttagaataa    1620
tttgttagag gtgtaggttt ttttgtaaag gagtaaattt gagagttatt ttaagttgat    1680
ggatttgtta aattttttttt tttttttttt tttttttatat tatttgttag ttataatgga    1740
atttttttagg tttaagttaa agaaaaattg gagagataaa attagatttt gtagtttttt    1800
tttttttttgg gaatgttttt tttttttttt ttagtttttg atgaatggtt attatttatt    1860
tttattaaat ttaaataagg attgttgttt tgtatgttta attaggtagg tagagggaat    1920
tggtttgttt aggaagtagt gattgagatg ttttggttaa gttagtgata gaggagggga    1980
gaaagaattt agattaattt gtatgtagta tattttattt ttatgaaata aaatatattt    2040
gttttatatt tgttgaaaag taaaataata atattgtatg aaatgttata tatagggtag    2100
gttgtatata gtagttttag aaatattatt gtatttatta gagaaattat tttaaaattg    2160
atatttatat atttttttata ataataataa tatgttagaa atatatagtg tggtatttag    2220
tatatatatt tttttgtttg taagtgaaaa attttaattg tttttgtata atatgtttta    2280
ttttaaagtt taatttttaa aaatattgtt gtgatattat taataattgt ttttataaaa    2340
ttaatttgat attttgatat atatatattt ttttagttat ttaaatgtta ataatgttaa    2400
```

```
atttaaaaaa taataagttt atagtaatgt taaaatgtta tatttagtta aatatttgtt    2460
tgtgtatgtg tttttgtaat tgttagaaat atttgtagtg aaagatgtta gatattgagg    2520
atattttttt gaaattaaag gagttttttt tttgatttag tggttttttt ttttttatat    2580
agtttttttt tttttttttt tttttagtgt ttatgatttt ttagtataat ttttagtttt    2640
ttaagggtgg agttgtttta tttggtaagg ttttaggatt ttggtgttgt gggtgtggtt    2700
tatatgggtt ggtttattgt atattggtaa gtatttaggt tggaggttgg gttttgtatg    2760
ttggtgtagt tgaagttgga gtgttgtttt gtttttagtt ttaggttggt taggtttgag    2820
ttatatgtgt ttttataaat atatggagga gttggtggtg tgtaaggata ggtaggtgtt    2880
ggtattgtgg aatttagtga tgggttattt aggttgttta agttatttag gttgttgaga    2940
ttggagtttg ggatgtttgt tattgttgag ggtattatgt tggatgatat gtttatggat    3000
gagatagagt tgggtgggga aaatatgttt tgtgatgata gggggttgat gtttatagag    3060
ttgaagaagg ggaagttttt ggtggatagg gaggtggatg taaggttttt ggtggtttag    3120
ttgttgtagg aatagtttgg gtatatgttg ttgtagggtt gtatgagttt attgaattgt    3180
ggtttgaagt tatttttgta tagtttggtt tgttggttgt gttttttttt tttttatttg    3240
gtttgatgat ttttgaatta aatttggggg tggttggggt aagggagtaa atagatgtta    3300
tagtgtagat tattaaaatt tttattggag gttaattttt gttttttttt gatatatatg    3360
ttagtgtatt tatatatttt ggttttgttt tattgtattg ttttgtatat taagatatta    3420
gggttagttt ttagttattg gtttgggttt ttattaagtg taggagattt ggtttgtttt    3480
ggtttgtgag ttgggatttg gagttatgtt ataaatttta gttgaatgta tggagatttg    3540
tggatggttt gattatttag ttaggtgttt ttttaggttt aaaaatattt aatgtaaaat    3600
aaatgtgggg tagtaggttt ttttaatttt ttttggggta ttttgtaaat ttgtttttat    3660
tttaaagtta tagatttatg gatgaggaga aggggttgga agggtattag aggattgttt    3720
tttttttat gtaatttttt tttttttttt ttgattttta ttgttgtttt ttattttttg    3780
gtatgtgttt ttttaatagg gattaggttg ttaatatttt tttttgttta gtaaaataat    3840
taaataaaga gtaaaagatt attttttgt tagtttgtta attttaggag tttggtatat    3900
taaattttgg gaatttggaa agggtagttt tggagatttt tttttttttt gttttgtttt    3960
tttttatttt taagtttatt ataggtttgt ttgtgtgtta ggtttagttg ggttgtttgg    4020
ttttgtaggt ggttatttag gttggttggt ttttatttgt gtttggtggt ttagttgtaa    4080
ttttgatttt aatttatatt gggttttttt gttgttttag atggtggttt ttgtgtattg    4140
gagagaggtt tggtttgaga tatttgagtt gatattagtg atgttttata ttatatattt    4200
ttgttgggtt tagttgtgta atttgttttt tttttttttt tttttatttt tgattttttt    4260
tttatttttt tttttttttg tatttgattg ttataaaaag tatgttttat ttttatttgg    4320
tttgataagt agttgttttg gaaggagagg tagttgtaag gagagtttag tgttgtggtt    4380
ataaagtatt agggtggagt tgtggaatag tgggtggggt gggagggtgt ttttgaagga    4440
ttttagaaaa tttatagatt ttgtttttaa ttatttgtta tttttatttt aggttattta    4500
aattttgttt aggtgagaag agtatgtgag aggtttgttt ttttgatgtg taagagagtt    4560
aatgaaagat tgattttgtt taaaattatg ttgtttagga tttagttttg gttttggata    4620
gttaaattaa aattattttt aatttttttt tggtttttta tttattagta tagttttatg    4680
ttttgtataa atgttattta gagagtgttt ttattttttt tgatttggga gagtattttg    4740
gtttttattt tttttattgt tgtttttttt tttttgtttg ttttgtttta attgggggtt    4800
ttattttttt tatttagagt atttaatttt ttttttttaa tagtaaagtt tttggatgtt    4860
gtttgatttg tttgattttg tttttgtttt ttagaatttt aataaatttg gaatttttta    4920
ttgattagta taaattagga tgttgttatt gggttattta tttgagttta tttttgttaa    4980
tttataaagt atagatttgt tataaagtta aggtaagttt tttttataaa attatgatta    5040
taatttagaa gagggggtgt gagttttaat ttttagagtt taatttttga gagaagataa    5100
ataaattaag tagaaaagtt tttttttttt tttttttttt ttttttaaga ggattagtag    5160
ttgtgtatta aaattttgtt tttggagatt ataaaattag gaaatagggt gtgtgggaga    5220
gatttgaatg gttgaaataa ttgtaaagaa ggtgtaagaa gtgtgagttt aggagggaaa    5280
aagttgggtt agggttggga taaaggtttt ttagggaggg ttaatttttt tgtgtttttg    5340
gtgggttttt tttgttaaag gtttataggt tggagtttgt ttgtggtttt tggtttggta    5400
gggattttat tagttttgtt ttggtaattg taagttagga atataatgtt ttgtgtaggg    5460
gattgtttat gtagtttagt ttgtgagatt gtgggatggt ggggtagtga gttggtgttg    5520
ttttgggagt ttgagttagg gtggtagttt tgttggtttt ggagagggaa ttgtaatttt    5580
gtaattaggt tgttgtgagg tttttttgttt ttgtaaagtt gtgttttatt ggtgtttttt    5640
taggtggtgt tgtttttttat attttttttt ggtttatttg gtttgtattt ttataatatt    5700
ttttttttat ttttttaga ttttgtgttg gtttttattt ggatttgggt ttttgtaagg    5760
ttggtttata tagtgatttt tttgtgtgtg gatatgtttg ggtagtggtt tttttggaaa    5820
gtggttttta gtttttggag ttgttggttg gtaaagtgag tttgttgttg tttttgttgt    5880
ttttttttag atgggttttt ggtgtttatg tttttatttt tttttgttg gtttttattt    5940
ttttttgaaa atgaaatata tatatttttt tgttagtatg tttatttgta atgtggatgt    6000
taattggatt ggtggtagaa gttgtggaag agttgggttg tttggtgttg gaggagggtg    6060
```

```
tgtgtggtgg ttttgggttg tgaggagtgt tgtgtttgtg gggtgtgtag gtgtaagtgt    6120
gggtgtttgt gttttatttt tttttttttt ttagtgttgt atgttttatt tatatgttta    6180
ttttattgta gtggtatatt tatttttata gtttgtgttt ttaagtatat ttatatattt    6240
ttgtgtagat atattaaatt ttttgggatg tgtatatgtg tgtggtttat agattttttt    6300
tttttttgta gaaagtttag atttttatgt ggtttgggaa ggttaggaaa agatgtgggg    6360
atttggttgg gtattgaagt ttgttggttt ttttttaaaa aaaaaaaaaa aatgtttttt    6420
tgtgaagggt atttttgagt ggttttaggt aattttttaa tgagtggagt tttttgggag    6480
ttgaaagttg agaggaaaat agggatagag gttggtggtt tttgaaggtt tttgaattaa    6540
gatgttggga tttttgtgat ttaggaaata gaagggaggt tagggtatga atagagaggg    6600
tggtagaatt gtttgtgttt ttagtgtttt aggagttggg ttggttgagg gagaattaaa    6660
gggatgtggg gtagttaaaa ttttggtttt tggaagtttt gtggggagtt aggtgaatga    6720
ttatttttat tatgtttttt tttggagggg ttgatttttt tggggtgaga gggagtgggt    6780
ggtgtagagt agttgagtgg gaatgtttgt agggtggtgt ggtgttttat ttgtggtttt    6840
tgggttggag gtgttggaga tggtgtgtat ttttagtttg tgtttggagg agtttagtga    6900
ttggggttga ttgggagtta gaattgaagt tatggttaat ggttggggat ggtgatagga    6960
agatgaggag atggttgata gtttggtttt tgttgtttgg tgttttaagt gaagtgggtt    7020
ttttatgtag tttatggatg agggagtgtg atgttttatt agtttttggt tattgttttg    7080
ttgagttttt gtagttgttg ttgtttgttt tgggttgtgt tttaggtgtg gagttttttt    7140
gttgtgggga gagttagggg atgtaatttt tgttgagttt ttaagttaag ttgtttttgt    7200
tttttttgga aggtttaagt gaaaaagttt ggagatggaa agttagtggg taaatgaaga    7260
tatgggatgt gggtagaagg gtattattta gagtgttttt agggagtagg tttttaagtt    7320
ttaaagtgaa ataagagtgg gtaaagattt tttttttttt tttttttttt ttttaagaat    7380
ttttttaata aggaaagtta atgttgattg tgttttgttt gttttttttt tatgtggtag    7440
ttttgataga gaagtgttaa gagtgatagg gataggtagg tgatattaga ttttttgtgg    7500
tggtagtagt tgttgtagtt atgatgtggt tttttgagtg tattttttgt aatgtgtata    7560
tgtatatttt ttgggtggtt gaataggagt tgggttttgt tgtagtttag ttttaggtat    7620
ttaggtgagt gatggattag atttgtggtt ttgtgttttt ttgttggttt aatattttaa    7680
aattagaggt gggtttttttg gtgttgagat gttattttgt tgtggttttt tttagttttt    7740
tttgtttttg ttttttttta gatttttttt tgggtgtgat tgatgtggtt ttgtattaat    7800
taggatgttt tgagttgtgg tggagggatt gttttgtttg tatttattag tagtgtgggg    7860
ttgggttatt gttttgttgt gtgtattggg tttatatagg taagtttttg ggaatttagt    7920
ttttgtttag tttaaggtga tttggttttt agtatgaatt taaaggtgaa gagatgaggt    7980
taggagttga aggtttggga gaagagagtg gaatggttaa gaagagaaag gtataaggat    8040
taataagata tttatttttt gtgttttatt atatttattt ttaatttttt attttatata    8100
aaaaggagat atgttattta aaattagaaa atttgaaaaa tagtaataaa ttattttttt    8160
gattttaaat tttttaaata gtttgttaag tgaatgttgt gttaatttga agaagtttta    8220
attgtaaaga agatagagtt ttgaaaaggt aggttaataa attagaaatt gagaagtaaa    8280
tggatttgtt aaaagaaaat tattttgatt ttaaatgaat aattgtttgg tggtttattt    8340
tggatttata taagaataaa aagttgtttt agattatgtt ttttgtgatg tttattagtt    8400
tttagataga aaatatataa tagaagagaa attttaattt agtgttttta aaatgttgaa    8460
agtttattta ttttatttaa tgttgattaa gatatatatt ttagattttt taaatttttt    8520
gtatattgta ttaagtttgt tttaatttga gagagttatg ttttaaattt gatttttttg    8580
tttattttat tattaattag atttaaattt ataaagtttg tagaattaat aattttgagt    8640
taattatata tgaaatatgt tttaatgaat ttttatataa ttaagaatgt tgttaaataa    8700
ttaattttaa ggataatttt taatagttat tttttttttt tagtgagttt aaggttgttt    8760
tgagttatta aagtttaagt aggtagaagg ggtgtgtgtg agttaagggt gaaaagttta    8820
gaattgtgtt taattagtaa aagtaaaatt ttatttatat aaaataaaaa aaattatttt    8880
tggagatatt aatttttttat agtattgttt ttaagtaaat ttaattttta aagaaattaa    8940
agaaagaaat ttaaatatat ttaaaataat ttttgaaagt ttttttgttt tttagtatag    9000
gttagttgga gaggataaat taattttttt tgggtttttg tatgggtgat tgttttatta    9060
tggagttagt gttattattt ttgaatgtgt atttgtttga tattatagtt aatgatttgt    9120
aatgttagta tgaagtattt ttaaaatatt tttttttttgt ttttgtttat aagattggga    9180
aatttatttg atgtggaata aagtggatga agtagattat aaatatattt gtaatttatg    9240
tgtttttttt ttgttttgat tatttttaaa ttttatttgt aatttttttt tattttaaat    9300
ttgtagttta aagatgtata tgagaattgt tttttagttt tttttttatta gtattatttt    9360
attttaagaa taatttagtt gtaagggagg aattttttta tagtaagttt taaattagta    9420
tttttgtttt taatttttta ttttatttta ttttatttta tatatataga tatttgttta    9480
gagtaaaata tatttttatg tgataggttt gtattagttg aggtttatat atttagttat    9540
attaggtttt gtaattttat tattaaatta tatatattat attagtagtt tgttggtaaa    9600
gaaggttaaa ttaatttata ttttgtttat tatttggtgt ttaaatgatg tattttattt    9660
tggagatttg gtggagaatt tttttttag atttatagt gttttattga agataatgtt    9720
```

```
tttatatttg tagtggtttt taatttgata agattttaat ttgtttaagt tttttaaata    9780
agggttttaa atgtttttag ttgttttttt attgaatttt ttttaatttt tttaagatta    9840
taaagtatat gtgtaaagta aatatttttt tttattgtat tgttagttga tgatttataa    9900
ttaagttaat aagaatttag tttttttttg ttgaatgtgt ttattaatta tattttagtt    9960
ttttttttta aattttagaa tagttgtggt ttttataata ttatgttttt taaagtttta   10020
ttttatgaag ggattttatt atattaaaga atgaaaaaaa tttttattgt agttagtata   10080
tatagttttt tattttttgt tttttaagat ttaaatttta gagttgtaaa tatttttgga   10140
agtttgggtg ttaatgtttt attttagaaa gttgagaagt tttatagagt tatatagatt   10200
tttaaattta ttttttataa atttatagaa ttttgataaa agttttggtg gttttatttt   10260
attgatggaa tttttattat gataaatata tatgtatgaa ggattttaat tagtttttaa   10320
agtggttgaa aaatttaagg gtatgtgatt gtttttttata gtgttaatgt gtgtgagatg   10380
ttggaagtat tggggattag tagtagttta gatgtttaaa aagataaggt gttttaattt   10440
gtgtggattt attgaagtta agtggtgaat aaagataatt atttagataa tttagattaa   10500
agtaaaagta aaattatatt tatttgtata tatatattta tatttatttt atattataga   10560
tatatatatg tatatatata ttggttttgt aaataattga tttaaagtga ggattttttt   10620
tgtatttttt tagtaggagt tttaatattt ttttaatttt ttaattattt tatatattta   10680
tagtagtggt gattgggtga tatttttttt aggtttttttg tgtggtagga tattaatatg   10740
ataagtttgt atggggaaaa ggaggtatgt ggtgggaatt aagaaatatt gtttagtgaa   10800
aatttgtgg tatggtggtg gttgattttg gagatttaat gtatataaga tttgtgggtg   10860
tataggtata ggtagtatgg atgagaaagg ggttagaaga aaataaattt tatgtatttt   10920
gtgattttag tattattgtg atttttggtt aagttttttt taattggttt tagaaattat   10980
tatgagttta gtttttaata tagaaatttt taatatggag aatattggtg ggattttggt   11040
agggaaatta gaggtgttgt atggtttatg tggggtaaag aaggaaagtt tagtgttggt   11100
gtgaggtttt gagtttggga gatattaggg gttgttttga ttggggtttt ttgtttattt   11160
ttttaaagaa agattttaga ggagggaaat gtgtgatatg gggttagttg tgttttgtgt   11220
tggtatttgt tattgattat tagttttaaa gttttattta attttatatt ttttagtgtt   11280
agttgtgtaa agttttttttg gttatggtag tgagtggttg ggttgtgttg ttaaattttt   11340
tgtattaatt tggtttggga tttaattaag tgatttttga tttttggaaa gagtttgttt   11400
ttagagttta tttagaagat ggtttaatta gatatttttt tgagttgtta ggttttagat   11460
gggtgggagt tttgttttgt ttaagttagt ttaaggatga ggtttgtttg gatttagttt   11520
ggagttatgt gatgggtgtg agtgtgtgag tttttggtaa ggtgtagagg ttagatggag   11580
attttgtatt ttgtttgaga agtgttttat tttttttaat atttggtttt tttttgtata   11640
taaattaagt tgaaaatagt ttattattta ttattttttta tagttatgga attaaataat   11700
ttagaaatta aaagttttat tgtagttgtt tttttttta tttttaaat ggaatttaaa   11760
aagttttggt ttgttaaaag gggaagatta ttttttgaat tggaagtttg tagatatatt   11820
gagtaatagt tatttttttt gggtttttgt aaatggtatt tatttttttta atttatagtt   11880
ttagttgttt aattatttga gatttggggt aattatttgg gggaatagtg tttagatggt   11940
agtgggagtt attattttat agtggtttgg ggaagagaag agaaagagat tagaggaggg   12000
ggtatttgtt aaaattattt aatgaatatg ttgttaatgt ttttttatat ttgtatgtta   12060
ttgttatagt tttttaggt gttattgagt ttttagaaag taattatttg ttgaattaag   12120
taaaataagg agaatggtat agtatatgtg tttggagaag gggaaggaag ggtggaatat   12180
gaaattgagt atagatattt aggttaggaa agaaggaagt ggtaaggggt taaatgaagt   12240
tttatttttt tgttattttt ttaaataata gttggattaa atatttattt gttttttttt   12300
ttttttttt ttttttttt ttagtttatg tttattttt tttttattt ttttgtttt   12360
ttttttttt tgttttttt tttttagata tgttggtagt taatatttag tattagttgt   12420
tatggtgatt ataaattatt taaatttaaa aatatttatt tataatttga gatgaagttt   12480
ttattttttt agtgaaataa tattttaaaa gttgttagtt gataaaaaaa aaggaattta   12540
ttttattgta gtaatttaag taatatatta tttttaaagg tttaaattaa aatgttagtt   12600
tgttaaaaat atgttggtag agttttggat attttttttg ttagattttt ataaagaagt   12660
tgattttgtt atttttggtt gtttttttaat atatatatat aattttgtat gttttttttt   12720
tttttattaa tttttttttt ttttattaat tattttagtt tttaaagatt ttatgtattg   12780
ggttttaaaa gaaaagaatt tttttttgga ttagaaaata gttttattgg ttgttgaagt   12840
gaaagatgtg gggtttaggg ggaaaggtta ttaggattat aatggtggtg gtggtaggag   12900
gttattttag aggagttaag aagaaaaaaa aatgtaggga gaaggattgg aggtggaaag   12960
atagagtaat agaaaattga gttgggtggt taggtgttgt ggtgaagttt agttttgaaa   13020
tgataggtat atattttttt atttttgttt tttttttttt tgagagaaaa ttttttttagt   13080
tagagatttt ggggggtagg aggtgggtaa atgttgttgt agttgggttt tttgtttttt   13140
attttgggtt tgttgttttt tgtttatttt ggattatagg gtatttgttt agatgaagag   13200
ttattaatta tttatttagt taatattagg aagatgataa agttttttat ataggattaa   13260
gaagatatta gattgtttta ttagtatatt tttgtttatg aataagtttt tgttatatat   13320
tttttttttt tttgagttgt tttaataatt gttgtatata ttagtagtgg gtggtgagga   13380
```

```
ataatagttg aattagtttt aagaaatttt gtgtattgag ttagtagtga ggaatgtgat   13440
ttgtgaagat tatttttgtg ggtagggatt tgtagggatt gattattttt ggataattgg   13500
tataattttt tttgggggtg aaaaattata atgtggtggg gtattttta agtgagttgt   13560
agatttgatt ggtgtggggg gtgggggagg ggaggggaga atgggatggt ggaggttggg   13620
tggaggaaag aaaatggaaa atttttttta tttttatttt gttgtttttt ttttaagttt   13680
tatgtttttt ttggtaagta tttgtttttt ttgtgttagt tatagttaga gtttttttatt   13740
tttttttata tatttttttt tttttgataa ggtttaggat tttggttatt attttatgta   13800
ttattatttt gtgttttgtt agagatggtt tgggttgatt ttgttggtgt ttatgttagg   13860
attaaaattt ttttatgatg gtgaggaaaa ttgtattatt tgtttttagg gggtatatta   13920
ggagtttatg tagtatatgt tttgtaaata tttgttgatt gaatgagagg tgtgggggtg   13980
gggtggtgga gaggggtttg tggttgttaa ggttgttagg gttaatttag gtttttttgaa   14040
gaaggttggg attgagttgt tgttgtgtgt gaaggtgtgt gttgtggttg ggggtgttat   14100

aatgggttat ggagtttatt ttttagaggg aggaagtttg tgtatattag tgatttgggt   14160
tgaatatttt tagtttattt attgggttaa agttatttaa taattaatgt gtttttgggg   14220
gaggttgggg gaagtatttg ttttttgttg ggatgtaaag ttaggtgtta ggtttaaagg   14280
ggttgtagtg tagtttgatt ttagtatgga atttagagtt gttgttttga aattttttaa   14340
gttagtgata gaggagggtt agtttgtttt tttttgagg gtgaagatta ttttatgagt   14400
tttttttagg atttttaaag taagaaaagt taaagaaagg ttttttttgtt ttagggggtt   14460
gtttttagtt ggtttttata ttattttttg ttagttgtgt ttattttttt ttaaattttt   14520
ggtttttagg ggtttttagt tgtttttgtg ttatttttgt tttggggttt tatttaggtt   14580
gggtatttgt ttaatggata ttaaggagaa tgggatttat tagggaagga aggtagagtt   14640
tggattgttt agaggtggat tttgtttata tagaatgttt agtttttaat gaggatatgg   14700
tatttttggg tggtgttggg ggtagaggtg gagagggtag tgtaatagat tattatggtt   14760
ttttgaagaa gttatatgtt attgtgaatt tttttttttt ttaaaagtaa agaaaaattt   14820
taaaaaaata taagaaataa atttttttgt tttataagta ggttgtggtt aggattttgg   14880
atattttata agttaattta aaattaggga aggataggtg ttttattttt tagtagtgtt   14940
atagttttgt ttatttgtgt gatttttttt tgttgtttat tagtttttta aaagttaatt   15000
aaattaagat ttttagtatt tttttttatt atatgttttt ttttaattaa tggtattaaa   15060
tgtgtttagg tagtaatttt tttttttggt taaaaagtag aaaaagatat attgagtgta   15120
ggggaagagt tttttatgtg tattaaaata atgtgggttt gaaagtaatg gttaagaaag   15180
taattattta ttatttttag tttttatgt gttagttatt aaaagtgaat gattaggggt   15240
gtttgtgtgg tttgtgattg gtgtaagtag aattttttttg tttttagttg tttttttgttt   15300
atttatgagg attttatttt attgtaggtt tttttatttg tttttagaat gtattttttta   15360
tgtttaggaa atttggggta gggattgggg gaaggagata ttttgtgttt ttttggtttt   15420
tagtataata agaaatgtta gttttggttt ggtgattgtg agtttgtttt gtgtgaagtg   15480
agattgggga gttttttttag tttggttgga gttagggttg agtttgtgta aagtattttt   15540
tttagaagtt attgttgttt ttgattttta attatatttt aaatatatta tggtttaata   15600
atttattttt attattgatt attaaataga agaagaattt aatataattt aaatgataga   15660
aattatgtga tgttattttt gtattgtttt tatttaattt tatggggtta attttggata   15720
agtgagtgtt taattggttt agtagggtga ttggtggtgt agtgtagtgt ttgggtgtga   15780
agtattggat tgttttagat gttttatttt aataaatgat tattttttttt tagatttatg   15840
gggaaatttt aatgtaagat ttttgttttt tttttagtaa tatggtttgt tttttttgatt   15900
ggggttttaa attgtttttt tttattttat attatattg tatttttata ttttaatttg   15960
gaaagagggg gttaggggtt gagggttgtg gggggggggg ggttttattt gtttatatta   16020
ttaaaggtta atagtttttt aaggttaggt atttatttat tatggagtta ggaaaataga   16080
ggaatagtaa atttgagggg ttttttttta tgtatttgaa aagaaaggta ttttttttttt   16140
tttatttttt atattttttt ttttgtttta tagaataagt tttaatttag gaaaggtttg   16200
tggtgtaggt tggagatttt ttaatttttt atataagttt gtagattttt tttggtaatg   16260
ttttttgatt tttttagagt gaaattagtt aattaagtaa tgatattgtt aaaatttaag   16320
gtttggtaat tagtatttta ggtaggtttg tgttgatagg ggtaaatttt tattttattt   16380
tgggttgtta agtatagtgg ttgtttttta gttttttagg gatgttgttg gtttttttgtt   16440
ttttttttaa ttagttaaag taaatttttg ttaatttaag ttttttttgt ttgtttttttg   16500
tgatgaattg tgtatttata agtttgggtg gggtgtggtt gagagtttga gtgattgagt   16560
gggttttggt ggtgttgtgt gtagtgggat ataatgagtg atagaggttg ttgttggatt   16620
atttttttat gttagtttag atgttgaggt ttgttggagt gtgtgtaggg gattagatta   16680
tagggagtga gtgagaggga gagagaggtg ttgggtttta ggagtgtagt ataatttggg   16740
gaaaggaatt aatgtttttg ggatggttgt tttttgtttt atttagaggt ggagtgttta   16800
agtttaagta gtaggtgtgt taggtttggt ggttttgttt ttttgtgttt tgtttgaggt   16860
ttagagtttt ggaggtgggt gtttagtgtg tggtttgtgt tttttttttg gttttattat   16920
tggtgttagg atgttgttgt gggaagaatt tgttgttggt tgtttttttt tggtttttag   16980
```

gagagtttgt gaatttgatt tttttgattt tggagttttt ggagaagaga tatttaatgg 17040
ttgttggttg tatgtttggg ttatgtgtgt gtttgtttta tgtgtggaga gaggtgtttt 17100
ggattgtggt tgaaaggagt tggggatggg aggaggggga ggggtgaggt aggttggagg 17160
agaaagaggg ataaagagta aagatttagt tagaggaaag agttgatggt atttttgttt 17220
tttggatttt ttggtaattt ggggtaggat ggtgtatttt ttttgtgttt ttttggttgt 17280
tggtggtttt agttgggagg agtaggttgg ggggttttgg tatatagtgt gttgttgttt 17340
tttagtttat tgttttgtta tagggagagg ttattggtga tttggttttg attttttttt 17400
tgtttaggtt ggtttttggg ggaagtgttt tttgttgggg ttttgttgta gggttagtgt 17460
tttttttgttg tttttatgtg gtgtggtttt ttgtttgatg atttgggtag gagaaggggg 17520
tttttattta attgtatata tgttgatatt agtttgtggt agttggtttt tattttttgt 17580
tatttgtaaa atagaagaga aggaaggttg taagaagtgg tggttgttga gtgagtaggg 17640
tttagatgag attatgttat attagttgtt aggtgtttat tgtgtgttag gttttaggtg 17700
tgtttttttg atttgatagt ttttggttgt gtagtagtat ttttagttta gttttgggtt 17760
taggatattt atttattaag aggggatttt tttttagagt tgttgtaaaa gtgtttagag 17820
gttagaggat tataaagtta tagtgtgttg gggaggttgt ggatttattt ttaagaattt 17880
tggtgttggg gttaagaatt tatttgaatg taatggtagt gggagtgggt gggtggagag 17940
gatttttttt tttgggaagt tgtatgtaaa gattattttt tagtgtttgt ttattagttg 18000
gagtttggta aatatttgta gaatattagt gttaatgtgt ttttgtttta gatagtagtt 18060
ttttttggtt ttttgtaatt ttgaaatgaa tgggtttttg gtttagggtg ttttaggagt 18120
gagttgagtt tgggtttttt atttattagg agttattttt ttatatttag ttatattttt 18180
ttttagagat attaatttgg ttatttattt atttattata aataattatt ttaaagtatg 18240
atttaagatt gtagaggaga gatattgggt ggattgagtg agattgagga gagtagggta 18300
aatgtttttg gagggtttat tgtttgttaa ggatggagaa atagttttgg tataattgtt 18360
atttagtttt tttttttttt tttttgggtg agttaaattt tttttatgtt tttaattata 18420
atgtagtgag ttaagtattt aatgtgtttt tttttttttg ttataggtaa gttgggagag 18480
gtgggttttg aggggtttta ttgggtgggt agaagagttg tggttgtttt aaagataaga 18540
aaagaaggtt tagggttttt taggtttttt tgattttagt gtttgttttt ttttatgtta 18600
attagggtat gttgatgatt ggagggttta ttttgtgtgg gtgtggggat tggggtggga 18660
gtaagtgttg ttgggttggt ggaggtatag aggtggggta gggagttgtg ggtttgtttt 18720
tggtttgagt attgtttttt tgtgttttgg ttttttttga agggagttgg gttttgggga 18780
gtttttggtt aaggttgttg tttataggag gggttgtttg gtgttgtggt gtggggattt 18840
agggtgggga tggttaggtg gtttttttat ttgttagtga gaatgtgggt ggggattttg 18900
ttgatttgat ttttgtgggt ttgtgggttt agaagtagta gtttggtggt tttagattta 18960
gtgattttgt agtaaaatta taggattagt ttttgattga gatgtttgtt tgtgagatat 19020
tataaaattt attattatag ttttttatta atttgatatg aagtaatata gatgggattt 19080
tattagttta gattttaaat gtttatttat gataatttgg gaggaaattt gtatgttatt 19140
attattttga taattttttt tttttatatg tttgaattgg ttgtattatt agttggtagt 19200
tggagtattg tagatggtaa ttgtaaatag tttttattta tttatttttt ttaaagaatg 19260
aaatatataa aagaaaaaga ttgtgttgtt tggtgtaaag ttagttaatt attatatatt 19320
ttttttttta tttttttgtg ttttagtgtt gaagattaaa taaagtaata taaaataaat 19380
ttttaagaat ttatagagtt ttattttaag gattgaaaag aaggttaagg tgtgtttttt 19440
agtttatttt tatatgtttt tgtgatttgg agatttattt tgtagttaaa atgagttttg 19500
agatttgtat ttttatgttt tatttaatga ttaggtttat tagaagaatt gagtttaaat 19560
aattggggaa gataattttt taaaaagaga tttttaattt ttgtttgttg atttttaaat 19620
ttgttttatt aagataagtt ttttgtgaga aatttggttg ttagattttg gaattggttt 19680
taatggttaa ttttataaat tgagatggga gatttttttt gatgggaggt agtttttatt 19740
tttaaagttt atgttttagt tggaatgtat atgttaagga tttttgtttt ggttaatttg 19800
ggttttatat tgtgagtata taaaaagtat tatatggtta atggaggatg aggaattatg 19860
gtaaagtagg taggtaagtt ttaagaaata aaataatttg ttaaaaaata atttttgatg 19920
attattgtaa gattgaaagt gtaggaaaaa tatagtttga ataattttag attttttttat 19980
attttttttt tttttatata ttttgttatt ttataataaa atttttaatg gaaagtttaa 20040
aaataaatag tataggaata tgtgttttaa atgaattaaa ttgtgaaatt agttagtaaa 20100
ttaatttgta gtaagtaatt atttaaggaa attaaaatat tgtttagttt agttttgtat 20160
tttattatgt gtatgtgttt tttataatta attaatataa gtgttttagg aatatttgaa 20220
gataaatatg tttaatttaa ggaataaagt atttaaataa tttaagtgta attttgttga 20280
gttaaagtaa aatattttat aaatgaagtg gttatttaat tttttaggga aagtttggtt 20340
attgaaatgt tgtatgttta tgttatatta ataaaaattt ttaatttatt ttgtttatgt 20400
gttttgtttt tttgatatta ttggtatttg aattttagat ggatttttgt taaaatgata 20460
ttttgtgtga taaaagtatt tttagttttg attgatagat taaaataaat gtaaggaaat 20520
ttttttaaat tagattaatt ttttataaaa atattttaga atgtatgaat tttgatattt 20580
atatttataa tggtaaaagt tttttttgtt tagtttagta agataatatt tatataaaag 20640

agtaaaaaaa aattatatta ttttatgata gtttgatttt taaattgttt aagaaagtaa 20700
agtggttaaa ttggaaaaga ggaatatatt ttggaggttt agaattgaaa attttttttt 20760
taatttttag ttggaaaata attttttgta tttatttaaa gtgtattttt tgaagtgtta 20820
gattggagtt gattggtgat taatttaaag gagttataat ttaaagaaat ggtgagagtt 20880
tggtatttag gtttggtttt taggtaattt gtttgggttt gagaggttat taattgttag 20940
ttaagatgga attttttttt tttttttttt tttttaatgg ataataatgg gaagggggtt 21000
aatttttag tagttgaaat tttgtattta gtttttttatt ttgagaatgt taatttttgg 21060
tttgaggatt tgtttttgta gtgttggtat tgagatttaa gggaagatat tttgttttaa 21120
atgttagtta tggtttggtt ttttttttga ttttagtatt ttgtagattg ttagtgtttg 21180
tggtggggga tgaaaggaat agggttttgt aaggtttgtt tgttgattgt gttattttgg 21240
gtgaaattta gttttaaaag ttataaatta tttatggtga agattttttg aagtggaata 21300
aatttttaga tttgtattat tttatatttt tgtgggatag atggttttta tttattggtt 21360
attgggagag agttgttgtt tttgtgtttt attgtttttt ggggtgattt ttagtgagtt 21420
gagtttttgg ttgtatggta agtgtttgaa agttgggttt gagaggattg tagggttttt 21480
gagggtgtta agttttgaag gagtttatgg gtgtattggg gtttttgaaa tttagttgtt 21540
attggtagtt tttttttgtt tttttttagt ttttttgttt ggttttgtat tttttttttt 21600
tttttttttt tttatttttt tttttttttt ttgtttttat tttgtgtggg gagtgatgtg 21660
atgttagtag agattttatt aaattttatt gtatagtggt gtgtgggtgg ttggttgagt 21720
ttggttgtgt ggttggtgat ttaggagtga gtatagtgtt tgggtgagtg ttggggggag 21780
tgagtagggg tgatgagaaa tgaggtaggg gagggaagta gatgttagtg ggttgaagag 21840
ttgggagttg gagttgggag agtgaaagga gaggggattt ggtggggtat ttaggagtta 21900
attgaggagt aggagtatgg atttttattg tggaaaggag gattagaagg gaggatggga 21960
tggaagagaa gaaaaagtaa tttgtgttaa tttggtagtt ttaataaatt aaagggggag 22020
tgttagggta gtggggagat agaaatgtat ttttggggag taaattagga tgggttggga 22080
ggaagtgata gggaaagtgg tttaagagat ggaataaagg ataatgttta tggggttgtt 22140
tgggatgagg tgtgtggagt gtgggtgtga gtgtgtgtgt gtgatttttt tttaggtttg 22200
tagagttgag gaaagaggtt atagtaaaga gggattgtgg agggaggaaa gtgagagatt 22260
ggtagagggt gggagtggag gtgggtgtgg tggggatggg agaggatgag tgaagagaaa 22320
tttagaagaa tggagtgagt tagtgggaga gggtgggagg gttatagttg ggagtgaatg 22380
agttaggttt gttagttggg gaaggttggg atgttgggtt tagtttagtt gggatattgt 22440
gtttgaggtt aaggtgggtg gattaggtat gttgagagtg ttggtgtata ggtgggtatg 22500
gttatgtatt gatttagtgt ttatgaaggg tttgtattgg ataaggttta gatgtttata 22560
gagtttagaa ttttttttgt tgtatttata tttaataagt ttattttggg ttatggatat 22620
tttatttttt aaaatgatga ggttaaggtt tttggtgagg atggtattaa attgtatggg 22680
atagaagtgg gggtggggga gagagttttt tttaagttta tatttgtttt tgtaaagtaa 22740
agagtatgtg aaattatagg gtatattttt atttgaaaag tgtgttttat ttttgaattt 22800
tgattttttg atttttttgat ttgagtaaag atgtgtattt tggtagtgag tagaatattt 22860
tggttttgtt ttgtttttga gtggaaggat tataaatata atttgtttgg aggattaggt 22920
gtgaaggttt ttgttaggta tatgggataa tgttttttta attttaaggg tattttgtta 22980
atgtatgttt ttggaaagtg ttggaatata gttattgttt ttggatttgg attttttat 23040
taatattaat ttttgtttga gagtaaaatt taggtttgtt attaaaaaga tattttttttg 23100
gtttttaatt gagaataaag tttttttttaa aagttgtatt gtttttttta aattaatata 23160
ttaatatttg taattttaga aatatatagt gatttgggag aatgtgtata aaatagatat 23220
gtttaaaaaa gtttggtgtt taaaattaat tttagttatt atataggtgt tgggtttttt 23280
ttatttttgg gggttgtttg gaatatgtta tgtgtttttt tgaattattt tgtgttttga 23340
atttatttga gttagtagta aaaataggta aataaatttg tttaatttgt tttgagtgtt 23400
aaattttttt attttgaaat agttaatagt tgatagatgg atttatttta tggaaagggt 23460
tagttttttt agttatgaag aaaattgatt agagatttat attttaagtt atttttaatt 23520
tttatgtaat atttgtgaaa atttaaattt tttttttttta tttagtggaa atttaaagta 23580
gtgttattta aggggagaga aatgaggggg aaaatgttta tgtgttgttt aattgtattt 23640
tttttttgat tttgagaatt tttattttttg gtttttgaaa ttttgttgag gtaagaaaat 23700
taaatttttt taataagttt tataattgaa ttttagttat aggatattgg aaagtgtagt 23760
ttgagaaaga tatttttatt tttgtttatt gatgattttt gtagtttttt tattttttttg 23820
agtaatgggt taataatttt tttttttttt tttttatttt tgtagagatt aagaggtgtt 23880
tgtagtagaa tggttttgtt tttagttggt ggtgaggata ggtaatttta tggaaaagtt 23940
ggaagagaat gagaaaatta aagatagaaa gatttagaga tttgtggaga gatataggga 24000
gagggaaggg agttgtgttg aaaagatgta aagatatgtg tgtgtaattt tttttttttt 24060
taggttttag aggtttgtaa attagggttg agaggaaggg gtttgggaag tttatgtttt 24120
ttttgttttt tttttgtttg gagttttgtt tgttagaggt tggttaattt tagttttggt 24180
tgttgtagat attgtgttga gtttttgggt ttttgttttg tttagtgtta gtgtagttga 24240
agtgagtagt tggtgggaaa tgtaaatggt ttttggagaa atagaagata tagaatgatt 24300

tttatttttt ttttgagtgt gtggaaggag ttggatatat gttttatgtt tttaattttt 24360
tttttatatt tttagttata tttttattaa ataattaatt aatgtttaga attattaggg 24420
aatatattag gtatgtaatt gtagaagtag ggtgttgggg ggttataaat tattgagttg 24480
atttaagatg tggattttag gttttttttt tgttaaagta gtaaaggaag agtgggtttt 24540
ggtgattgta tttagatttt gattatttta aattagaagg gggtggaggg agtgtttaag 24600
taaagtaagt aattttttgt tttgtagatg taaataagat tgtagtatta aaggtattag 24660
tttttttagg gttagattgt ttggattggg agtttgggga aggggagata ttaattttat 24720
gtatttgtga attttaagga tgttatattt ttatataaat aattttagtg tggatttttt 24780
ggaatggggg gagtaatatt tttattttag aatattaaaa tatttttttt ttaaagtgta 24840
tatttttttt attttttaa aattttgaat tatgtttaaa gataatagtt ttttagtaaa 24900
ttggagtatt ggattatttt ttttattttt ttttattgat attttgatga tttgatttta 24960
atgtgtgggg ggtataggga attaaatata gtttataaaa ttaagtttag atgaaatagt 25020
gttggttaag tgggtttaga taatttttaa tgagaatttt aattatattt ttttttttaa 25080
tatgttgaga taagtgatag aattgttaga atggtaatta aattggaaag tttagggaga 25140
ataataattt tgtgattaaa ttggggtaaa attgtggata aatgtggggt gatttttgtt 25200
aatttttttgt tatttaagag ttaggatttg ggaaaggtat agtattattt tagagtttgt 25260
tgtgatgggt tgtgtgttat tatttatttt ttttattttg gattatgatt ttaattttgg 25320
taagtaattt ttttagtttt ttatttgata ataagtgagt atgtaaatat taatggttag 25380
tgatgtttaa ttgttttaaa tattattgat ttgttggttg ttttaaattg tttttttagt 25440
ttaggttttg tttttgaatt gtttatttta gaggtttgat ttatgttttt gatgttataa 25500
tataataatt gttttttttaa aaaaggtatt taagatgaat taattgattt gtatataaat 25560
taaaattatt atgtgttgtt gattttggtg ttttataatt attttgaaat tagtatttaa 25620
ttatttgagt taaaagaata tataaatgtt tgtattgatt tattaatgaa ttatttaatt 25680
aaaatgtttg ggtaatgttg ggtgttggaa agattgttaa attaagatat attataggag 25740
ggatatgaag attagaaagg taatagatta atattttgta tttaaaatgg agtttttggt 25800
gatttttagt tttaattttg gagtaggggt tttttttttt tgttgttaaa aagattttgt 25860
gtttgtttgt gagtgagtgt atttaagtgg aaggaatgtt tttatggtta tggtggttta 25920
ggttttttgt ttggattggg attttatagt tttaatttag gagtgttaaa ttttggaaga 25980
ttttgggtta gttttggagg tgtgtggttt tgtaagttgt taggttaagt ttgttttttt 26040
tgtttgtttt tttggtaggt tgggtgtgtt atggtagtga gtttttttgtg taaatggaga 26100
gttggaatta aagttgatat ttaatagata tgttaattga gtatttattt ttgttttgag 26160
aataggaata aaaggtagtt tttttaaga gaggtggtgt aaaggtatgt tataggagtt 26220
tagaaaaggt tggtggtggg aaatttgtag tttgggggtt agttaatatt tttttttatt 26280
ttaagtattt attgatttgt tgttgttatt tttggtgatg tagaaggata tttgaaagaa 26340
tttttgatgg ggttttgatt tgagaaagga ggtgatttgt ttaggttttt attaaatttt 26400
taattattat attaattgtt tttttttatt ttttatttga tttttttttt tttgtttatt 26460
tttaattttt taattattta gaaatttttt tattttttag tggttttttt tttgtagtag 26520
tttttttattt gaattttttt tttgtttttt tgtggtaggg tttgtatatt gatttttttg 26580
atttttggta tatttgggtt ttttgaaatt ttttaatttt tttagatttg aggatggtag 26640
gttttatttt ttttattgtg tgtatatatt tagagatatg aaaatttata tagattgttt 26700
ttaaatttag ggtatttaat agatgttttt tttttagttt gtttttttgat ttgaaatgtt 26760
tgtttgattt taatttggat attatttttt tttgtttttt tttttttaaa gtagtttgga 26820
tatgtgtgta agtgagttta gaatagtttt atttatattt tttattaaat tgtaaataaa 26880
agaagaatta atgaagtaga ttggtatata gattgtatta agagtttgaa tttttagttt 26940
ttggattttt tatttaattt tggttgttat ttatattgat agagttattt taagtagagg 27000
tttagagaaa tttgtattgt gggataatag gtaaagttat agtaaaaagt ggaataattt 27060
taaagttatt ttattagaat gtaaattgta tttttgggtt ttgtttgtaa ttatttagtt 27120
ttaatatata tagagttaga taggaaaaaa taggttaata tagttattgg tattagagaa 27180
gataaatttt atgggttttt tagtgaaaag aagattttta aagtttataa tttttgatta 27240
tttaatttta tttataattg tgggaatgaa taagatatta attgttttat gtattttatt 27300
tatattaatt aatttgtgtt tttattaaaa gtagttatat agaatttttt ttaatttttg 27360
gtagtaagtt tagaaaatga agtttatagt tattttgaat tggatatatt ttttgagttg 27420
attattttttg taagtgtagg aatataatat tgtttttta tggtttttttt gtattttttt 27480
agggtttgta agtttttatt aggtttgata ttattgtttg ggtttatatt tattataagt 27540
aaatttgatt attatgttga ttttaaaata gtttatttgg ttagtataat tttagttttt 27600
aaattataaa aatttttaaa tatatgaagt ttttagtttt tattttttttt agtttttttgt 27660
ttatttaaaa tttttatttt aattggtgta agtaataata atttgtatta ttatttgtat 27720
tttttttatt tttttggaga ttgggttgga ttttagagag aatattagta ttattattat 27780
tataaataat aaaatttaaa agtaaagttt ttatttgtat gataattggt atttggaatg 27840
tttttgattt atttaatgtt attttataaa ggtattttgt aaattttttt ggaattttta 27900
gtaagagttt gtagtaattg gaataatttt ttgggaagat attttttttg atgggttttt 27960

```
agtttttgga ggaatagatt gagagtaatt agggagggag gggatattgg aaattggtag   28020
ttatgttagt tgaaataagt ttgggtttag taaggtgatt gatgttgtgg ttgatttttt   28080
attttgagtt tttttttaat tggggtattg attttttttta ttttgggatt ttaaggtatt   28140
tggtgtgtat gtagattttt tttttgtggt ttttattatg tggttttgta gtaggttttt   28200
ggtttaatga tattttatag ttatagtttt tatatttatt attatgattt taatgtttag   28260
gtttttagtg tatttatatt aaatttgttt tattagtaag ttggagtata taggagagat   28320
gggggtaagt aaggatttag tagagtttaa atttagatat gtttaaatgg ttttgattgt   28380
gtaaagtgtg gtaatgtttt ttgttgtttt agttttttat tttaagtttt atatgttttt   28440
tggttaatga agtgtgatat aggttatatg ttaggaataa tagtatttgt tgagaataaa   28500
gtgaatttag gaaatttggt atatataaaa tgtatt                              28536
```

<210> 965
<211> 28536
<212> DNA
<213> Artificial Sequence

<220>
<223> chemically treated genomic DNA (Homo sapiens)

<400> 965

```
gatgtatttt gtgtatatta ggttttttga gtttattttg tttttaataa atattattat      60
ttttagtatg tggtttatat tatattttat tagttagagg atatgtgaag tttagagtgg     120
aaagttaggg tagtaggaag tattgttata ttttatatag ttaaagttat ttaaatatgt     180
ttgaatttga gttttgttga gtttttattt gtttttattt tttttgtgtg ttttagttta     240
ttaataaagt aggtttaata taaatatatt aaaagtttaa atattgagat tatgataatg     300
aatatgaggg ttatgattat aaaatattat tgaattagag atttgttatg aaattatatg     360
gtgaagatta taagggagga atttgtatat atattgagtg ttttgggatt ttaaagtggg     420
aagagttagt gttttaatta aagagaaatt tggggtaggg aattaattat aatattagtt     480
attttattga atttaggttt gttttagttg atgtagttgt taatttttaa tgtttttttt     540
ttttttaatt gtttttaatt tatttttttta gaaattgaaa gtttattaaa gaggatattt     600
ttttagagag ttgttttagt tattataagt ttttgttaga aattttaaga aagtttataa     660
ggtattttta taaggtggta ttgagtaagt tagaagtatt ttaagtatta attattatgt     720
aaataagggt tttattttta gattttgttg tttgtggtgg tggtgatgtt ggtgtttttt     780
ttggaattta gtttaatttt taaaaaagta aaaggagtat aaatagtaat ataaattatt     840
attatttata ttaattaaaa tagaagtttt gaatgagtaa ggagttagag gaggtaaaaa     900
ttgggaattt tgtatattaa aaggttttta taatttgaaa attaagatta tgttggttaa     960
ataggttgtt ttaaaattag tatagtaatt aaatttgttt gtaatgaatg tagatttaaa    1020
tagtgatgtt agatttgata aggatttgta aattttaaaa gagtgtaaaa agattataga    1080
agaataatat tatattttttg tatttataga aatggttagt ttaagagatg tatttagttt    1140
agggtggttg taagttttat tttttgaatt tgttattaga agttaaaaga aattttgtat    1200
aattgttttt gatggaaata taaattggtt gatgtaaatg aaatatataa agtagttggt    1260
gtttattta tttttataat tataaatgaa attaaatgat taaaaattat agatttgggg    1320
gatttttttt ttattgagga gtttatggaa tttgtttttt ttagtattaa taattgtgtt    1380
gatttatttt tttttgttta attttgtata tattaaaatt aggtggttat gaataaaatt    1440
tagaaatata atttatattt taataaaatg attttaaaat tattttattt tttattgtgg    1500
ttttatttgt tgttttataa tgtaggtttt tttgggtttt tgtttagaat gattttgtta    1560
atgtagatga tagttagagt tgaatgggga atttagaaat tggggatttg ggtttttgat    1620
gtaatttata tgttaattta ttttattagt ttttttttta tttatagttt ggtaaagaat    1680
atgggtggag ttgttttggg tttatttgta tatatgttta aattgttttg aaaaaggaag    1740
ggtaagaaag agtggtattt aagttggaat taggtaggta ttttagatta agagatgaat    1800
tggaaaggga atatttgtta gatattttgg gtttgaaggt agtttgtgta agtttttata    1860
tttttgagtg tgtgtatata gtggagaggg tggagtttgt tatttttaaa tttgaaaaga    1920
ttgagagatt ttagagggtt tagatgtgtt aaaggttaga gggattaata tataggtttt    1980
attatggaaa ggtggggaaa aggtttgaat agaaaattgt tgtagaaggg aagttattga    2040
gaggtaaggg agtttttgaa taattaaaaa gttaagaata agtaaaagga aggaggttgg    2100
gtggggggata aaaaaaagta gttgatgtgg taattaagaa tttggtggga gtttgggtag    2160
gttatttttt tttttagatt agagttttat tagaaatttt tttaagtgtt tttttgtgtt    2220

gttaaagatg ataatagtaa attaataagt gtttgaaatg aaaggggatg ttgattagtt    2280
tttaggttat agattttttg ttgttagttt tttttgaatt tttatagtgt gtttttgtat    2340
```

```
tgttttttt aagaagagtt attttttatt tttattttta ggatgaaggt aagtgtttag 2400
ttagtatatt tattaaatgt tagttttggt tttagttttt tgtttgtgtg gaaagtttat 2460
tgttatagtg tgtttagttt gttggagggg tagatagaaa aagtaagttt ggtttggtga 2520
tttgtggggt tatgtatttt tagggttggt ttggagtttt ttagagttta atgtttttgg 2580
gttagaattg taaggttttg gtttgagtaa agggtttgag ttattgtagt tgtgggagtg 2640
ttttttttat ttgaatgtat ttatttataa ataagtataa aattttttta atagtagagg 2700
agaaagattt ttgttttaaa attaaagttg ggaattattg gaaattttgt tttgagtgtg 2760
agatattggt ttgttatttt tttaattttt atattttttt tgtaatatgt tttgatttaa 2820
taattttttt agtgtttagt attgtttgga tgttttaatt gggtaattta ttagtgagtt 2880
aatataggta tttatatatt tttttagttt aagtggttaa gtattaattt tgaaatgatt 2940
ataaaatatt ggaattggta atgtatagta attttaattt atatgtaaat taattggttt 3000
attttaaatg tttttttttaa aaaaataatt attgtattgt agtattggag gtatggatta 3060
aatttttaga atagataatt tggaaataag atttggatta ggaagataat ttagaatagt 3120
taataaatta ataatgtttg aggtagttaa atattgttag ttattggtat ttatatattt 3180
gtttgttgtt agataaggag ttggggaaat tgtttgttag ggttgagatt ataatttaga 3240
gtgaagaaag taaatggtag tatatagttt gttatagtgg gttttgaaat aatattgtat 3300
tttttttaaa ttttgatttt tgggtgatag ggagttggtg gaggttattt tatatttgtt 3360
tatggttttg ttttaatttg attatgaaat tgttgttttt tttgagtttt ttaatttgat 3420
tattatttta atggttttgt tatttgtttt aatatattgg ggggaggagt gtaattgaga 3480
tttttattaa aaattatttg aatttattta gttagtattg ttttatttaa gtttagtttt 3540
atgggttgta tttaattttt tgtgtttttt atatattaaa attagattat taaaatgttg 3600
gtaggaaagg gtgaaggaaa tggtttaatg ttttagttta ttggaagatt attattttta 3660
gatatagttt aaaattttga ggaaataaaa aggatatatg ttttgggggg aaaatgtttt 3720
aatattttag aatgggggta ttattttttt tattttagag aatttgtatt ggagttgttt 3780
atgtaaaaat gtaatatttt tgaaatttat agatatgtaa ggttagtgtt ttttttttt 3840
taggttttta gtttaggtga tttagtttta aaggagttag tatttttgat gttataattt 3900
tgtttatatt tgtagggtag agaattgttt gttttgtttg gatgtttttt ttattttttt 3960
ttaatttgaa gtaattggaa tttaaatata gttgttaagg tttgtttttt ttttattgtt 4020
ttgataaggg aaaaatttga aatttatgtt ttaaattagt ttggtggttt gtagtttttt 4080
agtattttgt ttttatgatt gtatgtttaa tgtatttttt ggtgattttg ggtattaatt 4140
agttgtttaa taggagtatg attaaaaatg taaaagaagg attaggagtg tgaaatgtat 4200
gtttagtttt ttttatatat ttgaggaggg aatgagaatt attttgtatt ttttattttt 4260
ttaggagtta tttgtatttt ttattagttg tttattttag ttgtattggt gttgggtaag 4320
gtgaggattt aaaagtttag tgtagtgttt gtggtggttg ggattggggt taattagttt 4380
ttggtgggtg agattttaga tagaaggggg gtgagaggaa tgtgagtttt ttgagttttt 4440
tttttttagt tttggtttgt aaatttttga aatttgaaag gggagggagt tgtatgtgtg 4500
tatttttgtg tttttttagt gtaatttttt tttttttttt tgtgtttttt tgtggatttt 4560
tgaatttttt tgtttttggt tttttattt ttttttaatt tttttatgag attgtttatt 4620
tttgttatta gttgaaggta aggttgtttt gttatgagtg ttttttaatt tttataaaat 4680
gaaaagaaaa aaagggagga ttattagttt attatttaga ggaatgggga ggttgtaaaa 4740
attgttgatg ggtagaggtg aagatgtttt ttttggattg tattttttgg tgttttgtaa 4800
ttagagttta gttgtgggat ttgttgaaga aatttgattt ttttgttttg gtgagatttt 4860
aaaaattaga aatagaaatt tttagagtta gagaggaaat ataattaaat agtatgtggg 4920
tatttttttt tttatttttt tttttttaaa taatattgtt ttgagttttt attgggtaaa 4980
gagagaaagt ttgagttttt atggatgtta tgtggaggtt agaaatggtt taaaatgtag 5040
atttttaatt agtttttttt gtggttgaag aggttaattt tttttataaa atgagtttat 5100
ttgttgattg ttagttattt taaagtgaag ggatttagta tttaaaataa attgagtaag 5160
tttgtttgtt tgtttttatt gttaatttaa atgaatttaa aatatggagt aatttaagaa 5220
aatatataat atgttttaga tagtttttaa aagtagggaa agtttagtat ttatatagtg 5280
attagggtta gttttaagtg ttaagttttt ttaaatgtat ttattttatg tatatttttt 5340
tgagttatta tatattttta aaattgtgag tattggtata ttgatttagg aagagtaata 5400
taatttttag agggaatttt atttttaatt agggattaaa gagatgtttt tttaatagtg 5460
ggtttgagtt ttgtttttaa gtaggaatta atattggtgg gaaaatttga atttaggagt 5520
aatggttgtg ttttggtatt ttttaaaaat atatattaat aggatgtttt tgagattgaa 5580
aaaatattgt tttatatgtt tggtagaagt ttttatattt ggttttttag gtgaattata 5640
tttatagttt ttttatttag aggtaggata gagttaaaat attttgttta ttattaaaat 5700
atatattttt gtttaagtta agaaattaga aaattagggt ttagaagtaa ggtatatttt 5760
ttgagtgaga atatgttttg taattttata tatttttgt tttgtaggag taaatgtgga 5820
tttgagggaa attttttttt ttatttttat ttttattttg tgtaatttaa tattattttt 5880
gttaggaatt ttaattttgt tattttaaaa aatgagatat ttgtgattta gggtgaattt 5940
gttgaatgta ggtatagtag aggaaatttt agattttatg agtgtttgag ttttgtttag 6000
```

```
tgtaaatttt ttgtgaatat tgggttagtg tgtggttgtg tttatttgtg tgttgatatt    6060
tttagtatgt ttggtttatt tgttttgatt ttgggtgtgg tgttttagtt aagttgggtt    6120
tagtgttttg gttttttta gttgataagt ttagtttgtt tgtttttggt tgtggttttt    6180
ttattttttt ttattagttt attttatttt tttagatttt tttttattta ttttttttta    6240
ttttattgt gtttattttt attttgtttt tttattggtt ttttattttt tttttttgt    6300
agttttttt tgttgtgatt ttttttttta attttgtagg tttgaaagaa ggttatatat    6360
gtatgtttat atttatattt tatatgtttt gttttaaata attttatgaa tattgttttt    6420
tgttttgttt tttgggttat tttttttgtt gttttttttt agtttgtttt gatttgtttt    6480
ttaaaagtat gtttttgttt ttttgttgtt ttggtgtttt tttttgatt tattagggtt    6540
gttgggttgg tgtagattgt ttttttttt ttttattttt atttttttt ttggtttttt    6600
ttttatagt gggagtttgt gtttttgttt tttggttggt ttttaagtgt tttgttaggt    6660
ttttttttt tttgtttttt tggttttggt ttttgatttt ttggtttgtt ggtatttgtt    6720
ttttttttt gttttgtttt ttgttgtttt tgtttgtttt ttttggtgtt tgtttgggtg    6780
ttgtgtttgt ttttggattg ttagttgtgt agttgggttt ggttggttgt ttgtgtgtta    6840
ttgtgtagtg gagtttggtg gaatttttgt tgatgttatg ttattttta tatggagtag    6900
gagtagaggg aagagagagg gatgagaggg agggagagga gagagagtgt gagattgagt    6960
gagaaagttg gagaggagta gaaagaaatt gttagtggtg gttagatttt ggaggtttta    7020
gtgtatttgt ggatttttt ggaatttggt atttttagga gttttgtagt ttttttaggt    7080
ttggttttg ggtgtttgtt gtgtagttgg aggtttggtt tgttggaaat tgttttggga    7140
agtagtggga tgtggagata gtagtttttt tttggtagtt ggtaagtgga ggttatttat    7200
tttgtaggga tgtgagataa tgtgagtttg gaaatttgtt ttattttgga gaatttttat    7260
tgtaggtgat ttgtggtttt tggggttaag ttttgtttaa ggtaatgtag ttggtaaata    7320
gattttgtaa agttttgttt tttttgtttt ttgttataga tattaataat ttatagggtg    7380
ttgaagttga gagggaagtt agattgtggt tggtatttaa aatgaggtat ttttttttaa    7440
attttggtgt taatattgta ggaataaatt tttgggttaa ggattagtat ttttaagata    7500
aagggttggg tataaagttt tagttattgg aagattagtt ttttttttat tgttatttat    7560
tgggaaaaaa aagaaaagaa aaagatttta ttttaattgg tagttagtga tttttaggt    7620
ttaagtgaat tatttgggag ttaggtttgg atgttaagtt tttattattt ttttggattg    7680
taattttttt aaattgatta ttagttaatt ttaatttggt attttaggag atatatttta    7740
aatggatgta gagaattatt ttttagttgg agattaagaa aaaaattttt gattttaaat    7800
ttttgaaata tgtttttttt ttttagttta attattttat tttttaagt aatttagaaa    7860
ttaaattatt ataaggtggt gtgatttttt tttatttttt tgtgtgagta ttgttttatt    7920
aaattaaatg gaaaaaattt ttattattat aaatgtaaat attagaattt atatatttta    7980
aaatattttt atgaaaaatt aatttgattt aaagaaattt ttttgtattt gttttagttt    8040
attaattaaa attaaagatg tttttattat ataaaatatt attttggtag aaatttattt    8100
aaaatttaaa tattaataat attaagaaaa taaagtatat aagtaaaata aattgaagat    8160
ttttgttgat gtaatatgag tatataatat tttaataatt aaattttttt taaaaaatta    8220
aatagttatt ttatttgtgg aatgttttat ttttaatttag taaaattata tttaaattat    8280
ttaggtgttt tgttttttaa gttaagtgtg tttgttttta aatgttttta aagtatttat    8340
attaattggt tgtaaagaat gtatatatat ggtaaaatat agaattgaat tgagtagtat    8400
tttaattttt ttaaataatt atttattata aattaattta ttggttaatt ttataattta    8460
gtttatttaa aatatatgtt tttgtgttgt ttattttaa atttttatt aaagatttg    8520
ttatggggta ataaagtgta tgaaaagggg ggaaatgtga aaggatttgg gattatttga    8580
attgtatttt ttttgtattt ttagttttgt ggtagttatt agaaattatt ttttagtaaa    8640
ttgttttatt ttttagggtt tgtttgtttg ttttgttatg gttttttgtt ttttgttagt    8700
tgtgtagtgt tttttgtgtg tttataatat aaaatttaag ttggttaaaa taagagtttt    8760
tggtatatat attttaatta gaatatgaat tttgggggtg agaattattt tttattagga    8820
aaagtttttt attttaattt gtgagattag ttattgaagt tagttttgaa gtttggtagt    8880
taaatttttt atagaagatt tgttttgata gggtaagttt aaggattagt aggtgggaat    8940
tggaggtttt tttttaaaaa attatttttt ttagttattt agatttagtt tttttagtag    9000
gtttggttat taaatgaagt ataaaaatgt aagttttaag gtttattttg attgtaaaat    9060
aaatttttaa gttataagga tatgtaggag tgagttaagg aatatgtttt gatttttttt    9120
ttagttttta gagtggagtt ttatgagttt ttgaagattt gttttgtatt gttttgtttg    9180
gtttttagta ttgaagtatg gggaagtggg gggaagaatg tgtaataatt gattgatttt    9240
atattaagta atgtaatttt ttttttttgt atattttatt ttttaaaaaa aataaataaa    9300
taaaaattat ttgtagttat tatttgtagt gttttggtta ttagttaata atgtagttag    9360
tttagatata taaaaaaaaa agattattga aatgatgatg atatgtaaat tttttttgaa    9420
attattataa gtaaatattt gaagtttgga ttaataaaat tttatttgtg ttattttata    9480
ttgagttagt agaaagttgt gataatgaat tttgtaatat tttatgaata gatattttaa    9540
ttagggatta attttgtgat tttattgtag aattattaaa tttggagttg ttaaattgtt    9600
atttttgggt ttatgggttt ataaggattg aattggtaga gtttttgttt gtgtttttgt    9660
```

```
tagtgggtgg gggaattgtt tggttgtttt tattttggat ttttatgtta tagtgttggg    9720
tagttttttt tgtaggtagt gattttggtt agaggttttt tagggtttag ttttttttag    9780
gagaggttga gatgtaggga aatggtattt aggttagagg taggtttgta gtttttttgtt   9840
ttgtttttgt gtttttgtta atttgataat gtttgttttt attttgattt ttgtatttgt    9900
gtgaagtggg ttttttggtt gttggtgtat tttggttagt gtggagagag gtaggtgttg    9960
agattgaagg ggtttaggga gttttggatt tttttttttt gtttttaaag taattgtggt   10020
tttttttattt atttggtgga gtttttttgag atttattttt tttggtttgt ttgtggtaga 10080
gaagggggag tgtgttaaat gtttggtttg ttgtgttgtg gttgaaaatg tgaaaaagat   10140
ttggtttgtt tgggagagaa agggggagaa ttgggtagta gttatattag agttattttt   10200
ttgtttttgg tgggtagtaa atttttaaag aatgtttgtt ttgttttttt tagttttgtt   10260
tagtttattt agtgtttttt ttttgtgatt ttaaattata ttttagggta attatttgta   10320
gtaagtaaat aaatggttgg gttagtattt ttaggagaaa gtgtggttaa atatggaaaa   10380
gtggtttttg atggatgaga ggtttgaatt tagtttgttt ttgaaatatt ttaggttaag   10440
agtttgtttg ttttagaatt atagaaaatt gagggaaatt gttgtttagg ataggggtat   10500
gttggtgttg atgttttata aatgtttatt gagttttaat taatggataa gtattgaagg   10560
gtggtttttg tatatagttt tttaaagaga aaagtttttt ttatttattt atttttgttg   10620
ttattgtgtt tagatgagtt tttaattttg gtattgagat ttttgaaagt aggtttatag   10680
tttttttagt atattgtggt tttatagttt tttaattttt gggtattttt gtggtaattt   10740
tggagggaga tttttttttg ataaataaat gttttgggtt tgaggttagg ttggagatgt   10800
tgttgtatag ttagaggttg ttaggttgga aaaatatgtt tgaagtttag tatatagtag   10860
gtgtttaata gttagtgtaa tgtagtttta tttgagtttt gtttatttga tggttgttgt   10920
tttttatagt tttttttttt ttttgttttg tagataatgg ggaatggaga ttaattgttg   10980
taaattggtg ttggtgtgtg tgtaattagg taagaatttt tttttttttgt ttgggttatt  11040
ggatgggagg ttgtgttatg tgagggtggt aagagggtat tggttttgtg gtgaggtttt   11100
agtgaggggt gtttttttga ggggttagtt tgggtaggaa ggaaattaga attaaattgt   11160
tagtggtttt tttttgtggt ggggtggtgg attaggaagt agtggtgtgt tgtgtattga   11220
agtttttttag tttatttttt ttggttggaa ttgttggtaa ttggggaggt gtagaaagag  11280
tatgttattt tgttttgggt tgttagaggg tttgggggat ggggatgttg ttagtttttt   11340
tttttaattg ggtttttgtt ttttgttttt tttttttttt tggtttgttt tgtttttttt   11400
tttttttttg tttttggttt tttttggttg tggtttggga tgtttttttt tgtatgtggg   11460
gtgggtgtgt gtgtggttta ggtgtgtagt tggtggttgt tgaatgtttt ttttttaaag   11520
attttgaaat taaaaaggtt gagtttatgg atttttttga gagttgaaaa gaggtagtta   11580
gtagtaagtt ttttttgtgg tagtattttg gtgttaatgg taaggttggg agggaagtgt   11640
aggttgtgtg ttgggtattt gtttttggga ttttgggttt tgggtgaagt gtaagaaggt   11700
gaggttgtta gatttgatgt gtttgttgtt tgaatttaga tattttgttt ttgggtggga   11760
tgggaagtag ttgttttagg gatgttaatt tttttttttta aattatattg tatttttgag  11820
atttaatatt tttttttttt ttttgtttgt tttttgtggt ttgatttttt gtgtatgttt   11880
tagtaaattt tggtgtttag gttggtgtgg aaaagtggtt taatagtgat ttttgttgtt   11940
tgttatattt tgttgtgtgt agtattatta gggtttattt agttatttag gtttttagtt   12000
atgttttatt tagatttgtg ggtgtgtggt ttattgtggg aggtaagtaa gggaaatttg   12060
agttggtgaa ggtttgtttt ggttggttgg gggaggggtg gggggttgat aatatttttg   12120
aagagttgga gggtagttat tgtgtttagt agtttagggt agaatggagg tttgtttttg   12180
ttgatgtgaa tttgtttgaa gtattggttg ttaggttttg ggttttggtg atgttgttgt   12240
ttgattggtt ggttttattt tggaggaatt gagggatatt gttagaggag gtttataggt   12300
ttatgtaaaa agttaaaaag tttttaattt atgttatagg tttttttttga attgaaattt  12360
gttttatggg gtggaggggg gggtgtaagg gatggaggag ggaagatgtt ttttttttta   12420
aatatatgga aaaaaatttt ttaaatttat tgtttttttta ttttttttggt tttgtagtaa 12480
ataagtgttt agttttagga ggttattgat ttttgataat gtgagtagat aaagtttttt   12540
ttttttttata gtttttggtt tttaattttt tttttttgga ttaaagtgta agaatataaa  12600
tgtaatatgg gatggagggg ggtgatttgg gattttggtt aaaaaaataa attgtattat   12660
taagaagaaa ataaaggttt tgtattggag ttttttttgtg aatttgagag aaaatgatta  12720
tttgttgaaa tgaagtgttt aaagtgattt agtgttttat gtttggatat tgtattatat   12780
tgttagttgt tttgttgggt tagttaaatg tttatttgtt tgggattaat tttatggggt   12840
taaatggggg taatgtagag ataatgttgt gtgatttttg ttatttagat tgtgttaaat   12900
tttttttttg tttgataatt ggtagtaaaa ataaattatt agattgtagt atgtttggga   12960
tatggttaaa aattaagagt agtgatgatt tttgggggaga atgttttgtg tgggtttagt  13020
tttggttttg gttagattag aggagttttt taattttgtt ttgtgtgggg tgggtttgta   13080
gttgttaagt tgaggttgat atttttttatt gtgttgggag ttagagagat gtaaaatgtt  13140
tttttttttt agtttttatt ttaggttttt tagatatggg gaatgtattt tgaggatagg   13200
tggagaagtt tatggtagga tggggttttt gtaggtgagt aggaaatggt taagagtaga   13260
ggagttttgt ttgtgttagt tataagttgt gtaggtgttt ttggttgttt gtttttgata   13320
```

attagtatat aaagaattag aaataatgaa tgattgtttt tttaattatt atttttaggt 13380
ttgtattgtt ttagtgtatg tgaaaggttt tttttttata tttaatatgt ttttttttat 13440
tttttgattg aaaagaaaaa ttgttgttta aatatgttta atgttattaa ttaagaaaag 13500
gtatgtaatg ggaagaaatg ttgaaaattt tgatttaatt ggtttttaag gaattagtag 13560
atgataaaaa aaaattatat gagtgggtaa agttatagta ttgttgaagg atagagtatt 13620
tatttttttt tgattttaag ttaatttatg gaatatttaa agttttggtt atagtttgtt 13680
tgtaaaataa aaggatttat tttttgtgtt tttttaaagt ttttttttgt ttttaaagag 13740
aaaaaaagtt tataatgata tatgattttt ttaaaaggtt gtgatagttt attatgttat 13800
tttttttgtt tttgttttta atgttgttta aaaatattat gtttttgtta aagattaaat 13860
gttttgtata ggtagagttt atttttaagt agtttaggtt ttgttttttt tttttagtga 13920
gttttatttt ttttggtatt tattgggtgg atgtttagtt tggatagaat tttgaaatgg 13980
gggtagtatg agagtgattg gagattttta aaagttagag gtttgagaga gggtggatgt 14040
agttagtaga agatggtgta gaagttagtt gagaatgatt ttttagagta aagagatttt 14100
tttttggttt tttttgtttt gggggttttg aaaggaattt ataaaatggt ttttattttt 14160
aggaggagga tggattgatt tttttttgtt attggtttaa aaagttttag ggtggtggtt 14220
ttgggttttg tgttgaaatt ggattgtatt gtagtttttt tggatttgat gtttggtttt 14280
gtgttttgat aaggggtggg tatttttttt ggtttttttt aggaatgtat taattgttaa 14340
atagttttgg tttagtggat gggttgaaag tgtttgattt aagttgttgg tgtgtataga 14400
tttttttttt ttgggaggtg ggttttatgg tttgttgtgg tatttttagt tgtgatatat 14460
atttttatat gtggtagtag tttggtttta atttttttttg aaggatttgg gttaattttg 14520
gtggttttgg tggttgtaga tttttttttg ttgttttgtt tttgtgtttt ttatttaatt 14580
agtgaatgtt tgtggagtat atattatgtg gatttttaat gtatttttttg aaagtaaata 14640
atatagtttt ttttgttgtt atgaagggat tttaatttta atatggatat tagtgagatt 14700
agtttagatt gtttttagta aaatgtaaaa tggtggtgtg tggggtggtg attaaggttt 14760
tgagttttgt tagaaagaag gggatgtgta gagaaaggtg gagaatttta gttgtggtta 14820
gtgtggaagg gataggtgtt tgttgaaggg ggtatgaggt ttgaggaaaa agtaatgaaa 14880
taggggtaag gagagttttt tatttttttt tttttgtttg atttttgtta ttttattttt 14940
tttttttttt ttttattttt tgtgttaatt aaatttgtag tttatttgaa aggtgtttttg 15000
ttgtgttgtg gtttttttatt tttaggggaa attgtattag ttgtttgaaa gtagttagtt 15060
tttgtggatt tttgtttgta aaagtggttt ttataggttg tgttttttgt tgttgatttg 15120
gtatataaag ttttttaagg ttggtttggt tgttattttt tattgtttgt tgttaatata 15180
tgtagtagtt gttagagtgg tttgggggaa aaggaaatgt ataatgaaag tttatttgtg 15240
agtaggaata tattaatgga ataatttgat gttttttttaa ttttatgtaa aaagttttgt 15300
tgtttttttta atattgattg aatgggtaat taatggtttt ttatttaggt gaatattttg 15360
taatttaaga taggtaaaag ataataagtt taaggtagaa gataaaaggt ttaattgtag 15420
tggtgtttgt ttgtttttta tttttttaggg tttttgatta ggaaagtttt tttttagagg 15480
agaaaaaggt aggagtggga gaatatatat ttattatttt ggggttagat tttattgtag 15540
tatttgatta tttagtttag tttttttgtta ttttgttttt ttatttttag tttttttttt 15600
tgtatttttt tttttttttta atttttttag gatgattttt tattattatt gttattatgg 15660
ttttaataat tttttttttt aaattttata tttttttattt tagtaattaa tgaggttgtt 15720
ttttgattta ggaggagatt tttttttttt agaatttaat gtgtagagtt tttgagaatt 15780
aaagtagttg gtaggggagg aagaaattaa tagaaaggga gagagtatat agaattgtgt 15840
gtgtatgtta aagagtgatt aggaatgata gagttaattt ttttgtgagg atttgatggg 15900
aagagtgttt aagattttat tagtatgttt ttaataggtt gatattttaa tttaaatttt 15960
tagaagtaat atattatttg ggttattata atgaggtggg tttttttttt tttgttagtt 16020
gatagttttt aaaatattat tttgttaggg aaataaaagt tttattttag attataggtg 16080
ggtattttttg gatttaggtg atttatggtt attatgataa ttaatgttga atgttagtta 16140
ttagtatgtt tgggagagag aaaatagaaa gaagggagag taaaagaaat agaaaaggga 16200
gatggatata agttggagag ggaagaaaag agaaaaagag gaagatagat gagtgtttaa 16260
tttaattgtt gtttaaaaaa gtggtggggg ggtgggattt tatttagttt tttgttattt 16320
tttttttttt tgatttggat atttatgttt aattttatat tttattttttt tttttttttt 16380
tttaaatata tgtgttatat tatttttttt attttattta gtttggtaag tagttgtttt 16440
ttggagattt agtgatattt aggaaaattg tggtagtaat atgtaaatgt gaggaagtat 16500
taatagtatg tttgttgagt gattttagta aatgtttttt tttttaattt tttttttttt 16560
tttttttttag gttattgtga ggtggtaatt tttattgtta tttgaatatt gtttttttag 16620
gtagttattt taaattttaa atggttgagt agttagagtt gtgggttgga aaaatgggta 16680
ttatttgtag ggatttagag agggtggttg ttgtttaata tatttataga tttttaattt 16740
agaaaataat tttttttttt tgataagtta gagtttttta aattttattt aggaaatggg 16800
gaaaaggata gttatagtga agtttttaat ttttgggtta tttggtttta tagttatgag 16860
gggtggtggg tagtggattg tttttagttt ggtttgtatg tagagaaaag ttagatattg 16920
gagggggtgg ggtatttttt gggtaggatg taaggttttt atttgatttt tgtgttttat 16980

```
taggagttta tatatttatg tttattatgt ggttttaagt tgagtttagg tgggttttgt   17040
ttttgagtta gtttgggtag ggtaggattt ttatttgttt aaggtttaat agtttaggga   17100
gatgtttaat taagttattt tttgggtgaa ttttgaagat agattttttt taaaagttag   17160
agattatttg gttgagtttt aggttagatt gatatggaga gtttggtggt atagtttaat   17220
tgtttattgt tatggttaga gggattttgt ataattaata ttgaagagtg tgaaattaaa   17280
taagatttta agattggtaa ttggtggtaa atattagtat aaaatatggt tgattttatg   17340
ttatatattt ttttttttta gggttttttt ttgaaagaat aagtaagaaa ttttaattga   17400
gataattttt gatgtttttt agatttaaaa ttttatgttg gtattgggtt tttttttttt   17460
gttttatgtg agttatgtag tatttttagt tttttttatta ggattttatt aatgtttttt   17520
gtattggaaa tttttgtgtt agaggttgaa tttatagtaa tttttaaaat taattaagaa   17580

gaatttagtt agaggttata gtaatgttgg aattataaaa tgtataagat ttattttttt   17640
ttggtttttt ttttatttat gttgtttatg tttgtgtatt tataagtttt atgtatatta   17700
aatttttaaa attaattatt attatgttat agagttttta ttggatagtg tttttttagtt   17760
tttattatat attttttttt ttttatgtag atttattatg ttggtgtttt gttatatagg   17820
gggtttgaga agaatgttat ttaattgttg ttgttgtgag tgtgtaaagt gattaggaga   17880
ttaggagaat gttgaaattt ttgttggaaa aatgtaaaga aaatttttat tttgagttag   17940
ttgtttatag agttagtgtg tgtgtgtgtg tgtgtgtttg taatataaaa tggatgtgaa   18000
tatatatata taaatagata tggttttgtt tttattttaa tttgaattat ttagataatt   18060
gtttttattt attatttgat tttaatgggt ttatataaat taggatattt tatttttta   18120
ggtatttagg ttgttgttga tttttagtgt ttttaatatt ttgtatatgt tggtattatg   18180
aggagtagtt atgtgttttt gggtttttta attattttgg aggttgattg aggtttttta   18240
tatatgtata tttgttgtga tgaaagtttt attggtagag tggagttatt agagtttta   18300
ttaaaatttt gtgggtttat gagagatggg tttagaaatt tatatggttt tgtggggttt   18360
tttggttttt taaaataagg tattaatatt taagttttta aaaatatttg tagttttggg   18420
gtttgaattt tgaaaaataa ggagtgaggg gttgtgtata ttaattatag tggagatttt   18480
ttttattttt taatgtgatg gagttttttt atgaaatgaa gttttaaggg gtatggtatt   18540
gtggggatta tagttatttt gaggtttaaa agaagaaatt ggaatatgat tagtaaatat   18600
atttagtaga aaagagttgg atttttattg atttagttat aggttattgg ttggtagtgt   18660
aatgggagga aatatttatt ttatatatat attttatgat tttgggggaa ttagaggaaa   18720
tttaataaga aaatggttag aaatatttaa aatttttatt taaaagattt aagtaaatta   18780
gagttttatt agattaaaaa ttattataaa tgtaagagta ttgtttttag tgaaatgttg   18840
tggggtttga gaaggagatt ttttgttaaa tttttgggat aaaatgtgtt atttaagtat   18900
tagataatga gtagaatgta aattaattta atttttttta ttaataggtt gttagtgtaa   18960
tgtgtataat ttagtgataa gattgtagga tttaatatag ttggatgtat gagttttagt   19020
taatgtagat ttgttatatg aggatgtgtt ttattttgag taggtgtttg tatgtgtgga   19080
atggggtaaa gtggaataaa aggttaaaag tagaaatgtt gatttaaagt ttattatgaa   19140
gaaatttttt ttttgtagtt aaattatttt taaagtggga tgatattggt gaagaaagat   19200
tgaaaaataa tttttatgtg tgtttttgga ttgtaagttt aaaatgggga ggagttgtag   19260
atagggtttg ggggtggtta gggtaaagga gagatatata agttgtaaat atatttgtag   19320
tttgttttat ttattttgtt ttatattgaa taagtttttt aattttgtga ataaggataa   19380
ggagggagtg ttttaaagat attttatgtt ggtattgtaa attattgatt gtaatgttaa   19440
ataaatatat atttagagat gataatatta attttatagt aaaataattg tttatgtaga   19500
aatttagagg agattagttt gtttttttta gttgatttat gttgggggat aaaaggattt   19560
ttaaaaatta ttttgaatat gtttggattt ttttttttaa tttttttgga aattaaattt   19620
gtttggaaat agtgttataa agagttgatg tttttaaagg tgattttttt tgttttatat   19680
aaataaggtt ttgtttttgt tagttgagtg tagttttagg tttttttgttt ttagtttata   19740
tatatttttt ttgtttgttt ggattttaat ggtttaagat agttttgagt ttattgggaa   19800
aagaaaatga ttgttaaaaa ttatttttga aattggttat ttggtaatat ttttaattgt   19860
atggaaattt attaaggtat attttatata taattagttt aaggttgttg attttatagg   19920
ttttatggat ttaaatttga ttgataataa agtaaataag agagttgaat ttaaagtgtg   19980
gttttttttgg gttaggatga gtttaatata gtgtataagg aatttgaaag atttaggata   20040
tgtgttttaa ttaatgttaa gtagaatgga taagttttta gtattttgaa aatgttgggt   20100
tagggttttt tttttattgt gtgtttttttg tttggggatt aataagtatt atagagaatg   20160
tgatttgagg tgattttttta tttttgtata aatttagagt gaattattaa atagttgttt   20220
gtttaaagtt aaggtaattt ttttttgatg ggtttatttg ttttttgatt tttaatttat   20280
tagtttgttt ttttagggtt ttgttttttt tgtaattaaa gttttttttag attagtgtag   20340
tatttatttg ataggttgtt tggaaaattt aagattggag aggtgatttg ttgttgtttt   20400
ttaaattttt tagttttaag taatgtgttt tttttttata tggggtgggg gattggaaat   20460
ggatgtagtg agatataaag agtgggtgtt ttgttgattt ttgtattttt tttttttga   20520
ttattttatt ttttttttttt aagtttttga tttttagttt tattttttta tttttgggtt   20580
```

```
tgtattaaaa gttggattgt tttgggttgg gtaggagttg aatttttggg agtttgtttg   20640
tgtagattta gtgtgtatgg tgaggtagta gtttggtttt gtattgttga taggtgtagg   20700
taggatagtt tttttattgt ggtttggggt gttttgattg gtgtggagtt atgttagttg   20760
tatttggaga agggtttggg aggaggtgga ggtggagagg gttggggagg gttgtggtgg   20820
agtgatgttt tggtattagg aagtttgttt ttggttttaa gatgttaggt taatagggaa   20880
gtgtggagtt gtagatttgg tttgttgttt gtttgggtgt ttggagttga gttgtggtaa   20940
ggtttggttt ttgtttgatt gtttgagggg tgtgtgtgtg tgtgttgtgg agggtgtgtt   21000
tagagggttg tgttgtggtt gtagtggttg ttgttgttgt aggggattta atattattta   21060
tttgtttttg ttatttttga tatttttttg ttagggttgt tgtgtggggg gggggtgggt   21120
agagtgtggt tggtgttagt ttttttattt ggaggggttt ttgggggagg gagggagaga   21180
agaagggggt ttttgtttat ttttgttttg ttttggagtt tggaagtttg tttttttaaag   21240
atgttttgag tggtgttttt ttgtttatat tttatgtttt tgtttgtttg ttgatttttt   21300
gtttttggat tttttttgttt gagttttttg gaggagatgg gggtagtttg gtttgagaat   21360
ttggtggggg ttgtgttttt tggttttttt tgtagtgggg aaattttgtg tttagagtgt   21420
gatttggagt gggtagtggt ggttatgggg gtttggtggg gtagtagtta aggattagta   21480
gagtgttgtg tttttttgtt tatgaattgt atgaaaggtt tgttttattt ggagtattga   21540
gtagtgggga ttaagttgtt ggttgttttt ttattttttt gttattattt ttagttgtta   21600
gttatggttt tggttttggt ttttggttag ttttggttgt tggatttttt taagtatagg   21660
ttggaggtgt atattatttt tgatattttt agtttggagg ttgtaggtaa ggtgttgtgt   21720
tgttttgtag atatttttgt ttagttgttt tgtgttattt gtttttttttt gttttaagga   21780
agttagtttt tttgggggga ggtgtggtgg gagtggttgt ttgtttggtt ttttgtagaa   21840
tttttgggag ttggaatttt gattattttg tattttttta gttttttttt gattggtttg   21900
gtttttgggg tgttaagggt gtgagtaatt ttgttgtttt ttttatttgt attttggttt   21960
ttttttgtt ttttgggtta taaaaatttt agtattttga tttgaggatt tttagaggtt   22020
gttgattttt gtttttgttt tttttttggt ttttagtttt tgaggagttt tatttgttag   22080
gaaattgttt gaaattattt agaaatgttt tttgtgaaga ggtatttttt tttttttttt   22140
gggaaagggt tggtgaattt tggtgtttaa ttgaattttt atattttttt ttagtttttt   22200
taaattgtat ggaaatttga gttttttgtg agggggaggg gggtttgtaa attatgtgtg   22260
tgtgtgtgtt ttaggagatt tggtgtgttt gtgtagaggt gtataaatat atttgaaagt   22320
ataggttata aaagtgaatg tgttgttgta gtgagataaa tatgtaaata aaatgtgtgg   22380
tgttgggggga ggggaggaaa tggggtgtgg atatttatat ttgtgtttgt atattttata   22440
ggtgtagtgt tttttgtggt ttggagttgt tgtgtgtatt tttttttggt gttaggtagt   22500
ttagtttttt tatggttttt gttgttggtt tagttggtgt ttgtgttgta ggtgggtatg   22560
ttgatgggaa agtgtgtgtg ttttgttttt agagaaagat aaaagttagt aggggaagaa   22620
tgaggatgtg ggtgttgagg atttgtttaa gaagaagtgg taaaggtggt agtggattta   22680
ttttattagt tagtagtttt aggagttgga ggttattttt tagaggaatt gttatttgga   22740
tatgtttata tgtgaagaaa ttgttgtgtg gattaatttt atggaagttt gagtttgggt   22800
aggagttagt atggagtttg ggagggatgg ggggaggatg ttgtggaggt ataggttaag   22860
tagattagga gagaatgtgg aaggtagtgt tgtttgggag ggtgttggtg gggtgtagtt   22920
ttgtaaaggt agaaggtttt gtggtggttt ggttgtgaga ttatagtttt tttttttgagg   22980
ttgataggat tgttgttttg gtttaggttt ttagagtggt attggtttat tgttttgtta   23040
ttttgtgatt ttatgagttg ggttgtatgg gtaatttttt gtataggata ttgtgttttt   23100
ggtttgtagt tgttagagta gagttaataa aatttttatt aggttaagag ttgtgaatag   23160
gttttaattt gtgagttttt aataaggaaa atttgttaga gatatggaag agttggtttt   23220
ttttgggaaa tttttgtttt ggttttggtt tagttttttt tttttttgggt ttgtgttttt   23280
tatatttttt ttatggttgt tttggttatt taggtttttt ttatatattt tattttttag   23340
ttttgtgatt tttgggagta aagttttaat atataattat tagtttttttt agaaggagaa   23400
agaaaaaaag aagaaagatt tttttgtttg gtttatttat tttttttttag gagttgaatt   23460
ttggaaattg aaatttatat tttttttttt aaattataat tatagttttg taaaaagggt   23520
ttattttaat tttgtagtaa atttgtattt tatggattgg taaaaatgag tttaaataaa   23580
taatttaata gtaatgtttt ggtttatgtt ggttggtgga agattttaaa tttgttagga   23640
ttttggaagt agaaaataga attaagtaaa ttaagtggta tttagaggtt ttgttgttaa   23700
aaaaaaaaaa ttaagtgttt tgggtagaaa aaataaagtt tttggttaga gtagagtaaa   23760
taaaaagaag aaaataatga taaaaagaat aaagattaaa atgttttttt aaattagagg   23820
gaatgaagat attttttggg tggtatttgt gtaaggtatg aggttatgtt ggtggataaa   23880
aggttgggaa gaagttgaaa atggttttag tttaattgtt tagagttaga gttgggtttt   23940
gggtggtgtg gttttgagta aggttagttt tttattagtt tttttgtata ttaagggaat   24000
gggtttttta tgtatttttt ttgtttgagt aaagtttaga tggtttaggg tagaaatggt   24060
aagtaattaa agatagagtt tatgggtttt ttgggatttt ttgaaaatgt tttttttattt   24120
tgtttgttat tttgtagttt tattttagtg ttttgtagtt gtggtgttgg gttttttttg   24180
tagttgtttt tttttttagg gtggttgttt gttgagttaa gtgggagtga ggtgtgtttt   24240
```

ttatagtagt tgggtgtaaa gaggaagggg gataaaaagg aaattaagaa tgaaaggaaa 24300
aagagaaaaa gtggattata tggttgggtt tggtggagat gtgtaatgtg aaatattatt 24360
ggtgttagtt tggatatttt aggttaggtt ttttttaat atataaaagt tgttgtttgg 24420
ggtgataggg aggtttgatg tggattggga ttggggttgt ggttgggtta ttggatatgg 24480
gtggaagttg gttggtttgg gtggttgttt gtaaagttaa atgatttggt tgggtttggt 24540
gtgtggatag gtttgtggtg ggtttagggt aaagaagagg tagagtgaaa gaagggggaa 24600
tttttaaaat tatttttttt gggtttttgg agtttaatat gttaagtttt tggagttaat 24660
gagttgatga agaggtggtt ttttgttttt tatttggttg ttttgttagg tgagaaagag 24720
tgttggtggt ttagtttttg ttaagggagt atgtattagg gggtggggga tgatagtgga 24780
ggttagggaa ggaagggagg aattgtgtgg gagaaagagt gattttttag tgttttttta 24840
gttttttttt tttatttgtg ggtttgtggt tttggaatgg aagtaagttt gtaaggtgtt 24900
ttgggaaggg ttggaaaagt ttgttgtttt gtgtttgttt tatattaagt gtttttggat 24960
ttggagaaat gtttggttga gtgattaaat tgtttgtagg tttttatgtg tttggttgag 25020
gtttgtggtg tagttttgag ttttagtttg taggttagag tagattaggt tttttgtgtt 25080
tggtggagat ttgggttagt aattgaaagt tggttttggt attttggtgt gtagggtggt 25140
gtagtgaagt gaggttaggg tgtgtgagtg tgttagtgtg tgtgttgggg gaaggtgggg 25200
gttggttttt gatggaagtt ttagtaattt gtattgtggt atttgtttgt ttttttgttt 25260
taattgtttt taggtttggt ttaagaattg ttgggttaaa tggagaaaga gggagtgtaa 25320
ttagtaggtt gagttatgta agaatggttt tgggttgtag tttaatgggt ttatgtagtt 25380
ttatgatgat atgtatttag gttatttta taataattgg gttgttaagg gtttatatt 25440
tgtttttta tttattaaga gttttttttt ttttaatttt atgaatgtta atttttgtt 25500
attatagagt atgttttttt tatttaattt tattttgttt atgagtatgt tgtttagtat 25560
ggtgttttta gtagtgatag gtgttttggg ttttagtttt aatagtttga ataatttgaa 25620
taatttgagt agtttgttgt tgaattttgt ggtgttgatg tttgtttgtt tttatgtgtt 25680
gttgattttt ttgtatgttt atagggatat gtgtaatttg agtttggtta gtttgagatt 25740
gaaagtaaag tagtatttta gttttggtta tgttagtgtg tagaatttgg tttttaattt 25800
gagtgtttgt tagtatgtag tggattggtt tgtgtgagtt gtatttatag tgttgggatt 25860
ttaggatttt gttggatggg gtaattttgt ttttgaaaga ttgggaatta tgttagaagg 25920
ttgtgggtat taaagaaagg gagagaaaga gaagttatat agagaaaagg aaattattga 25980
attaaagaga gagttttttt gattttaaag ggatgttttt agtgtttgat atttttttatt 26040
ataagtattt ttaatagttg taaggatata tatataaata aatgtttgat tggatatgat 26100
attttaatat tattataagt ttgttatttt ttaagtttag tattgttaat atttaaatga 26160
ttgaaaggat gtatatatat tgaaatgtta aattaatttt ataaaagtag ttgttagtaa 26220
tattataata gtgtttttaa aggttaggtt ttaaaataaa gtatgttata tagaagtgat 26280
taggattttt tgtttgtgag taagggagtg tatatattaa atgttatatt gtatgttttt 26340
aatatattat tattattata aaaaatgtgt gaatattagt tttagaatag tttttttggt 26400
ggatgtaatg atgtttttga aattgttatg tataatttat tttgtgtata atattttgta 26460
taatattatt gttttatttt ttagtaaata tgaaataaat gtgttttatt ttatgggagt 26520
aaaatatatt gtatataaat tggtttggat tttttttttt tttttttgtt attaatttgg 26580
ttaggatatt ttagttattg tttttaaat aaattagttt tttttgtttg tttagttaaa 26640
tatataaggt agtagttttt atttaaattt ggtagaaata aatgatagtt atttattaga 26700
aattaaaaag aaaaaaaaag gtatttttgg gggggaaaag ggttataaaa tttaattttg 26760
tttttttaat ttttttttgg tttaaattta gaggatttta ttatggttag taaataatat 26820
gaaaaagaaa aaagaagaaa gaaatttagt aagtttatta gtttaaaatg atttttaagt 26880
ttattttttt atggggaaat ttatattttt agtaaattgt tttggagaaa tatttgtgta 26940
tgtatatatg tatagtttat atgtattttt tttaggagga atatatttat aataaattta 27000
tagggaaata tttttagttt aaaatattta ggtttttatg tttattttta ggtttaagta 27060
gagagatttt ttatgttata ttgtattatt atttttaaat tttttggaga tattaaaaga 27120
aataaagatg atttttaata attatagttt tttagttttt taaagaattt aggggttgag 27180
aggttagagt ggagtttttt gagttttgtt gagtaatatg tagttgaggt aaaggttatg 27240
tttttggtgt tttgttttaa ataatattga tttattaatt ttaaatttgt ttgtttttga 27300
aattatatag gattatagtt tgtaaattgt aggataatga agtaaattaa gatgaattat 27360
agttttggtt ttttttgtta tttttttgata tttaaatagg gaatgagttt ggtgtgagtg 27420
tttaaatgaa ttttaagtat ttgatttttt tttatttgtg atttttagtt ttaaaaaaat 27480
gtgaaatttg attttataat aaatagaaat aaatattatt tagttttaga gaatttattt 27540
ttatggtgtt aggagggttg ttgtggaggt gggggggaggg atgtgttgag attttttgtt 27600
atgtttgtta atttttttgta taattaaagt gggtgagaat aaatattatg ttggggaatt 27660
tagagtaaaa agtaattgtt gatttttttgg agttgataat attattgttt ttttgtttta 27720
gttgttttta tttgaatgaa attttattta gaagtttttg gagtttgaat attgagtttt 27780
tgttttgtaa gaaattgttg ttgttattta aagagattgt agataatgtt tttgatttaa 27840
gattagtgtt taatgtatat tttttttttt tttaaagttt tgtttttgat ttgtggaggg 27900

```
attatgtagt agtgggggt agataaaagt tttttggatg agggttattt tttattatgt   27960
tgtttatttg agagtagtgg agaggaaaat ggtttttatg ggtggatttt ggtttttgga   28020
gttgtagggt ttagtggttt tggtttttttt gtttttagt ttggtagggg gtggggaggg   28080
ttaaagggtg gaggggaagg aaggagttag aagaggggat ttggggatgg gggttggaag   28140
tgttaatgag atttgtttgg aggatttagg ttttttgtag gttggtgagt gatttgggag   28200
ttttgggtaa aagaggtgta ttttggttta gtttggttgt tgaattagaa taatgtgagg   28260
atattaatta atttgtagga aataaaaaat ggaagttgag gtttaggaag agttgttatt   28320
tttttgattt gagtggtgta ggttgggggt ggagatttgg gatttaagag aggttatttt   28380
ttttatttta gttttttttt tttggatttt taaaaggaat aattttattt tttttttgt   28440
ttttttata atttttattt ttgttagttt gtaggttgtt tgttttttttt tgtattttt   28500
tttttattt agtgagagaa atttagtttt tagagt                               28536
```

<210> 966
<211> 20
<212> DNA
<213> Artificial Sequence

<220>
<223> chemically treated genomic DNA (Homo sapiens)

<400> 966
ggagtggagg aaattgagat 20

<210> 967
<211> 22
<212> DNA
<213> Artificial Sequence

<220>
<223> chemically treated genomic DNA (Homo sapiens)

<400> 967
ccacacaaca aatactcaaa ac 22

<210> 968
<211> 33
<212> DNA
<213> Artificial Sequence

<220>
<223> chemically treated genomic DNA (Homo sapiens)

<400> 968
tgggtgtttg taatttttgt tttgtgttag gtt 33

<210> 969
<211> 22
<212> DNA
<213> Artificial Sequence

<220>
<223> chemically treated genomic DNA (Homo sapiens)

<400> 969
gtaggggagg gaagtagatg tt 22

<210> 970

## Claims

1. A method for providing a prognosis of a subject with a prostate cell proliferative disorder comprising the following steps of:

    a. determining the methylation status of the gene PITX2 and/or regulatory regions thereof in a biological sample obtained from said subject; and
    b. determining therefrom the prognosis of said subject whereby hypermethylation is indicative of poor prognosis.

2. A method according to claim 1, wherein at least one further prognostic variable is factored in when determining the prognosis of b).

3. A method according to claim 2, wherein said prognostic variable is selected from the group consisting of nomogram, PSA level and Gleason score.

4. A method according to claim 1 to 3, comprising the following steps of:

    a) isolating genomic DNA from a biological sample taken from said subject;
    b) treating the genomic DNA, or a fragment thereof, with one or more reagents to convert 5-position unmethylated cytosine bases to uracil or to another base that is detectably dissimilar to cytosine in terms of hybridization properties;
    c) contacting the treated genomic DNA, or the treated fragment thereof, with an amplification enzyme and at least two primers comprising, in each case a contiguous sequence at least 18 nucleotides in length that is complementary to, or hybridizes under moderately stringent or stringent conditions to a sequence selected from the group consisting of SEQ ID NOS 962-965 and complements thereof, wherein the treated DNA or a fragment thereof is either amplified to produce one or more amplificates, or is not amplified;
    d) determining, based on the presence or absence of, or on the quantity or on a property of said amplificate, the methylation state of at least one CpG dinucleotide sequence of the gene PITX2 and/or regulatory regions thereof or an average, or a value reflecting an average methylation state of a plurality of CpG dinucleotide sequences of the gene PITX2 and/or regulatory regions thereof; and
    e) determining from said methylation state the prognosis of said subject

5. The use of an oligomer, comprising a sequence of at least 9 contiguous nucleotides that is complementary to, or hybridizes under moderately stringent or stringent conditions to a treated genomic DNA sequence selected from the group consisting of SEQ ID NOS: 962-965, and sequences complementary thereto in providing a prognosis of a subject with a prostate cell proliferative disorder.

6. The use of a kit in providing a prognosis of a subject with a prostate cell proliferative disorder, said kit comprising:

    - at least one of a bisulfite reagent; and
    - at least two nucleic acid molecules comprising, in each case a contiguous sequence at least 16 nucleotides that is complementary to, or hybridizes under moderately stringent or stringent conditions to a sequence selected from the group consisting of SEQ ID NOS: 962-965 and complements thereof.

7. The use of a composition in providing a prognosis of a subject with a prostate cell proliferative disorder, said composition comprising the following:

    a) a nucleic acid comprising a sequence at least 18 bases in length of a segment of the chemically pretreated genomic DNA according to one of the sequences taken from the group consisting of SEQ ID NOS: 962-965 and sequences complementary thereto, and
    b. a buffer comprising at least one of the following substances: magnesium chloride, dNTP, of taq polymerase, an oligomer, in particular an oligonucleotide or peptide nucleic acid (PNA)-oligomer, said oligomer comprising in each case at least one base sequence having a length of at least 9 contiguous nucleotides which is complementary to, or hybridizes under moderately stringent or stringent conditions to a pre-treated genomic DNA according to one of the SEQ ID NOS: 962-965 and sequences complementary thereto.

## Patentansprüche

1. Verfahren zum zur Verfügung stellen einer Prognose für ein Subjekt mit einer proliferativen Erkrankung von Prostatazellen, umfassend die folgenden Schritte von:

   a) Bestimmen des Methylierungsstatus des Gens PITX2 und/oder regulatorischen Regionen davon in einer biologischen Probe, erhalten von dem Subjekt; und
   b) Bestimmen daraus der Prognose für das Subjekt, wobei Hypermethylierung auf eine schlechte Prognose hinweist.

2. Verfahren nach Anspruch 1, wobei mindestens eine weitere prognostische Variable faktoriert wird, wenn die Prognose in b) bestimmt wird.

3. Verfahren nach Anspruch 2, wobei die prognostische Variable ausgewählt ist aus der Gruppe bestehend aus Nomogramm, PSA Spiegel und Gleason-Wert.

4. Verfahren nach Anspruch 1 bis 3, umfassend die folgenden Schritte von:

   a) Isolieren genomischer DNA von einer biologischen Probe, die von dem Subjekt genommen wurde;
   b) Behandeln der genomischen DNA, oder eines Fragments davon, mit einem oder mehreren Reagenzien, um an der 5-Position unmethylierte Cytosinbasen zu Uracil oder in eine anderen Base umzuwandeln, die in ihren Hybridisierungseigenschaften von Cytosin nachweisbar verschieden ist;
   c) in Kontakt bringen der behandelten genomischen DNA, oder des behandelten Fragments davon, mit einem Amplifikationsenzym und mindestens zwei Primem, umfassend in jedem Fall eine durchgehende Sequenz von mindestens 18 Nukleotiden Länge, die komplementär ist zu, oder unter moderat stringenten oder stringenten Bedingungen an eine Sequenz ausgewählt aus der Gruppe bestehend aus SEQ ID NOS 962-965 und Komplementen davon hybridisiert, wobei die behandelte DNA oder das Fragment davon entweder amplifiziert wird, um so eines oder mehrer Amplifikate zu ergeben oder nicht amplifiziert wird;
   d) Bestimmen, basierend auf der Anwesenheit oder Abwesenheit von, oder auf der Menge oder anhand einer Eigenschaft des Amplifikats, des Methylierungsstatus von mindestens einer CpG Dinukleotidsequenz des Gens PITX2 und/oder regulatorischer Regionen davon oder eines Durchschnitts, oder eines Wertes, der einen durchschnittlichen Methylierungsstatus einer Vielzahl an CpG Dinukleotidsequenzen des Gens PITX2 und/oder regulatorischer Regionen davon widerspiegelt; und
   e) Bestimmen der Prognose des Subjekts aus dem Methylierungsstatus.

5. Verwendung eines Oligomers, umfassend eine Sequenz von mindestens 9 durchgehenden Nukleotiden die komplementär ist zu, oder unter moderate stringenten oder stringenten Bedingungen an eine behandelte genomische DNA Sequenz ausgewählt aus der Gruppe bestehend aus SEQ ID NOS: 962-965, und dazu komplementären Sequenzen hybridisiert, zum zur Verfügung stellen einer Prognose für ein Subjekt mit einer proliferativen Erkrankung von Prostatazellen.

6. Verwendung eines Kits zum zur Verfügung stellen einer Prognose für ein Subjekt mit einer proliferativen Erkrankung von Prostatazellen, wobei das Kit umfasst:

   - mindestens ein Bisulfitreagenz; und
   - mindestens zwei Nukleinsäuremoleküle umfassend, in jedem Fall, eine durchgehende Sequenz von mindestens 16 Nukleotiden die komplementär ist zu, oder unter moderat stringenten oder stringenten Bedingungen an eine Sequenz ausgewählt aus der Gruppe bestehend aus SEQ ID NOS: 962-965 und Komplementen davon hybridisiert.

7. Verwendung einer Zusammensetzung zum zur Verfügung stellen einer Prognose für ein Subjekt mit einer proliferativen Erkrankung von Prostatazellen, wobei die Zusammensetzung das folgende umfasst:

   a) eine Nukleinsäure umfassend eine Sequenz von mindestens 18 Basen Länge, eines Segments der chemisch vorbehandelten genomische DNA gemäß einer der Sequenzen ausgewählt aus der Gruppe bestehend aus SEQ ID NOS: 962-965 und dazu komplementären Sequenzen, und
   b) einen Puffer umfassend mindestens eine der folgenden Substanzen: Magnesiumchlorid, dNTP, taq Polymerase, ein Oligomer, insbesondere ein Oligonukleotid oder Peptidnukleinsäure (PNA)-Oligomer, wobei das Oligomer in jedem Fall mindestens eine Basensequenz mit einer Länge von mindestens 9 durchgehenden Nukleotiden umfasst, die komplementär ist zu, oder unter moderat stringenten oder stringenten Bedingungen an eine vorbehandelte genomische DNA gemäß einer der SEQ ID NOS: 962-965 und dazu komplementärer Sequenzen hybridisiert.

## Revendications

**1.** Procédé pour fournir un pronostic d'un sujet souffrant d'un trouble de prolifération de cellules de la prostate comprenant les étapes suivantes consistant à :

a) déterminer l'état de méthylation du gène PITX2 et/ou des régions régulatrices de celui-ci dans un échantillon biologique obtenu à partir dudit sujet ; et
b) déterminer à partir de là le pronostic dudit sujet par lequel une hyperméthylation est indicative d'un pronostic médiocre.

**2.** Procédé selon la revendication 1, dans lequel au moins une autre variable pronostique est prise en considération lors de la détermination du pronostic de b).

**3.** Procédé selon la revendication 2, dans lequel ladite variable pronostique est choisie dans le groupe constitué du nomogramme, du taux de PSA et du score de Gleason.

**4.** Procédé selon la revendication 1 à 3, comprenant les étapes suivantes consistant à :

a) isoler l'ADN génomique à partir d'un échantillon biologique prélevé à partir dudit sujet ;
b) traiter l'ADN génomique, ou un fragment de celui-ci, avec un ou plusieurs réactifs pour convertir les bases de cytosine non méthylées en position 5 en uracile ou en une autre base qui est dissemblable de manière détectable de la cytosine en termes de propriétés d'hybridation ;
c) mettre en contact l'ADN génomique traité, ou le fragment traité de celui-ci, avec une enzyme d'amplification et au moins deux amorces comprenant, dans chaque cas une séquence contiguë d'une longueur d'au moins 18 nucléotides qui est complémentaire de, ou s'hybride dans des conditions modérément stringentes ou stringentes à une séquence choisie dans le groupe constitué des SEQ ID NO : 962 à 965 et des complémentaires de celles-ci, où l'ADN traité ou un fragment de celui-ci est soit amplifié pour produire un ou plusieurs amplificats soit non amplifié ;
d) déterminer, en se basant sur la présence ou l'absence de, ou sur la quantité ou sur une propriété dudit amplificat, l'état de méthylation d'au moins une séquence de dinucléotide CpG du gène PITX2 et/ou des régions régulatrices de celui-ci ou une moyenne, ou une valeur reflétant un état de méthylation moyen d'une pluralité de séquences de dinucléotide CpG du gène PITX2 et/ou des régions régulatrices de celui-ci ; et
e) déterminer à partir dudit état de méthylation le pronostic dudit sujet.

**5.** Utilisation d'un oligomère, comprenant une séquence d'au moins 9 nucléotides contigus qui est complémentaire de, ou s'hybride dans des conditions modérément stringentes ou stringentes à une séquence d'ADN génomique traité choisie dans le groupe constitué des SEQ ID NO : 962 à 965, et des séquences complémentaires de celles-ci en fournissant un pronostic d'un sujet souffrant d'un trouble de prolifération de cellules de la prostate.

**6.** Utilisation d'un kit dans la fourniture d'un pronostic d'un sujet souffrant d'un trouble de prolifération de cellules de la prostate, ledit kit comprenant :

au moins un d'un réactif de bisulfite ; et
au moins deux molécules d'acide nucléique comprenant, dans chaque cas une séquence contiguë d'au moins 16 nucléotides qui est complémentaire de, ou s'hybride dans des conditions modérément stringentes ou stringentes à une séquence choisie dans le groupe constitué des SEQ ID NO : 962 à 965 et des complémentaires de celles-ci.

**7.** Utilisation d'une composition dans la fourniture d'un pronostic d'un sujet souffrant d'un trouble de prolifération de cellules de la prostate, ladite composition comprenant les composants suivants :

a) un acide nucléique comprenant une séquence d'une longueur d'au moins 18 bases d'un segment de l'ADN génomique prétraité chimiquement selon l'une des séquences prises dans le groupe constitué des SEQ ID NO : 962 à 965 et des séquences complémentaires de celles-ci, et

b) un tampon comprenant au moins l'une des substances suivantes : du chlorure de magnésium, des dNTP, de la taq polymérase, un oligomère, en particulier un oligomère d'oligonucléotide ou d'acide nucléique peptidique (PNA), ledit oligomère comprenant dans chaque cas au moins une séquence de bases ayant une longueur d'au moins 9 nucléotides contigus qui est complémentaire de, ou s'hybride dans des conditions modérément stringentes ou stringentes à un ADN génomique prétraité selon l'une des SEQ ID NO : 962 à 965 et des séquences complémentaires de celles-ci.

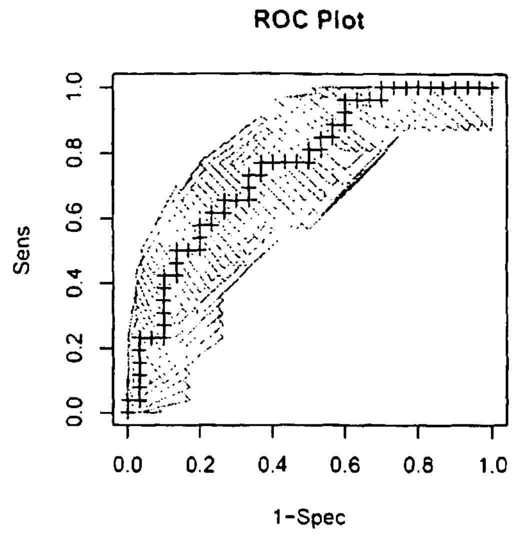


Figure 1

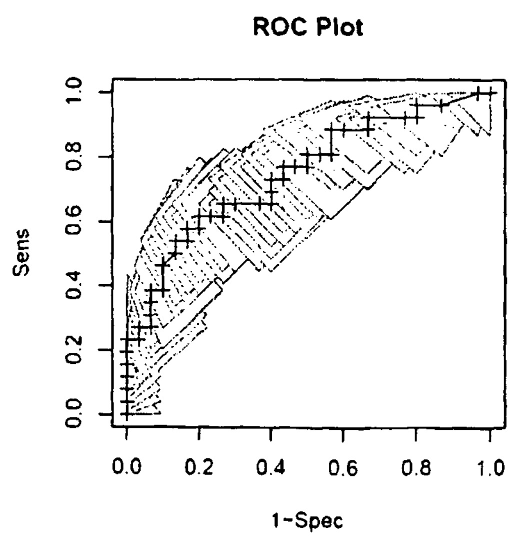


Figure 2

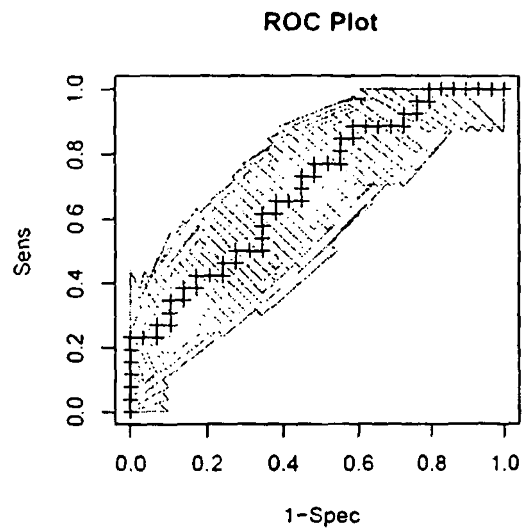


Figure 3

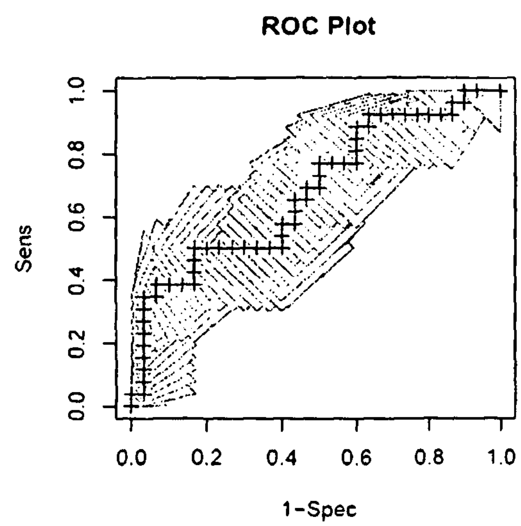


Figure 4

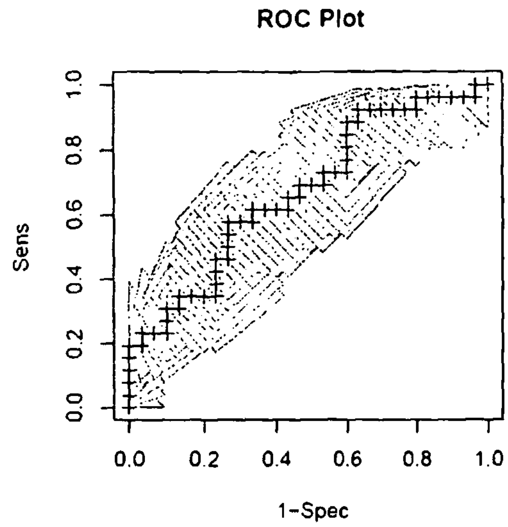


Figure 5

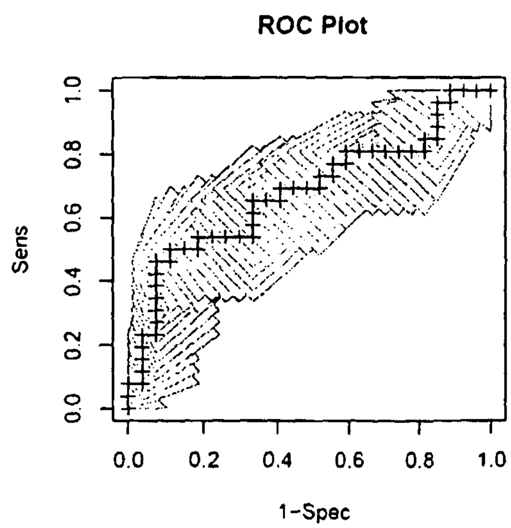


Figure 6

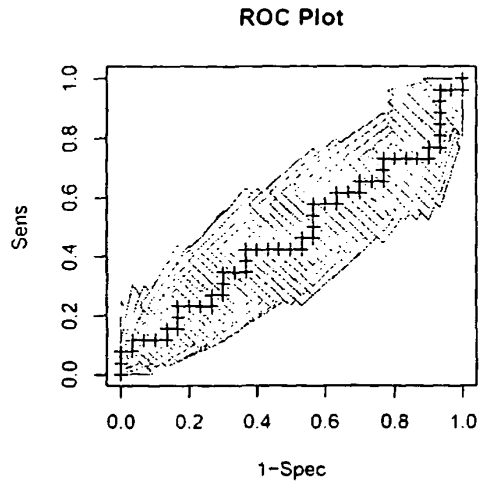


Figure 7

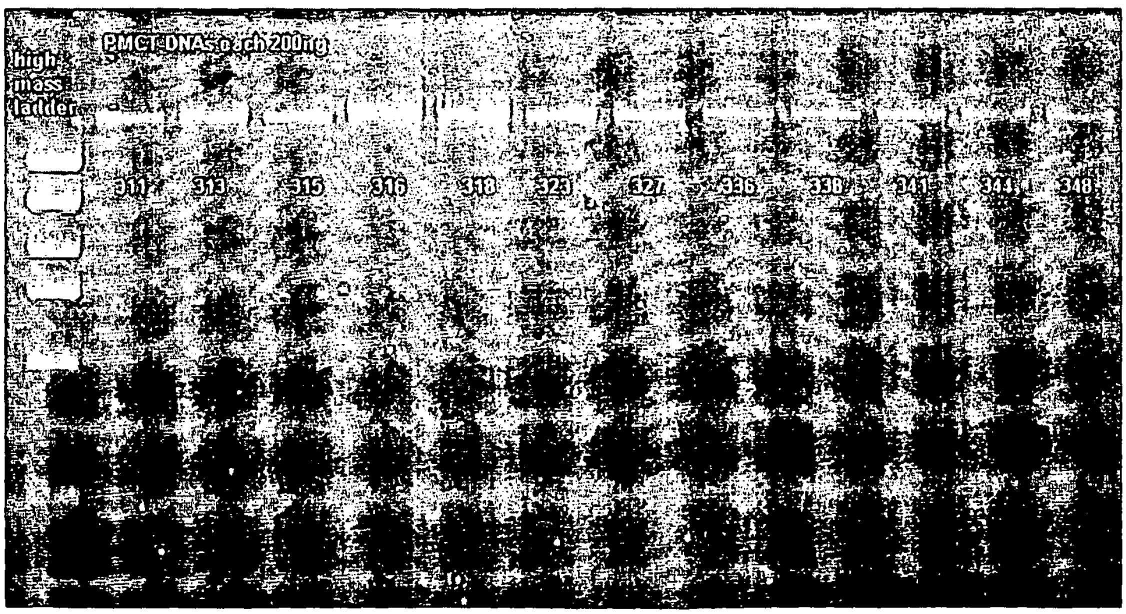


Figure 8

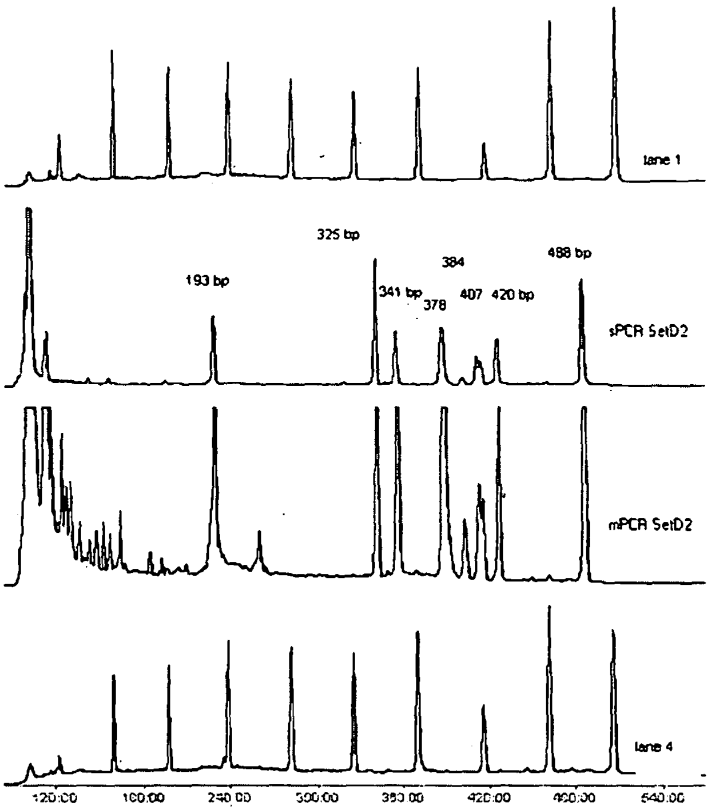


Figure 9

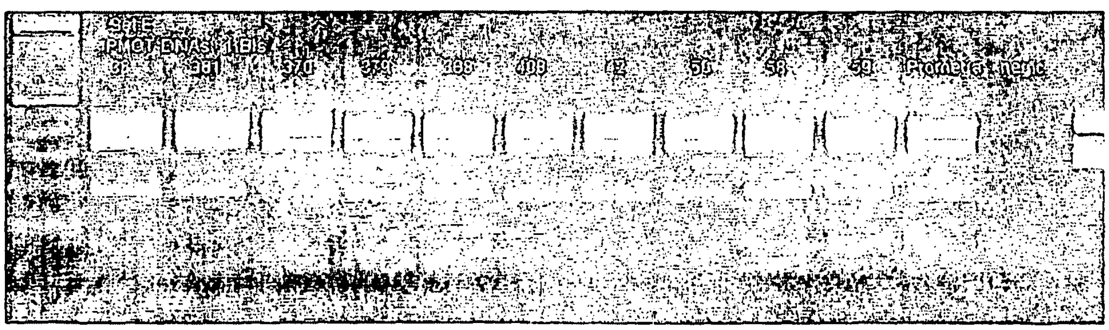

Figure 10

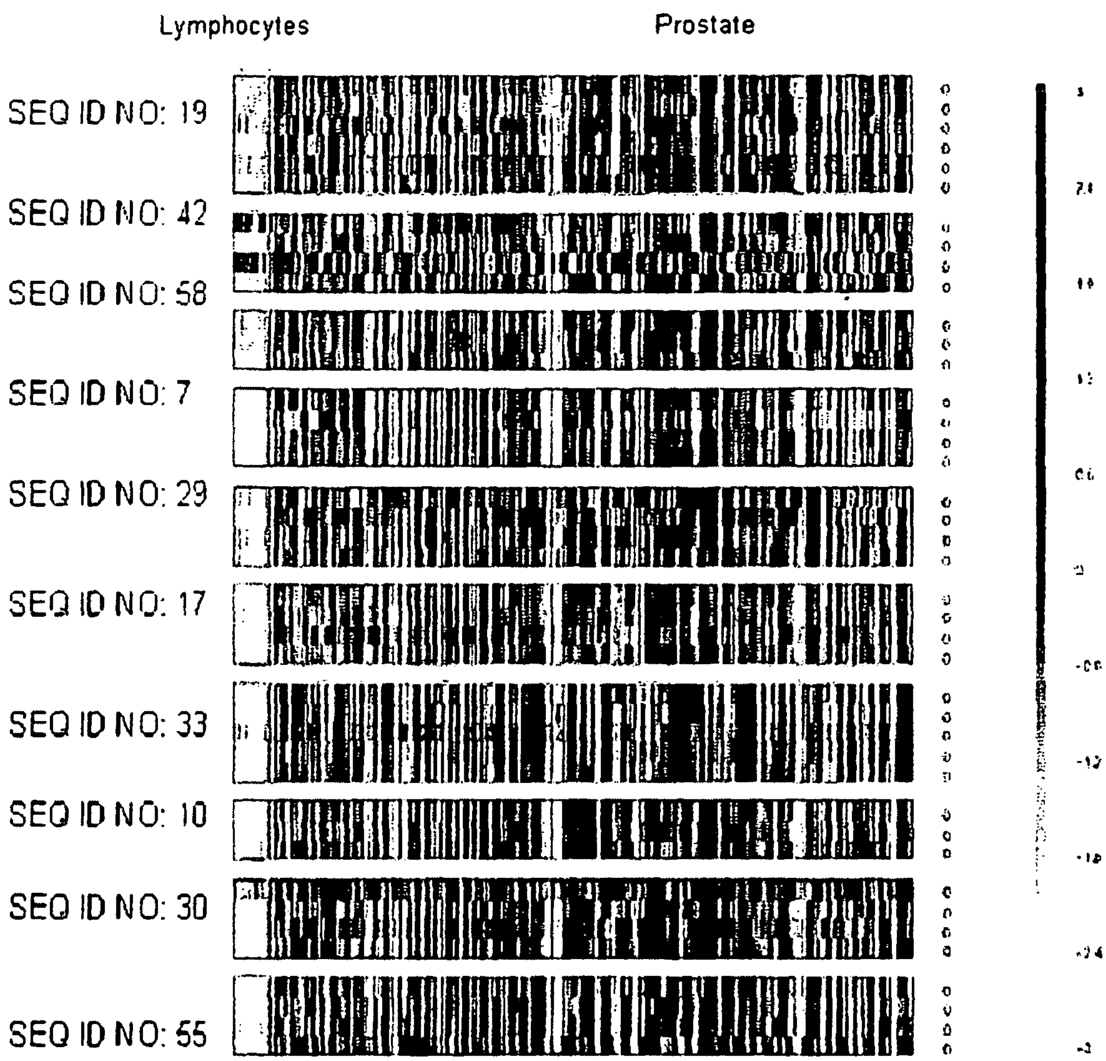
Lymphocytes
Prostate
SEQ ID NO: 19
SEQ ID NO: 42
SEQ ID NO: 58
SEQ ID NO: 7
SEQ ID NO: 29
SEQ ID NO: 17
SEQ ID NO: 33
SEQ ID NO: 10
SEQ ID NO: 30
SEQ ID NO: 55

Figure 11

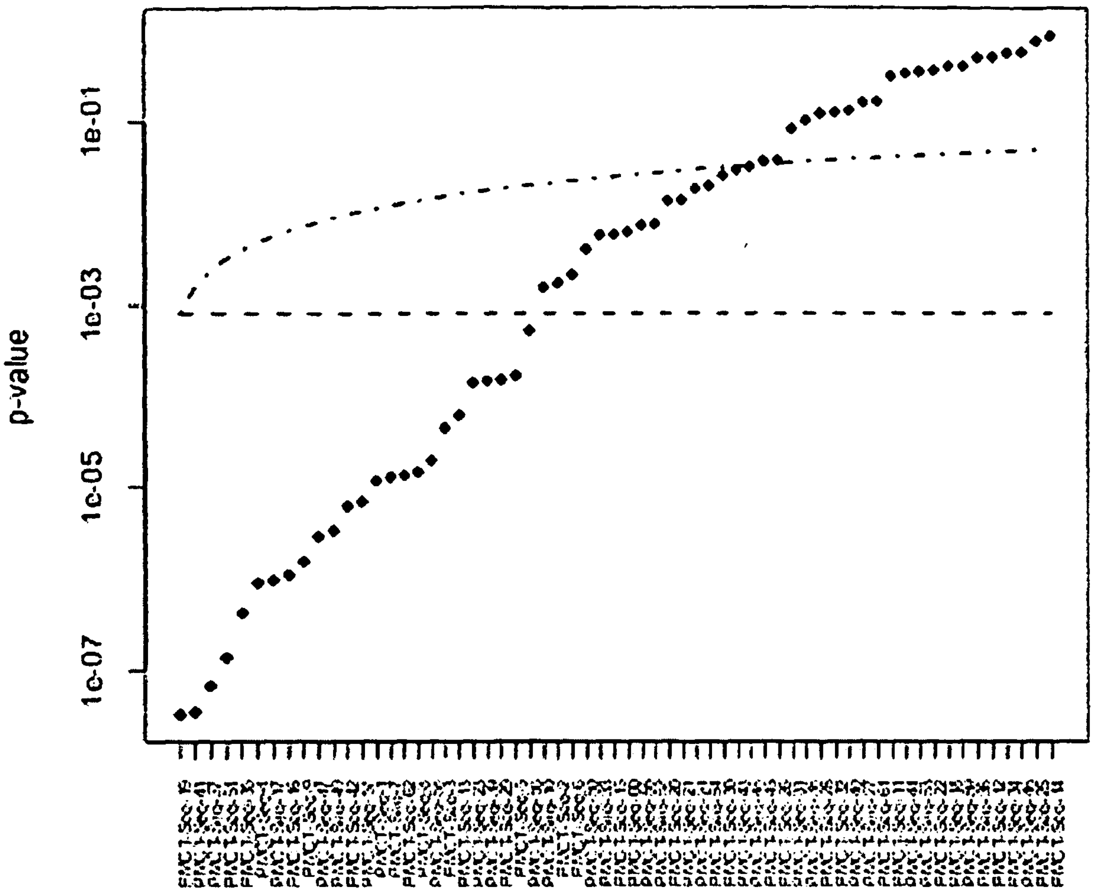


Figure 12

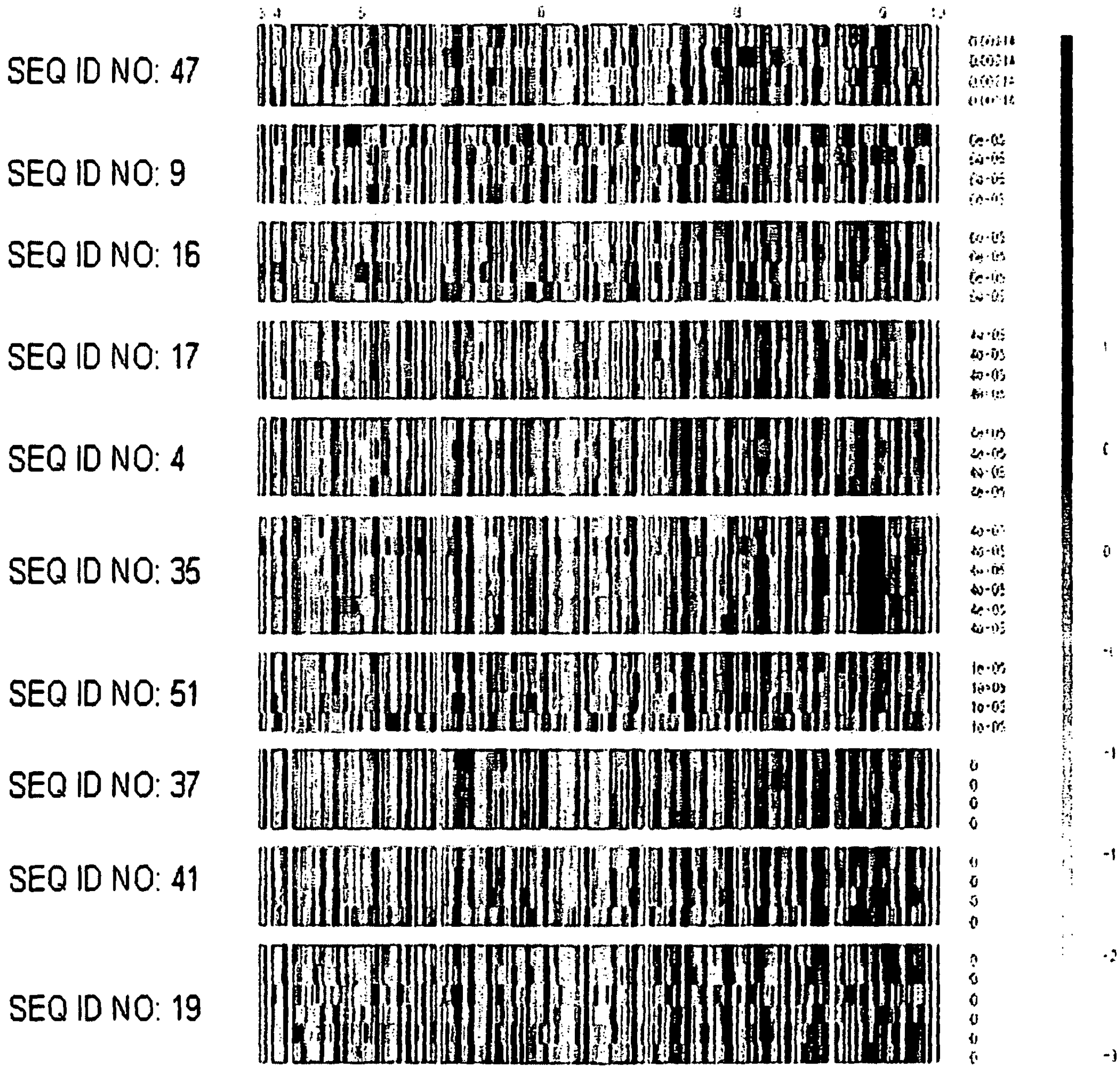

Figure 13

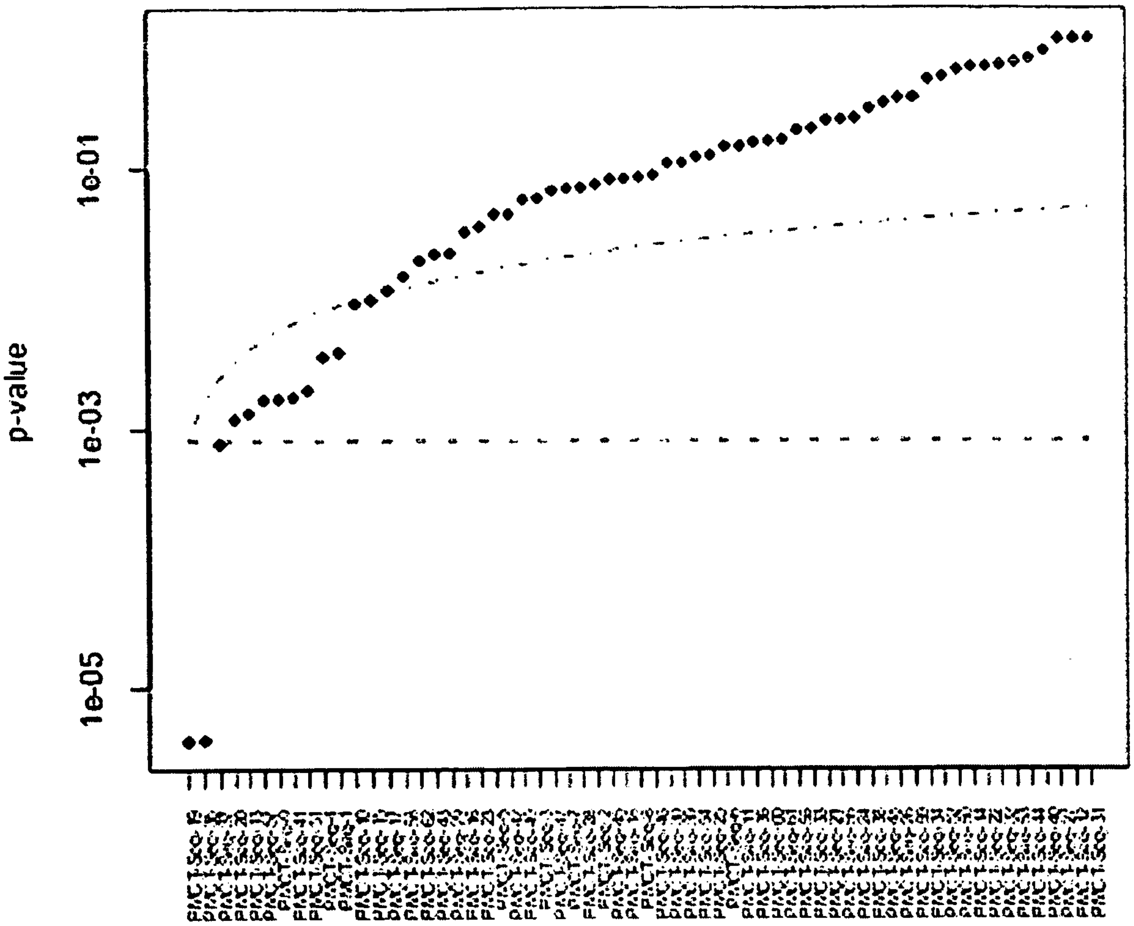


Figure 14

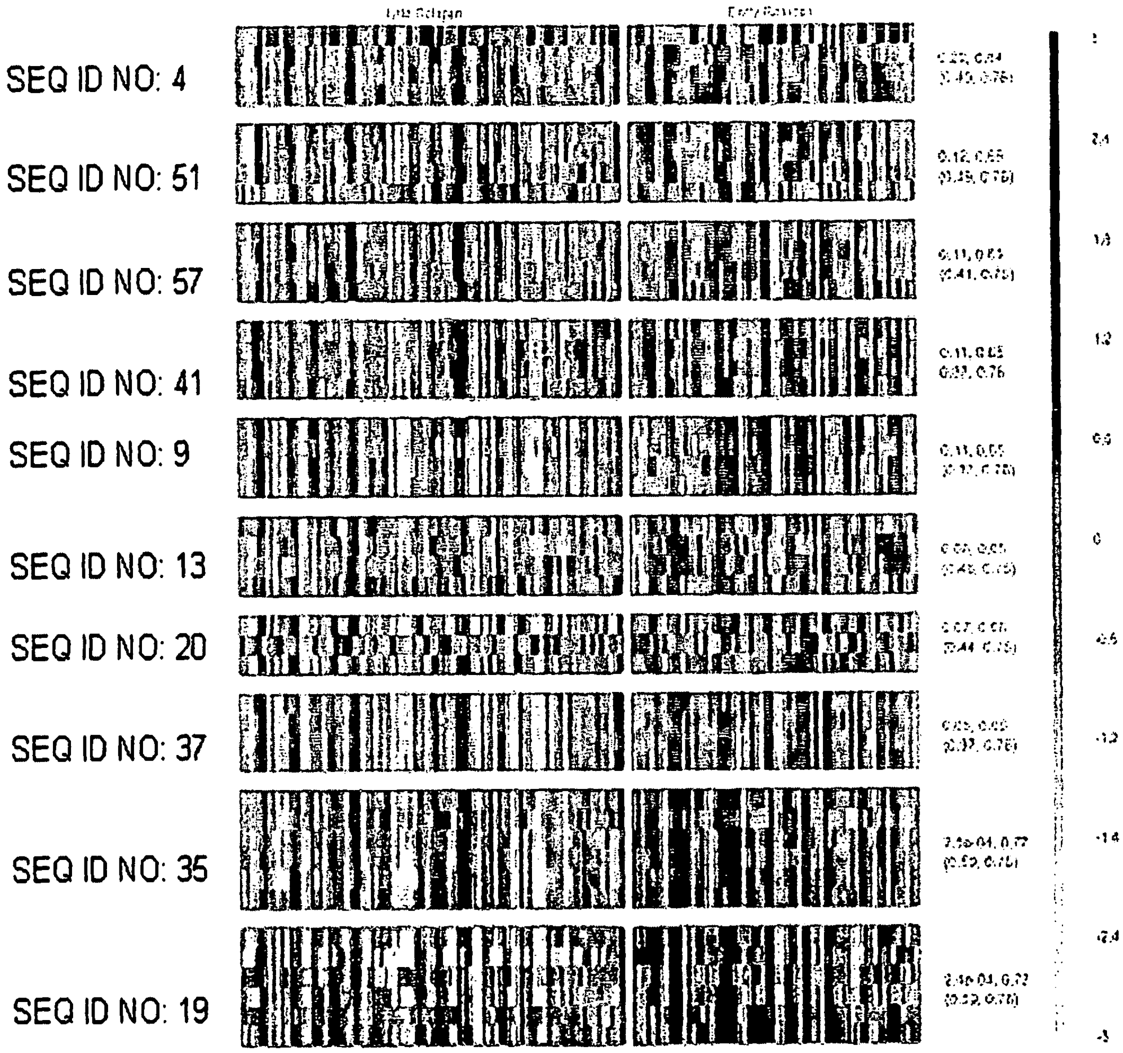
SEQ ID NO: 4
SEQ ID NO: 51
SEQ ID NO: 57
SEQ ID NO: 41
SEQ ID NO: 9
SEQ ID NO: 13
SEQ ID NO: 20
SEQ ID NO: 37
SEQ ID NO: 35
SEQ ID NO: 19

Figure 15

Figure 16 Amplificate of SEQ ID NO: 14

1 GAAGAGGTGCTGAGAAATTAAAAATTCAGGTTAGTTAATGCATCCCTGCCGCCGGCTGCAGGCTCCGCCTTTGCATTAAG 80

1 **GAAGAGGTGTTGAGAAATTAAAA** 80

1 GAAGAGGTGtTGAGAAATTAAAAATTtAGGTTAGTTAATGtATtttTGtCGtCGGtTGtAGGtTtCGttTTTGtATTAAG 80

1 TTAAG 80

1 TTAAG 80

---

81 CGGGCGCTGATTGTGCGCGCCTGGCGACCGCGGGGAGGACTGGCGGCCCGCGGGAGGGGACGGGTAGAGGCGCGGGTTAC 160

81 CGGGCGtTGATTGTGCGCGttTGGCGAtCGCGGGGAGGAtTGGCGGttCGCGGGAGGGGACGGGTAGAGGCGCGGGTTAt 160

81 CGGGCGTTGAT TAGAGGCGCGGGTTAT 160

81 TGGGTGTTGAT TAGAGGTGTGGGTTAT 160

---

161 ATTGTTCTGGAGCCGGCTCGGCTCTTTGTGCCTCCTCTAGCGGCCAAGCTGCGAGGTACAGCCCTCTATTGTTCTAGGAG 240

161 ATTGTTtTGGAGtCGGtTCGGtTtTTTGTGttTttTtTAGCGGttAAGtTGCGAGGTAtAGtttTtTATTGTTtTAGGAG 240

---

241 CACAGAAACCTCCTGTGTGGGCGGCGGGTGCGCGAGCTAGAGGGAAAGATGCAGTAGTTACTGCGACTGGCACGCAGTTG 320

241 tAtAGAAAttTttTGTGTGGGCGGCGGGTGCGCGAGtTAGAGGGAAAGATGtAGTAGTTAtTGCGAtTGGtACGtAGTTG 320

241 TTGCGATTGGTACGTA 320

241 TGTGATTGGTATGTAGT 320

---

321 CGCGCTTTTGTGCGCACGGACCCCGCGCGGTGTGCGTGGCGACTGCGCTGCCCCTAGGAGCAAGCCACGGGCCCAGAGGG 400

321 CGCGtTTTTGTGCGtACGGAtttCGCGCGGTGTGCGTGGCGAtTGCGtTGttttTAGGAGtAAGttACGGGtttAGAGGG 400

321 TTTTTGTGTGTATGGAT 400

321 TTTTGTGCGTACGGAT

---

401 GCAAAATGTCCAGGTCCCCCGCTGGGAAGGACACACTATACCCTATGGCAAGCCAGGGTGGG 480

401 **TTTATGGTAAGTTAGGGTGGG** 480

401 GtAAAATGTttAGGTttttCGtTGGGAAGGAtAtAtTATAtttTATGGtAAGtAGGGTGGG 480

Figure 17: Amplificate of SEQ ID NO: 15

| | | |
|---|---|---|
| 1 | TGCAGGAGAGGTTGGGAAGGGGTGGGGGACGGGGCTCGGGGGAGGTCTCCGAGGGACTCTAGTAAGCGGGGAAGGGCGCC | 80 |
| 1 | **TGTAGGAGAGGTTGGGAAG** | 80 |
| 1 | TGtAGGAGAGGTTGGGAAGGGGTGGGGGACGGGGtTCGGGGGAGGTtTtCGAGGGAtTtTAGTAAGCGGGGAAGGGCGtC | 80 |
| 81 | GGGAAAGTTTCAGATCCACGGCTGCGCGGGCCACGAGCCCACCCGAACGCCGACCACTGCTTTCCGTCGACTTCTATTTC | 160 |
| 81 | GGGAAAGTTTtAGATttACGGtTGCGCGGGttACGAGtttAttCGAACGtCGAttAtTGtTTTtCGTCGAtTTtTATTTt | 160 |
| 81 | GGTTATGAGTTTATTTGAA | 160 |
| 81 | GTTACGAGTTTATTCGA | 160 |
| 161 | CTGGGAACGCGCGAAAGCAAACCCAAGTCAGACTGCGGAGGTCGCTGGGGAGGGAAGGTTCAAGGAGTTCTCGCCGATCC | 240 |
| 161 | tTGGGAACGCGCGAAAGtAAAtttAAGTtAGAtTGCGGAGGTCGtTGGGGAGGGAAGGTTtAAGGAGTTtTCGtCGATtt | 240 |
| 161 | TTGGGAATGTGTGAAA AGGAGTTTTTGTTGATT | 240 |
| 161 | AACGCGCGAAAGTAAA GAGTTTTCGTCGATTT | 240 |
| 241 | TGCTGAATAAAGGGGGTTCCGAGCTGGGCCGAGATGGGGCATGCGCGGGAAGACCCCTGCCCGCTGTTCCCCCCCACCGC | 320 |
| 241 | **T** | 320 |
| 241 | TGtTGAATAAAGGGGGTTtCGAGtTGGGtCGAGATGGGGtATGCGCGGGAAGAttttTGttCGtTGTTtttttAtCGt | 320 |
| 241 | GTATGTGTGGGAAGAT | 320 |
| 241 | TATGCGCGGGAAGATT | |
| 321 | CCCAGTGGATGCCATGCCTGG | 400 |
| 321 | **TTTAGTGGATGTTATGTTTGG** | 400 |
| 321 | tttAGTGGATGttATGttTGG | 400 |

Figure 18: Amplificate of SEQ ID NO: 16

459 GCTTCTAAGTGCTGGGTGATTTCCGCGGGAAGTCCCTGGGGCAGCAGCCAGCATCTGATGCTAGGATCCTATCCATACCA 380

459 **GTTTTTAAGTGTTGGGTGATTT** 380

459 GtTTtTAAGTGtTGGGTGATTTtCGCGGGAAGTtttTGGGGtAGtAGttAGtATtTGATGtTAGGATttTATttATAttA 380

459 ATTTTCGCGGGAAGTT 380

459 GTGATTTTTGTGGGAA 380

379 ACCCAGATGTTCCTGCGTTGCAGTAGGTCCACACTGCCGGGGAGAGGTGGCAAGAGACAAATCAACTCTAGTGGAAACGG 300

379 AtttAGATGTTttTGCGTTGtAGTAGGTttAtAtTGtCGGGGAGAGGTGGtAAGAGAtAAATtAAtTtTAGTGGAAACGG 300

379 ATATTGTTGGGGAGAG 300

379 TATATTGTCGGGGAGA 300

299 GAAGCGAGTGGGAACAACAGCCTCACAGCAAGACCGGGGCTCAGCAGCCGCGCTCTTGGCCTGCGTTTGGTGGACCGGAA 220

299 GAAGCGAGTGGGAAtAAtAGttTtAtAGtAAGAtCGGGGtTtAGtAGtCGCGtTtTTGGttTGCGTTTGGTGGAtCGGAA 220

299 AGTAGTTGTGTTTTTGG - ATTGGAA 220

299 TAGTAGTCGCGTTTTT GATCGGAA 220

219 TCCGGGTTGTGTGTTTCATTTTCTTTTTTGCCAGAAAATATCATTAGGAACTAAACGGCTCTTTTTAAGAGTGTGAAAGT 140

219 TtCGGGTTGTGTGTTTtATTTTtTTTTTTGttAGAAAATATtATTAGGAAtTAAACGGtTtTTTTTAAGAGTGTGAAAGT 140

219 TTTGGGTTG 140

219 TTCGGGTT

139 GTTACTCTTGCCAGTCACCAATGAGGAGGGCGCCAAAAGCTTCCCAGAGCCAGTTCCCACAGCCAGGACTATTCTCTAAA 60

139 GTTAtTtTTGttAGTtAttAATGAGGAGGGCGttAAAAGtTTtttAGAGttAGTTtttAtAGttAGGAtTATTtTtTAAA 60

59 GCCAGAGGTCGGAAAGTTGAACAGAACGGAAGCAAATTGCCTGGCAGAGGGAAGAGGCA -20

59 **GTTTGGTAGAGGGAAGAGGTA** -20

59 GttAGAGGTCGGAAAGTTGAAtAGAACGGAAGtAAATTGttTGGtAGAGGGAAGAGGtA -20

Figure 19: Amplificate of SEQ ID NO: 17

| | | |
|---|---|---|
| 1 | AAGAGTCCCAGGAAATGTGCCCCCCGGGATTACTGGTATTTGCTGGCTCCTCGGAACAAGATGCCAACTTGGCTAAG CAG | 80 |
| 1 | **AAGAGTTTTAGGAAATGTGTTTTT** | 80 |
| 1 | AAGAGTtttAGGAAATGTGttttCGGGATTAtTGGTATTTGtTGGtTttTCGGAAtAAGATGttAAtTTGGtTAAGtAG | 80 |
| 1 | TTGGTTTTTCGGAATAA | 80 |
| 1 | GGTTTTTTGGAATAAGAT | 80 |
| 81 | TTCTGGATCTCGGCGTCGATGTATCCCCCTAGCGAATCTCAGCTGGTGCTGCGCAGAGACAGCAGTCAGCGTCTGCC GGT | 160 |
| 81 | TTtTGGATtTCGGCGTCGATGTATttttTAGCGAATtTtAGtTGGTGtTGCGtAGAGAtAGtAGTtAGCGTtTGtCGGT | 160 |
| 81 | TTCGGCGTCGATGTAT GGTGTTGCGTAGAGAT TAGTTAGTGTTTGTTGG | 160 |
| 81 | TTTGGTGTTGATGTATT GTGTTGCGTAGAGATA AGTTAGCGTTTGTCGG | |
| 161 | GGCGCGGCCCAGGAGGAGCAGAGGGTCTGGTGAGTAGAA | 240 |
| 161 | **GTAGAGGGTTTGGTGAGTAGAA** | 240 |
| 161 | GGCGCGGtttAGGAGGAGtAGAGGGTtTGGTGAGTAGAA | 240 |

Figure 20: Amplificate of SEQ ID NO: 18

| | | |
|---|---|---|
| 1 | CAAGGCAAGGGAAGGCCAGAAACGAGGCAACCCGTGCACAGACCGGGCTTGGTCAACGCCCCAGGGGCGGGGCCA GGCGG | 80 |
| 1 | **TAAGGTAAGGGAAGGTTAGAAA** | 80 |
| 1 | tAAGGtAAGGGAAGGttAGAAACGAGGtAAttCGTGtAtAGAtCGGGtTTGGTtAACGttttAGGGGCGGGGttAGGCGG | 80 |
| 1 | AATTCGTGTATAGATCGG | 80 |
| 1 | ATTTGTGTATAGATTGGG | 80 |
| 81 | GCCGTTCCTGGGGGGCGGGGCCAGCCTCGGTCCAATAAGGGGTACTCGACGCCCCATTGGCCACCTGCCTCGAAAGG GGG | 160 |
| 81 | GtCGTTttTGGGGGGCGGGGttAGttTCGGTttAATAAGGGGTAtTCGACGtttATTGGttAttTGttTCGAAAGGGGG | 160 |
| 81 | AGGGGTATTTGATGTTT | 160 |
| 81 | GGGTATTCGACGTTTT | 160 |
| 161 | AAGACAGTCTGGGCGGGCAGGACGTGCGGAGGGAGAGGCAGCGGTGGCGCGAGGCTCAACTCGAAGCGCTATTGGT GGGA | 240 |
| 161 | AAGAtAGTtTGGGCGGGtAGGACGTGCGGAGGGAGAGGtAGCGGTGGCGCGAGGtTtAAtTCGAAGCGtTATTGGTGGGA | 240 |
| 161 | ATTCGAAGCGTTATTG | 240 |
| 161 | TTTGAAGTGTTATTGGT | 240 |
| 241 | CTGATAGTCTTGTGCGCCAAGAAGCTGGCAGAAGGGAAGGGGCGGGACATCAGTTCAGGATCTCAAACCGCATTGG TCGT | 320 |
| 241 | tTGATAGTtTTGTGCGttAAGAAGtTGGtAGAAGGGAAGGGGCGGGAtATtAGTTtAGGATtTtAAAtCGtATTGGTCGT | 320 |
| 241 | AAATCGTATTGGTCGT | 320 |
| 241 | AAATTGTATTGGTTGT | 320 |
| 321 | CTGCCTCGGCTGAGAGAGGCGATGCTTGAAGTTCATTGGTCTTTGTGAGACAGGGTAAGAAAAGCAGCGCGAGCTTG GCG | 400 |
| 321 | tTGttTCGGtTGAGAGAGGCGATGtTTGAAGTTtATTGGTtTTTGTGAGAtAGGGTAAGAAAAGtAGCGCGAGtTTGGCG | 400 |
| 321 | AAAAGTAGCGCGAGTT | 400 |
| 321 | TT AGTAGTGTGAGTTTGG | |
| 401 | GTTCCTCTGGCCACTTTCTCACAGTGTCTTTGGGCGTCTTCTTGACTGAATCTGACTCCATTGGAGGCTGTGGTAAC | 480 |
| 401 | **TTTTATTGGAGGTTGTGGTAAT** | 480 |
| 401 | GTTttTtTGGttAtTTTtTtAtAGTGTtTTTGGGCGTtTTtTTGAtTGAATtTGAtTttATTGGAGGtTGTGGTAAt | 480 |

Figure 21: Amplificate of SEQ ID NO: 19

1 CTATTATTTCAGATGGAGTGAGGTTGCACGACTGGGATGGAAGAAAGGAATCCCTTAAATTTGGGGGAATTTCTGTT CTC 80

1 **TTATTATTTTAGATGGAGTGAGGTT** 80

1 tTATTATTTtAGATGGAGTGAGGTTGtACGAtTGGGATGGAAGAAAGGAATttTTAAATTTGGGGGAATTTtTGTTtTt 80

---

81 TGTTCTAAGACCATTTTACTTGGGGTGTGGGGGTGGGCGCGGCGGTCAGGGCAGTGGAACGCAGTCGCGGCTGCGCC ATC 160

81 TGTTtTAAGAttATTTTAtTTGGGGTGTGGGGGTGGGCGCGGCGGTtAGGGtAGTGGAACGtAGTCGCGGtTGCGttATt 160

81 GGAATGTAGTTGTGGT 160

81 GAACGTAGTCGCGGTT 160

---

161 CCTGCACTTCCAGGCGCGCGGGAGGGACCGGCGGGGACGCGAGCTGCGGACTCTGGCGAACTCGGGGGAGGCAGAC AGGG 240

161 ttTGtAtTTtAGGCGCGCGGGAGGGAtCGGCGGGGACGCGAGtTGCGGAtTtTGGCGAAtTCGGGGGAGGtAGAtAGGG 240

161 ATTTTGGCGAATTCGG 240

161 TGGTGAATTTGGGGGA 240

---

241 GGAGGCGGACACCCAGCCGGCAGGCGTCTCAGCCTCCCCGCAGCCGGCGGGCTTTTCTCCTGACAGCTCCAGGAAAG GCA 320

241 GGAGGCGGAtAtttAGtCGGtAGGCGTtTtAGttTttCGtAGtCGGCGGGtTTTTtTttTGAtAGtTtAGGAAAGGtA 320

241 ATTTAGTTGGTAGGTGT TTTTTTGTAGTTGGTGG 320

241 TTAGTCGGTAGGCGTT TTTTCGTAGTCGGCGG 320

---

321 GACCCCTTCCCCAGCCAGCCAGGTAAGGTAAAGACTGCTGTTGAGCTTGCTGTTACTGAGGGCGCACAGACCCTGGG GAG 400

321 GAttttTTttttAGttAGttAGGTAAGGTAAAGAtTGtTGTTGAGtTTGtTGTTAtTGAGGGCGtAtAGAtttTGGGGAG 400

321 ATTGAGGGCGTATAGA GGGGAG 400

321 TGAGGGTGTATAGATTT GGAG 400

---

401 ACCGAAGCTTGCCACTGCGGGATTCTGTGGGGTAACCTGGGT 480

401 **TTGTGGGGTAATTTGGGT** 480

401 AtCGAAGtTTGttAtTGCGGGATTtTGTGGGGTAAttTGGGT 480

401 ATTGAAGTTT 480

401 ATCGAAGTTTGT

Figure 22: Amplificate of SEQ ID NO: 20

1 AGATGTTTTACATCTGTCTGGGAATCCCGATTTTCCAGGCTCCCTGAGAAATCTATCTGCCTGGAAGAAGTTGCACTTCG 80

1 **AGATGTTTTATATTTGTTTGGGA** 80

1 AGATGTTTTAtATtTGTtTGGGAATttCGATTTTttAGGtTtttGAGAAATtTATtTGttTGGAAGAAGTTGtAtTTCG 80

1 TTGTATTTCG 80

1 TTTTG 80

81 TCGCCACAGGAGCCGCCGAGAGCCAGGGATGGAGATTTCAGACTTAGGACTCTGCCACGATTCTACTGATCCTTAAGCGC 160

81 TCGttAtAGGAGtCGtCGAGAGttAGGGATGGAGATTTtAGAtTTAGGAtTtTGttACGATTtTAtTGATttTTAAGCGt 160

81 TCGTTAT TAAGCGT 160

81 TTGTTATAGGAGT AGTGT 160

81 ATAGGAGTCGTCGAGA 160

81 ATAGGAGTTGTTGAGAG 160

161 CAACAGAACGAAATGGACATGCCTATTAGCTACCACAAATCGAATTATTTTATATGAGAGAGAGAAATGTGGTTCAAGTT 240

161 tAAtAGAACGAAATGGAtATGttTATTAGtTAttAtAAATCGAATTATTTTATATGAGAGAGAGAAATGTGGTTtAAGTT 240

161 TAATAGAACGA 240

161 TAATAGAATGAAAT

241 CCACATTTGTCCTTTAAATAATCGACCCTCCCTAACCCTGTCCCCAAAGGCTCGTGGAAATTAGGGAGGGGGTTGGGGAG 320

241 ttAtATTTGTttTTTAAATAATCGAtttTtttTAAtttTGTttttAAAGGtTCGTGGAAATTAGGGAGGGGGTTGGGGAG 320

321 ACGGTTCCAGAAACAAAACGCTTTCCTCCAGCACGGCCTCTACATCCAGTCCACCCAAGCTGATTTTTGTAACTCATATA 400

321 ACGGTTttAGAAAtAAAACGtTTTttTtAGtACGGttTtTAtATttAGTtAttAAGtTGATTTTTGTAAtTtATATA 400

401 AAGAATGGGCCCGGGTGGGTGGTCAGTGAAGACTGGGGTGAGCA 480

401 **TAGTGAAGATTGGGGTGAGTA** 480

401 AAGAATGGGttCGGGTGGGTGGTtAGTGAAGAtTGGGGTGAGtA 480

Figure 23: Amplificate of SEQ ID NO: 21

1 TAAAAATGGAAGGGAAGACAGATCCAATTTGAAATCCTCCTTGAGAAAAAACAAATCAAAACTAGTTCCTGGGGAA GAAA 80

1 **TAAAAATGGAAGGGAAGATAGA** 80

1 TAAAAATGGAAGGGAAGAtAGATttAATTTGAAATttTttTTGAGAAAAAAtAAATtAAAAtTAGTTttTGGGGAAGAAA 80

---

81 GCCTCAGCTAGGTCAGGAGGAAACTTACGCATCTTTGATTCCCTTTCCGCTTTGGAGAGGCATTACCCGTTCAAGCCC AG 160

81 GttTtAGtTAGGTtAGGAGGAAAtTTACGtATtTTTGATTttTTTtCGtTTTGGAGAGGtATTAttCGTTtAAGttAG 160

81 AG 160

81 160

---

161 CCAATGCGGCCGGCCAGGATTTACAGCTCTTATCAAGGGTTAGATTTTGCGAAAGATATAAAGAAAGGAGTTTCCTA ACA 240

161 ttAATGCGGtCGGttAGGATTTAtAGtTtTTATtAAGGGTTAGATTTTGCGAAAGATATAAAGAAAGGAGTTTttTAAtA 240

161 TTAATGTGGTTGGT TTAGATTTTGTGAAAGATA 240

161 TTAATGCGGTCGGTTA AGATTTTGCGAAAGATA 240

---

241 CACCGGGTCTTCTCACAGCAACAAGACACCGAAATTAAGCCTTCTATGGTTTGTGTCTGTAGGACTTTTTAGATAAAA CT 320

241 tAtCGGGTtTTtTtAtAGtAAtAAGAtAtCGAAATTAAGttTTtTATGGTTTGTGTtTGTAGGAtTTTTTAGATAAAAtT 320

241 AAGATATCGAAATTAAGTT 320

241 AAGATATTGAAATTAAGTTT .

---

321 GGCAGCCCCATTAGATAAGAAATGGCTTTTTAGAAGCTTTGGGGGAAGCGCGGGGGTTCTTCGTGGCCTTCCTGTGA CTG 400

321 **TGTGATTG** 400

321 GGtAGtttATTAGATAAGAAATGGtTTTTTAGAAGtTTTGGGGGAAGCGCGGGGGTTtTTCGTGGttTTttTGTGAtTG 400

---

401 TCTTTGGGAGAC 480

401 **TTTTTGGGAGAT** 480

401 TtTTTGGGAGAt 480

Figure 24: Amplificate of SEQ ID NO: 22

1 TTGTGGTCACTCTGAGATGGCAGATTGGGGTCCCTGTTGTCCTTGGACAGATGAGAATGCCGAGAGCTCGTATGCCT TGC 80
1 **TTGTGGTTATTTTGAGATGGTA** 80
1 TTGTGGTtAtTtTGAGATGGtAGATTGGGGTttTGTTGTttTTGGAtAGATGAGAATGtCGAGAGtTCGTATGttTTGt 80
1 ATGTCGAGAGTTCGTA 80
1 GTTGAGAGTTTGTATGT 80

81 CCAAGGGCACACAGCAAGGCCGGGTGCCCATGCGGCTGTCCCAGGGACCCACTGACCCTGCTGTCCCCCTCAGGCCA CAT 160
81 ttAAGGGtAtAtAGtAAGGtCGGGTGttATGCGGtTGTttAGGGAttAtTGAttTGtTGTttttTtAGGttAtAT 160

161 TAGGATCTCAGACCTGGGCTTGGCTGTGAAGATCCCCGAGGGAGACCTGATCCGCGGCCGGGTGGGCACTGTTGGCT ACA 240
161 TAGGATtTtAGAttTGGGtTTGGtTGTGAAGATttCGAGGGAGAttTGATtCGCGGtCGGGTGGGtAtTGTTGGtTAtA 240
161 AGATTTTCGAGGGAGA 240
161 AGATTTTTGAGGGAGAT 240
161 ATTTGATTTGTGGTTGG 240
161 TTTGATTCGCGGTCGG 240

241 TGGGTGAGTGCTGGGCTGCCTGTGTCAATGCACCTTGAGACCCACCACCTGCTCACCGCCGGCCGAGCCCCCAGAGC CTG 320
241 TGGGTGAGTGtTGGGtTGttTGTGTtAATGtAttTTGAGAttAttAttTGtTtAtCGtCGGtCGAGttttAGAGttTG 320
241 TTTATTGTTGGTTGAGT 320
241 TATCGTCGGTCGAGTT

321 CCCGCAGAGCCTCTCGCCTCTTCATGCACTGCTGATCACACACAGAGTCACAGTCTCTGCAGGGCTGCTGGGGGGCG GCC 400
321 **TT** 400
321 ttCGtAGAGttTtTCGttTtTTtATGtAtTGtTGATtAtAtAtAGAGTtAtAGTtTtTGtAGGGtTGtTGGGGGGCGGtt 400

401 TTAGCCAGGGGGAGGG 480
401 **TTAGTTAGGGGGAGGG** 480
401 TTAGttAGGGGGAGGG 480

Figure 25: Amplificate of SEQ ID NO: 23

1 TCCACTCACCAGGTGAAGAGTTTGCCTTTTATCCTGCGCAGCAGGCTGGAGAACTCGGCCGCCGCTCCCTCCGCGAGGGG 80

1 **TTTATTTATTAGGTGAAGAGTTTGTT** 80

1 TttAtTtAttAGGTGAAGAGTTTGttTTTTATttTGCGtAGtAGGtTGGAGAAtTCGGtCGtCGtTttTtCGCGAGGGG 80

1 AGAATTTGGTTGTTGTT 80

1 AATTCGGTCGTCGTTT 80

---

81 CTCTGGGGCGCCGGGGCCCGCTGGCGAGTCTGCCTCCATCGGCGCCCTTGCGCGTCTCCGGCGCCCAAAACCCCCGAAGT 160

81 tTtTGGGGCGtCGGGGttCGtTGGCGAGTtTGttTttATCGGCGtttTTGCGCGTtTtCGGCGtttAAAAttttCGAAGT 160

81 TTTTTGGTGTTTAAAATTT 160

81 TTTCGGCGTTTAAAAT 160

81 ATTTTCGAAGT 160

81 TTTGAAGT 160

---

161 GCCGGGTCCCGGAAGACGGGAGGCGGTGCCTGTGCGGCCGGCTGGGGCTCCGCTCTGGCCCTGCGCTCCCTCCCTCGCTC 240

161 GtCGGGTttCGGAAGACGGGAGGCGGTGttTGTGCGGtCGGtTGGGGtTtCGtTtTGGtttTGCGtTttTttTCGtTt 240

161 TTTCGGAAGACGGGAG 240

161 TTTTGGAAGATGGGAG 240

161 GTCGG 240

161 GTTGGGTT

---

241 TCTTGCTCGCGTCCCTCTCGCTCCTCCGCTCGGCTCCTTCAGCGACCAATCATTAACTGTCAAGCCCTAAGGGCCAGACA 320

241 TtTTGtTCGCGTttTtTCGtTtTtCGtTCGGtTtTTtAGCGAttAATtATTAAtTGTtAAGtttTAAGGGttAGAtA 320

---

321 ATACATTTGCAGATTACAACCTGGCACCTCCAGGGTGGCAGGAGGA 400

321 **TTTTAGGGTGGTAGGAGGA** 400

321 ATAtATTTGtAGATTAtAAttTGGtAttTttAGGGTGGtAGGAGGA 400

Figure 26: Amplificate of SEQ ID NO: 24

---

49 TTGGAGAGATGTGCTGGCCAGGACCCGCTGACACAGGTATTCTGGTTGCGCCCCCACCTCACCCGGTGCCCGAGAGC 41
2 GAG 3

49 **TTGGAGAGATGTGTTGGTTAG** 41
2 3

49 TTGGAGAGATGTGtTGGttAGGAttCGtTGAtAtAGGTATTtTGGTTGCGttttAttTtAttCGGTGttCGAGAGCGAG 41
2 3

49 TGTTTGAGAGTGAG 41
2 3

49 GTGTTCGAGAGCGAG 41
2 3

49 TATTTGGTGTTTGAGAG 41
2 3

49 TTTATTCGGTGTTCGA 41
2 3

---

41 TGTGAGTGTATCGATTGTTTCAATTGATTTTTTTAAAGCTAGATAGCAGAGGCTGCGTGTCTGTACACGCAGATATAT 33
2 AC 3

41 TGTGAGTGTATCGATTGTTTtAATTGATTTTTTTAAAGtTAGATAGtAGAGGtTGCGTGTtTGTAtACGtAGATATATAt 33
2 3

41 TGT 33
2 3

41 T 33
2 3

---

33 ATACACAAATACAGATACTCAAGCGCACACAGATTACACGTACAGAGTCACAGACAAGGACACACACACACACATC 25
2 GTGG 3

33 ATAtAtAAATAtAGATAtTtAAGCGtAtAtAGATTAtACGTAtAGAGTtAtAGAtAAGGAtAtAtAtAtAtAtATCGTGG 25
2 3

33 ATTGTGG 25
2 3

33 TATCGTGG 25
2 3

---

25 TCCCTCACGCACACATACCTCCAAAGAGCCTTGGGTAACCTGGTGAGGTCCCTGAGTGCTGCCCAAGATGTGTGTGT 17
2 GGG 3

25 TtttTtACGtAtAtATAttTtAAAGAGttTTGGGTAAttTGGTGAGGTtttTGAGTGtTGttAAGATGTGTGTGTGGG 17
2 3

25 TTTTTTATGTATA 17
2 3

25 TTTTTTACGTAT 17
2 3

---

17 TGGGGACGCAGGGACGGAGAAAGGGGAGTGTGAAGCAGGATTTCCAGCAGGTCAAGTGCCGCTGATTCTCCTGCCC 93
2 CCGA

17 TGGGGACGtAGGGACGGAGAAAGGGGAGTGTGAAGtAGGATTTttAGtAGGTtAAGTGtCGtTGATTtTtTGtttCGA 93
2

17 GACGTAGGGACGGAGA 93
2

17 GATGTAGGGATGGAGA
2

---

92 CCCCAAATCCTTCCCTTTCTCCTCCTAGGGAGGTCTGAACCAGAAATCCCAAACCTGGGCTTGTTTCCACATCCACAG 13
AG

92 **ATTTATAGAG** 13

92 tttAAATttTTtttTTTtTttTttTAGGGAGGTtTGAAttAGAAATtttAAAttTGGGtTTGTTTttAtATttAtAGAG 13

---

12 GGGAGGAGTCCT -67

12 **GGGAGGAGTTTT** -67

12 GGGAGGAGTttT -67

Figure 27: Amplificate of SEQ ID NO: 25

40 TCTCCAGAACAAAGGACTTTAGGGCCCAAATTCCGTTTATTCAGTACTCCAAGTCCTAAAAACTTGGAATATCTGAT 32
7 GAA 8

407 **TTTTTAGAATAAAGGATTTTAGGG** 328

407 TtTttAGAAtAAAGGAtTTTAGGGtttAAATTtCGTTTATTtAGTAtTttAAGTttTAAAAAtTTGGAATATtTGATGAA 328

---

32 TAAAAGTGGCCGCTCCCCAGGCTGTCTCTTGAGAGAAGCCACCGGCACAGCTGACCTTGCCCGCTCCATCGCGTCAC 24
7 TGA 8

327 TAAAAGTGGtCGtTtttAGGtTGTtTtTTGAGAGAAGttAtCGGtAtAGtTGAttTTGttCGtTttATCGCGTtAtTGA 248

---

24 CCGCTCCTCAGACAGATGCGTCAGGCATCTCCGGCGGCCGCTCCACTCTGCGCCAGACTCGCTGCAGCAGCGGCAGG 16
7 CTT 8

247 tCGtTttTtAGAtAGATGCGTtAGGtATtTtCGGCGGtCGtTttAtTtTGCGttAGAtTCGtTGtAGtAGCGGtAGGtTT 168

247 TGTGTTAGATTTGTTTGT TAGCGGTAGGTTT 168

247 TTTGCGTTAGATTCGT TAGTGGTAGGTTT 168

---

16 CGCACACATCCCCGCCTGAGCATGCGCGCCAGCCTGCCTCTGCGGCCGCGCAGGCGTGCTTGTTTGCCGCAGTGCAG 88
7 GGG

167 CGtAtAtATtttCGttTGAGtATGCGCGttAGttTGttTtTGCGGtCGCGtAGGCGTGtTTGTTTGtCGtAGTGtAGGGG 88

167 CGTA AGTATGCGCGTTAGTT TTTTGTGGTTGTGTAGG 88

167 TGTA TGAGTATGTGTGTTAGT TTTTTGCGGTCGCGTA

---

87 TCCCAGCTCCCTCCCTCACCGGAATGACCTGGGGGGAGGGGGCTACTGGACCCCTAGGGCCCCACAGCACTGTTGCA 8
ATG

87 **AGTATTGTTGTAATG** 8

87 TttAGtTttTttTtAtCGGAATGAttTGGGGGGAGGGGGtTAtTGGAtttTAGGGtttAtAGtAtTGTTGtAATG 8

---

7 AGAGGGG -72

7 **AGAGGGG** -72

7 AGAGGGG -72

Figure 28: Amplificate of SEQ ID NO: 26

1 TCCCAGGAATGCAGGACACAAGGGGGCAGCCTACCTCAGGGCAGGGCCAGCGGGGTTCCTCATCCTTAGGCCAGTC CCCG 80
1 **TTTTAGGAATGTAGGATATAAGGG** 80
1 TttAGGAATGtAGGAtAtAAGGGGGtAGttTAttTtAGGGtAGGGttAGCGGGGTTttTtATttTTAGGttAGTtttCG 80

---

81 GGGCTCAGATGATTCCAGGGAAGGCCGACCCGGCTGACTGCCACCTCCACCATCACTCTGCAGAGCGGTTGAGCATG AAA 160
81 GGGtTtAGATGATTttAGGGAAGGtCGAttCGGtTGAtTGttAttTttAttATtAtTtTGtAGAGCGGTTGAGtATGAAA 160
81 AAGGTTGATTTGGTTG 160
81 GTCGATTCGGTTGATT 160

---

161 GACCAGCTGATCGCACAGAGCCTCCTAGAGAAACAGCAGATCTACCTGGAGATGGCCGAGATGGGCGGCCTCGAAG ACCT 240
161 GAttAGtTGATCGtAtAGAGttTttTAGAGAAAtAGtAGATtTAttTGGAGATGGtCGAGATGGGCGGttTCGAAGAttT 240
161 AGATGGGTGGTTTTGA 240
161 GGCGGTTTCGAAGATT 240

---

241 GCCCCAGCCCCGAGGCCTATTCCGTGGAGGGGACCCATCCGAGACCCTGCAGGGGGAGCTAATTCTCAAGTCGGCCA TGA 320
241 GtttAGtttCGAGGttTATTtCGTGGAGGGGAtttATtCGAGAtttTGtAGGGGGAGtTAATTtTtAAGTCGGttATGA 320
241 AAGTCGGTTATGA 320
241 AAGTTGGTTATGA 320

---

321 GCGAGAGTAAGTTGGCTGCCCACACCTCAAGGGTGCAGTCTTGCCGGGGTGGGCTCCTCAGGGAACCCCAGGCCAG GCTC 400
321 GCGAGAGTAAGTTGGtTGtttAtAttTtAAGGGTGtAGTtTTGtCGGGGTGGGtTttTtAGGGAAttttAGGttAGGtTt 400
321 GCGA 400
321 GTGA 400

---

401 ACGGCTCATTGTGGCCGACACGGCACCCTGTCCAGGACAGGCTTCTATGTGGGG 480
401 **GGATAGGTTTTTATGTGGGG** 480
401 ACGGtTtATTGTGGtCGAtACGGtAtttTGTttAGGAtAGGtTTtTATGTGGGG 480
401 TTGTGGTTGATATGGT 480
401 TGGTCGATACGGTATT

Figure 29: Amplificate of SEQ ID NO: 27

27 CAGGGTCTGATGATGTGATTTTCACCCCGCCGCATGTGAAGGCCACAGAGCAGATCATTCAGCTCACGCTGAAGGGC 19
3 ACA 4

27 3 **TAGGGTTTGATGATGTGATTTT** 19 4

27 3 tAGGGTtTGATGATGTGATTTTtAttCGtCGtATGTGAAGGttAtAGAGtAGATtATTtAGtTtACGtTGAAGGGtAtA 19 4

27 3 ATTTTGTTGTATGTGAAG 19 4

27 3 ATTTCGTCGTATGTGA 19 4

19 CGGGCGCTCAGGGCAGCAGAGAGCAGAGGACGCGACAGGGATGTGTGATGCTGGCTCAGGCCTGGGCATCCGAGTT 11
3 GATG 4

19 3 CGGGCGtTtAGGGtAGtAGAGAGtAGAGGACGCGAtAGGGATGTGTGATGtTGGtTtAGGttTGGGtATtCGAGTTGATG 11 4

19 3 AGGACGCGATAGGGAT 11 4

19 3 AGGATGTGATAGGGAT 11 4

11 AGCGCTGGCTGGCGTTCAGCGCCTGGAAACTGCACACGCGCGCTGGGCCGCTTGCGCATTTAAGGAAGTGCAGCCTC
3 ATC 34

11 3 AGCGtTGGtTGGCGTTtAGCGttTGGAAAtTGtAtACGCGCGtTGGGtCGtTTGCGtATTTAAGGAAGTGtAGttTtATC 34

11 3 GGTTGTTTGTGTATTTAA 34

11 3 ATTGTATACGCGCGTT GTCGTTTGCGTATTTA 34

11 3 TTGTATATGTGTGTTGG

33 GGAAGAGGCCTGCCAGTGCTGAGCCAGGAGGGA -46
33 **TTAGTGTTGAGTTAGGAGGGA** -46
33 GGAAGAGGttTGttAGTGtTGAGttAGGAGGGA -46

Figure 30: Amplificate of SEQ ID NO: 28

1 GAGAGGGAGGGCCAAGGTCCGGGCGCCGAGAGGAGGGGCCCGGGGGGCCGGCTGGAGGGGGCGAGGCGCTAGGCG GCTTT 80

1 **GAGAGGGAGGGTTAAGGTT** 80

1 GAGAGGGAGGGttAAGGTtCGGGCGtCGAGAGGAGGGGttCGGGGGGtCGGtTGGAGGGGGCGAGGCGtTAGGCGGtTTT 80

81 GCTTCCCGTCCCCGTCAATTGGCCGCTAGCTCTGTTTTGACTGCCTGTACAAAGCGAGTAGACGGCGGGGGATGGGG CGA 160

81 GtTTttCGTtttCGTtAATTGGtCGtTAGtTtTGTTTTGAtTGttTGTAtAAAGCGAGTAGACGGCGGGGGATGGGGCGA 160

81 GTTAATTGGTCGTTAGTTT TATAAAGTGAGTAGATGG A 160

81 AATTGGTCGTTAGTTTT TAAAGCGAGTAGACGG 160

161 TTAGGGACCGGATTTGGGAAAGGAGGCAGCAGC 240

161 **ATTTGGGAAAGGAGGTAGTAGT** 240

161 TTAGGGAtCGGATTTGGGAAAGGAGGtAGtAGt 240

161 TTAGGGATCGGATTTG 240

161 TTAGGGATCGGATTTG

Figure 31: Amplificate of SEQ ID NO: 29

35 GTGGAAAAAGGAGAGCAAACTGCTCAGCGCGTCTCAGCTCAGTGCCGAGGAGGAGGAAGAAAAACAGGCCGAGTG 27
6 AAGGT 7

35 6 **GTGGAAAAAGGAGAGTAAATTG** 27 7

35 6 GTGGAAAAAGGAGAGtAAAtTGtTtAGCGCGTtTtAGtTtAGTGtCGAGGAGGAGGAAGAAAAAtAGGtCGAGTGAAGGT 27 7

27 GCTGGAAAGGGAGGGAGGACGCGAGGGGAAAGGCCTGTGGGGAGCCGAGGGCGTCAGAGAGACCCGGGAAGGAA 19
6 GGCTCT 7

27 6 GtTGGAAAGGGAGGGAGGACGCGAGGGGAAAGGttTGTGGGGAGtCGAGGGCGTtAGAGAGAttCGGGAAGGAAGGtTtT 19 7

27 6 AGTCGAGGGCGTTAGA 19 7

27 6 AGTTGAGGGTGTTAGA 19 7

19 CGGGTGGGGGAGCCAGGAGACCTGCTCTCCGGCGCAGACAGGCGGGGCCCAGCGCTCTCCTGGACGCCCCCGCCCG 11
6 CACA 7

19 6 CGGGTGGGGGAGttAGGAGAttTGtTtTtCGGCGtAGAtAGGCGGGGtttAGCGtTtTttTGGACGttttCGttCGtAtA 11 7

19 6 TTTTTCGGCGTAGATA ATA 11 7

19 6 TGTTTTTTGGTGTAGAT TATA 11 7

11 GCTCCCGGCGGGTGCTCTGAGGCCTCACTACTCGAGCCCACCCAGCATCCCGCGCGCCCTTCCTTCCCGAGGAACTCG 37
6 CC

11 6 GtTttCGGCGGGTGtTtTGAGGttTtAtTAtTCGAGtttAttAGtATttCGCGCGtttTTttTTttCGAGGAAtTCGtt 37

11 6 GTTTTCGGCGGGT TTTTTTGAGGAATTTGTT 37

11 6 GTTTTTGGTGGGT TTTTCGAGGAATTCGT

36 TCAGCCTGATCAGGCTTCCTGGTGAGAACTGAGGAG -43
36 **TTTTGGTGAGAATTGAGGAG** -43
36 TtAGttTGATtAGGtTTttTGGTGAGAAtTGAGGAG -43

Figure 32: Amplificate of SEQ ID NO: 30

---

420 AGGGAGGCCTGGTGCATTCCTCCATGGGGGTGGGGAATGCGGAGGCTGGGCCACCGGAGGTGGTGGCGGTGGCAGGGGCG 341

420 **AGGGAGGTTTGGTGTATTTT** 341

420 AGGGAGGttTGGTGtATTttTttATGGGGGTGGGGAATGCGGAGGtTGGGttAtCGGAGGTGGTGGCGGTGGtAGGGGCG 341

---

340 GCAGGAGACGCCACGTCAGGGTCACTGCCCCACTCGGTGCACAGCAGGGGGCCGGCAGTTCACCGGATCACCGCGGGGGT 261

340 GtAGGAGACGttACGTtAGGGTtAtTGtttAtTCGGTGtAtAGtAGGGGGtCGGtAGTTtAtCGGATtAtCGCGGGGGT 261

340 AGATGTTATGTTAGGGT ATTGGATTATTGTGGGG 261

340 TAGGAGACGTTACGTT TATCGGATTATCGCGG 261

340 GTCGGTAGTTTATCGGAT 261

340 GTTGGTAGTTTATTGGAT 261

---

260 GGGGCGGAGGACGGAAGTTGGTGAGGGGGGTGGTCTACAGGGCACCTCCCGTCCCAGGGCTCTAACCTAATTCGAAAGGG 181

260 GGGGCGGAGGACGGAAGTTGGTGAGGGGGGTGGTtTAtAGGGtAttTttCGTtttAGGGtTtTAAttTAATTCGAAAGGG 181

260 TTAATTTGAAAGGG 181

260 TTTAATTCGAAAGGG 181

---

180 AAAGGAGTGCTGCAGCCGGGAGGCCAGGCCGACTTTGGGTCCTGCCCTCGCAGAAGCCCCCACGCCTGGTGCAGACCAAT 101

180 AAAGGAGTGtTGtAGtCGGGAGGttAGGtCGAtTTTGGGTttTGttTCGtAGAAGttttACGttTGGTGtAGAttAAT 101

180 AAAG 101

180 AA

---

100 GACTCGCTTCCTGCCCTTACACCGATCACACATGACCTCGGTCACAGGGGTGCCACATCACAGGAGTGCCCCTCACTCCT 21

100 **TT** 21

100 GAtTCGtTTttTGttTTAtAtCGATtAtAtATGAttTCGGTtAtAGGGGTGttAtATtAtAGGAGTGtttTtAtTtT 21

---

20 GCCTGCAGGAAGCAGGGCCC -59

20 **GTTTGTAGGAAGTAGGGTTT** -59

20 GttTGtAGGAAGtAGGGttt -59

---

Figure 33: Amplificate of SEQ ID NO: 31

| | | |
|---|---|---|
| 1 | AAGGCACTACTCCTGGGGTTCCCCGAAGCGGATCCTGGGACTGGGACCTGGGGACGCGGTGAGAGGCAGCCCTGAAGCTG | 80 |
| 1 | **AAGGTATTATTTTTGGGGTTTT** | 80 |
| 1 | AAGGtAtTAtTttTGGGGTTtttCGAAGCGGATttTGGGAtTGGGAttTGGGGACGCGGTGAGAGGtAGtttTGAAGtTG | 80 |
| 1 | TTTTTCGAAGCGGATT | 80 |
| 1 | TTTGAAGTGGATTTTGG | 80 |
| 81 | AGTCCAGAGGTCTGGGGCGTCATTGTCCCAGGGCGGACCCAGCCCCAGACCCTACAACTAAACCCAAGGCCAGCCGAGAA | 160 |
| 81 | AGTttAGAGGTtTGGGGCGTtATTGTtttAGGGCGGAtttAGttttAGAtttTAtAAtTAAAtttAAGGttAGtCGAGAA | 160 |
| 81 | AGGTTAGTCGAGAA | 160 |
| 81 | AGGTTAGTTGAGAA | 160 |
| 161 | GCACTACCACTGGCGGGCTGAGCTGAGCTCTCATTCTTCTTAGGACTCTGGACCTTTGGAGGAAATGAAAGGGACCCAGT | 240 |
| 161 | GtAtTAttAtTGGCGGGtTGAGtTGAGtTtTtATTtTTtTTAGGAtTtTGGAttTTTGGAGGAAATGAAAGGGAtttAGT | 240 |
| 161 | GT | 240 |
| 161 | GTA | 240 |
| 241 | TGGAAATATAGTCTTTCTTCGCGCCCCCTACATCTCTCTCCCCCGGCGGAAACGCGGCCCAAACTGTTACGAAGTGGAAG | 320 |
| 241 | **AAGTGGAAG** | 320 |
| 241 | TGGAAATATAGTtTTTtTTCGCGttttTAtATtTtTtTtttCGGCGGAAACGCGGtttAAAtTGTTACGAAGTGGAAG | 320 |
| 241 | GGAAATGTGGTTTAAATT | 320 |
| 241 | GAAACGCGGTTTAAAT | 320 |
| 241 | ATTGTTACGAAGTGGA | 320 |
| 241 | TTGTTATGAAGTGGAAG | |
| 321 | AGGCTGGATAGCT | 400 |
| 321 | **AGGTTGGATAGTT** | 400 |
| 321 | AGGtTGGATAGtT | 400 |

Figure 34: Amplificate of SEQ ID NO: 32a

1 GGGTGCTGGGGAGCCCCATGCGCCAGGAGGTGGATAATTTAGGGGGCAAGGGGCTCGCACAAGGGCGAAGATGCGC TCTC 80

1 **GGGTGTTGGGGAGTTTTA** 80

1 GGGTGtTGGGGAGttttATGCGttAGGAGGTGGATAATTTAGGGGGtAAGGGGtTCGtAtAAGGGCGAAGATGCGtTtTt 80

---

81 CTCCCGGCACCGACCCACGCGTTTAAAGGCCTCTGCGGCAACGTTTAGGGACGCGAACTAGGTGCCGGGGGTTGGGG AGG 160

81 tTttCGGtAtCGAttACGCGTTTAAAGGttTtTGCGGtAACGTTTAGGGACGCGAAtTAGGTGtCGGGGGTTGGGGAGG 160

81 TTTGCGGTAACGTTTA 160

81 TTGTGGTAATGTTTAGG 160

81 TTAGGGACGCGAATTA 160

81 AGGGATGTGAATTAGG 160

---

161 AGGTGGGACTGGAAGCGAGAGCAGTCTGCGCCCAAGACCGCGCAAGAGGTGGCCTTTTGTTCGCTGGCTCCAGCAC ATCC 240

161 AGGTGGGAtTGGAAGCGAGAGtAGTtTGCGtttAAGAtCGCGtAAGAGGTGGttTTTTGTTCGtTGGtTttAGtAtATtt 240

161 TGCGTTTAAGATCGCGT ATATTT 240

161 - TGTGTTTAAGATTGTGT ATTT 240

---

241 CGGCTCTCGAAAAAACACTACCTCTGTCCCCTGCCACCCTCTGCATTTTAGAAAATCCTCTTCAGGGCAGGAAAGGA CAG 320

241 **GGAAAGGATAG** 320

241 CGGtTtTCGAAAAAAtAtTAttTtTGTtttTGttAttTtTGtATTTTAGAAAATttTtTTtAGGGtAGGAAAGGAtAG 320

241 TGGTTTTTGAAAA 320

241 CGGTTTTCGAAA

---

321 TGAGTCACTGCAAG 400

321 **TGAGTTATTGTAAG** 400

321 TGAGTtAtTGtAAG 400

Figure 35: Amplificate of SEQ ID NO: 32b

31 AGAGTGTGGTACCAGATCTGGTTCCATTTTTACGAGAGCCAGGAACCACGCACGTCTGGAGGGAGAGCGCAGCTGA 23
7 CCCG 8

31 **TCCTTTCC** 23
7 8

31 AGAGTGTGGTAttAGATtTGGTTttATTTTTACGAGAGttAGGAAttACGtACGTtTGGAGGGAGAGCGtAGtTGAttCG 23
7 8

31 AGGAATTACGTACGTT AGAGCGTAGTTGATTCG 23
7 8

31 AGAGTGtAGTTGATTTG 23
7 8

31 TATGTATGTTTGGAGGG 23
7 8

23 AGGGCGCGACCGTGTTCTGGGAGTGTTTGAATCCCCGGCCTTTGGTGAAAATTTCCTGTGTCCGCAAGGCCCAGAGG 15
7 AGA 8

23 AGGGCGCGAtCGTGTTtTGGGAGTGTTTGAATttCGGttTTTGGTGAAAATTTttTGTGTtCGtAAGGttAGAGGAGA 15
7 8

23 A 15
7 8

23 A 15
7 8

15 TCGTGTGATGTCCGGGGGGTGCTGCGTGCGGCCGGCTGGGTAGCGGCCGCGGACCACCACTAAACCGGGGCATGTTT 78
7 GCC

15 TCGTGTGATGTtCGGGGGGTGtTGCGTGCGGtCGGtTGGGTAGCGGtCGCGGAttAttAtTAAAtCGGGGtATGTTTGtC 78
7

15 TAGCGGTCGCGGATTA 78
7

15 GTAGTGGTTGTGGATT 78
7

77 GTCCTTCCTGGTGCTCTGCCCCTCTGTAACGCGGGGAAGTAGGAGGGCGCGGGGAGAAATTACTAAAGGATGCTTGC -2

77 **TAAAACTCCCCAACACCC** -2

77 GTttTTttTGGTGtTtTGtttTtTGTAACGCGGGGAAGTAGGAGGGCGCGGGGAGAAATTAtTAAAGGATGtTTGt -2

77 TTTGTAACGCGGGGAA -2

77 TTTTGTAATGTGGGGA

Figure 36: Amplificate of SEQ ID NO: 33

1 TCAGCTGAGAAAGTGGGGGCCCTAAAAAGGGCCTTTTGTTGATAGAAAGGGACGCTCAACCACCGAAACCGTAGAG GGTG 80

1 **TTAGTTGAGAAAGTGGGGGT** 80

1 TtAGtTGAGAAAGTGGGGGtttTAAAAAGGGttTTTTGTTGATAGAAAGGGACGtTtAAttAtCGAAAtCGTAGAGGGTG 80

1 ATCGAAATCGTAGAGG 80

1 ATTGAAATTGTAGAGGG 80

---

81 CGGCCCTGGCGCTTGAGCGCGTAGACCACATCCATGGCGGTGACCGTCTTGCGCTTGGCGTGCTCTGTATAGGTCAC GGC 160

81 CGGtttTGGCGtTTGAGCGCGTAGAttAtATttATGGCGGTGAtCGTtTTGCGtTTGGCGTGtTtTGTATAGGTtACGGC 160

81 TTTATGGTGGTGATTGT TTACGGC 160

81 TATGGCGGTGATCGTT TTATGGT 160

---

161 GTCCCGGATCACGTTCTCCAGGAACACCTTCAGCACCCCGCGAGTCTCCTCGTAGATGAGGCCGGAGATGCGCTTCA CGC 240

161 GTttCGGATtACGTTtTttAGGAAtAttTTtAGtAtttCGCGAGTtTttTCGTAGATGAGGtCGGAGATGCGtTTtACGt 240

161 GTTTCGGAT AGATGTGTTTTATGT 240

161 GTTTTGGATT ATGCGTTTTACGT 240

---

241 CGCCGCGGCGAGCAAGGCGCCGGATGGCCGGCTTGGTGATGCCCTGGATATTGTCGCGCAGTACTTTACGGTGGCGC TTA 320

241 CGtCGCGGCGAGtAAGGCGtCGGATGGtCGGtTTGGTGATGtttTGGATATTGTCGCGtAGTAtTTTACGGTGGCGtTTA 320

241 TGT GGATATTGTTGTGTAGT 320

241 CGT TATTGTCGCGTAGTAT

---

321 GCGCCGCCTTTGCCAAGACCCTTCCCGCCTTTGCCGCGGCCAGACATGACGAGCAAGAGGAGTCTCACCCAACGCTT TGT 400

321 GCGtCGttTTTGttAAGAttTTttCGttTTTGtCGCGGttAGAtATGACGAGtAAGAGGAGTtTtAtttAACGtTTTGT 400

---

401 GAGGACTCTGGCCTGAGGCAG 480

401 **GAGGATTTTGGTTTGAGGTAG** 480

401 GAGGAtTtTGGttTGAGGtAG 480

---

Figure 37: Amplificate of SEQ ID NO: 34

---

49 GTGAGAGTGGGTGCTGAAACCCCATCCCGGGCCCCAAAGGACACTGGCCGATTCTCGCGCAGAAAGCCACAGGCCT 41
8 TGTC 9

49 **GTGAGAGTGGGTGTTGAAAT** 41
8 9

49 GTGAGAGTGGGTGtTGAAAttttATttCGGGttttAAAGGAtAtTGGtCGATTtTCGCGtAGAAAGttAtAGGttTTGTC 41
8 9

49 GGTCGATTTTCGCGTA 41
8 9

49 TGGTTGATTTTTGTGTA 41
8 9

---

41 GCACTCCCTCCCTGTGGACGGGTGTGGATGTGTCATCATCTGACTGCTCCCTGGGAGGGCTGGGAGTTTCACCCGCTC 33
8 CT 9

41 GtAtTttTttTGTGGACGGGTGTGGATGTGTtATtATtTGAtTGtTttTGGGAGGGtTGGGAGTTTtAttCGtTtT 33
8 9

---

33 GCCTTGCCAAGGCACTGACCGGCTGGAGGCGCGGAATCCGGCCTGGTTGTTGGCGGTCCGATCTGGGGACCGGCTCT 25
8 TCG 9

33 GttTTGttAAGGtAtTGAtCGGtTGGAGGCGCGGAATtCGGttTGGTTGTTGGCGGTtCGATtTGGGGAtCGGtTtTTCG 25
8 9

33 TATTGATTGGTTGGAGG ATTGGTTTTTTG 25
8 9

33 TATTGATCGGTTGGAG ATCGGTTTTTCG 25
8 9

---

25 AGGTCTGTCCTGTTCAGCCCCGCCTGGGGCATAGGGTCTTCTAGGTCTCGGGTTGTGGTGCGGGGTTGCCAGATAAAT 17
8 GC 9

25 AGGTtTGTttTGTTtAGttCGttTGGGGtATAGGGTtTTtTAGGTtTCGGGTTGTGGTGCGGGGTTGttAGATAAATGt 17
8 9

25 AGGTT 17
8 9

25 AGGT
8

---

17 AGTAATGGCAGCAAGGAGAACCGGCATTTCCACTGGGCCTTTTACGTCTACCATGCCTGTTTTCCCATGTGGCCCTCC 99
8 GA

17 AGTAATGGtAGtAAGGAGAAtCGGtATTTttAtTGGGttTTTTACGTtTAttATGttTGTTTTtttATGTGGtttTtCGA 99
8

---

98 CTCCTGTGAGGGAGGTGAGGCTGCACTATAGGTGAGGATGTGGGGGATCAAGGCAGGGGCATCCCCCACCCACCCT 19
CAGC

98 **TTAGT** 19

98 tTtTGTGAGGGAGGTGAGGtTGtAtTATAGGTGAGGATGTGGGGGATtAAGGtAGGGGtATttttAttAttTtAGt 19

---

18 CATGCAGCTGACTGGGGC -61

18 **TATGTAGTTGATTGGGGT** -61

18 tATGtAGtTGAtTGGGGt -61

---

Figure 38: Amplificate of SEQ ID NO: 35

1 ACCTCAGGTGTAAGCCCAAGGCTGCTGCCTGCAAAGGCAATGCCGTGGAGACTGGGTTCCACAGCGACCTGGGTTTT TAA 80

1 **ATTTTAGGTGTAAGTTTAAGGTTGT** 80

1 AttTtAGGTGTAAGtttAAGGtTGtTGttTGtAAAGGtAATGtCGTGGAGAtTGGGTTttAtAGCGAttTGGGTTTTTAA 80

---

81 GTCACCGAAAGCTACAGGGCAGGGTCACAAACACTCTCCCGCCTTCTCTGCAGAACCGTGCGGCTGACAGGAGAGC GTTG 160

81 GTtAtCGAAAGtTAtAGGGtAGGGTtAtAAAtAtTtTttCGttTTtTtTGtAGAAtCGTGCGGtTGAtAGGAGAGCGTTG 160

81 TGTAGAATTGTGTGGT 160

81 AATCGTGCGGTTGATA 160

---

161 CGCAGAAAATTCGAGGCCGGGCGCTGGAGGAGTCTCCGCGGCCCGGAGAAGGCACAGAGGCGCCCCTGAGAGGCG CAGCT 240

161 CGtAGAAAATTCGAGGtCGGGCGtTGGAGGAGTtTtCGCGGttCGGAGAAGGtAtAGAGGCGttttTGAGAGGCGtAGtT 240

161 AAAATTCGAGGTCGGG TTTTCGCGGTTCGGAG 240

161 AAAATTTGAGGTTGGG TTTGTGGTTTGGAGAA 240

---

241 GGAACAGGCGATGCACGGGTTCGGATCTGGCCGGCCAACCGAGCCCAGCGGTCGCGAAAACCAGAGTCGCCACAGA GGTT 320

241 GGAAtAGGCGATGtACGGGTTCGGATtTGGtCGGttAAtCGAGttAGCGGTCGCGAAAAttAGAGTCGttAtAGAGGTT 320

241 AATAGGCGATGTACGG TTTGGTTGGTTAATTGA TAGCGGTCGCGAAAAT 320

241 TAGGTGATGTATGGGT TTGGTCGGTTAATCGA 320

241 AGTTTAGTGGTTGTGA

---

321 GAGCACGATTCCATTTCTGGGGATCGGGTCCGGTGGGGAGCCACTGTCCCTAACGCCTCCAGAGACTTTAAGTAGGA CAA 400

321 GAGtACGATTttATTTtTGGGGATCGGGTtCGGTGGGGAGttAtTGTtttTAACGttTtAGAGAtTTTAAGTAGGAtAA 400

---

401 TATAATGCTGGTAGGAGCAAGGTGCAAGGAATTTAGCGGCAGAAGCCTTTCGGGGGGGTGGGGAAAGGCAGAT 480

401 **GGGTGGGGAAAGGTAGAT** 480

401 TATAATGtTGGTAGGAGtAAGGTGtAAGGAATTTAGCGGtAGAAGttTTTCGGGGGGGTGGGGAAAGGtAGAT 480

---

Figure 39: Amplificate of SEQ ID NO: 13

---

488 TGTCCTAGGTCTGATGGACCAGACCCCAGCGGGCCTGAACTTTGTCGGAAAGCGGAGCCGCCAGGCATTTGGCAGCTGCC 409

488 **TGTTTTAGGTTTGATGGATTAGA** 409

488 TGTttTAGGTtTGATGGAttAGAtttAGCGGGttTGAAtTTTGTCGGAAAGCGGAGtCGttAGGtATTTGGtAGtTGtt 409

488 TTTGTCGGAAAGCGGA 409

488 TTTGTTGGAAAGTGGA 409

---

408 AGAGGCGCACCAGGCCGGGTCTCAACCTGGGAGGTCAGCGGGCCAAAGCGGCCTGGGGAGGCAGTGGCCCCACCTGGACG 329

408 AGAGGCGtAttAGGtCGGGTtTtAAttTGGGAGGTtAGCGGGttAAAGCGGttTGGGGAGGtAGTGGttttAttTGGACG 329

408 TATTTGGACG 329

408 TATTTGGATG 329

---

328 CCGGGCCGTCGGGCGTCGGAGCCCCGCTCTGCCTCCACCAAGCTGTCCCGCTGCGCCCTACGCCGCCACCAGGCAGGAGG 249

328 tCGGGtCGTCGGGCGTCGGAGtttCGtTtTGttTtAttAAGtTGTttCGtTGCGtttTACGtCGttAttAGGtAGGAGG 249

328 TCGGGT TTACGTCGTTATTAGGT 249

328 TTGGGT TTTTATGTTGTTATTAGGT 249

---

248 AGCCGCTACCAGTACCTCAGAGTGGGACTCTCCATCCTCAAGTCTGCGCTCCAGAAAAGTGTAGCAAGCCTCCCACGCGG 169

248 AGtCGtTAttAGTAttTtAGAGTGGGAtTtTtATttTtAAGTtTGCGtTttAGAAAAGTGTAGtAAGttTttACGCGG 169

248 TTTATGTGG 169

248 TTTACGCGG 169

---

168 GGCTTCAGACACCTAAAAGTCGCCACTCCCCCAAGAGTAACCTCCACCCCCACCCCCACCCCCAACTTCTCTTTCCTCCT 89

168 GGtTTtAGAtAttTAAAAGTCGttAtTttttAAGAGTAAttTttAttttAttttAttttAAtTTtTtTTTttTttT 89

168 GGTTTTAG 89

168 GGTTTTA

---

88 TTTCTTCAGCAATGTGGACCAACAGTTACTTTTCCCTCTCTTCCCTTCCCCTCCGAAGTCCCCGGGGCAAAGTGGGACTT 9

88 **AGTGGGATTT** 9

88 TTTtTTtAGtAATGTGGAttAAtAGTTAtTTTTttTtTtTTttTTtttTtCGAAGTttCGGGGtAAAGTGGGAtTT 9

---

8 TGGGGAGC -71

8 **TGGGGAGT** -71

8 TGGGGAGt -71

---

Figure 40: Amplificate of SEQ ID NO: 36

1 GTTGAGAGGTAAGGCATGAAGGGCGCAATATCCAATATGAGCAACGCGTGTGATGCATCTGGTCAAAATGCATACAGAGG 80

1 **GTTGAGAGGTAAGGTATGAAGG** 80

1 GTTGAGAGGTAAGGtATGAAGGGCGtAATATttAATATGAGtAACGCGTGTGATGtATtTGGTtAAAATGtATAtAGAGG 80

1 TATGAGTAATGTGTGTG 80

1 GAGTAACGCGTGTGAT 80

---

81 ACTTGTCTCTGTCCCTAGATAGAAGTCCTCCGTCCTGCAGTCATGAGGGTCAATTGCTGAGGCTTCACAGTTCCCTTCTC 160

81 AtTTGTtTtTGTttTAGATAGAAGTttTtCGTttTGtAGTtATGAGGGTtAATTGtTGAGGtTTtAtAGTTttTTtTt 160

---

161 TCTTACACTCGGACCGTCACGCTCCTCACCTACTACCCCGATGCAGAGGTAGACTCAGGATCCCTGCACTTGTCAAGGAT 240

161 TtTTAtAtTCGGAtCGTtACGtTttTtAttTAtTAttCGATGtAGAGGTAGAtTtAGGATttTGtAtTTGTtAAGGAT 240

161 ATTCGGATCGTTACGT 240

161 ATTTGGATTGTTATGTTT 240

---

241 TCCTCGGCAAGCTCACGGGGCGGGAGTGGCCACAAGACGGAGCTCGCCTGGTCCTGGCCTTCCCGGCCTATACAAGCCTG 320

241 TttTCGGtAAGtTtACGGGGCGGGAGTGGttAtAAGACGGAGtTCGttTGGTttTGGttTTttCGGttTATAtAAGttTG 320

241 ATAAGACGGAGTTCGT 320

241 AAGATGGAGTTTGTTTG 320

---

321 CCCCCTTCCCAATTCCCAATCTCCACAGCCTTCCATCCTCCCACTTTCGATTCACCTTGCGCCACCGACGCCCCTGGCCT 400

321 tttTTtttAATTtttAATtTttAtAGttTTttATttTttAtTTTCGATTtAttTTGCGttAtCGACGttttTGGttT 400

321 ATTTTGTGTTATTGATGT 400

321 TTGCGTTATCGACGTT

---

401 TCGTTTGCAGCAAGTTTACCCCCACCATTACCTCTCGCATAAAAGCCTGCATTTACCAGGTCAAAGAGGGGAACCAA 480

401 **TTATTAGGTTAAAGAGGGGAATTAA** 480

401 TCGTTTGtAGtAAGTTTAttttAttATTAttTtTCGtATAAAAGttTGtATTTAttAGGTtAAAGAGGGGAAttAA 480

Figure 41: Amplificate of SEQ ID NO: 37

1 GTGTCAGGGCTCAGGGGCCCATCGCCCTTGGCCTGGGGCCCCTCCGTGCCGTCGGGATTTGTGGGACGCGCTGAAGG AAG 80

1 **GTGTTAGGGTTTAGGGGTTT** 80

1 GTGTtAGGGtTtAGGGGtttATCGtttTTGGttTGGGGttttTtCGTGtCGTCGGGATTTGTGGGACGCGtTGAAGGAAG 80

1 TGTGGGATGTGTTGAA 80

1 GTGGGACGCGTTGAAG 80

81 AGGCGGGCGGCTCCGACAGAAAACAGCCAGAGCGCACCACTCACCTGAGTGCCAGGTAAACACCTGGGCGCGACAG GGAC 160

81 AGGCGGGCGGtTtCGAtAGAAAAtAGttAGAGCGtAttAtTtAttTGAGTGttAGGTAAAtAttTGGGCGCGAtAGGGAt 160

81 GGGTGGTTTTGATAGA AGTTAGAGCGTATTATTT TATTTGGGCGCGATAG 160

81 GGCGGTTTCGATAGAA ATTTGGGTGTGATAGG 160

81 [illegible]

161 AGGAAACAAGGGTAGGGTGCGGAGGCTGGGGAGGAAGAGGTTGGAAAGG 240

161 **AGGAAGAGGTTGGAAAGG** 240

161 AGGAAAtAAGGGTAGGGTGCGGAGGtTGGGGAGGAAGAGGTTGGAAAGG 240

Figure 42: Amplificate of SEQ ID NO: 1

50 TCTGGAGGGATAGAATGTGATCCTCGAGCAGAAACCATCGTGTTTCTGGGGGCTCTTCGGGCGCCGTGGGGCGACGC 42
4 GGT 5

50 4 **TTTGGAGGGATAGAATGTGA** 42 5

50 4 TtTGGAGGGATAGAATGTGATttTCGAGtAGAAAttATCGTGTTTtTGGGGGtTtTTCGGGCGtCGTGGGGCGACGCGGT 42 5

42 GCAGCGTCTAGGGCCGCCGCAGGCCGGGGGCAGGGCTCCCAGCGGTTCCCCCGCGGCCAGCGGCCACGCAGGAAAA 34
4 ACCC 5

42 4 GtAGCGTtTAGGGtCGtCGtAGGtCGGGGGtAGGGtTttAGCGGTTtttCGCGGttAGCGGttACGtAGGAAAAAttC 34 5

42 4 TTAGGGTCGTCGTAGGT AGTCGGTTACGTAGGAAA 34 5

42 4 TTAGGGTTGTTGTAGGT TAGCGGTTACGTAGGA 34 5

34 GCTTCCTCGCCCCTGCATCTGCTGACAAGCCGCCCGAGGTGTCTGCGCTGAGCGTGGCCACACCGATGCAGCTTTCTA 26
4 GG 5

34 4 GtTTttTCGtttTGtATtTGtTGAtAAGtCGttCGAGGTGTtTGCGtTGAGCGTGGttAtAtCGATGtAGtTTTtTAGG 26 5

34 4 AAGTTGTTTGAGGTGT 26 5

34 4 ATAAGTCGTTCGAGGT 26 5

26 AAATGGCCCGGGAGGGGGAGGGGGCGAGTGAGGGATTAGGTCCGGCTCCCCCTCGCTCCCCCTGCGCAAACACCAC 18
4 CACC 5

26 4 AAATGGttCGGGAGGGGGAGGGGGCGAGTGAGGGATTAGGTtCGGtTttttTCGtTttttTGCGtAAAtAttAttAtt 18 5

18 CCTTCCTTTCCCCGGAGGCATGAGGCCTCCACGCTCGGTCCTTCGCCCGCATCCTCGCCGACCCCTCCGGTTTCGCGC 10
4 CT 5

18 4 ttTTttTTTttCGGAGGtATGAGGttTtACGtTCGGTttTTCGttCGtATttTCGtCGAtttTtCGGTTTCGCGttT 10 5

10 CACTCGCCAGGCTTTCTCCCTGGGAGCCGACTGCGGTGAAGTCGCCGCCAGCACGGTCGGCCCTGACACGTGCTCTA 25
4 ACG

10 4 tAtTCGttAGGtTTTtTttTGGGAGtCGAtTGCGGTGAAGTCGtCGttAGtACGGTCGGtttTGAtACGTGtTtTAACG 25

10 4 TTAGTACGGTCGGTTT TACGTGTTTTAACG 25

10 4 TGATATGTGTTTTAATG 25

10 4 GTTAGTATGGTTGGTTT 25

24 CATGCTTGAGTCAAGGCTGTCTTT -55

24 **ATGTTTGAGTTAAGGTTGTTTTT** -55

24 tATGtTTGAGTtAAGGtTGTtTTT -55

24 TAT -55

24 TA

Figure 43: Amplificate of SEQ ID NO: 2

---

1 AGATCATCAGTCCTCACCTGAGGGACCTTCCGCGGCATCTATGCGGGCATGGTTACTGCCTCTGGTGCCCCCCGCAGCCG 80

1 **AGATTATTAGTTTTTATTTGAGGGATT** 80

1 AGATtATtAGTttTtAttTGAGGGAttTTtCGCGGtATtTATGCGGGtATGGTTAtTGttTtTGGTGttttCGtAGtCG 80

1 TAGTCG 80

1 TAGTTG 80

---

81 CGCGCAGGTACCGTGCGACATCGCGATGGCCCAGCTCCTCAGCCAGGTCCACGGGCAGACGGCCCCAGGCATCGCGCACG 160

81 CGCGtAGGTAtCGTGCGAtATCGCGATGGttAGtTtTtAGttAGGTtACGGGtAGACGGttttAGGtATCGCGtACG 160

81 CGCGTAGGTA TTAGGTATTGTGTATG 160

81 TGTGTAGGTAT TAGGTATCGCGTACG 160

81 GTAGGTATTGTGTGATAT 160

81 TAGGTATCGTGCGATA 160

---

161 TCCAGCCGCGCCCCGGCCCGGTGCAGCACCACCAGCGTGTCCAGGAAGCCCTCCCGGGCAGCGTCGTGCACGGGTCGGGT 240

161 TttAGtCGCGtttCGGttCGGTGtAGtAttAttAGCGTGTttAGGAAGtttTttCGGGtAGCGTCGTGtACGGGTCGGGT 240

161 TT 240

161 T 240

---

241 GAGAGTGGCGGGGTCGGCGCAGTTGGGCTCCGCGCCGTGGAGCAGCAGCAGCTCCGCCACTCGGGCGCTGCCCATCATCA 320

241 GAGAGTGGCGGGGTCGGCGtAGTTGGGtTtCGCGtCGTGGAGtAGtAGtAGtTtCGttAtTCGGGCGtTGtttATtATtA 320

241 TTTTGTGTTGTGGAGTA 320

241 TTCGCGTCGTGGAGTA

---

321 TGACCTGCCAGAGAGAACAGAATGGTCAGAGCCAGGGTGG 400

321 **GAATGGTTAGAGTTAGGGTGG** 400

321 TGAttTGttAGAGAGAAtAGAATGGTtAGAGttAGGGTGG 400

---

Figure 44: Amplificate of SEQ ID NO: 3

1 CCCTTGCTTGGGTGTGTCCTTCGCTCGCTCCCTCCCTCCGTCTTAGGTCACTGTTTTCAACCTCGAATAAAAACTGCAGC 80

1 **TTTTTGTTTGGGTGTGTTTT** 80

1 ttTTGtTTGGGTGTGTttTTCGtTCGtTttTttTtCGTtTTAGGTtAtTGTTTTtAAttTCGAATAAAAAtTGtAGt 80

81 CAACTTCCGAGGCAGCCTCATTGCCCAGCGGACCCCAGCCTCTGCCAGGTTCGGTCCGCCATCCTCGTCCCGTCCTCCGC 160

81 tAAtTTtCGAGGtAGttTtATTGttAGCGGAtttAGttTtTGttAGGTTCGGTtCGttATttTCGTttCGTttTtCGt 160

81 <u>TAGGTTCGGTTCGTTAT</u> 160

81 <u>TAGGTTTGGTTTGTTATT</u> 160

161 CGGCCCCTGCCCCGCGCCCAGGGATCCTCCAGCTCCTTTCGCCCGCGCCCTCCGTTCGCTCCGGACACCATGGACAAGTT 240

161 CGGtttTGttCGCGttAGGGATttTttAGtTtTTTCGttCGCGttTtCGTTCGtTtCGGAtAttATGGAtAAGTT 240

161 <u>GTTTCGCGTTTAGGGA</u> <u>GTTCGTTTCGGATATTA</u> 240

161 <u>GTTTTGTGTTTAGGGAT</u> <u>TTTGTTTTGGATATTATGG</u> 240

241 TTGGTGGCACGCAGCCTGGGGACTCTGCCTCGTGCCGCTGAGCCTGGCGCAGATCGGTGAGTGCCCGCCGCAGCCTGGGC 320

241 TTGGTGGtACGtAGttTGGGGAtTtTGttTCGTGtCGtTGAGttTGGCGtAGATCGGTGAGTGttCGtCGtAGttTGGGt 320

241 <u>**TTT**GGCGTAGATCGGT</u> 320

241 <u>TTTGGTGTAGATTGGT</u>

321 AGCAAGATGGGTGCGGGGTGCTCAGCGCGGACCCGGCGGCAGCCCCTCCGGCTGAGTCGGCCCTGGGGGACTGGAGTCAA 400

321 **GGGGGATTGGAGTTAA** 400

321 AGtAAGATGGGTGCGGGGTGtTtAGCGCGGAttCGGCGGtAGtttTtCGGtTGAGTCGGtttTGGGGGAtTGGAGTtAA 400

401 GTG 480

401 **GTG** 480

401 GTG 480

Figure 45: Amplificate of SEQ ID NO: 4

---

44 GAAGGGATGAATGAATAAAAGTACTTGTCTGATGGCAGCAGAGACCCCGAGCAAACGGTGGAGGCTACACTGTCTG 36
6 GCAT 7

44 **GAAGGGATGAATGAATAAAAGT** 36
6 7

44 GAAGGGATGAATGAATAAAAGTAtTTGTtTGATGGtAGtAGAGAtttCGAGtAAACGGTGGAGGtTAtAtTGTtTGGtAT 36
6 7

44 TTTGAGTAAATGGTGGA GTAT 36
6 7

44 ATTTCGAGTAAACGGT TGGTAT 36
6 7

---

36 TCTCGCAGCGTTTCGTCAGAGCCGGACCCGCCTGCAGCTCAAGGGAGGCGTGCTCCTCTCCCAGAGCAGGCTGGAAC 28
6 CCA 7

36 TtTCGtAGCGTTTCGTtAGAGtCGGAttCGttTGtAGtTtAAGGGAGGCGTGtTttTtTttAGAGtAGGtTGGAAtttA 28
6 7

36 TTTCGTAGCGTT AGTTGGATTTGTTTGTA 28
6 7

36 TTTTGTAGTGTT TAGAGTCGGATTCGTT 28
6 7

---

28 GCTGGGTTCCGCCTCCCGGGAAGGTGGTCTCCATTCGTCGCTCTGCATCTGGTTTGTCAGATCCGAGAGGTAAACATT 20
6 CG 7

28 GtTGGGTTtCGttTttCGGGAAGGTGGTtTttATTCGTCGtTtTGtATtTGGTTTGTtAGATtCGAGAGGTAAAtATTCG 20
6 7

28 TTTGTTTTTTGGGAAGG 20
6 7

28 TTCGTTTTTCGGGAAG
6

---

20 GGCTTGGTGTTGAATTAAAATCATTGATTGAACCTTATTCTGGGGCTTCGGTTTGGCTTACTAGTTTGGGATTTTAAA 12
6 AA 7

20 GGtTTGGTGTTGAATTAAAATtATTGATTGAAttTTATTtTGGGGtTTCGGTTTGGtTTAtTAGTTTGGGATTTTAAAAA 12
6 7

---

12 AATAAAAATTAAGCCTATAGAGAGGGCAAATTAAAATTAGGTTGGGTAAAGGAAGGAGCGCGAGTGTTTGAAGCCG 47
6 TTTG

12 AATAAAAATTAAGttTATAGAGAGGGtAAATTAAAATTAGGTTGGGTAAAGGAAGGAGCGCGAGTGTTTGAAGtCGTT 47
6 TG

---

46 GAGGGAACAGCGGTTTCCAAGTTCCTGCTGACTTGAGAAGTCTCTG -33

46 **TTGTTGATTTGAGAAGTTTTTG** -33

46 GAGGGAAtAGCGGTTTttAAGTTttTGtTGAtTTGAGAAGTtTtTG -33

---

Figure 46: Amplificate of SEQ ID NO: 5

1 ACATGGGATTGATGGAAGACAGCCCCCAAGGACGGGGGTGGGTGGCCCTGTTTTTCTCTGATTGGCGGACGAGGGACTTC 80

1 **ATATGGGATTGATGGAAGATAG** 80

1 AtATGGGATTGATGGAAGAtAGttttAAGGACGGGGGTGGGTGGttTGTTTTTtTtTGATTGGCGGACGAGGGAtTTt 80

1 TTGATTGGCGGACGAG 80

1 TTGATTGGTGGATGAG 80

---

81 TAGACCCCTGCCAAAACACCAAGGGGGCGGTGTCGCACGCCATGCTGCTCAGCGGAAGCAGGGCTTGTATAGAAATGGGC 160

81 TAGAttttTGttAAAAtAttAAGGGGGCGGTGTCGtACGttATGtTGtTtAGCGGAAGtAGGGtTTGTATAGAAATGGGC 160

81 GGTGTTGTATGTTATGT ATGGGT 160

81 TGTCGTACGTTATGTT TGGGC 160

---

161 GTAGCAGCCGGCGGGAGTGGGTCGGACTGGGCTCCGCGTAGGCGGGCAGGGCAGCTCTACGGTTAGACAAGACCATAGAG 240

161 GTAGtAGtCGGCGGGAGTGGGTCGGAtTGGGtTtCGCGTAGGCGGGtAGGGtAGtTtTACGGTTAGAtAAGAttATAGAG 240

161 GTAGTAGTTGG ATTGGGTTTCGCGTAGG 240

161 GTAGTAGTCGG ATTGGGTTTTGTGTAGG

---

241 CTGGGTAAAGACGAATTTAGAACACAGCGGAGGTAGGAGGGCAGGATGGCTGTCAGGCACACGAAAGAGCATTGAGTGGC 320

241 tTGGGTAAAGACGAATTTAGAAtAtAGCGGAGGTAGGAGGGtAGGATGGtTGTtAGGtAtACGAAAGAGtATTGAGTGGt 320

---

321 AGAAACGAAATGCTTTCAGAGGGCAGGGTTGAACTGCGGCCAGAGTTAAAAAGGGGAGGAACCTGGCTCTGCATTGATTG 400

321 AGAAACGAAATGtTTTtAGAGGGtAGGGTTGAAtTGCGGttAGAGTTAAAAAGGGGAGGAAttTGGtTtTGtATTGATTG 400

---

401 GCGGTGGCTGGACTCAACCTAGAATAGGGGCCCGACTAGGGAGAGCGGGATCCAGGTCTGTGCCCATTGGA 480

401 **GATTTAGGTTTGTGTTTATTGGA** 480

401 GCGGTGGtTGGAtTtAAttTAGAATAGGGGttCGAtTAGGGAGAGCGGGATttAGGTtTGTGtttATTGGA 480

Figure 47: Amplificate of SEQ ID NO: 6

1 CTTTCATCCAGGATGAGGGACATTTAAGATGAAATGTCCGTGGCAGGATCGTTTCTCTTCACTGCTGCATGCGGCACTGG 80

1 **TTTTTATTTAGGATGAGGGATATT** 80

1 tTTTtATttAGGATGAGGGAtATTTAAGATGAAATGTtCGTGGtAGGATCGTTTtTtTTtAtTGtTGtATGCGGtAtTGG 80

---

81 GAACTCGCCCCACCTGTGTCCGGAACCTGCTCGCTCACGTCGGCTTTCCCCTTCTGTTTTGTTCTAGGACTTCTGCACGG 160

81 GAAtTCGtttAttTGTGTtCGGAAttTGtTCGtTtACGTCGGtTTTtttTTtTGTTTTGTTtTAGGAtTTtTGtACGG 160

81 TACGG 160

81 TATGG 160

---

161 ACCTGGCCGTCTCCAGTGCCAACTTCATTCCCACGGTCACTGCCATCTCGACCAGTCCGGACCTGCAGTGGCTGGTGCAG 240

161 AttTGGtCGTtTttAGTGttAAtTTtATTtttACGGTtAtTGttATtTCGAttAGTtCGGAttTGtAGTGGtTGGTGtAG 240

161 ATTTGGTCGTTT TATTTTGATTAGTTTGGAT 240

161 ATTTGGTTGTTT TTCGATTAGTTCGGAT 240

---

241 CCCGCCCTCGTCTCCTCCGTGGCCCCATCGCAGACCAGAGCCCCTCACCCTTTCGGAGTCCCCGCCCCCTCCGCTGGGGC 320

241 ttCGtttTCGTtTttTtCGTGGttttATCGtAGAttAGAGtttTtAttTTTCGGAGTttCGttttTtCGtTGGGGt 320

241 TTTCGTGGTTTTATCGTA 320

241 TTTTGTGGTTTTATTGTA 320

---

321 TTACTCCAGGGCTGGCGTTGTGAAGACCATGACAGGAGGCCGAGCGCAGAGCATTGGCAGGAGGGGCAAGGTGGAACAGG 400

321 TTAtTttAGGGtTGGCGTTGTGAAGAttATGAtAGGAGGtCGAGCGtAGAGtATTGGtAGGAGGGGtAAGGTGGAAtAGG 400

321 TAGGAGGTTGAGTGTA 400

321 GTCGAGCGTAGAGTAT

---

401 TGAGGAACTCTAGCGTACTCTTCCTGGGAATGTGGGGGCTGGGTGGGAAGCAGC 480

401 **GGTTGGGTGGGAAGTAGT** 480

401 TGAGGAAtTtTAGCGTAtTtTTttTGGGAATGTGGGGGtTGGGTGGGAAGtAGt 480

Figure 48: Amplificate of SEQ ID NO: 7

34 TTGGCCCCATGCTGGGAGCTCTGAGCCCCATCCCCGGGGACGCGGGCCGCGCGTACTCACTGGTGGCGAAGACTGCG 26
7 GCG 8

34
7 **TTGGTTTTATGTTGGGAGTTTTG** 26
8

34
7 TTGGtttATGtTGGGAGtTtTGAGtttATttCGGGGACGCGGGtCGCGCGTAtTtAtTGGTGGCGAAGAtTGCGGCG 26
8

34
7 GTCGCGCGTATTTATT 26
8

34
7 GGGTTGTGTGTATTTAT 26
8

26 GCGAAACTCCAGCGAAGGCCTCGCGGCCTCCGAGCCTTATAAGGGTGGTCCCGCCCCGCTCCGCCCCAGTGCTGAGT 18
7 CAC 8

26
7 GCGAAAtTttAGCGAAGGttTCGCGGttTtCGAGttTTATAAGGGTGGTttCGttCGtTtCGtttAGTGtTGAGTtAC 18
8

26
7 TTTGTGGTTTTTGAGTT 18
8

26
7 TTCGCGGTTTTCGAGT 18
8

18 GGCGCCGGCCGCTCTTCTGGAGGGTCCCGCGGACTCCCGCCGGCCCCAGCCCCGGCGGCCGCTGCACCCCGGGCGTC 10
7 GGC 8

18
7 GGCGtCGGtCGtTtTTtTGGAGGGTttCGCGGAtTttCGtCGGtttAGttCGGCGGtCGtTGtAttCGGGCGTCGGt 10
8

18
7 GTCGGT 10
8

18
7 GTTGGT 10
8

10 CGCAGAGGGGCGCCCTGGAGTCCCCGGAGTCGCCGCGCAGCTGGCCGGGGAAGCCTTTCCCTCTTTCCCAGGTCCCC
7 AGC 28

10
7 CGtAGAGGGGCGttTGGAGTttCGGAGTCGtCGCGtAGtTGGtCGGGGAAGttTTTttTtTTTttAGGTtttAGC 28

10
7 CGTAGAGGGG GAGTCGTCGCGTAGTT 28

10
7 TGTAGAGGGG GGAGTTGTTGTGTAGTT

27 GGGGCCTAGGGAGTAAACAGACAGCAG -52
27 **GTTTAGGGAGTAAATAGATAGTAG** -52
27 GGGGttTAGGGAGTAAAtAGAtAGtAG -52

Figure 49: Amplificate of SEQ ID NO: 38

---

46 CAGATGGGGACAGGAAGCTGTGGACGAAAGCCCCAGGTCCCGTGGGAGAGGTGACAGCAGCAGGGGCACGCAGCC 38
6 ACGTG 7

46 **TAGATGGGGATAGGAAGTTGT** 38
6 7

46 tAGATGGGGAtAGGAAGtTGTGGACGAAAGttttAGGTttCGTGGGAGAGGTGAtAGtAGtAGGGGtACGtAGttACGTG 38
6 7

46 TATGTAGTTATGTG 38
6 7

46 GGGTACGTAGTTACGT 38
6 7

---

38 GGTCCCCAGGGGAATGTGAAGGCGGAGGGCTCCAGGCGAACTGGGGATTAAACAAATATTTACAGGCAGCAGGGAA 30
6 GTGC 7

38 GGTttttAGGGGAATGTGAAGGCGGAGGGtTttAGGCGAAtTGGGGATTAAAtAAATATTTAtAGGtAGtAGGGAAGTGt 30
6 7

38 GGT 30
6 7

38 30
6 7

---

30 CCAGCGCACGTGACGGGGGCGGGGCGGGACTTTGGGGAGGGCGGGGCCTAACGGTATCGAGCGAGCCGGTTGTAGA 22
6 CGTG 7

30 ttAGCGtACGTGACGGGGGCGGGGCGGGAtTTTGGGGAGGGCGGGGttTAACGGTATCGAGCGAGtCGGTTGTAGACGTG 22
6 7

30 AGTGTATGTGATGGGG TATTGAGTGAGTTGGTT 22
6 7

30 TTAGCGTACGTGACGG TATCGAGCGAGTCGGT 22
6 7

---

22 GTCCAGGTTTCTGCACAGGAATATCGAGAGCGTCATGAACCCGAGCTATAGAGAAAGGAGATGAGGTCAGAGAAGT 14
6 CGAA 7

22 GTttAGGTTTtTGtAtAGGAATATCGAGAGCGTtATGAAttCGAGtTATAGAGAAAGGAGATGAGGTtAGAGAAGTCGAA 14
6 7

22 ATCGAGAGCGTTATGA 14
6 7

22 AGGAATATTGAGAGTGT
6

---

14 GGGGTGCTGGCGAGACGGGGGTGATGCCCACCCGGGCGAGAAAGGTCAGGGGTGGGGCCGTGAATTGGGCGGGAA 67
6 GGGGC

14 GGGGTGtTGGCGAGACGGGGGTGATGtttAttCGGGCGAGAAAGGTtAGGGGTGGGGtCGTGAATTGGGCGGGAAGGGGt 67
6

---

66 TGGAGAAGGGGATAGGTAGAAAAGGGCGGGGCTCGACGGGAACTGCCGCAGGGCTGGCTGATGAGG -13
66 **TAGGGTTGGTTGATGAGG** -13
66 TGGAGAAGGGGATAGGTAGAAAAGGGCGGGGtTCGACGGGAAtTGtCGtAGGGtTGGtTGATGAGG -13

---

Figure 50: Amplificate of SEQ ID NO: 39

466 CCACCAGGAGAGGGGAAGAAGCCAGCACCTACCGACAGGGGTGGAGCTGGGTCAAGAATGGTGTGGTCCCTGCTTTGGGG 387

466 **TTATTAGGAGAGGGGAAGAAGT** 387

466 ttAttAGGAGAGGGGAAGAAGttAGtAttTAtCGAtAGGGGTGGAGtTGGGTtAAGAATGGTGTGGTtttTGtTTTGGGG 387

386 GAATGCTGGGGAGGTAGAAAGCCCCTTCTAACGGGGCGTCACTGCAATTACTGCTTCCTCTTTCCCATAAAACTCCCCCT 307

386 GAATGtTGGGGAGGTAGAAAGttttTTtTAACGGGGCGTtAtTGtAATTAtTGtTTttTtTTTttATAAAAtTtttT 307

386 AATGGGGTGTTATTGT 307

386 TTTAACGGGGCGTTAT 307

306 AGTGTATCAGAACCCCCAAGGAGTTTCAGTAAGCGGTTCTTCTGTTGTCTCCGGCTGAGACTCCAGGGGAACCTCAAGCT 227

306 AGTGTATtAGAAttttAAGGAGTTTtAGTAAGCGGTTtTTtTGTTGTtTtCGGtTGAGAtTttAGGGGAAttTtAAGtT 227

226 CACATGGCCCTGGCGGGCCCCTGGGCAGGAGCAGGCGAGAGGTCTGCGCGGCCGCTCTCCTACCTGCGTCCGACTCCGCG 147

226 tAtATGGtttTGGCGGGttttTGGGtAGGAGtAGGCGAGAGGTtTGCGCGGtCGtTtTttTAttTGCGTtCGAtTtCGCG 147

226 TTCGATTTCGCG 147

226 GTTTGATTTTGTG 147

146 GTCCTTGGGCAGCAGCAACCGGGTAGCGCTCAGACTACAGACCCCAGCGCGATGACCCGCCTACCTCAGTTTCCATTGGC 67

146 GTttTTGGGtAGtAGtAAtCGGGTAGCGtTtAGAtTAtAGAtttAGCGCGATGAttCGttTAttTtAGTTTttATTGGt 67

146 GTTT TAATCGGGTAGCGTTT AGTGTGATGATTTGTTT 67

146 GTTT TAGTAATTGGGTAGTGT TAGCGCGATGATTCGT

66 CAGGGATCAGGGCTACCCGCTCCCATTGGCTACTTATCAATATAGAGGTGGGGCCAGCCTGGAATG -13

66 **TGGGGTTAGTTTGGAATG** -13

66 tAGGGATtAGGGtTAttCGtTttATTGGtTAtTTATtAATATAGAGGTGGGGttAGttTGGAATG -13

Figure 60: Amplificate of SEQ ID NO: 40

| | | |
|---|---|---|
| 1 | CACTGGATGTCCCTGGCAGGTCCGGGTGTCTGTCTCCGAAGAGGACAGAGAAGGGCAGCCACGATCTGCCCGCCTGCCCT | 80 |
| 1 | **TATTGGATGTTTTTGGTAGGTT** | 80 |
| 1 | tAtTGGATGTtttTGGtAGGTtCGGGTGTtTGTtTtCGAAGAGGAtAGAGAAGGGtAGttACGATtTGttCGttTGtttT | 80 |
| 1 | TT | 80 |
| 1 | TT | 80 |
| 81 | CGCGAGCCTCTCGGCACTGGGTGAGAGGCAACTCTGGCCATTTCTTGCTGCCCTCTCGCCCTCTCCCGGCCGCTCCCAGT | 160 |
| 81 | CGCGAGttTtTCGGtAtTGGGTGAGAGGtAAtTtTGGttATTTtTTGtTGtttTtTCGtttTtTttCGGtCGtTttAGT | 160 |
| 81 | CGCGAGTTTTTCGG | 160 |
| 81 | TGTGAGTTTTTTGGTA | 160 |
| 161 | CCAGCCGGGCCCGGCGCTACCCGAGCGAGGGTTCGAGCCCTCTGCGCGGCCGCGCAGAAGCAGGCAGGGCCTCAACTTCT | 240 |
| 161 | ttAGtCGGGttCGGCGtTAttCGAGCGAGGGTTCGAGtttTtTGCGCGGtCGCGtAGAAGtAGGtAGGGttTtAAtTTtT | 240 |
| 161 | GGTTTGGTGTTATTTGA | 240 |
| 161 | TTCGGCGTTATTCGAG | 240 |
| 241 | GCAAATGTGTGCGGCTCGCCGCCTGTCCCCTTTCTCCTCCTGTCTCCACCCGTGCGGCCCCAGTGGCCTGGAGCTTCCAG | 320 |
| 241 | GtAAATGTGTGCGGtTCGtCGttTGTtttTTTtTttTttTGTtTttAttCGTGCGGttttAGTGGttTGGAGtTTttAG | 320 |
| 241 | TATTCGTGCGGTTTTA | 320 |
| 241 | ATTTGTGTGGTTTTAGT | 320 |
| 321 | CCCCGCGCTTGGCCGCGGCTTGGCGAGGCTATGCTGCGGGAAGCTGGAATCCAACGCGCGGCCGGCTGAACCGCCTGAGC | 400 |
| 321 | tttCGCGtTTGGtCGCGGtTTGGCGAGGtTATGtTGCGGGAAGtTGGAATttAACGCGCGGtCGGtTGAAtCGttTGAGt | 400 |
| 321 | GGAATTTAATGTGTGGT | 400 |
| 321 | GAATTTAACGCGCGGT | |
| 401 | CGCGGGAAACCAGCGGCGGAGCGGCGGTATAGAGGTGCGAGGATGAGGAGGACCGACTCGCTAAGGGAGGGAGGTGACT | 480 |
| 401 | **TAAGGGAGGGAGGTGATT** | 480 |
| 401 | CGCGGGAAAttAGCGGCGGAGCGGCGGTATAGAGGTGCGAGGATGAGGAGGAtCGAtTCGtTAAGGGAGGGAGGTGAtT | 480 |

igure 61: Amplificate of SEQ ID NO: 41

---

42 GGAGGGGCTTGAGTAATTGATCCCTTCTCGAGATGGGGTCGAATTCCTTCCGAATGGGGGACCTTCATCCCCCTCCTG 34
4 TG 5

42 **GGAGGGGCTTTGAGTAATTG** 34
4 5

42 GGAGGGGtTTGAGTAATTGATttTTtTCGAGATGGGGTCGAATTttTTtCGAATGGGGGAttTTtATtttTttTGTG 34
4 5

---

34 GGTGTATGGGGGCTGCCCTGGAGATGCGCGCCCGCGGAGGCAGGTATTGGGTGTCGGCGGAGGCGGGGCCGCGTCC 26
4 CCAG 5

34 GGTGTATGGGGGtTGttTGGAGATGCGCGttCGCGGAGGtAGGTATTGGGTGTCGGCGGAGGCGGGGtCGCGTtttAG 26
4 5

---

26 GGTGCTGTCCCGGTGCCCCTGGAGGCGGCCCCGACTCCACAATGGGCCGCTCTGATTCTGAGGCGGAGGCCGGCGCT 18
4 TTG 5

26 GGTGtTGTttCGGTGtttTGGAGGCGGttCGAtTtAtAATGGGtCGtTtTGATTtTGAGGCGGAGGtCGGCGtTTTG 18
4 5

26 AGGCGGTTTCGATTTT 18
4 5

26 TGGAGGTGGTTTTGAT 18
4 5

---

18 TTGGCGGGCGGGGTAGCGGGCGAGCAGCTGTCGCATTTTCCCACGGGCGGGAGCTGAGTGTGGCCACCCCCCCTCCC 10
4 CCC 5

18 TTGGCGGGCGGGGTAGCGGGCGAGtAGtTGTCGtATTTTttACGGGCGGGAGtTGAGTGTGGttAttttttTttttC 10
4 5

18 TAGCGGGCGAGTAGTT TTTACGGGCGGGAGTT 10
4 5

18 TAGTGGGTGAGTAGTT TTTTATGGGTGGGAGT 10
4 5

---

10 GTCCAGCTCTGCCCCCTTGCAGAACGGGTTTGAACAGAGGCAATCTCGGCGGCTGGATGGGGGGCCCTGCCCTGCCA 25
4 GAC

10 GTttAGtTtTGttttTTGtAGAACGGGTTTGAAtAGAGGtAATtTCGGCGGtTGGATGGGGGGtttTGttTGttAGAt 25
4

10 ATTTCGGCGGTTGGAT 25
4

10 AGGTAATTTTGGTGGTT
4

---

24 TCCTCAGTGAGCCTCCAGGGTGGG -55

24 **TAGTGAGTTTTTAGGGTGGG** -55

24 TttTtAGTGAGttTttAGGGTGGG -55

---

Figure 62: Amplificate of SEQ ID NO: 42

1 GACCAGGCTTGGAGTTGTTTTCTTCGGGGGGCTTCCCCTCCTGACAGCCTACTGAGTTGCATCAGAACGTGGAGGATTGT 80

1 **GATTAGGTTTGGAGTTGTTTTT** 80

1 GAttAGGtTTGGAGTTGTTTTtTTCGGGGGGtTTtttTttTGAtAGttTAtTGAGTTGtATtAGAACGTGGAGGATTGT 80

81 CCTCTGAGGACGCGCGGGGCAACCGGGAGTCTTAAAATAGCAGATAAAGTTAATACTGACTGTAATGTGCGTAAATTGGA 160

81 ttTtTGAGGACGCGCGGGGtAAtCGGGAGTtTTAAAATAGtAGATAAAGTTAATAtTGAtTGTAATGTGCGTAAATTGGA 160

161 TTATAATCTTTAGGCGTATTGGTGACAACCAGCGTAATCCATCTCTGAGTATTAATCCGGTTAGACTCCCGGGTGACAGC 240

161 TTATAATtTTTAGGCGTATTGGTGAtAAttAGCGTAATttATtTtTGAGTATTAATtCGGTTAGAtTttCGGGTGAtAGt 240

161 TAATTCGGTTAGATTTTCGG 240

161 TAATTTGGTTAGATTTTTGG 240

241 CAGTCGCAGGAGTGTTCCCAGAGTCGCGCCTCTGCAAATGTTCTCACTCGCGTTGTATTTGATCTCAGTGGCCGGAGATA 320

241 tAGTCGtAGGAGTGTTttAGAGTCGCGttTtTGtAAATGTTtTtAtTCGCGTTGTATTTGAtTtTAGTGGtCGGAGATA 320

241 TTTAGAGTCGCGTTTT TTAGTGGTCGGAGATA 320

241 TAGAGTTGTGTTTTTGT AGTGGTTGGAGATA 320

241 GATA 320

241 TA 320

321 GAACAGCGCGGCCACGCGTGGGGCGCGGAGAGGAGTATCAACTACTGGCTTCCTTTACAGACGCAGATAACCTTTAAAAA 400

321 GAAtAGCGCGGttACGCGTGGGGCGCGGAGAGGAGTATtAAtTAtTGGtTTtTTTAtAGACGtAGATAAttTTTAAAAA 400

321 GA 400

321 GAATAGTGTGGTTA 400

321 GAATAGCGCGGTTA

401 GGAAACGGATAAGATCTAGACGATTGATTTGAAAGTGAAAGATGGATGTGACT 480

401 **AAAGTGAAAGATGGATGTGATT** 480

401 GGAAACGGATAAGATtTAGACGATTGATTTGAAAGTGAAAGATGGATGTGAtT 480

Figure 63: Amplificate of SEQ ID NO: 43

389 GGGGACAAGTGGTTAATGAGCAGGGCCAACTGGCCGGTCCCTGTCCTGTATGCCACACACACACCCCGTGAGGGCAGCAA 310

389 **GGGGATAAGTGGTTAATGAGT** 310

389 GGGGAtAAGTGGTTAATGAGtAGGGttAAtTGGtCGGTtttTGTttTGTATGttAtAtAtAtAtttCGTGAGGGtAGtAA 310

389 TATATTTGTGAGGGTAG 310

389 TATATTTCGTGAGGGTA 310

309 GGAGCATCCAGCCAGGGGACGTGCACGAGAAGGGCCAGGATGATCACACTGCCCGGGGCTCAGCACAGGGCTGAGTCCCG 230

309 GGAGtATttAGttAGGGGACGTGtACGAGAAGGGttAGGATGATtAtAtTGttCGGGGtTtAGtAtAGGGtTGAGTttCG 230

309 TAGGGGACGTGTACGA 230

309 TAGGGGATGTGTATGA 230

229 GAGGTGACCATTGCCGCCCCGCTCCATCTGCGGCCACTGAGGTCTGCTGGGGGTCTCTGGTGCTGGCCTTGACAGCCCAC 150

229 GAGGTGAttATTGtCGttCGtTttATtTGCGGttAtTGAGGTtTGtTGGGGGTtTtTGGTGtTGGttTTGAtAGttAt 150

149 CCGTGGCAGGACAGGGTCTCGAGGTGCCTCTCGGTAGGTGGTGGGCTCAGTGGGGGTGACCCGTGGGGACGGAACGCATG 70

149 tCGTGGtAGGAtAGGGTtTCGAGGTGttTtTCGGTAGGTGGTGGGtTtAGTGGGGGTGAttCGTGGGGACGGAACGtATG 70

149 GACGGAACGTATG 70

149 TGGGGATGGAATGTAT 70

149 ATTCGTGGGGACGGAA 70

149 ATTTGTGGGGATGGAA 70

69 CTCCTGAGGCTCCAGTGGATGCAGGCAAAGGCACCTCCCGGGGTCAGCGTCCCTACCTGGGGAGCTGGG -10

69 **TTTATTTGGGGAGTTGGG** -10

69 tTttTGAGGtTttAGTGGATGtAGGtAAAGGtAttTtCGGGGTtAGCGTttTAttTGGGGAGtTGGG -10

69

Figure 64: Amplificate of SEQ ID NO: 44

---

1 ACAGTGTGGACCCTCAGGGACACCAGAGTCTCCGTGATGTTCTTGCGTATCTGGGCTCGGCGCTGCCGCTCGTGCTTGGG 80

1 **ATAGTGTGGATTTTTAGGGATATT** 80

1 AtAGTGTGGAtttTtAGGGAtAttAGAGTtTtCGTGATGTTtTTGCGTATtTGGGtTCGGCGtTGtCGtTCGTGtTTGGG 80

---

81 CTCTGCCGCCACGTCCAGGGCCCGCTCGTACTGGGGCAGGCAGGGGGCACAGCAAGCTGTCAGCAGGGCAGGAGGCCGGC 160

81 tTtTGtCGttACGTttAGGGttCGtTCGTAtTGGGGtAGGtAGGGGGtAtAGtAAGtTGTtAGtAGGGtAGGAGGtCGGt 160

81 TGTCGTTACGTTTAGG 160

81 GTTGTTTATGTTTAGGGT 160

81 TTTAGGGTTTGTTTGTAT 160

81 TAGGGTTCGTTCGTATT 160

---

161 AGGAGGCCAGCAGATGCCCACGACTCCCGGGGTGCAGTTACGTGCTAGATGCTGTGTGATGTGGGCACTGACCCGCAACA 240

161 AGGAGGttAGtAGATGtttACGAtTttCGGGGTGtAGTTACGTGtTAGATGtTGTGTGATGTGGGtAtTGAttCGtAAtA 240

161 TTTATGATTTTTGGGGT 240

161 TTACGATTTTCGGGGT 240

---

241 CTGAGCTGTTTCTTCATGGGCAAAACAGGGTAAGCACATGGGCCCTCCTGGGCGGGGGCTGCATTGTGGAAAGCAGACGC 320

241 tTGAGtTGTTTtTTtATGGGtAAAAtAGGGTAAGtAtATGGGtttTttTGGGCGGGGGtTGtATTGTGGAAAGtAGACGt 320

241 AAGTAGACGT 320

241 TAGATGT 320

---

321 CGGAGAGGGCCCGGTGGGTGTGGCTGCTGGGAGCGGAACGTCGGGGTGCTGCTTCAGGGTCACTGGGATTTATCTCTGGG 400

321 CGGAGAGGGttCGGTGGGTGTGGtTGtTGGGAGCGGAACGTCGGGGTGtTGtTTtAGGGTtAtTGGGATTTATtTtTGGG 400

321 CGGAGA 400

321 TGGAGAGGG

---

401 GCCCGGGATGAGCCCTCCGCAAAGCTCCAGGCAGGGGAACAGGTCTTG 480

401 **AGGTAGGGGAATAGGTTTTG** 480

401 GttCGGGATGAGtttTtCGtAAAGtTttAGGtAGGGGAAtAGGTtTTG 480

---

Figure 65: Amplificate of SEQ ID NO: 45

---

1 GTAGCAAATGGGCTATGGACTTCAGTAACTAGAGTCACGTGTTACCGGCGACACAACCAATGAGGGCGCGAGATCTGTGT 80

1 **GTAGTAAATGGGTTATGGATTTTAG** 80

1 GTAGtAAATGGGtTATGGAtTTtAGTAAtTAGAGTtACGTGTTAtCGGCGAtAtAAttAATGAGGGCGCGAGATtTGTGT 80

1 TACGTGTTATCGGCGA 80

1 TATGTGTTATTGGTGATA 80

---

81 AAGCAGGGGGAGGCACGTGCCGGGCCGCACGTGTCTGGGAGGGGGGGAAGGGGCGGGGCCCACTGAGAGAGGCGGAGGTG 160

81 AAGtAGGGGGAGGtACGTGtCGGGtCGtACGTGTtTGGGAGGGGGGGAAGGGGCGGGGtttAtTGAGAGAGGCGGAGGTG 160

81 GTATGTGTTGGGTTGTA 160

81 TACGTGTCGGGTCGTA 160

---

161 GGTAGATAGACCCGGAGAGACGGCGAAGGAGCTGGAAACCGAGCCAGGGCTGAGCCGGCTGACAACAGGTAAGGAGATTC 240

161 GGTAGATAGAttCGGAGAGACGGCGAAGGAGtTGGAAAtCGAGttAGGGtTGAGtCGGtTGAtAAtAGGTAAGGAGATTC 240

161 TAGATTTGGAGAGATGG 240

161 AGATTCGGAGAGACGG 240

---

241 GGAGGACAAAGCGAGCTGTTAGGGAGTATTTGGGAGTGGATTTGGGGGCTGAATGCAGATCTTGGTATTGGGAGGGTTTG 320

241 GGAGGAtAAAGCGAGtTGTTAGGGAGTATTTGGGAGTGGATTTGGGGGtTGAATGtAGATtTTGGTATTGGGAGGGTTTG 320

---

321 CATCACGTGGCAGGTGCGAGCCCAGAGAGACCGGCGCAGGGATCCTGATCTTGAAAGATTGGGAGAGGGCAGGAGGCCAG 400

321 tATtACGTGGtAGGTGCGAGtttAGAGAGAtCGGCGtAGGGATttTGATtTTGAAAGATTGGGAGAGGGtAGGAGGttAG 400

321 AGATCGGCGTAGGGAT 400

321 AGATTGGTGTAGGGAT

---

401 TCTCTATGGGGGCGGTTCCCGTAGGATCACCAGGTGGGTTCGCGTGCCCAGATTGACTGCTTGTGGGTGGCATGG 480

401 **TGTTTGTGGGTGGTATGG** 480

401 TtTtTATGGGGGCGGTTtCGTAGGATtAttAGGTGGGTTCGCGTGttAGATTGAtTGtTTGTGGGTGGtATGG 480

---

Figure 66. Amplificate of SEQ ID NO: 46

| | | |
|---|---|---|
| 1 | CCCCAGCAGTGGACCTGGTCAGCACCTCTGGGGCTGGGACATCAGTGTCCTAAAGGTGGAAGGGTTAGACCCTCTAG GGG | 80 |
| 1 | **TTTTAGTAGTGGATTTGGTTAGTATT** | 80 |
| 1 | tttAGtAGTGGAttTGGTtAGtAttTtTGGGGtTGGGAtATtAGTGTttTAAAGGTGGAAGGGTTAGAtttTtTAGGGG | 80 |
| 81 | AAGGGCCCACCGCCCTCCACAGAGGCTCTGGCTTAGGCCGCGTGGACACGTGAGGGGGGCACCTACCGTGTTCTCCA TGG | 160 |
| 81 | AAGGGtttAtCGtttTttAtAGAGGtTtTGGtTTAGGtCGCGTGGAtACGTGAGGGGGGtAttTAtCGTGTTtTttATGG | 160 |
| 81 | GGTTGTGTGGATATGT | 160 |
| 81 | GTCGCGTGGATACGTG | 160 |
| 161 | ACTTGCTGGCGACTCCCACGAGAAGGCCAGCCAGGAGGGCGAGGTGCCGCAGCGCCATGCCAGGAGCAGATGCGCA GAGC | 240 |
| 161 | AtTTGtTGGCGAtTttACGAGAAGGttAGttAGGAGGGCGAGGTGtCGtAGCGttATGttAGGAGtAGATGCGtAGAGt | 240 |
| 161 | TTGGCGATTTTTACGA AGGTGTTGTAGTGTTAT | 240 |
| 161 | TTGGTGATTTTTATGAGA TGTCGTAGCGTTATGT | 240 |
| 161 | GATTTTTATGAGAAGGTT | 240 |
| 161 | ATTTTTACGAGAAGGTT | |
| 241 | CTGCCACAGGGAGGAGCATGCGGAGCCAAAGAGATGGAGTGGGGCTGAGGCAGGGTGGGTGGGGCCAAATGAAAG TGGGG | 320 |
| 241 | **TGAAAGTGGGG** | 320 |
| 241 | tTGttAtAGGGAGGAGtATGCGGAGttAAAGAGATGGAGTGGGGtTGAGGtAGGGTGGGTGGGGttAAATGAAAGTGGGG | 320 |
| 321 | TCAAGAGATG | 400 |
| 321 | **TTAAGAGATG** | 400 |
| 321 | TtAAGAGATG | 400 |

Figure 67: Amplificate of SEQ ID NO: 47

335 GAGGAAAGAAGGAGGATACAGGCGGCCAATGTGTGGCCTACGTGGGAGCGCCCCGAGCGCTCCACGTCCCCGGCCTGCGC 256

335 **GAGGAAAGAAGGAGGATATAGG** 256

335 GAGGAAAGAAGGAGGATAtAGGCGGttAATGTGTGGttTACGTGGGAGCGtttCGAGCGtTttACGTtttCGGttTGCGt 256

335 TTTTGAGTGTTTTATGTT 256

335 TTCGAGCGTTTTACGT 256

335 TTACGTGGGAGCGTTT 256

335 TTATGTGGGAGTGTTT 256

---

255 CGGGCGGCCCCTTCCCCGGGCATCTTGGCAACGCGCCTGGCCCAAGCCGGAGGGGCCCCGCGGCCCCGCACGCCTCCTGG 176

255 CGGGCGGttttTTtttCGGGtATtTTGGtAACGCGttTGGtttAAGtCGGAGGGGtttCGCGGtttCGtACGttTttTGG 176

255 GTAACGCGTTTGGTTT 176

255 TGGTAATGTGTTTGGT 176

---

175 ACCAGTCAGGATCCAGGCTCCTCTTTGAGCCCGCGGGTGTTTCATGGGGGTAGAAGTCTAACCCCTCCACCCCCTCTCCT 96

175 AttAGTtAGGATttAGGtTttTtTTTGAGttCGCGGGTGTTTtATGGGGGTAGAAGTtTAAttttTttAttttTtTttT 96

---

95 CCCCAGCAGTCCCACGCGGGTATGGGAGAGAATGAAGTTCTTTGTCTCTAAGGGATTCAAACCAGAAACGGAGGGACCTC 16

95 **GATTTT** 16

95 tttAGtAGTtttACGCGGGTATGGGAGAGAATGAAGTTtTTTGTtTtTAAGGGATTtAAAttAGAAACGGAGGGAttTt 16

95 AGTTTTATGTGGGTATG 16

95 TAGTTTTACGCGGGTA

---

15 TGGTTCCCAGAGGGA -64

15 **TGGTTTTTAGAGGGA** -64

15 TGGTTtttAGAGGGA -64

Figure 68: Amplificate of SEQ ID NO: 48

1 GCCAAAGGGGCATTGGTTCCCCAAGGGCCAAACGAGCTTCCTCCAGGAGTTTCCCGTCGCGTGCAGTGGACCACCGT GGG 80

1 **GTTAAAGGGGTATTGGTTTTT** 80

1 GttAAAGGGGtATTGGTTttttAAGGGttAAACGAGtTTttTttAGGAGTTTttCGTCGCGTGtAGTGGAttAtCGTGGG 80

---

81 CCTGACTGAGGCCTGGTCCTTGCCAGGGTTTTTGGCAACGGCCCTGGATGAGTGAAGGGATGAAAGGCGCGAGAA GAAA 160

81 ttTGAtTGAGGttTGGTttTTGttAGGGTTTTTGGtAACGGtttTGGATGAGTGAAGGGATGAAAGGCGCGAGAAGAAA 160

81 ATGAAAGGCGCGAGAA 160

81 ATGAAAGGTGTGAGAA 160

---

161 TGGGCTGAGAAACCAAAGTGCGGGGCCCTGAGTGTTTGAGGGGACGCGATGGGAACCGTGGCTCCGCTGCCCTGAT TTTA 240

161 TGGGtTGAGAAAttAAAGTGCGGGGtttTGAGTGTTTGAGGGGACGCGATGGGAAtCGTGGtTtCGtTGtttTGATTTTA 240

161 ATGGGAATCGTGGTTT 240

161 ATGGGAATTGTGGTTT 240

161 GGAATTGTGGTTTTGT 240

161 GAATCGTGGTTTCGTT 240

---

241 AGGGCAGTTACCCTGTCACAGCCACGCTGGAACTGAGGTGTCCTTCCAGCTTCTGTCCACCTCAGAGGAATTCAAAA CAA 320

241 AGGGtAGTTAtttTGTtAtAGttACGtTGGAAtTGAGGTGTttTTttAGtTTtTGTttAttTtAGAGGAATTtAAAAtAA 320

---

321 GGGCCCAGTTCTAAGCCCCACAGACCCCAGGATCCCCTAGGGGCCATTAGTCCACCGCGGCAAATGGTTTCCTCACT TTG 400

321 GGGtttAGTTtTAAGttttAtAGAttttAGGATttttTAGGGGttATTAGTttAtCGCGGtAAATGGTTTttTtAtTTTG 400

321 TAGTTTATCGCGGTAA 400

321 TTTATTGTGGTAAATGGT

---

401 CTCATTTGTGTTTGTTTTGAAATCCAATTGTCACCTCACTGGGCTGCACAGTTGCTACTGAAACGGTTCAATAGGTGA GA 480

401 **GTTTAATAGGTGAGA** 480

401 tTtATTTGTGTTTGTTTTGAAATttAATTGTtAttTtAtTGGGtTGtAtAGTTGtTAtTGAAACGGTTtAATAGGTGAGA 480

---

481 GAAAAAGAAA 560

481 **GAAAAAGAAA** 560

481 GAAAAAGAAA 560

Figure 69: Amplificate of SEQ ID NO: 49

1 GTAAGAGGGACAGGGAACTGGGGCGCTGGAGCCGGGAAGCGGGTGAGCGAGTCTTCCCAACCGCTATGTCCCCTATTAAA 80

1 **GTAAGAGGGATAGGGAATTGG** 80

1 GTAAGAGGGAtAGGGAAtTGGGGCGtTGGAGtCGGGAAGCGGGTGAGCGAGTtTTttAAtCGtTATGTtttTATTAAA 80

---

81 AAGAACCCCGCTCCTTCGTGAAACCCCTTTTCCCTTAGCCTTTAAGCTGCCGGGCCGCGACGGGGAACTGGCCAACTCAT 160

81 AAGAAtttCGtTtTTCGTGAAAtttTTTTttTTAGttTTTAAGtTGtCGGGtCGCGACGGGGAAtTGGttAAtTtAT 160

---

161 CCTTCGCCGGGCTCTCCGCCCTCCGGGCTCTGGGGCAGAACAGGGTCCTGAGGCGGCGTGGGCGAGGGCAGTCAATGAGC 240

161 ttTTCGtCGGGtTtTtCGttTtCGGGtTtTGGGGtAGAAtAGGGTttTGAGGCGGCGTGGGCGAGGGtAGTtAATGAGC 240

---

241 GGGAAGGACGTGAAGAAGGTGACACAGGCTGCCCCCCACTGGAAAATGGGTTGGGAGGGGGTGCGGCCCGAAGACCAGAG 320

241 GGGAAGGACGTGAAGAAGGTGAtAtAGGtTGtttttAtTGGAAAATGGGTTGGGAGGGGGTGCGGttCGAAGAttAGAG 320

241 GGTGTGGTTTGAAGAT 320

241 TGCGGTTCGAAGATTA 320

---

321 GATAGCAAAGGTCCACGGAAAAGGGAGCTCCTGGCCCGGACGCGGCGTGGGGAACGCGTCCTAGCGCCGGGGATGCGGAG 400

321 GATAGtAAAGGTttACGGAAAAGGGAGtTttTGGttCGGACGCGGCGTGGGGAACGCGTttTAGCGtCGGGGATGCGGAG 400

321 AGGTTTATGGAAAAGG GGAATGTGTTTTAGTGT 400

321 AGGTTTACGGAAAAGG GAACGCGTTTTAGCGT 400

321 TTTTAGCGTCGGGGAT 400

321 TTTTAGTGTTGGGGAT

---

401 CTGGGGAGAGGGGTGGGAGCCCCATCCTGGTTTGGGTGCC 480

401 **TTTTATTTTGGTTTGGGTGTT** 480

401 tTGGGGAGAGGGGTGGGAGttttATttTGGTTTGGGTGtt 480

---

Figure 70: Amplificate of SEQ ID NO: 50

1 GGAGTTTGTGAAAAGTGGGGCTCGGGGCCCAGTCAATGGGGCGCCCCGCGGCGCGGGCTGAGTGGAGCTAGCGCGAACCG 80
1 **GGAGTTTGTGAAAAGTGGG** 80
1 GGAGTTTGTGAAAAGTGGGGtTCGGGGtttAGTtAATGGGGCGtttCGCGGCGCGGGtTGAGTGGAGtTAGCGCGAAtCG 80
1 TAGCGCGAATCG 80
1 AGTGTGAATTG 80

81 CTCAGCCGCGGCCCCAATTAATCCGCCCTTTGTGCGGCCCGCCCGGCCGCCCCCGCCGCAGCCGCACCAGCGGCCCATTG 160
81 tTtAGtCGCGGttttAATTAATtCGtttTTTGTGCGGttCGttCGGtCGtttCGtCGtAGtCGtAttAGCGGtttATTG 160
81 TTTA TAGTCGTATTAGCGGT 160
81 TTTAGT TAGTTGTATTAGTGGTTT 160

161 TTCGGCCTCGCCGGGCCGCGGGATTTACCCTTTTCAAACAGCCGGTTTTGTCCAGGGCAGTTCGAGCGGAAGTTTCTCAC 240
161 TTCGGttTCGtCGGGtCGCGGGATTTAtttTTTTtAAAtAGtCGGTTTTGTttAGGGtAGTTCGAGCGGAAGTTTtTtAt 240

241 TGACAATTTGCCCCAAATAGATAGATTTGTCAGAAGCGACCTTCGGGAAGGAAGCAAAAAGCCACCGGCCCGAAGGTTGG 320
241 TGAtAATTTGttttAAATAGATAGATTTGTtAGAAGCGAttTTCGGGAAGGAAGtAAAAAGttAtCGGttCGAAGGTTGG 320

321 GCCCAAAACAGGGACTCTGCGTCCCACCCGCGGCCGCGCCGCCCCCCCGCGCCCCCGGCCCTGCAGTCCCGAAGCCCCGC 400
321 GtttAAAAtAGGGAtTtTGCGTtttAttCGCGGtCGCGtCGtttttCGCGtttCGGtttTGtAGTttCGAAGtttCGC 400
321 TGCGTTTTATTCGCGG TTTCGAAGTTTCGC 400
321 TGTGTTTTATTTGTGGT TTTGAAGTTTTGT 400

401 GGGGGTCCAGACCACTGCACCTGCTGAGC 480
401 **GGTTTAGATTATTGTATTTGTTGAGT** 480
401 GGGGGTttAGAttAtTGtAttTGtTGAGt 480
401 GG 480
401 GGG

Figure 71: Amplificate of SEQ ID NO: 51

22 GAAGGTTGAGGAGGAGCCAGAGCCGGGTCCTGCAGCGTTTCTCGCCATCAGCGCCCGTCGCCATCTCCACCATGCAG 14
8 TCC 9

22 **GAAGGTTGAGGAGGAGTTAGA** 14
8 9

22 GAAGGTTGAGGAGGAGttAGAGtCGGGTttTGtAGCGTTTtTCGttATtAGCGttCGTCGttATtTttAttATGtAGTtt 14
8 9

22 TAGAGTCGGGTTTTGTA TATTAGCGTTCGTCGT 14
8 9

22 TAGAGTTGGGTTTTTGTA GTTATTAGTGTTTGTTGT 14
8 9

22 TTT 14
8 9

22 AGTTT 14
8 9

14 CGGGAAGACGCCCCGCGCTCTCGCCGCCTAGCCAGTCCCCGTGGTGGGAAGCGGCCCAAGAAGATTCACAAACCCA 69
8 CAGT

14 CGGGAAGACGtttCGCGtTtTCGtCGttTAGttAGTtttCGTGGTGGGAAGCGGtttAAGAAGATTtAtAAAtttAtAGT 69
8

14 AGACGTTTCGCGTTTT 69
8

14 GAAGATGTTTTGTGTTT 69
8

14 CGGGAAGACGTTT 69
8

14 TGGGAAGATGT
8

68 TTCGGCCTTTTTCACGGGTCCAGAGGAATTAAAGGACACGGCCCATTCTGCAGCCCTGCTGGCACAGC -11

68 **TTTATTTTGTAGTTTTGTTGGTATAGT** -11

68 TTCGGttTTTTTtACGGGTttAGAGGAATTAAAGGAtACGGtttATTtTGtAGtttTGtTGGtAtAGt -11

Figure 72 Amplificate of SEQ ID NO: 52

| | | |
|---|---|---|
| 1 | GGCTGGAAAGTGGAGGATCCGGTTTGCTCTGGGCGGGTCTGGAAGCAGAGCCGGCGGAGGGAGCGCCGGGGCCCTG<br>GGCT | 80 |
| 1 | **GGTTGGAAAGTGGAGGATT** | 80 |
| 1 | GGtTGGAAAGTGGAGGATtCGGTTTGtTtTGGGCGGGTtTGGAAGtAGAGtCGGCGGAGGGAGCGtCGGGGtttTGGGtT | 80 |
| 1 | AGTAGAGTCGGCGGAG | 80 |
| 1 | AGTAGAGTTGGTGGAG | 80 |
| 81 | GCAGGAGGTTGCGGCGGCCGCGGCAGCATGGTGGTGCCGGAGAAGGAGCAGGTGAGCGCCGGACCAGGGTCTGCG<br>GGAGC | 16<br>0 |
| 81 | GtAGGAGGTTGCGGCGGtCGCGGtAGtATGGTGGTGtCGGAGAAGGAGtAGGTGAGCGtCGGAttAGGGTtTGCGGGAGC | 16<br>0 |
| 81 | TAGGTGAGCGTCGGAT | 16<br>0 |
| 81 | TAGGTGAGTGTTGGAT | 16<br>0 |
| 16<br>1 | GCGGAGCTGGGGACCTCGCCTCCAGGCTCCCAGAGAGGAGGGCGCTGGACACCCAGATTCCTGGGTCAGACGGAGG<br>AGGG | 24<br>0 |
| 16<br>1 | GCGGAGtTGGGGAttTCGttTtAGGtTttAGAGAGGAGGGCGtTGGAtAttAGATTtTGGGTtAGACGGAGGAGGG | 24<br>0 |
| 24<br>1 | GGATGGGAGGGTCCAGGTTCCAGGGTCCAGGGCATGGGGGTCGAGACTCCTGAGTCTGGAGCGGGAGGGCGCTGGG<br>GGCC | 32<br>0 |
| 24<br>1 | GGATGGGAGGGTttAGGTTtAGGGTtAGGGtATGGGGGTCGAGAtTtTGAGTtTGGAGCGGGAGGGCGtTGGGGGtt | 32<br>0 |
| 24<br>1 | ATGGGGGTCGAGATTT | 32<br>0 |
| 24<br>1 | ATGGGGGTTGAGATTT | |
| 32<br>1 | CGGACTCCTGTGTCCGTGGGGAGCGCACGGGGTGGGTGGCTCTGTGTCCCATAGGACAGAACTGGGTGCCCTCTCAC<br>CCC | 40<br>0 |
| 32<br>1 | CGGAtTtTGTGTtCGTGGGGAGCGtACGGGGTGGGTGGtTtTGTGTttATAGGAtAGAAtTGGGTGttTtTtAttt | 40<br>0 |
| 40<br>1 | ACTTCCACCCCTACATTTGTTCCTGTCCCCAGAGCTGG | 48<br>0 |
| 40<br>1 | **TTGTTTTTGTTTTTAGAGTTGG** | 48<br>0 |
| 40<br>1 | AtTTtAtttTAtATTTGTTtTGTtttAGAGtTGG | 48<br>0 |

Figure 73: Amplificate of SEQ ID NO: 53

1 GAGCTGGCCCTGCTGAGGTGCCGGGCCGAGTAGTTGGAGAGAGGGTCATACTCCAGGTCTGTGGGTGGCCTGGAGTTGTC 80

1 **GAGTTGGTTTTGTTGAGGTGT** 80

1 GAGtTGGtttTGtTGAGGTGtCGGGtCGAGTAGTTGGAGAGAGGGTtATAtTttAGGTtTGTGGGTGGttTGGAGTTGTt 80

1 TGTCGGGTCGAGTAGT 80

1 • TGTTGGGTTGAGTAGT 80

81 CACCACGTACTTGCCACTGGGAACGGGGCGGCTGTGCCGCCGGGGCTGGCTCACAGCCTTGGGGACGTATTCCAGGGCAC 160

81 tAttACGTAtTTGttAtTGGGAACGGGGCGGtTGTGtCGtCGGGGtTGGtTtAtAGttTTGGGGACGTATTttAGGGtAt 160

161 CGCCACCCCCTCCACCTCTGCCCTGACCCCTGTCCAGGGACGCCAGCGAGTACTTGCTGCCCGGCTCGGCAGGGGCGGCC 240

161 CGttAttttTttAttTtTGtttTGAttttTGTttAGGGACGttAGCGAGTAtTTGtTGttCGGtTCGGtAGGGGCGGtt 240

161 AGGGATGTTAGTGAGT 240

161 GACGTTAGCGAGTATT 240

241 AGTGGGGTGGGCTGGTAGCCGGCATCAGGGCTTAATAGGCCGTGGCTGCCGGGGCTGTAGTCGAAGGCCAGTGGGAAGGC 320

241 **AAGGTTAGTGGGAAGGT** 320

241 AGTGGGGTGGGtTGGTAGtCGGtATtAGGGtTTAATAGGtCGTGGtTGtCGGGGtTGTAGTCGAAGGtAGTGGGAAGGt 320

241 TGGTAGTTGGTATTAGG TGTAGTCGAAGGTTAG 320

241 GGTAGTCGGTATTAGG TGTAGTTGAAGGTTAGT

321 ATCCT 400

321 **ATTTT** 400

321 ATttT 400

Figure 74: Amplificate of SEQ ID NO: 54

1 CAGTGGAACCATGAGGGGGGAGCCCACCCGGCTCACACGACCTCTCCCTCCGGAGGCCGCTCTAAGCACACCCTCCAGCC 80

1 **TAGTGGAATTATGAGGGGG** 80

1 tAGTGGAAttATGAGGGGGGAGtttAttCGGtTtAtACGAttTtTttTtCGGAGGtCGtTtTAAGtAtAtttTttAGtt 80

1 GGAGTTTATTCGGTTTAT TT 80

1 GGAGTTTATTTGGTTTATA T 80

1 ATTTGGTTTATATGATTTTT 80

1 TTCGGTTTATACGATTT 80

81 ACGCCGACCACCGGGGCTGTCCCCGATTCTCACCTAAAACCCCCTTCAGCCCACAGCCCCCACGGCCTTCCTGTGGTCTC 160

81 ACGtCGAttAtCGGGGtTGTtttCGATTtTtAttTAAAAttttTTtAGttAtAGttttACGGttTTttTGTGGTtTt 160

81 ACGTCGATTATCGG 160

81 ATGTTGATTATTGGGGT 160

161 CTCCCTCCTTCACGGCGGGTCCTCATCCTTTACCTTCTCTCTCTGGAGCCCAAATTGGAACTCCGCTCCTCCCCTCCGCG 240

161 tTttTttTTtACGGCGGGTttTtATttTTTAttTTtTtTtTtTGGAGtttAAATTGGAAtTtCGtTtTtttTtCGCG 240

161 TTACGGCGGGTTTTTA 240

161 TTATGGTGGGTTTTTATT

241 CAGCTCCATGCACTGGCCCTTTGGGACGCCTCTTTCTTTCTTTGGCAGAGGTGCATCTGG 320

241 **TTTTGGTAGAGGTGTATTTGG** 320

241 tAGtTttATGtAtTGGtttTTTGGGACGttTtTTTtTTTtTTTGGtAGAGGTGtATtTGG 320

Figure 75: Amplificate of SEQ ID NO: 55

38 TAGGACTTGTGGCTGGTGTTCTCGGCCATGCCCTGGCCCAAGAAGTCGTTGGTGAAGATGCCTCAGCGGAGCGGGGC 30
4 GCC 5

38 **TAGGATTTGTGGTTGGTGTT** 30
4 5

38 TAGGAtTTGTGGtTGGTGTTtTCGGttATGtttTGGtttAAGAAGTCGTTGGTGAAGATGttTtAGCGGAGCGGGGCGtt 30
4 5

---

30 CGCCGGGCCGCACGTTGGGGTTGTTCACGGTCGCCCACACGTCGTCGTGCACGAACTCGCCCTGCAGGGAGCGGCCT 22
4 TAG 5

30 CGtCGGGtCGtACGTTGGGGTTGTTtACGGTCGtttAtACGTCGTCGTGtACGAAtTCGtttTGtAGGGAGCGGttTTAG 22
4 5

30 GTTGTGTATGAATTTGTT 22
4 5

30 GTCGTGTACGAATTCGT 22
4 5

---

22 CACAGGCAGCTCGCCCCGGCCAGCAGCACCTCTTCCTCCCCACTGCGAATGCCCCGATCATGCTCCCCCAGACCCAA 14
4 CAC 5

22 tAtAGGtAGtTCGtttCGGttAGtAGtAttTtTTttTtttAtTGCGAATGtttCGATtATGtTttttAGAtttAAtAt 14
4 5

22 AGTTTGTTTTGGTTAGT 14
4 5

22 TAGTTCGTTTCGGTTA 14
4 5

---

14 ACAAACACCCTTGGGAACCCACGCGGGTGCCTTCTGTTTACCGCAGCGCTCAAGCCGCGGGCGCTTGAGTCCCAGAT 65
4 GGC

14 AtAAAtAtttTTGGGAAtttACGCGGGTGttTTtTGTTTAtCGtAGCGtTtAAGtCGCGGGCGtTTGAGTtttAGATGGC 65
4

14 AATTTACGCGGGTGTT AGCGTTTAAGTCGCGG 65
4

14 GGAATTTATGTGGGTG TAGTGTTTAAGTTGTGG
4

---

64 GAAAGTTCGCGCGCAGGTGGAGTGGGGATCCCGGCGTGCGGCTGCCCAGGAGAGGAGGGCAAGG -15
64 **TTTAGGAGAGGAGGGTAAGG** -15
64 GAAAGTTCGCGCGtAGGTGGAGTGGGGATttCGGCGTGCGGtTGtttAGGAGAGGAGGGtAAGG -15

Figure 76: Amplificate of SEQ ID NO: 56

| | | |
|---|---|---|
| 1 | GGCTCAGGGCACCCTAAGGGGCCGGATTTCCAGGGGTCCCATCTTGGCTGTGGTGGAGAGCTGGACTCAGGTCTCTC TTG | 80 |
| 1 | **GGTTTAGGGTATTTTAAGGGG** | 80 |
| 1 | GGtTtAGGGtAtttTAAGGGGtCGGATTTttAGGGGTtttATtTTGGtTGTGGTGGAGAGtTGGAtTtAGGTtTtTtTTG | 80 |
| 81 | GGGGCCCTGGGGACCAGAGCCACGATCCCGGTGCCCTGATGTCCCCGTCTCTTCCCCACCTTCCTCTCCCCCAGTGTC TG | 160 |
| 81 | GGGGtttTGGGGAttAGAGttACGATttCGGTGtttTGATGTtttCGTtTtTTttttAttTTtTtTtttAGTGTtTG | 160 |
| 81 | TAGAGTTATGATTTTGGT | 160 |
| 81 | TTACGATTTCGGTGTT | 160 |
| 161 | CCTCAGGACGAGGCGTGCGCCGACACTGGCAGTGGCAGCGCCGAGGGCCTGGCTGCTGACGGCCCCCACCTGCACA CGCT | 240 |
| 161 | ttTtAGGACGAGGCGTGCGtCGAtAtTGGtAGTGGtAGCGtCGAGGGttTGGtTGtTGACGGttttAttTGtAtACGtT | 240 |
| 161 | AGGCGTGCGTCGATAT GTAGCGTCGAGGGTTT TTGTATATGTT | 240 |
| 161 | AGGTGTGTGTTGATATT GTAGTGTTGAGGGTTT ATACGTT | 240 |
| 241 | GACGCAGCCTATCGTGGTCACCGTGCCGCGGCCGCCCCCCAGTGAGCACGCAGTCCTCCTCCGCATGGGGCCGTGGG GCG | 320 |
| 241 | GACGtAGttTATCGTGGTtAtCGTGtCGCGGtCGttttttAGTGAGtACGtAGTttTttTtCGtATGGGGtCGTGGGGCG | 320 |
| 241 | GATGTAGT | 320 |
| 241 | GACGTAGTT | |
| 321 | GCAGAGCGGGTGGGAAGGACGCCTGGGAGCTGGACCCAGTCT | 400 |
| 321 | **TTTGGGAGTTGGATTTAGTTT** • | 400 |
| 321 | GtAGAGCGGGTGGGAAGGACGttTGGGAGtTGGAttAGTtT | 400 |

Figure 77: Amplificate of SEQ ID NO: 57

37 GGGGAGGTGCTCAGTGGTCCTAGTCGCCCCGAGACCCTAGCTCTTCTCGCCCGCTGCGGGCCTGACTGCGCCAGGGG 29
8 CCG 9

37 **GGGGAGGTGTTTAGTGGTT** 29
8 9

37 GGGGAGGTGtTtAGTGGTttTAGTCGtttCGAGAtttTAGtTtTTtTCGttCGtTGCGGGttTGAtTGCGttAGGGGtCG 29
8 9

37 TTAGTCGTTTCGAGAT 29
8 9

37 GTTTTAGTTGTTTTGAGA 29
8 9

---

29 GGGGTTATGTGGGCAGGCTCGAACGACTGGGAGCGTGGCCTGTGATTCTGCTCCACTCCTTTCGCCCGCAGAGCGGT 21
8 GGT 9

29 GGGGTTATGTGGGtAGGtTCGAACGAtTGGGAGCGTGGttTGTGATTtTGtTttAtTttTTTCGttCGtAGAGCGGTGGT 21
8 9

---

21 CTAGGCCAGACCATTGACCTGGGTAGGGGGAAGGGCAAGGACGCAGCTCGCACGCCTGGGTTTTCACCGCCGTCTCC 13
8 CTT 9

21 tTAGGttAGAttATTGAttTGGGTAGGGGGAAGGGtAAGGACGtAGtTCGtACGttTGGGTTTTtAtCGtCGTtTtttTT 13
8 9

21 GACGTAGTTCGTACGT 13
8 9

21 GGATGTAGTTTGTATGT 13
8 9

---

13 CGGCGTTTTGGGCTGGCGGCCGACATTGGCCTGGACCAAATGGTGGCAAAGTCTCACCAGTTCCGCGTCTCGCAAGA 59
8 CAC

13 CGGCGTTTTGGGtTGGCGGtCGAtATTGGttTGGAttAAATGGTGGtAAAGTtTtAttAGTTtCGCGTtTCGtAAGAtAt 59
8

13 TTTGTGTTTTGTAAGATAT 59
8

13 TTCGCGTTTCGTAAGA 59
8

---

58 TTTCTACGCGCATCGGGACCTAAACACTCGGGAGGACCCTGGTAAAGTGAGCAGAGGG -21

58 **TTTTGGTAAAGTGAGTAGAGGG** -21

58 TTTtTACGCGtATCGGGAttTAAAtAtTCGGGAGGAtttTGGTAAAGTGAGtAGAGGG -21

58 TATGTGTATTGGGATTTA -21

58 TTACGCGTATCGGGAT

Figure 78: Amplificate of SEQ ID NO: 58

---

1 TGTTGGTGGCCATCTTTAATCCTCCTCCTCCTCCTGCTTTCTCCACCTCCCGCTGGCTGTCTGACTGACTGACTGGATC C 80

1 **TGTTGGTGGTTATTTTTAATTTTT** 80

1 TGTTGGTGGttATtTTTAATttTttTttTttTttTGtTTTtTtAttTtCGtTGGtTGTtTGAtTGAtTGAtTGGATtt 80

---

81 TCCTCTTTCCCCTCCTGCTCCTCCTACTGAGCCGGCCGCAGAAATTGCAGCCGCTCAGCTTCTACCCCCTCCTGCCTTT C 160

81 TttTtTTTtttTttTGtTttTttTAtTGAGtCGGtCGtAGAAATTGtAGtCGtTtAGtTTtTAttttTttTGttTTTt 160

81 TATTGAGTCGGTCGTA AATTGTAGTCGTTTAGTT 160

81 ATTGAGTTGGTTGTAGA 160

81 AAATTGTAGTTGTTTTAGTT 160

---

161 CTTCCTCTTTCCTTACTTCCTTCCCTTCCCTCGGCTTCCCGCTCTTGCCTCACTCTCAGCGGCTGCCTTCGCCCCTGTCT 240

161 tTTtTtTTTttTTAtTTttTTttTTttTCGGtTTtCGtTtTTGttTtAtTtTtAGCGGtTGttTTCGtttTGTtT 240

---

241 GCAGACAGCGCCGCTGGATGCTCCCAGCTGGACTTCAACCCCACTCCTCTCAGTCCCTCTCCCCACTGCCTTCCAGAC GC 320

241 GtAGAtAGCGtCGtTGGATGtTttAGtTGGAtTTtAAtttAtTttTtTtAGTttTtTttAtTGttTTtAGACGC 320

241 TAGATAGCGTCGTTGG 320

241 TAGATAGTGTTGTTGGA

---

321 GCCTCTTCCCCGCCCCGCGCCCCTCTCTCCTCTCCCACCCCTGCCCCTCTCCGCGGCGCTCACCCTCCTCAGTCCCAGT T 400

321 GttTtTTttCGttCGCGtttTtTtTtTtTttAtttTGtttTtTtCGCGGCGtTtAttTtTtAGTttAGTT 400

---

401 TCTGAAAGGACTCAGCTGAGAAAGGACAACTGGGT 480

401 **GTTGAGAAAGGATAATTGGGT** 480

401 TtTGAAAGGAtTtAGtTGAGAAAGGAtAAtTGGGT 480

---

Figure 79: Amplificate of SEQ ID NO: 59

| | | |
|---|---|---|
| 1 | GAGGACCTGTGCTAAGGCTTTCTCATCCACCAGGCCACCATGGGCTGCGTTCACAAGGAATGCTCCCTGTCTCATCTGCT | 80 |
| 1 | **GAGGATTTGTGTTAAGGTTTTT** | 80 |
| 1 | GAGGAttTGTGtTAAGGtTTTtTtATttAttAGGttAttATGGGtTGCGTTtAtAAGGAATGtTttTGTtTtATtTGtT | 80 |
| 1 | ATGGGTTGCGTTTATA | 80 |
| 1 | TGGGTTGTGTTTATAAG | 80 |
| 81 | TTATAGTAAAGTCATTGACGAGGTGGTGGTTATGTTCATTGAGATTGCAGTGCAACGAGACACAGTCACTCTGATACAGC | 160 |
| 81 | TTATAGTAAAGTtATTGACGAGGTGGTGGTTATGTTtATTGAGATTGtAGTGtAACGAGAtAtAGTtAtTtTGATAtAGt | 160 |
| 161 | AAACCCTGCAGGGTGTATCAGGGTCCCCTCTGCATGCCCTGGGACCTCTCTATCTTGTCCTACAAGTAGGGGTCATAAAA | 240 |
| 161 | AAAtttTGtAGGGTGTATtAGGGTtttTtTGtATGttTGGGAttTtTtTATtTTGTtTAtAAGTAGGGGTtATAAAA | 240 |
| 161 | ATAAAA | 240 |
| 161 | ATAAAA | 240 |
| 241 | TACGACGCTGAATCCAAAGGCCTTGGCTCAAACTGCAACCGCCTGCCTCATGCAACCGAAGCCCATGAGGCCTAGCGTCT | 320 |
| 241 | TACGACGtTGAATttAAAGGttTTGGtTtAAAtTGtAAtCGttTGttTtATGtAAtCGAAGtttATGAGGttTAGCGTtT | 320 |
| 241 | TACGACGTTGA TAGTGTTT | 320 |
| 241 | TATGATGTTGAATT AGCGTTT | 320 |
| 321 | TCCACGAATGAGGGCCACTCCCATGGCCACCTCGAGAATCTGCTCCACGCTCTGAACCCGCGCGCCTTCCCACAGTGTCT | 400 |
| 321 | TttACGAATGAGGGttAtTttATGGttAttTCGAGAATtTGtTtACGtTtTGAAttCGCGCGttTTttAtAGTGTtT | 400 |
| 321 | TTTATGAATGA | 400 |
| 321 | TTTACGAAT | 400 |
| 401 | GGTACAGCCACATATTCCTCCGGCAGAGATTGAGGATGTGGCAGATAGTGGAGTTGG | 480 |
| 401 | **ATGTGGTAGATAGTGGAGTTGG** | 480 |
| 401 | GGTAtAGttAtATATTttTtCGGtAGAGATTGAGGATGTGGtAGATAGTGGAGTTGG | 480 |
| 401 | TATTTTTTCGGTAGAGAT | 480 |
| 401 | TTTTTTGGTAGAGATTG | |

Figure 80: Amplificate of SEQ ID NO: 60

1 GGGGTCTCAAGAGCTGGCTCCTACACATAAAGGGCTTAGAACAGTGGCTGGTACCTAGGCCACCAATAGTCTTTTGCCCC 80

1 **GGGGTTTTAAGAGTTGGTTTT** 80

1 GGGGTtTtAAGAGtTGGtTtTAtAtATAAAGGGtTTAGAAtAGTGGtTGGTAttTAGGttAttAATAGTtTTTGttt 80

---

81 ACCCAACCGGTGACACAGCCCGGGGCCCCGCCGCCCCCTGGCGGCCATTACGGATTTCCGCCTCCCTCACAGAAGGCAGT 160

81 AtttAAtCGGTGAtAtAGttCGGGGtttCGtCGttttTGGCGGttATTACGGATTTtCGttTttTtAtAGAAGGtAGT 160

81 GTTATTATGGATTTTTGTT 160

81 TATTACGGATTTTCGTT 160

---

161 CACTGCAACGTGCGTGGCCTCAGTTGCGTCATATCCGGCCCTTGCGATCAGGGCTTGAGGAACCCGCGCCATGAAGTGCG 240

161 tAtTGtAACGTGCGTGGttTtAGTTGCGTtATATtCGGtttTTGCGATtAGGGtTTGAGGAAttCGCGttATGAAGTGCG 240

161 AGTTGTGTTATATTTGGT TTGAGGAATTTGTGTTAT 240

161 TGCGTTATATTCGGTT AATTCGCGTTATGAAG 240

---

241 TGTTTGTTACCGTAGGGACCACCAGCTTTGACGACCTCATTGCGTGTGTGTCGGCGCCCGACAGTCTGCAAGTGAGTGAG 320

241 **TTGTAAGTGAGTGAG** 320

241 TGTTTGTTAtCGTAGGGAttAttAGtTTTGACGAttTtATTGCGTGTGTGTCGGCGttCGAtAGTtTGtAAGTGAGTGAG 320

241 TGTTGGTGTTTGATAGT 320

241 GTCGGCGTTCGATAGT

---

321 GGAGG 400

321 **GGAGG** 400

321 GGAGG 400

Figure 81: AmplificatE of SEQ ID NO: 61

1 TGGGGACACTGGATGTTTTTCCTCTGAGGCCGTGGAGGTTCACATCCCTGCCGAGGTGTGGGTGGCCCCTTTTCCGCT 80
GG

1 **TGCGGATATTGGATGTTTTT** 80

1 TGGGGAtAtTGGATGTTTTTttTtTGAGGtCGTGGAGGTTtAtATttTGtCGAGGTGTGGGTGGtttTTTTtCGtTGG 80

81 TAAACAATCCCACACCTGGGGCTGTGCTTCTCCCCAGGGCGAGGCTACTGTGCCGTTTTCCTGGGCTGCTCAAAAGCT 16
GG 0

81 TAAAtAATtttAtAttTGGGGtTGTGtTTtTtttAGGGCGAGGtTAtTGTGtCGTTTTttTGGGtTGtTtAAAAGtTGG 16
0

81 TTTAGGGCGAGGTTAT 16
0

81 TTTAGGGTGAGGTTAT 16
0

16 GGGCACTCTTGTGGGGCCGTCTGTCTTGCTGCTGCCTCTCCGGGGCAAGCTGTTAGAGCAGCCCCACCCGCAAACCT 24
1 GCA 0

16 GGGtAtTtTTGTGGGGtCGTtTGTtTTGtTGtTGttTtTCGGGGtAAGtTGTTAGAGtAGttttAttCGtAAAttTGtA 24
1 0

24 AACCAGGCTGGCCCCACAGAACCTGAAAACCTGCAAACCGGGCTGGCCCCACAGAACCCACAAACCTGCAGACTGG 32
1 GCTG 0

24 AAttAGGtTGGttttAtAGAAttTGAAAAttTGtAAAtCGGGtTGGttttAtAGAAtttAtAAAttTGtAGAtTGGGtTG 32
1 0

32 GCCCCACAGAACCCGCAAACGGGGGCTGGCCCCACAGAACCCGCCTTTGCAGCTCAGTGCTGAATGTTCTCGTCCCC 40
1 GGT 0

32 GttttAtAGAAttCGtAAACGGGGGtTGGttttAtAGAAttCGttTTTGtAGtTtAGTGtTGAATGTTtTCGTttCGGT 40
1 0

32 AGAATTCGTAAACGGG 40
1 0

32 ATTTGTAAATGGGGGT 40
1 0

40 GGTACCCTGGCCTGAGAGAGGAAGGGCTCTGGGAATTCGGTCACCGACGGCTGCACACCCCGTAGGGTAGGTGGGT 48
1 TCTG 0

40 **G** 48
1 0

40 GGTAtttTGGttTGAGAGAGGAAGGGtTtTGGGAATTCGGTtAtCGACGGtTGtAtAttCGTAGGGTAGGTGGGTTtTG 48
1 0

40 TTCGGTTATCGACGGT 48
1 0

40 TTTGGTTATTGATGGTT 48
1 0

40 TTGTATATTTTGTAGGGT 48
1 0

40 TGTATATTTCGTAGGGT
1

48 GAGGCCAGGCTGGAAGG 56
1 0

48 **GAGGTTAGGTTGGAAGG** 56
1 0

48 GAGGttAGGtTGGAAGG 56
1 0

Figure 82: Amplificate of SEQ ID NO: 62

342 GGAGCTTCAATTCCTGGGACCCCTCCTACTGCGGGGAGAGTGGTTTCCCTGTCCCCAGAGGATGCTCCAGGCCCGAGTCT 263

342 **GGAGCTTTTAATTTTTGGGATTT** 263

342 GGAGtTTtAATTttTGGGAtttTtTAtTGCGGGGAGAGTGGTTTttTGTtttAGAGGATGtTtAGGttCGAGTtT 263

342 AGGTTCGAGTTT 263

342 TAGGTTTGAGTTT 263

262 GCGGCGCTCTGGGGGATGCTCTCCGAGCTCCGACACCGTGTTCGGACCGGGTGCGCCCGCTGCCGCTGGGGCTGAAGCCT 183

262 GCGGCGtTtTGGGGGATGtTtTtCGAGtTtCGAtAtCGTGTTCGGAtCGGGTGCGttCGtTGtCGtTGGGGtTGAAGttT 183

262 GCGG ATCGTGTTCGGATCGG 183

262 GTGG TATTGTGTTTGGATTGG 183

182 GCAGGCGTGAGAACCGGGGGACTCTCTATGGCACCAGGAGCTTCACCGTGAGCGTAGCGCAGAAGCGCTTCGCTTTGATC 103

182 GtAGGCGTGAGAAtCGGGGGAtTtTtTATGGtAttAGGAGtTTtAtCGTGAGCGTAGCGtAGAAGCGtTTCGtTTTGATt 103

182 TAGGCGTGAGAATCGG ATTGTGAGTGTAGTGTA 103

182 TAGGTGTGAGAATTGG ATCGTGAGCGTAGCGT 103

102 CTAGCGCTTACAAAAGTCGTCCTTTGGCTGCCCATGATGGTAAAAGTGCAGTTGCTCACAAAGCGCGAGTGTGTGTGCCA 23

102 **A** 23

102 tTAGCGtTTAtAAAAGTCGTttTTTGGtTGttATGATGGTAAAAGTGtAGTTGtTtAtAAAGCGCGAGTGTGTGTGttA 23

102 TATAAAGTGTGAGTGTG 23

102 TATAAAGCGCGAGTGT

22 GACAGTGTAAATGAGTGTTGGG -57

22 **GATAGTGTAAATGAGTGTTGGG** -57

22 GAtAGTGTAAATGAGTGTTGGG -57

Figure 83: Amplificate of SEQ ID NO: 8

---

1 GGGAGTTTTTAAGCTCTGTGAGAATCCTGGGAGTTGGTGATGTCAGACTAGTTGGGTCATTTGAAGGTTAGCAGCCC GGG 80

1 **GGGAGTTTTTAAGTTTTGTGAG** 80

1 GGGAGTTTTTAAGtTtTGTGAGAATttTGGGAGTTGGTGATGTtAGAtTAGTTGGGTtATTTGAAGGTTAGtAGttCGGG 80

---

81 TAGGGTTCACCGAAAGTTCACTCGCATATATTAGGCAATTCAATCTTTCATTCTGTGTGACAGAAGTAGTAGGAAGT GAG 160

81 TAGGGTTtAtCGAAAGTTtAtTCGtATATATTAGGtAATTtAATtTTTtATTtTGTGTGAtAGAAGTAGTAGGAAGTGAG 160

---

161 CTGTTCAGAGGCAGGAGGGTCTATTCTTTGCCAAAGGGGGGACCAGAATTCCCCCATGCGAGCTGTTTGAGGACTGG GAT 240

161 tTGTTtAGAGGtAGGAGGGTtTATTtTTTGttAAAGGGGGGAttAGAATTttttATGCGAGtTGTTTGAGGAtTGGGAT 240

161 GGAT 240

161 **AT** 240

---

241 GCCGAGAACGCGAGCGATCCGAGCAGGGTTTGTCTGGGCACCGTCGGGGTAGGATCCGGAACGCATTCGGAAGGCT TTTT 320

241 GtCGAGAACGCGAGCGATtCGAGtAGGGTTTGTtTGGGtAtCGTCGGGGTAGGATtCGGAACGtATTCGGAAGGtTTTTT 320

241 GTTGAGAATGTGA TATCGTCGGGGTAGGA GAATGTATTTGGAAGGT 320

241 GTCGAGAACGCGAG TATTGTTGGGGTAGGA AACGTATTCGGAAGGT 320

241 AGCGATTCGAGTAGGG 320

241 AGTGATTTGAGTAGGG

---

321 GCAAGCATTTACTTGGAAGGAGAACTTGGGATCTTTCTGGGAACCCCCCGCCCCGGCTGGATTGGCCGAGCAAGCCT GGA 400

321 GtAAGtATTTAtTTGGAAGGAGAAtTTGGGATtTTTtTGGGAAtttttCGtttCGGtTGGATTGGtCGAGtAAGttTGGA 400

---

401 AAATGGTAAATGATCATTTGGATCAATTACAGGCTTTTAGCTGGCTTGTCTGTCATAATTCATGATTCGGGGCTGGGA AA 480

401 **GGGTTGGGAAA** 480

401 AAATGGTAAATGATtATTTGGATtAATTAtAGGtTTTTAGtTGGtTTGTtTGTtATAATTtATGATTCGGGGtTGGGAAA 480

---

481 AAGACCAA 560

481 **AAGATTAA** 560

481 AAGAttAA 560

---

Figure 84: Amplificate of SEQ ID NO: 9

1 AGAGGGGGTAGTCCCCACTCTCCTGTCTGATCCCTCCCTCTCCTCCCCGAGTTCCACCGCCCCAGGCGCACAGGTTTCCG 80
1 **AGAGGGGGTAGTTTTTATTTTT** 80
1 AGAGGGGGTAGTtttAtTtTttTGTtTGATttTttTtTttTttCGAGTTtAtCGtttAGGCGtAtAGGTTTtCG 80
1 TTTATTGTTTTAGGTGTAT 80
1 TATCGTTTTAGGCGTA 80

---

81 CCAGATGTCTTTTCTTCTTCGCAGTCTTTGCCCGAGCGCTTCCGAGAGCCAGTTCTGGACTGATCGCCTTGGATGGGATA 160
81 ttAGATGTtTTTTtTTtTTCGtAGTtTTTGttCGAGCGtTTtCGAGAGttAGTTtTGGAtTGATCGttTTGGATGGGATA 160
81 GAGTGTTTTTGAGAGTT 160
81 AGCGTTTTCGAGAGTT 160

---

161 CCGGGGGAGGGCAGAAGGACACTTGGCTTCCTCTCCAGGAATCTGAGCGGCCCTGAGGTCCGGGGGCGCAGGGAATCCCC 240
161 tCGGGGGAGGGtAGAAGGAtAtTTGGtTTttTtTtAGGAATtTGAGCGGtttTGAGGTtCGGGGGCGtAGGGAATtttt 240
161 AATTGAGTGGTTTTGA 240
161 ATTTGAGCGGTTTTGA 240

---

241 TCTCCCGCCGCCGCCGCCGTGTCTGGTCTGTACGTCTTTAGAGGGTCGAGGAAGTCACGTCGGGACAGACTGGGGCGAGT 320
241 TtTttCGtCGtCGtCGtCGTGTtTGGTtTGTACGTtTTTAGAGGGTCGAGGAAGTtACGTCGGGAtAGAtTGGGGCGAGT 320
241 GGAAGTTATGTTGGGA 320
241 TTACGTCGGGATAGAT

---

321 AAGGTTAAGAAAGGCTGACATGTT 400
321 **AAGGTTAAGAAAGGTTGATATGTT** 400
321 AAGGTTAAGAAAGGtTGAtATGTT 400

Figure 85: Amplificate of SEQ ID NO: 10

| | | |
|---|---|---|
| 1 | AGTGGGTAGGCCAAGTGTGTTGCTTCAGCAAACCGGACCAGGAGGGCCAGGGCCGGATGTGGGGACCCTCTTCCTCTAGC | 80 |
| 1 | **AGTGGGTAGGTTAAGTGTGTTG** | 80 |
| 1 | AGTGGGTAGGttAAGTGTGTTGtTTtAGtAAAtCGGAttAGGAGGGttAGGGtCGGATGTGGGGAtttTtTTttTtTAGt | 80 |
| 1 | AGTAAATCGGATTAGGA | 80 |
| 1 | AGTAAATTGGATTAGGAG | 80 |
| 81 | ACAGTAAAGCTGGCCTCCAGAAACACGGGTATCTCCGCGTGGTGCTTTGCGGTCGCCGTCGTTGTGGCCGTCCGGGGTGG | 160 |
| 81 | AtAGTAAAGtTGGttTttAGAAAtACGGGTATtTtCGCGTGGTGtTTTGCGGTCGtCGTCGTTGTGGtCGTtCGGGGTGG | 160 |
| 81 | ATATGGGTATTTTTGTGT | 160 |
| 81 | TACGGGTATTTTCGCGT | 160 |
| 161 | GGTGTGAGGAGGGGACGAAGGAGGGAAGGAAGGGCAAGGCGGGGGGGGCTCTGCGAGAGCGCGCCCAGCCCCGCCTTCGG | 240 |
| 161 | GGTGTGAGGAGGGGACGAAGGAGGGAAGGAAGGGtAAGGCGGGGGGGGtTtTGCGAGAGCGCGtttAGtttCGttTTCGG | 240 |
| 161 | GAGAGTGTGTTTAGTTT | 240 |
| 161 | AGAGCGCGTTTAGTTT | |
| 241 | GCCCCACAGTCCCTGCACCCAGGTTTCCATTGCGCGGCTCTCCTCAGCTCCTTCCCGCCGCCCAGTCTGGATCCTGGGG | 320 |
| 241 | **TTTAGTTTGGATTTTGGGG** | 320 |
| 241 | GtttAtAGTtttTGtAtttAGGTTTttATTGCGCGGtTtTttTtAGtTtTTttCGtCGttAGTtTGGATttTGGGG | 320 |

Figure 86: Amplificate of SEQ ID NO: 11

1 AGCTGGAGAAACTGAAAAGATCACAAGCGACTTAACGATAAGCCCCTTCTTCCTTTTAAAGACCGAGAGGAGGGTAGAGG 80

1 **AGTTGGAGAAATTGAAAAGATTA** 80

1 AGtTGGAGAAAtTGAAAAGATtAtAAGCGAtTTAACGATAAGttttTTtTTttTTTTAAAGAtCGAGAGGAGGGTAGAGG 80

1 ATAAGCGATTTAACGAT 80

1 AGTGATTTAATGATAAGTT 80

---

81 GGAGTAGTGCCTGAGCCCACGTGACCGAGCCAGGGAGCCCGACGGTCTCAGGAACGCCCGACGCCGCGCGTGACCTCTAA 160

81 GGAGTAGTGttTGAGtttACGTGAtCGAGttAGGGAGttCGACGGTtTtAGGAACGttCGACGtCGCGCGTGAttTtTAA 160

81 TTTAGGAATGTTTGATGT 160

81 TAGGAACGTTCGACGT 160

---

161 GTGGGAGCACCCTCGAACCGACTCCTGGTCCACCCACAAGGATAGTGGCGCACAGATGGCGCTCCCCGCAGCCCCAGTCT 240

161 GTGGGAGtAtttTCGAAtCGAtTttTGGTttAtttAtAAGGATAGTGGCGtAtAGATGGCGtTtttCGtAGttttAGTtT 240

161 TAGATGGTGTTTTTTGT 240

161 ATGGCGTTTTTCGTAG 240

---

241 CAGATTTAAGAGGTCTGGAGTAGGGCCTGAGAATATGCATTTCCAACCAGGTCCTGGGGGATGCCGACACTGATACAGCC 320

241 tAGATTTAAGAGGTtTGGAGTAGGGttTGAGAATATGtATTTttAAttAGGTttTGGGGGATGtCGAtAtTGATAtAGtt 320

---

321 AGTCTGGGGACCACACTTCGAGGATCACGTCCTCAGCCCCTGACTCACATAAGTGTCATTCAGAACAGATGTCTGATTCT 400

321 AGTtTGGGGAttAtAtTTCGAGGATtACGTttTtAGttttTGAtTtAtATAAGTGTtATTtAGAAtAGATGTtTGATTtT 400

321 ATATTTTGAGGATTATGTT 400

321 ATTTCGAGGATTACGT

---

401 AAGGAGCCAGTGGTGAAACCAGAGAGGCTTTGGGTTTGTCA 480

401 **TAGAGAGGTTTTGGGTTTGTTA** 480

401 AAGGAGttAGTGGTGAAAttAGAGAGGtTTTGGGTTTGTtA 480

---

Figure 87: Amplificate of SEQ ID NO: 12a

| | | |
|---|---|---|
| 1 | CCCAGCTGTGAAAAAGGGACCCAGGACCCGCAGATGCTGGCGAGGAACAGGGGTGCGGAGCATGAGTCGGCTTTGC ACCA | 80 |
| 1 | **TTTAGTTGTGAAAAAGGGATTT** | 80 |
| 1 | tttAGtTGTGAAAAAGGGAtttAGGAttCGtAGATGtTGGCGAGGAAtAGGGGTGCGGAGtATGAGTCGGtTTTGtAttA | 80 |
| 81 | CGCCCTGGGGTCTCCAGCACTGAAGCGTTCAGTACCATGCAGAGCAGCTGGGAGCGGGTCCCGCTGGGCGGGGCCG AGCT | 160 |
| 81 | CGtttTGGGGTtTttAGtAtTGAAGCGTTtAGTAttATGtAGAGtAGtTGGGAGCGGGTttCGtTGGGCGGGGtCGAGtT | 160 |
| 161 | GCGCGCGACCCTCGGCGCGCTCGGGGAGGCCCAGACAGGGGTGGCCTCTCTGGCCTCCGCTCCCGCGGGCTCTATGA CAC | 240 |
| 161 | GCGCGCGAtttTCGGCGCGtTCGGGGAGGttAGAtAGGGGTGGttTtTtTGGttTtCGtTttCGCGGGtTtTATGAtAt | 240 |
| 161 | AT | 240 |
| 161 | AT | 240 |
| 241 | CGCACTGGCTCGCGGGACGGGGCGGGGTCGGGTGAGGGGGAGGAGACAGCCCCACGCTTTGCGACTCGCGGTGACC CCTA | 320 |
| 241 | CGtAtTGGtTCGCGGGACGGGGCGGGGTCGGGTGAGGGGGAGGAGAtAGttttACGtTTTGCGAtTCGCGGTGAttttTA | 320 |
| 241 | CGTATTGGTTCGCGG AGTTTTATGTTTTGTGAT GTGATTTTTA | 320 |
| 241 | TGTATTGGTTTGTGG TTTACGTTTTGCGATT ATTTTTA | 320 |
| 321 | CGCGGAACTCTCTCGCGGTAATTCGAAGTACCGCGCCTGCGTGCTGCAGTAGCGCCTGGTGGCGGTGGCAGTTTGCC CGC | 400 |
| 321 | CGCGGAAtTtTtTCGCGGTAATTCGAAGTAtCGCGttTGCGTGtTGtAGTAGCGttTGGTGGCGGTGGtAGTTTGttCGC | 400 |
| 321 | TGTGGAAT ATTCGAAGTATCGCGT | 400 |
| 321 | CGCGGAATT ATTTGAAGTATTGTGTTT | |
| 401 | GGGTGTGTGAAGGGAGACAGTGTGGAGGCCACAGGG | 480 |
| 401 | **GAGATAGTGTGGAGGTTATAGGG** | 480 |
| 401 | GGGTGTGTGAAGGGAGAtAGTGTGGAGGttAtAGGG | 480 |

Figure 88: Amplificate of SEQ ID NO: 12b

---

497 GTCTTGGAGGGAGTAGAGACCCATCTTCAGCCTGCCTCACCCCCTTGGATAAAGAAAATGCCCCCTTTCTATCATTCAAA 418

497 **CCCTATAACCTCCACACTA** 418

497 GTtTTGGAGGGAGTAGAGAtttATtTTtAGttTGttTtAttttTTGGATAAAGAAAATGttttTTTtTATtATTtAAA 418

---

417 ATTATTTTTAGGTCGGTTTTGTTTACTCCGAACCCCGAGTAGAGCACATGGGGAGCCCAAAATAAGTGCATACTTGAATG 338

417 **TCTC** 338

417 ATTATTTTTAGGTCGGTTTTGTTTAtTtCGAAtttCGAGTAGAGtAtATGGGGAGtttAAAATAAGTGtATAtTTGAATG 338

417 TTTGAATTTTGAGTAGAG 338

417 ATTTCGAATTTCGAGTA 338

---

337 GATAATTCCCAGAGAACTGCTGCTTGCAACATAGTTTGTAATACCAAAAAATTGAAATCTATATTAACACCCATCAGTAG 258

337 GATAATTtttAGAGAAtTGtTGtTTGtAAtATAGTTTGTAATAttAAAAAATTGAAATtTATATTAAtAttATtAGTAG 258

---

257 GAAAATGGATAAATGAATAATGCCTCTATCCTGTTTTTGAAATATGATTACAGCCGTTAAAACGTAGATCTGTGGTCTGA 178

257 GAAAATGGATAAATGAATAATGttTtTATttTGTTTTTGAAATATGATTAtAGtCGTTAAAACGTAGATtTGTGGTtTGA 178

257 AGTTGTTAAAATGTAGATT 178

257 TAGTCGTTAAAACGTAG 178

---

177 CTGGCTACCCAGGACATGTTAAATGGGAAAACAACTAGCAGAAGGGCACCTACGTAGTGATACCATTTATGTTATTTTTA 98

177 tTGGtTAtttAGGAtATGTTAAATGGGAAAAtAAtTAGtAGAAGGGtAttTACGTAGTGATAttATTTATGTTATTTTTA 98

---

97 AAATTAAATGTACATTTATATAGGTGTACGTGTATGAACGTGCAGTTTAAAGGCCTAAACAGAAAAATACATCAGAAGCT 18

97 **AAATC** 18

97 AAATTAAATGTAtATTTATATAGGTGTACGTGTATGAACGTGtAGTTTAAAGGttTAAAtAGAAAAATAtATtAGAAGtT 18

97 TGTATGAACGTGTAGTT 18

97 GTGTATGAATGTGTAGT

---

17 CTTAACTGTGGGATGGG -62

17 **CCTTTTTCACAACTAAA** -62

17 tTTAAtTGTGGGATGGG -62

---

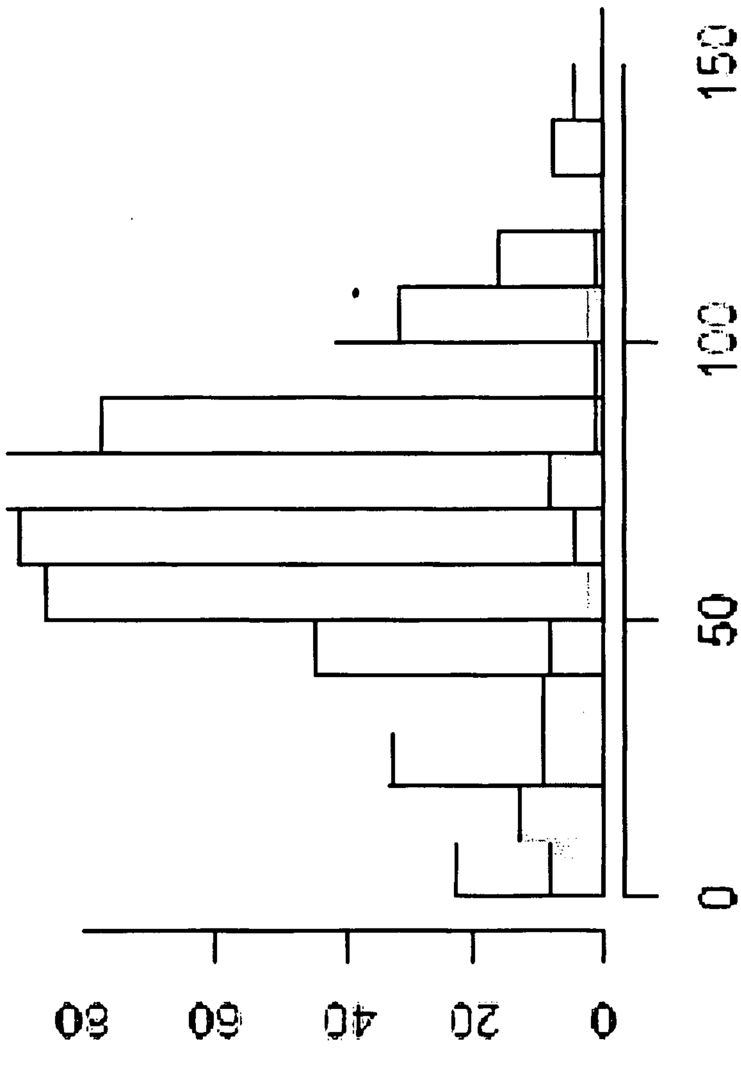


Figure 89

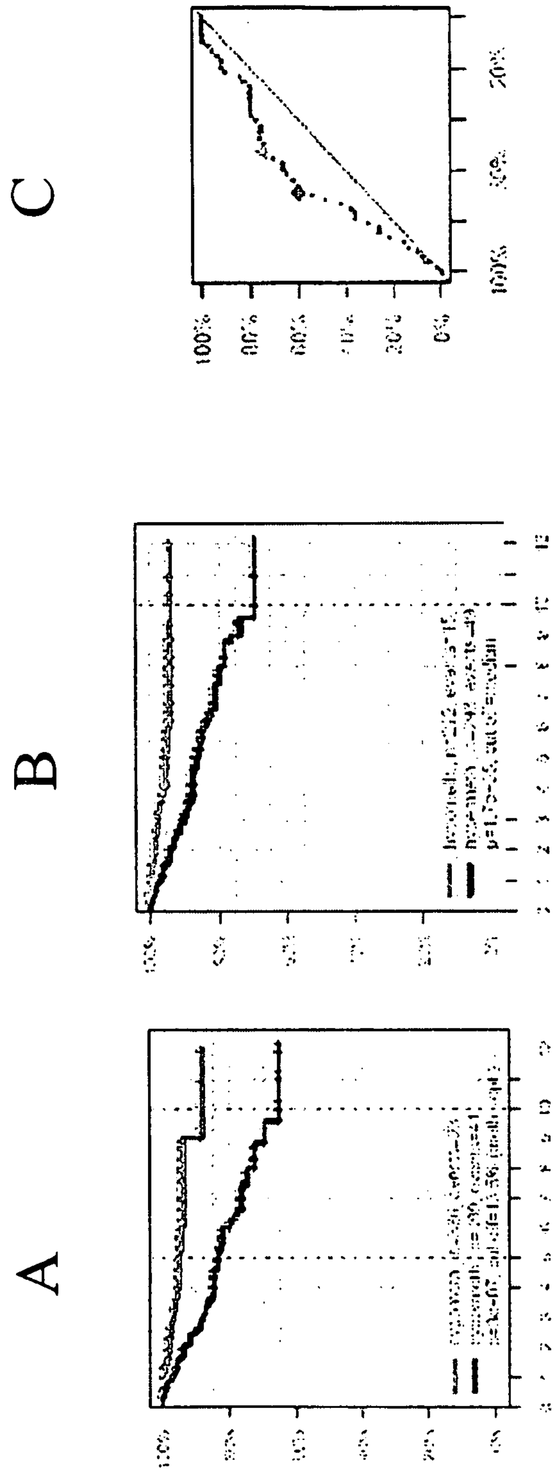

Figure 90

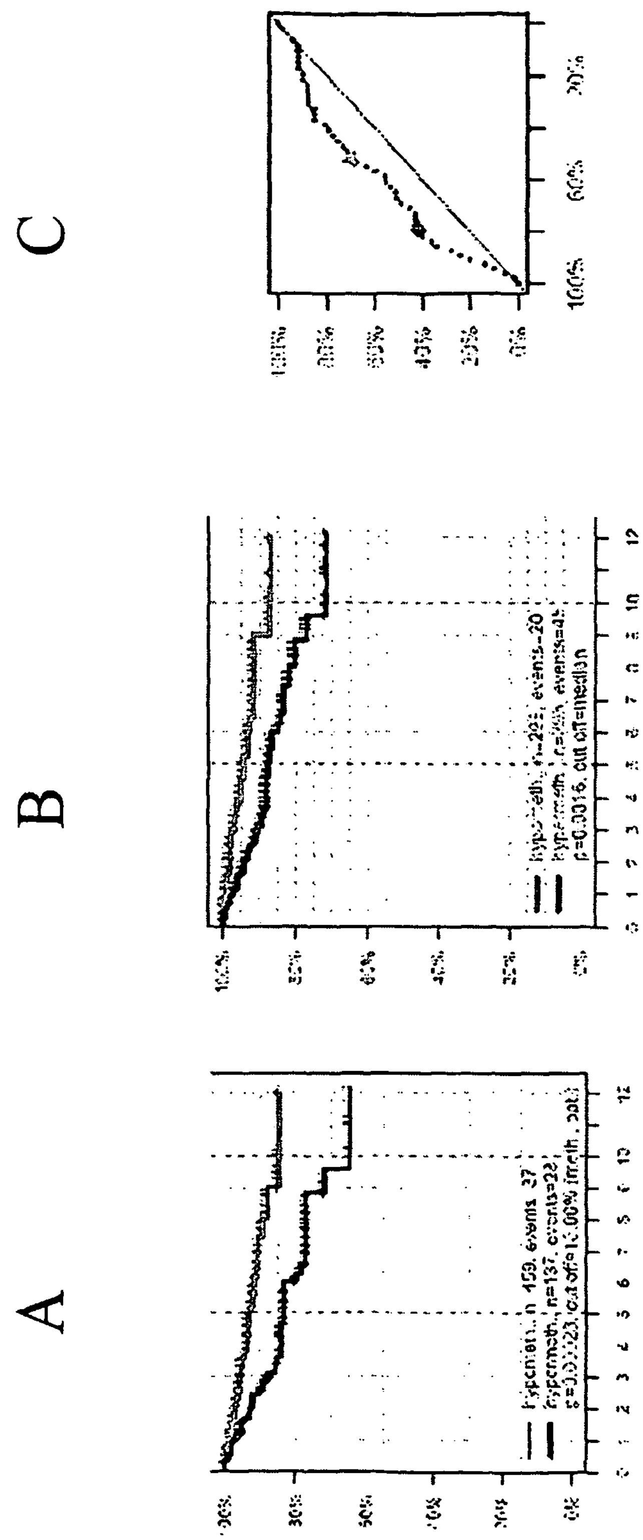


Figure 91

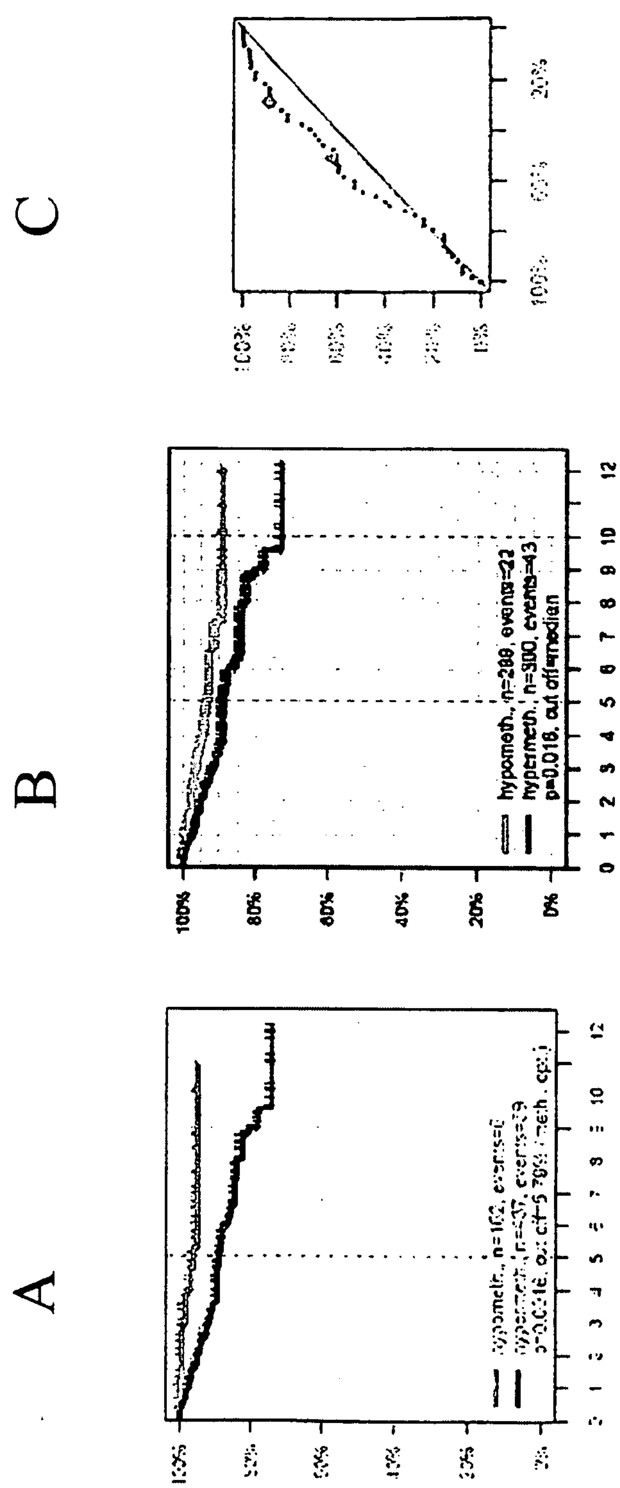
A
B
C
hypometh., n=288, events=22
hypermeth., n=300, events=43
p=0.016, cut off=median

Figure 92

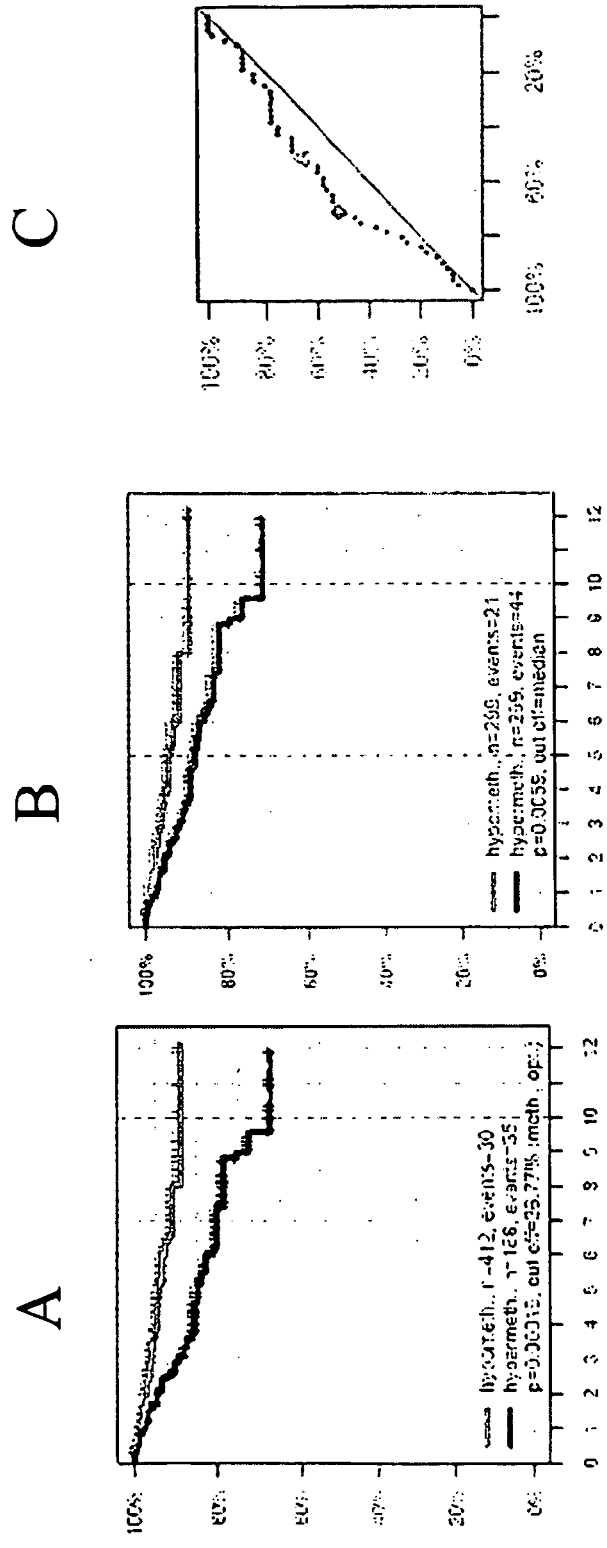
A
B
C
hypometh., n=299, events=21
hypermeth., n=299, events=44
p=0.0059, cut off=median

Figure 93

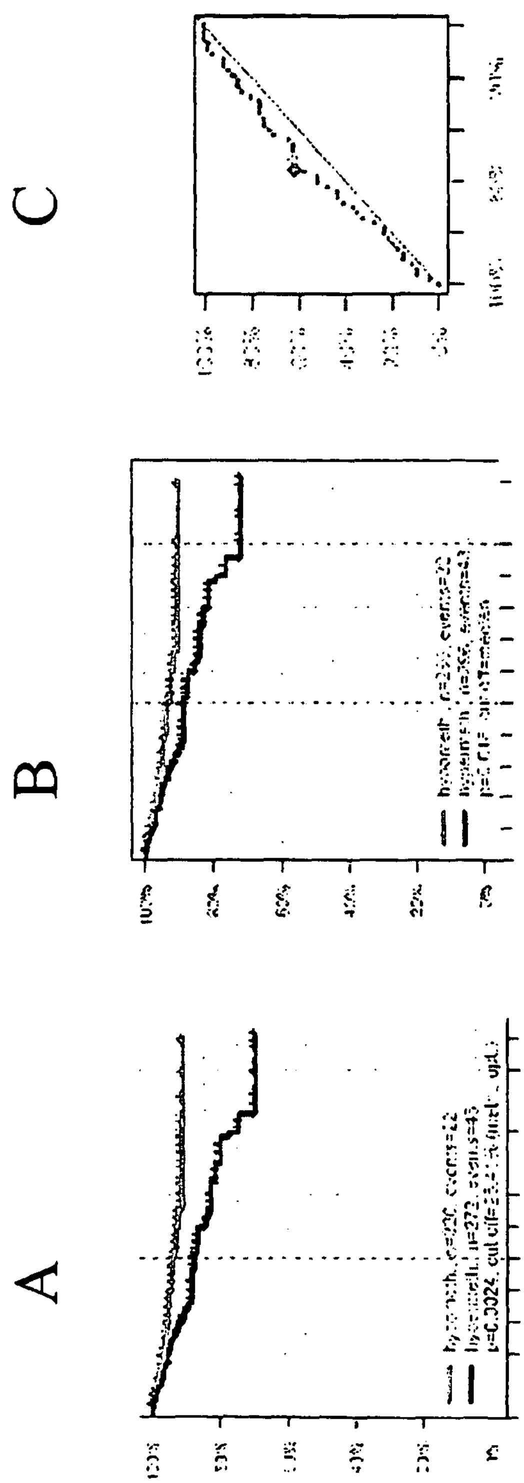

Figure 94

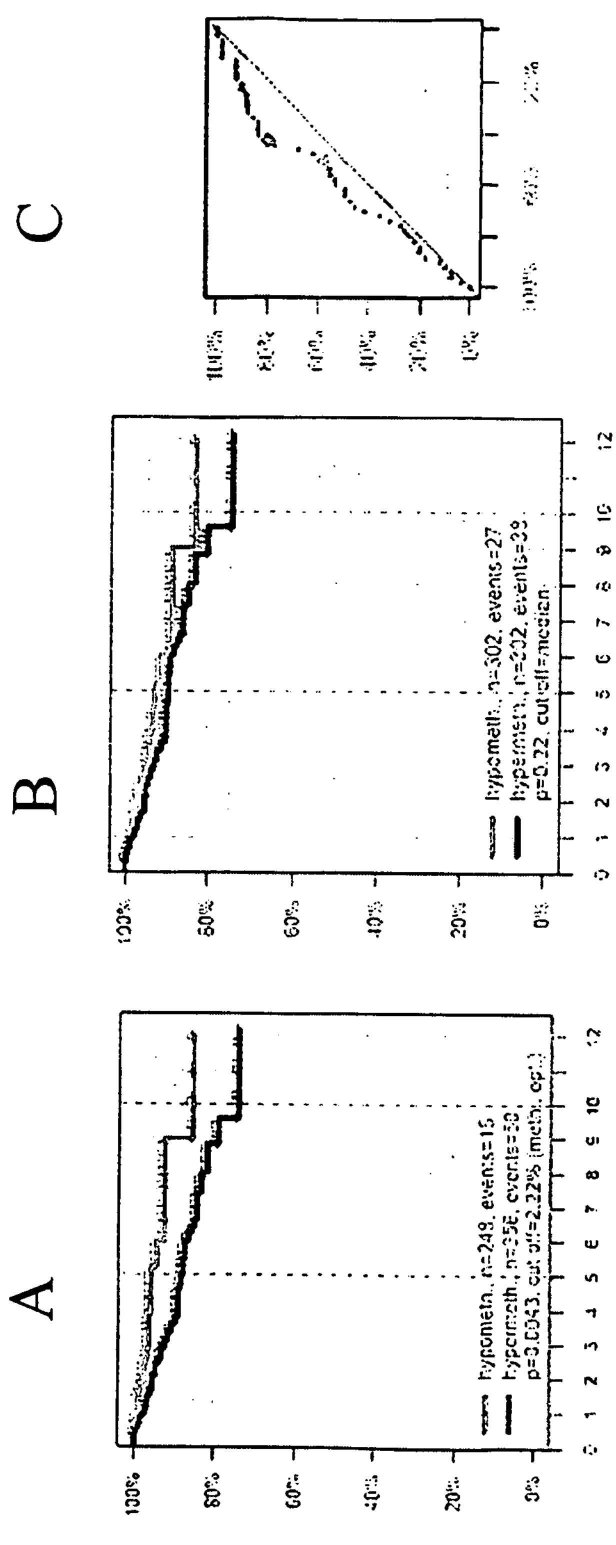
A
B
C
hypometh., n=249, events=15
hypermeth., n=256, events=50
p=0.0043, cut off=2.22% (meth. opt.)
hypometh., n=302, events=27
hypermeth., n=202, events=39
p=0.22, cutoff=median
100%
80%
60%
40%
20%
0%

Figure 95

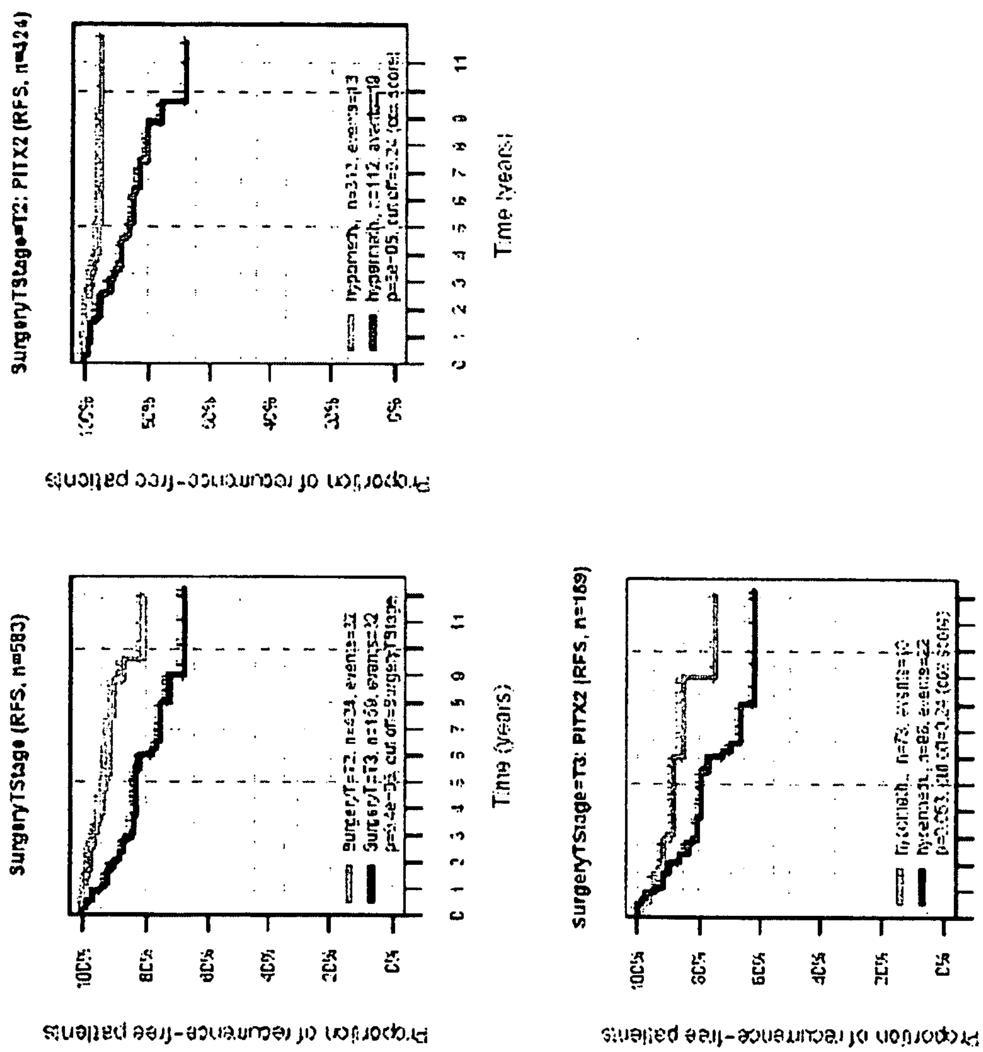
SurgeryTStage (RFS, n=583)
SurgeryTStage=T2: PITX2 (RFS, n=424)
SurgeryTStage=T3: PITX2 (RFS, n=169)
Proportion of recurrence-free patients
Time (years)

Figure 96

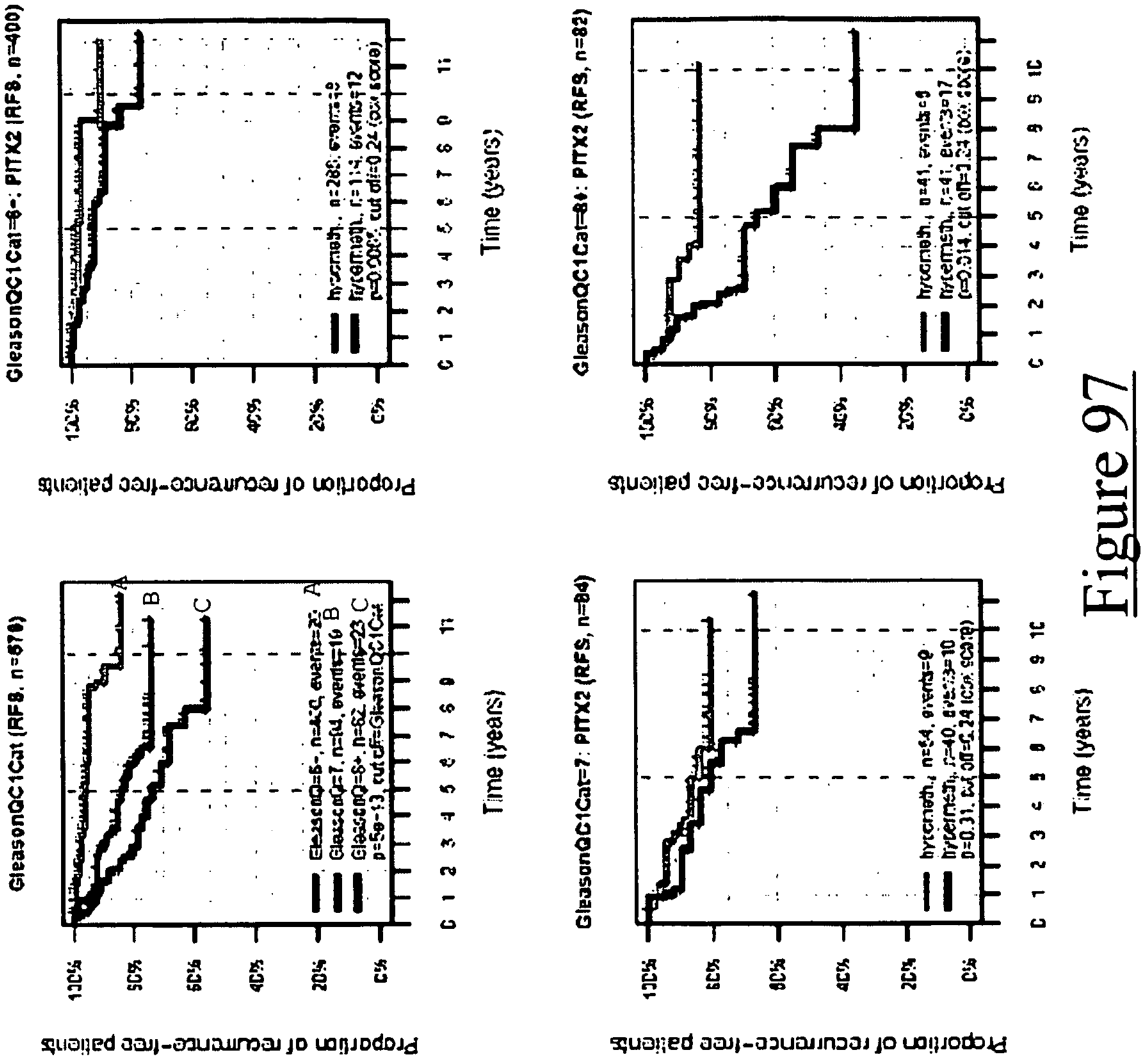
GleasonQC1Cat (RFS, n=576)
GleasonQC1Cat=6-: PITX2 (RFS, n=400)
GleasonQC1Cat=7: PITX2 (RFS, n=84)
GleasonQC1Cat=8+: PITX2 (RFS, n=82)
Proportion of recurrence-free patients
Time (years)
A
B
C

Figure 97

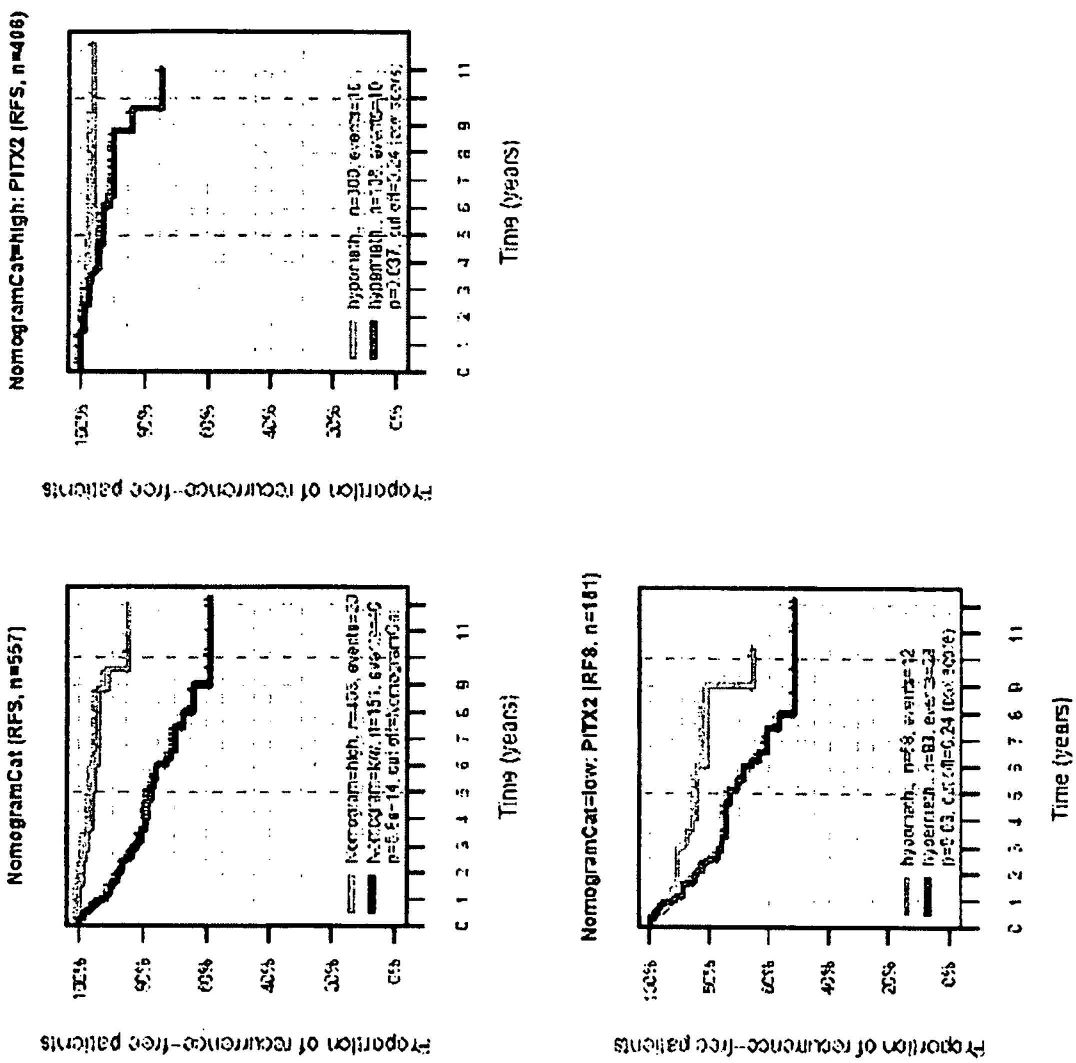
NomogramCat (RFS, n=557)
NomogramCat=high: PITX2 (RFS, n=406)
NomogramCat=low: PITX2 (RFS, n=181)
Proportion of recurrence-free patients
Time (years)

Figure 98

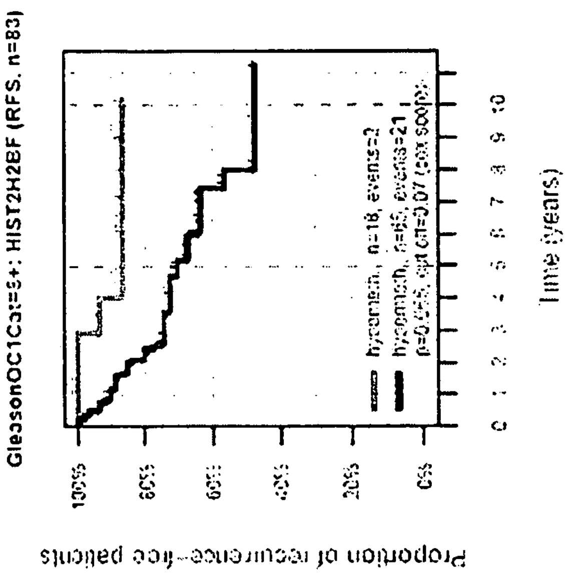
GleasonOC1Cat=3+; HIST2H2BF (RFS, n=83)
Proportion of recurrence-free patients
Time (years)
0 1 2 3 4 5 6 7 8 9 10

Figure 99

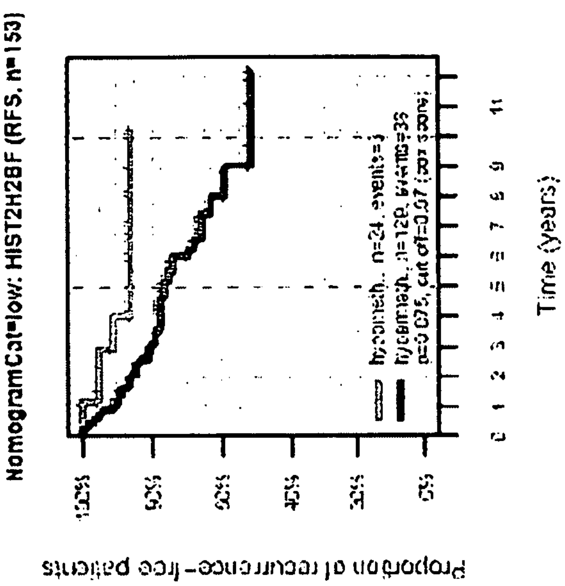
NomogramCat=low: HIST2H2BF (RFS, n=153)
Proportion of recurrence-free patients
Time (years)

Figure 100

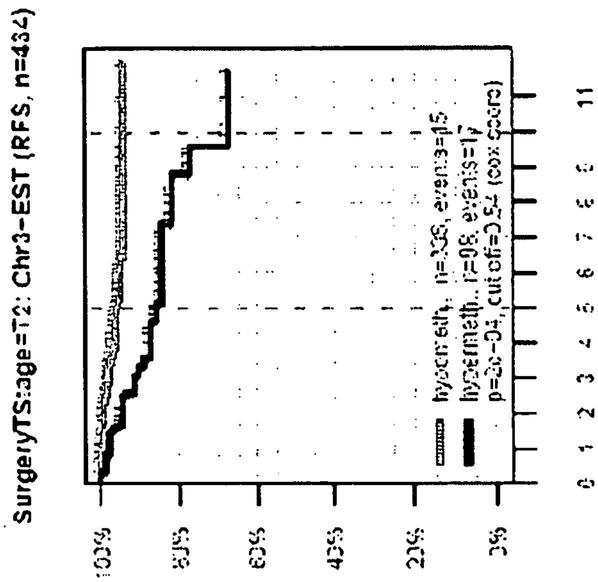


Figure 101

Figure 102

Binary Distribution

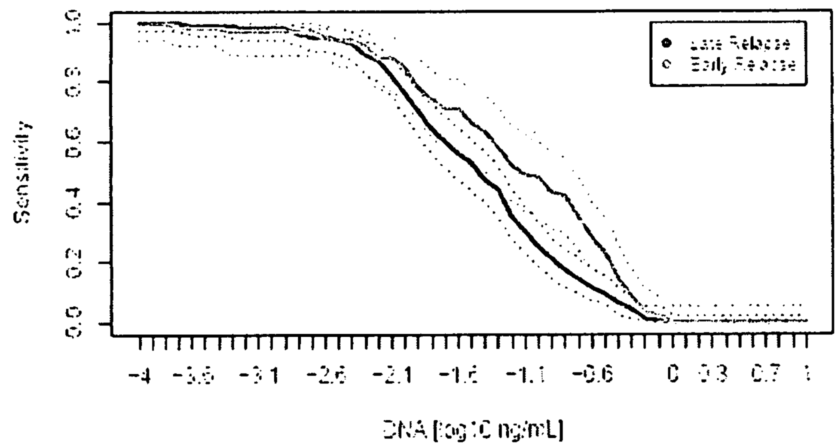


ROC Plot

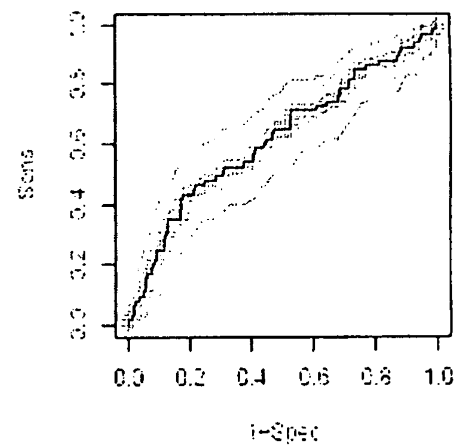


Multiclass Distribution

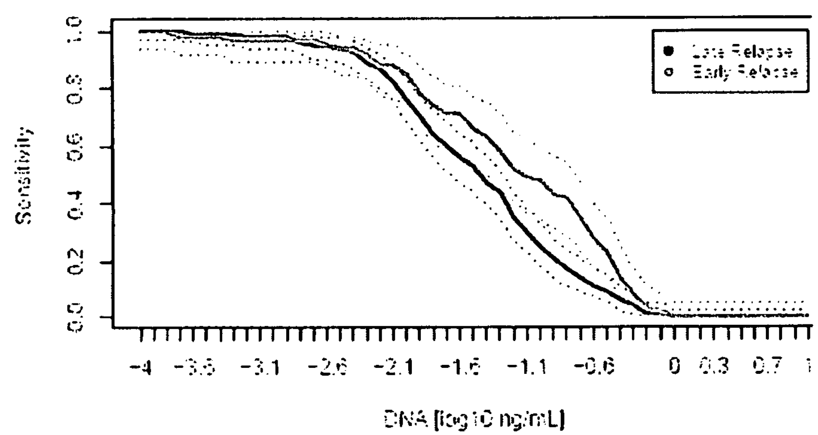


| | |
|---|---|
| AUC | 0.62 [0.54,0.68] |
| Sens / Spec | 0.35 / 0.86 |
| Wilcoxon *P* | 0.0072 |
| Early Relapse (pos) | 65 |
| Late Relapse (neg) | 140 |

Figure 103

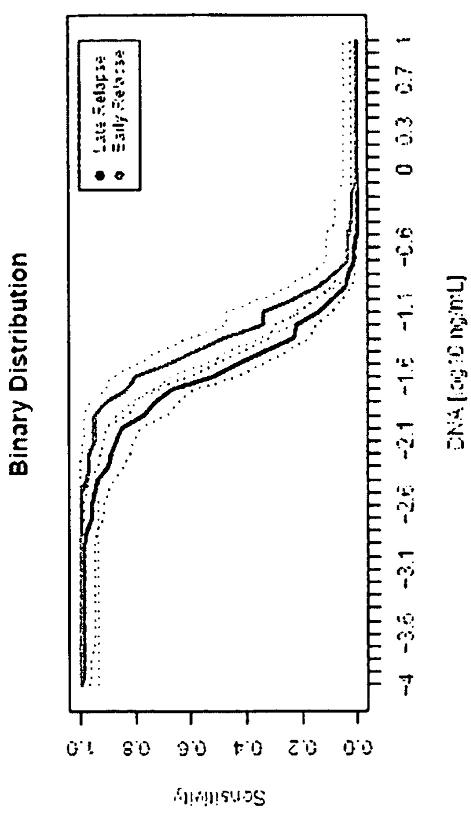


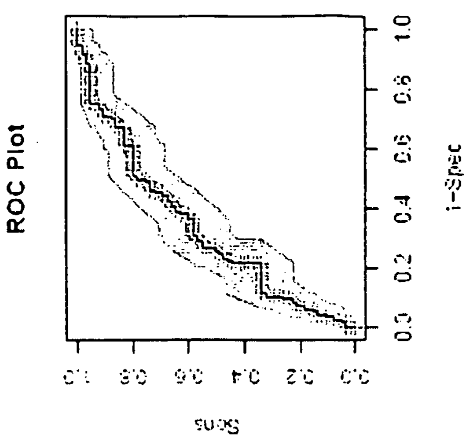


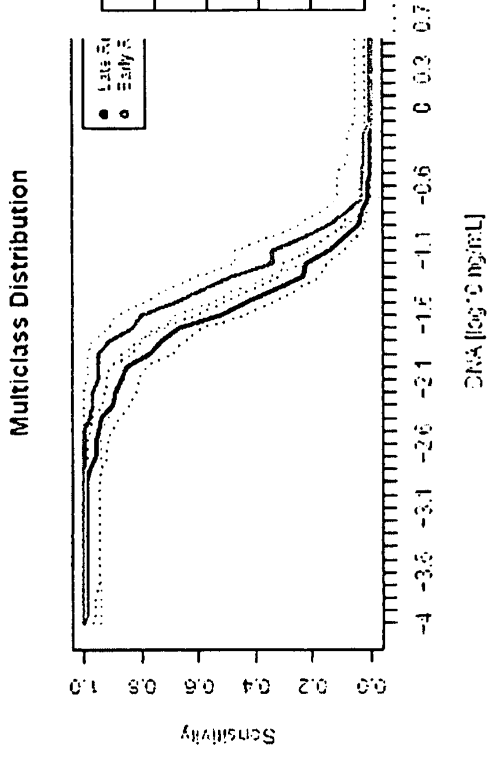


| | |
|---|---|
| AUC | 0.68 [0.61,0.74] |
| Sens / Spec | 0.34 / 0.85 |
| Wilcoxon *P* | 1e-04 |
| Early Relapse (pos) | 65 |
| Late Relapse (neg) | 141 |

Figure 104

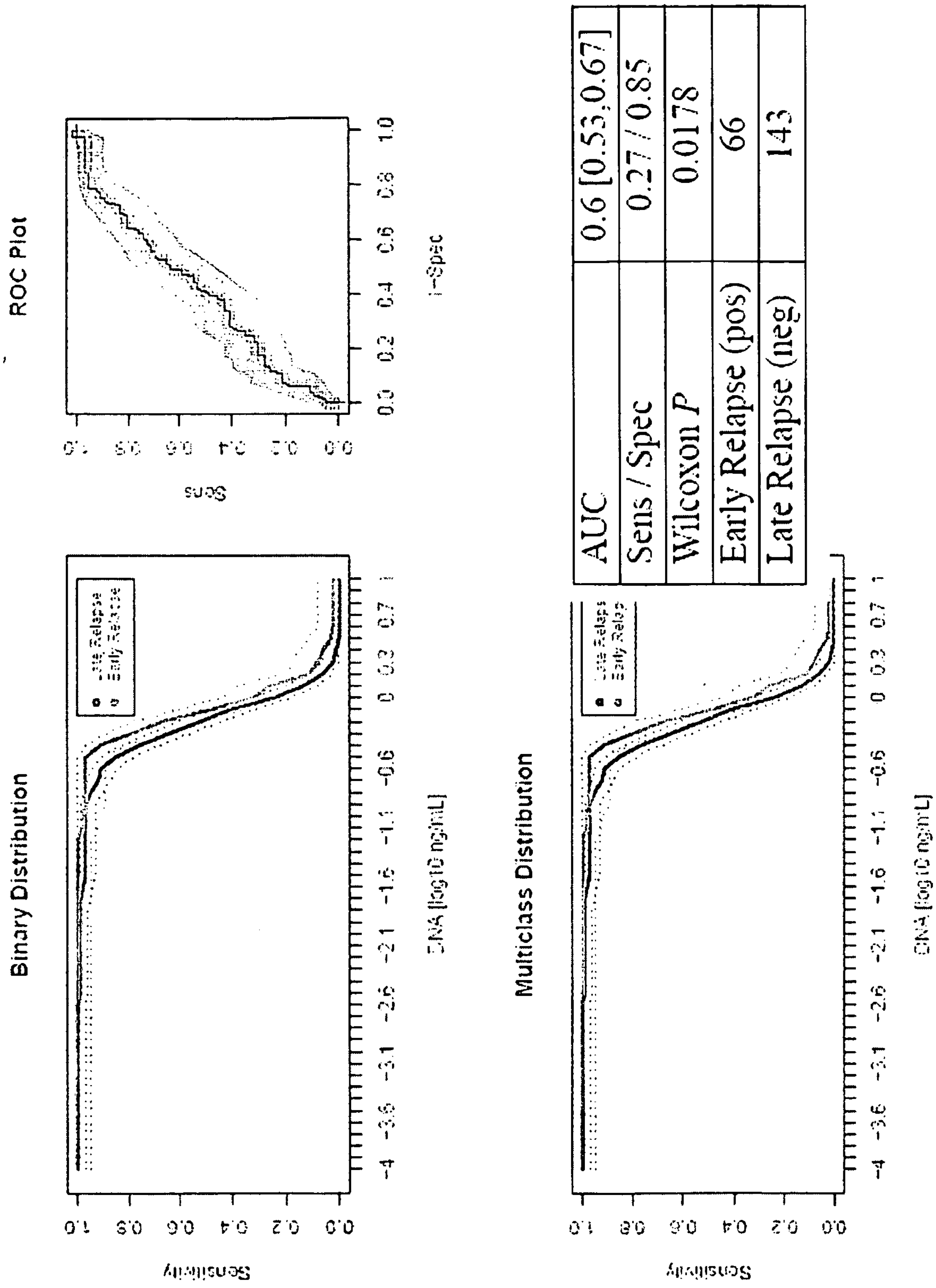


| | |
|---|---|
| AUC | 0.6 [0.53,0.67] |
| Sens / Spec | 0.27 / 0.85 |
| Wilcoxon *P* | 0.0178 |
| Early Relapse (pos) | 66 |
| Late Relapse (neg) | 143 |

Figure 105

Binary Distribution

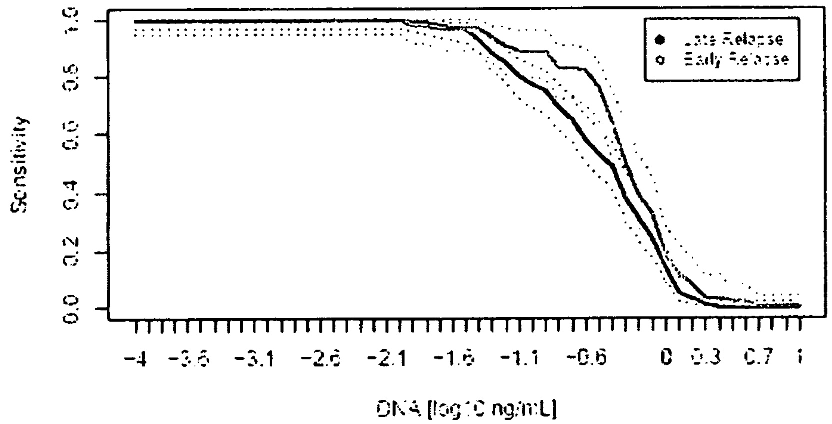


ROC Plot

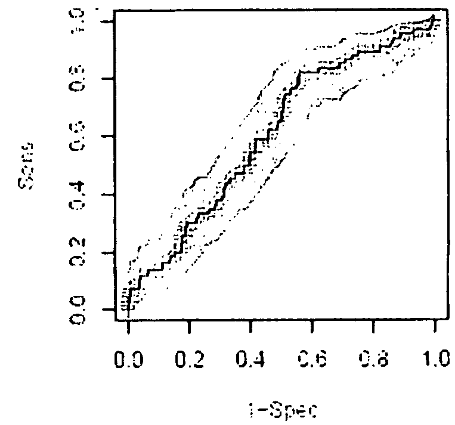


Multiclass Distribution

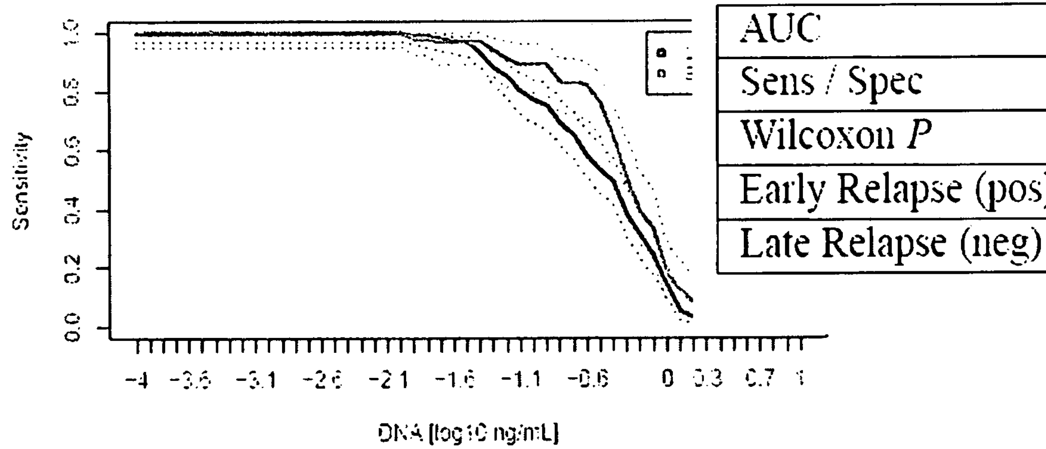


| | |
|---|---|
| AUC | 0.61 [0.53,0.67] |
| Sens / Spec | 0.18 / 0.85 |
| Wilcoxon *P* | 0.0146 |
| Early Relapse (pos) | 66 |
| Late Relapse (neg) | 143 |

Figure 106

Binary Distribution

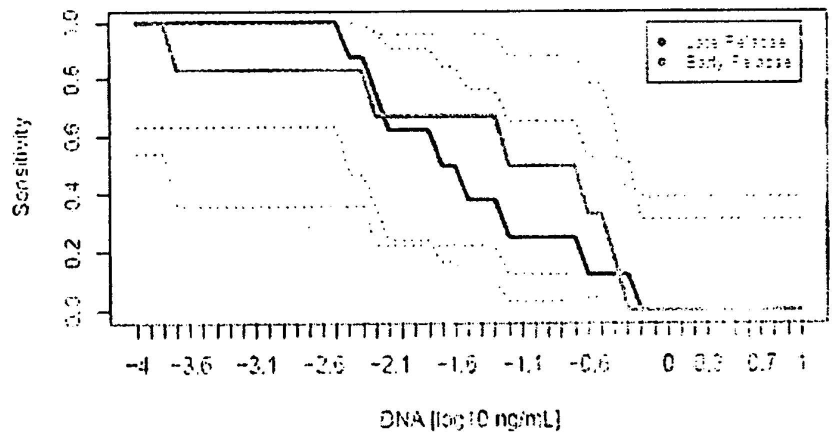


ROC Plot

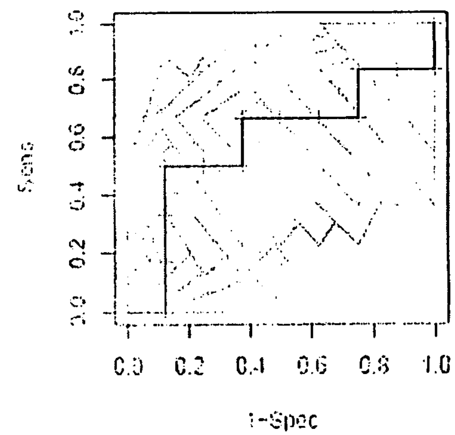


Multiclass Distribution

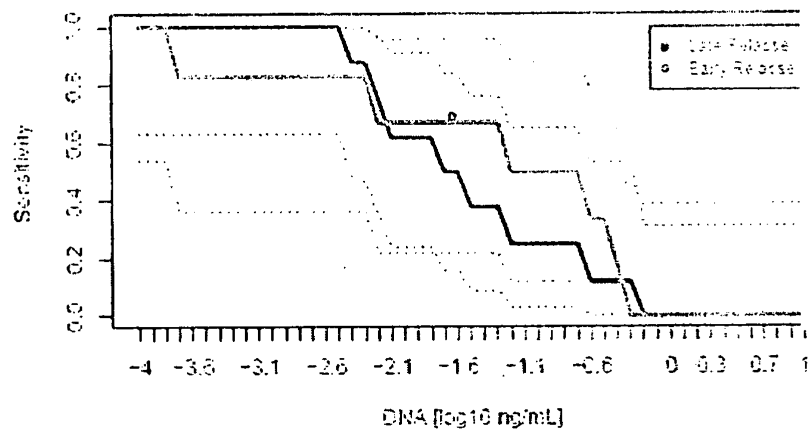


| | |
|---|---|
| AUC | 0.58 [0.29,0.82] |
| Sens / Spec | 0.5 / 0.88 |
| Wilcoxon *P* | 0.662 |
| Early Relapse (pos) | 6 |
| Late Relapse (neg) | 8 |

Figure 107

Binary Distribution

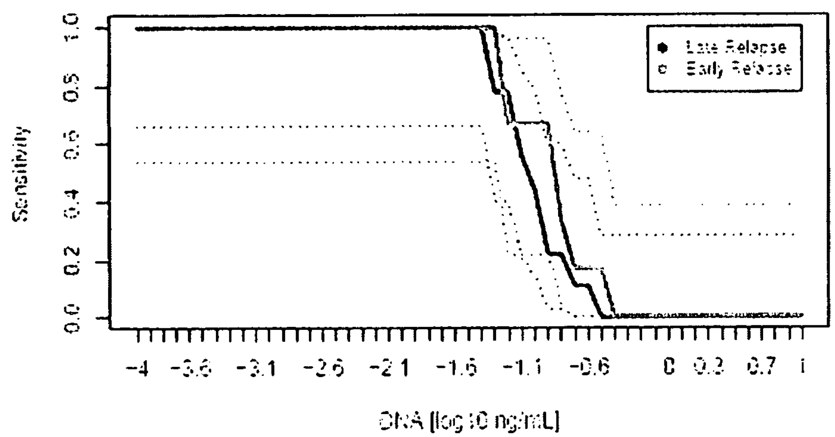


ROC Plot

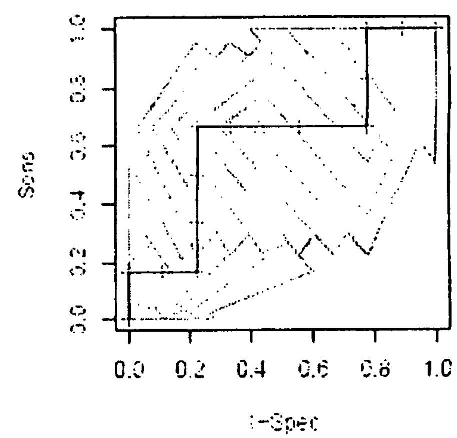


Multiclass Distribution

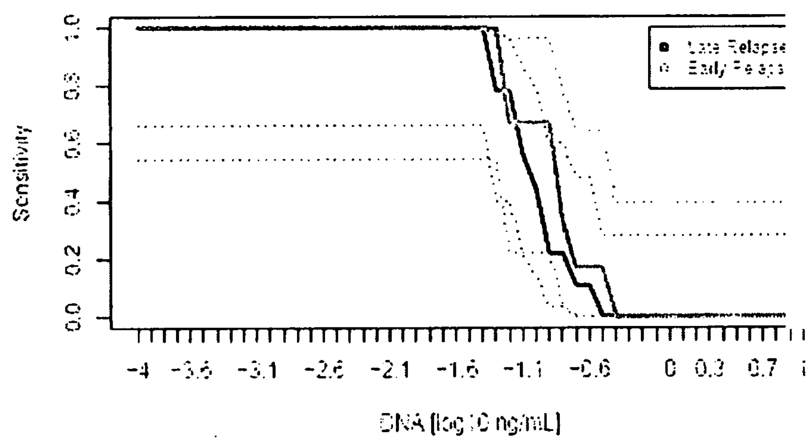


| | |
|---|---|
| AUC | 0.63 [0.32,0.84] |
| Sens / Spec | 0.17 / 0.89 |
| Wilcoxon *P* | 0.4559 |
| Early Relapse (pos) | 6 |
| Late Relapse (neg) | 9 |

Figure 108

Binary Distribution

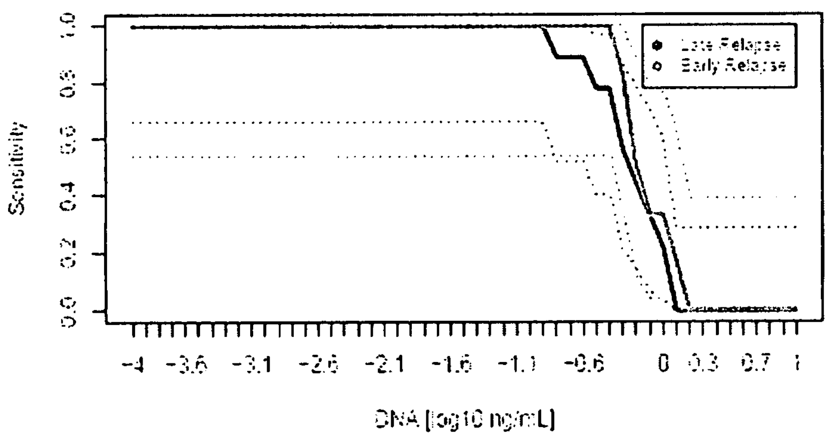


ROC Plot

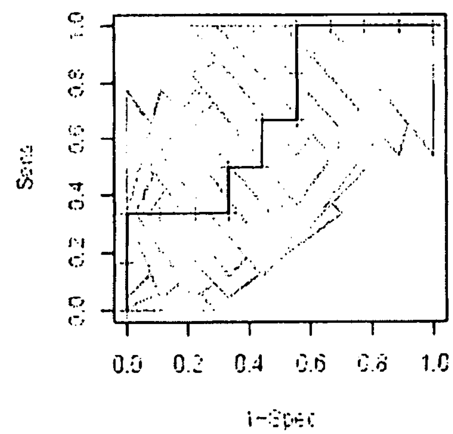


Multiclass Distribution

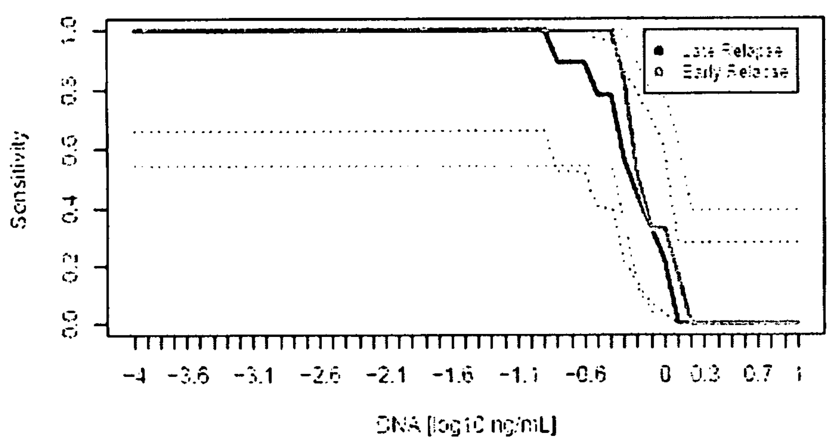


| | |
|---|---|
| AUC | 0.69 [0.38.0.88] |
| Sens / Spec | 0.33 / 0.89 |
| Wilcoxon *P* | 0.2721 |
| Early Relapse (pos) | 6 |
| Late Relapse (neg) | 9 |

Figure 109

Binary Distribution

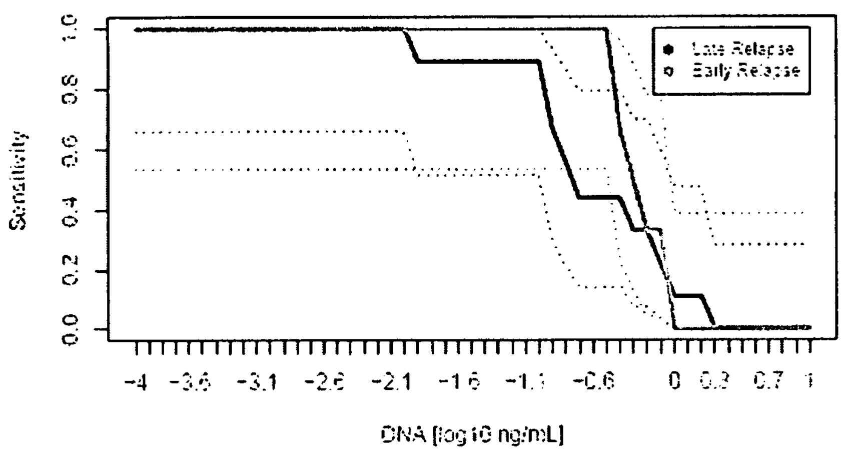


ROC Plot

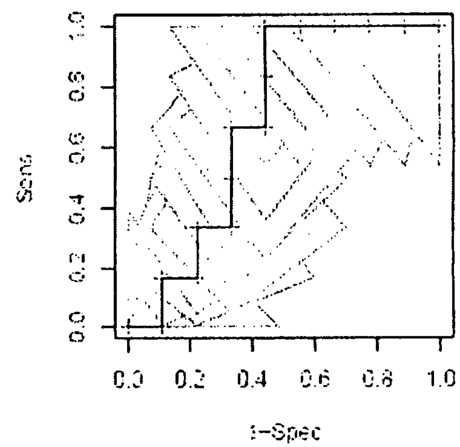


Multiclass Distribution

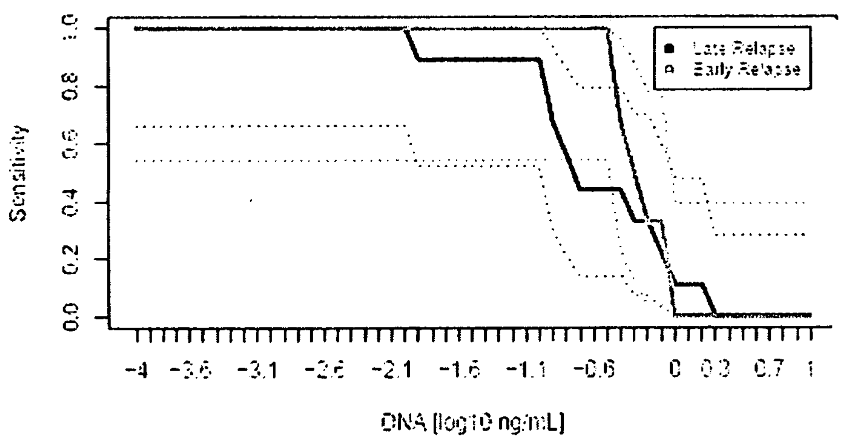


| | |
|---|---|
| AUC | 0.69 [0.38,0.88] |
| Sens / Spec | 0.17 / 0.89 |
| Wilcoxon *P* | 0.2721 |
| Early Relapse (pos) | 6 |
| Late Relapse (neg) | 9 |

Figure 110

Binary Distribution

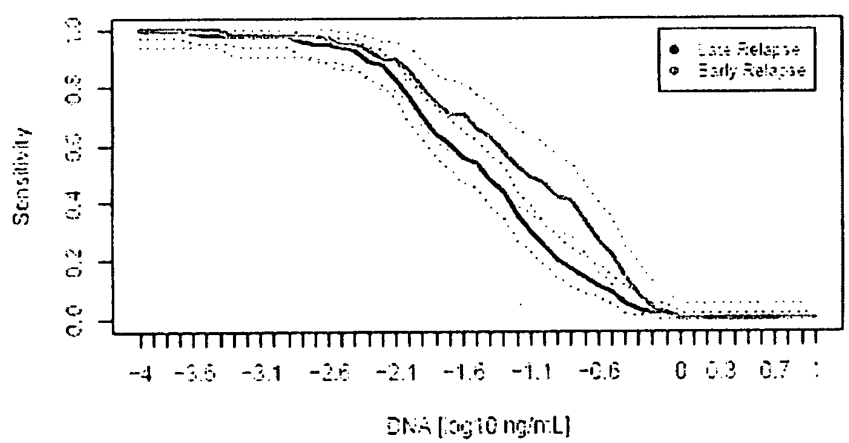


ROC Plot

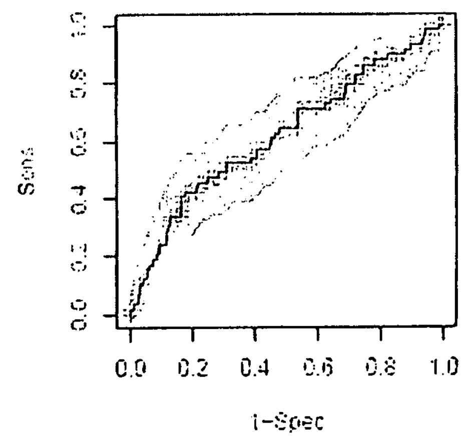


Multiclass Distribution

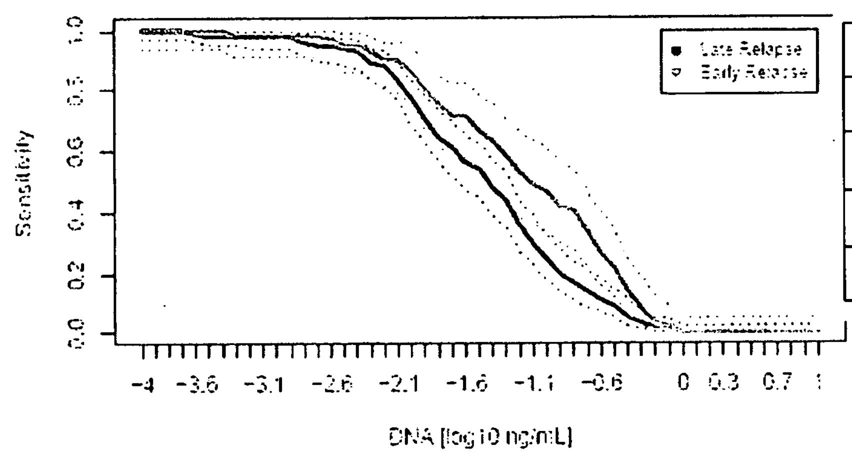


| | |
|---|---|
| AUC | 0.62 [0.54,0.69] |
| Sens / Spec | 0.34 / 0.86 |
| Wilcoxon *P* | 0.0085 |
| Early Relapse (pos) | 59 |
| Late Relapse (neg) | 132 |

Figure 111

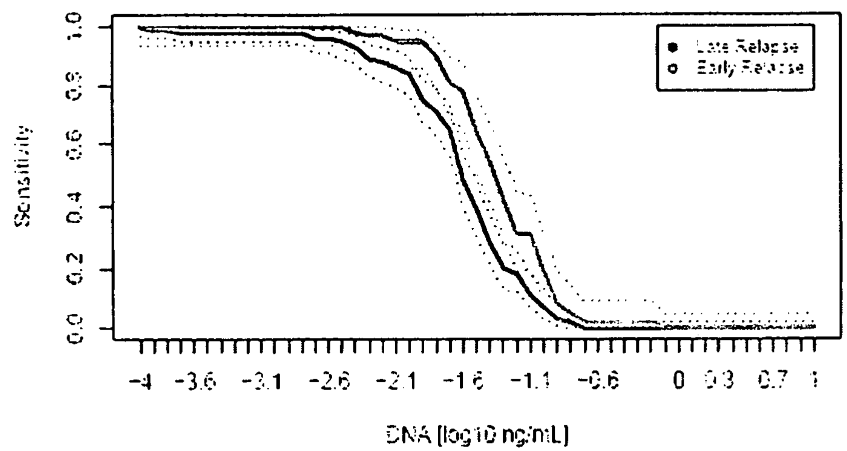


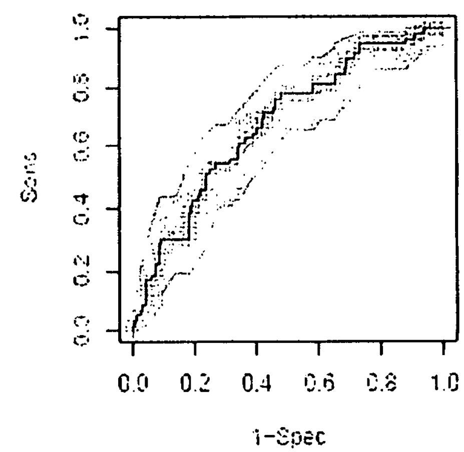


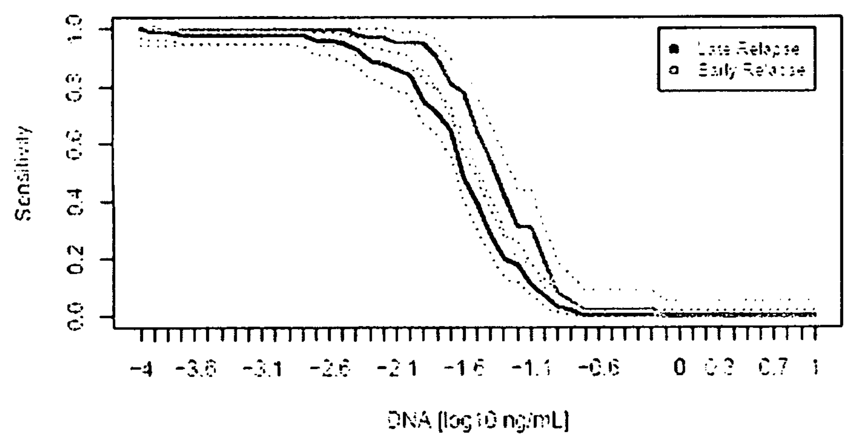


| | |
|---|---|
| AUC | 0.68 [0.61,0.75] |
| Sens / Spec | 0.31 / 0.86 |
| Wilcoxon *P* | 1e-04 |
| Early Relapse (pos) | 59 |
| Late Relapse (neg) | 132 |

Figure 112

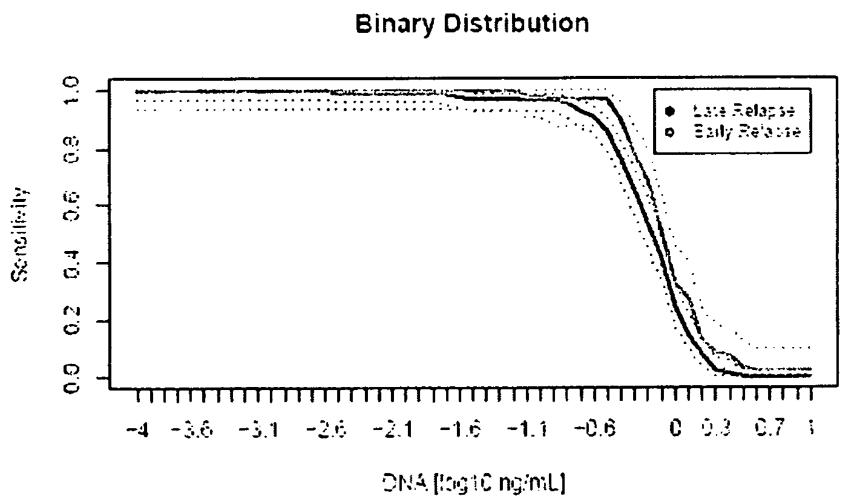


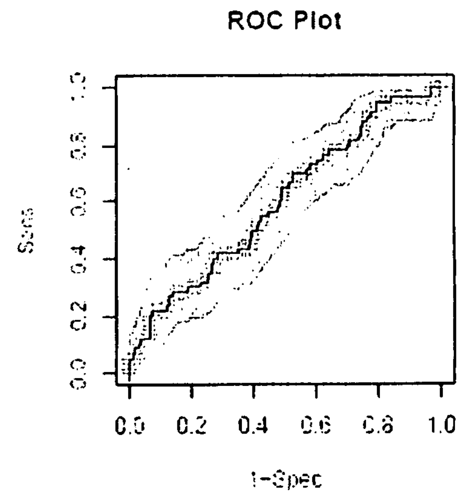


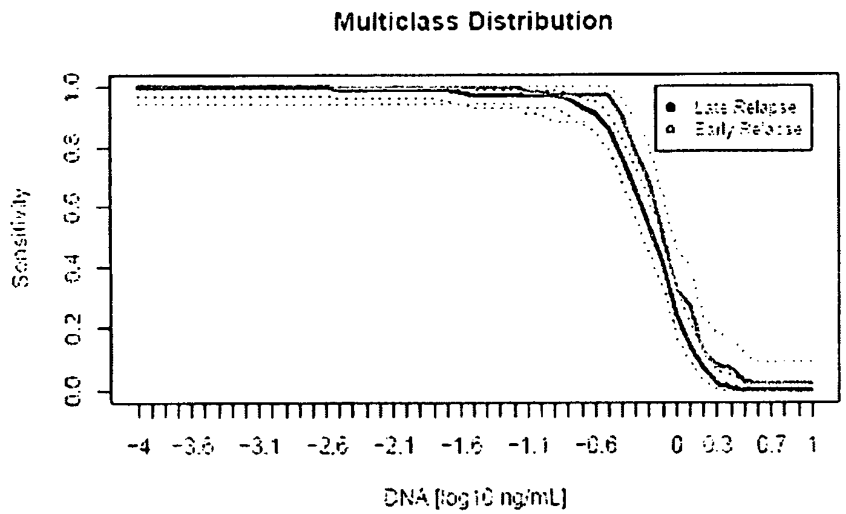


| | |
|---|---|
| AUC | 0.6 [0.53,0.67] |
| Sens / Spec | 0.28 / 0.85 |
| Wilcoxon *P* | 0.0264 |
| Early Relapse (pos) | 60 |
| Late Relapse (neg) | 134 |

Figure 113

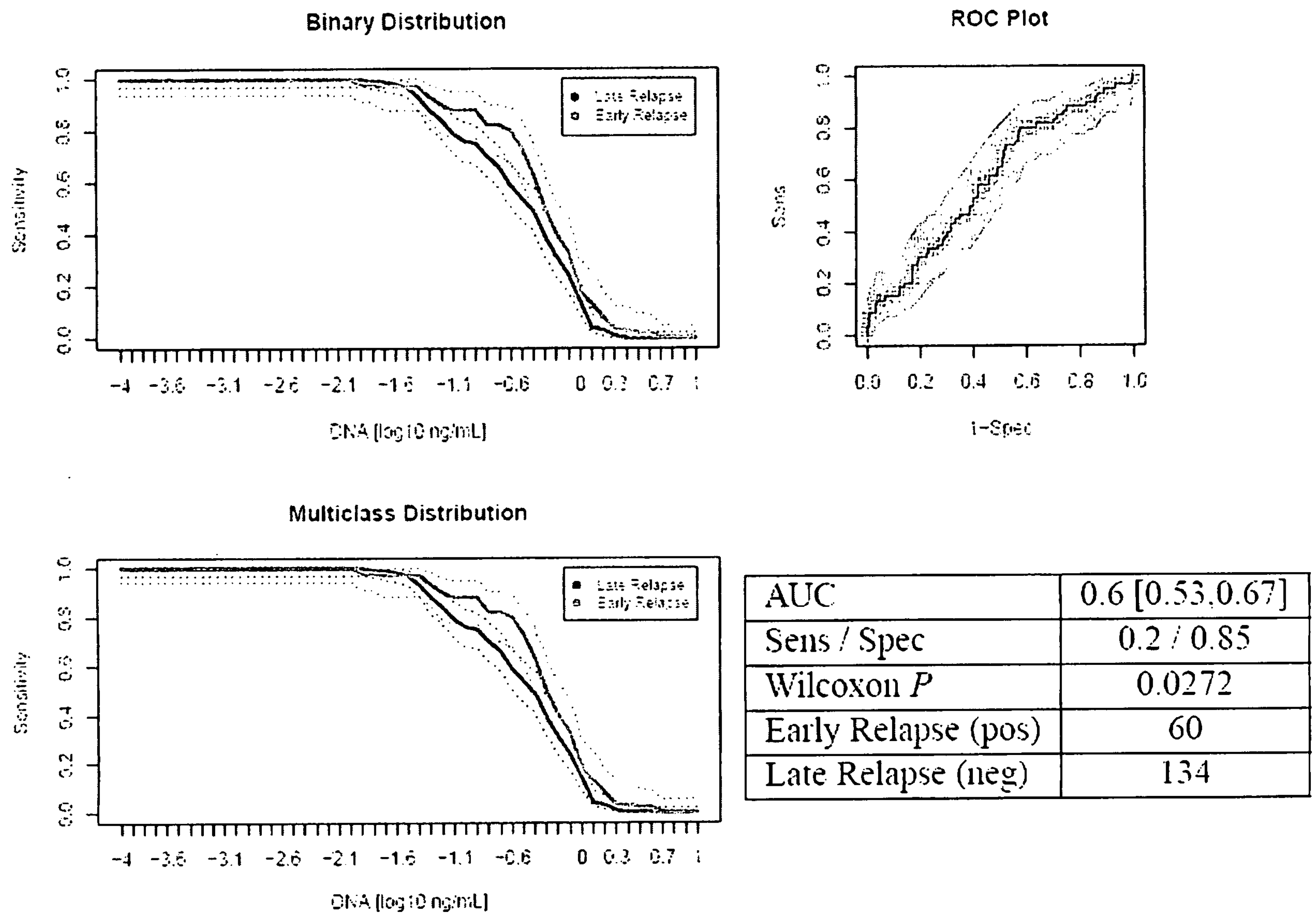


| | |
|---|---|
| AUC | 0.6 [0.53,0.67] |
| Sens / Spec | 0.2 / 0.85 |
| Wilcoxon *P* | 0.0272 |
| Early Relapse (pos) | 60 |
| Late Relapse (neg) | 134 |

Figure 114

Binary Distribution

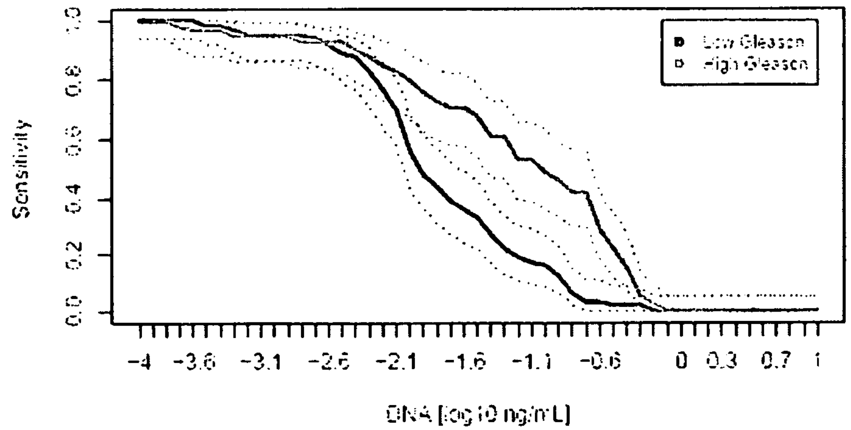


ROC Plot

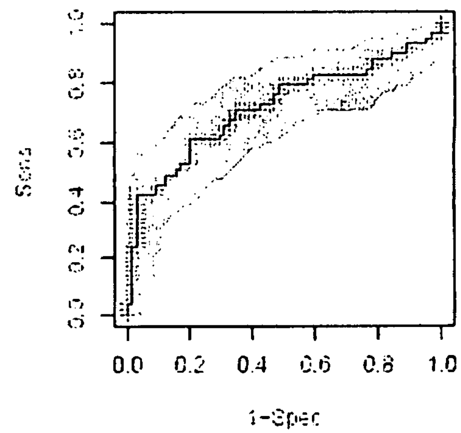


Multiclass Distribution

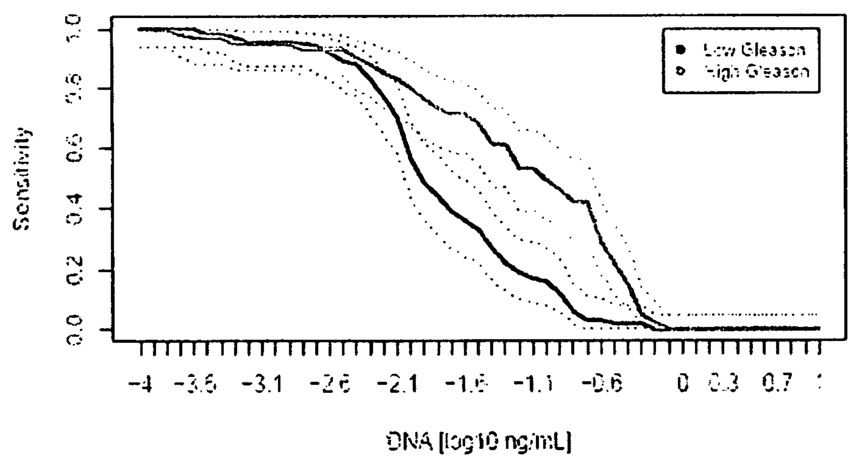


| | |
|---|---|
| AUC | 0.72 [0.63,0.79] |
| Sens / Spec | 0.49 / 0.86 |
| Wilcoxon *P* | 0 |
| High Gleason (pos) | 59 |
| Low Gleason (neg) | 64 |

Figure 115

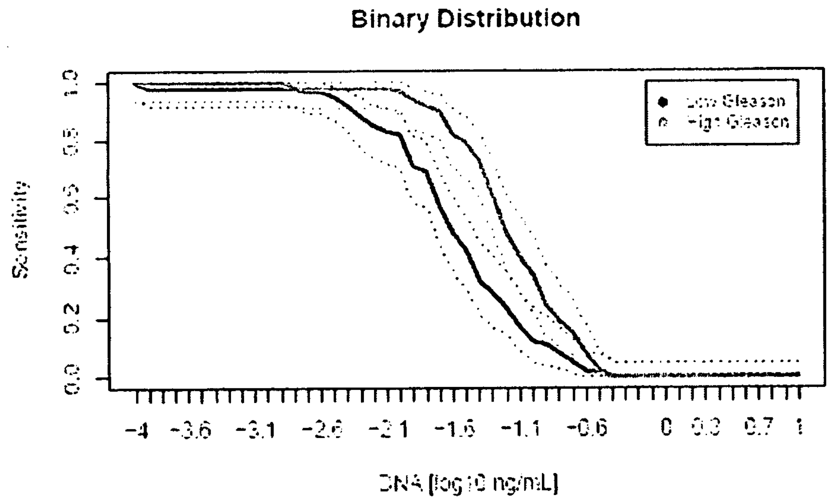


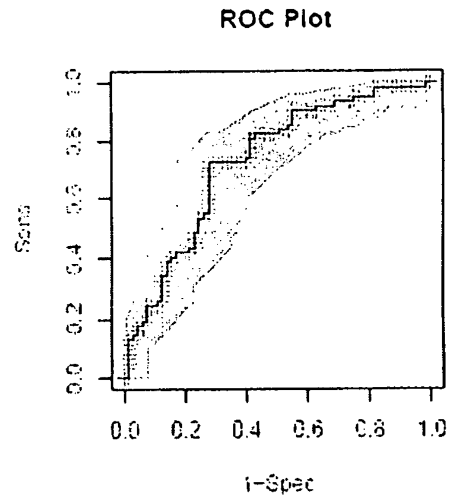


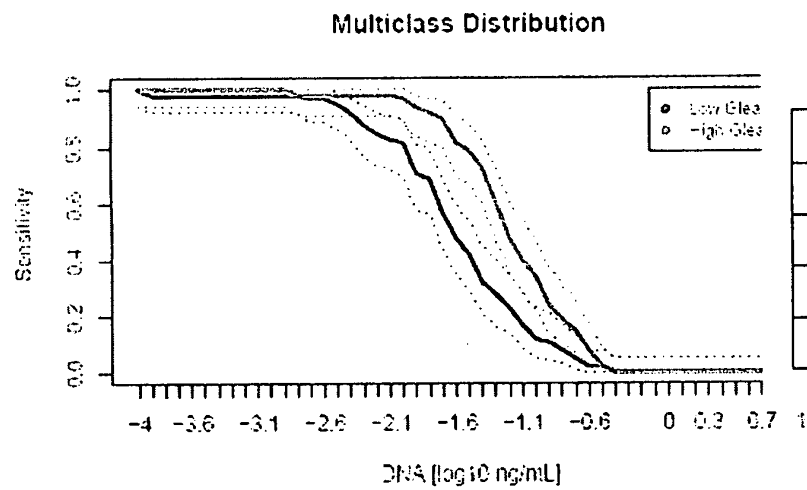


| | |
|---|---|
| AUC | 0.73 [0.64.0.8] |
| Sens / Spec | 0.39 / 0.86 |
| Wilcoxon *P* | 0 |
| High Gleason (pos) | 62 |
| Low Gleason (neg) | 65 |

Figure 116

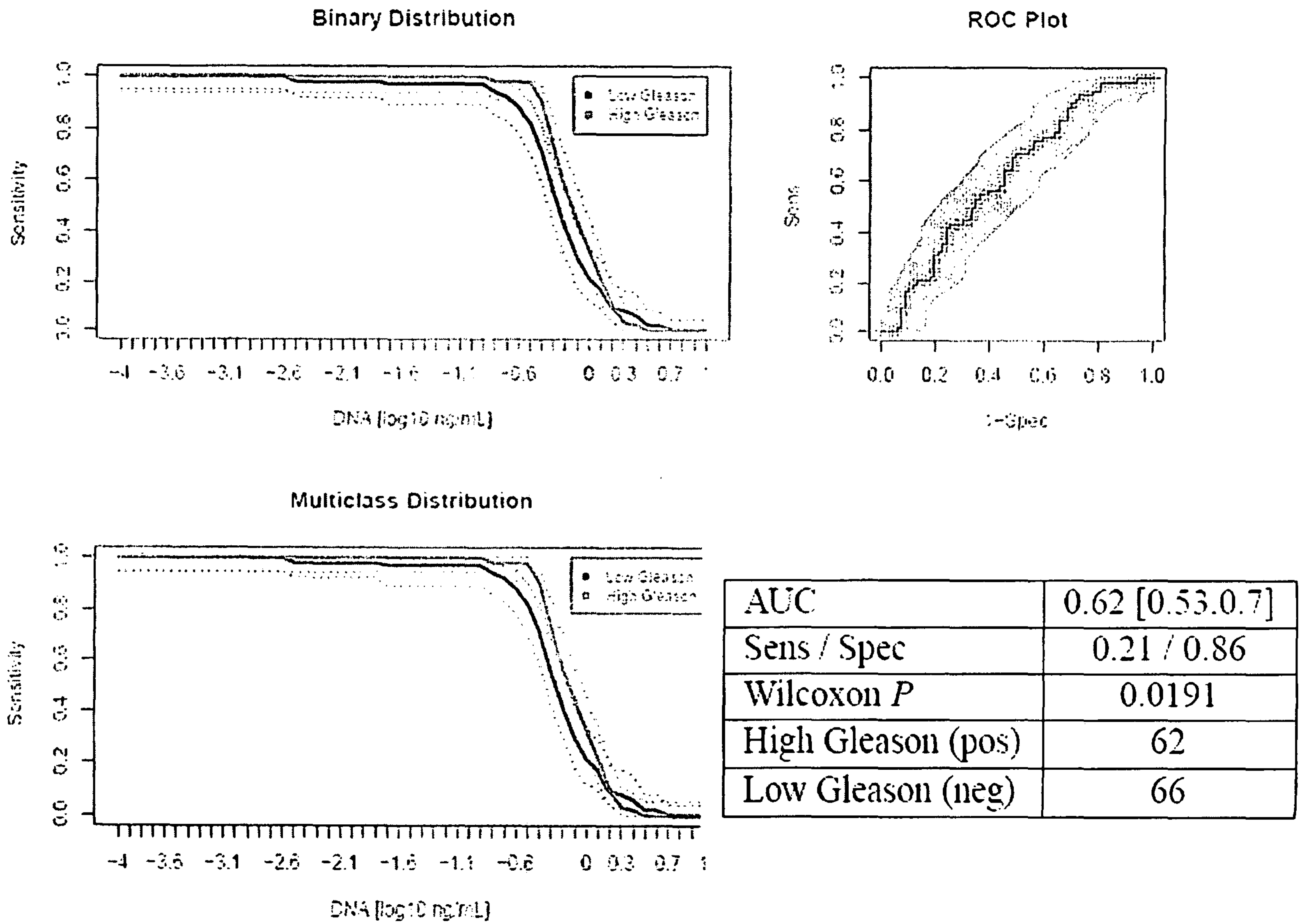


| | |
|---|---|
| AUC | 0.62 [0.53.0.7] |
| Sens / Spec | 0.21 / 0.86 |
| Wilcoxon *P* | 0.0191 |
| High Gleason (pos) | 62 |
| Low Gleason (neg) | 66 |

Figure 117

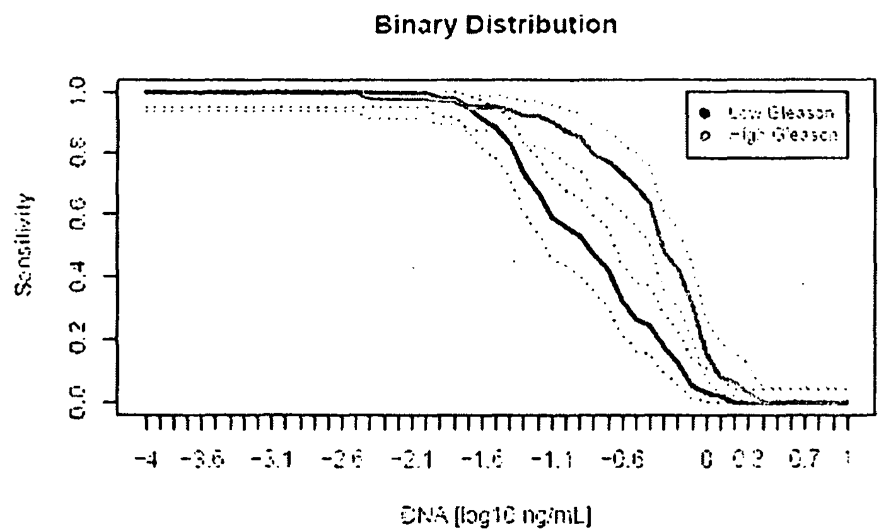


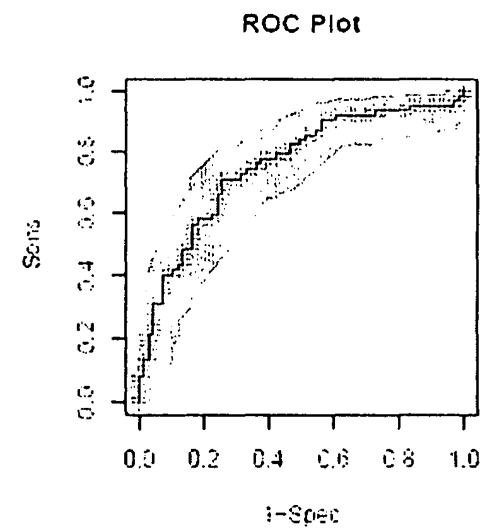


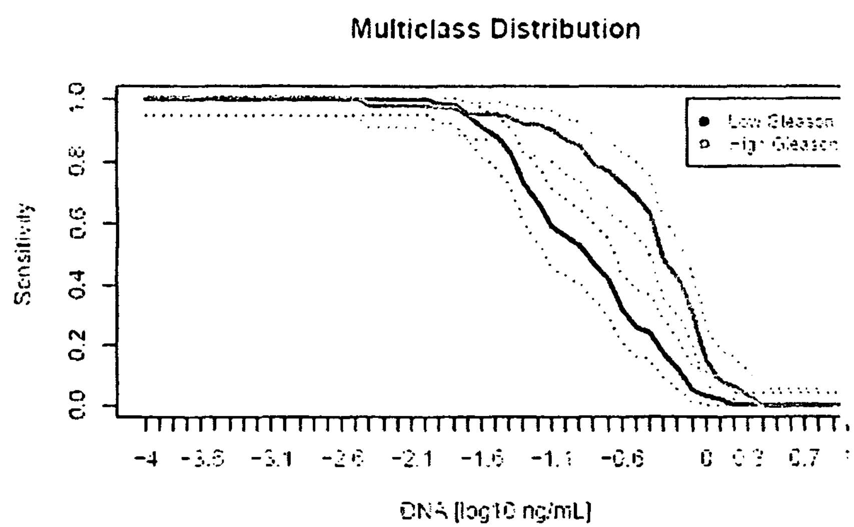


| | |
|---|---|
| AUC | 0.76 [0.67,0.83] |
| Sens / Spec | 0.48 / 0.86 |
| Wilcoxon *P* | 0 |
| High Gleason (pos) | 62 |
| Low Gleason (neg) | 66 |

Figure 118

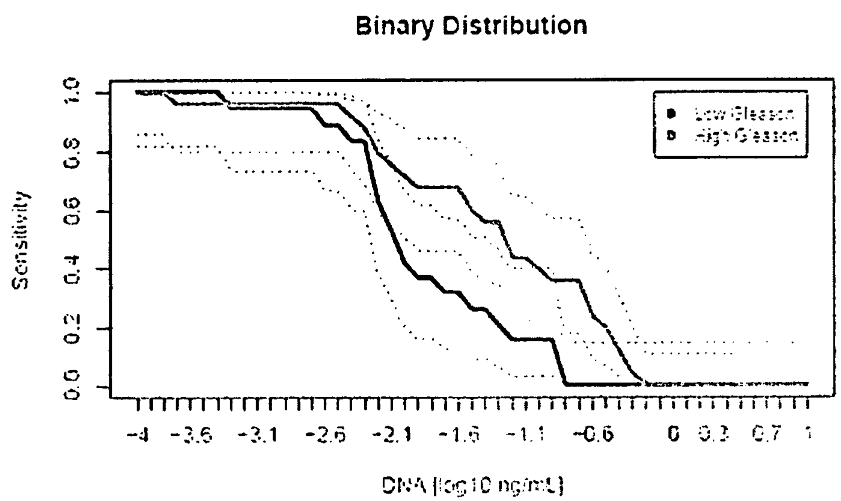


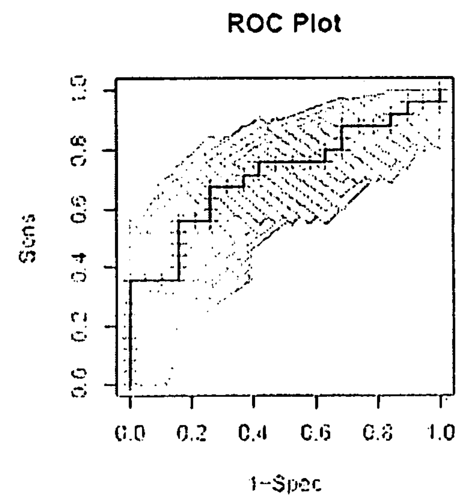


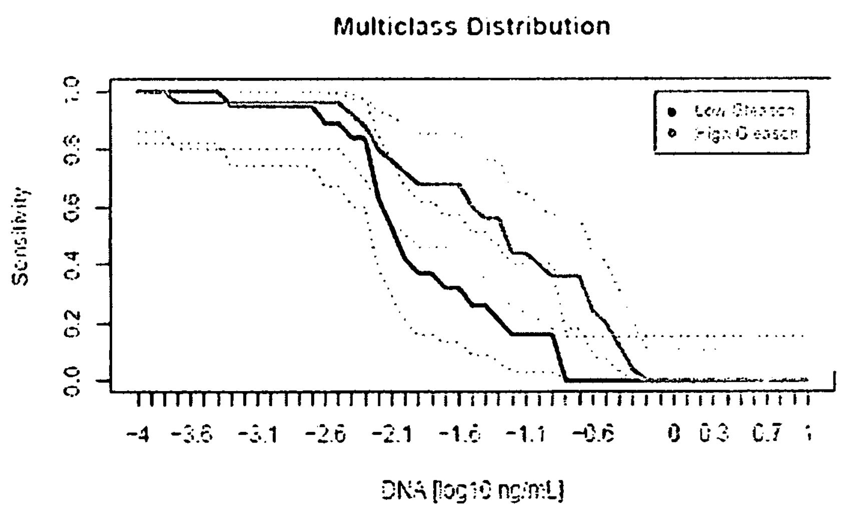


| | |
|---|---|
| AUC | 0.72 [0.55,0.83] |
| Sens / Spec | 0.36 / 0.89 |
| Wilcoxon *P* | 0.0146 |
| High Gleason (pos) | 25 |
| Low Gleason (neg) | 19 |

Figure 119

Binary Distribution

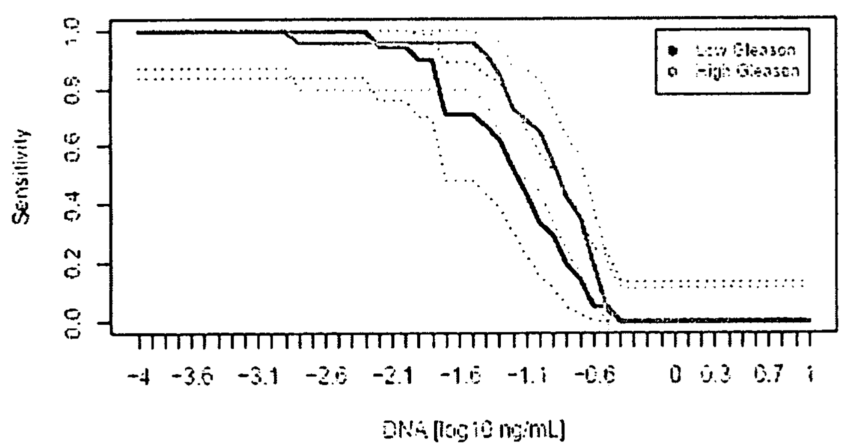


ROC Plot

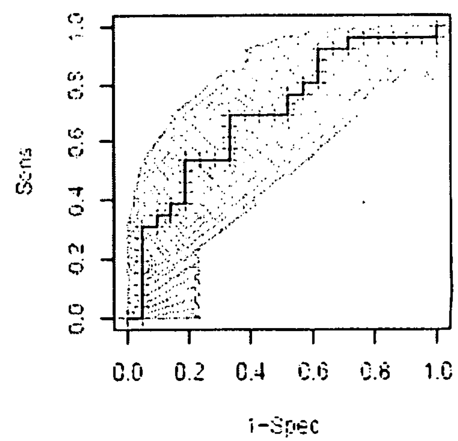


Multiclass Distribution

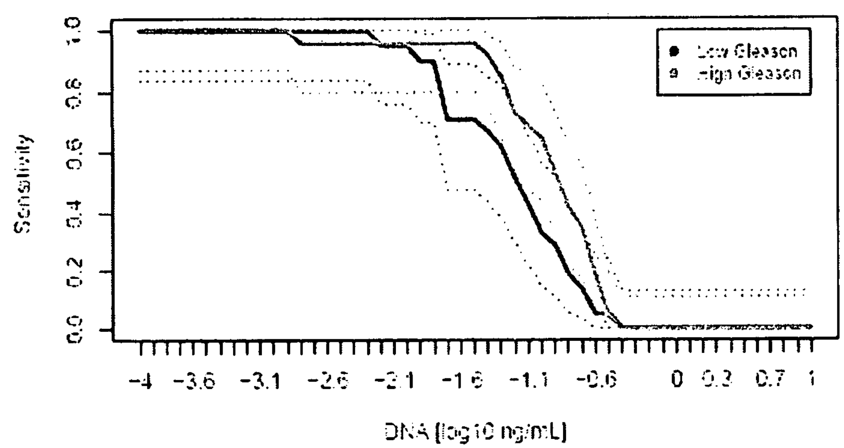


| | |
|---|---|
| AUC | 0.7 [0.55,0.83] |
| Sens / Spec | 0.38 / 0.86 |
| Wilcoxon *P* | 0.0217 |
| High Gleason (pos) | 26 |
| Low Gleason (neg) | 21 |

Figure 120

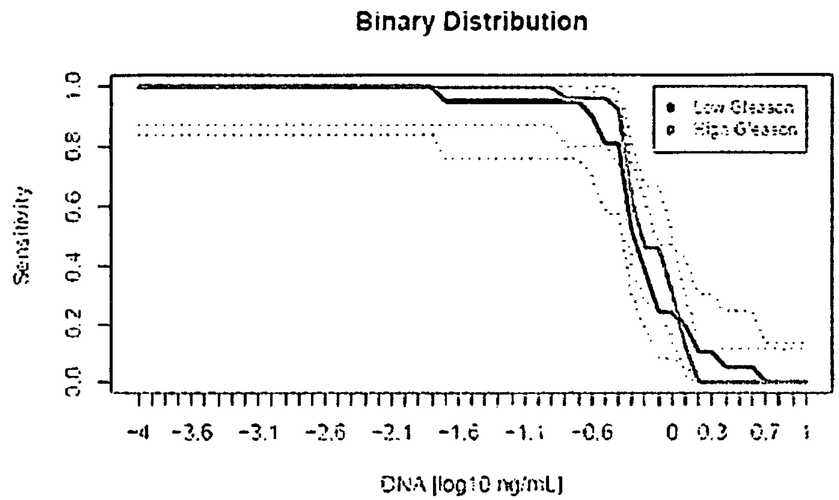


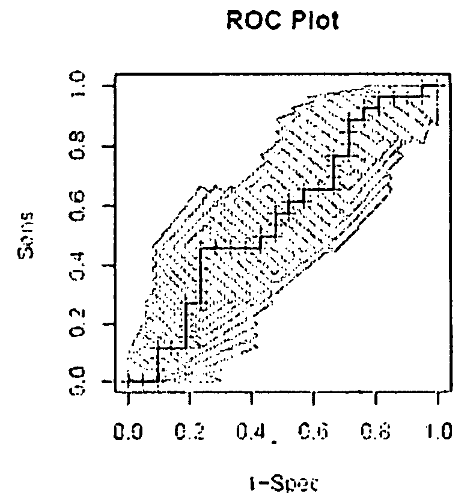


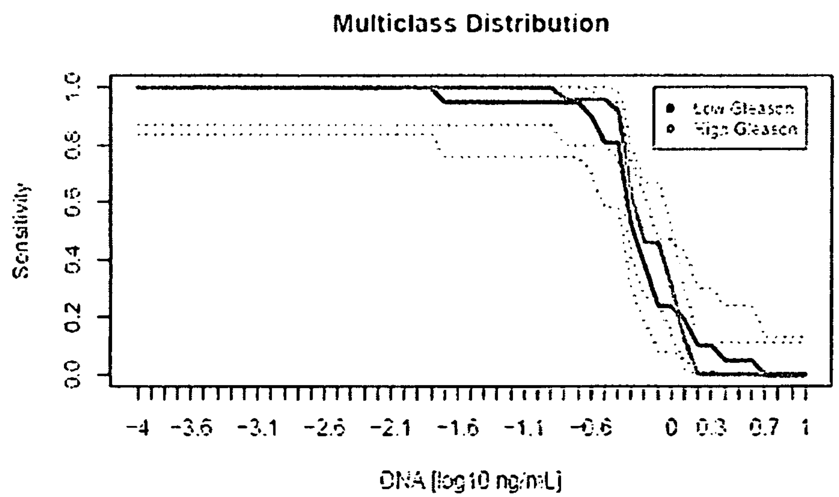


| | |
|---|---|
| AUC | 0.56 [0.4,0.7] |
| Sens / Spec | 0.12 / 0.86 |
| Wilcoxon *P* | 0.4771 |
| High Gleason (pos) | 26 |
| Low Gleason (neg) | 21 |

Figure 121

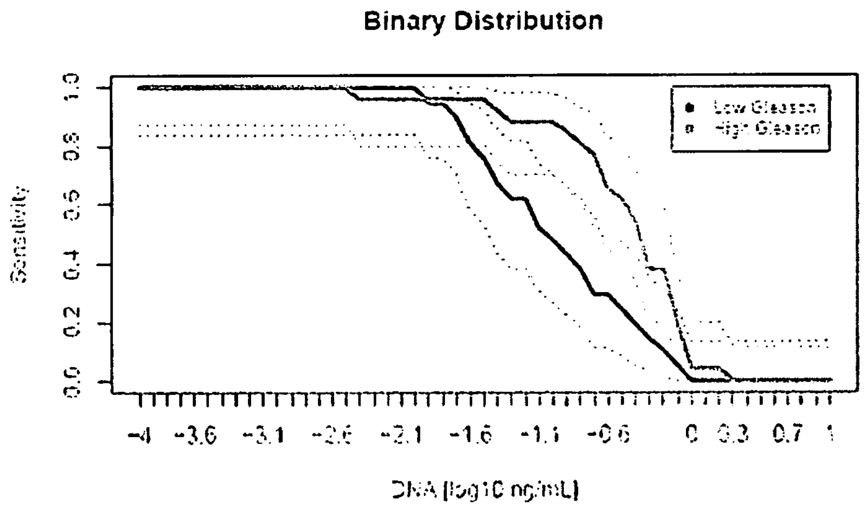


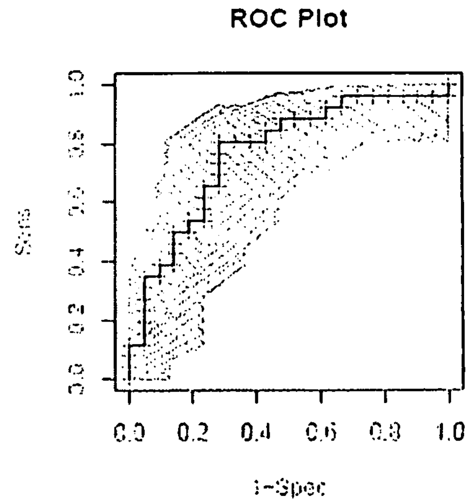


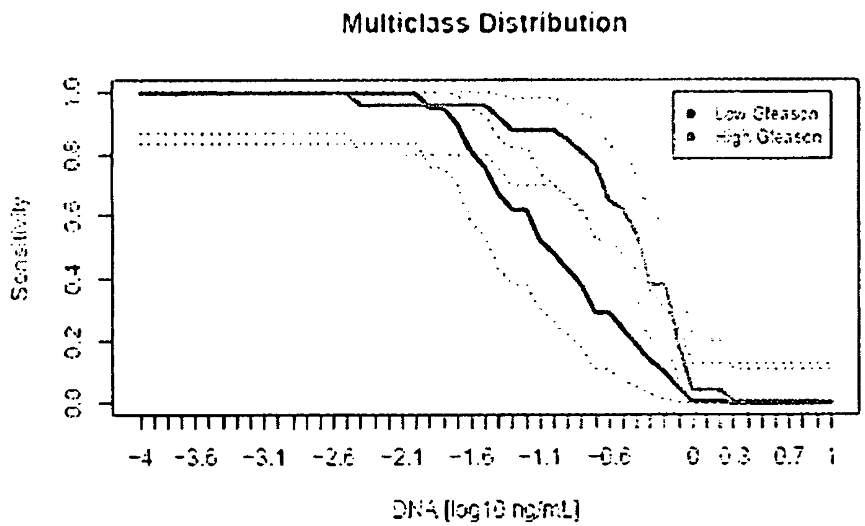


| | |
|---|---|
| AUC | 0.77 [0.62,0.88] |
| Sens / Spec | 0.5 / 0.86 |
| Wilcoxon *P* | 0.0014 |
| High Gleason (pos) | 26 |
| Low Gleason (neg) | 21 |

Figure 122

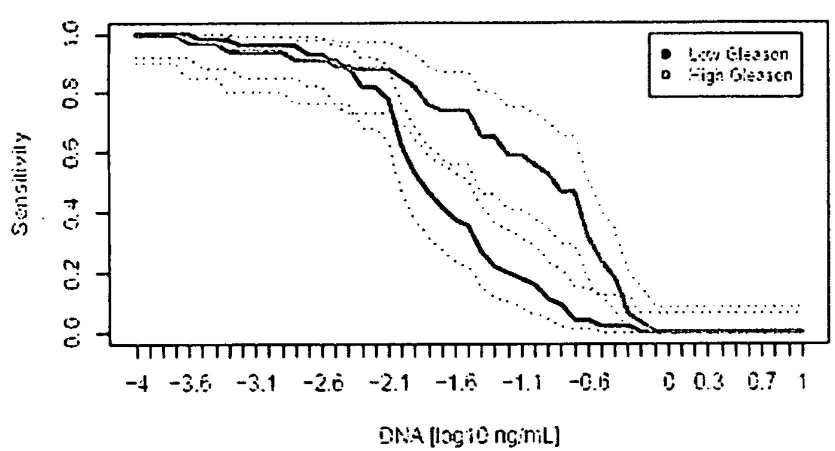


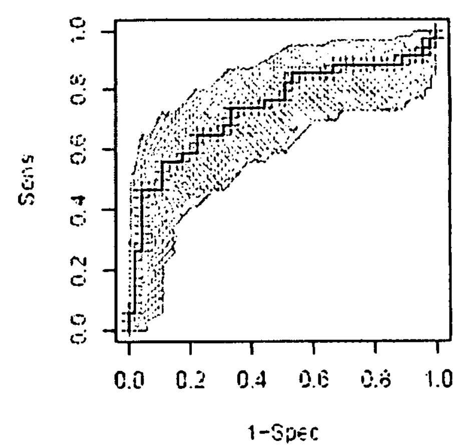


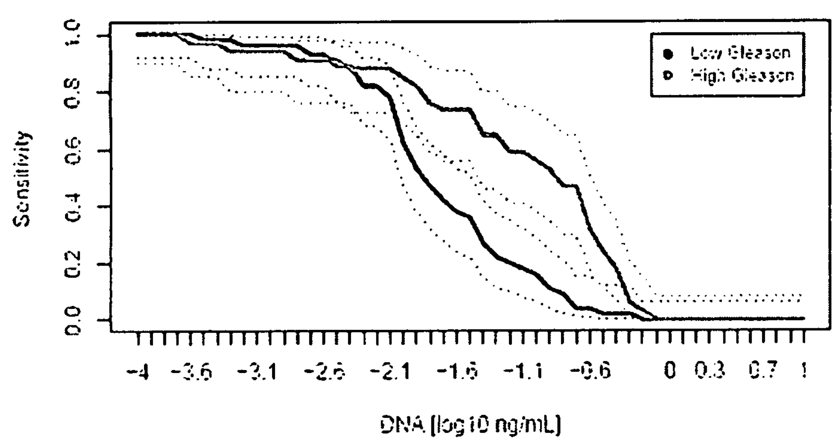


| | |
|---|---|
| AUC | 0.74 [0.62,0.83] |
| Sens / Spec | 0.56 / 0.87 |
| Wilcoxon *P* | 2e-04 |
| High Gleason (pos) | 34 |
| Low Gleason (neg) | 45 |

Figure 123

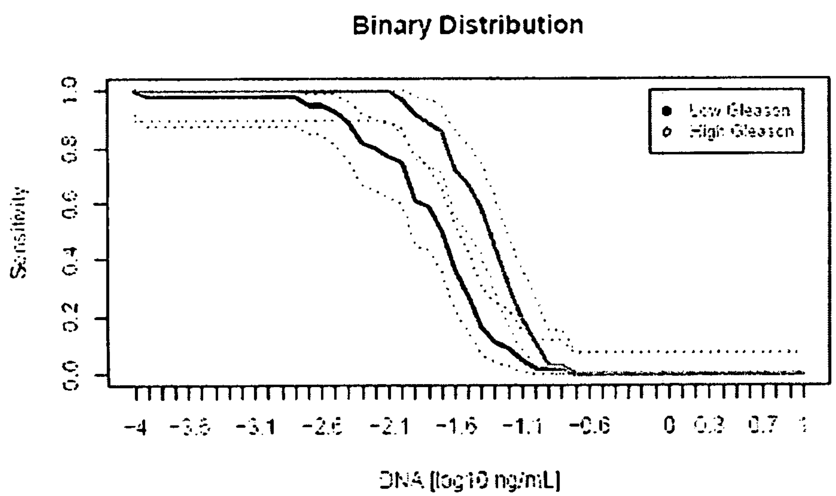


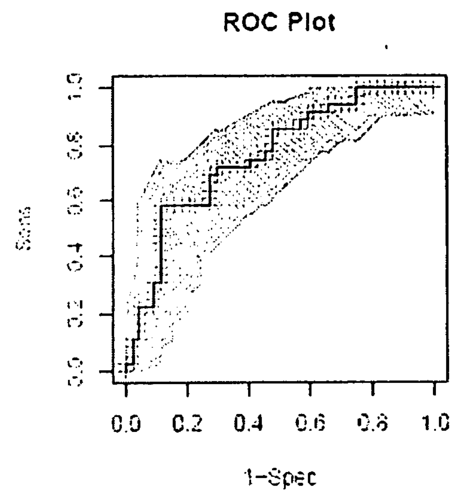


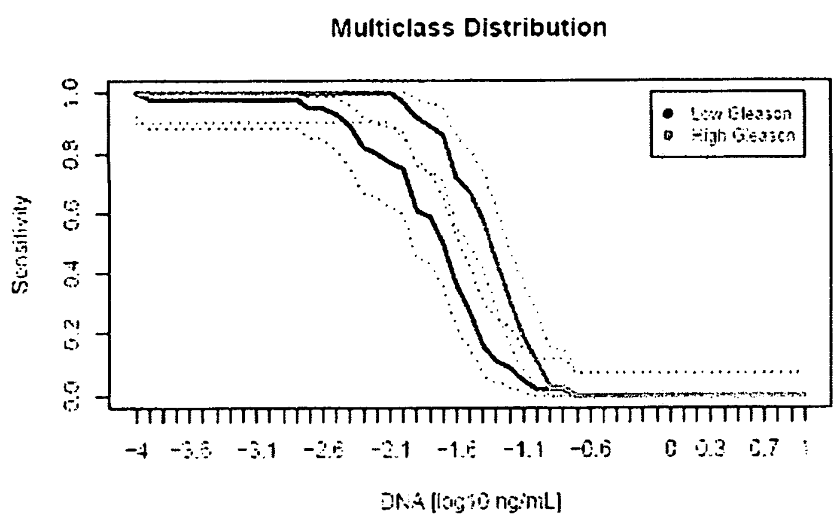


| | |
|---|---|
| AUC | 0.76 [0.65,0.85] |
| Sens / Spec | 0.58 / 0.86 |
| Wilcoxon *P* | 0 |
| High Gleason (pos) | 36 |
| Low Gleason (neg) | 44 |

Figure 124

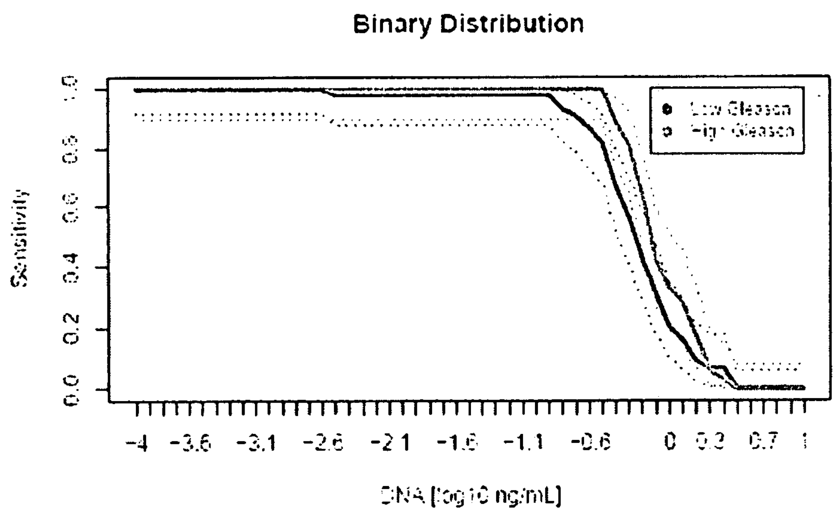


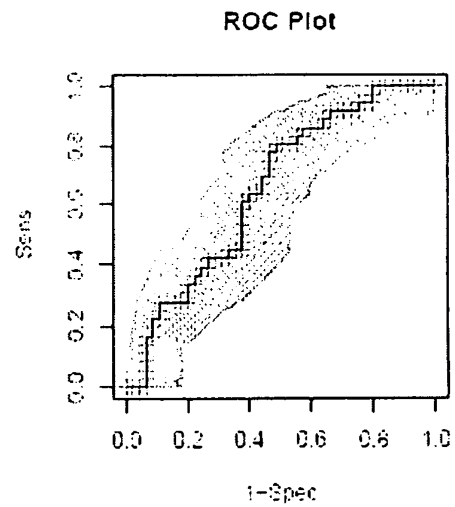


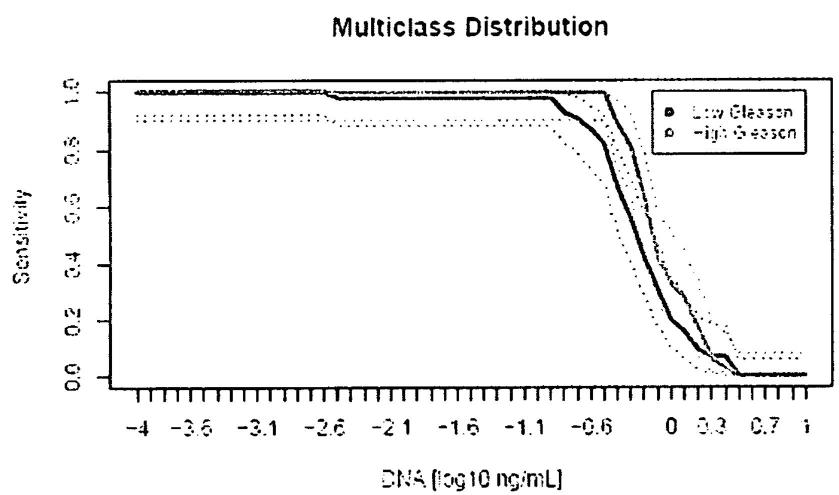


| | |
|---|---|
| AUC | 0.65 [0.54,0.76] |
| Sens / Spec | 0.28 / 0.87 |
| Wilcoxon *P* | 0.0186 |
| High Gleason (pos) | 36 |
| Low Gleason (neg) | 45 |

Figure 125

**Binary Distribution**

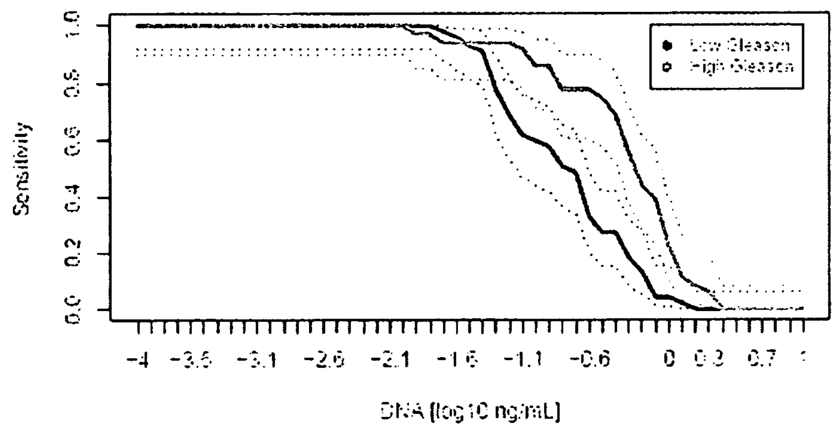


**ROC Plot**

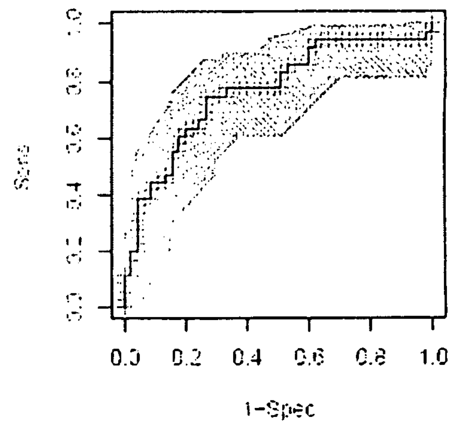


**Multiclass Distribution**

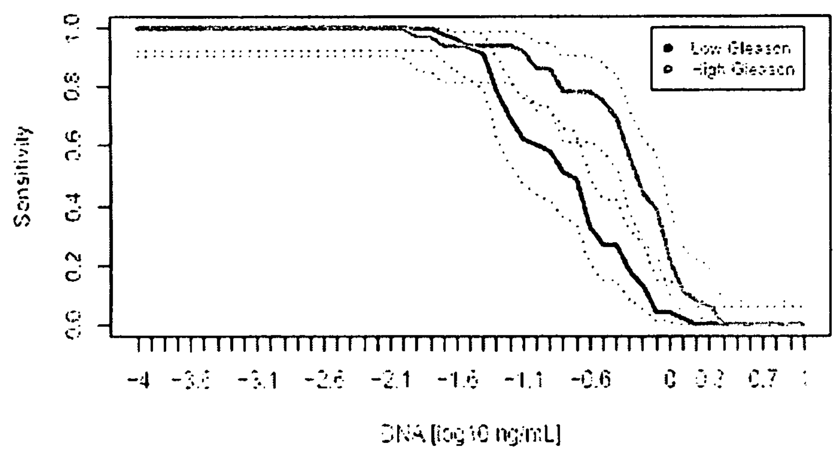


| | |
|---|---|
| AUC | 0.77 [0.66.0.85] |
| Sens / Spec | 0.47 / 0.87 |
| Wilcoxon *P* | 0 |
| High Gleason (pos) | 36 |
| Low Gleason (neg) | 45 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2004146868 A
- WO 2005059172 A
- WO 0119845 A
- EP 1379694 A
- WO 02070742 A
- WO 2004035803 A
- US 5786146 A
- WO 0026401 A1
- WO 9500669 A
- US 6251594 B
- WO 9928498 A, Olek
- WO 9746705 A
- WO 9515373 A
- US 5514758 A
- US 5565552 A
- US 5567810 A
- US 5574142 A
- US 5585481 A
- US 5587371 A
- US 5597696 A
- US 5958773 A
- US 20030013091 A
- US 09898743 B
- US 6265171 B, Herman
- US 6331393 B


### Non-patent literature cited in the description


- **Lipshutz, R. J. et al.** *Nature Genetics,* 1999, vol. 21, 20-24
- **Bowtell, D. D. L.** *Nature Genetics,* 1999, 25-32
- **Santourlidis, S. et al.** *Prostate,* 1999, vol. 39, 166-74
- *Jpn J Clin Oncol.,* 2004, vol. 4, 414-9
- *J Urol,* 2004, vol. 171, 187-91
- **Zhou et al.** *J Urol,* 2004, vol. 171, 2195-8
- **Rabbani et al.** *The FASEB Journal,* 2003, vol. 17, 1081-1088
- **J.P. Egan.** Signal Detection Theory and ROC Analysis. Academic Press, 1975
- **Gonzalgo et al.** *Cancer Research,* 1997, vol. 57, 594-599
- **Eads et al.** *Cancer Res.,* 1999, vol. 59, 2302-2306
- **Gonzalgo ; Jones.** *Nucleic Acids Res.,* 1997, vol. 25, 2529-2531
- **Herman et al.** *Proc. Natl. Acad. Sci. USA,* 1996, vol. 93, 9821-9826
- **Xiong ; Laird.** *Nucleic Acids Res.,* 1997, vol. 25, 2532-2534
- **Toyota et al.** *Cancer Res.,* 1999, vol. 59, 2307-12
- **Sambrook et al.** Molecular Cloning, A Laboratory Manual. Cold Spring Harbor Press, 1989
- Current Protocols in Molecular Biology. John Wiley & Sons, 1995
- **Olek A et al.** A modified and improved method for bisulfite based cytosine methylation analysis. *Nucleic Acids Res.,* 1996, vol. 24, 5064-6
- **Rein, T. et al.** *Nucleic Acids Res.,* 1998, vol. 26, 2255
- **Zeschnigk M et al.** *Eur J Hum Genet.,* 1997, vol. 5, 94-98
- **Olek ; Walter.** *Nat Genet.,* 1997, vol. 17, 275-6
- **Gonzalgo ; Jones.** *Nucleic Acids Res.,* 1997, vol. 25, 2529-31
- **Xiong ; Laird.** *Nucleic Acids Res.,* 1997, vol. 25, 2532-4
- **Grigg ; Clark.** *Bioessays,* 1994, vol. 16, 431-6
- **Zeschnigk M et al.** *Hum Mol Genet.,* 1997, vol. 6, 387-95
- **Feil R et al.** *Nucleic Acids Res.,* 1994, vol. 22, 695
- **Martin V et al.** *Gene,* 1995, vol. 157, 261-4
- **Frommer et al.** *Proc. Natl. Acad. Sci. USA,* 1992, vol. 89, 1827-1831
- **Sadri ; Homsby.** *Nucl. Acids Res.,* 1996, vol. 24, 5058-5059
- **Xiong ; Laird.** *Nucleic Acids Res,* 1997, vol. 25, 2532-2534
- **Krol et al.** *BioTechniques,* 1988, vol. 6, 958-976
- **Zon.** *Pharm. Res.,* 1988, vol. 5, 539-549
- *Nature Genetics Supplement,* January 1999, vol. 21
- **Yu et al.** *BioTechniques,* 1997, vol. 23, 714-720
- **Karas ; Hillenkamp.** *Anal Chem.,* 1988, vol. 60, 2299-301

- **Gut ; Beck.** *Current Innovations and Future Trends,* 1995, vol. 1, 147-57
- **Gut ; Beck.** *Nucleic Acids Res.,* 1995, vol. 23, 1367-73
- **Heid et al.** *Genome Res.,* 1996, vol. 6, 986-994
- **Sanger F. et al.** *Proc Natl Acad Sci USA,* 1977, vol. 74, 5463-5467
- **Olek et al.** *Nucleic Acids Res,* 15 December 1996, vol. 24 (24), 5064-6